# EUROPEAN PATENT APPLICATION

(11) **EP 4 151 621 A1**
(43) Date of publication of application: **22.03.2023**
(21) Application number: 21817699.8
(22) Date of filing: 31.05.2021
(51) Int. Cl.: C07C 229/56, C07C 311/16, C07C 255/55

(54) **SMALL-MOLECULE INHIBITOR TARGETING EB VIRUS NUCLEAR ANTIGEN PROTEIN, PREPARATION METHOD THEREFOR AND USE THEREOF**

(30) Priority: 02.06.2020 CN 202010491053
(71) Applicant: Shenzhen Bay Laboratory, Shenzhen, Guangdong 518132 (CN); Peking University Shenzhen Graduate School, Shenzhen, Guangdong 518055 (CN)
(72) Inventor: ZHU, Zhendong, Westborough MA 01581 (US); XU, Zhengshuang, Shenzhen, Guangdong 518055 (CN); LI, Ting, Shenzhen, Guangdong 518055 (CN); CHE, Chao, shenzhen, Guangdong 518055 (CN); ZHANG, Qingzhou, Shenzhen, Guangdong 518055 (CN); CHEN, Sigui, Shenzhen, Guangdong 518055 (CN); CHEN, Huangcan, Shenzhen, Guangdong 518055 (CN); XIANG, Dehu, Shenzhen, Guangdong 518055 (CN); YANG, Zhen, Shenzhen, Guangdong 518055 (CN)
(74) Representative: Boult Wade Tennant LLP
(86) International application number: PCT/CN2021/097140
(87) International publication number: WO 2021/244463

(57) **Abstract**

The present invention provides a small-molecule inhibitor targeting an EB virus nuclear antigen protein, and/or a pharmaceutical composition containing the same, which can be used for the treatment of a disease caused by EBNA1 activity, such as, but not limited to, cancer, infectious mononucleosis, chronic fatigue syndrome, multiple sclerosis, systemic lupus erythematosus and/or rheumatoid arthritis. The present invention further provides a small-molecule inhibitor targeting an EB virus nuclear antigen protein, and/or a pharmaceutical composition containing the same, which can be used for the treatment of a disease caused by EBV infection in a lytic and/or latent phase.

## Description

### TECHNICAL FIELD

The present invention provides a small-molecule inhibitor targeting an EB virus nuclear antigen protein, a preparation method therefor, and its use of treating and/or preventing of a disease caused by EBNA1 activity.

### BACKGROUND

Epstein-Barr virus (EBV) is a human herpes virus (Human Herpes Virus 4, HHV-4), which belongs to the γ-herpesvirus subfamily. EBV is a B-lymphotropic virus which commonly infects human. The initial infection of EBV occurs in oropharyngeal squamous epithelial cells, and then the existence of which occurs in B lymphocytes for a long period, showing a latent infection state. Once EBV in the the latent infection state is activated, it may become a cause of many diseases including tumors, so EBV is listed as the first human oncogenic virus by IARC (1997).

EBV invades into the human body through epithelial cells and naive B cells in a dormant state, major gene products expressed by EBV-infected B lymphocytes include: EBV nuclear antigen family (EBNA1, EBNA2, EBNA3a, EBNA3b, EBNA3c, and LP), latent membrane protein (LMP-1, LMP-2A, and LMP-2B), and EBV-encoded RNA (EBER-1, EBER-2). EBV has two forms of infection in B cells: (1) proliferative infection: that is, after EBV infects B cells, gene products in early stage such as EA (early antigen) are first synthesized, followed by DNA replication, VCA (capsular protein) and MA (membrane antigen) synthesis, finally, the complete virus particles are formed and released, and the cells are lysed and die at the same time; (2) non-proliferative infection: after EBV infects B cells, most viral genes are in a latent state, and the cells only synthesize EBNA and LMP at this stage. According to the expression of EBV in different tumors, it can be classified into four types: type I latency causing Burkitt lymphoma and gastric cancer; type II latency causing Hodgkin's lymphoma, nasopharyngeal carcinoma, and T/NK-cell lymphoma; type III causing infectious mononucleosis; and type IV refering to a healthy carrier. Among the three pathogenic latent types, the EBV nuclear antigen EBNA1 is one of the key proteins that the virus must express.

In addition to causing Burkitt lymphoma, EBV was found to be associated with Hodgkin's disease, non-Hodgkin's lymphoma, nasopharyngeal carcinoma, NK/T lymphoma, leiomyosarcoma, and malignant epithelial tumors in the stomach, breast, lung, and so on. Studies have shown that EBNA1 is a protein expressed by EBV virus and related to the replication process of viral DNA, maintaining virus latency and inducing tumor by mutation, causing tumor cell migration and inducing immune escape and other important pathological processes. EBNA1 (virus nuclear antigen 1) is detected in the cases where the above tumor cells are detected to be infected with EBV Therefore, the development of early screening of EBV infection based on EBNA1, and prevention of tumor occurrence and development have received full attention from academic research and clinical medical research, which is particularly important for early clinical diagnosis of nasopharyngeal carcinoma.

In recent years, research on targeting EBNA1 to develop targeted chemotherapeutic medicaments for specific tumors has made some progress, which has laid a foundation for target evaluation of EBNA1 proteins, structure-based drug design, activity evaluation of small molecules, etc. For example, Messick TE et al. applied for two world patent applications WO2016183534A1 and WO2015073 864A1 in 2015 and 2016, disclosing the compound structure of two aromatic rings having alkyne conjugate connections, which are used to inhibit the combination of EBNA1 and DNA, and block the replication of EBV-infected cells, so as to play an anti-tumor effect. In 2019, the group reported detailed research contents, where the VK series of molecules had good inhibitory activity against EBNA1, and had shown good effect at the cellular level and in the nasopharyngeal carcinoma model of mouse. Currently, this series of molecules is in the clinical phase I [Sci. Transl. Med. 2019, 11, eaau5612.]. This result demonstrates that EBNA1 protein can be used as a new drug target for the research and development of a therapeutic medicament against diseases related to EBV infection, including tumors.

The present invention is intended to, for a complexation site for the interaction of EBNA1 protein and DNA, optimize the molecular structure by utilizing a structure-based drug design method and improving the bridging functional groups of the two main fragments of the molecule. The pharmaceutical small molecular compound of the present invention has the activity of inhibiting EBNA1 function at a low protein level, selectively inhibits the proliferation of EBV positive tumor cell chains at a cell level, and simultaneously exhibits excellent medicinal ADMET function, and has better druggability.

### SUMMARY

The present invention provides a compound having a general formula (I), or an enantiomer, a diastereomer, a tautomer, a salt, a crystalline form, a solvate and/or an isotopically substituted derivative thereof serving as an EBNA1 inhibitor:

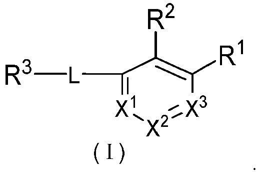

The present invention further provides a pharmaceutical composition, which includes at least one compound having a general formula (I), or an enantiomer, a diastereomer, a tautomer, a salt, a crystalline form, a solvate and/or an isotopically substituted derivative thereof according to the present invention. The present invention further provides a pharmaceutical composition, which includes at least one compound having a general formula (I), or an enantiomer, a diastereomer, a tautomer, a salt, a crystalline form, a solvate and/or an isotopically substituted derivative thereof according to the present invention, and at least one pharmaceutically acceptable carrier, excipient or diluent. The present invention further provides a method of treating and/or preventing a disease or disorder caused by EBNA1 activity. The present invention further provides use in the preparation of a medicament of treating and/or preventing a disease or disorder caused by EBNA1 activity. The present invention further provides a method of treating and/or preventing a disease or disorder caused by and/or associated with Epstein-Barr virus (EBV) infection. The present invention further provides use in the preparation of a medicament of treating and/or preventing a disease caused by and/or associated with Epstein-Barr virus (EBV) infection. The present invention further provides a method of treating and/or preventing EBV infection in a lytic and/or latent phase. The present invention further provides use in the preparation of a medicament of treating and/or preventing a disease caused by EBV infection in a lytic and/or latent phase. The present invention further provides a method for preparing the compound having a general formula (I) according to the present invention.

The present invention relates to a compound having a general formula (I), or an enantiomer, a diastereomer, a tautomer, a salt, a crystalline form, a solvate and/or an isotopically substituted derivative thereof: 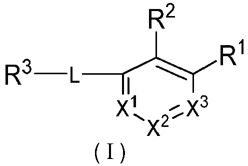 where:
X¹, X², and X³ are the same or different, and are each independently selected from CR^{xa} or a nitrogen atom;
R^{xa} is selected from hydrogen or halogen; the halogen being selected from the group consisting of: fluorine, chlorine, bromine and iodine;
R¹ is selected from the group consisting of: -COOH, -C(=O)-O-R^{1a}, -C(=O)-NH₂, -C(=O)-NHR^{1b}, -C(=O)-NR^{1b}R^{1c}, -C(=O)-NH-S(=O)₂-R^{1b}, -C(=O)-NH-(CH₂)_{q}-R^{1d}, 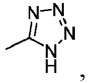 -S(=O)₂OH, -B(OH)₂, -S(=O)₂-NH₂, -S(=O)₂-NHR^{1b}, and -S(=O)₂-NR^{1b}R^{1C},
q is 0, 1, 2 or 3;
where, R^{1a} is independently selected from: Li; or
R^{1a}, R^{1b}, and R^{1c} are the same or different, and are each independently selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, R^{1a}, R^{1b}, and R^{1c} are the same or different, and are each independently selected from the group consisting of: hydrogen, optionally substituted C1-C3 straight chain alkyl, optionally substituted C1-C3 branched chain alkyl, and optionally substituted C1-C3 cycloalkyl;
more preferably, R^{1a}, R^{1b}, and R^{1c} are the same or different, and are each independently selected from: hydrogen or methyl; or
R^{1a}, R^{1b}, and R^{1c} are the same or different, and are each independently selected from the group consisting of: aryl, heterocyclyl and heteroaryl;
the aryl is selected from the group consisting of: phenyl, naphthyl and anthryl;
the heterocyclyl is selected from the group consisting of: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
preferably, the aryl is selected from: phenyl;
the heteroaryl is selected from the group consisting of: thienyl, furyl and imidazolyl;
more preferably, R^{1a}, R^{1b}, and R^{1c} are the same or different, and are each independently selected from the group consisting of: phenyl, thienyl and furyl; or
R^{1b} and R^{1c} together with an atom to which they are attached form a cyclic group; the cyclic group is selected from the group consisting of:
   optionally substituted morpholinyl, optionally substituted pyrrolidinyl, and optionally substituted piperazinyl;
R^{1d} is selected from the group consisting of:
   hydrogen, hydroxyl, amino, cyano, -O-C(=O)-R¹⁷, -O-R¹², -NR⁹R¹⁰, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur; the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
preferably, R^{1d} is selected from the group consisting of: hydrogen, hydroxyl, amino, -O-R¹², -NR⁹R¹⁰, optionally substituted phenyl, optionally substituted thienyl, optionally substituted furyl, and optionally substituted imidazolyl;
more preferably, R^{1d} is selected from the group consisting of: hydrogen, hydroxyl, methyl ether group, amino, dimethylamino, piperazinyl, phenyl, thienyl, and furyl;
R³ is selected from the following groups:
   hydrogen, amino, halogen, nitro, optionally substituted C1-C4 alkyl, 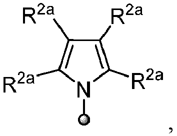 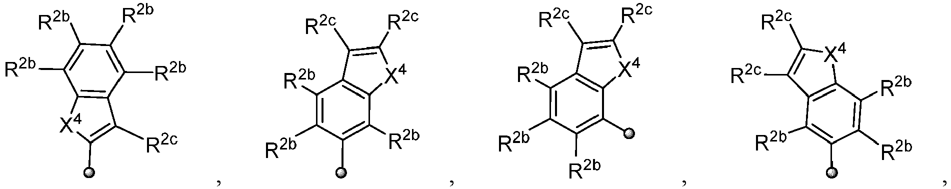 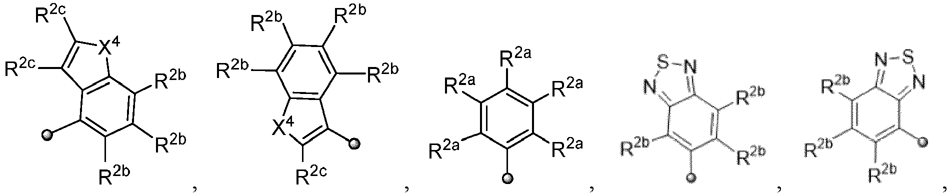 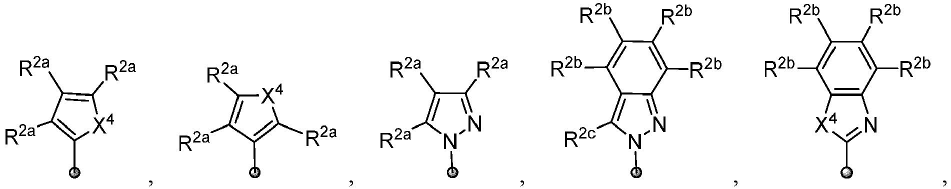 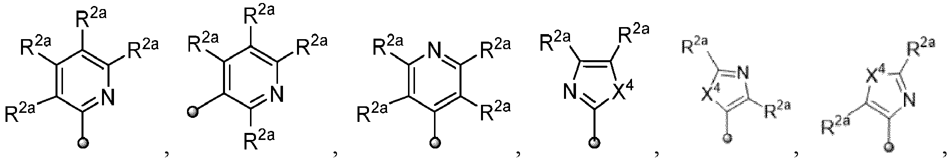 and 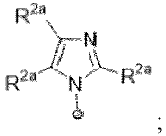
where a dot ● represents a junction of the R² group and other group of a compound molecule of the general formula (I);
X⁴ is selected from the group consisting of O, S and NR^{2d};
where R^{2a}, R^{2b}, and R^{2c} are the same or different, and are each independently selected from the group consisting of:
   hydrogen, halogen, amino, nitro, hydroxyl, C1-C8 hydroxyalkyl, cyano, -(CH₂)_{q}-COOH, -(CH₂)_{q}-C(=O)-O-R⁸, -(CH₂)_{q}-C(=O)-NH-R⁸, -N(H)C(=O)-R⁸, -N(H)S(=O)₂-R⁸, -O-R⁸, -NH-R⁸, -S(=O)₂NH-R⁸, -S(=O)₂-R⁸, -C(=O)-R⁸, -O-C(=O)-R⁸, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, and phenyl; the phenyl being optionally substituted by a substituent R⁴ for one or more times identically or differently;
q is 0, 1, 2 or 3; or
R^{2a} is selected from the group consisting of aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur; the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently; or
adjacent R^{2a} groups together with an atom to which they are attached form a fused ring structure, or, the adjacent R^{2b} groups together with the atom to which they are attached form a fused ring structure, the fused ring structure being selected from: phenyl or heteroaryl, the heteroaryl being selected from the group consisting of: thienyl, furyl, pyrrolyl, imidazolyl, oxazolyl, triazolyl, thiazolyl, pyridyl, pyrimidinyl, indolyl, quinolinyl, quinazolinyl, indazolyl, benzofuryl , benzothienyl, benzoxazolyl, benzotriazolyl, and benzothiazolyl; the phenyl and the heteroaryl being optionally substituted by a substituent R⁴ for one or more times identically or differently;
R^{2d} is selected from the group consisting of hydrogen, optionally halogenated or non-halogenated C1-C3 straight chain alkyl, isopropyl, and cyclopropyl; preferably, the halogenated C1-C3 straight chain alkyl is selected from fluoro C1-C3 straight chain alkyl; or R^{2d} is selected from: -C(=O)-R⁵, or -S(=O)₂-R⁵; or
R^{2d} is selected from the group consisting of aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
L is selected from the following groups: 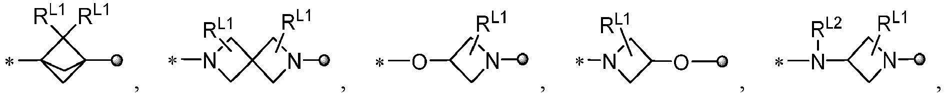 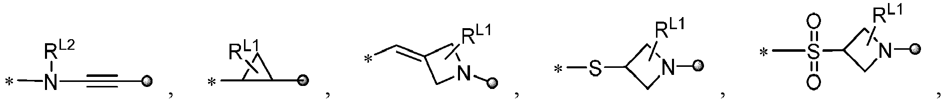 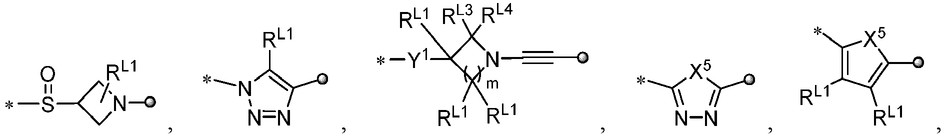 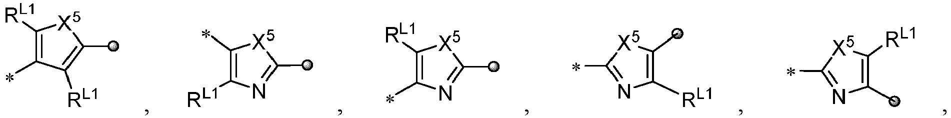 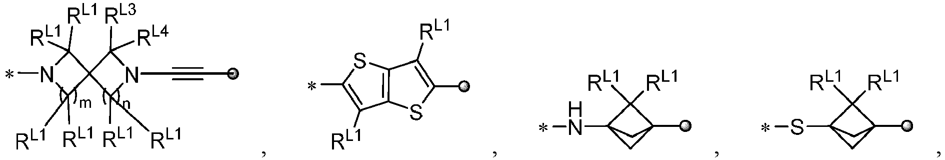 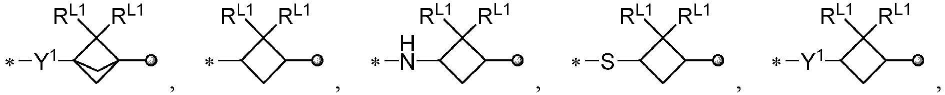 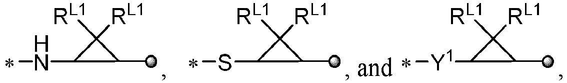
where a dot • represents a junction where L is attached to a fragment on a right side of the compound of the general formula (I);
where an asterisk * represents a junction where L is attached to R³;
X⁵ is selected from the group consisting of: O, NH and S;
Y¹ is selected from: O or NR^{L2};
the L is substituted by a substituent R^{L1} for one or more times at the same or different positions;
R^{L1} is selected from: a hydrogen atom or a fluorine atom;
R^{L2} is selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R^{L2} is selected from the group consisting of: -(CH₂)_{q}-R⁷, -C(=O)-N(H)-R¹⁹, -C(=S)-N(H)-R¹⁹, -C(=O)-R²⁰, and -S(=O)₂-R²⁰; or
R^{L2} is selected from the group consisting of:
   aryl-(CH₂)_{q}-, heterocyclyl-(CH₂)_{q}-, and heteroaryl-(CH₂)_{q}-;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{L2} and R³ are cyclized together with an atom to which they are attached to form 5-10 membered heterocyclyl containing nitrogen and/or sulfur, the 5-10 membered heterocyclyl containing nitrogen and/or sulfur including -C(=O)- and -S(=O)₂- groups;
R^{L3} and R^{L4} are the same or different, and are each independently selected from: a hydrogen atom or a fluorine atom; or
R^{L3} and R^{L4} together with the atom to which they are attached form carbonyl or thiocarbonyl; or
R^{L3} and R^{L4} together with the atom to which they are attached form a heterocyclyl containing nitrogen and/or sulfur,
   where C atom attached to both R^{L3} and R^{L4} is replaced by -S(=O)₂-;
m and n are the same or different, and are each independently selected from: 1, 2 or 3, so as to form a ring system with various numbers of atoms;
q is 0, 1, 2 or 3;
R³ is selected from the following groups:
   C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl; the C1-C8 straight chain alkyl, the C3-C8 branched chain alkyl, the C3-C8 cycloalkyl, the halogenated C1-C8 straight chain alkyl, the halogenated C3-C8 branched chain alkyl, and the halogenated C3-C8 cycloalkyl being optionally substituted by a substituent R⁶ for one or more times identically or differently; or
R³ is selected from the following groups:
   (1) 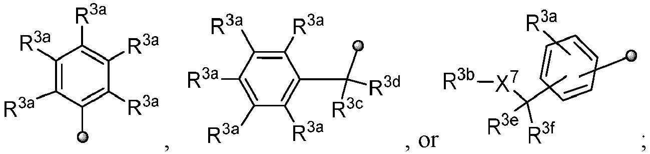 or
   (2) 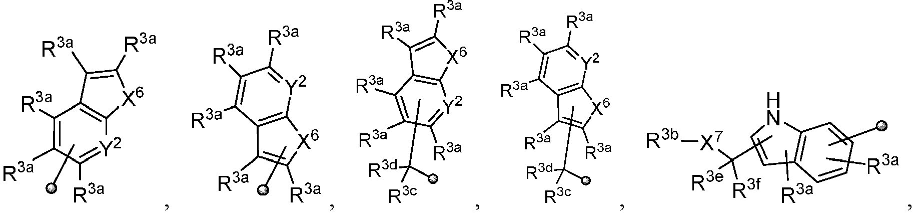 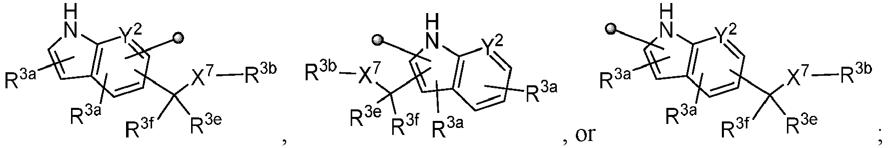 or
   (3) 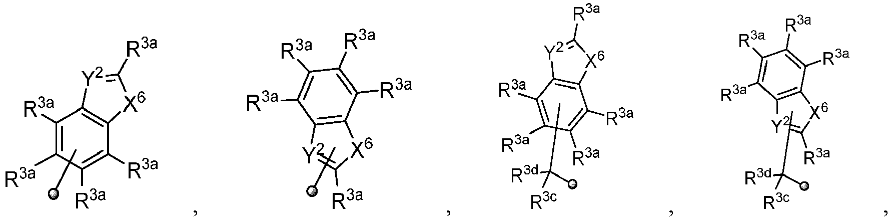 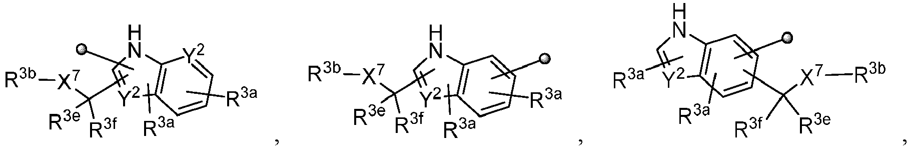 or 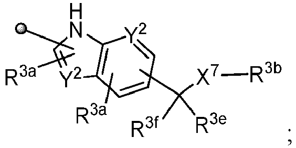 or
   (4) 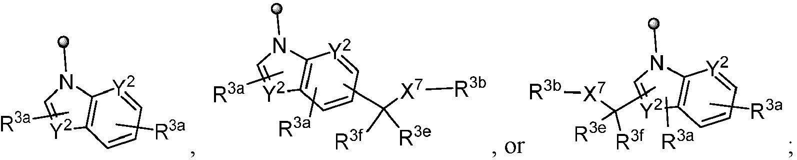 or
   (5) 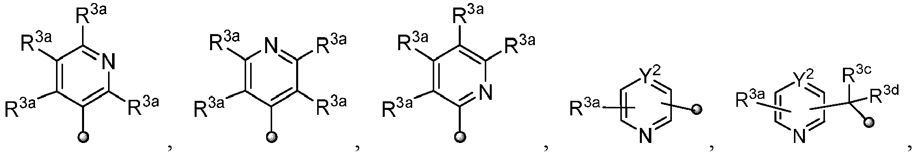 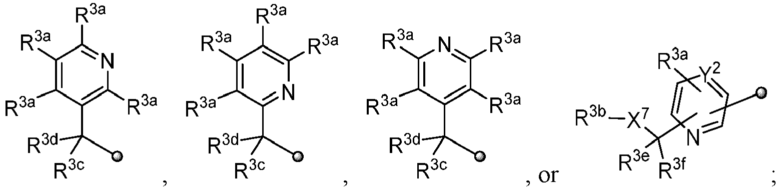 or
   (6) 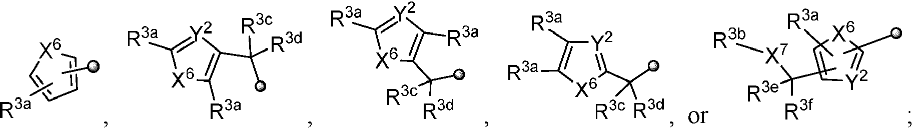 or
   (7) 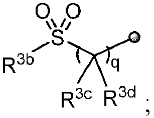 or
   (8) 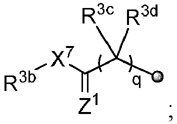
where a dot ● represents a junction which is attached to a fragment on a right side of the compound of the general formula (I);
X⁶ is selected from the group consisting of O, S and NR^{3g};
X⁷ is selected from the group consisting of O, S, NR^{3g} and CHR^{3a};
Y² is selected from the group consisting of CH, CR^{3a} and N, and Y² may replace a C atom at an optional position of an aromatic ring;
Z¹ is selected from the group consisting of O, S and NR^{3h};
where R^{3a} is selected from the group consisting of: hydrogen, halogen, nitro, cyano, sulfo-group, optionally substituted sulfonamido, optionally substituted C1-C8 straight chain alkyl, optionally substituted C3-C8 branched chain alkyl, optionally substituted C3-C8 cycloalkyl, optionally substituted halogenated C1 -C8 straight chain alkyl, optionally substituted halogenated C3-C8 branched chain alkyl, optionally substituted halogenated C3-C8 cycloalkyl, hydroxyl, C1-C8 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C8 hydroxyalkyl being C1-C8 alkyl substituted with hydroxyl;
p and q are each independently 0, 1, 2 or 3;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
adjacent R^{3a} groups together with an atom to which they are attached form a cyclic structure, or R^{3a} and its adjacent R^{3c} together with an atom to which they are attached form a cyclic structure, or R^{3a} and its adjacent R^{3d} together with an atom to which they are attached form a cyclic structure, the cyclic structure including a saturated or aromatic cyclic structure, the cyclic structure being selected from the group consisting of: cycloalkyl, aryl, heterocyclyl and heteroaryl;
preferably, the cyclic structure is selected from the group consisting of: phenyl, pyridyl, pyrrolyl, thienyl, furyl, oxazolyl, thiazolyl, isoxazolyl, indazolyl, and 5-7 membered saturated heterocyclyl containing nitrogen/oxygen, the saturated heterocyclyl being selected from: tetrahydrofuranyl, tetrahydropyranyl, oxepanyl, pyrrolidinyl, piperidinyl, azepanyl, morpholinyl and piperazinyl;
the cyclic structure is optionally substituted by a substituent R¹⁸ for one or more times identically or differently;
R^{3b} is selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S)N(H)-R¹⁹, -C(=O)O-R¹⁹, and -S(=O)2-R²⁰;
q is 0, 1, 2 or 3; or
R^{3b} is selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3c} and R^{3d} are the same or different, and are each independently selected from the group consisting of:
   hydrogen, fluorine, C1-C8 alkyl, aryl-(CH₂)_{q}-, heterocyclyl-(CH₂)_{q}-, and heteroaryl-(CH₂)_{q}-; or
when L is selected from the group consisting of 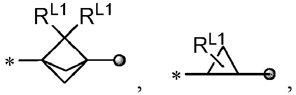 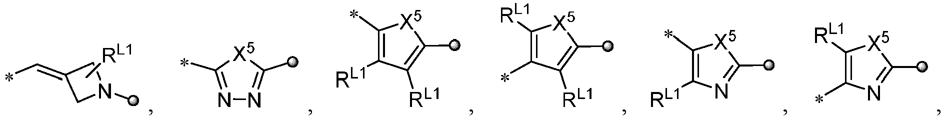 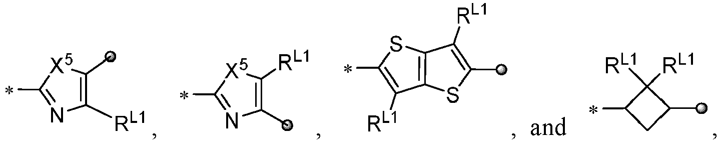 R^{3c} and R^{3d} are the same or different, and are each independently selected from the group consisting of: hydrogen, fluorine, C1-C8 alkyl, hydroxyl, C1-C8 hydroxyalkyl, -O-C(=O)-R²⁰, aryl-(CH₂)_{q}-, heterocyclyl-(CH₂)_{q}-, and heteroaryl-(CH₂)_{q}-;
the aryl is selected from: optionally substituted phenyl, naphthyl, anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{3c} and R^{3d} together with an atom to which they are attached form carbonyl, thiocarbonyl, or imino;
R^{3e} and R^{3f} are the same or different, and are each independently selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S) N(H)-R¹⁹, -C(=O)O-R¹⁹, and -S(=O)2-R²⁰; or
R^{3e} and R^{3f} together with an atom to which they are attached form carbonyl or thiocarbonyl; or
R^{3e} and R^{3f} are the same or different, and are each independently selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3h} is selected from the group consisting of: hydrogen, nitro and cyano;
the R⁴ is selected from the group consisting of: hydrogen, fluorine, chlorine, hydroxyl, C1-C3 hydroxyalkyl, optionally substituted C1-C3 alkyl, optionally substituted halogenated C1-C3 alkyl, optionally substituted C1-C3 alkoxy, optionally substituted halogenated C1-C3 alkoxy, nitro, cyano, amino, acetyl, methylsulfonyl, acetamido, and methylsulfonamido;
the R⁵ is selected from the group consisting of: hydrogen, optionally substituted C1-C3 alkyl, and optionally substituted halogenated C1-C3 alkyl;
the R⁶ is selected from the group consisting of: hydroxy, C1-C8 straight chain alkoxy, C3-C8 branched chain alkoxy, C3-C8 cycloalkoxy, -O-C(=O)-R²⁰, amino, -NH-C(=O)-R²⁰, -NH-S(=O)₂-R²⁰, -NH-C(=O)-O-R²⁰, -NH-C(=O)-NH-R²⁰, and -NH-C(=S)-NH-R²⁰;
the R⁷ is selected from the group consisting of: phenyl, naphthyl, heterocyclyl, and heteroaryl; the heterocyclyl being selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the phenyl, the naphthyl, the heterocyclyl, and the heteroaryl being optionally substituted by a substituent R¹⁸ for one or more times identically or differently;
the R⁸ is selected from the group consisting of: optionally substituted C1-C3 alkyl, and optionally substituted halogenated C1-C3 alkyl;
the R⁹ and R¹⁰ are the same or different, and are each independently selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
the R⁹ and R¹⁰ are the same or different, and are each independently selected from the group consisting of: optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl; the heterocyclyl containing nitrogen being substituted by the following substituents for one or more times identically or differently: hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, and optionally substituted sulfonamido;
the R¹¹ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
R¹¹ is selected from the group consisting of: aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl and the heteroaryl are substituted by the following substituents for one or more times identically or differently: halogen, hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, optionally substituted sulfonamido, cyano, nitro, sulfo-group, optionally substituted ureido, and guanidyl;
the R¹² is selected from the group consisting of: hydrogen, C1-C8 straight chain alkyl, C3-C8 branched chain alkyl, C3-C8 cycloalkyl, halogenated C1-C8 straight chain alkyl, halogenated C3-C8 branched chain alkyl, halogenated C3-C8 cycloalkyl, aryl, heterocyclyl, and heteroaryl; the C1-C8 alkyl bejing substituted by the following substituents for one or more times identically or differently: -O-R²⁰, -OH, -N(H)C(=O)-R²⁰, and -NR⁹R¹⁰;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R¹³ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
the R¹³ is selected from the group consisting of: -(CH₂)_{q}-phenyl, -(CH₂)_{q}-halogenated phenyl, -(CH₂)_{q}-naphthyl, -(CH₂)_{q}-halogenated naphthyl, -(CH₂)_{q}-anthryl, -(CH₂)_{q}-halogenated anthryl, -(CH₂)_{q}-heterocyclyl, -(CH₂)_{q}-halogenated heterocyclyl, -(CH₂)_{q}-heteroaryl, and -(CH₂)_{q}-halogenated heteroaryl;
q is 0, 1, 2 or 3;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the phenyl, the naphthyl, the anthryl, the heterocyclyl, and the heteroaryl are optionally substituted by a substituent R¹⁸ for one or more times identically or differently;
the R¹⁴ is selected from: heteroaryl, the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the heteroaryl being optionally substituted by a substituent R¹⁸ for one or more times identically or differently;
the R¹⁵ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently;
the R¹⁶ is selected from the group consisting of: hydrogen, optionally substituted C1-C8 straight chain alkyl, optionally substituted C3-C8 branched chain or cycloalkyl, optionally substituted halogenated C1-C8 straight chain alkyl, optionally substituted halogenated C3-C8 branched chain or cycloalkyl, optionally substituted acyl, and optionally substituted sulfonyl; or
R¹⁶ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently; or
R¹⁶ is selected from the group consisting of: aryl-(CH₂)_{q}-, and heteroaryl-(CH₂)_{q}-; the aryl being selected from the group consisting of: phenyl, naphthyl and anthryl; the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the aryl and the heteroaryl being optionally substituted by a substituent R¹⁸ for one or more times identically or differently;
q is 0, 1, 2 or 3;
the R¹⁷ is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the R¹⁸ is selected from the group consisting of: hydrogen, halogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, nitro, optionally substituted amino, hydroxy, C1-C8 hydroxyalkyl, aryloxy, heterocyclyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
the R¹⁹ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R²⁰ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
where, p and q are each independently 0, 1, 2 or 3;
m and n are the same or different, and are each independently selected from: 1, 2 or 3, so as to form a ring system with various numbers of atoms.

The present invention further relates to a pharmaceutical composition, which inlcudes the compound having the above general formula (I), or an enantiomer, a diastereomer, a tautomer, a salt, a crystalline form, a solvate and/or an isotopically substituted derivative thereof.

The present invention further relates to a pharmaceutical composition, which inlcudes the compound having the above general formula (I), or an enantiomer, a diastereomer, a tautomer, a salt, a crystalline form, a solvate and/or an isotopically substituted derivative thereof, and a pharmaceutically acceptable carrier or excipient or diluent.

The present invention further relates to a method for treating and/or preventing a disease or disorder caused by EBNA1 activity, the method includes administrating an effective amount of the compound having the above general formula (I), or an enantiomer, a diastereomer, a tautomer, a salt, a crystalline form, a solvate and/or an isotopically substituted derivative thereof or a pharmaceutical composition thereof to a subject.

The present invention further relates to use of the compound having the above general formula (I), or an enantiomer, a diastereomer, a tautomer, a salt, a crystalline form, a solvate and/or an isotopically substituted derivative thereof or a pharmaceutical composition thereof in the preparation of a medicament for treating and/or preventing a disease caused by EBNA1 activity.

The present invention further relates to a method for treating and/or preventing a disease or disorder caused by and/or associated with Epstein-Barr virus (EBV) infection, the method includes administrating an effective amount of the compound having the above general formula (I), or an enantiomer, a diastereomer, a tautomer, a salt, a crystalline form, a solvate and/or an isotopically substituted derivative thereof or a pharmaceutical composition thereof to a subject.

The present invention further relates to use of the compound having the above general formula (I), or an enantiomer, a diastereomer, a tautomer, a salt, a crystalline form, a solvate and/or an isotopically substituted derivative thereof or a pharmaceutical composition thereof in the preparation of a medicament for treating and/or preventing a disease caused by and/or associated with Epstein-Barr virus (EBV) infection. The present invention further relates to a method for treating and/or preventing EBV infection in the lytic and/or latent phase, the method includes administrating an effective amount of the compound having the above general formula (I), or an enantiomer, a diastereomer, a tautomer, a salt, a crystalline form, a solvate and/or an isotopically substituted derivative thereof or a pharmaceutical composition thereof to a subject.

The present invention further relates to use of the compound having the above general formula (I), or an enantiomer, a diastereomer, a tautomer, a salt, a crystalline form, a solvate and/or an isotopically substituted derivative thereof or a pharmaceutical composition thereof in the preparation of a medicament for treating and/or preventing a disease caused by EBV infection in the lytic and/or latent phase.

The compound having the above general formula (I), or an enantiomer, a diastereomer, a tautomer, a salt, a crystalline form, a solvate and/or an isotopically substituted derivative thereof or a pharmaceutical composition thereof according to the present invention, and the above method can be used to treat and/or prevent the following diseases: a disease or disorder caused by EBNA1 activity. The present invention is further useful for treating and/or preventing EBV infection, and/or a disease or disorder associated with EBV infection, and a disease caused by EBV infection in the lytic and/or latent phase. The above disease or disorder is selected from at least one of the following: cancer, infectious mononucleosis, chronic fatigue syndrome, multiple sclerosis, systemic lupus erythematosus and rheumatoid arthritis, etc. Particularly, the cancer is selected from the group consisting of nasopharyngeal carcinoma, gastric cancer, non-Hodgkin's lymphoma, anaplastic large cell lymphoma, angioimmunoblastic T-cell lymphoma, hepatosplenic T-cell lymphoma, B-cell lymphoma, Burkitt lymphoma, reticuloendotheliosis, reticulocytosis, microglioma, diffuse large B-cell lymphoma, extranodal T/NK lymphoma/angiocentric lymphoma, follicular lymphoma, immunoblastic lymphoma, mucosa-associated lymphoid tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma, primary exudative lymphoma, lymphomatoid granuloma, angioimmunoblastic lymphadenopathy, leiomyosarcoma, X-linked lymphoproliferative disorder, posttransplant lymphoproliferative disorder, Hodgkin's lymphoma, breast cancer, etc.

### DESCRIPTION OF EMBODIMENTS

In the detailed description of the present invention, numerous specific details are set forth for the purpose of explanation, so as to enable those skilled in the art to understand the disclosed embodiments. However, it will be understood by those skilled in the art that the specific details of these embodiments do not limit the protection scope of the present invention. Furthermore, those skilled in the art can easily appreciate that the specific order of related description and implementation methods of the present invention is illustrative only, and the related order may be changed while remaining within the spirit and scope of the disclosed embodiments of the present invention.

Unless defined otherwise, all technical and scientific terms used herein have the same meanings as commonly understood by those skilled in the art. In the event that the definitions of terms in incorporated references differ from those provided in the present specification, the definitions provided in the present specification shall prevail.

Throughout the specification and claims, the following terms have the meanings explicitly associated herein, unless the context clearly specifies otherwise.

The phrase "in an embodiment" as used herein does not necessarily refer to the same embodiment, although it may. Additionally, the phrase "in another embodiment" as used herein does not necessarily refer to a different embodiment, although it may. Hence, it may be easy to combine various embodiments of the present invention without departing from the scope or spirit of the present invention.

As used herein, the term "EBNA1 inhibitor" refers to a compound that inhibits EBNA1.

As used herein, the term "EBV" refers to the Epstein-Barr (EB) virus.

As used herein, an "effective amount", "therapeutically effective amount" or "pharmaceutically effective amount" of a compound is an amount of the compound sufficient to provide a beneficial effect to a subject to whom the compound is administered.

As used herein, the term "pharmaceutically acceptable carrier" means a pharmaceutically acceptable material, composition or vehicle, such as a liquid or solid filler, a stabilizer, a dispersant, a suspending agent, a diluent, an excipient, a thickener, a solvent or an encapsulating material, which involves carrying or delivering a compound that can be used in the present invention within or to a subject so that it can perform its intended function.

As used herein, the term "alkyl" refers to a straight chain alkyl, branched chain alkyl, or cycloalkyl having a designated number of carbon atom (i.e., C1-8 means 1 to 8 carbons). Examples of the alkyl include methyl, ethyl, n-propyl, isopropyl, n-butyl, t-butyl, isobutyl, sec-butyl, n-pentyl, n-hexyl, n-heptyl, n-octyl, and the like. The alkyl group may be optionally substituted. Non-limiting examples of the substituted alkyl group include hydroxymethyl, chloromethyl, trifluoromethyl, aminomethyl, 1-chloroethyl, 2-hydroxyethyl, 1,2-difluoroethyl, 3-carboxypropyl, and the like.

As used herein, the term "alkoxy" refers to the group -O-alkyl, where the alkyl group is as defined above. The alkoxy group may be optionally substituted. The term C3-C8 cycloalkoxy refers to a ring comprising 3 to 8 carbon atoms and at least one oxygen atom (e.g., tetrahydrofuran, tetrahydro-2H-pyrane). The C3-C8 cycloalkoxy group may be optionally substituted.

As used herein, the term "haloalkyl" refers to a group which includes a straight and branched chain saturated aliphatic hydrocarbon group having a designated number of carbon atom substituted by one or more halogens. The haloalkyl group includes a perhaloalkyl group in which all hydrogen atoms of the alkyl group have been substituted by halogen (e.g., -CF₃, CF₂CF₃). The haloalkyl group may be optionally substituted by one or more substituents other than halogen. Examples of the haloalkyl group include, but are not limited to, fluoromethyl, dichloroethyl, trifluoromethyl, trichloromethyl, pentafluoroethyl, and pentachloroethyl.

As used herein, the term "halogen" refers to chlorine, bromine, fluorine and iodine.

As used herein, the term "heterocyclyl" refers to a cyclic group in which at least one cyclic member is a heteroatom selected from N, O and S. Preferably, the number of the heteroatom is 1, 2, 3 or 4, for example, 5-10 membered heterocyclyl containing oxygen, 5-10 membered heterocyclyl containing sulfur, and 5-10 membered heterocyclyl containing nitrogen. For example, 4-7 membered saturated heterocyclyl containing oxygen, nitrogen, and/or sulfur, specific examples of the saturated heterocyclyl include, but are not limited to, oxetanyl, tetrahydrofuranyl, tetrahydropyranyl, oxepanyl, azetidinyl, pyrrolidinyl, piperidinyl, azepanyl, morpholinyl, piperazinyl, and the like.

As used herein, the term "heteroaryl" refers to a cyclic group in which at least one cyclic member is a heteroatom selected from N, O and S. Preferably, the number of the heteroatom is 1, 2, 3 or 4, for example, 5-10 membered heteroaryl containing oxygen, 5-10 membered heteroaryl containing sulfur, and 5-10 membered heteroaryl containing nitrogen. Specific examples include, but are not limited to, thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl, and the like.

As used herein, the term "optionally substituted" means a substitution of any substituent that can be used for substitution, or being not substituted.

At various sections of this specification, substituents of compounds are disclosed as groups or ranges. Specifically, this specification includes each individual subcombination of members of such groups and ranges. For example, the term "C1-C8 alkyl" is specifically intended to disclose C1, C2, C3, C4, C5, C6, C7, C8, C1-C8, C1-C7, C1-C6, C1-C5, C1-C4, C1-C3, C1-C2, C2-C8, C2-C7, C2-C6, C2-C5, C2-C4, C2-C3, C3-C8, C3-C7, C3-C6, C3-C5, C3-C4, C4-C8, C4-C7, C4-C6, C4-C5, C5-C8, C5-C7, and C5-C6 alkyls, separately. The term "C1-C6 alkyl" is specifically intended to disclose C1, C2, C3, C4, C5, C6, C1-C6, C1-C5, C1-C4, C1-C3, C1-C2, C2-C6, C2-C5, C2-C4, C2-C3, C3-C6, C3-C5, C3-C4, C4-C6, C4-C5 and C5-C6 alkyls, separately. The term "C1-C4 alkyl" is specifically intended to disclose C1, C2, C3, C4, C1-C4, C1-C3, C1-C2, C2-C4, C2-C3 and C3-C4 alkyls, separately.

At various sections of this specification, the term "aryl" refers to phenyl, naphthyl and anthryl; the term "heterocyclyl" refers to 4-7 membered saturated heterocyclyl containing oxygen, nitrogen, and/or sulfur, the saturated heterocyclyl is selected from: oxetanyl, tetrahydrofuranyl, tetrahydropyranyl, oxepanyl, azetidinyl, pyrrolidinyl, piperidinyl, azepanyl, morpholinyl, and piperazinyl; the term "heteroaryl" refers to thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl.

The present invention relates to a compound having a general formula (I), or an enantiomer, a diastereomer, a tautomer, a salt, a crystalline form, a solvate and/or an isotopically substituted derivative thereof: 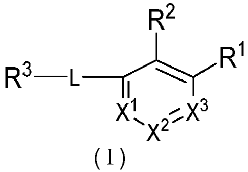 where:
X¹, X², and X³ are the same or different, and are each independently selected from CR^{xa} or a nitrogen atom;
R^{xa} is selected from hydrogen or halogen, the halogen being selected from the group of consisting of: fluorine, chlorine, bromine and iodine;
R¹ is selected from the group of consisting of: -C(=O)-O-R^{1a}, -C(=O)-NH₂, -C(=O)-NHR^{1b}, -C(=O)-NR^{1b}R^{1c}, -C(=O)-NH-S(=O)₂-R^{1b}, -C(=O)-NH-(CH₂)_{q}-R^{1d}, 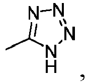 -S(=O)₂OH, -B(OH)₂, -S(=O)₂-NH₂, -S(=O)₂-NHR^{1b}, and -S(=O)₂-NR^{1b}R^{1c},
q is 0, 1, 2 or 3;
where, R^{1a} is independently selected from Li; or
R^{1a}, R^{1b}, and R^{1c} are the same or different, and are each independently selected from the group of consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, R^{1a}, R^{1b}, and R^{1c} are the same or different, and are each independently selected from the group of consisting of: hydrogen, optionally substituted C1-C3 straight chain alkyl, optionally substituted C3 branched chain alkyl,and optionally substituted C3 cycloalkyl;
more preferably, R^{1a}, R^{1b}, and R^{1c} are the same or different, and are each independently selected from: hydrogen or methyl; or
R^{1a}, R^{1b}, and R^{1c} are the same or different, and are each independently selected from the group of consisting of: aryl, heterocyclyl and heteroaryl;
the aryl is selected from the group of consisting of phenyl, naphthyl and anthryl;
the heterocyclyl is selected from: 4-7 membered saturated heterocyclyl containing oxygen, nitrogen, and/or sulfur, the saturated heterocyclyl being selected from the group of consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group of consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
preferably, the aryl is selected from: phenyl;
the heteroaryl is selected from the group consisting of: thienyl, furyl and imidazolyl;
more preferably, R^{1a}, R^{1b}, and R^{1c} are the same or different, and are each independently selected from the group consisting of: phenyl, thienyl and furyl; or
R^{1b} and R^{1c}, together with an atom to which they are attached, form a cyclic group, the cyclic group being selected from the group consisting of: optionally substituted morpholinyl, optionally substituted pyrrolidinyl, and optionally substituted piperazinyl;
R^{1d} is selected from the group consisting of: hydrogen, hydroxyl, amino, cyano, -O-C(=O)-R¹⁷, -O-R¹², -NR⁹R¹⁰, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
preferably, R^{1d} is selected from the group consisting of: hydrogen, hydroxyl, amino, -O-R¹², -NR⁹R¹⁰, optionally substituted phenyl, optionally substituted thienyl, optionally substituted furyl, and optionally substituted imidazolyl;
more preferably, R^{1d} is selected from the group consisting of: hydrogen, hydroxyl, methyl ether group, amino, dimethylamino, piperazinyl, phenyl, thienyl, and furyl;
R² is selected from the following groups:
   hydrogen, amino, halogen, nitro, optionally substituted C1-C4 alkyl, 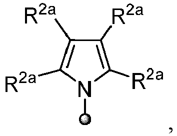 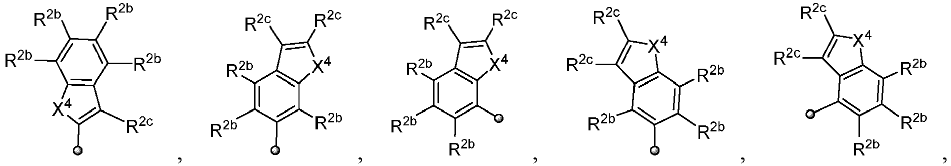 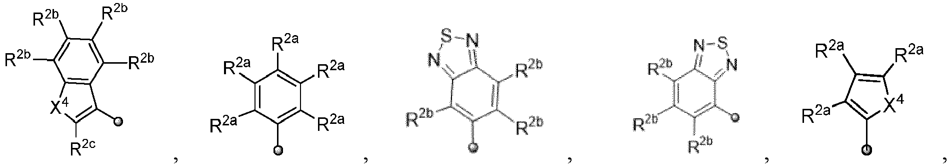 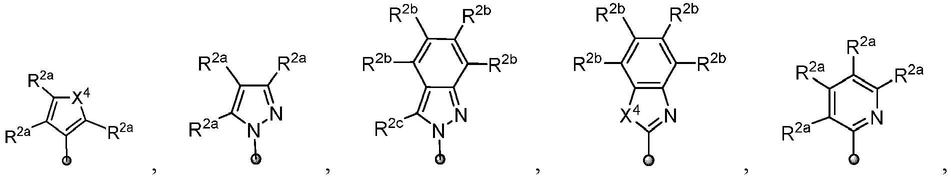 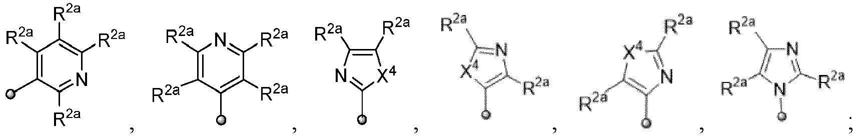
where a dot ● represents a junction of the R² group and other group of a compound molecule of the general formula (I);
X⁴ is selected from the group consisting of O, S, and NR^{2d};
where R^{2a}, R^{2b}, R^{2c} are the same or different, and are each independently selected from the group consisting of:
   hydrogen, halogen, amino, nitro, hydroxyl, C1-C8 hydroxyalkyl, cyano, -(CH₂)_{q}-COOH, -(CH₂)_{q}-C(=O)-O-R⁸, -(CH₂)_{q}-C(=O)-NH-R⁸, -N(H)C(=O)-R⁸, -N(H)S(=O)₂-R⁸, -O-R⁸, -NH-R⁸, -S(=O)₂NH-R⁸, -S(=O)₂-R⁸, -C(=O)-R⁸, -O-C(=O)-R⁸, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, and phenyl; the phenyl being optionally substituted by a substituent R⁴ for one or more times identically or differently;
q is 0, 1, 2 or 3; or
R^{2a} is selected from the group consisting of aryl, heterocyclyl and heteroaryl:
   the aryl is selected from: phenyl, naphthyl, anthryl;
the heterocyclyl is selected from: 4-7 membered saturated heterocyclyl containing oxygen, nitrogen, and/or sulfur, the saturated heterocyclyl being selected from: oxetanyl, tetrahydrofuranyl, tetrahydropyranyl, oxepanyl, azetidinyl, pyrrolidinyl, piperidinyl, azepanyl, morpholinyl, piperazinyl;
the heteroaryl is selected from: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl;
the aryl, the heterocyclyl, and the heteroaryl are optionally substituted by a substituent R⁴ for one or more times identically or differently; or
the adjacent R^{2a} groups, together with an atom to which they are attached, form a fused ring structure, or, the adjacent R^{2b} groups, together with the atoms to which they are attached, form a fused ring structure, the fused ring structure being selected from phenyl or heteroaryl; the heteroaryl being selected from the group consisting of: thienyl, furyl, pyrrolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the phenyl and the heteroaryl are optionally substituted by a substituent R⁴ for one or more times identically or differently;
R^{2d} is selected from the group consisting of hydrogen, optionally halogenated or non-halogenated C1-C3 straight chain alkyl, isopropyl, and cyclopropyl; preferably, the halogenation is selected from fluoro; or
R^{2d} is selected from: -C(=O)-R⁵, or -S(=O)₂-R⁵; or
R^{2d} is selected from the group consisting of aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
L is selected from the following groups: 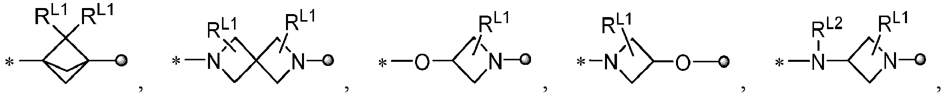 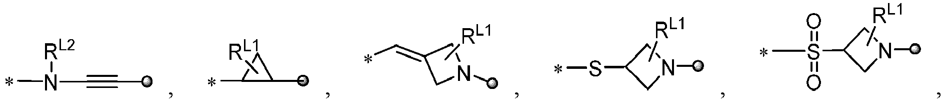 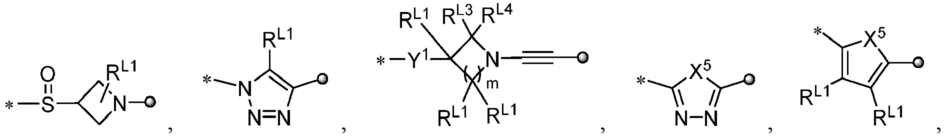 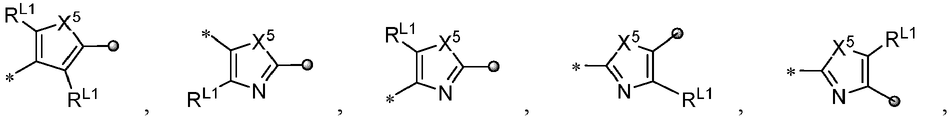 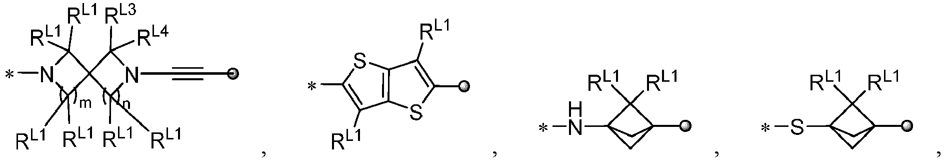 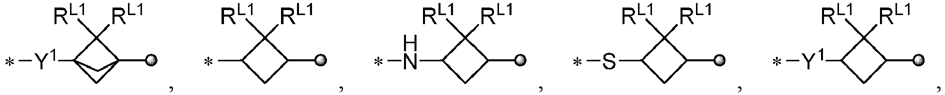 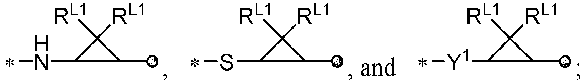
where a dot ● represents a junction where L is attached to a fragment on a right side of a compound of the general formula (I);
where an asterisk ^{∗} represents a junction where L is attached to R³;
X⁵ is selected from the group consisting of: O, NH and S;
Y¹ is selected from: O or NR^{L2};
the L is substituted by a substituent R^{L1} for one or more times at the same or different positions;
R^{L1} is selected from: a hydrogen atom or a fluorine atom;
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R^{L2} is selected from the group consisting of: -(CH₂)_{q}-R⁷, -C(=O)-N(H)-R¹⁹, -C(=S)-N(H)-R¹⁹, -C(=O)-R²⁰, and -S(=O)₂-R²⁰; or
R^{L2} is selected from the group consisting of: aryl-(CH₂)_{q}-, heterocyclyl-(CH₂)_{q}-, and heteroaryl-(CH₂)_{q}-;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{L2} and R³ are cyclized together with an atom to which they are attached to form 5-10 membered heterocyclyl containing nitrogen and/or sulfur, the 5-10 membered heterocyclyl containing nitrogen and/or sulfur including -C(=O)- and -S(=O)₂- groups;
R^{L3} and R^{L4} are the same or different, and are each independently selected from: a hydrogen atom or a fluorine atom; or
R^{L3} and R^{L4}, together with an atom to which they are attached, form carbonyl, or thiocarbonyl; or
R^{L3} and R^{L4}, together with the atom to which they are attached, form a heterocyclyl containing nitrogen and/or sulfur, where C atom attached to both R^{L3} and R^{L4} is replaced by -S(=O)₂-;
m and n are the same or different, and are each independently selected from: 1, 2 or 3, so as to form a ring system with various numbers of atoms;
q is 0, 1, 2 or 3;
R³ is selected from the following groups:
   C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl; the C1-C8 straight chain alkyl, the C3-C8 branched chain alkyl, the C3-C8 cycloalkyl; the halogenated C1-C8 straight chain alkyl, the halogenated C3-C8 branched chain alkyl, and the halogenated C3-C8 cycloalkyl being optionally substituted by a substituent R⁶ for one or more times identically or differently; or
R³ is selected from the following groups:
   (1) 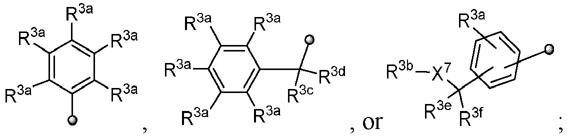 or
   (2) 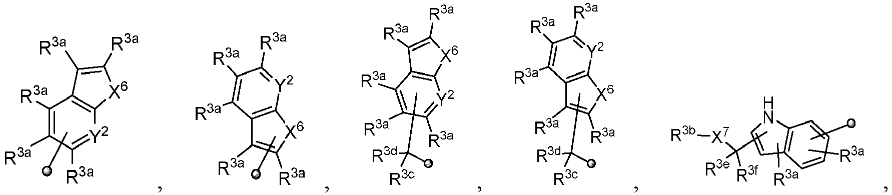 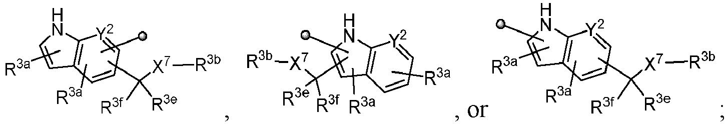 or
   (3) 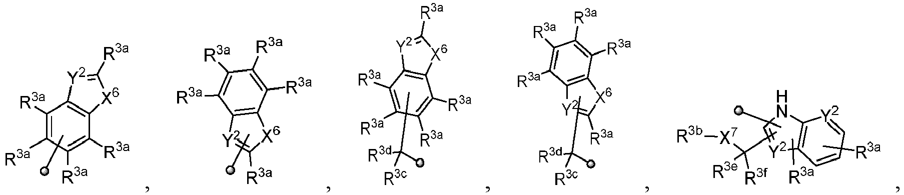 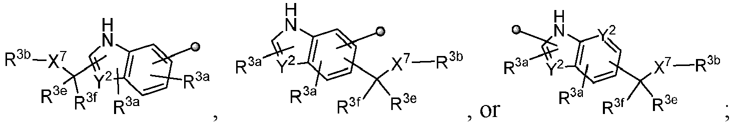 or
   (4) 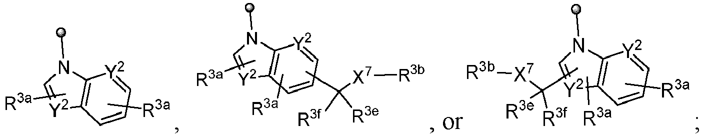 or
   (5) 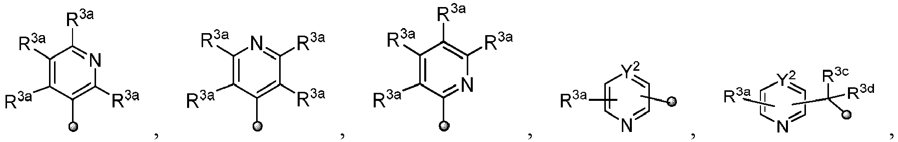 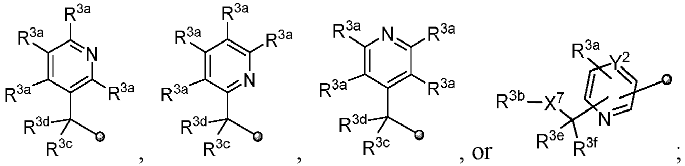 or
   (6) 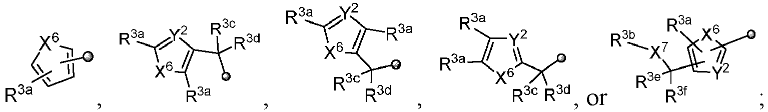 or
   (7) 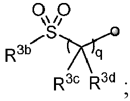 or
   (8) 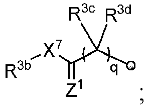
where a dot ● represents a junction which is attached to a fragment on a right side of the compound of the general formula (I);
X⁶ is selected from the group consisting of O, S and NR^{3g};
X⁷ is selected from the group consisting of O, S, NR^{3g} and CHR^{3a};
Y² is selected from the group consisting of CH, CR^{3a} and N, and Y² may replace a C atom at an optional position of an aromatic ring;
Z¹ is selected from the group consisting of O, S and NR^{3h};
where R^{3a} is selected from the group consisting of: hydrogen, halogen, nitro, cyano, sulfo-group, optionally substituted sulfonamido, optionally substituted C1-C8 straight chain alkyl, optionally substituted C3-C8 branched chain alkyl, optionally substituted C3-C8 cycloalkyl, optionally substituted halogenated C1 -C8 straight chain alkyl, optionally substituted halogenated C3-C8 branched chain alkyl, optionally substituted halogenated C3-C8 cycloalkyl, hydroxyl, C1-C8 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)_{P}-R¹¹, aryl-(CH₂)_{q}-, heterocyclyl-(CH₂)_{q}-, heteroaryl-(CH₂)_{q}-; the C1-C8 hydroxyalkyl being C1-C8 alkyl substituted with hydroxyl;
p and q are each independently 0, 1, 2 or 3;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
adjacent R^{3a} groups together with an atom to which they are attached form a cyclic structure, or R^{3a} and its adjacent R^{3c} together with an atom to which they are attached form a cyclic structure, or R^{3a} and its adjacent R^{3d} together with an atom to which they are attached form a cyclic structure, the cyclic structure comprising a saturated or aromatic cyclic structure, the cyclic structure being selected from the group consisting of: cycloalkyl, aryl, heterocyclyl and heteroaryl;
preferably, the cyclic structure is selected from the group consisting of: phenyl, pyridyl, pyrrolyl, thienyl, furyl, oxazolyl, thiazolyl, isoxazolyl, indazolyl, and 5-7 membered saturated heterocyclyl containing nitrogen/oxygen, the saturated heterocyclyl being selected from the group consisting of: tetrahydrofuranyl, tetrahydropyranyl, oxepanyl, pyrrolidinyl, piperidinyl, azepanyl, morpholinyl and piperazinyl;
the cyclic structure is optionally substituted by a substituent R¹⁸ for one or more times identically or differently;
R^{3b} is selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S)N(H)-R¹⁹, -C(=O)O-R¹⁹, and -S(=O)₂-R²⁰;
q is 0, 1, 2 or 3; or
R^{3b} is selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3c} and R^{3d} are the same or different, and are each independently selected from the group consisting of: hydrogen, fluorine, C1-C8 alkyl, aryl-(CH₂)_{q}-, heterocyclyl-(CH₂)_{q}-, and heteroaryl-(CH₂)_{q}-; or when L is selected from the group consisting of 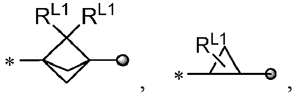 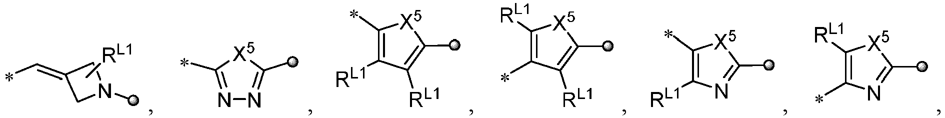 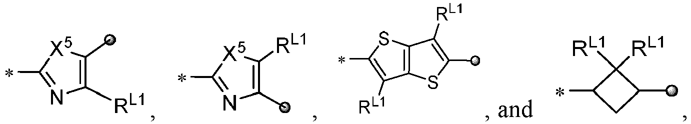 R^{3c} and R^{3d} are the same or different, and are each independently selected from the group consisting of: hydrogen, fluorine, C1-C8 alkyl, hydroxyl, C1-C8 hydroxyalkyl, -O-C(=O)-R²⁰, aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{3c} and R^{3d} together with an atom to which they are attached form carbonyl, thiocarbonyl, or imino;
R^{3e} and R^{3f} are the same or different, and are each independently selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S) N(H)-R¹⁹, -C(=O)O-R¹⁹, and -S(=O)₂-R²⁰; or
R^{3e} and R^{3f} together with an atom to which they are attached form carbonyl, or thiocarbonyl; or
R^{3e} and R^{3f} are the same or different, and are each independently selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3h} is selected from the group consisting of: hydrogen, nitro and cyano;
the R⁴ is selected from the group consisting of: hydrogen, fluorine, chlorine, hydroxyl, C1-C3 hydroxyalkyl, optionally substituted C1-C3 alkyl, optionally substituted halogenated C1-C3 alkyl, optionally substituted C1-C3 alkoxy, optionally substituted halogenated C1-C3 alkoxy, nitro, cyano, amino, acetyl, methylsulfonyl, acetamido, and methylsulfonamido;
the R⁵ is selected from the group consisting of: hydrogen, optionally substituted C1-C3 alkyl, and optionally substituted halogenated C1-C3 alkyl;
the R⁶ is selected from the group consisting of: hydroxy, C1-C8 straight chain alkoxy, C3-C8 branched chain alkoxy, C3-C8 cycloalkoxy, -O-C(=O)-R²⁰, amino, -NH-C(=O)-R²⁰, -NH-S(=O)₂-R²⁰, -NH-C(=O)-O-R²⁰, -NH-C(=O)-NH-R²⁰, and -NH-C(=S)-NH-R²⁰;
the R⁷ is selected from the group consisting of: phenyl, naphthyl, heterocyclyl, and heteroaryl; the heterocyclyl being selected from: 4-7 membered saturated heterocyclyl containing oxygen, nitrogen, and/or sulfur; the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the phenyl, the naphthyl, the heterocyclyl, and the heteroaryl being optionally substituted by a substituent R¹⁸ for one or more times identically or differently;
the R⁸ is selected from the group consisting of: optionally substituted C1-C3 alkyl, and optionally substituted halogenated C1-C3 alkyl;
the R⁹ and R¹⁰ are the same or different, and are each independently selected from: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
R⁹ and R¹⁰ are the same or different, and are each independently selected from the group consisting of: optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl; the heterocyclyl containing nitrogen being substituted by the following substituents for one or more times identically or differently: hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, and optionally substituted sulfonamido;
the R¹¹ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
R¹¹ is selected from the group consisting of: aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl;
the aryl, the heterocyclyl and the heteroaryl are substituted by the following substituents for one or more times identically or differently: halogen, hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, optionally substituted sulfonamido, cyano, nitro, sulfo-group, optionally substituted ureido, and guanidyl;
the R¹² is selected from the group consisting of: hydrogen, C1-C8 straight chain alkyl, C3-C8 branched chain alkyl, C3-C8 cycloalkyl, halogenated C1-C8 straight chain alkyl, halogenated C3-C8 branched chain alkyl, halogenated C3-C8 cycloalkyl, aryl, heterocyclyl, and heteroaryl; the C1-C8 alkyl being substituted by the following substituents for one or more times identically or differently: -O-R²⁰, -OH, -N(H)C(=O)-R²⁰, and -NR⁹R¹⁰;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R¹³ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
the R¹³ is selected from the group consisting of: -(CH₂)_{q}-phenyl, -(CH₂)_{q}-halogenated phenyl, -(CH₂)_{q}-naphthyl, -(CH₂)_{q}-halogenated naphthyl, -(CH₂)_{q}-anthryl, -(CH₂)_{q}-halogenated anthryl, -(CH₂)_{q}-heterocyclyl, -(CH₂)_{q}-halogenated heterocyclyl, -(CH₂)_{q}-heteroaryl, and -(CH₂)_{q}-halogenated heteroaryl;
q is 0, 1, 2 or 3;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the phenyl, the naphthyl, the anthryl, the heterocyclyl, and the heteroaryl are optionally substituted by a substituent R¹⁸ for one or more times identically or differently;
the R¹⁴ is selected from: heteroaryl, the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the heteroaryl being optionally substituted by a substituent R¹⁸ for one or more times identically or differently;
the R¹⁵ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently;
the R¹⁶ is selected from the group consisting of: hydrogen, optionally substituted C1-C8 straight chain alkyl, optionally substituted C3-C8 branched chain or cycloalkyl, optionally substituted halogenated C1-C8 straight chain alkyl, optionally substituted halogenated C3-C8 branched chain or cycloalkyl, optionally substituted acyl, and optionally substituted sulfonyl; or
R¹⁶ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently; or
R¹⁶ is selected from the group consisting of: aryl-(CH₂)_{q}-, and heteroaryl-(CH₂)_{q}-; the aryl being selected from the group consisting of: phenyl, naphthyl and anthryl; the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the aryl and the heteroaryl being optionally substituted by a substituent R¹⁸ for one or more times identically or differently;
q is 0, 1, 2 or 3;
the R¹⁷ is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the R¹⁸ is selected from the group consisting of: hydrogen, halogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, nitro, optionally substituted amino, hydroxy, C1-C8 hydroxyalkyl, aryloxy, heterocyclyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
the R¹⁹ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R²⁰ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
where, p and q are each independently 0, 1, 2 or 3;
m and n are the same or different, and are each independently selected from: 1, 2 or 3, so as to form a ring system with various numbers of atoms.

In a preferred embodiment, the present invention relates to the compound having the above general formula (I), or the enantiomer, the diastereomer, the tautomer, the salt, the crystalline form, the solvate and/or the isotopically substituted derivative thereof, where the compound is selected from at least one compound of the following formulae:

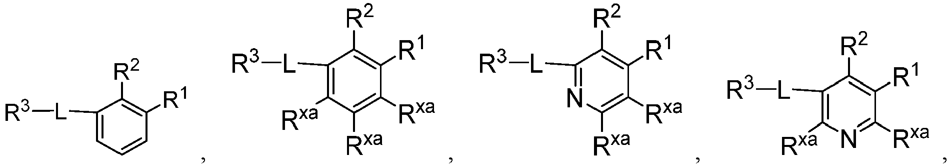

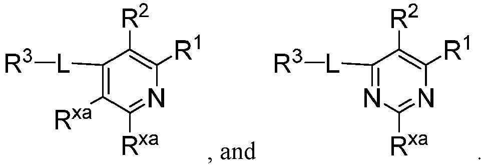

In a preferred embodiment, the present invention relates to the compound having the above general formula (I), or the enantiomer, the diastereomer, the tautomer, the salt, the crystalline form, the solvate and/or the isotopically substituted derivative thereof, where
R² is selected from the following groups:
hydrogen, amino, nitro, 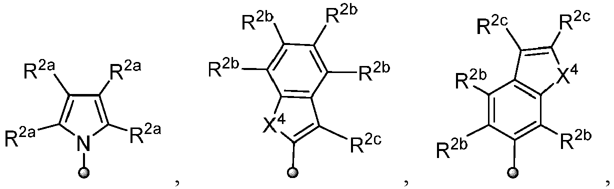 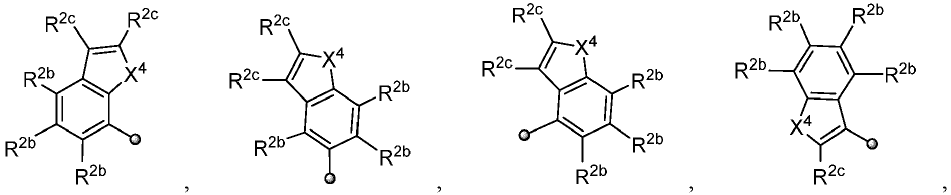 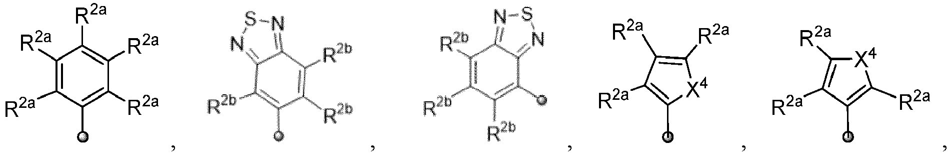 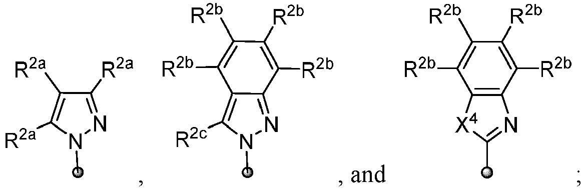
where a dot ● represents a junction where the R² group is attached to other fragments of the compound of the general formula (I);
X⁴ is selected from the group consisting of O, S and NR^{2d};
where R^{2a}, R^{2b}, and R^{2c} are the same or different, and are each independently selected from the group consisting of:
   hydrogen, halogen, amino, nitro, hydroxyl, C1-C8 hydroxyalkyl, cyano, -(CH₂)_{q}-COOH, -(CH₂)_{q}-C(=O)-O-R⁸, -(CH₂)_{q}-C(=O)-NH-R⁸, -N(H)C(=O)-R⁸, -N(H)S(=O)₂-R⁸, -O-R⁸, -NH-R⁸, -S(=O)₂NH-R⁸, -S(=O)₂-R⁸, -C(=O)-R⁸, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, and phenyl; the phenyl being optionally substituted by a substituent R⁴ for one or more times identically or differently;
preferably, where R^{2a}, R^{2b}, and R^{2c} are the same or different, and are each independently selected from the group consisting of:
hydrogen, halogen, cyano, -(CH₂)_{q}-COOH, -N(H)S(=O)₂-R⁸, -O-R⁸, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, and phenyl, the phenyl being optionally substituted by a substituent R⁴ for one or more times identically or differently;
further preferably, R^{2a}, R^{2b}, and R^{2c} are the same or different, and are each independently selected from the group consisting of:
   hydrogen, halogen, cyano, -(CH₂)_{q}-COOH, -N(H)S(=O)₂-R⁸, -O-R⁸,C1-C6 straight chain alkyl or alkoxy, C3-C6 branched chain alkyl or alkoxy, C3-C6 cycloalkyl or alkoxy, halogenated C1-C6 straight chain alkyl or alkoxy, halogenated C3-C6 branched chain alkyl or alkoxy, halogenated C3-C6 cycloalkyl or alkoxy, and phenyl, the phenyl being optionally substituted by a substituent R⁴ for one or more times identically or differently;
further preferably, R^{2a}, R^{2b}, and R^{2c} are the same or different, and are each independently selected from the group consisting of:
   hydrogen, halogen, cyano, -(CH₂)_{q}-COOH, -N(H)S(=O)₂-R⁸, -O-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
q is 0, 1, 2 or 3;
R^{2d} is selected from the group consisting of: hydrogen, optionally fluorinated or non-fluorinated C1-C3 straight chain alkyl, isopropyl, cyclopropyl, -C(=O)-R⁵, and -S(=O)₂-R⁵;
preferably, R^{2d} is selected from the group consisting of: hydrogen, fluoro- or non-fluoromethyl, fluoro- or non-fluoro- ethyl, fluoro- or non-fluoro- propyl, isopropyl, cyclopropyl, acetyl, methylsulfonyl, benzoyl, and phenylsulfonyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from the following groups: 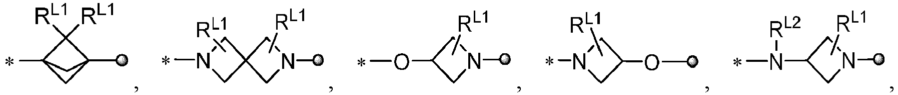 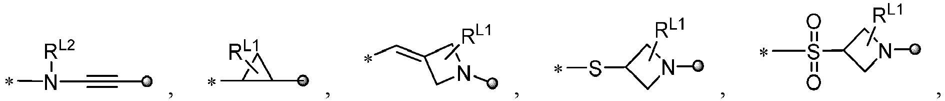 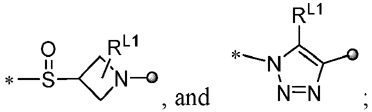
where a dot ● represents a junction where L is attached to a fragment on the right side of the compound of the general formula (I);
where an asterisk ^{∗} represents a junction where L is attached to R³;
the L is substituted by a substituent R^{L1} for one or more times at the same or different positions;
R^{L1} is selected from: a hydrogen atom or a fluorine atom;
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, the R^{L2} is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
more preferably, R^{L2} is selected from the group consisting of: hydrogen, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl; or
R^{L2} is selected from the group consisting of: -(CH₂)_{q}-R⁷, -C(=O)N(H)-R¹⁹, -C(=S)N(H)-R¹⁹, -C(=O)-R²⁰, and -S(=O)₂-R²⁰; or
R^{L2} and R³ are cyclized together with an atom to which they are attached to form 5-10 membered heterocyclyl containing nitrogen and/or sulfur, the 5-10 membered heterocyclyl containing nitrogen and/or sulfur comprising -C(=O)-, -S(=O)₂- group;
q is 0, 1, 2 or 3;
the R⁷ is selected from: phenyl, heterocyclyl, heteroaryl, the heterocyclyl being selected from: 4-7 membered saturated heterocyclyl containing oxygen, nitrogen the saturated heterocyclyl being selected from the group consisting of: oxetanyl, tetrahydrofuranyl, tetrahydropyranyl, azetidinyl, pyrrolidinyl, piperidinyl, morpholinyl, piperazinyl; the heteroaryl being selected from: thienyl, furyl, pyrrolyl, pyridyl, pyrimidinyl, indolyl, quinolinyl, quinazolinyl, benzofuryl, benzothienyl, benzoxazolyl, and benzothiazolyl; the phenyl, the heterocyclyl, and the heteroaryl being optionally substituted by a substituent R¹⁸ for one or more times identically or differently;
preferably, R¹⁸ is selected from the group consisting of: hydrogen, F, Cl, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, nitro, optionally substituted amino, hydroxy, C1-C8 hydroxyalkyl, phenyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl; more preferably, the acyl is an acyl substituted by C1-C4 alkyl; the sulfonyl is a sulfonyl substituted by C1-C4 alkyl; further preferably, the acyl is acetyl; the sulfonyl is a methylsulfonyl;
the aryl is selected from: phenyl;
the heterocyclyl is selected from: 4-7 membered saturated heterocyclyl containing oxygen, nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R⁴ for one or more times identically or differently;
more preferably, the amino is substituted by C1-C4 alkyl or C1-C4 acyl.

In a preferred embodiment, the present invention relates to the compound having the above general formula (I), or the enantiomer, the diastereomer, the tautomer, the salt, the crystalline form, the solvate and/or the isotopically substituted derivative thereof, where
R³ is selected from the following groups:
   (1) 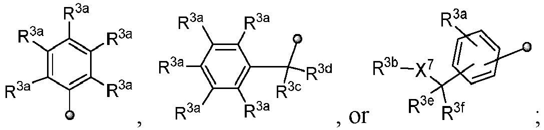 or
   (2) 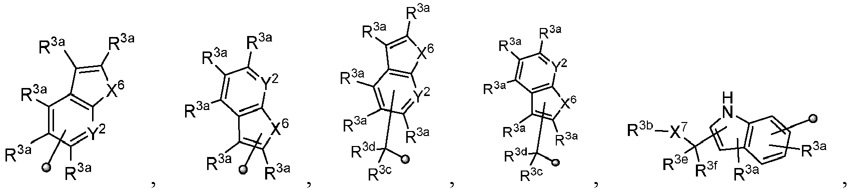 or 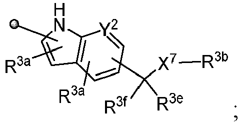 or
   (5) 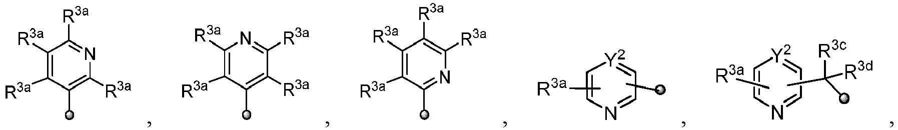 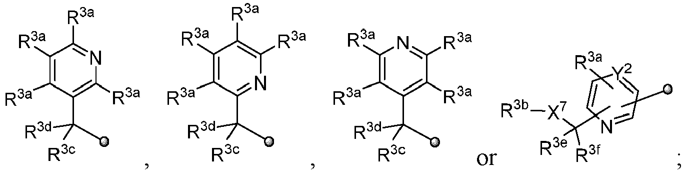 or
   (6) 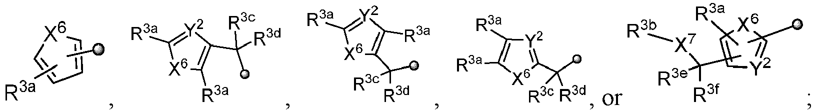 or
   (7) 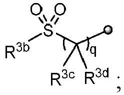 or
   (8) 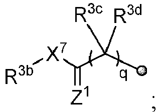
where a dot • represents a junction which is attached to a fragment on the right side of the compound of the general formula (I);
X⁶ is selected from O, S or NR^{3g};
X⁷ is selected from the group consisting of O, S, NR^{3g} and CHR^{3a};
Y² is selected from CH, CR^{3a} or N; and Y² may replace a C atom at an optional position of an aromatic ring;
Z¹ is selected from O, S or NR^{3h};
where R^{3a} is selected from the group consisting of:
   hydrogen, halogen, nitro, cyano, sulfo-group, optionally substituted sulfonamido, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
the aryl is selected from: phenyl;
the heterocyclyl is selected from the group consisting of: 4-7 membered saturated heterocyclyl containing oxygen, nitrogen; the saturated heterocyclyl is selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
p and q are each independently 0, 1, 2 or 3;
preferably, the R¹¹ is selected from the group consisting of: hydrogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy, aryl, heterocyclyl and heteroaryl;
the aryl is selected from: phenyl;
the heterocyclyl is selected from: 4-7 membered saturated heterocyclyl containing oxygen and/or nitrogen, the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
preferably, the R¹⁷ is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and phenyl, the phenyl being optionally substituted by a substituent R⁴ for one or more times identically or differently;
R^{3b} is selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
preferably, the aryl is selected from: phenyl;
the heteroaryl is selected from the group consisting of: thienyl, pyridyl, pyrimidinyl and indolyl;
q is 0, 1, 2 or 3;
R^{3c} and R^{3d} are the same or different, and are each independently selected from the group consisting of: hydrogen, fluorine and C1-C8 alkyl; or
when L is selected from 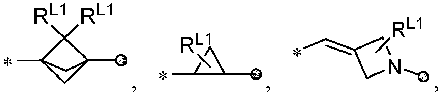 R^{3c} and R^{3d} are the same or different, and are each independently selected from the group consisting of: hydrogen, fluorine, C1-C8 alkyl, hydroxyl, C1-C8 hydroxyalkyl, and -O-C(=O)-R²⁰;
preferably, R^{3c} and R^{3d} are the same or different, and are each independently selected from the group consisting of: hydrogen, fluorine, C1-C8 alkyl, hydroxyl, C1-C8 hydroxyalkyl, and -O-C(=O)-R⁸; or
R^{3c} and R^{3d} together with an atom to which they are attached form carbonyl, thiocarbonyl, imino;
R^{3e} and R^{3f} are the same or different, and are each independently selected from the group consisting of:
   hydrogen, C1-C8 straight chain alkyl, C3-C8 branched chain alkyl, C3-C8 cycloalkyl, halogenated C1-C8 straight chain alkyl, halogenated C3-C8 branched chain alkyl, halogenated C3-C8 cycloalkyl, aryl-(CH₂)_{q}-, and heteroaryl-(CH₂)_{q}-;
the aryl and the heteroaryl are optionally substituted by a substituent R¹⁸ for one or more times identically or differently;
preferably, the aryl is selected from: phenyl;
the heterocyclyl is selected from: 4-7 membered saturated heterocyclyl containing oxygen and/or nitrogen, the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl; or
R^{3e} and R^{3f} together with an atom to which they are attached form carbonyl or thiocarbonyl.

In a preferred embodiment, the present invention relates to the compound having the above general formula (I), or the enantiomer, the diastereomer, the tautomer, the salt, the crystalline form, the solvate and/or the isotopically substituted derivative thereof, where R¹ is selected from the group consisting of -COOH, -C(=O)-O-R^{1a}, -C(=O)-NH-S(=O)₂-R^{1b}, -C(=O)-NH-(CH₂)_{q}-R^{1d}, and 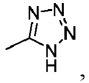
where, R^{1a} is selected from: Li; or
R^{1a}, R^{1b}, and R^{1c} are the same or different, and are each independently selected from the group consisting of: hydrogen, optionally substituted C1-C4 straight chain alkyl, optionally substituted C3-C4 branched chain or cycloalkyl, optionally substituted halogenated C1-C4 straight chain alkyl, and optionally substituted halogenated C3-C4 branched chain or cycloalkyl;
further preferably, R^{1a}, R^{1b}, and R^{1c} are the same or different, and are each independently selected from the group consisting of: methyl, ethyl and propyl;
R^{1d} is selected from the group consisting of hydroxyl, amino, cyano, -O-R¹², -NR⁹R¹⁰, aryl, heterocyclyl and heteroaryl;
preferably, the aryl is selected from: phenyl;
the heterocyclyl is selected from: azetidinyl or pyrrolidinyl;
the heteroaryl is selected from: thienyl or furyl;
the R⁹ and R¹⁰ are the same or different, and are each independently selected from the group consisting of: hydrogen, optionally substituted C1-C4 alkyl, optionally substituted halogenated C1-C4 alkyl, optionally substituted C1-C4 alkoxy, optionally substituted halogenated C1-C4 alkoxy; or
R⁹ and R¹⁰ are the same or different, and are each independently selected from the group consisting of: optionally substituted aryl, optionally substituted heterocyclyl, and optionally substituted heteroaryl;
the aryl is selected from: phenyl;
the heterocyclyl is selected from: 4-7 membered saturated heterocyclyl containing oxygen, nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from: thienyl, furyl, pyrrolyl, pyridyl, pyrimidinyl, indolyl, quinolinyl, quinazolinyl, benzofuryl, benzothienyl, benzoxazolyl, benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl.

In a preferred embodiment, the present invention relates to the compound having the above general formula (I), or the enantiomer, the diastereomer, the tautomer, the salt, the crystalline form, the solvate and/or the isotopically substituted derivative thereof, where R² is selected from the group consisting of: hydrogen, amino, nitro, optionally substituted pyrrolyl, optionally substituted benzofuryl, optionally substituted indolyl, optionally substituted benzothienyl, optionally substituted phenyl, optionally substituted benzothiadiazolyl, optionally substituted furyl, optionally substituted pyrazolyl, optionally substituted indazolyl, optionally substituted benzothiazolyl, optionally substituted pyridyl, optionally substituted imidazolyl, and optionally substituted thienyl;
preferably, the R² is optionally substituted by H, F, or Cl for one or more times identically or differently.

In a more preferred embodiment, the present invention relates to the compound having the above general formula (I), or the enantiomer, the diastereomer, the tautomer, the salt, the crystalline form, the solvate and/or the isotopically substituted derivative thereof, where:
R² is selected from the group consisting of: hydrogen, amino, nitro, pyrrolyl, benzofuryl, indolyl, benzo[b]thienyl, phenyl, halophenyl, cyanophenyl, cyanohalophenyl, carboxyphenyl, halogenated C1-C4 alkylphenyl, biphenyl, methylsulfonamidophenyl, acetylphenyl, methanesulfonylphenyl, aminosulfonylphenyl, C1-C4 alkoxyphenyl, benzo[c][1,2,5]thiadiazolyl, furyl, pyrazolyl, indazolyl, and benzo[d]thiazolyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 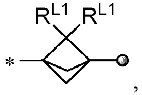
R² is selected from the group consisting of: hydrogen, 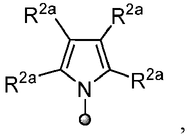 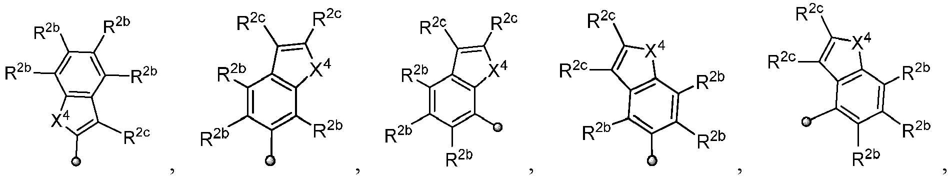 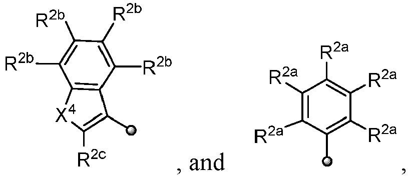 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 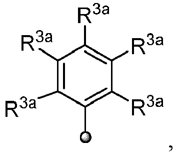 where R^{3a} is selected from the group consisting of:
   hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the aryl, the heterocyclyl, and the heteroaryl being optionally substituted by a substituent R⁴ for one or more times identically or differently;
preferably, R^{3a} is selected from the group consisting of: hydrogen, halogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and -(CH₂)_{q}-O-R¹²;
the R¹² is selected from the group consisting of: C1-C4 straight chain alkyl, C3-C4 branched chain alkyl, halogenated C1-C4 straight chain alkyl, and halogenated C3-C4 branched chain alkyl; more preferably, the R¹² is selected from the group consisting of: methyl, ethyl, propyl, trifluoromethyl, and trichloromethyl.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 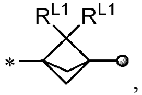
R² is selected from the group consisting of: hydrogen, 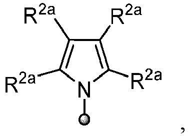 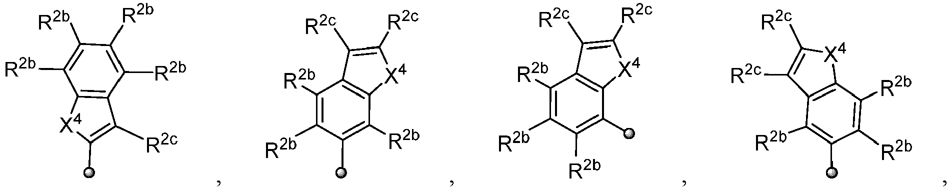 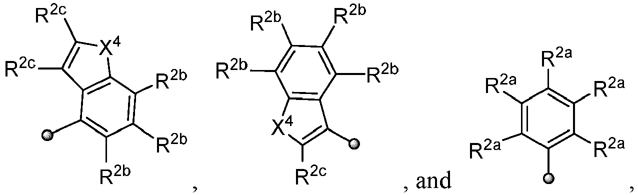
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 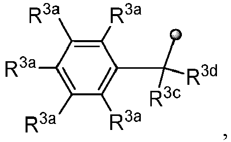 where
R^{3a} is selected from the group consisting of: hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the phenyl, the heterocyclyl, and the heteroaryl being optionally substituted by a substituent R⁴ for one or more times identically or differently;
preferably, R^{3a} is selected from the group consisting of: hydrogen, halogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and -(CH₂)_{q}-O-R¹²;
more preferably, R^{3a} is selected from the group consisting of: hydrogen, F, Cl, methyl, ethyl, propyl, trifluoromethyl, trichloromethyl, and methoxy;
R^{3c} and R^{3d} are the same or different, and are each independently selected from the group consisting of: hydrogen, hydroxyl, C1-C8 hydroxyalkyl, and -O-C(=O)-R²⁰;
R^{3c} and R^{3d} are the same or different, and are each independently selected from the group consisting of: hydrogen, hydroxyl, C1-C4 hydroxyalkyl, and -O-C(=O)-R²⁰;
the R¹² is selected from the group consisting of: C1-C4 straight chain alkyl, C3-C4 branched chain alkyl, halogenated C1-C4 straight chain alkyl, and halogenated C3-C4 branched chain alkyl; more preferably, the R¹² is selected from the group consisting of: methyl, ethyl, propyl, trifluoromethyl, and trichloromethyl;
the R²⁰ is selected from the group consisting of: C1-C4 straight chain alkyl, C3-C4 branched chain alkyl, halogenated C1-C4 straight chain alkyl, and halogenated C3-C4 branched chain alkyl; more preferably, the R²⁰ is selected from the group consisting of: methyl, ethyl, propyl, trifluoromethyl, and trichloromethyl.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 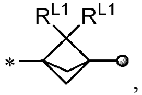
R² is selected from the group consisting of: hydrogen, 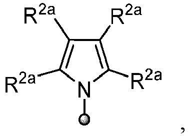 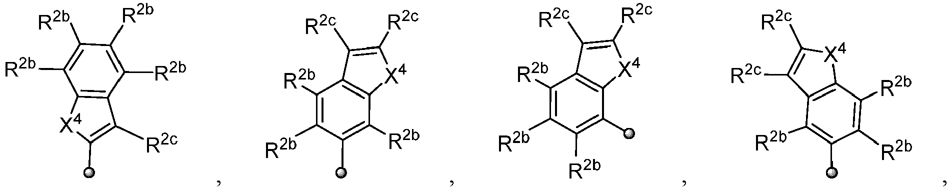 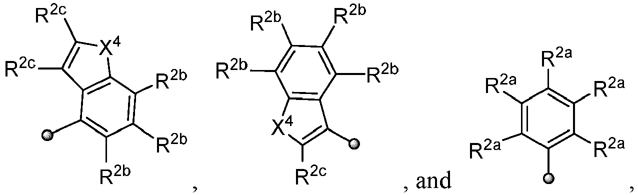 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from the group consisting of: 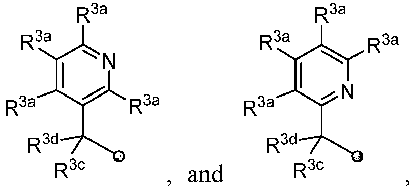 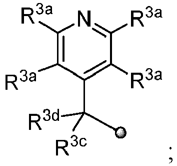 where
R^{3a} is selected from the group consisting of: hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the phenyl, the heterocyclyl, and the heteroaryl being optionally substituted by a substituent R⁴ for one or more times identically or differently;
preferably, R^{3a} is selected from the group consisting of: hydrogen, halogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and -(CH₂)_{q}-O-R¹²;
more preferably, R^{3a} is selected from the group consisting of: hydrogen, F, Cl, methyl, ethyl, propyl, trifluoromethyl, trichloromethyl, and methoxy;
R^{3c} and R^{3d} are the same or different, and are each independently selected from the group consisting of: hydrogen, hydroxyl, C1-C8 hydroxyalkyl, and -O-C(=O)-R²⁰;
R^{3c} and R^{3d} are the same or different, and are each independently selected from the group consisting of: hydrogen, hydroxyl, C1-C4 hydroxyalkyl, and -O-C(=O)-R²⁰;
the R¹² is selected from the group consisting of: C1-C4 straight chain alkyl, C3-C4 branched chain alkyl, halogenated C1-C4 straight chain alkyl, and halogenated C3-C4 branched chain alkyl; more preferably, the R¹² is selected from the group consisting of: methyl, ethyl, propyl, trifluoromethyl, and trichloromethyl;
the R²⁰ is selected from the group consisting of: C1-C4 straight chain alkyl, C3-C4 branched chain alkyl, halogenated C1-C4 straight chain alkyl, and halogenated C3-C4 branched chain alkyl; more preferably, the R²⁰ is selected from the group consisting of: methyl, ethyl, propyl, trifluoromethyl, and trichloromethyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 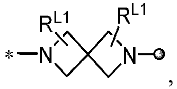
R² is selected from the group consisting of: hydrogen, amino, nitro, 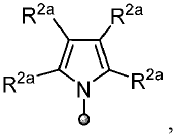 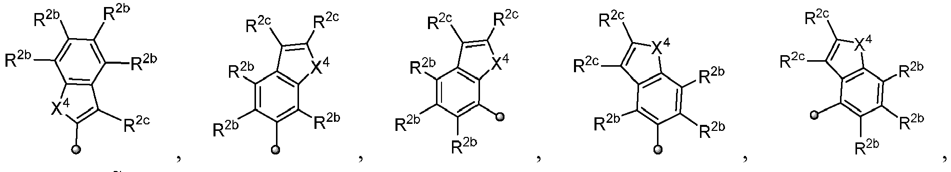 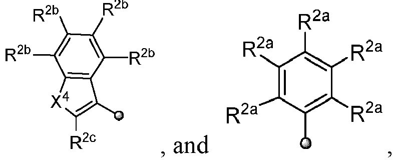 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R² is selected from the group consisting of: hydrogen, amino, nitro, pyrrolyl, indolyl, and phenyl, the pyrrolyl, the indolyl, and the phenyl are optionally substituted by the following substituents for one or more times identically or differently, the substituent being selected from the group consisting of: fluorine, chlorine, methyl, trifluoromethyl, methoxy, and trifluoromethoxy;
R³ is selected from: 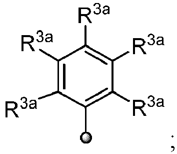 where,
R^{3a} is selected from the group consisting of: hydrogen, -(CH₂)_{q}-NR⁹R¹⁰, nitro, C1-C8 straight chain alkyl, C3-C8 branched chain alkyl, halogenated C1-C8 straight chain alkyl, halogenated C3-C8 branched chain alkyl, -(CH₂)_{q}-COOH, -C(=O)-O-R²⁰, -(CH₂)_{q}-NH-C(=O)-R¹¹, and -(CH₂)_{q}-NR¹⁶-S(=O)₂-R¹⁷;
preferably, R^{3a} is selected from the group consisting of: hydrogen, -NH₂, nitro, C1-C6 straight chain alkyl, C3-C6 branched chain alkyl, halogenated C1-C6 straight chain alkyl, halogenated C3-C6 branched chain alkyl, -(CH₂)_{q}-COOH, -C(=O)-O-R²⁰, -(CH₂)_{q}-NH-C(=O)-R¹¹, and -(CH₂)_{q}-NR¹⁶-S(=O)₂-R¹⁷;
the R¹¹ is selected from the group consisting of: C1-C4 straight chain alkyl, C3-C4 branched chain alkyl, halogenated C1-C4 straight chain alkyl, and halogenated C3-C4 branched chain alkyl; more preferably, R¹¹ is selected from the group consisting of: methyl, ethyl, propyl, and trifluoromethyl;
the R¹⁶ is selected from: hydrogen;
the R¹⁷ is selected from the group consisting of: C1-C4 straight chain alkyl, C3-C4 branched chain alkyl, C3-C4 cycloalkyl, halogenated C1-C4 straight chain alkyl, halogenated C3-C4 branched chain alkyl, halogenated C3-C4 cycloalkyl, and optionally substituted phenyl; more preferably, the R¹⁷ is selected from the group consisting of: methyl, ethyl, propyl, cyclopropyl, trifluoromethyl, trichloromethyl, and tolyl;
the R²⁰ is selected from the group consisting of: C1-C4 straight chain alkyl, C3-C4 branched chain alkyl, halogenated C1-C4 straight chain alkyl, and halogenated C3-C4 branched chain alkyl; more preferably, the R²⁰ is selected from the group consisting of: methyl, ethyl, propyl, trifluoromethyl, and trichloromethyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from:

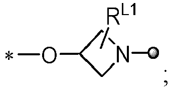

R² is selected from the group consisting of: hydrogen, amino, halogen, optionally substituted C1-C4 alkyl, 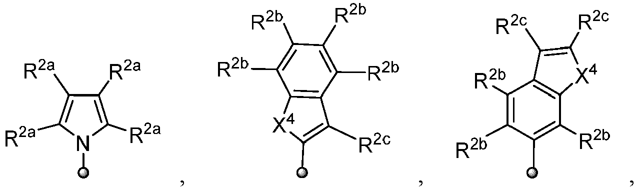 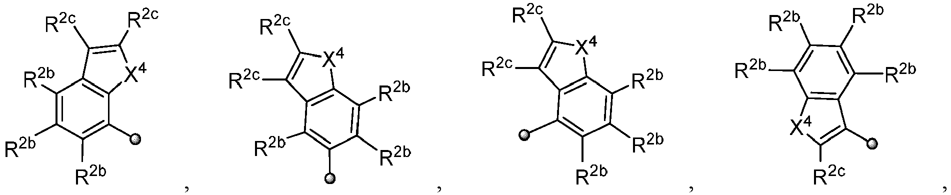 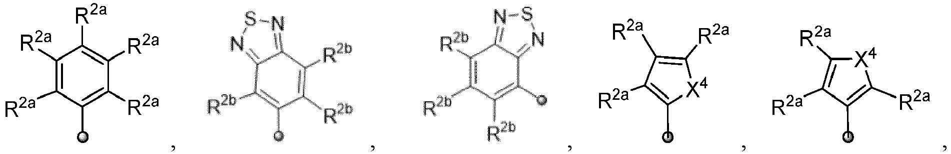 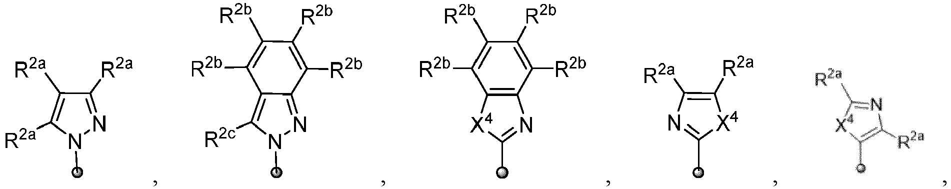 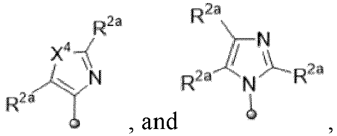
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl; the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl being optionally substituted by the following substituents for one or more times identically or differently, the substituents being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl.

R³ is selected from: 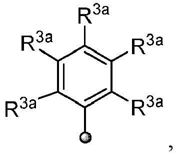 where
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
the R⁹ and R¹⁰ are the same or different, and are each independently selected from the group consisting of: hydrogen, optionally substituted C1-C4 alkyl, optionally substituted halogenated C1-C4 alkyl, optionally substituted C1-C4 alkoxy, and optionally substituted halogenated C1-C4 alkoxy; or
R⁹ and R¹⁰ are the same or different, and are each independently selected from the group consisting of: optionally substituted aryl, optionally substituted heterocyclyl, and optionally substituted heteroaryl;
the aryl is selected from: phenyl;
the heterocyclyl is selected from the group consisting of: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl;
the R¹¹ is selected from the group consisting of: hydrogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, and optionally substituted halogenated C3-C6 cycloalkyl or alkoxy; or
R¹¹ is selected from the group consisting of: aryl, heterocyclyl and heteroaryl;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl and the heteroaryl are substituted by the following substituents for one or more times identically or differently: halogen, hydroxy, C1-C6 hydroxyalkyl, C1-C6 alkyl, C1-C6 alkoxy, C1-C6 haloalkyl or alkoxy, amino, acetyl, acetamido, methylsulfonamido, methyl-benzenesulfonamido, cyano, nitro, sulfo-group, ureido, and guanidyl;
the R¹² is selected from the group consisting of: hydrogen, C1-C6 alkyl, halogenated C1-C6 alkyl, aryl, heterocyclyl, and heteroaryl; the C1-C6 alkyl being substituted by the following substituents for one or more times identically or differently: -O-R²⁰, -OH, -N(H)C(=O)-R²⁰, and -NR⁹R¹⁰;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the phenyl, the heterocyclyl, and the heteroaryl are optionally substituted by a substituent R¹⁸ for one or more times identically or differently;
the R¹³ is selected from the group consisting of: hydrogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, and optionally substituted halogenated C3-C6 cycloalkyl or alkoxy ; or, the R¹³ is selected from the group consisting of: -(CH₂)_{q}-phenyl, -(CH₂)_{q}-halogenated phenyl, -(CH₂)_{q}-heterocyclyl, -(CH₂)_{q}-halogenated heterocyclyl, -(CH₂)_{q}-heteroaryl, and -(CH₂)_{q}-halogenated heteroaryl;
the heterocyclyl is selected from the group consisting of: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the phenyl, the heterocyclyl, and the heteroaryl are optionally substituted by the following substituents for one or more times: methyl, ethyl, Cl, F, and cyano;
the R¹⁵ is selected from: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the R¹⁷ is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, optionally substituted aryl, optionally substituted heterocyclyl, and optionally substituted heteroaryl;
the aryl is selected from: phenyl;
the heterocyclyl is selected from the group consisting of: 4-7 membered saturated heterocyclyl containing oxygen or nitrogen; the saturated heterocyclyl being selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
more preferably, the R¹⁷ is selected from the group consisting of: methyl, cyclopropyl, phenyl, thienyl, and indolyl; the phenyl, the thienyl, and the indolyl being optionally substituted by the following substituents for one or more times: methyl, ethyl, Cl, F, and cyano;
the R¹⁸ is selected from the group consisting of: hydrogen, F, Cl, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy, nitro, optionally substituted amino, hydroxyl, C1-C6 hydroxyalkyl, phenyloxy, heteroaryloxy, -(CH₂)_{q}-COOH, -O-C(=O)-R²⁰, optionally substituted amido, cyano, sulfo-group, optionally substituted sulfonamido, optionally substituted acyl, and optionally substituted sulfonyl;
the R²⁰ is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, phenyl, and morpholinyl; the phenyl being substituted by the following substituents for one or more times: methyl, ethyl, Cl, F, and cyano;
more preferably, R^{3a} is selected from the group consisting of: hydrogen, fluorine, chlorine, amino, dimethylamino, nitro, cyano, methyl, isopropyl, trifluoromethyl, hydroxymethyl, hydroxyethyl, -(CH₂)_{q}-COOH, thiophenyl, acetyl, propionyl, isobutyryl, ethylcarbamoyl, (fluorophenyl)carbamoyl, phenylcarbamoyl, (chlorophenyl)thiocarbamoyl)oxy)methyl, (ethylthiocarbamoyl)oxy)methyl, acetoxylmethyl, (octanoyloxy)methyl, morpholine carbonyl, acetylamino, acetylaminomethyl, thiophene-formamido, (cyclopropylureido)methyl, hexylureido, cyclohexylureido, (chlorophenyl)ureido, ((trifluoromethyl)phenyl)ureido, ((trifluoromethoxy)phenyl)ureido, (cyanophenyl)ureido, propylthioureido, (chlorophenyl)thioureido, ((trifluoromethyl)phenyl)thioureido, (methoxyphenyl)thioureido, (cyanophenyl)thioureido, cyclopropylsulfonamido, cyclopropylsulfonamidomethyl, ((methylphenyl)sulfonamido)methyl, N-(mesyl)methylsulfonamido, (methylsulfonamido)thiazolyl, morpholinylmethyl, ((chlorophenoxy)carbonyl)amino, (phenylamino)methyl, (benzylamino)methyl, methoxy, ethoxy, propoxy, butoxy, isopropoxy, ethoxymethyl, methoxyethoxy, trifluoromethoxy, acetylaminoethoxy, phenoxy, fluorophenoxy, chlorophenoxy, (trifluoromethyl)phenoxy, cyanophenoxy, methoxyphenoxy, acetylphenoxy, (methylsulfonyl)phenoxy, phenoxymethyl, (fluorophenoxy)methyl, (chlorophenoxy)methyl, (trifluoromethyl)phenoxy)methyl, (tetrahydro-2H-pyranyl)oxy), (tetrahydro-2H-pyranyl)oxy)methyl, (pyridyloxy)methyl, ((fluoropyridyl)oxy)methyl, (pyrimidinyloxy)methyl, (tetrahydrofuranyl)oxymethyl, (oxetanyloxy)methyl, (acetylpiperidinyl)oxymethyl), (hydroxyethoxy)methyl, (acetylaminoethoxy)methyl), acetylaminoethoxy, (methoxyethoxy)methyl, aminoethoxy, (methylamino)ethoxy, ((((tetrahydro-2H-pyran-4-yl)oxy)carbonyl)oxy)methyl, and methoxyphenylureido;
p and q are each independently 0, 1, 2 or 3.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 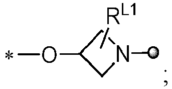
R² is selected from the group consisting of: hydrogen, amino, halogen, optionally substituted C1-C4 alkyl, 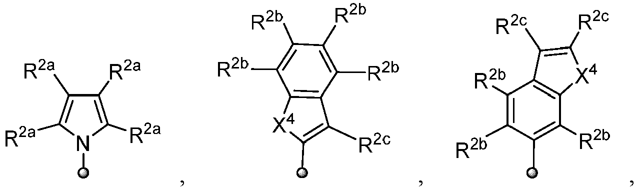 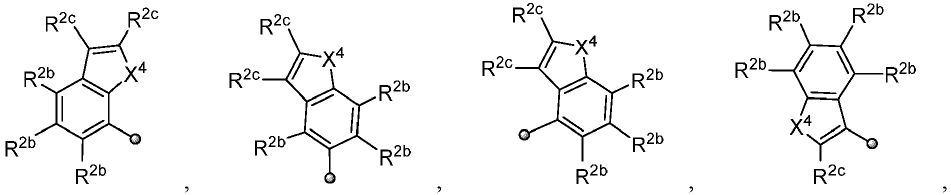 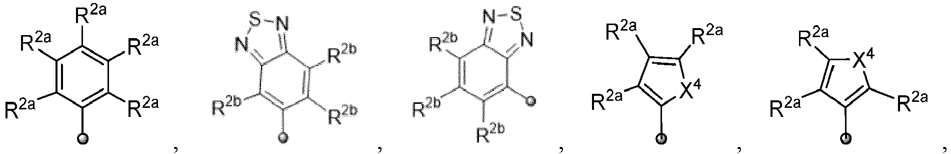 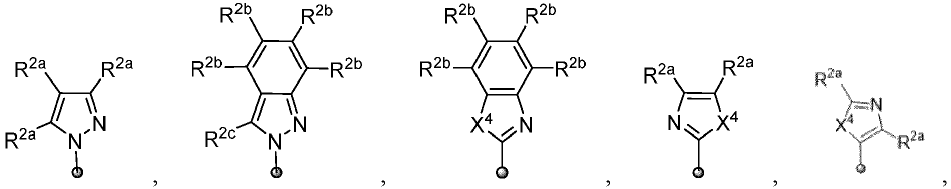 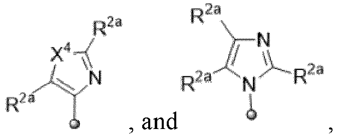
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from 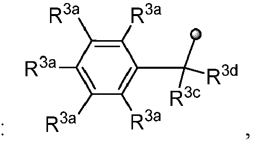 where R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, or imino;
preferably, R^{3a} is selected from: methoxy.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 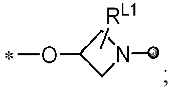
R² is selected from: hydrogen, amino, halogen, optionally substituted C1-C4 alkyl, 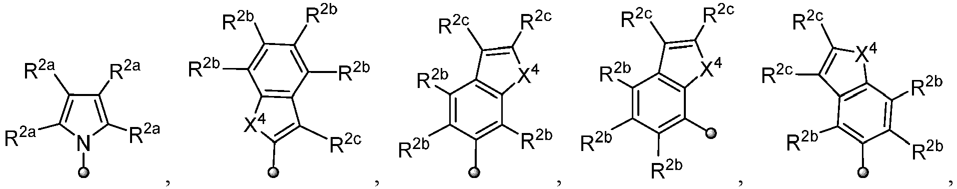 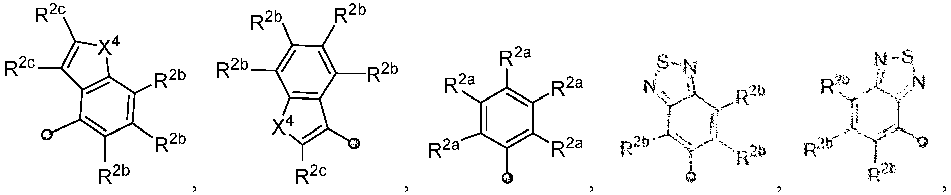 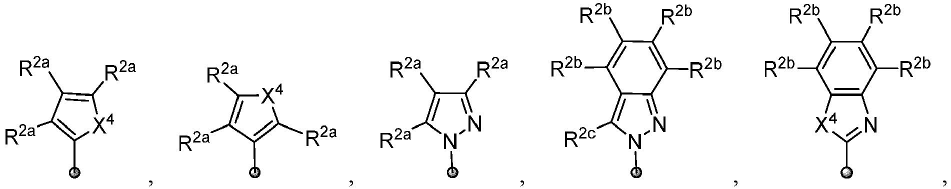 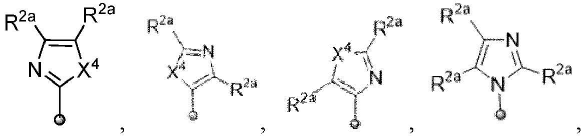
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from: 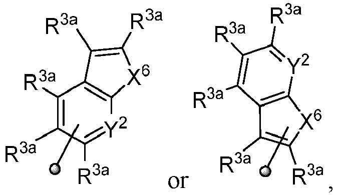 where
X⁶ is selected from the group consisting of O, S and NR^{3g},
Y² is selected from the group consisting of CH, CR^{3a} and N, and Y² may replace a C atom at an optional position of an aromatic ring,
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl; R^{3g} being selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl, and optionally substituted phenylsulfonyl;
the acyl is selected from the group consisting of: acetyl, and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl, F;
preferably, R^{3a} is selected from: hydrogen or tosyl.

In another preferred embodiment, the present invention relates to the compound having the above general formula (I), or an enantiomer, a diastereomer, a tautomer, a salt, a crystalline form, a solvate and/or an isotopically substituted derivative thereof, where
L is selected from:

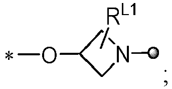

R² is selected from the group consisting of: hydrogen, amino, halogen, optionally substituted C1-C4 alkyl, 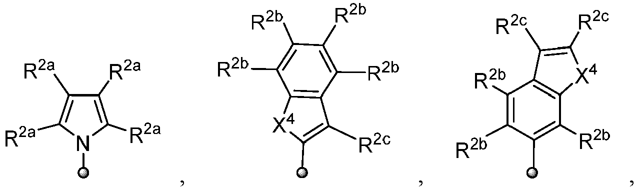 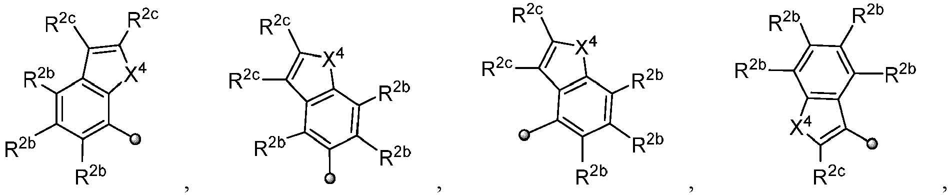 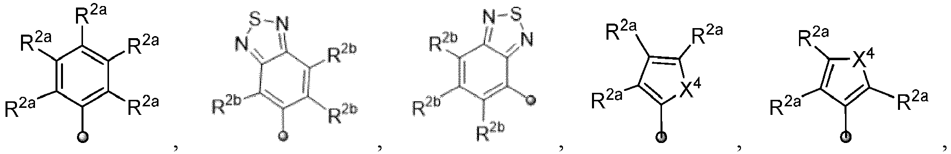 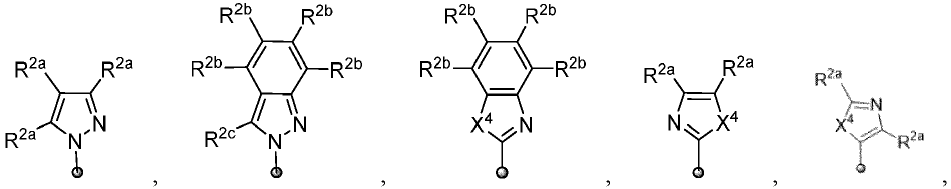 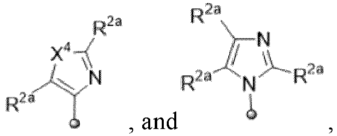
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from: 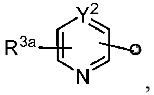
where Y² is selected from the group consisting of CH, CR^{3a}, and N, and Y² may replace a C atom at an optional position of an aromatic ring,
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
preferably, R^{3a} is selected from the group consisting of: hydrogen, fluorine, chlorine, and tosyl.

In another preferred embodiment, the present invention relates to the compound having the above general formula (I), or an enantiomer, a diastereomer, a tautomer, a salt, a crystalline form, a solvate and/or an isotopically substituted derivative thereof, where
L is selected from 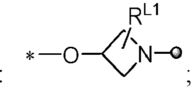
R² is selected from the group consisting of: hydrogen, amino, halogen, optionally substituted C1-C4 alkyl, 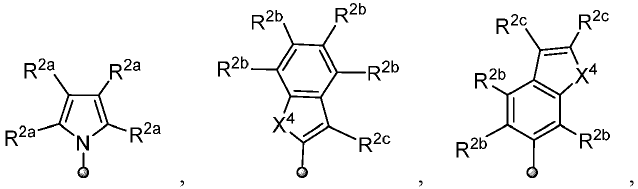 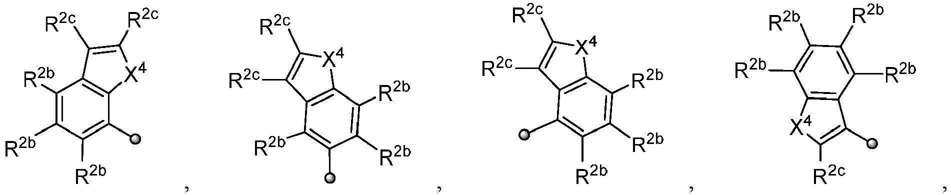 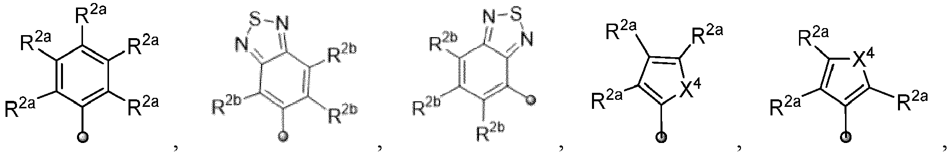 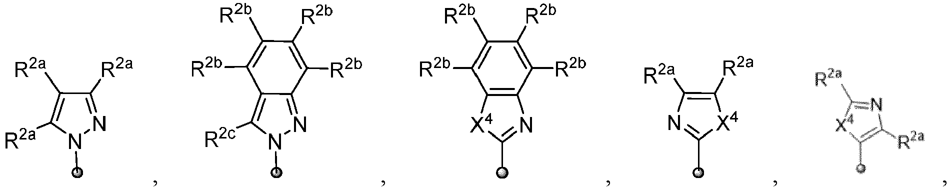 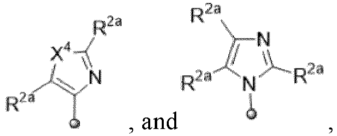
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from the group consisting of: 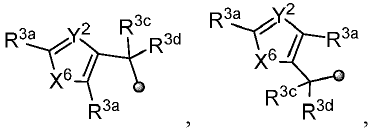 and 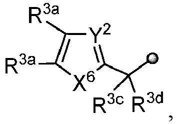 where
X⁶ is selected from the group consisting of O, S and NR^{3g},
Y² is selected from the group consisting of CH, CR^{3a} and N, and Y² may replace a C atom at an optional position of an aromatic ring,
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3g} is selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl, and optionally substituted phenylsulfonyl;
the acyl is selected from the group consisting of: acetyl, and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, or imino.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 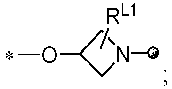
R² is selected from the group consisting of: hydrogen, amino, halogen, optionally substituted C1-C4 alkyl, 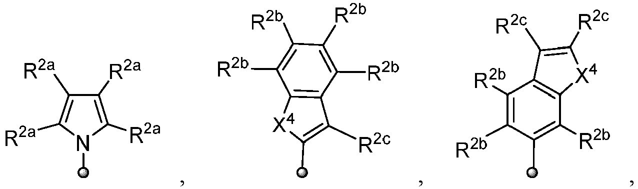 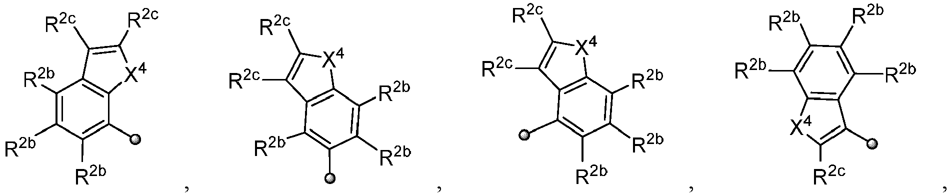 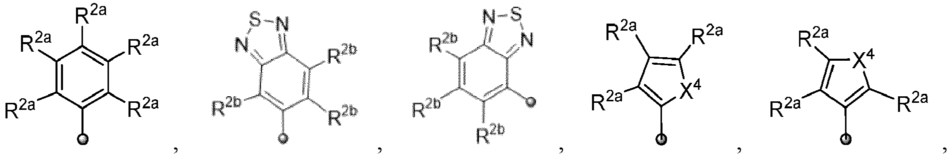 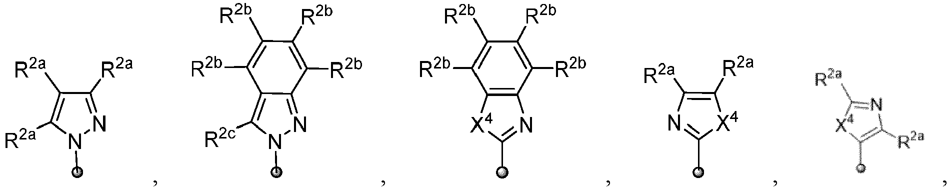 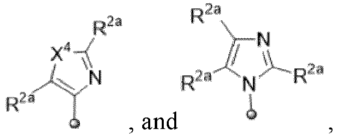
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from: 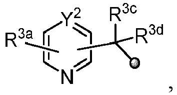 where
Y² is selected from the group consisting of CH, CR^{3a} and N, and Y² may replace a C atom at an optional position of an aromatic ring;
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, or imino.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 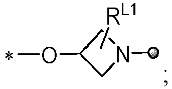
R² is selected from the group consisting of: hydrogen, amino, halogen, optionally substituted C1-C4 alkyl,

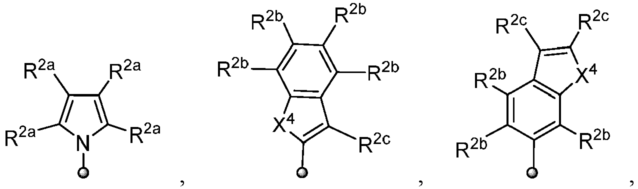

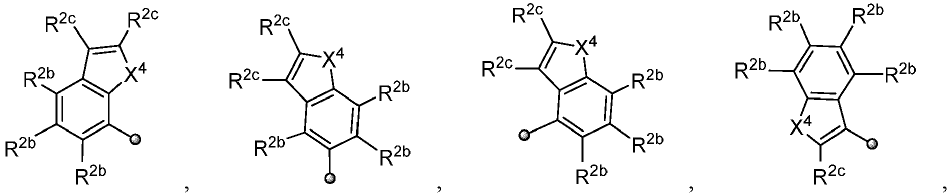

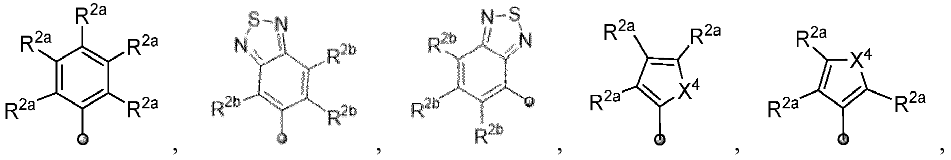

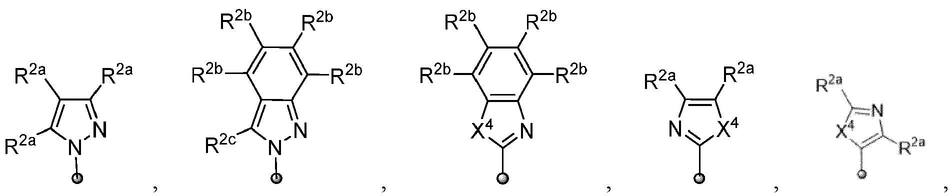

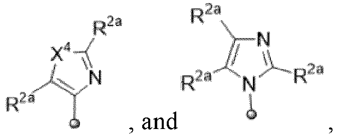

X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from: 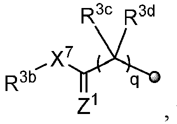 where
X⁷ is selected from O or NR^{3g},
Z¹ is selected from O or S,
R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
preferably, R^{3b} is selected from the group consisting of:
   optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, aryl-(CH₂)_{q}-, and heteroaryl-(CH₂)_{q}-; the aryl and the heteroaryl being optionally substituted by a substituent R¹⁸ for one or more times identically or differently;
the aryl is selected from: phenyl;
the heterocyclyl is selected from: 4-7 membered saturated heterocyclyl containing oxygen and/or nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl; the R¹⁸ is selected from the group consisting of: hydrogen, halogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy , nitro, optionally substituted amino, hydroxyl, C1-C6 hydroxyalkyl, aryloxy, heterocyclyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R⁴ for one or more times identically or differently;
more preferably, the R¹⁸ is selected from the group consisting of: halogen, C1-C6 straight chain alkyl or alkoxy, C3-C6 branched chain alkyl or alkoxy, C3-C6 cycloalkyl or alkoxy, halogenated C1-C6 straight chain alkyl or alkoxy, halogenated C3-C6 branched chain alkyl or alkoxy, halogenated C3-C6 cycloalkyl or alkoxy, morpholinyl and phenoxy;
further preferably, the R¹⁸ is selected from the group consisting of: hydrogen, fluorine, chlorine, bromine, methyl, ethyl, propyl, isopropyl, cyclopropyl, methoxy, ethoxy, propoxy, isopropoxy, cyclopropoxy, trifluoromethyl, trichloromethyl, trifluoromethoxy, trichloromethoxy, morpholinyl and phenoxy;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, and heteroaryl-(CH₂)_{q}-; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
R^{3c} and R^{3d} are the same or different, and are each independently selected from the group consisting of: hydrogen, fluorine, and C1-C6 alkyl;
q is 0, 1, 2 or 3;
preferably, R^{3b} is selected from the group consisting of: methyl, ethyl, cyclopropyl, cyclohexyl, and phenyl;
more preferably, R³ is selected from:

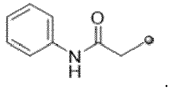

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 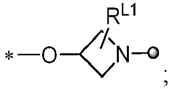
R² is selected from the group consisting of: hydrogen, amino, halogen, optionally substituted C1-C4 alkyl, 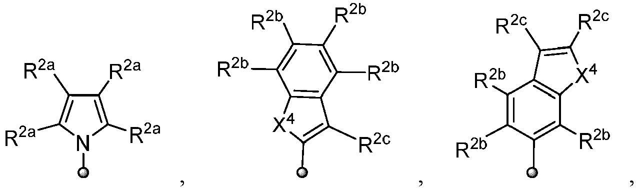 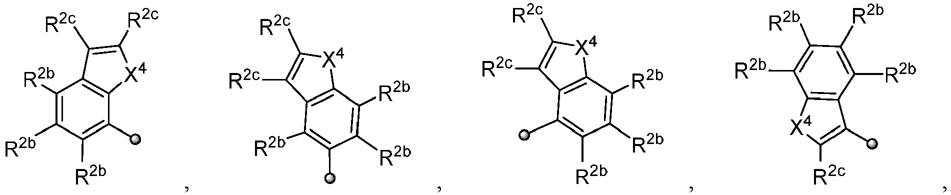 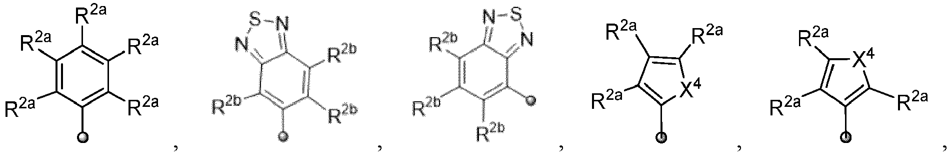 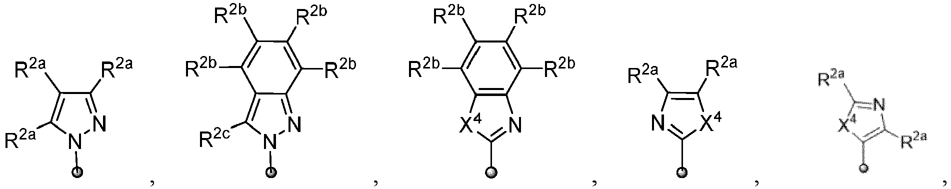 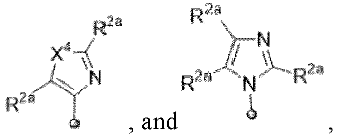
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from the group consisting of: 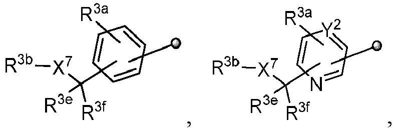 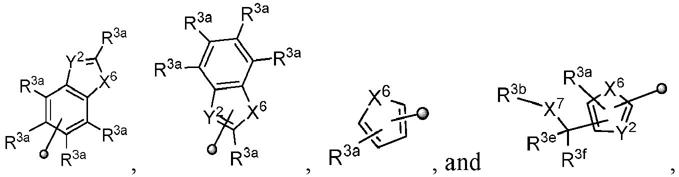 where
X⁶ is selected from the group consisting of O, S and NR^{3g};
X⁷ is selected from the group consisting of O, S and NR^{3g};
Y² is selected from the group consisting of CH, CR^{3a} and N, and Y² may replace a C atom at an optional position of an aromatic ring;
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
preferably, R^{3b} is selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O) N(H)-R¹⁹, -C(=S) N(H)-R¹⁹, and -C(=O) O-R¹⁹; the aryl and the heteroaryl being optionally substituted by a substituent R¹⁸ for one or more times identically or differently;
preferably, the R¹⁸ is selected from the group consisting of: hydrogen, halogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy, nitro, optionally substituted amino, hydroxy, C1-C6 hydroxyalkyl, aryloxy, heteroaryloxy, -(CH₂)_{q}-COOH, -O-C(=O)-R²⁰, optionally substituted amido, cyano, sulfo-group, optionally substituted sulfonamido, optionally substituted acyl, and optionally substituted sulfonyl;
preferably, R^{3b} is selected from the group consisting of:
   optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, aryl-(CH₂)_{q}-, and heteroaryl-(CH₂)_{q}-; the aryl and the heteroaryl being optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl and optionally substituted phenylsulfonyl;
the acyl is selected from the group consisting of: acetyl and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 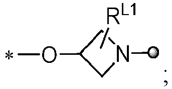
R² is selected from the group consisting of: hydrogen, amino, halogen, optionally substituted C1-C4 alkyl, 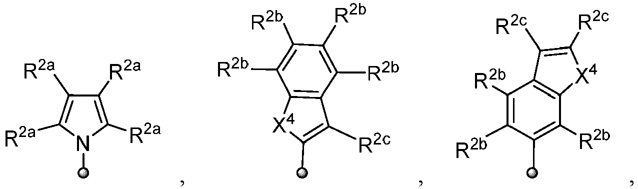 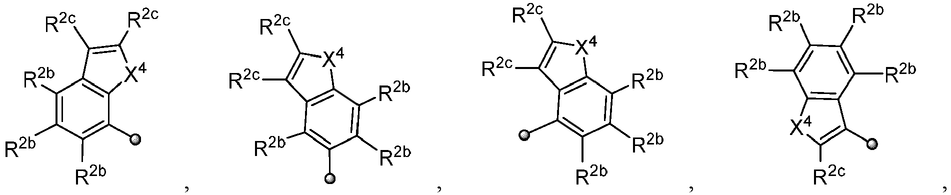 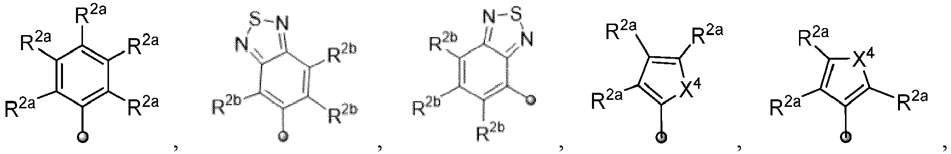 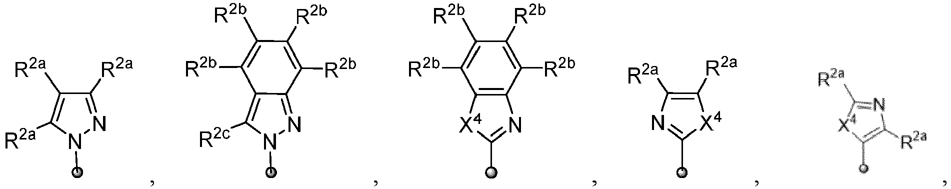 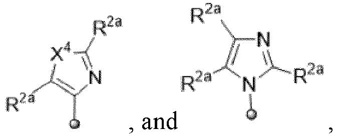
X⁴ is selected from the group consisting of O, S and NR^{2d};
where R^{2a}, R^{2b}, and R^{2c} are the same or different, and are each independently selected from the group consisting of:
   hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl, the phenyl being optionally substituted by a substituent R⁴ for one or more times identically or differently;
preferably, R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl are optionally substituted by the following substituents for one or more times identically or differently, the substituent being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
more preferably, R² is selected from the group consisting of: hydrogen, amino, pyrrolyl, benzofuryl, benzo[b]thienyl, indolyl, phenyl, halogenated phenyl, cyanophenyl, cyano-halogenated phenyl, carboxyphenyl, halogenated C1-C3 alkylphenyl, biphenyl, methylsulfonamidophenyl, C1-C3 alkoxyphenyl, benzo[c][1,2,5]thiadiazolyl, furyl, pyrazolyl, indazolyl, and benzo[d]thiazolyl;
R³ is selected from: 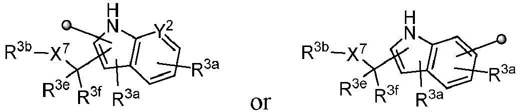 or 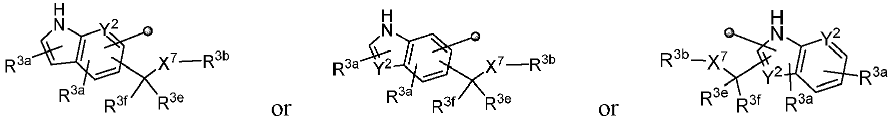 or 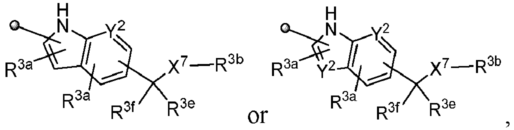
where R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
X⁷ is selected from O or NR^{3g},
R^{3b} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O)N(H)-R¹⁹, -C(=S)N(H)-R¹⁹, and -C(=O)O-R¹⁹; the phenyl, the aryl, and the heteroaryl being optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl and optionally substituted phenylsulfonyl;
the acyl is selected from the group consisting of: acetyl and, optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F;
Y² is selected from the group consisting of CH, CR^{3a} and N, and Y² may replace a C atom at an optional position of an aromatic ring;
R^{3e} and R^{3f} are the same or different, and are each independently selected from the group consisting of:
   hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O)N(H)-R¹⁹, -C(=O)O-R¹⁹, and -S(=O)₂-R²⁰; or
R^{3e} and R^{3f} together with an atom to which they are attached form carbonyl; or
R^{3e} and R^{3f} are the same or different, and are each independently selected from the group consisting of: aryl, heterocyclyl and heteroaryl;
the aryl is selected from: phenyl;
the heterocyclyl is selected from: 4-7 membered saturated heterocyclyl containing oxygen or nitrogen the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 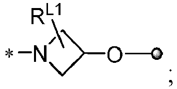
R² is selected from the group consisting of: hydrogen, 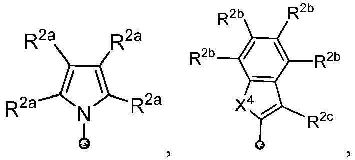 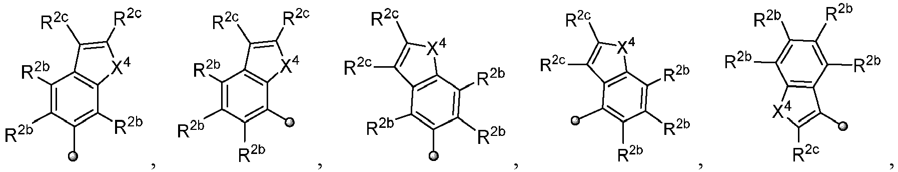 and 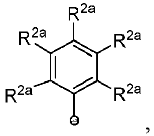
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, R³ is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
more preferably, R³ is selected from the group consisting of: optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 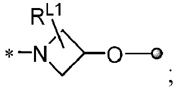
R² is selected from the group consisting of: hydrogen, 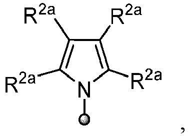 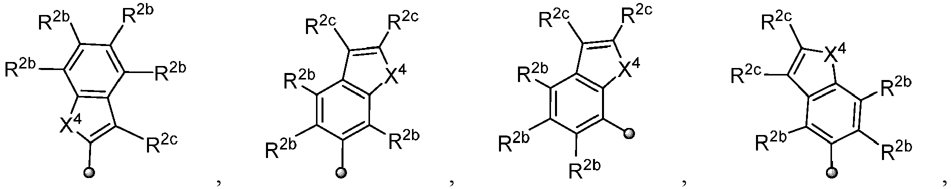 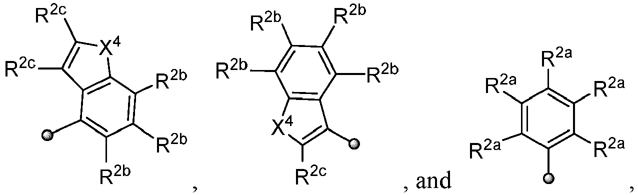
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from 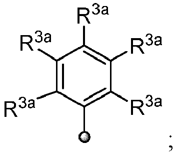
R^{3a} is selected from the group consisting of: hydrogen, halogen, nitro, cyano, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, optionally substituted halogenated C3-C4 cycloalkyl, and -C(=O)-NHR¹³;
the R¹³ is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl; or
the R¹³ is selected from: -(CH₂)_{q}-phenyl or -(CH₂)_{q}-halogenated phenyl;
preferably, R¹³ is selected from the group consisting of: hydrogen, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl; or
preferably, the R¹³ is selected from the group consisting of: -(CH₂)_{q}-phenyl, and -(CH₂)_{q}-halogenated phenyl; the halogenation is selected from the group consisting of: fluoro and chloro;
further preferably, R¹³ is selected from: (benzylcarbamoyl)phenyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 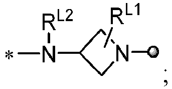
R^{L1} is selected from: hydrogen or F,
R^{L2} is selected from: hydrogen,
R² is selected from the group consisting of: hydrogen, 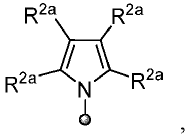 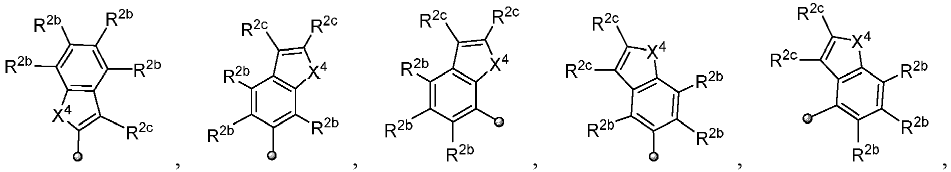 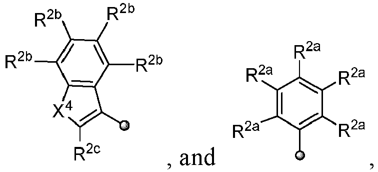
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from the group consisting of: C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl; the C1-C8 straight chain alkyl, the C3-C8 branched chain alkyl, the C3-C8 cycloalkyl, the halogenated C1-C8 straight chain alkyl, the halogenated C3-C8 branched chain alkyl, and the halogenated C3-C8 cycloalkyl being optionally substituted by a substituent R⁶ for one or more times identically or differently.

In a preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 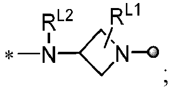
R^{L1} is selected from: hydrogen or F,
R^{L2} is selected from: hydrogen,
R^{L2} is selected from: hydrogen,
R² is selected from the group consisting of: hydrogen, 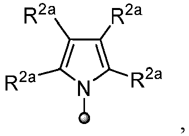 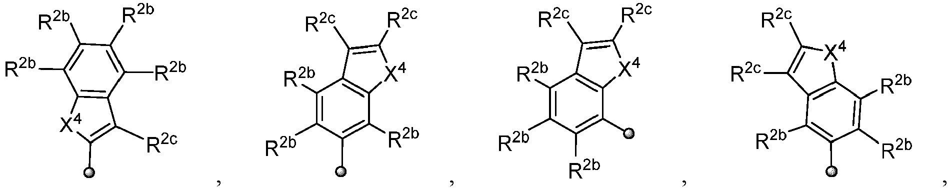 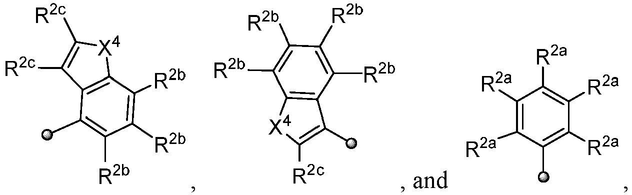
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 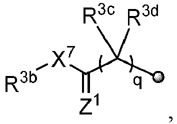 where
X⁷ is selected from: NR^{3g},
Z¹ is selected from: O or S,
q is 0, 1, 2 or 3;
R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heterocyclyl-(CH₂)_{q}-, and heteroaryl-(CH₂)_{q}-;
the aryl is selected from: phenyl;
the heterocyclyl is selected from: 4-7 membered saturated heterocyclyl containing oxygen and/or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
preferably, R^{3b} is selected from the group consisting of:
   optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and aryl-(CH₂)_{q}-; the aryl is selected from: phenyl;
the phenyl is optionally substituted by a substituent R¹⁸ for one or more times identically or differently; the R¹⁸ is selected from the group consisting of: F, Cl, nitro, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenoxy; preferably, the R¹⁸ is selected from the group consisting of: F, Cl, methyl, ethyl, propyl, isopropyl, cyclopropyl, methoxy, ethoxy, propoxy, isopropoxy, cyclopropoxy, trifluoromethyl, trichloromethyl, trifluoromethoxy, trichloromethoxy, and phenoxy;
more preferably, R^{3b} is selected from the group consisting of:
   optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, optionally substituted halogenated C3-C4 cycloalkyl, and aryl-(CH₂)_{q}-; the aryl is selected from: phenyl;
further preferably, R^{3b} is selected from the group consisting of:
   methyl, ethyl, propyl, isopropyl, cyclopropyl, trifluoromethyl, and trichloromethyl;
R^{3g} is selected from: hydrogen.

In another preferred embodiment, the present invention relates to the compound having the above general formula (I), or an enantiomer, a diastereomer, a tautomer, a salt, a crystalline form, a solvate and/or an isotopically substituted derivative thereof, where
L is selected from:

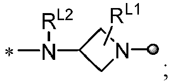

R^{L1} is selected from: hydrogen or F,
R^{L2} is selected from: hydrogen,
R² is selected from: hydrogen, 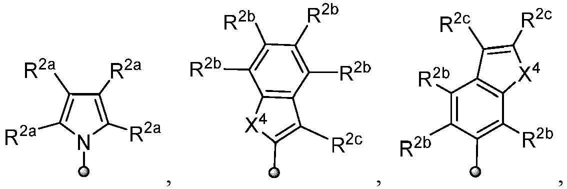 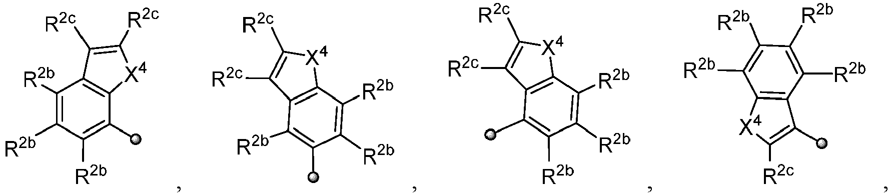 and 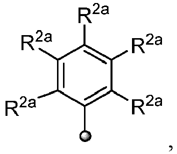
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 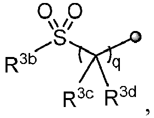
where R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
preferably, the R¹⁸ is selected from the group consisting of: F, Cl, C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl;
more preferably, R^{3b} is selected from the group consisting of:
   optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, and aryl-(CH₂)_{q}-; the aryl being optionally substituted by a substituent R¹⁸ for one or more times identically or differently;
the aryl is selected from: phenyl;
the R¹⁸ is selected from the group consisting of: F, Cl, C1-C4 straight chain alkyl, C3-C4 branched chain alkyl, C3-C4 cycloalkyl, halogenated C1-C4 straight chain alkyl, halogenated C3-C4 branched chain alkyl, and halogenated C3-C4 cycloalkyl; further preferably, the R¹⁸ is selected from the group consisting of: methyl, ethyl, propyl, isopropyl, cyclopropyl, trifluoromethyl, and trichloromethyl;
further preferably, R^{3b} is selected from the group consisting of:
methyl, ethyl, propyl, isopropyl, cyclopropyl, trifluoromethyl, and trichloromethyl;
R^{3g} is selected from: hydrogen or aryl-(CH₂)_{q}-; the aryl being optionally substituted by a substituent R¹⁸ for one or more times identically or differently; the aryl being selected from: phenyl; preferably, R^{3g} being selected from: hydrogen or phenylmethyl.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 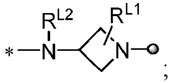
R^{L1} is selected from: hydrogen or F,
R^{L2} is selected from: hydrogen,
R² is selected from the group consisting of: hydrogen, 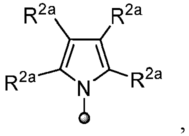 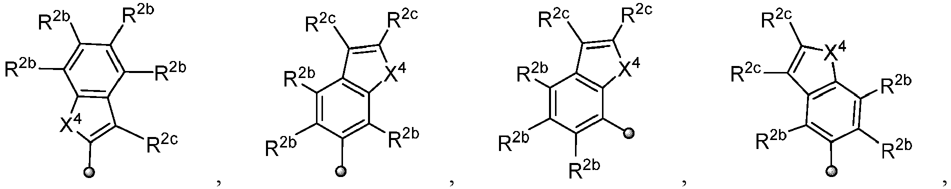 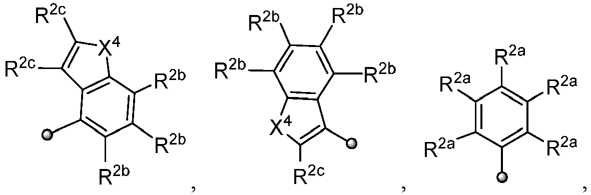
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 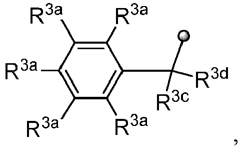 where
R^{3a} is selected from the group consisting of:
   hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)_{P}-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)_{P}-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently; R^{3c} and R^{3d} together with the atom to which they are attached form carbonyl.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 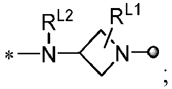
R^{L1} is hydrogen, and R^{L2} is aryl-(CH₂)_{q}-; the aryl being selected from: phenyl;
R² is selected from the group consisting of: hydrogen, 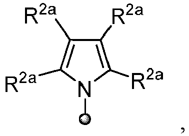 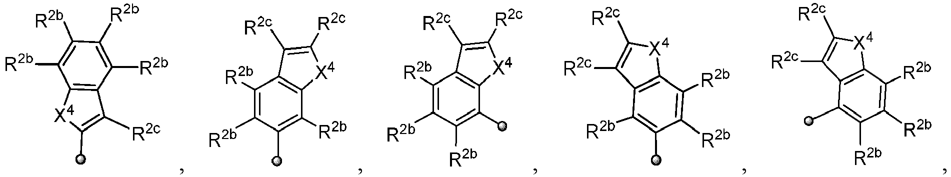 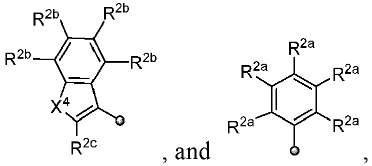 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
R³ is selected from: 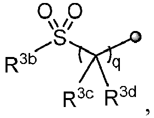 where
R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently;
preferably, R^{3b} is selected from: aryl-(CH₂)_{q}-; the aryl bejing optionally substituted by a substituent R¹⁸ for one or more times identically or differently;
the aryl is selected from: phenyl;
the R¹⁸ is selected from the group consisting of: F, Cl, C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl.

Preferably, the R¹⁸ is selected from the group consisting of: F, Cl, C1-C6 straight chain alkyl, C3-C6 branched chain alkyl, C3-C6 cycloalkyl, halogenated C1-C6 straight chain alkyl, halogenated C3-C6 branched chain alkyl, and halogenated C3-C6 cycloalkyl;
more preferably, the R¹⁸ is selected from the group consisting of: F, Cl, C1-C4 straight chain alkyl, C3-C4 branched chain alkyl, C3-C4 cycloalkyl, halogenated C1-C4 straight chain alkyl, halogenated C3-C4 branched chain alkyl, and halogenated C3-C4 cycloalkyl;
further preferably, the R¹⁸ is selected from the group consisting of: methyl, ethyl, propyl, isopropyl, cyclopropyl, trifluoromethyl, and trichloromethyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 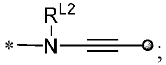
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, R^{L2} is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
preferably, R^{L2} is selected from the group consisting of: hydrogen, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl;
more preferably, R^{L2} is selected from the group consisting of: methyl, ethyl, propyl, isopropyl, isobutyl, and tert-butyl;
R² is selected from the group consisting of: 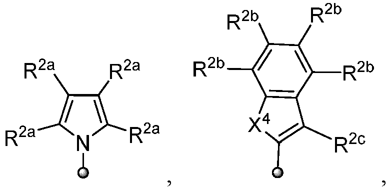 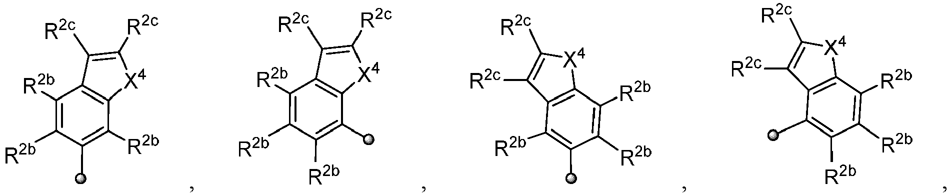 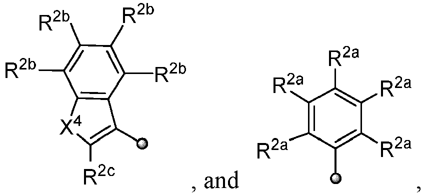 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: pyrrolyl, indolyl and phenyl, the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
more preferably, R² is selected from the group consisting of: pyrrolyl, indolyl, and phenyl, the pyrrolyl, the indolyl, and the phenyl are substituted by the following substituents for one or more times, the substituent bing: fluorine, chlorine or methoxy;
R³ is selected from: 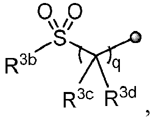 where
R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently;
the R¹⁸ is selected from the group consisting of: F, Cl, nitro, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenoxy;
more preferably, the R¹⁸ is selected from the group consisting of: methyl, ethyl, propyl, isopropyl, cyclopropyl, methoxy, ethoxy, propoxy, isopropoxy, cyclopropoxy, trifluoromethyl, trichloromethyl, trifluoromethoxy, trichloromethoxy, and phenoxy.

In another preferred embodiment, the present invention relates to the compound having the above general formula (I), or an enantiomer, a diastereomer, a tautomer, a salt, a crystalline form, a solvate and/or an isotopically substituted derivative thereof, where:
L is selected from: 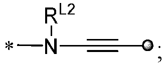
R^{L2} is selected from the group consisting of: aryl-(CH₂)_{q}-, heterocyclyl-(CH₂)_{q}-, and heteroaryl-(CH₂)_{q}-;
the aryl-(CH₂)_{q}- is selected from: phenyl-CH₂-;
the heterocyclyl-(CH₂)_{q}- is selected from: piperidinyl-CH₂-;
the heteroaryl-(CH₂)_{q}- is selected from the group consisting of: indolyl-CH₂-, thienyl-CH₂-, benzothienyl-CH₂-, furanyl-CH₂-, benzofuryl-CH₂-, and pyridyl-CH₂-;
where phenyl, indolyl, thienyl, benzothienyl, furanyl, benzofuryl, pyridyl, and piperidinyl are substituted by the following substituents for one or more times, the substituent being selected from the group consisting of: halogen, cyano, -NR⁹R¹⁰, hydroxyl, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, optionally substituted amido, and optionally substituted sulfonamido;
preferably, the substituents are selected from the group consisting of: fluorine, chlorine, cyano, -NR⁹R¹⁰, hydroxyl, C1-C6 hydroxyalkyl, C1-C6 straight chain alkyl or alkoxy, C3-C6 branched chain alkyl or alkoxy, C3-C6 cycloalkyl or alkoxy, halogenated C1-C6 straight chain alkyl or alkoxy, halogenated C3-C6 branched chain alkyl or alkoxy, halogenated C3-C6 cycloalkyl or alkoxy, optionally substituted amido, and optionally substituted sulfonamido;
the sulfonamido is selected from the group consisting of: methylsulfonamido, and optionally substituted phenylsulfonamido;
the amido is selected from the group consisting of: acetamido, and optionally substituted benzamido;
the phenylsulfonamido and the benzamido are optionally substituted by the following substituents for one or more times: methyl, ethyl, methoxy, Cl, F, and cyano; the methyl, the ethyl, and the methoxy are optionally substituted by the following substituents for one or more times: Cl and F;
more preferably, the substituent are selected from the group consisting of: fluorine, chlorine, cyano, -NR⁹R¹⁰, hydroxyl, C1-C4 hydroxyalkyl, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, optionally substituted amido, and optionally substituted sulfonamido;
further preferably, the substituent is selected from the group consisting of: hydrogen, fluorine, chlorine, cyano, -NH₂, hydroxyl, hydroxymethyl, methyl, trifluoromethyl, methoxy, and trifluoromethoxy;
R² is selected from the group consisting of: 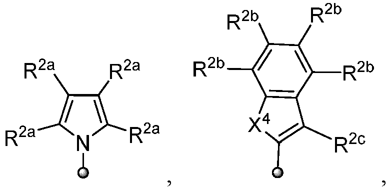 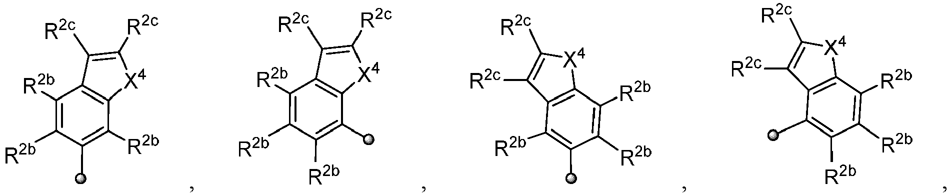 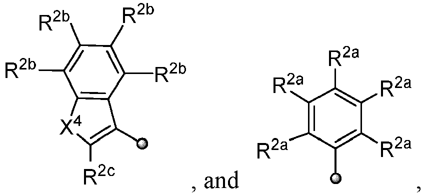
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: pyrrolyl, indolyl, and phenyl, the pyrrolyl, the indolyl, and the phenyl are optionally substituted by the following substituents for one or more times identically or differently, the substituent being selected from: hydrogen, fluorine, chlorine, methyl, trifluoromethyl, methoxy, and trifluoromethoxy;
more preferably, R² is selected from the group consisting of: pyrrolyl, indolyl, and phenyl, the pyrrolyl, the indolyl, and the phenyl are substituted by the following substituents for one or more times, the substituent being: fluorine, chlorine and methoxy;
R³ is selected from 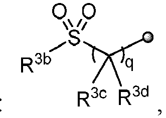 where
R^{3b} is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently;
preferably, the R¹⁸ is selected from the group consisting of: hydrogen, halogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, aryl, heteroaryl, and heterocyclyl; the aryl being selected from: phenyl;
preferably, R^{3b} is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, aryl-(CH₂)_{q}-, and heteroaryl-(CH₂)_{q}-;
the R¹⁸ is selected from the group consisting of: F, Cl, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenoxy;
more preferably, the R¹⁸ is selected from the group consisting of: methyl, ethyl, propyl, isopropyl, cyclopropyl, methoxy, ethoxy, propoxy, isopropoxy, cyclopropoxy, trifluoromethyl, trichloromethyl, trifluoromethoxy, trichloromethoxy, and phenoxy;
more preferably, R^{3b} is selected from the group consisting of: methyl, ethyl, propyl, cyclopropyl, isopropyl, isobutyl, and t-butyl.

In another preferred embodiment, the present invention relates to the compound having the above general formula (I), or an enantiomer, a diastereomer, a tautomer, a salt, a crystalline form, a solvate and/or an isotopically substituted derivative thereof, where
L is selected from: 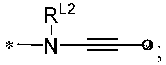
R² is selected from the group consisting of: 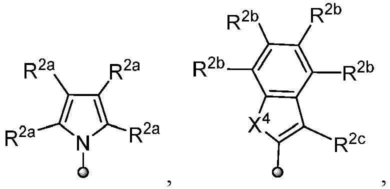 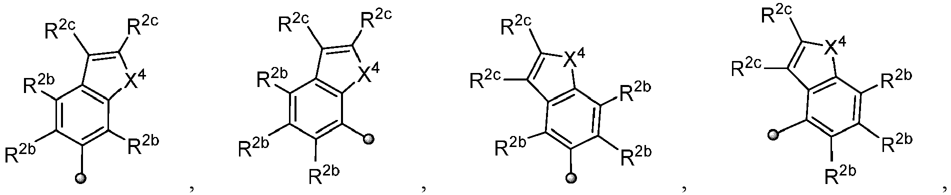 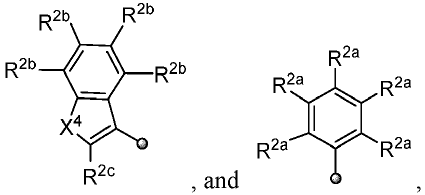
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: pyrrolyl, indolyl, and phenyl, the pyrrolyl, the indolyl, and the phenyl are optionally substituted by the following substituents for one or more times identically or differently, the substituent being selected from: hydrogen, fluorine, chlorine, methyl, trifluoromethyl, methoxy, and trifluoromethoxy;
more preferably, R² is selected from the group consisting of: pyrrolyl, indolyl, and phenyl, the pyrrolyl, the indolyl, and the phenyl are substituted by the following substituents for one or more times, the substituent being: fluorine, chlorine and methoxy;
R^{L2} and R³ are cyclized together with an atom to which they are attached to form 5-10 membered heterocyclyl containing nitrogen and/or sulfur, the 5-10 membered heterocyclyl containing nitrogen and/or sulfur comprising -C(=O)- or -S(=O)₂- groups;
preferably, the 5-10 membered heterocyclyl containing nitrogen is selected from: 1,1-dioxindibenzo[d]isothiazol-2(3H)-yl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 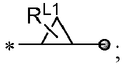
R^{L1} is selected from: hydrogen or fluorine;
R² is selected from the group consisting of: hydrogen, halogen, amino, 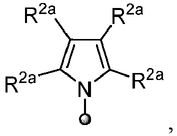 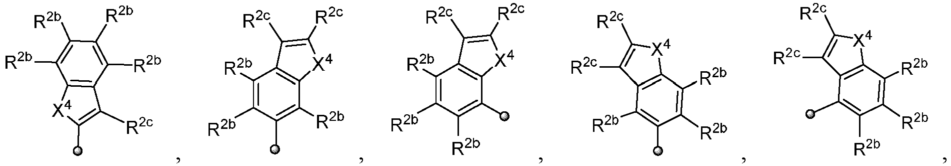 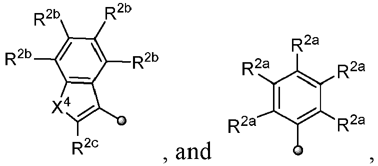
X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, halogen, amino, pyrrolyl, indolyl, and phenyl; the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
more preferably, R² is selected from the group consisting of: hydrogen, halogen, amino, pyrrolyl, indolyl, and phenyl; the pyrrolyl, the indolyl, and the phenyl are substituted by the following substituents for one or more times, the substituent being: fluorine, chlorine and methoxy;
R³ is selected from: 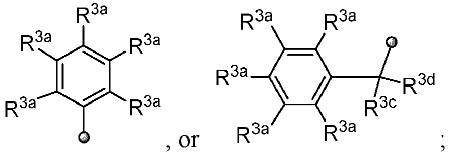
R^{3a} is selected from the group consisting of: hydrogen, halogen, nitro, cyano, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from: 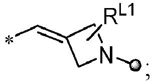
where
R^{L1} is selected from: hydrogen or fluorine;
R² is selected from the group consisting of: hydrogen, halogen, amino, 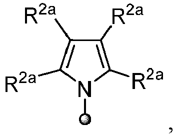 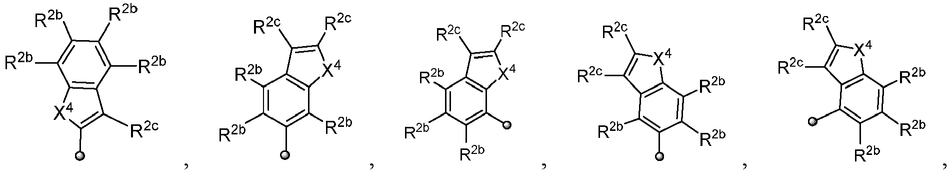 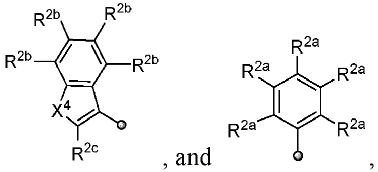 X⁴ is selected from NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, halogen, amino, pyrrolyl, indolyl, and phenyl; the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
more preferably, R² is selected from the group consisting of: hydrogen, halogen, amino, pyrrolyl, indolyl, and phenyl; the pyrrolyl, the indolyl, and the phenyl are substituted by the following substituents for one or more times, the substituent being: fluorine, chlorine and methoxy;
R³ is selected from: 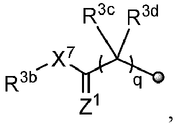 where
X⁷ is selected from: O or NR^{3g};
Z¹ is selected from: O;
R^{3b} is selected from the group consisting of:
   optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heterocyclyl-(CH₂)_{q}-, and heteroaryl-(CH₂)_{q}-;
q is 0, 1, 2 or 3;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
preferably, R^{3b} is selected from the group consisting of:
   optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, optionally substituted halogenated C3-C4 cycloalkyl, and aryl-(CH₂)_{q}-; the aryl being optionally substituted by a substituent R¹⁸ for one or more times identically or differently;
q is 0, 1, 2 or 3;
the aryl is selected from: phenyl;
the R¹⁸ is selected from the group consisting of: F, Cl, C1-C6 straight chain alkyl, C3-C6 branched chain alkyl, C3-C6 cycloalkyl, halogenated C1-C6 straight chain alkyl, halogenated C3-C6 branched chain alkyl, halogenated C3-C6 cycloalkyl, morpholinyl, and phenoxy;
more preferably, R^{3b} is selected from the group consisting of: methyl, ethyl, propyl, isopropyl, isobutyl, and tert-butyl;
R^{3g} is selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen and/or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from: methylsulfonyl, or optionally substituted phenylsulfonyl;
the acyl is selected from: acetyl, or optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl and F.

In a preferred embodiment, the present invention relates to the compound having the above general formula (I), or the enantiomer, the diastereomer, the tautomer, the salt, the crystalline form, the solvate and/or the isotopically substituted derivative thereof, where
L is selected from the group consisting of:

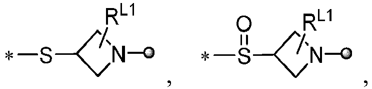

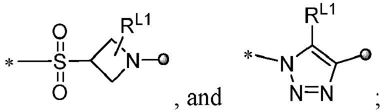

R^{L1} is selected from: hydrogen or fluorine;
R² is selected from the group consisting of: hydrogen, halogen, amino, 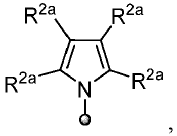 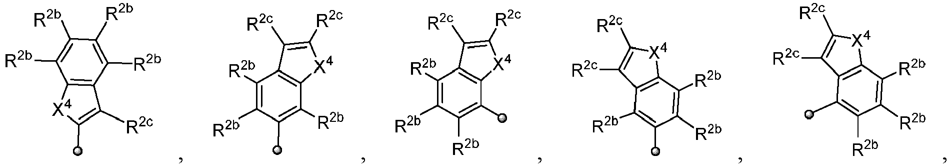 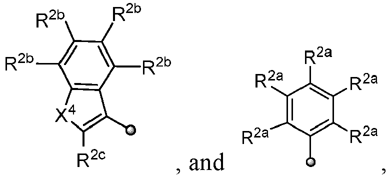 X⁴ is selected from O, S or NR^{2d};
where R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
preferably, R² is selected from the group consisting of: hydrogen, halogen, amino, pyrrolyl, indolyl, and phenyl; the pyrrolyl, the indolyl, and the phenyl are optionally substituted by the following substituents for one or more times identically or differently, the substituent being selected from the group consisting of: hydrogen, fluorine, chlorine, methyl, trifluoromethyl, methoxy, and trifluoromethoxy;
more preferably, R² is selected from the group consisting of: hydrogen, halogen, amino, pyrrolyl, indolyl, and phenyl; the pyrrolyl, the indolyl, and the phenyl are substituted by the following substituents for one or more times, the substituent being: fluorine, chlorine and methoxy;
R³ is selected from: 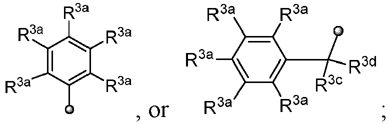
R^{3a} is selected from the group consisting of: hydrogen, halogen, nitro, cyano, hydroxyl, C1-C6 hydroxyalkyl, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
preferably, R^{3a} is selected from the group consisting of: hydrogen, F, Cl, nitro, cyano, hydroxyl, C1-C6 hydroxyalkyl, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
more preferably, R^{3a} is selected from the group consisting of: hydrogen, F, Cl, hydroxyl, C1-C4 hydroxyalkyl, methyl, ethyl, propyl, isopropyl, isobutyl, tert-butyl, and cyclohexyl.

In a more preferred embodiment, the present invention relates to the compound having the above general formula (I), or the enantiomer, the diastereomer, the tautomer, the salt, the crystalline form, the solvate and/or the isotopically substituted derivative thereof, where: the acyl is selected from acetyl, and the sulfonyl is selected from methylsulfonyl.

In a more preferred embodiment, the present invention relates to the compound having the above general formula (I), or an enantiomer, a diastereomer, a tautomer, a salt, a crystalline form, a solvate and/or an isotopically substituted derivative thereof, where:
the aryl-(CH₂)_{q}- is selected from the group consisting of: aryl-(CH₂)₃-, aryl-(CH₂)₂-, aryl-CH₂-, and aryl-;
the heterocyclyl-(CH₂)_{q}- is selected from the group consisting of: heterocyclyl-(CH₂)₃-, heterocyclyl-(CH₂)₂-, heterocyclyl-CH₂-, and heterocyclyl-;
the heteroaryl-(CH₂)_{q}- is selected from the group consisting of: heteroaryl-(CH₂)₃-, heteroaryl-(CH₂)₂-, heteroaryl-CH₂-, and heteroaryl-.

In a more preferred embodiment, the present invention relates to the compound having the above general formula (I), or an enantiomer, a diastereomer, a tautomer, a salt, a crystalline form, a solvate and/or an isotopically substituted derivative thereof, where:
the halogenation is selected from: fluoro and chloro; or
the halogen is selected from the group consisting of: fluorine, chlorine, bromine, and iodine; preferably, the halogen is selected from the group consisting of: fluorine and chlorine.

In a more preferred embodiment, the present invention relates to the compound having the above general formula (I), or an enantiomer, a diastereomer, a tautomer, a salt, a crystalline form, a solvate and/or an isotopically substituted derivative thereof, where:
R^{3a} is selected from: -(CH₂)_{q}-NR¹⁶-S(=O)₂-R¹⁷, the R¹⁶ is preferably selected from: hydrogen.

In a more preferred embodiment, the present invention relates to the compound having the above general formula (I), or an enantiomer, a diastereomer, a tautomer, a salt, a crystalline form, a solvate and/or an isotopically substituted derivative thereof, where:
R^{3a} is selected from: -(CH₂)_{q}-S-(CH₂)ₚ-R¹¹, the R¹¹ is preferably selected from: phenyl.

In a more preferred embodiment, the present invention relates to the compound having the above general formula (I), or an enantiomer, a diastereomer, a tautomer, a salt, a crystalline form, a solvate and/or an isotopically substituted derivative thereof, where:
C1-C4 straight chain alkyl, C3-C4 branched chain alkyl, and C3-C4 cycloalkyl are each selected from the group consisting of: methyl, ethyl, propyl, butyl, cyclopropyl, cyclobutyl, isopropyl, isobutyl, and tert-butyl; or
C1-C4 alkyl is selected from the group consisting of: methyl, ethyl, propyl, butyl, cyclopropyl, cyclobutyl, isopropyl, isobutyl, and tert-butyl; or
C1-C3 alkyl is selected from the group consisting of: methyl, ethyl, propyl, isopropyl, and cyclopropyl.

In each of the above embodiments, the present invention relates to the compound having the above general formula (I), or an enantiomer, a diastereomer, a tautomer, a salt, a crystalline form, a solvate and/or an isotopically substituted derivative thereof, where:
p and q are each independently 0, 1, 2 or 3;
m and n are the same or different, and are each independently selected from: 1, 2 or 3, so as to form a ring system with various numbers of atoms.

In a preferred embodiment, the present invention relates to the compound having the above general formula (I), or an enantiomer, a diastereomer, a tautomer, a salt, a crystalline form, a solvate and/or an isotopically substituted derivative thereof, where:
L is selected from the group consisting of:
   1,3-substituted-bicyclo[1.1.1]pentanyl, 2,6-substituted-diazecyclo[3.3]heptanyl, oxy-azetidinyl, 3-amino-azetidin-1-yl, 3-amino(benzyl)-azetidin-1-yl, amino acetylenyl, 1,2-substituted cyclopropyl, 3-alkenylazetidin-1-yl, 3-thio-azetidin-1-yl, 3-sulfinylazetidin-1-yl, 3-sulfonylazetidin-1-yl, 1H-1,2,3-triazol-4-yl;
where, the oxy-azetidinyl is selected from: 3-oxy-azetidin-1-yl or azetidinyl-3-oxyl;
the amino acetylenyl is selected from the group consisting of:
   aminoethynyl, amino(alkyl)ethynyl, amino(aryl-CH₂-)ethynyl, amino(heterocyclyl-CH₂-)ethynyl, amino(heteroaryl-CH₂-)ethynyl, and amino(sulfonyl)ethynyl;
the alkyl is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, the alkyl is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
the aryl aryl, the heterocyclyl, and the heteroaryl are optionally substituted by a substituent R¹⁸ for one or more times identically or differently;
the aryl is selected from the group consisting of: phenyl and naphthyl, preferably phenyl;
the heterocyclyl is selected from: 4-7 membered saturated heterocyclyl containing oxygen or nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from: methylsulfonyl, optionally substituted phenylsulfonyl;
the acyl is selected from: acetyl, optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl and F;
more preferably, the amino(alkyl)ethynyl is selected from: amino(methyl)ethynyl;
the amino(aryl-CH₂-)ethynyl is selected from the group consisting of: amino(benzyl)ethynyl, amino(4-fluorobenzyl)ethynyl, amino(4-methylbenzyl)ethynyl, amino(4-trifluoromethylbenzyl)ethynyl, amino(methoxy benzyl)ethynyl, amino(2-fluorobenzyl)ethynyl, and amino(4-(hydroxymethyl)benzyl)ethynyl;
the amino(heterocyclyl-CH₂-)ethynyl is selected from: amino((1-methylpiperidin-4-yl)methyl)ethynyl;
the amino(heteroaryl-CH₂-)ethynyl is selected from the group consisting of: amino(pyridin-3-ylmethyl)ethynyl, amino(furan-2-ylmethyl)ethynyl, amino(1-methyl-1H-indol-5-yl)methylethynyl, and amino(thiophene-2-ylmethyl)ethynyl;
the amino(sulfonyl)ethynyl is selected from the group consisting of: amino(methylsulfonyl)ethynyl, amino(cyclopropylsulfonyl)ethynyl, amino(phenylsulfonyl)ethynyl, amino((4-methylphenyl)sulfonyl)ethynyl, amino(benzo[b]thiophen-3-sulfonyl)ethynyl, amino(thiophene-2-sulfonyl)ethynyl, and amino(benzofuran-5-sulfonyl)ethynyl.

In a preferred embodiment, the present invention relates to the compound having the above general formula (I), or the enantiomer, the diastereomer, the tautomer, the salt, the crystalline form, the solvate and/or the isotopically substituted derivative thereof, where
R³ is selected from the group consisting of: 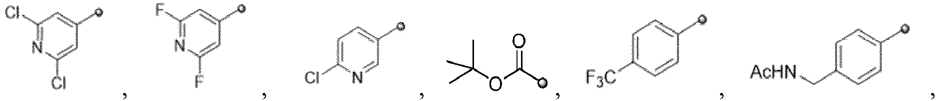 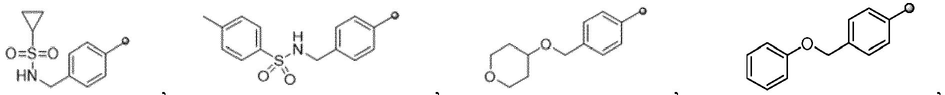 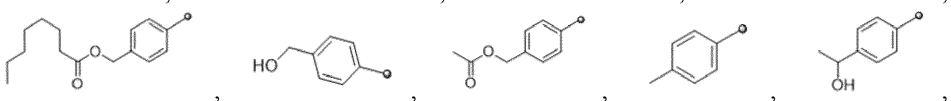 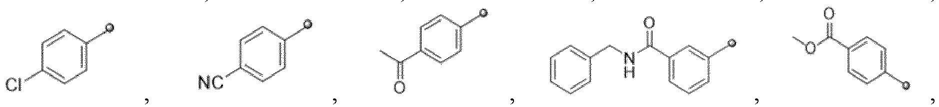 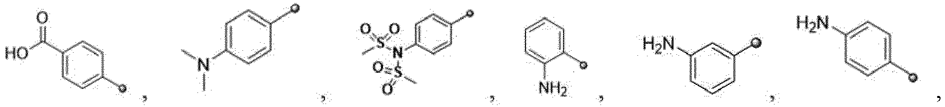 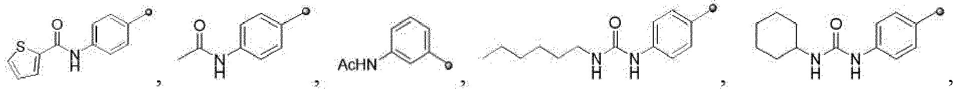 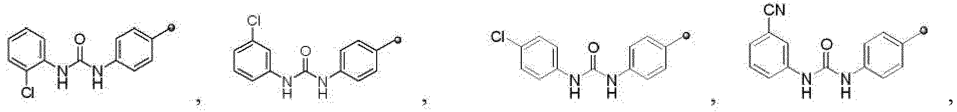 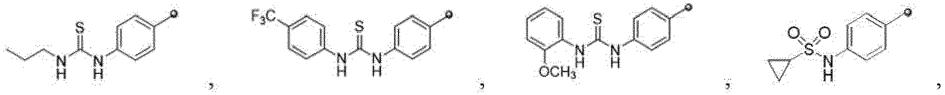 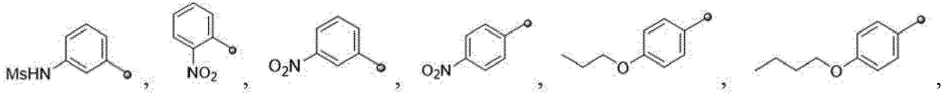 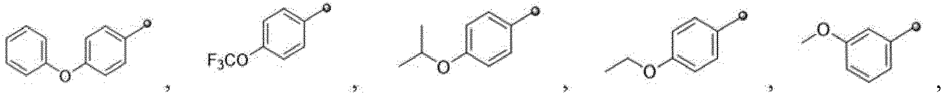 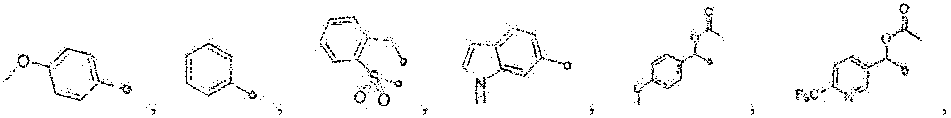 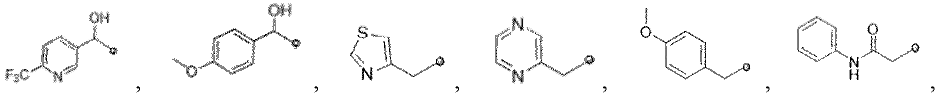 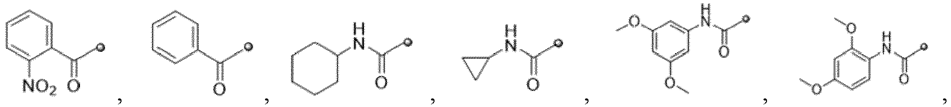 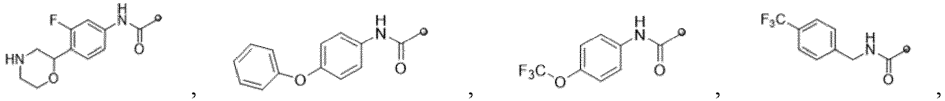 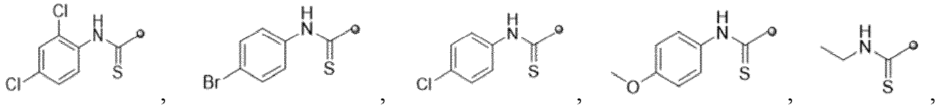 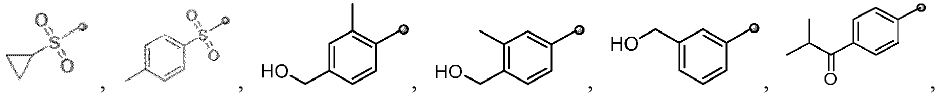 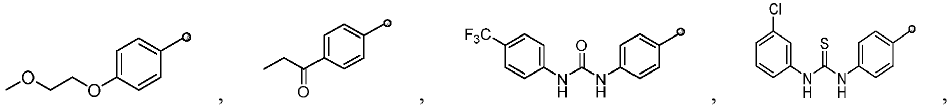 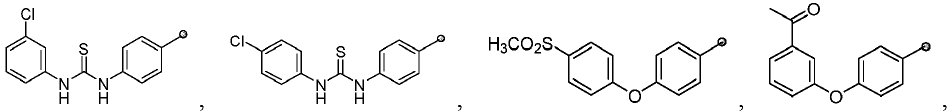 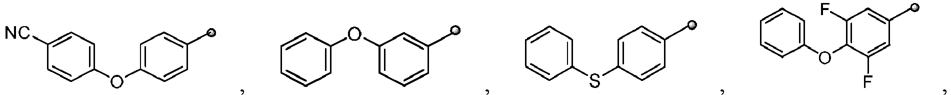 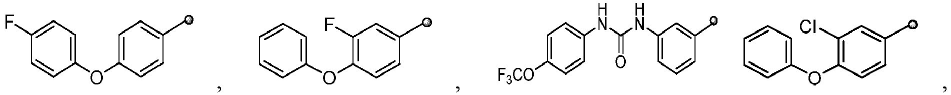 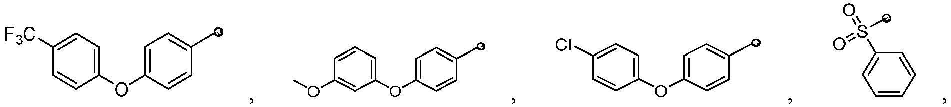 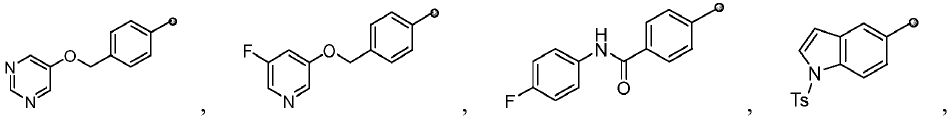 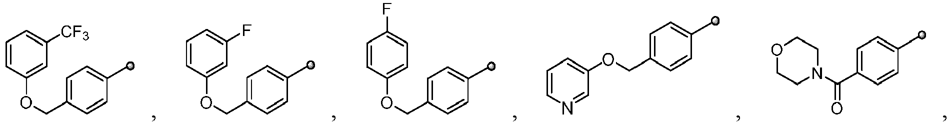 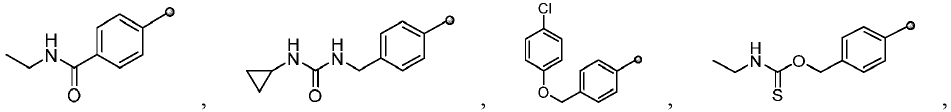 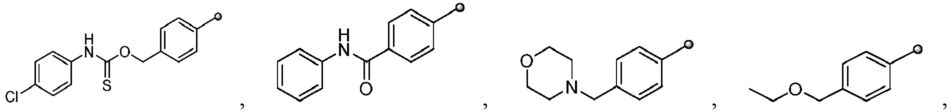 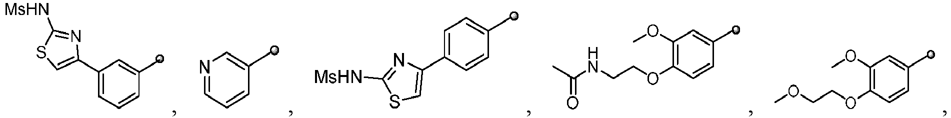 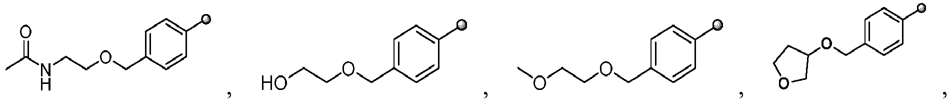 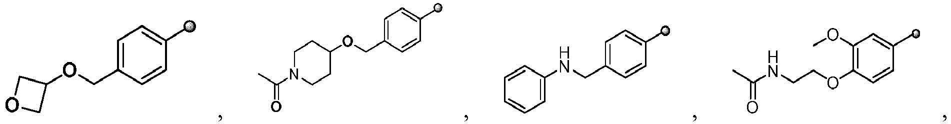 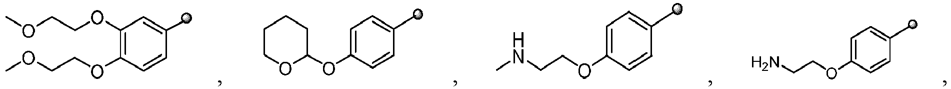 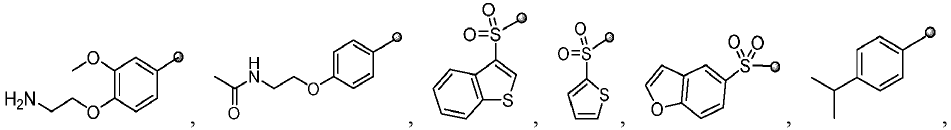 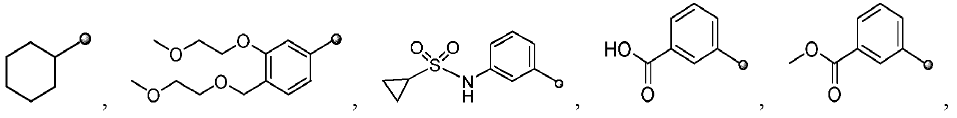 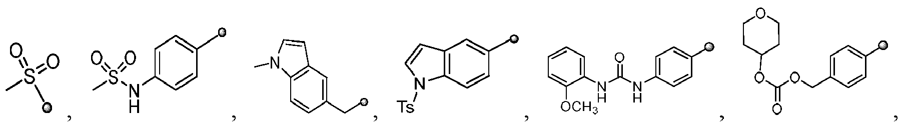 and

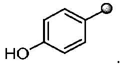

In a preferred embodiment, the present invention relates to the compound having the above general formula (I), or the enantiomer, the diastereomer, the tautomer, the salt, the crystalline form, the solvate and/or the isotopically substituted derivative, where the compound is selected from the group consisting of: 3-(3-(acetoxyl(4-methoxyphenyl)methyl)bicyclo[1.1.1]pentan-1-yl)benzoic acid, 3-(3-(acetoxyl(6-(trifluoromethyl)pyridin-3-yl)methyl)bicyclo[1.1.1]pentan-1-yl)benzoic acid, 3-(3-(hydroxyl(6-(trifluoromethyl)pyridin-3-yl)methyl)bicyclo[1.1.1]pentan-1-yl)benzoic acid, 3-(3-phenylbicyclo[1.1.1]pentan-1-yl)benzoic acid, Methyl 3-(3-phenylbicyclo[1.1.1]pentan-1-yl)-2-(1H-pyrrol-1-yl)benzoate, 3-(3-phenylbicyclo[1.1.1]pentan-1-yl)-2-(1H-pyrrol-1-yl)benzoic acid, Methyl 3-(3-(4-methoxyphenyl)bicyclo[1.1.1]pentan-1-yl)-2-(1H-pyrrol-1-yl)benzoate, 3-(3-(4-methoxyphenyl)bicyclo[1.1.1]pentan-1-yl)-2-(1H-pyrrol-1-yl)benzoic acid, Methyl 2-amino-3-(6-phenyl-2,6-diazaspiro[3.3]heptan-2-yl)benzoate, 2-amino-3-(6-phenyl-2,6-diazaspiro[3.3]heptan-2-yl)benzoic acid, 3-(6-phenyl-2,6-diazaspiro[3.3]heptan-2-yl)-2-(1H-pyrrol-1-yl)benzoic acid, 2-amino-3-(6-(2-aminophenyl)-2,6-diazaspiro[3.3]heptan-2-yl)benzoic acid, Methyl 2-amino-3-(6-(4-aminophenyl)-2,6-diazaspiro[3.3]heptan-2-yl)benzoate, 2-amino-3-(6-(4-aminophenyl)-2,6-diazaspiro[3.3]heptan-2-yl)benzoic acid, Methyl 2-amino-3-(6-(3-(methoxycarbonyl)phenyl)-2,6-diazaspiro[3.3]heptan-2-yl)benzoate, 2-amino-3-(6-(3-carboxylphenyl)-2,6-diazaspiro[3.3]heptan-2-yl)benzoic acid, Methyl 3-(6-(3-(methoxycarbonyl)phenyl)-2,6-diazaspiro[3.3]heptan-2-yl)-2-(1H-pyrrol-1-yl)benzoat e, 3-(6-(3-carboxylphenyl)-2,6-diazaspiro[3.3]heptan-2-yl)-2-(1H-pyrrol-1-yl)benzoic acid, Methyl 3-(6-(2-aminophenyl)-2,6-diazaspiro[3.3]heptan-2-yl)-2-(1H-pyrrol-1-yl)benzoate, 3-(6-(2-aminophenyl)-2,6-diazaspiro[3.3]heptan-2-yl)-2-(1H-pyrrol-1-yl)benzoic acid, Methyl 3-(6-(2-nitrophenyl)-2,6-diazaspiro[3.3]heptan-2-yl)-2-(1H-pyrrol-1-yl)benzoate, 3-(6-(2-nitrophenyl)-2,6-diazaspiro[3.3]heptan-2-yl)-2-(1H-pyrrol-1-yl)benzoic acid, Methyl 3-(6-(4-(methoxycarbonyl)phenyl)-2,6-diazaspiro[3.3]heptan-2-yl)-2-(1H-pyrrol-1-yl)benzoat e, 3-(6-(4-carboxylphenyl)-2,6-diazaspiro[3.3]heptan-2-yl)-2-(1H-pyrrol-1-yl)benzoic acid, Methyl 3-(6-(3-nitrophenyl)-2,6-diazaspiro[3.3]heptan-2-yl)-2-nitrobenzoate, 3-(6-(3-nitrophenyl)-2,6-diazaspiro[3.3]heptan-2-yl)-2-nitrobenzoic acid, Methyl 3-(6-(3-(methylsulfonamido)phenyl)-2,6-diazaspiro[3.3]heptan-2-yl)-2-nitrobenzoate, 3-(6-(3-(methylsulfonamido)phenyl)-2,6-diazaspiro[3.3]heptan-2-yl)-2-nitrobenzoic acid, Methyl 3-(6-(3-acetylaminophenyl)-2,6-diazaspiro[3.3]heptan-2-yl)-2-aminobenzoate, 3-(6-(3-acetylaminophenyl)-2,6-diazaspiro[3.3]heptan-2-yl)-2-aminobenzoic acid, Methyl 2-amino-3-(6-(3-(cyclopropylsulfonamido)phenyl)-2,6-diazaspiro[3.3]heptan-2-yl)benzoate, 2-amino-3-(6-(3-(cyclopropylsulfonamido)phenyl)-2,6-diazaspiro[3.3]heptan-2-yl)benzoic acid, Methyl 2-amino-3-(6-(3-aminophenyl)-2,6-diazaspiro[3.3]heptan-2-yl)benzoate, 2-amino-3-(6-(3-aminophenyl)-2,6-diazaspiro[3.3]heptan-2-yl)benzoic acid, 3-(3-(4-(hydroxymethyl)phenoxy)azetidin-1-yl)benzoic acid, 2-amino-3-(3-(4-(hydroxymethyl)phenoxy)azetidin-1-yl)benzoic acid, Methyl 3-(3-(4-(hydroxymethyl)phenoxy)azetidin-1-yl)-2-(1H-pyrrol-1-yl)benzoate, 3-(3-(4-(hydroxymethyl)phenoxy)azetidin-1-yl)-2-(1H-pyrrol-1-yl)benzoic acid, Methyl 3-(3-(4-(hydroxymethyl)phenoxy)azetidin-1-yl)-2-(1H-indol-6-yl)benzoate, 3-(3-(4-(hydroxymethyl)phenoxy)azetidin-1-yl)-2-(1H-indol-6-yl)benzoic acid, Methyl 6-(3-(4-(hydroxymethyl)phenoxy)azetidin-1-yl)-[1,1'-biphenyl]-2-formate, 6-(3-(4-(hydroxymethyl)phenoxy)azetidin-1-yl)-[1,1'-biphenyl]-2-formic acid, Methyl 2-(benzo[b]thiophen-6-yl)-3-(3-(4-(hydroxymethyl)phenoxy)azetidin-1-yl)benzoate, 2-(benzo[b]thiophen-6-yl)-3-(3-(4-(hydroxymethyl)phenoxy)azetidin-1-yl)benzoic acid, Methyl 2-(benzo[c][1,2,5]thiadiazol-5-yl)-3-(3-(4-(hydroxymethyl)phenoxy)azetidin-1-yl)benzoate, 2-(benzo[c][1,2,5]thiadiazol-5-yl)-3-(3-(4-hydroxymethyl)phenoxy)azetidin-1-yl)benzoic acid, Methyl 2-(furan-2-yl)-3-(3-(4-hydroxymethyl)phenoxy)azetidin-1-yl)benzoate, Methyl 2-(furan-3-yl)-3-(3-(4-hydroxymethyl)phenoxy)azetidin-1-yl)benzoate, 2-(furan-3-yl)-3-(3-(4-hydroxymethyl)phenoxy)azetidin-1-yl)benzoic acid, Methyl 3'-fluoro-6-(3-(4-hydroxymethyl)phenoxy)azetidin-1-yl)-[1,1'-biphenyl]-2-formate, 3'-fluoro-6-(3-(4-hydroxymethyl)phenoxy)azetidin-1-yl)-[1,1'-biphenyl]-2-formic acid, 2-(benzofuran-6-yl)-3-(3-(4-hydroxymethyl)phenoxy)azetidin-1-yl)benzoic acid, Methyl 3'-chloro-6-(3-(4-(hydroxymethyl)phenoxy)azetidin-1-yl)-[1,1'-biphenyl]-2-formate, 3'-chloro-6-(3-(4-hydroxymethyl)phenoxy)azetidin-1-yl)-[1,1'-biphenyl]-2-formic acid, Methyl 3'-cyano-6-(3-(4-hydroxymethyl)phenoxy)azetidin-1-yl)-[1,1'-biphenyl]-2-formate, 6-(3-(4-hydroxymethyl)phenoxy)azetidin-1-yl)-[1,1'-biphenyl]-2,3'-di-formic acid, Methyl 4'-chloro-6-(3-(4-hydroxymethyl)phenoxy)azetidin-1-yl)-[1,1'-biphenyl]-2-formate, 4'-chloro-6-(3-(4-(hydroxymethyl)phenoxy)azetidin-1-yl)-[1,1'-biphenyl]-2-formic acid, Methyl 3'-(trifluoromethyl)-6-(3-(4-(hydroxymethyl)phenoxy)azetidin-1-yl)-[1,1'-biphenyl]-2-format e, 3'-(trifluoromethyl)-6-(3-(4-(hydroxymethyl)phenoxy)azetidin-1-yl)-[1,1'-biphenyl]-2-formic acid, Methyl 3',5'-difluoro-6-(3-(4-(hydroxymethyl)phenoxy)azetidin-1-yl)-[1,1'-biphenyl]-2-formate, 3',5'-difluoro-6-(3-(4-(hydroxymethyl)phenoxy)azetidin-1-yl)-[1,1'-biphenyl]-2-formic acid, Methyl 6-(3-(4-(hydroxymethyl)phenoxy)azetidin-1-yl)-[1, 1' :4', 1"-terphenyl]-2-formate, 6-(3 -(4-(hydroxymethyl)phenoxy)azetidin-1-yl)-[1, 1':4', 1"-terphenyl]-2-formic acid, Methyl 3 '-(methylsulfonamido)-6-(3 -(4-(hydroxymethyl)phenoxy)azetidin-1-yl)-[1,1'-biphenyl]-2-for mate, Methyl 3'-methoxy-6-(3-(4-(hydroxymethyl)phenoxy)azetidin-1-yl)-[1,1'-biphenyl]-2-formate, 3'-methoxy-6-(3-(4-(hydroxymethyl)phenoxy)azetidin-1-yl)-[1,1'-biphenyl]-2-formic acid, Methyl 3'-cyano-4'-fluoro-6-(3-(4-(hydroxymethyl)phenoxy)azetidin-1-yl)-[1,1'-biphenyl]-2-formate, Methyl 3-(3-(4-(hydroxymethyl)phenoxy)azetidin-1-yl)-2-(1H-pyrazol-1-yl)benzoate, 3-(3-(4-(hydroxymethyl)phenoxy)azetidin-1-yl)-2-(1H-pyrazol-1-yl)benzoic acid, 3-((1-(3-(benzylcarbamoyl)phenyl)azetidin-3-yl)oxy)benzoic acid, 3-(3-(3-carboxylphenoxy)azetidin-1-yl)-2-aminobenzoic acid, 3-(3-(3-carboxylphenoxy)azetidin-1-yl)-2-(1H-pyrrol-1-yl)benzoic acid, Methyl 3-(3-(4-(acetoxylmethyl)phenoxy)azetidin-1-yl)-2-(1H-pyrrol-1-yl)benzoate, Methyl 3-(3-(4-((octanoyloxy)methyl)phenoxy)azetidin-1-yl)-2-(1H-pyrrol-1-yl)benzoate, Methyl 3-(3-(4-(acetylaminomethyl)phenoxy)azetidin-1-yl)-2-(1H-pyrrol-1-yl)benzoate, 3-(3-(4-(acetylaminomethyl)phenoxy)azetidin-1-yl)-2-(1H-pyrrol-1-yl)benzoic acid, Methyl 3-(3-(4-(cyclopropylsulfonamidomethyl)phenoxy)azetidin-1-yl)-2-(1H-pyrrol-1-yl)benzoate, Methyl 3 -(3 -(3 -methoxyphenoxy)azeti din-1-yl)-2-(1H-pyrrol-1-yl)benzoate, 3-(3-(3-methoxyphenoxy)azetidin-1-yl)-2-(1H-pyrrol-1-yl)benzoic acid, Methyl 2-(1H-pyrrol-1-yl)-3-(3-(4-(trifluoromethyl)phenoxy)azetidin-1-yl)benzoate, 2-(1H-pyrrol-1-yl)-3-(3-(4-(trifluoromethyl)phenoxy)azetidin-1-yl)benzoic acid, Methyl 2-(1H-pyrrol-1-yl)-3-(3-(4-(trifluoromethoxy)phenoxy)azetidin-1-yl)benzoate, 2-(1H-pyrrol-1-yl)-3-(3-(4-(trifluoromethoxy)phenoxy)azetidin-1-yl)benzoic acid, Methyl 3-(3-((6-chloropyridin-3-yl)oxy)azetidin-1-yl)-2-(1H-pyrrol-1-yl)benzoate, 3-(3-((6-chloropyridin-3-yl)oxy)azetidin-1-yl)-2-(1H-pyrrol-1-yl)benzoic acid, Methyl 3-(3-(4-acetylphenoxy)azetidin-1-yl)-2-(1H-pyrrol-1-yl)benzoate, 3-(3-(4-acetylphenoxy)azetidin-1-yl)-2-(1H-pyrrol-1-yl)benzoic acid, Methyl 3-(3-(4-(1-hydroxyethyl)phenoxy)azetidin-1-yl)-2-(1H-pyrrol-1-yl)benzoate, 3-(3-(4-(1-hydroxyethyl)phenoxy)azetidin-1-yl)-2-(1H-pyrrol-1-yl)benzoic acid, Methyl 3-(3-((2,6-dichloropyridin-4-yl)oxy)azetidin-1-yl)-2-(1H-pyrrol-1-yl)benzoate, 3-(3-((2,6-dichloropyridin-4-yl)oxy)azetidin-1-yl)-2-(1H-pyrrol-1-yl)benzoic acid, Methyl 3-(3-(4-cyanophenoxy)azetidin-1-yl)-2-(1H-pyrrol-1-yl)benzoate, 3-(3-(4-cyanophenoxy)azetidin-1-yl)-2-(1H-pyrrol-1-yl)benzoic acid, Methyl 3-(3-(4-chlorophenoxy)azetidin-1-yl)-2-(1H-pyrrol-1-yl)benzoate, 3-(3-(4-chlorophenoxy)azetidin-1-yl)-2-(1H-pyrrol-1-yl)benzoic acid, Methyl 3-(3-(4-(dimethylamino)phenoxy)azetidin-1-yl)-2-(1H-pyrrol-1-yl)benzoate, 3-(3-(4-(dimethylamino)phenoxy)azetidin-1-yl)-2-(1H-pyrrol-1-yl)benzoic acid, Methyl 3-(3-(4-ethoxyphenoxy)azetidin-1-yl)-2-(1H-pyrrol-1-yl)benzoate, 3-(3-(4-ethoxyphenoxy)azetidin-1-yl)-2-(1H-pyrrol-1-yl)benzoic acid, Methyl 3-(3-phenoxyazetidin-1-yl)-2-(1H-pyrrol-1-yl)benzoate, 3-(3-phenoxyazetidin-1-yl)-2-(1H-pyrrol-1-yl)benzoic acid, Methyl 3-(3-((1H-indol-6-yl)oxy)azetidin-1-yl)-2-(1H-pyrrol-1-yl)benzoate, 3-(3-((1H-indol-6-yl)oxy)azetidin-1-yl)-2-(1H-pyrrol-1-yl)benzoic acid, Methyl 3-(3-(4-propoxyphenoxy)azetidin-1-yl)-2-(1H-pyrrol-1-yl)benzoate, 3-(3-(4-propoxyphenoxy)azetidin-1-yl)-2-(1H-pyrrol-1-yl)benzoic acid, Methyl 3-(3-(4-butoxyphenoxy)azetidin-1-yl)-2-(1H-pyrrol-1-yl)benzoate, 3-(3-(4-butoxyphenoxy)azetidin-1-yl)-2-(1H-pyrrol-1-yl)benzoic acid, Methyl 3-(3-(4-isopropoxyphenoxy)azetidin-1-yl)-2-(1H-pyrrol-1-yl)benzoate, 3-(3-(4-isopropoxyphenoxy)azetidin-1-yl)-2-(1H-pyrrol-1-yl)benzoic acid, Methyl 3-(3-(4-phenoxyphenoxy)azetidin-1-yl)-2-(1H-pyrrol-1-yl)benzoate, 3-(3-(4-phenoxyphenoxy)azetidin-1-yl)-2-(1H-pyrrol-1-yl)benzoic acid, Methyl 2-(1H-pyrrol-1-yl)-3-(3-(4-(((tetrahydro-2H-pyran-4-yl)oxy)methyl)phenoxy)azetidin-1-yl)be nzoate, Methyl 3-(3-(4-(phenoxymethyl)phenoxy)azetidin-1-yl)-2-(1H-pyrrol-1-yl)benzoate, 3-(3-(4-(phenoxymethyl)phenoxy)azetidin-1-yl)-2-(1H-pyrrol-1-yl)benzoic acid, 3-(3-(4-nitrophenoxy)azetidin-1-yl)-2-(1H-pyrrol-1-yl)benzoic acid, Methyl 3-(3-(4-(3-propylthioureido)phenoxy)azetidin-1-yl)-2-(1H-pyrrol-1-yl)benzoate, Methyl 2-(1H-pyrrol-1-yl)-3-(3-(4-(3-(4-(trifluoromethyl)phenyl)thioureido)phenoxy)azetidin-1-yl)be nzoate, Methyl 3-(3-(4-(3-(2-methoxyphenyl)thioureido)phenoxy)azetidin-1-yl)-2-(1H-pyrrol-1-yl)benzoate, Methyl 3-(3-(4-acetylaminophenoxy)azetidin-1-yl)-2-(1H-pyrrol-1-yl)benzoate, 3-(3-(4-acetylaminophenoxy)azetidin-1-yl)-2-(1H-pyrrol-1-yl)benzoic acid, Methyl 2-(1H-pyrrol-1-yl)-3-(3-(4-(thiophene-2-formamido)phenoxy)azetidin-1-yl)benzoate, 2-(1H-pyrrol-1-yl)-3-(3-(4-(thiophene-2-formamido)phenoxy)azetidin-1-yl)benzoic acid, Methyl 3-(3-(4-(N-(mesyl)methylsulfonamido)phenoxy)azetidin-1-yl)-2-(1H-pyrrol-1-yl)benzoate, 3-(3-(4-(methylsulfonamido)phenoxy)azetidin-1-yl)-2-(1H-pyrrol-1-yl)benzoic acid, Methyl 3-(3-(4-(cyclopropylsulfonamido)phenoxy)azetidin-1-yl)-2-(1H-pyrrol-1-yl)benzoate, 3-(3-(4-(cyclopropylsulfonamido)phenoxy)azetidin-1-yl)-2-(1H-pyrrol-1-yl)benzoic acid, Methyl 3-(3-(4-(3-(4-chlorophenyl)ureido)phenoxy)azetidin-1-yl)-2-(1H-pyrrol-1-yl)benzoate, 3-(3-(4-(3-(4-chlorophenyl)ureido)phenoxy)azetidin-1-yl)-2-(1H-pyrrol-1-yl)benzoic acid, Methyl 3-(3-(4-(3-cyclohexylureido)phenoxy)azetidin-1-yl)-2-(1H-pyrrol-1-yl)benzoate, 3-(3-(4-(3-cyclohexylureido)phenoxy)azetidin-1-yl)-2-(1H-pyrrol-1-yl)benzoic acid, Methyl 3-(3-(4-(3-hexylureido)phenoxy)azetidin-1-yl)-2-(1H-pyrrol-1-yl)benzoate, Methyl 3-(3-(4-(3-(3-chlorophenyl)ureido)phenoxy)azetidin-1-yl)-2-(1H-pyrrol-1-yl)benzoate, 3-(3-(4-(3-(3-chlorophenyl)ureido)phenoxy)azetidin-1-yl)-2-(1H-pyrrol-1-yl)benzoic acid, Methyl 3-(3-(4-(3-(2-chlorophenyl)ureido)phenoxy)azetidin-1-yl)-2-(1H-pyrrol-1-yl)benzoate, 3-(3-(4-(3-(2-chlorophenyl)ureido)phenoxy)azetidin-1-yl)-2-(1H-pyrrol-1-yl)benzoicacid, Methyl 3-(3-(4-(3-(3-cyanophenyl)ureido)phenoxy)azetidin-1-yl)-2-(1H-pyrrol-1-yl)benzoate, 3-(3-(4-(3-(3-cyanophenyl)ureido)phenoxy)azetidin-1-yl)-2-(1H-pyrrol-1-yl)benzoic acid, Methyl 3-(3-((4-methoxybenzyl)oxy)azetidin-1-yl)-2-(1H-pyrrol-1-yl)benzoate, Methyl 2-(1H-pyrrol-1-yl)-3-(3-(thiazol-4-yl)methoxy)azetidin-1-yl)benzoate, 2-(1H-pyrrol-1-yl)-3-(3-(thiazol-4-yl)methoxy)azetidin-1-yl)benzoic acid, Methyl 3-(3-(pyrazin-2-yl)methoxy)azetidin-1-yl)-2-(1H-pyrrol-1-yl)benzoate, 3-(3-(pyrazin-2-yl)methoxy)azetidin-1-yl)-2-(1H-pyrrol-1-yl)benzoic acid, Methyl 3-(3-(2-oxo-2-(phenylamino)ethoxy)azetidin-1-yl)-2-(1H-pyrrol-1-yl)benzoate, 3-(3-(2-oxo-2-(phenylamino)ethoxy)azetidin-1-yl)-2-(1H-pyrrol-1-yl)benzoic acid, Methyl 3-(3-(((2,4-dimethoxyphenyl)carbamoyl)oxy)azetidin-1-yl)-2-(1H-pyrrol-1-yl)benzoate, Methyl 3-(3-(((4-chlorophenyl)thiocarbamoyl)oxy)azetidin-1-yl)-2-(1H-pyrrol-1-yl)benzoate, 3-(3-(((4-chlorophenyl)thiocarbamoyl)oxy)azetidin-1-yl)-2-(1H-pyrrol-1-yl)benzoic acid, Methyl 3-(3-((cyclohexylcarbamoyl)oxy)azetidin-1-yl)-2-(1H-pyrrol-1-yl)benzoate, 3-(3-((cyclohexylcarbamoyl)oxy)azetidin-1-yl)-2-(1H-pyrrol-1-yl)benzoic acid, Methyl 3-(3-(((3-fluoro-4-(morpholin-2-yl)phenyl)carbamoyl)oxy)azetidin-1-yl)-2-(1H-pyrrol-1-yl)b enzoate, Methyl 3-(3-(((4-phenoxyphenyl)carbamoyl)oxy)azetidin-1-yl)-2-(1H-pyrrol-1-yl)benzoate, Methyl 2-(1H-pyrrol-1-yl)-3-(3-(((4-(trifluoromethoxy)phenyl)carbamoyl)oxy)azetidin-1-yl)benzoate, Methyl 2-(1H-pyrrol-1-yl)-3-(3-(((4-(trifluoromethyl)benzyl)carbamoyl)oxy)azetidin-1-yl)benzoate, Methyl 3-(3-((cyclopropylcarbamoyl)oxy)azetidin-1-yl)-2-(1H-pyrrol-1-yl)benzoate, Methyl 3-(3-(((4-bromophenyl)thiocarbamoyl)oxy)azetidin-1-yl)-2-(1H-pyrrol-1-yl)benzoate, 3-(3-(((4-bromophenyl)thiocarbamoyl)oxy)azetidin-1-yl)-2-(1H-pyrrol-1-yl)benzoic acid, Methyl 3-(3-(((4-methoxyphenyl)thiocarbamoyl)oxy)azetidin-1-yl)-2-(1H-pyrrol-1-yl)benzoate, 3-(3-(((4-methoxyphenyl)thiocarbamoyl)oxy)azetidin-1-yl)-2-(1H-pyrrol-1-yl)benzoic acid, Methyl 3-(3-((ethylthiocarbamoyl)oxy)azetidin-1-yl)-2-(1H-pyrrol-1-yl)benzoate, 3-(3-((ethylthiocarbamoyl)oxy)azetidin-1-yl)-2-(1H-pyrrol-1-yl)benzoic acid, Methyl 3-(3-(((2,4-dichlorophenyl)thiocarbamoyl)oxy)azetidin-1-yl)-2-(1H-pyrrol-1-yl)benzoate, 3-(3-(((2,4-dichlorophenyl)thiocarbamoyl)oxy)azetidin-1-yl)-2-(1H-pyrrol-1-yl)benzoic acid, Methyl 3-(3-(3-cyclopropylureido)azetidin-1-yl)-2-(1H-pyrrol-1-yl)benzoate, Methyl 3-(3-(3-(4-phenoxyphenyl)ureido)azetidin-1-yl)-2-(1H-pyrrol-1-yl)benzoate, Methyl 2-(1H-pyrrol-1-yl)-3-(3-(3-(4-(trifluoromethoxy)phenyl)ureido)azetidin-1-yl)benzoate, 3-(3-(3-(4-phenoxyphenyl)ureido)azetidin-1-yl)-2-(1H-pyrrol-1-yl)benzoic acid, 2-(1H-pyrrol-1-yl)-3-(3-(3-(4-(trifluoromethoxy)phenyl)ureido)azetidin-1-yl)benzoic acid, 3-(3-(3-cyclopropylureido)azetidin-1-yl)-2-(1H-pyrrol-1-yl)benzoic acid, Methyl 3 -(3 -(3 -(3,5-dimethoxyphenyl)ureido)azetidin-1-yl)-2-(1H-pyrrol-1-yl)benzoate, 3-(3-(3-(3,5-dimethoxyphenyl)ureido)azetidin-1-yl)-2-(1H-pyrrol-1-yl)benzoic acid, Methyl 3-(3-(3-ethylthioureido)azetidin-1-yl)-2-(1H-pyrrol-1-yl)benzoate, 3-(3-(3-ethylthioureido)azetidin-1-yl)-2-(1H-pyrrol-1-yl)benzoic acid, Methyl 3-(3-(3-(4-chlorophenyl)thioureido)azetidin-1-yl)-2-(1H-pyrrol-1-yl)benzoate, 3-(3-(3-(4-chlorophenyl)thioureido)azetidin-1-yl)-2-(1H-pyrrol-1-yl)benzoic acid, Methyl 3-(3-((4-methylphenyl)sulfonamido)azetidin-1-yl)-2-(1H-pyrrol-1-yl)benzoate, 3-(3-((4-methylphenyl)sulfonamido)azetidin-1-yl)-2-(1H-pyrrol-1-yl)benzoic acid, Methyl 3-(3-benzamidoazetidin-1-yl)-2-(1H-pyrrol-1-yl)benzoate, 3-(3-benzamidoazetidin-1-yl)-2-(1H-pyrrol-1-yl)benzoic acid, Methyl 3-(3-(2-nitrobenzamido)azetidin-1-yl)-2-(1H-pyrrol-1-yl)benzoate, 3-(3-(2-nitrobenzamido)azetidin-1-yl)-2-(1H-pyrrol-1-yl)benzoic acid, Methyl 3-(3-(cyclopropylsulfonamido)azetidin-1-yl)-2-(1H-pyrrol-1-yl)benzoate, 3-(3-(cyclopropylsulfonamido)azetidin-1-yl)-2-(1H-pyrrol-1-yl)benzoic acid, Methyl 3-(3-((N-benzyl-4-methylphenyl)sulfonamido)azetidin-1-yl)-2-(1H-pyrrol-1-yl)benzoate, 3-(3-((N-benzyl-4-methylphenyl)sulfonamido)azetidin-1-yl)-2-(1H-pyrrol-1-yl)benzoic acid, 3-(1-(4-(hydroxymethyl)phenyl)-1H-1,2,3-triazol-4-yl)-2-(1H-indol-6-yl)benzoic acid, Methyl 3-(3-(4-hydroxylphenoxy)azetidin-1-yl)-2-(1H-pyrrol-1-yl)benzoate, 3-(3-(4-hydroxylphenoxy)azetidin-1-yl)-2-(1H-pyrrol-1-yl)benzoic acid, Methyl 3-(3-(2-(tert-butoxy)-2-oxovinyl)azetidin-1-yl)-2-(1H-pyrrol-1-yl)benzoate, Methyl 2-(1H-pyrrol-1-yl)-3-(3-(p-tolylthio)azetidin-1-yl)benzoate, Methyl 2-(1H-pyrrol-1-yl)-3-(3-(p-tolylsulfinyl)azetidin-1-yl)benzoate, Methyl 2-(1H-pyrrol-1-yl)-3-(3-(p-tolylsulfonyl)azetidin-1-yl)benzoate, 2-(1H-pyrrol-1-yl)-3-(3-p-tolylsulfinyl)azetidin-1-yl)benzoic acid, Methyl 3-(3-(4-(4-chlorophenoxy)phenoxy)azetidin-1-yl)-2-(1H-pyrrol-1-yl)benzoate, 3-(3-(4-(4-chlorophenoxy)phenoxy)azetidin-1-yl)-2-(1H-pyrrol-1-yl)benzoic acid, Methyl 3-(3-(4-(3-methoxyphenoxy)phenoxy)azetidin-1-yl)-2-(1H-pyrrol-1-yl)benzoate, 3-(3-(4-(3-methoxyphenoxy)phenoxy)azetidin-1-yl)-2-(1H-pyrrol-1-yl)benzoic acid, Methyl 3-(3-(3-chloro-4-phenoxyphenoxy)azetidin-1-yl)-2-(1H-pyrrol-1-yl)benzoate, 3-(3-(3-chloro-4-phenoxyphenoxy)azetidin-1-yl)-2-(1H-pyrrol-1-yl)benzoic acid, Methyl 3-(3-(3-fluoro-4-phenoxyphenoxy)azetidin-1-yl)-2-(1H-pyrrol-1-yl)benzoate, 3-(3-(3-fluoro-4-phenoxyphenoxy)azetidin-1-yl)-2-(1H-pyrrol-1-yl)benzoic acid, Methyl 3-(3-(4-(4-fluorophenoxy)phenoxy)azetidin-1-yl)-2-(1H-pyrrol-1-yl)benzoate, 3-(3-(4-(4-fluorophenoxy)phenoxy)azetidin-1-yl)-2-(1H-pyrrol-1-yl)benzoic acid, Methyl 3-(3-(3,5-difluoro-4-phenoxyphenoxy)azetidin-1-yl)-2-(1H-pyrrol-1-yl)benzoate, 3-(3-(3,5-difluoro-4-phenoxyphenoxy)azetidin-1-yl)-2-(1H-pyrrol-1-yl)benzoic acid, Methyl 3-(3-(4-(4-acetylphenoxy)phenoxy)azetidin-1-yl)-2-(1H-pyrrol-1-yl)benzoate, 3-(3-(4-(4-acetylphenoxy)phenoxy)azetidin-1-yl)-2-(1H-pyrrol-1-yl)benzoic acid, Methyl 3-(3-(4-(thiophenyl)phenoxy)azetidin-1-yl)-2-(1H-pyrrol-1-yl)benzoate, 3-(3-(4-(thiophenyl)phenoxy)azetidin-1-yl)-2-(1H-pyrrol-1-yl)benzoic acid, Methyl 3-(3-(4-(4-cyanophenoxy)phenoxy)azetidin-1-yl)-2-(1H-pyrrol-1-yl)benzoate, 3-(3-(4-(4-cyanophenoxy)phenoxy)azetidin-1-yl)-2-(1H-pyrrol-1-yl)benzoic acid, Methyl 3-(3-(4-(4-(methylsulfonyl)phenoxy)phenoxy)azetidin-1-yl)-2-(1H-pyrrol-1-yl)benzoate, 3-(3-(4-(4-(methylsulfonyl)phenoxy)phenoxy)azetidin-1-yl)-2-(1H-pyrrol-1-yl)benzoic acid, Methyl 3-(3-(4-propionylphenoxy)azetidin-1-yl)-2-(1H-pyrrol-1-yl)benzoate, 3-(3-(4-propionylphenoxy)azetidin-1-yl)-2-(1H-pyrrol-1-yl)benzoic acid, Methyl 3-(3-(4-isobutyrylphenoxy)azetidin-1-yl)-2-(1H-pyrrol-1-yl)benzoate, 3-(3-(4-isobutyrylphenoxy)azetidin-1-yl)-2-(1H-pyrrol-1-yl)benzoic acid, Methyl 3-(3-(3-methoxy-4-(2-methoxyethoxy)phenoxy)azetidin-1-yl)-2-(1H-pyrrol-1-yl)benzoate, 3-(3-(3-methoxy-4-(2-methoxyethoxy)phenoxy)azetidin-1-yl)-2-(1H-pyrrol-1-yl)benzoic acid, Methyl 3-(3-(4-(2-acetylaminoethoxy)-3-methoxyphenoxy)azetidin-1-yl)-2-(1H-pyrrol-1-yl)benzoate, 3-(3-(4-(2-acetylaminoethoxy)-3-methoxyphenoxy)azetidin-1-yl)-2-(1H-pyrrol-1-yl)benzoic acid, Methyl 3-(3-(3-phenoxyphenoxy)azetidin-1-yl)-2-(1H-pyrrol-1-yl)benzoate, 3-(3-(3-phenoxyphenoxy)azetidin-1-yl)-2-(1H-pyrrol-1-yl)benzoic acid, Methyl 3-(3-(4-(2-methoxyethoxy)phenoxy)azetidin-1-yl)-2-(1H-pyrrol-1-yl)benzoate, 3-(3-(4-(2-methoxyethoxy)phenoxy)azetidin-1-yl)-2-(1H-pyrrol-1-yl)benzoic acid, Methyl 3-(3-(4-(3-(4-chlorophenyl)thioureido)phenoxy)azetidin-1-yl)-2-(1H-pyrrol-1-yl)benzoate, Methyl 3-(3-(4-(3-(3-chlorophenyl)thioureido)phenoxy)azetidin-1-yl)-2-(1H-pyrrol-1-yl)benzoate, Methyl 3-(3-(4-(3-(3-cyanophenyl)thioureido)phenoxy)azetidin-1-yl)-2-(1H-pyrrol-1-yl)benzoate, Methyl 3 -(3 -(4-(3 -(4-(trifluoromethyl)phenyl)ureido)phenoxy)azetidin-1-yl)-2-(1H-pyrrol-1-yl)b enzo ate, 3 -(3 -(4-(3 -(4-(trifluoromethyl)phenyl)ureido)phenoxy)azetidin-1-yl)-2-(1H-pyrrol-1-yl)b enzo ic acid, N-(thiophen-2-yl-methyl)-3-(3-(4-(hydroxymethyl)phenoxy)azetidin-1-yl)-2-(1H-pyrrol-1-yl) benzamide, Ethyl 3-(3-(4-(ethoxymethyl)phenoxy)azetidin-1-yl)-2-(1H-pyrrol-1-yl)benzoate, 3-(3-(4-(ethoxymethyl)phenoxy)azetidin-1-yl)-2-(1H-pyrrol-1-yl)benzoic acid, Methyl 3-(3-(4-(((((tetrahydro-2H-pyran-4-yl)oxy)carbonyl)oxy)methyl)phenoxy)azetidin-1-yl)-2-(1 H-pyrrol-1-yl)benzoate, Methyl 3-(3-(4-(morpholinylmethyl)phenoxy)azetidin-1-yl)-2-(1H-pyrrol-1-yl)benzoate, Methyl 3-(3-(4-((((4-chlorophenyl)thiocarbamoyl)oxy)methyl)phenoxy)azetidin-1-yl)-2-(1H-pyrrol-1 -yl)benzoate, 3-(3-(4-((((4-chlorophenyl)thiocarbamoyl)oxy)methyl)phenoxy)azetidin-1-yl)-2-(1H-pyrrol-1 -yl)benzoic acid, Methyl 3-(3-(4-(((ethylthiocarbamoylamino)oxy)methyl)phenoxy)azetidin-1-yl)-2-(1H-pyrrol-1-yl)be nzoate, 3-(3-(4-(((ethylthiocarbamoylamino)oxy)methyl)phenoxy)azetidin-1-yl)-2-(1H-pyrrol-1-yl)be nzoic acid, Methyl 3-(3-(4-((4-chlorophenoxy)methyl)phenoxy)azetidin-1-yl)-2-(1H-pyrrol-1-yl)benzoate, 3-(3-(4-((4-chlorophenoxy)methyl)phenoxy)azetidin-1-yl)-2-(1H-pyrrol-1-yl)benzoic acid, Methyl 3-(3-(4-((3-cyclopropylureido)methyl)phenoxy)azetidin-1-yl)-2-(1H-pyrrol-1-yl)benzoate, Methyl 3-(3-(4-((pyridin-3-yl-oxy)methyl)phenoxy)azetidin-1-yl)-2-(1H-pyrrol-1-yl)benzoate, 3-(3-(4-((pyridin-3-yl-oxy)methyl)phenoxy)azetidin-1-yl)-2-(1H-pyrrol-1-yl)benzoic acid, Methyl 3-(3-(4-((4-fluorophenoxy)methyl)phenoxy)azetidin-1-yl)-2-(1H-pyrrol-1-yl)benzoate, 3-(3-(4-((4-fluorophenoxy)methyl)phenoxy)azetidin-1-yl)-2-(1H-pyrrol-1-yl)benzoic acid, Methyl 3-(3-(4-((3-fluorophenoxy)methyl)phenoxy)azetidin-1-yl)-2-(1H-pyrrol-1-yl)benzoate, 3-(3-(4-((3-fluorophenoxy)methyl)phenoxy)azetidin-1-yl)-2-(1H-pyrrol-1-yl)benzoic acid, Methyl 3-(3-(4-((3-(trifluoromethyl)phenoxy)methyl)phenoxy)azetidin-1-yl)-2-(1H-pyrrol-1-yl)benzo ate, 3-(3-(4-((3-(trifluoromethyl)phenoxy)methyl)phenoxy)azetidin-1-yl)-2-(1H-pyrrol-1-yl)benzo ic acid, Methyl 3-(3-(4-(hydroxymethyl)-3-methylphenoxy)azetidin-1-yl)-2-(1H-pyrrol-1-yl)benzoate, 3-(3-(4-(hydroxymethyl)-3-methylphenoxy)azetidin-1-yl)-2-(1H-pyrrol-1-yl)benzoic acid, Methyl 3-(3-(4-aminophenoxy)azetidin-1-yl)-2-(1H-pyrrol-1-yl)benzoate, 3-(3-(4-aminophenoxy)azetidin-1-yl)-2-(1H-pyrrol-1-yl)benzoic acid, Methyl 3-(3-(2-aminophenoxy)azetidin-1-yl)-2-(1H-pyrrol-1-yl)benzoate, 3-(3-(2-aminophenoxy)azetidin-1-yl)-2-(1H-pyrrol-1-yl)benzoic acid, Methyl 3-((3-phenoxy)azetidin-1-yl)-2-(1H-pyrrol-1-yl)benzoate, 3-((3-phenoxy)azetidin-1-yl)-2-(1H-pyrrol-1-yl)benzoic acid, Methyl 3-(3-(pyridin-3-yloxy)azetidin-1-yl)-2-(1H-pyrrol-1-yl)benzoate, 3-(3-(pyridin-3-yloxy)azetidin-1-yl)-2-(1H-pyrrol-1-yl)benzoic acid, Methyl 3-(3-(4-(2-(methylsulfonamido)thiazol-4-yl)phenoxy)azetidin-1-yl)-2-(1H-pyrrol-1-yl)benzoa te, 3-(3-(4-(2-(methylsulfonamido)thiazol-4-yl)phenoxy)azetidin-1-yl)-2-(1H-pyrrol-1-yl)benzoi c acid, Methyl 3-(3-(4-(phenylcarbamoyl)phenoxy)azetidin-1-yl)-2-(1H-pyrrol-1-yl)benzoate, 3-(3-(4-(ethylcarbamoyl)phenoxy)azetidin-1-yl)-2-(1H-pyrrol-1-yl)benzoic acid, Methyl 3-(3-(4-(morpholinyl-4-carbonyl)phenoxy)azetidin-1-yl)-2-(1H-pyrrol-1-yl)benzoate, 3-(3-(4-(morpholinyl-4-carbonyl)phenoxy)azetidin-1-yl)-2-(1H-pyrrol-1-yl)benzoic acid, Methyl 3-(3-(4-((4-fluorophenyl)carbamoyl)phenoxy)azetidin-1-yl)-2-(1H-pyrrol-1-yl)benzoate, 3-(3-(4-((4-fluorophenyl)carbamoyl)phenoxy)azetidin-1-yl)-2-(1H-pyrrol-1-yl)benzoic acid, Methyl 3-(1-(4-(hydroxymethyl)phenyl)-1H-1,2,3-triazol-4-yl)-2-(1H-indol-6-yl)benzoate, Methyl 3-(1,1-dioxindibenzo[d]isothiazol-2(3H)-yl)ethynyl)-2-(1H-pyrrol-1-yl)benzoate, Methyl 3-(((N-benzyl-4-methylphenyl)sulfonamido)ethynyl)-2-(1H-pyrrol-1-yl)benzoate, Methyl 3-(2-phenylcyclopropyl)-2-(1H-pyrrol-1-yl)benzoate, 3-(3-(hydroxyl(4-methoxyphenyl)methyl)bicyclo[1.1.1]pentan-1-yl)benzoic acid, Methyl 3-(6-phenyl-2,6-diazaspiro[3.3]heptan-2-yl)-2-(1H-pyrrol-1-yl)benzoate, Methyl 2-amino-3-(6-(2-aminophenyl)-2,6-diazaspiro[3.3]heptan-2-yl)benzoate, 2-(furan-2-yl)-3-(3-(4-hydroxymethyl)phenoxy)azetidin-1-yl)benzoic acid, Methyl 2-(benzofuran-6-yl)-3-(3-(4-hydroxymethyl)phenoxy)azetidin-1-yl)benzoate, 3'-cyano-6-(3-(4-hydroxymethyl)phenoxy)azetidin-1-yl)-[1,1'-biphenyl]-2-formic acid, Methyl 3'-carboxyl-6-(3 -(4-hydroxymethyl)phenoxy)azetidin-1-yl)-[1,1'-biphenyl]-2-formate, 6-(3-(4-(hydroxymethyl)phenoxy)azetidin-1-yl)-3'-(methylsulfonamido)-[1,1'-biphenyl]-2-for mic acid, 3'-cyano-4'-fluoro-6-(3-(4-(hydroxymethyl)phenoxy)azetidin-1-yl)-[1,1'-biphenyl]-2-formic acid, Methyl 2-amino-3-(3-(3-carboxylphenoxy)azetidin-1-yl)benzoate, Methyl 3-(3-(4-(((4-methylphenyl)sulfonamido)methyl)phenoxy)azetidin-1-yl)-2-(1H-pyrrol-1-yl)ben zoate, Methyl 3-(3-((2,6-difluoropyridin-4-yl)oxy)azetidin-1-yl)-2-(1H-pyrrol-1-yl)benzoate, 3-(3-((2,6-difluoropyridin-4-yl)oxy)azetidin-1-yl)-2-(1H-pyrrol-1-yl)benzoic acid, Methyl 3-(3-(4-nitrophenoxy)azetidin-1-yl)-2-(1H-pyrrol-1-yl)benzoate, lithium 3-(((N-(pyridin-3-yl-methyl)-N-(4-methylphenyl)sulfonamido)ethynyl)-2-(1H-pyrrol-1-yl)ben zoate, Methyl 3-(3-(3-(2-(methylsulfonamido)thiazol-4-yl)phenoxy)azetidin-1-yl)-2-(1H-pyrrol-1-yl)benzoa te, 3-(3-(4-(3-(2-methoxyphenyl)ureido)phenoxy)azetidin-1-yl)2-(1H-pyrrol-1-yl)benzoic acid, 3-(3-(4-(3-hexylureido)phenoxy)azetidin-1-yl)2-(1H-pyrrol-1-yl)benzoic acid, Methyl 3 -(3 -(4-(4-(trifluoromethyl)phenoxy)phenoxy)azetidin-1-yl)2-(1H-pyrrol-1 -yl)benzoate, 3-(3-(4-(4-(trifluoromethyl)phenoxy)phenoxy)azetidin-1-yl)2-(1H-pyrrol-1-yl)benzoic acid, Methyl 3-(3-(4-(3-acetylphenoxy)phenoxy)azetidin-1-yl)-2-(1H-pyrrol-1-yl)benzoate, 3 -(3 -(4-(3 -acetylphenoxy)phenoxy)azetidin-1-yl)-2-(1H-pyrrol-1-yl)benzoic acid, Methyl 3-(3-(3-(3-(4-(trifluoromethoxy)phenyl)ureido)phenoxy)azetidin-1-yl)-2-(1H-pyrrol-1-yl)ben zoate, 3-(3-(3-(3-(4-(trifluoromethoxy)phenyl)ureido)phenoxy)azetidin-1-yl)-2-(1H-pyrrol-1-yl)ben zoic acid, Methyl 3-(3-(3-(hydroxymethyl)phenoxy)azetidin-1-yl)-2-(1H-pyrrol-1-yl)benzoate, 3-(3-(3-(hydroxymethyl)phenoxy)azetidin-1-yl)-2-(1H-pyrrol-1-yl)benzoic acid, Methyl 3-(3-(4-(hydroxymethyl)-2-methylphenoxy)azetidin-1-yl)-2-(1H-pyrrol-1-yl)benzoate, 3-(3-(4-(hydroxymethyl)-2-methylphenoxy)azetidin-1-yl)-2-(1H-pyrrol-1-yl)benzoic acid, 3-(3-(4-nitrophenoxy)azetidin-1-yl)-2-(1H-pyrrol-1-yl)benzoic acid, 3-(3-(4-(phenylcarbamoyl)phenoxy)azetidin-1-yl)-2-(1H-pyrrol-1-yl)benzoic acid, Methyl 3-(3-(4-(ethylcarbamoyl)phenoxy)azetidin-1-yl)-2-(1H-pyrrol-1-yl)benzoate, Methyl 3-(3-((1-p-tolylsulfonyl-1H-indol-5-yl)oxy)azetidin-1-yl)-2-(1H-pyrrol-1-yl)benzoate, 3-(3-((1-p-tolylsulfonyl-1H-indol-5-yl)oxy)azetidin-1-yl)-2-(1H-pyrrol-1-yl)benzoic acid, Methyl 3-(3-(3,4-bis(2-methoxyethoxy)phenoxy)azetidin-1-yl)-2-(1H-pyrrol-1-yl)benzoate, 3-(3-(3,4-bis(2-methoxyethoxy)phenoxy)azetidin-1-yl)-2-(1H-pyrrol-1-yl)benzoic acid, Methyl 3-(3-(4-((tetrahydro-2H-pyran-2-yl)oxy)phenoxy)azetidin-1-yl)-2-(1H-pyrrol-1-yl)benzoate, Methyl 3-(3-(4-(2-(methylamino)ethoxy)phenoxy)azetidin-1-yl)-2-(1H-pyrrol-1-yl)benzoate, Methyl 3-(3-(4-(2-aminoethoxy)phenoxy)azetidin-1-yl)-2-(1H-pyrrol-1-yl)benzoate, Methyl 3-(3-(4-(2-aminoethoxy)-3-methoxyphenoxy)azetidin-1-yl)-2-(1H-pyrrol-1-yl)benzoate, Methyl 3-(3-(4-(2-acetylaminoethoxy)phenoxy)azetidin-1-yl)-2-(1H-pyrrol-1-yl)benzoate, 3-(3-(4-(2-acetylaminoethoxy)phenoxy)azetidin-1-yl)-2-(1H-pyrrol-1-yl)benzoic acid, Methyl 3-(((N-(furan-2-yl-methyl)-4-methylphenyl)sulfonamido)ethynyl)-2-(1H-pyrrol-1-yl)benzoate, Lithium 3-(((N-(furan-2-yl-methyl)-4-methylphenyl)sulfonamido)ethynyl)-2-(1H-pyrrol-1-yl)benzoate, Methyl 3-(((4-methyl-N-((1-methyl-1H-indol-5-yl)methyl)phenyl)sulfonamido)ethynyl)-2-(1H-pyrrol -1-yl)benzoate, Lithium 3-(((4-methyl-N-((1-methyl-1H-indol-5-yl)methyl)phenyl)sulfonamido)ethynyl)-2-(1H-pyrrol -1-yl)benzoate, Methyl 3-(((4-methyl-N-((1-methylpiperidin-4-yl)methyl)phenyl)sulfonamido)ethynyl)-2-(1H-pyrrol-1-yl)benzoate, Lithium 3-(((4-methyl-N-((1-methylpiperidin-4-yl)methyl)phenyl)sulfonamido)ethynyl)-2-(1H-pyrrol-1-yl)benzoate, Methyl 3-((N-benzylbenzo[b]thiophen-3-sulfonamido)ethynyl)-2-(1H-pyrrol-1-yl)benzoate, Lithium 3-((N-benzylbenzo[b]thiophen-3-sulfonamido)ethynyl)-2-(1H-pyrrol-1-yl)benzoate, Methyl 3-((N-benzylthiophen-2-sulfonamido)ethynyl)-2-(1H-pyrrol-1-yl)benzoate, Lithium 3-((N-benzylthiophen-2-sulfonamido)ethynyl)-2-(1H-pyrrol-1-yl)benzoate, Methyl 3-((N-(2-fluorobenzyl)thiophen-2-sulfonamido)ethynyl)-2-(1H-pyrrol-1-yl)benzoate, Lithium 3-((N-(2-fluorobenzyl)thiophen-2-sulfonamido)ethynyl)-2-(1H-pyrrol-1-yl)benzoate, Methyl 3-((N-(4-fluorobenzyl)thiophen-2-sulfonamido)ethynyl)-2-(1H-pyrrol-1-yl)benzoate, Lithium 3-((N-(4-fluorobenzyl)thiophen-2-sulfonamido)ethynyl)-2-(1H-pyrrol-1-yl)benzoate, Methyl 3-(((4-methyl-N-(thiophen-2-yl-methyl)phenyl)sulfonamido)ethynyl)-2-(1H-pyrrol-1-yl)benz oate, Lithium 3-(((4-methyl-N-(thiophen-2-yl-methyl)phenyl)sulfonamido)ethynyl)-2-(1H-pyrrol-1-yl)benz oate, Methyl 3-((N-benzylbenzofuran-5-sulfonamido)ethynyl)-2-(1H-pyrrol-1-yl)benzoate, Lithium 3-((N-benzylbenzofuran-5-sulfonamido)ethynyl)-2-(1H-pyrrol-1-yl)benzoate, Methyl 3-(((N-(4-(hydroxymethyl)benzyl)-4-methylphenyl)sulfonamido)ethynyl)-2-(1H-pyrrol-1-yl)b enzoate, Lithium 3-(((N-(4-(hydroxymethyl)benzyl)-4-methylphenyl)sulfonamido)ethynyl)-2-(1H-pyrrol-1-yl)b enzoate, Methyl 3-((N-((1-methyl-1H-indol-5-yl)methyl)benzo[b]thiophen-3-sulfonamido)ethynyl)-2-(1H-pyrr ol-1-yl)benzoate, Lithium 3-((N-((1-methyl-1H-indol-5-yl)methyl)benzo[b]thiophen-3-sulfonamido)ethynyl)-2-(1H-pyrr ol-1-yl)benzoate, Methyl 3-((N-(4-(trifluoromethyl)benzyl)thiophen-2-sulfonamido)ethynyl)-2-(1H-pyrrol-1-yl)benzoat e, Lithium 3-((N-(4-(trifluoromethyl)benzyl)thiophen-2-sulfonamido)ethynyl)-2-(1H-pyrrol-1-yl)benzoat e, Methyl 3-((N-((1-methyl-1H-indol-5-yl)methyl)benzofuran-5-sulfonamido)ethynyl)-2-(1H-pyrrol-1-y 1)benzoate, Lithium 3-((N-((1-methyl-1H-indol-5-yl)methyl)benzofuran-5-sulfonamido)ethynyl)-2-(1H-pyrrol-1-y 1)benzoate, Methyl 3-((N-benzyl-N-phenylsulfonamido)ethynyl)-2-(1H-pyrrol-1-yl)benzoate, 3-((N-benzyl-N-phenylsulfonamido)ethynyl)-2-(1H-pyrrol-1-yl)benzoic acid, Methyl 3-(((N-(4-methylbenzyl)-N-(4-tolylsulfonamido)ethynyl)-2-(1H-pyrrol-1-yl)benzoate, Lithium 3-(((N-(4-methylbenzyl)-N-(4-tolylsulfonamido)ethynyl)-2-(1H-pyrrol-1-yl)benzoate, Methyl 3-(((N-(4-fluorobenzyl)-4-tolylsulfonamido)ethynyl)-2-(1H-pyrrol-1-yl)benzoate, Lithium 3-(((N-(4-fluorobenzyl)-4-tolylsulfonamido)ethynyl)-2-(1H-pyrrol-1-yl)benzoate, Methyl 3-(((N-(4-(trifluoromethyl)benzyl)-N-(4-tolylsulfonamido)ethynyl)-2-(1H-pyrrol-1-yl)benzoat e, Lithium 3-(((N-(4-(trifluoromethyl)benzyl)-(4-tolylsulfonamido)ethynyl)-2-(1H-pyrrol-1-yl)benzoate, Methyl 3-(3-((4-isopropylphenyl)thio)azetidin-1-yl)-2-(1H-pyrrol-1-yl)benzoate, Methyl 3-(3-((4-chlorophenyl)thio)azetidin-1-yl)-2-(1H-pyrrol-1-yl)benzoate, Methyl 3-(3-(cyclohexylthio)azetidin-1-yl)-2-(1H-pyrrol-1-yl)benzoate, Methyl 3 -(3 -(phenylthio)azetidin-1-yl)-2-(1H-pyrrol-1-yl)benzoate, Methyl 3-(3-((4-(hydroxymethyl)phenyl)thio)azetidin-1-yl)-2-(1H-pyrrol-1-yl)benzoate, Methyl 3-(3-((4-isopropylphenyl)sulfonyl)azetidin-1-yl)-2-(1H-pyrrol-1-yl)benzoate, Methyl 3-(3-((4-chlorophenyl)sulfonyl)azetidin-1-yl)-2-(1H-pyrrol-1-yl)benzoate, Methyl 3-(3-(cyclohexylsulfonyl)azetidin-1-yl)-2-(1H-pyrrol-1-yl)benzoate, Methyl 3-(3-(phenylsulfonyl)azetidin-1-yl)-2-(1H-pyrrol-1-yl)benzoate, Methyl 3-(3-((4-(hydroxymethyl)phenyl)sulfonyl)azetidin-1-yl)-2-(1H-pyrrol-1-yl)benzoate, Methyl 3-(3-(4-(((5-fluoropyridin-3-yl)oxy)methyl)phenoxy)azetidin-1-yl)-2-(1H-pyrrol-1-yl)benzoat e, 3-(3-(4-(((5-fluoropyridin-3-yl)oxy)methyl)phenoxy)azetidin-1-yl)-2-(1H-pyrrol-1-yl)benzoic acid, Methyl 3-(3-(4-((pyrimidin-5-yl-oxy)methyl)phenoxy)azetidin-1-yl)-2-(1H-pyrrol-1-yl)benzoate, 3-(3-(4-((pyrimidin-5-yl-oxy)methyl)phenoxy)azetidin-1-yl)-2-(1H-pyrrol-1-yl)benzoic acid, Methyl 3-(3-(4-(phenylamino)methyl)phenoxy)azetidin-1-yl)-2-(1H-pyrrol-1-yl)benzoate, 3-(3-(4-(phenylamino)methyl)phenoxy)azetidin-1-yl)-2-(1H-pyrrol-1-yl)benzoic acid, Methyl 3-(3-(4-(benzylamino)methyl)phenoxy)azetidin-1-yl)-2-(1H-pyrrol-1-yl)benzoate, 3-(3-(4-(benzylamino)methyl)phenoxy)azetidin-1-yl)-2-(1H-pyrrol-1-yl)benzoic acid, Methyl 3-(3-(3-(2-methoxyethoxy)-4-((2-methoxyethoxy)methyl)phenoxy)azetidin-1-yl)-2-(1H-pyrro 1-1-yl)benzoate, 3-(3-(3-(2-methoxyethoxy)-4-((2-methoxyethoxy)methyl)phenoxy)azetidin-1-yl)-2-(1H-pyrro 1-1-yl)benzoic acid, N-(2-cyanoethyl)-3-(3-(4-(hydroxymethyl)phenoxy)azetidin-1-yl)-2-(1H-pyrrol-1-yl)benzami de, N-methylsulfonyl-(3-(3-(4-(hydroxymethyl)phenoxy)azetidin-1-yl)-2-(1H-pyrrol-1-yl))benza mide, Methyl 3-(3-(4-(2-methoxyethoxy)methyl)phenoxy)azetidin-1-yl)-2-(1H-pyrrol-1-yl)benzoate, 3-(3-(4-(2-methoxyethoxy)methyl)phenoxy)azetidin-1-yl)-2-(1H-pyrrol-1-yl)benzoic acid, 3-(3-(4-(2-hydroxylethoxy)methyl)phenoxy)azetidin-1-yl)-2-(1H-pyrrol-1-yl)benzoic acid, 3-(3-(4-(2-hydroxylethoxy)methyl)phenoxy)azetidin-1-yl)-2-(1H-pyrrol-1-yl)benzoic acid, Methyl 3-(3-(4-(2-acetylaminoethoxy)methyl)phenoxy)azetidin-1-yl)-2-(1H-pyrrol-1-yl)benzoate, Methyl 3-(3-(4-(tetrahydrofuran-3-yl)oxymethyl)phenoxy)azetidin-1-yl)-2-(1H-pyrrol-1-yl)benzoate, 3-(3-(4-(tetrahydrofuran-3-yl)oxymethyl)phenoxy)azetidin-1-yl)-2-(1H-pyrrol-1-yl)benzoic acid, Methyl 3-(3-(4-((oxetanyl-3-oxy)methyl)phenoxy)azetidin-1-yl)-2-(1H-pyrrol-1-yl)benzoate, Methyl 3-(3-(4-(1-acetylpiperidin-4-yl)oxymethyl)phenoxy(azetidin-1-yl)-2-(1H-pyrrol-1-yl)benzoate, 3-(1,1-dioxindibenzo[d]isothiazol-2(3H)-yl)ethynyl)-2-(1H-pyrrol-1-yl)benzoic acid, 3-(((N-benzyl-4-methylphenyl)sulfonamido)ethynyl)-2-(1H-pyrrol-1-yl)benzoic acid, Methyl 3-((N-benzylmethylsulfonamido)ethynyl)-2-(1H-pyrrol-1-yl)benzoate, Lithium 3-((N-benzylmethylsulfonamido)ethynyl)-2-(1H-pyrrol-1-yl)benzoate, Methyl 3-(((N-methyl-4-methylphenylsulfonamido)ethynyl)-2-(1H-pyrrol-1-yl)benzoate, Methyl 3-(((N-methyl-4-methylphenylsulfonamido)ethynyl)-2-(1H-pyrrol-1-yl)benzoate, Methyl 3-(((N-(4-methoxybenzyl)-4-methylphenylsulfonamido)ethynyl)-2-(1H-pyrrol-1-yl)benzoate, Lithium 3-(((N-(4-methoxybenzyl)-4-methylphenylsulfonamido)ethynyl)-2-(1H-pyrrol-1-yl)benzoate, Methyl 3-(((N-(pyridin-3-yl-methyl)-N-(4-methylphenyl)sulfonamido)ethynyl)-2-(1H-pyrrol-1-yl)ben zoate, N-(furan-2-yl-methyl)-3-(3-(4-(hydroxymethyl)phenoxy)azetidin-1-yl)-2-(1H-pyrrol-1-yl)ben zamide, Methyl 3-(((N-(pyridin-4-yl-methyl)-N-(4-methylphenyl)sulfonamido)ethynyl)-2-(1H-pyrrol-1-yl)ben zoate, Lithium 3-(((N-(pyridin-4-yl-methyl)-N-(4-methylphenyl)sulfonamido)ethynyl)-2-(1H-pyrrol-1-yl)ben zoate, Methyl 2-amino-3-(2-phenylcyclopropyl)benzoate, 2-amino-3-(2-phenylcyclopropyl)benzoic acid, 3-(2-phenylcyclopropyl)-2-(1H-pyrrol-1-yl)benzoic acid, 2-(1H-pyrrol-1-yl)-3-(3-(4-(((tetrahydro-2H-pyran-4-yl)oxy)methyl)phenoxy)azetidin-1-yl)be nzoic acid, Methyl 2-(benzo[b]thiophen-2-yl)-3-(3-(4-(hydroxymethyl)phenoxy)azetidin-1-yl)benzoate, and N-(2-dimethylamino)ethyl)-3-(3-(4-(hydroxymethyl)phenoxy)azetidin-1-yl)-2-(1H-pyrrol-1-y 1)benzamide.

In another embodiment, the present invention further relates to a pharmaceutical composition, which includes one or more compounds having a general formula (I), or an enantiomer, a diastereomer, a tautomer, a salt, a crystalline form, a solvate and/or an isotopically substituted derivative thereof according to the present invention.

In another embodiment, the present invention further relates to a pharmaceutical composition, which includes one or more compounds having a general formula (I), or an enantiomer, a diastereomer, a tautomer, a salt, a crystalline form, a solvate and/or an isotopically substituted derivative thereof according to the present invention, and a pharmaceutically acceptable carrier or excipient or diluent.

In another embodiment, the present invention further relates to a method for treating and/or preventing a disease or disorder caused by EBNA1 activity. The method includes administrating an effective amount of the compound having the above general formula (I), or an enantiomer, a diastereomer, a tautomer, a salt, a crystalline form, a solvate and/or an isotopically substituted derivative thereof to a subject.

In another embodiment, the present invention further relates to use of one or more compounds having the above general formula (I), or an enantiomer, a diastereomer, a tautomer, a salt, a crystalline form, a solvate and/or an isotopically substituted derivative thereof according to the present invention in the preparation of a medicament for treating and/or preventing a disease caused by EBNA1 activity, which includes administrating an effective amount of at least one compound, or an enantiomer, a diastereomer, a tautomer, a salt, a crystalline form, a solvate and/or an isotopically substituted derivative thereof according to the present invention to a subject.

In another embodiment, the present invention further relates to a method for treating and/or preventing a disease or disorder caused by EBNA1 activity, which includes administrating an effective amount of at least one pharmaceutical composition according to the present invention to a subject.

In another embodiment, the present invention further relates to use of one or more of the above pharmaceutical compositions according to the present invention in the preparation of a medicament for treating and/or preventing a disease caused by EBNA1 activity, which includes administrating an effective amount of at least one pharmaceutical composition according to the present invention to a subject.

In a preferred embodiment, the disease or disorder is cancer, infectious mononucleosis, chronic fatigue syndrome, multiple sclerosis, systemic lupus erythematosus or rheumatoid arthritis.

In a more preferred embodiment, the cancer is nasopharyngeal carcinoma, gastric cancer, non-Hodgkin's lymphoma, anaplastic large cell lymphoma, angioimmunoblastic T-cell lymphoma, hepatosplenic T-cell lymphoma, B-cell lymphoma, Burkitt lymphoma, reticuloendotheliosis, reticulocytosis, microglioma, diffuse large B-cell lymphoma, extranodal T/NK lymphoma/angiocentric lymphoma, follicular lymphoma, immunoblastic lymphoma, mucosa-associated lymphoid tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma, primary exudative lymphoma, lymphomatoid granuloma, angioimmunoblastic lymphadenopathy, leiomyosarcoma, X-linked lymphoproliferative disorder, posttransplant lymphoproliferative disorder, Hodgkin's lymphoma, or breast cancer.

In another embodiment, the present invention further relates to a method for treating and/or preventing a disease or disorder caused by and/or associated with Epstein-Barr virus (EBV) infection, the method includes administrating an effective amount of at least one compound, or an enantiomer, a diastereomer, a tautomer, a salt, a crystalline form, a solvate and/or an isotopically substituted derivative thereof or a pharmaceutical composition thereof according to the present invention to a subject.

In another embodiment, the present invention further relates to further use of the compound having the above general formula (I), or an enantiomer, a diastereomer, a tautomer, a salt, a crystalline form, a solvate and/or an isotopically substituted derivative thereof or a pharmaceutical composition thereof in the preparation of a medicament for treating and/or preventing a disease caused by and/or associated with Epstein-Barr virus (EBV) infection, which includes administrating an effective amount of at least one compound or pharmaceutical composition according to the present invention to a subject.

In another embodiment, the present invention further relates to a method for treating and/or preventing EBV infection in a lytic and/or latent phase. The method includes administrating an effective amount of at least one compound, or an enantiomer, a diastereomer, a tautomer, a salt, a crystalline form, a solvate and/or an isotopically substituted derivative thereof or a pharmaceutical composition thereof according to the present invention to a subject.

In another embodiment, the present invention further relates to further use of the compound having the above general formula (I), or an enantiomer, a diastereomer, a tautomer, a salt, a crystalline form, a solvate and/or an isotopically substituted derivative thereof or a pharmaceutical composition thereof in the preparation of a medicament for treating and/or preventing a disease caused by EBV infection in a lytic and/or latent phase, which includes administrating an effective amount of at least one compound, or an enantiomer, a diastereomer, a tautomer, a salt, a crystalline form, a solvate and/or an isotopically substituted derivative thereof or a pharmaceutical composition thereof according to the present invention to a subject.

The examples and preparation examples provided in the present invention further illustrate and exemplify compounds and preparation methods therefor according to the present invention. It should be understood that the scope of the following preparation examples and examples does not limit the scope of the present invention in any way.

The following synthetic routes have described preparation methods for a derivative having a general formula (I) according to the present invention, the raw materials, reagents, catalysts, solvents, etc. used in the following synthetic schemes can be prepared by methods well known to those skilled in the art of organic chemistry or can be commercially available. All final derivatives according to the present invention can be prepared by the methods described in the schematic diagrams or analogous methods, which are well known to those skilled in the art of organic chemistry. All variables used in these schematic diagrams are as defined below or as defined in the claims.

### Preparation Method

Unless otherwise specified, all reagents were purchased from reagent companies and used directly without purification; most of reactions were performed under anhydrous conditions and nitrogen protection. The purification of reagents and solvents was conducted with reference to Purification of Laboratory Chemicals (W. L. F. Armarego, Christina Li Lin Chai, Elsevier Inc. 2009). Unless otherwise specified, solvents were redistilled before use. Tetrahydrofuran (THF) was treated with the sodium-benzophenone system, dichloromethane and N,N'-dimethylformamide (DMF) were treated with calcium hydride, methanol was treated with magnesium chips and redistilled for later use. Triethylamine, diisopropylethylamine (DIPEA) and pyridine were all treated with calcium hydride and redistilled. The reaction was monitored by thin layer chromatography (TLC) with a thin layer silica gel plate GF254 (60-F250, 0.2 mm), using UV (wavelength of 254 nm) or iodine for color development, or soaking in phosphomolybdic acid and ninhydrin solution before heating for color development. The column for flash column chromatography was packed with silica gel 60 (230-400 mesh ASTM), and a system of ethyl acetate/n-hexane or dichloromethane/methanol was usually used as an eluent. 1H NMR was measured by DRX-300 or Brüker Avance-400 or Brüker Avance-500 nuclear magnetic resonance spectrometer, and deuterated solvent residual peaks were used to locate chemical shifts; high resolution mass spectrum was determined by ABI Q-star Elite mass spectrometer or Q Exactive Focus mass spectrometer from Thermo Company; and the liquid phase mass spectrometer was Agilent 6125.

### General Experimental Operation I:

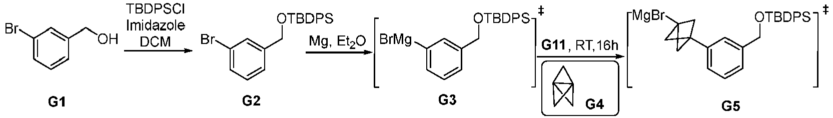

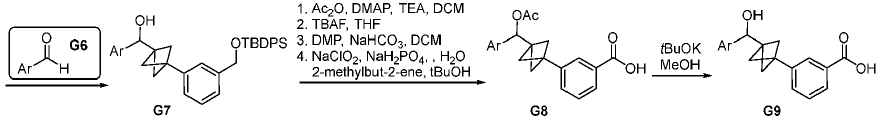

M-bromobenzyl alcohol G1 (1 eq.) was dissolved in anhydrous dichloromethane in a round bottom flask, followed by adding imidazole (2 eq.) and then adding tert-butylchlorodiphenylsilane (TBDPSC1) (1.5 eq.) under ice bath. The reaction system was warmed naturally, and monitored by thin layer chromatography. After the reaction, dichloromethane was removed under reduced pressure, and the residue was dissolved in a large amount of ethyl acetate and washed with saturated ammonium chloride solution, saturated sodium bicarbonate solution and saturated brine, respectively. The organic phase was dried with anhydrous sodium sulfate, filtered, concentrated, and separated by silica gel column chromatography, so as to obtain G2 (99% yield). The compound G2 (1 eq.) was taken and dissolved in anhydrous ether for later use. Metal magnesium chips (2 eq., used after grinding) were placed in a two-necked round bottom flask, anhydrous ether was added under nitrogen; iodine (elementary substance) and 1,2-dibromoethane were added successively, followed by heating slightly to initiate Grignard reaction. After initiation, the solution of the compound G2 in ether was added slowly, and after the addition was completed, the reaction was maintained in a slight-boiling reflux state for 2 hours, and the solution of the Grignard reagent G3 in ether was obtained after stirring slowly at room temperature for 12 hours. The concentration of the Grignard reagent G7 was about 1.17 mmol/ml. After resting, the Grignard reagent G7 was filtered with a filter membrane and sealed, and stored at low temperature for later use. The Grignard reagent G7 (1.2 eq.) was placed in a round bottom flask, and cooled in ice bath; and the solution of tricyclo[1.1.1.0^{1.3}]pentane G4 in ether/diethoxymethane (1 eq.) was introduced under N₂ protection to give a mixed solution. After heating the mixed solution to room temperature and stirring it overnight in dark, a new solution of Grignard reagent G12 was obtained. An appropriate volume of the Grignard reagent G5 was taken, aromatic aldehyde G6 (2 eq.) was added to it in ice bath for cooling, and warmed to room temperature and continued stirring for 20-30 minutes, and then quenched with water to give a reaction solution. The reaction solution was diluted with a large amount of ethyl acetate, and washed with saturated ammonium chloride solution, saturated sodium bicarbonate solution and saturated brine, respectively. The organic phase obtained was dried with anhydrous sodium sulfate, filtered, concentrated, and separated by silica gel column chromatography, so as to obtain yellow oil G7 (total yield 30-50%).

The compound G7 (1.0 eq.) was dissolved in anhydrous dichloromethane, followed by adding triethylamine (10.0 eq.), DMAP (0.5 eq.) and acetic anhydride (4.0 eq.) respectively, reacting at room temperature for 0.5 hours, and then adding saturated ammonium chloride solution for quenching, giving a reaction solution. The reaction solution was diluted with a large amount of ethyl acetate, and washed with saturated ammonium chloride solution, saturated sodium bicarbonate solution and saturated brine, respectively, giving an orgiani phase. The organic phase was dried with anhydrous sodium sulfate. After filtration, a resulting crude product was concentrated and directly dissolved in anhydrous tetrahydrofuran without further purification, tetrabutylammonium fluoride (TBAF) solution (1.5 eq., 0.5 mol/L tetrahydrofuran solution) was added, reacting at room temperature for 0.5-1 hour, and then saturated ammonium chloride solution was added for quenching. The resulting reaction solution was diluted with a large amount of ethyl acetate, and washed with saturated ammonium chloride solution, saturated sodium bicarbonate solution and saturated brine, respectively. The resulting organic phase was dried with anhydrous sodium sulfate. After filtration, the resulting crude product was concentrated, and directly dissolved in anhydrous dichloromethane without further purification, anhydrous sodium bicarbonate (2.0 eq.) and Dess-Martin reagent (1.5 eq.) were added, reacting at room temperature for 3-4 hours. The completion of the reaction was monitored by TLC and addition of saturated ammonium chloride solution was used for quenching. The resulting reaction solution was diluted with a large amount of ethyl acetate, and washed with saturated ammonium chloride solution, saturated sodium bicarbonate solution and saturated brine, respectively. The resulting organic phase was dried with anhydrous sodium sulfate, filterd, and concentrated to obtain the benzaldehyde intermediate. This intermediate was directly dissolved in tert-butanol without isolation, and 2-methyl-2-butene (20.0 eq.) was added. Sodium chlorite solution (10 N) and sodium dihydrogen phosphate buffer solution (0.5 mol/L, pH 6.0) were added under cooling in ice bath, and the reaction was continued for 2-3 hours after warming to room temperature. The completion of the reaction was monitored by TLC, the resulting reaction solution was diluted with a large amount of ethyl acetate, and washed with saturated ammonium chloride solution, saturated sodium bicarbonate solution and saturated brine, respectively. The resulting organic phase was dried with anhydrous sodium sulfate, filtered, concentrated and separated by silica gel column chromatography, so as to obtain carboxylic acid G8 (the yield as shown in examples).

The compound G8 (1 eq.) was dissolved in anhydrous methanol, and potassium tert-butoxide (2.0 eq., 0.05 mol/L methanol solution) was added, stirring overnight at room temperature. Water was added for dilution, the pH was adjusted to 3-4 with dilute hydrochloric acid (0.5 mol/L), and the extraction was performed with ethyl acetate. The resulting organic phase was dried with anhydrous sodium sulfate, filtered, concentrated, and separated by an analytical silica gel chromatoplate, so as to obtain compound G9 (the yield as shown in examples).

### General Experimental Operation II:

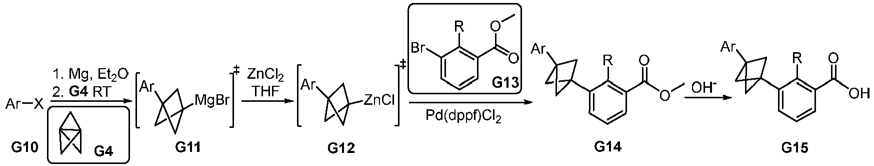

The preparation of the Grignard reagent from aromatic halide ArX G9 was omitted. The Grignard reagent was reacted with G4 to obtain a new Grignard reagent G11. Freshly prepared anhydrous zinc chloride (1.1 eq., tetrahydrofuran solution) was added to the reaction solution that was not separated, stirring at room temperature for 2 hours to obtain zinc reagent G12 for later use. Methyl m-bromobenzoate G13 (1.1 eq.) and Pd(dppf)Cl₂ (0.01 eq.) were added in a round bottom flask equipped with a magnetic stirring bar, after stirring at room temperature for 5 minutes, the zinc reagent G12 prepared above was added, performing a reaction overnight at 50°C under nitrogen protection. The reaction was quenched by adding saturated ammonium chloride aqueous solution, and the resulting reaction solution was extracted with a large amount of ethyl acetate to give a organic phase, the organic phase was washed with saturated brine, dried with anhydrous sodium sulfate, filtered, concentrated to dryness, and passed through a silica gel column (eluted with n-hexane: ethyl acetate = 30:1), so as to obtain a crude compound G14. The crude compound was separated by high performance liquid chromatography (Instrument brand and model, column brand and model, solvent composition, flow rate, detector, and wavelength) to obtain pure compound G14 (the yield as shown in examples). The hydrolysis operation of G14 was carried out under standard conditions as described above, so as to obtain carboxylic acid G15.

### General Experimental Operation III:

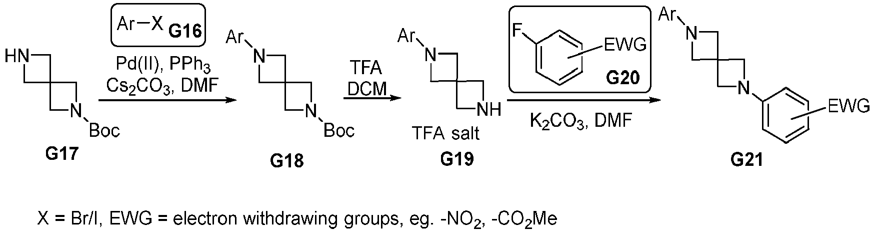

Dioxalate salt of compound G17 (1.0 eq.), tetrakis(triphenylphosphine)palladium (0.1 eq.) and cesium carbonate (5.0 eq.) were added in a round bottom flask. Dry N,N'-dimethylformamide was introduced under nitrogen protection, G23 (5.0 eq.) was added under stirring, warming to 100°C under nitrogen protection to perform a reaction overnight. After the resulting reaction solution was cooled to room temperature, it was diluted with a large amount of ethyl acetate, and washed with saturated sodium bicarbonate solution and saturated brine, respectively. The resulting organic phase was dried with anhydrous sodium sulfate, filtered, concentrated and separated by column chromatography, so as to obtain compound G18.

The compound G18 (1.0 eq.) was dissolved in anhydrous dichloromethane, anhydrous trifluoroacetic acid was added in ice bath, followed by returning to room temperature and continuing to stir for more than 30 minutes, and a reaction was monitored by TLC until the reaction was completed. A solvent was removed by rotary evaporation under reduced pressure, an appropriate amount of dichloromethane was added, followed by performing concentration under reduced pressure, and such operations were repeated twice. The residue was dried with an oil pump to obtain intermediate G19. Without further purification of the intermediate G19, fluorinated compound G20 (2.0 eq.) and potassium carbonate (5.0 eq.) were added to the reaction flask. Afterwards, N,N'-dimethylformamide was introduced under nitrogen protection, stirring at room temperature for 1-5 hours under nitrogen protection, and the reaction was monitored by TLC until the reaction was completed. The resulting reaction solution was diluted with ethyl acetate and then washed with saturated sodium bicarbonate solution and saturated brine, respectively. The resulting organic phase was dried with anhydrous sodium sulfate, filtered, concentrated and separated by column chromatography, so as to obtain compound G21 (the yield as shown in examples).

For an aromatic ring containing an electron withdrawing substituent, the first step of coupling may also be accomplished by an aromatic substitution reaction (SN_{Ar}), as shown in the figure below. The coupling of G22 and G17 occurred successfully under alkaline condition, and after derivatization and removal of Boc, the resultant was further reacted with G20 to obtain G25.

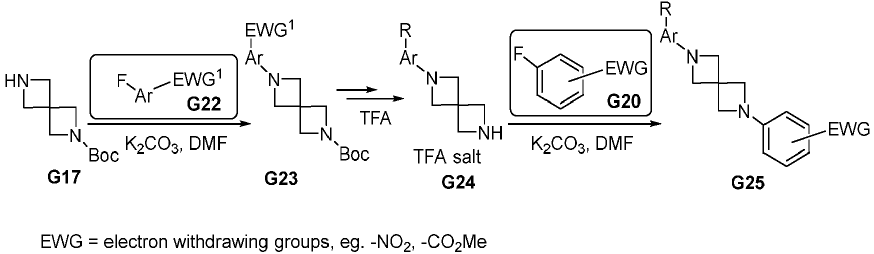

**Nitro reduction operation:** the nitro-substituted compound (1.0 eq.) in need of reduction was dissolved in ethanol, an equal volume of saturated ammonium chloride aqueous solution was added, and then reductant iron powder/zinc powder (20.0 eq.) was added, giving a reaction solution. The reaction solution was heated to reflux under nitrogen protection and stirred for more than 2 hours, and was monitored by TLC until the reaction was completed. After cooling to room temperature, the reaction solution was diluted with a large amount of ethyl acetate, and washed with saturated sodium bicarbonate aqueous solution. The resulting organic phase was dried with anhydrous sodium sulfate, and the resulting aqueous phase was extracted twice with ethyl acetate. The organic phases were combined, dried with anhydrous sodium sulfate, filtered, concentrated, and then separated by column chromatography to obtain a compound.

**Hydrolysis operation:** the same as described above, the methyl ester was hydrolyzed to obtain a carboxylic acid under alkaline condition.

### Synthesis of pyrrole:

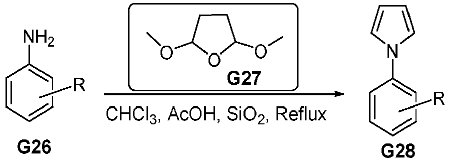

Compound G26 (1.0 eq.) was dissolved in anhydrous chloroform, appropriate amounts of dry silica gel, the compound G27 (5 eq.) and anhydrous acetic acid (50 eq.) were added, respectively. Afterwards, under nitrogen protection, a reaction was performed under reflux for more than 2 hours, and was monitored by TLC until the reaction was completed. The resulting reaction solution was cooled to room temperature, followed by filtering to remove a solid and then washing a filter cake with dichloromethane. The resulting organic phase was concentrated under reduced pressure, and then separated by a preparative thin layer chromatoplate, so as to obtain a target compound G28 containing pyrrole as a heterocycle.

### General Experimental Operation IV:

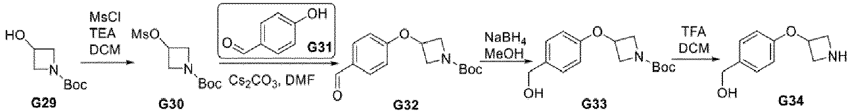

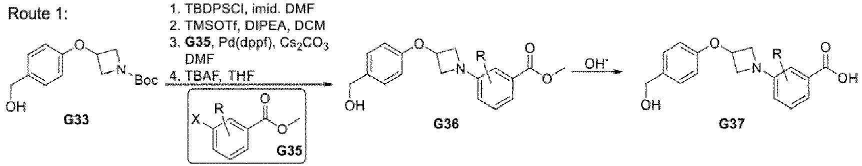

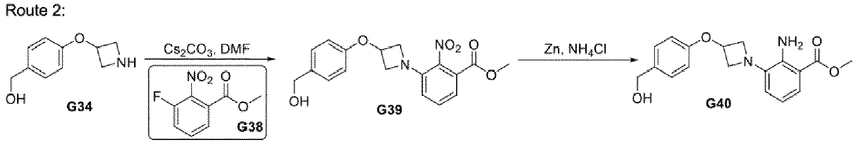

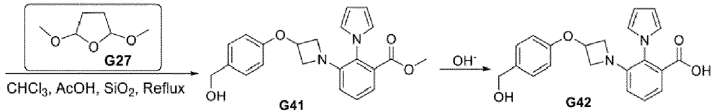

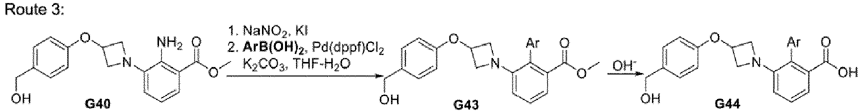

N-Boc-3-hydroxyazetidine G29 (1 eq.) was dissolved in dichloromethane. Under nitrogen protection, triethylamine (1.5 eq.) was added under ice bath cooling, and then methylsulfonyl chloride (1.5 eq.) was slowly added dropwise, and the mixture was warmed to room temperature, reacting for 2 hours. The solvent was removed by rotary evaporation under reduced pressure, and the residue was diluted with a large amount of ethyl acetate and washed once with water. The resulting organic phase was dried with anhydrous sodium sulfate, filtered and concentrated, so as to obtain light yellow oily intermediate compound G30. Without further purification, G30 was dissolved in N,N'-dimethylformamide, 4-hydroxybenzaldehyde G31 (1.2 eq.) and cesium carbonate (2.2 eq.) were added, and heated to 100°C under nitrogen protection, for reacting overnight. After cooling to room temperature, the resulting reaction solution was diluted with a large amount of ethyl acetate, washed twice with a saturated aqueous solution of ammonium chloride, and washed once with a saturated aqueous solution of sodium bicarbonate. The resulting organic phase was dried with anhydrous sodium sulfate, filtered, concentrated and separated by silica gel column chromatography, so as to obtain compound G32 (54%).

The compound G32 (1 eq.) was dissolved in a solvent, and sodium borohydride (3.0 eq.) was added under nitrogen protection and ice bath cooling, then a small amount of methanol was added, and a reaction was performed at room temperature for 2 hours. The reaction was quenched with the saturated aqueous solution of ammonium chloride, followed by removing the solvent by rotary evaporation under reduced pressure, and extracting with a large amount of ethyl acetate. The resulting organic phase was washed three times with the saturated aqueous solution of sodium chloride, dried with anhydrous sodium sulfate, filtered and concentrated, so as to obtain compound G33. The compound G33 was dissolved in an appropriate amount of dichloromethane and treated with excess trifluoroacetic acid so as to remove the Boc protection, thereby obtaining compound G34.

**Route 1:** The compound G33 (1 eq.) and imidazole (2.0 eq.) were dissolved in dichloromethane, and then tert-butylchlorodiphenylsilane (TBDPSCl) (1.5 eq.) was added under nitrogen protection and ice bath cooling, and a reaction was performed at room temperature overnight. After adding water to quench the reaction, the solvent was removed by rotary evaporation under reduced pressure, and the residue was extracted with a large amount of ethyl acetate. The resulting organic phase was washed three times with pure water, and dried with anhydrous sodium sulfate, filtered, concentrated, and separated by silica gel column chromatography, so as to obtain a silicon-protected product (88% yield). The silicon-protected product (1.0 eq.) was dissolved in dichloromethane, diisopropylethylamine (5.0 eq.) was added under ice bath cooling, and then trimethylsilyl trifluoromethanesulfonate (TMSOTf) (4.0 eq.) was added dropwise, and a reaction was performed at room temperature for 2 hours. After quenching the reaction with the saturated aqueous solution of ammonium chloride, an extraction was performed with a large amount of ethyl acetate. The resulting organic phase was washed twice with the saturated aqueous solution of sodium bicarbonate, washed once with the saturated aqueous solution of sodium chloride, dried with anhydrous sodium sulfate, filtered, concentrated and separated by silica gel column chromatography, so as to obtain a secondary amine intermediate free of Boc (yield 100%). The secondary amine intermediate (1.0 eq.), substituted halogenated methyl benzoate G35 (1.0 eq.), tetrakis(triphenylphosphine)palladium (0.05 eq.), cesium carbonate (2.0 eq.), and triphenylphosphine (0.15 eq.) were dissolved in N,N'-dimethylformamide, and subjected to a reaction overnight at 110°C under nitrogen protection. After cooling to room temperature, the resulting reaction solution was diluted with a large amount of ethyl acetate, washed twice with the saturated aqueous solution of ammonium chloride, and washed once with the saturated aqueous solution of sodium chloride. The resulting organic phase was dried with anhydrous sodium sulfate, filtered, concentrated, and separated by a preparative thin layer chromatoplatet, so as to obtain a coupling compound (65%). The coupling compound (1.0 eq.) was dissolved in tetrahydrofuran, followed by adding tetrabutylammonium fluoride (2.0 eq., 1 mol/L tetrahydrofuran solution), and performing a reaction at room temperature for 3 hours. The reaction was quenched with the saturated aqueous solution of ammonium chloride, and the solvent was removed by rotary evaporation under reduced pressure, and the resisude was extracted with a large amount of ethyl acetate. The resulting organic phase was washed twice with the saturated aqueous solution of ammonium chloride and once with the saturated aqueous solution of sodium chloride, dried with anhydrous sodium sulfate, filtered, concentrated, and then separated by silica gel column chromatography, so as to obtain compound G36 (the yield as shown in examples). The reaction to obtain G37 by hydrolysis was the same as described above (omitted).

**Route 2:** The intermediate G34 (1.0 eq.), methyl 3-fluoro-2-nitrobenzoate G38 (1.0 eq.), and cesium carbonate (2.0 eq.) were dissolved in N,N'-dimethylformamide, and heated to 50°C under nitrogen protection, and subjected to a reaction for 2 hours. After cooling the reaction solution to room temperature, it was diluted with a large amount of ethyl acetate, and washed three times with the saturated aqueous solution of ammonium chloride. The resulting organic phase was dried with anhydrous sodium sulfate, filtered, concentrated, and then separated by silica gel column chromatography, so as to obtain compound G39 (74% yield). The steps of nitro reduction, synthesis of pyrrole and hydrolysis reaction to obtain G40-G42 were the same as described above (omitted).

**Route 3:** The compound G40 (1 eq.) was dissolved in 1,4-dioxane-water, and the concentrated hydrochloric acid was added under ice bath cooling. After stirring a mixture for 10 minutes, an aqueous solution of sodium nitrite (1.1 eq.) was slowly added. After a reaction was performed for 1 hour, an aqueous solution of potassium iodide (1.5 eq.) was added in ice bath, and the reaction solution was slowly warmed to room temperature, and subjected to a reaction for 16 hours. The pH of reaction solution was adjusted to 7 by adding the saturated solution of sodium bicarbonate. The reaction solution after pH adjustment was extracted with ethyl acetate, dried with anhydrous sodium sulfate, subjected to a rotary evaporation to dryness, and separated by silica gel column chromatography to obtain an iodo-intermediate (yield 20-50%). The iodo-intermediate and aromatic boronic acid (ArB(OH)₂) (1.5 eq.) were dissolved in tetrahydrofuran-water, and then a catalyst Pd(dppf)Cl₂ (0.1 eq.) and K₂CO₃ (3 eq.) were added, followed by heating to 90°C and performing a reaction for 16 hours. After the reaction was completed, a rotary evaporation was performed for dryness, and the residue was dissolved in ethyl acetate, washed twice with saturated brine, dried with anhydrous sodium sulfate, subjected to rotary evaporation for dryness, and separated by chromatography, so as to obtain compound G43. The reaction steps of preparing G44 by hydrolysis were the same as described above (omitted).

### General Experimental Operation V:

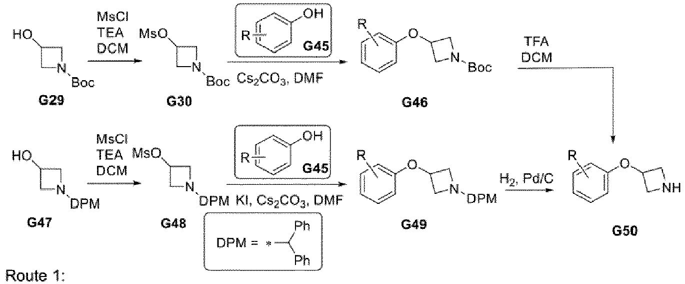

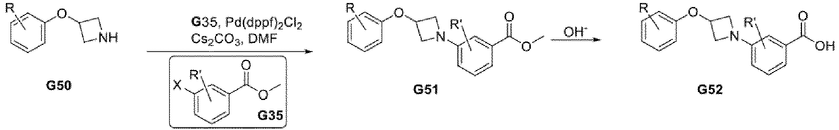

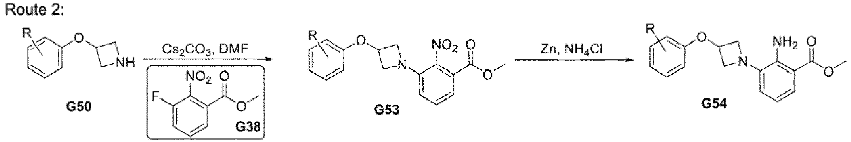

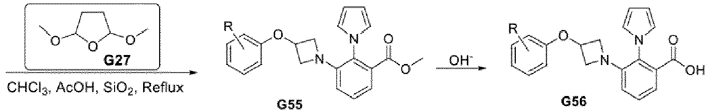

The above synthetic routes were implemented by changing the form of substituents of G45 using the reaction routes shown in "General Experimental Operation IV", and the experimental operations were omitted.

### General Experimental Operation VI:

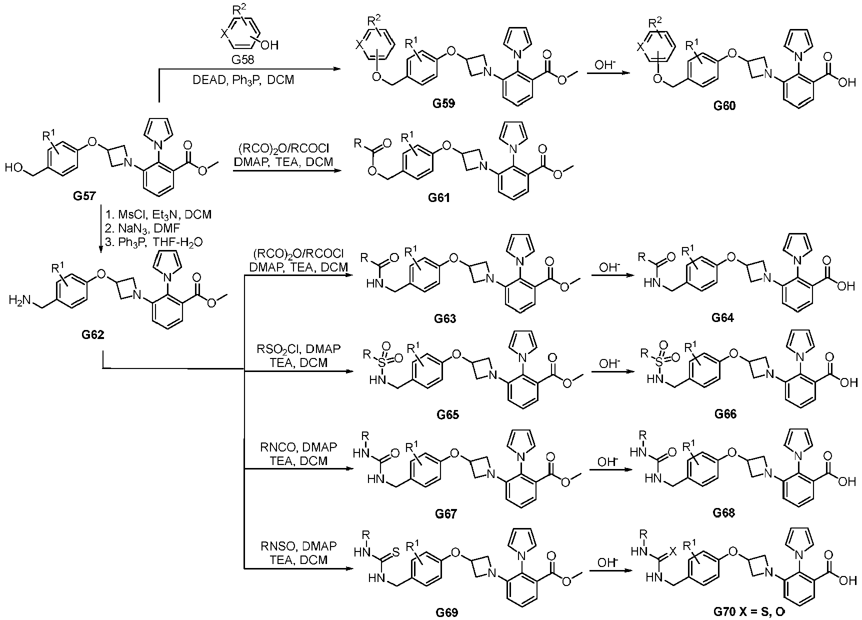

Compound G57 (1 eq.) and phenolic compound G58 (1.2 eq.) were dissolved in dichloromethane, triphenylphosphine (2.2 eq.) and diethyl azodicarboxylate (DEAD) (2.0 eq.) were added under nitrogen protection and ice bath cooling. A reaction was performed overnight at room temperature. After adding water to quench the reaction, the solvent was removed by rotary evaporation under reduced pressure, and the residue was extracted with a large amount of ethyl acetate. The resulting organic phase was washed three times with pure water, dried with anhydrous sodium sulfate, filtered, concentrated, and separated by silica gel column chromatography or thin layer preparative chromatography, so as to obtain a G59 intermediate.

The esterification operations of the compound G57 were the same as described above (omitted).

The operations of the compound G57 to obtain an amino intermediate G62 through azidation-reduction reaction and the subsequent reactions with electrophile were the same as described above.

The hydrolysis reaction operations were the same as described above (omitted). It should be noted that some thiourea compound G69 was unstable and may partially decompose into the urea structure during hydrolysis.

### General Experimental Operation VII:

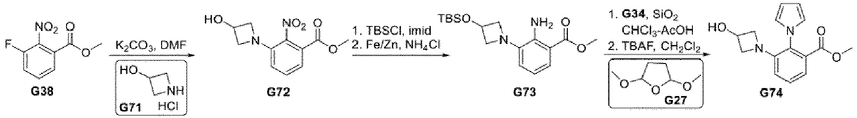

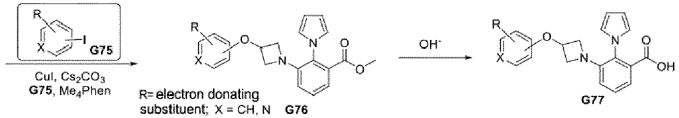

Compound G71 (1 eq.) and Compound G38 (1 eq.) were dissolved in DMF. Potassium carbonate (4 eq.) was added, followed by heating to 60°C, and performing a reaction for 3 hours. After the reaction was completed, the reaction was quenched with water, and the reaction solution was extracted with dichloromethane, and the resulting organic phase was washed with saturated brine, dried with anhydrous sodium sulfate and concentrated. The residue was purified by silica gel column chromatography, so as to obtain G72 (yield 89%). G72 was dissolved in dichloromethane, imidazole (2 eq.) and TBSCl (1.5 eq.) were added, and a reaction was performed under stirring at room temperature for 1 hour, and quenched with water. The quenched reaction solution was extracted with dichloromethane. The resulting organic phase was washed with saturated brine, dried with anhydrous sodium sulfate, and concentrated; and the residue was purified by silica gel column chromatography, so as to obtain a silyl ether intermediate (90% yield). The silyl ether intermediate was dissolved in ethanol, reductant iron powder (7.5 eq.) was added, and then the saturated solution of ammonium chloride was added, and a reaction was performed under refluxing at 75°C for 1 hour. After the completion of the reaction, the solid was removed by filtration and was extracted with dichloromethane. The resulting organic phase was dried with anhydrous sodium sulfate and concentrated, and the residue was separated by silica gel column chromatography, so as to obtain G73 (yield 71%).

The synthesis of pyrrole was the same as described above (omitted). The intermediate for forming a pyrrole ring was dissolved in dichloromethane, and tetra-tert-butylammonium fluoride (TBAF, 1.2 eq.) was added under ice bath cooling. After performing a reaction at room temperature for 1 hour, the reaction solution was diluted with a large amount of dichloromethane, washed with saturated brine, dried with anhydrous sodium sulfate and concentrated. The residue was separated by silica gel column chromatography, so as to obtain G74 (90% yield).

Compound G74 (1 eq.) was dissolved in toluene, then cuprous iodide (0.15 eq.), a ligand 3,4,7,8-tetramethyl-1,10-phenanthroline (0.3 eq.) and cesium carbonate (2 eq.) were added. The replacement system is an inert nitrogen environment. After adding iodide G75 (1 eq.), a reaction was performed under stirring at 110°C for 12 hours. After the completion of the reaction, the solvent was removed by rotary evaporation, and G76 was obtained through separation by preparative thin layer chromatography (the yield as shown in examples).

The steps of hydrolysis to obtain G77 were the same as described above (omitted).

### General Experimental Operation VIII:

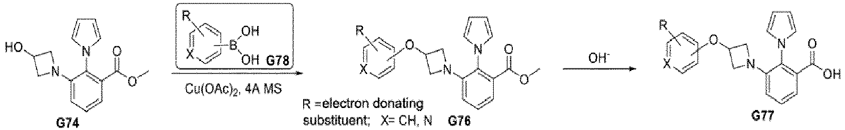

The synthesis of the compound G74 was the same as described above.

The compound G74 (1 eq.), boronic acid compound G78 (3 eq.), anhydrous copper acetate (0.3 eq.) and 4A molecular sieve of 40-60 mesh were dissolved in dichloromethane; the oxygen environment was replaced, triethylamine was added and a reaction was performed under stirring at 35°C for 12 hours. After the filtration and concentration of the reaction solution, the residue was separated by thin layer preparative chromatography, so as to obtain the product G76 (the yield as shown in examples).

The steps of hydrolysis to obtain G77 were the same as described above (omitted).

### General Experimental Operation IX:

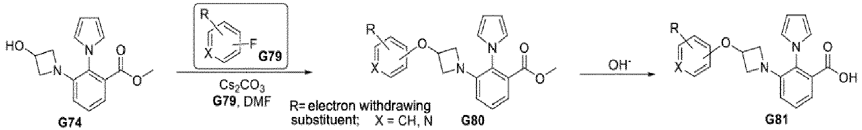

The synthesis of the compound G74 was the same as described above.

Compound G74 (1 eq.), fluorinated compound G79 (2 eq.) and anhydrous cesium carbonate (2 eq.) were dissolved in DMF, and heated to 100°C, and subjected to a reaction for 12 hours. After the reaction was completed, the reaction solution was filtered and concentrated, and the residue was separated by the preparative thin layer chromatography, so as to obtain a product G80 (the yield as shown in examples).

The steps of hydrolysis to obtain G81 were the same as described above (omitted).

### General Experimental Operation X:

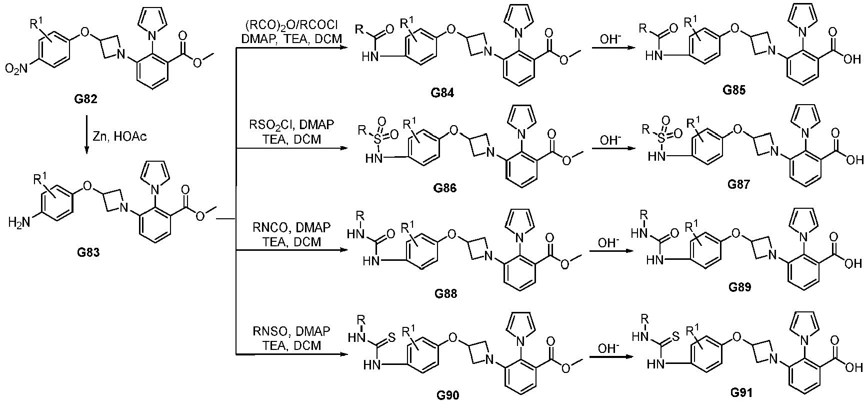

The nitro reduction operation was the same as described above (omitted).

Operations of coupling with an electrophile to form an amide were the same as described above (omitted).

The hydrolysis reaction operations were the same as described above (omitted). It should be noted that some thiourea compound was unstable and may decompose into the urea structure during hydrolysis.

### General Experimental Operation XI:

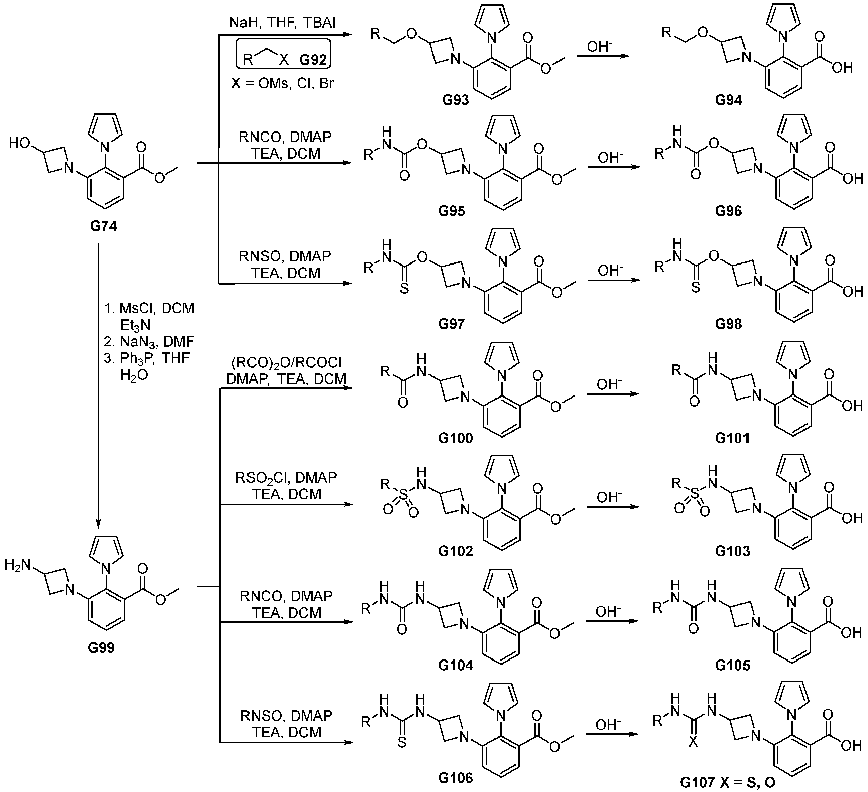

The compound G74 (1 eq.) was dissolved in dry tetrahydrofuran, sodium hydride (1.5 eq.) was added under ice bath cooling, subsequently, compound G92 (1.2 eq.) and a catalytic amount of tetrabutylammonium iodide (0.2 eq.) were added. After a reaction was performed at room temperature for 1 hour, the reaction was quenched with ice water and ethyl acetate was added for extaction. The resulting organic phase was washed with the saturated aqueous solution of ammonium chloride, dried with anhydrous sodium sulfate and concentrated. The residue was purified by silica gel column chromatography to obtain a compound G93 (the yield as shown in examples). The operations of the hydrolysis to obtain the compound G94 were the same as described above (omitted).

The operations of the reaction of the compound G74 with an electrophile such as isocyanate and thioisocyanate were the same as described above (omitted).

The operations of the conversion of the compound G74 to azide after activation by methylsulfonyl and of its reduction reaction were the same as described above (omitted).

The operations of the reaction of the compound G99 coupling with an electrophile to obtain an amide product were the same as described above (omitted).

The hydrolysis reaction operations were the same as described above (omitted). It should be noted that some thiourea compound G106 was unstable and may decompose into the urea structure during hydrolysis.

### General Experimental Operation XII:

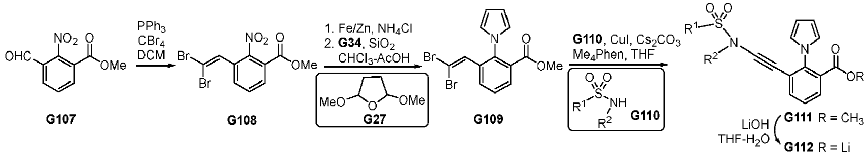

G107 (1 eq.) and carbon tetrabromide (2 eq.) were dissolved in dichloromethane, and a solution of triphenylphosphine (4 eq.) in dichloromethane was added dropwise at 0°C. After performing a reaction at room temperature for 0.5 hours, the reaction solution was diluted with dichloromethane, and the reaction was quenched with water; the organic phase was separated, washed with water once, dried with anhydrous sodium sulfate, and concentrated to dryness. The residue was separated by silica gel column chromatography to obtain G108 (95% yield).

The operations of nitro reduction and the synthesis of pyrrole were the same as described above (omitted).

Sulfonamide G110 (1 eq.) and G109 (1 eq.) were dissolved in tetrahydrofuran, cuprous iodide (0.1 eq.), cesium carbonate (3 eq.), and a ligand Me4Phen (0.2 eq.) were added, and subjected to stirring at room temperature for 2 hours, and then water was added to quench a reaction. After extraction with dichloromethane, the resulting organic phase was dried with anhydrous sodium sulfate and concentrated to dryness. The residue was separated by silica gel column chromatography to obtain a target compound G111 (the yield as shown in examples).

The hydrolysis reaction operations were the same as described above (omitted). In some examples, a concentration step was directly performed without acidification steps to obtain the lithium salt form of carboxylic acid G112 (the yield as shown in examples).

### General Experimental Operation XIII:

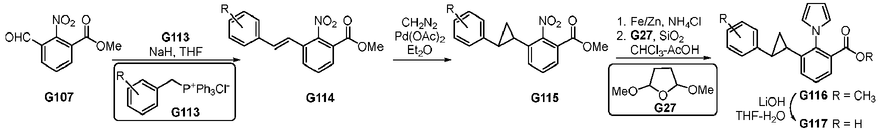

G113 (1 eq.) was dissolved in tetrahydrofuran, sodium hydride (1.5 eq.) was added at 0 °C, and then a solution of G107 (1 eq.) in THF was added dropwise after stirring at room temperature for 0.5 hours. After a rection was performed overnight at room temperature, the reaction was quenched with water and dichloromethane was used for extraction. The resulting organic phase was washed once with water, dried with anhydrous sodium sulfate and concentrated to dryness. The residue was separated by silica gel column chromatography to obtain G114 (yield 85%).

Alkene G114 (1 eq.) was dissolved in ether, palladium acetate (0.5 eq.) and freshly prepared diazomethane (10 eq., 1.0 mol/L solution; note: for a substance with low boiling point and high toxicity, please refer to its physical data for protection and careful operation) were added at 0°C. A reaction was performed at 0°C for 0.5 hours, and quenched with an excess of dilute acetic acid (0.1 mol/L aqueous solution). Dichloromethane was used for exatraction. The resulting organic phase was washed once with water, dried with anhydrous sodium sulfate and concentrated to dryness. The residue was separated by silica gel column chromatography to obtain G115 (yield 80%).

The operations of nitro reduction and the synthesis of pyrrole were the same as described above (omitted).

The hydrolysis reaction operations were the same as described above (omitted).

### General Experimental Operation XIV:

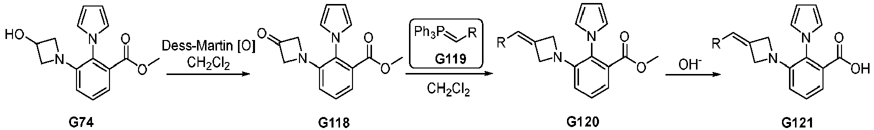

The compound G74 (1 eq.) was dissolved in dichloromethane, and Dess-Martin reagent (3 eq.) was added under ice bath cooling, and subjected to a reaction for 1 hour. After the reaction was monitored to be complete by thin layer chromatography, then, the reaction was quenched by adding the saturated aqueous solution of ammonium chloride, and the quenched reaction solution was extracted with ethyl acetate. The resulting organic phase was washed with water, dried with anhydrous sodium sulfate and concentrated to dryness. The residue was separated by silica gel column chromatography to obtain a ketone G118 (90.9% yield). The ketone G118 (1 eq.) was dissolved in dichloromethane, and Wittig reagent G119 (1.2 eq.) was added under ice bath cooling. A reaction was performed under stirring at room temperature and quenched with water after monitoring the completion of the reaction by TLC. The quenched reaction solution was extracted with dichloromethane, dried with anhydrous sodium sulfate and concentrated to dryness. The residue was separated by silica gel column chromatography to obtain G120 (the yield as shown in examples).

The hydrolysis operations of preparing G121 were the same as described above (omitted).

### General Experimental Operation XV:

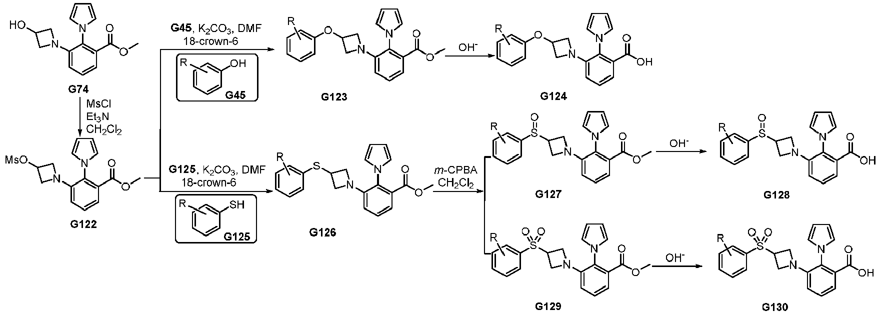

The compound G74 (1 eq.) was dissolved in dimethylformamide after activation with methanesulfonate, and anhydrous potassium carbonate (2 eq.) and substituted thiophenol G125 (1.5 eq.) and 18-crown-6 (0.1 eq.) were added and subjected to performing a reaction overnight at room temperature. After quenching the reaction with water, the quenched reaction solution was extracted with ethyl acetate. The resulting organic phase was washed with water, dried with anhydrous sodium sulfate and concentrated to dryness. The residue was separated by preparative thin layer chromatography to obtain compound G126 (the yield as shown in examples). The compound G126 (1 eq.) was dissolved in dichloromethane, m-chloroperoxybenzoic acid (mCPBA) (2 eq.) was added dropwise under ice bath cooling. After 5 minutes of reaction, sodium sulfite was added to remove excess mCPBA. The reaction solution was extracted with dichloromethane, washed with water, dried with anhydrous sodium sulfate, and concentrated to dryness under reduced pressure. The residue was separated by preparative thin layer chromatography to obtain G127 and G129 (the yield as shown in examples).

G123, G127, G129 were hydrolyzed to obtain corresponding carboxylic acid, respectively, where the hydrolysis reaction operations were the same as described above (omitted).

### General Experimental Operation XVI:

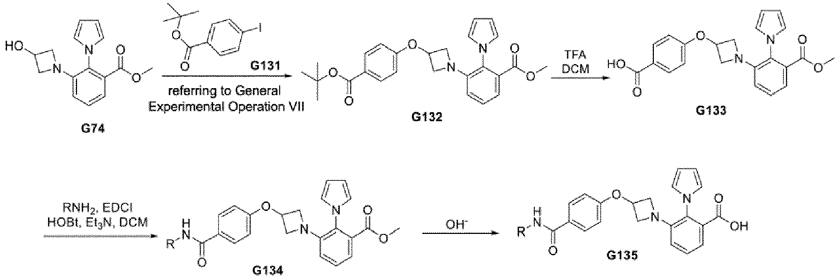

The coupling reaction of the compound G74 (1 eq.) with tert-butyl 4-iodobenzoate (G131) was conducted with reference to General Experimental Operation VII, so as to prepare compound G132.

The compound G132 (1 eq.) was dissolved in dichloromethane, trifluoroacetic acid (3 eq.) was added, and a reaction was reacting at room temperature for 1 hour. After rotary evaporation to dryness, a white solid carboxylic acid was obtained.

The carboxylic acid (1 eq.), aniline (1.2 eq.), 1-ethyl-(3-dimethylaminopropyl) carbodiimide hydrochloride (1.5 eq.), 1-hydroxybenzotriazole (1.5 eq.), and triethylamine (3 eq.) were reacted in dichloromethane (2 ml) overnight at room temperature. Dichloromethane and dilute hydrochloric acid solution (1 mol/L) were added for extraction, and the resulting organic phase was dried by rotary evaporation and passed through a column to obtain G134 (60%, yield).

The G134 was hydrolyzed to obtain the corresponding carboxylic acid G135, where the hydrolysis reaction operations were the same as described above (omitted).

### Example

### Example 1:

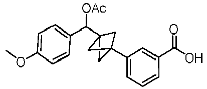

Compound 1 was synthesized according to the General Experimental Operation I (24mg, 40%). ¹H NMR (400 MHz, Chloroform-*d*) *δ* 7.99-7.92 (m, 1H), 7.92-7.88 (m, 1H), 7.44-7.35 (m, 2H), 7.28-7.20 (m, 2H), 6.97-6.85 (m, 2H), 5.85 (s, 1H), 3.82 (s, 3H), 2.14 (s, 3H), 2.05-1.85 (m, 6H) ppm; High resolution mass spectrum (mass-to-charge ratio): C₂₂H₂₂NaO₅⁺ [M+Na]⁺ Calculated value: 389.1359, Measured value: 389.1357.

### Example 2:

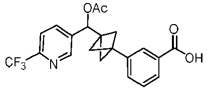

Compound 2 was synthesized according to the General Experimental Operation I (17mg, 48%). ¹H NMR (500 MHz, Chloroform-*d*) *δ* 8.70 (d, *J* = 2.0 Hz, 1H), 7.98-7.94 (m, 1H), 7.89 (s, 1H), 7.85-7.79 (m, 1H), 7.76-7.62 (m, 1H), 7.43-7.35 (m, 2H), 5.98 (s, 1H), 2.19 (s, 3H), 2.03-1.97 (m, 6H) ppm; High resolution mass spectrum (mass-to-charge ratio): C₂₁H₁₉F₃NO₄⁺ [M+H]⁺ Calculated value: 406.1261, Measured value: 406.1253.

### Example 3:

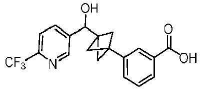

Compound 3 was synthesized according to the General Experimental Operation I (5.3mg, 65%). ¹H NMR (400 MHz, Methanol-*d*₄) δ 8.67 (d, *J* = 2.0 Hz, 1H), 8.08-7.97 (m, 1H), 7.91-7.74 (m, 3H), 7.52-7.19 (m, 2H), 4.95 (s, 1H), 2.08-1.82 (m, 6H) ppm; High resolution mass spectrum (mass-to-charge ratio): C₁₉H₁₇F₃NO₃⁺ [M+H]⁺ Calculated value: 364.1155, Measured value: 364.1151.

### Example 4:

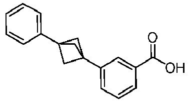

Compound 4 was synthesized according to the General Experimental Operation II (1.5mg, yield 50%). ¹H NMR (400 MHz, Chloroform-*d*) δ 8.02-7.94 (m, 2H), 7.53 (d, *J* = 7.7 Hz, 2H), 7.43 (s, 1H), 7.37-7.28 (m, 4H), 2.37 (s, 6H) ppm; High resolution mass spectrum (mass-to-charge ratio): C₁₈H₁₅O₂⁻ [M-H]⁻ Calculated value: 263.1078, Measured value: 263.1077.

### Example 5:

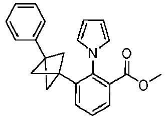

Compound 5 was synthesized according to the General Experimental Operation II (10mg, 65%). ¹H NMR (300 MHz, Chloroform-*d*) δ 7.70 (dd, *J* = 7.5, 1.8 Hz, 1H), 7.57-7.39 (m, 3H), 7.24-7.11 (m, 4H), 6.68-6.66 (m, 2H), 6.28-6.25 (m, 2H), 3.64 (s, 3H), 2.08 (s, 6H).High resolution mass spectrum (mass-to-charge ratio): C₂₃H₂₂NO₂⁺ [M+H]⁺ Calculated value: 344.1645, Measured value: 344.1638.

### Example 6:

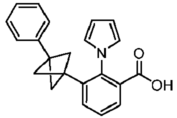

Compound 6 was synthesized according to the General Experimental Operation II (1.0mg, yield 50%). ¹H NMR (500 MHz, Chloroform-*d*) δ 7.84 (dd, *J* = 7.7, 1.7 Hz, 1H), 7.55 (dd, *J* = 7.7, 1.6 Hz, 1H), 7.48 (dd, *J* = 8.6, 6.9 Hz, 1H), 7.28 (d, *J* = 7.3 Hz, 1H), 7.25-7.14 (m, 4H), 6.70 (t, *J* = 2.1 Hz, 2H), 6.29 (t, *J* = 2.1 Hz, 2H), 2.06 (s, 6H). High resolution mass spectrum (mass-to-charge ratio): C₂₂H₁₉NNaO₂⁺ [M+Na]⁺ Calculated value: 352.1308, Measured value: 352.1293.

### Example 7:

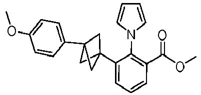

Compound 7 was synthesized according to the General Experimental Operation II (5mg, 72%). ¹H NMR (400 MHz, Chloroform-*d*) δ 7.69 (dd, *J* = 7.6, 1.7 Hz, 1H), 7.50 (dd, *J* = 7.7, 1.8 Hz, 1H), 7.43 (t, *J* = 7.7 Hz, 1H), 7.08 (d, *J* = 8.7 Hz, 2H), 6.87-6.77 (m, 2H), 6.67 (t, *J* = 2.1 Hz, 2H), 6.26 (t, *J* = 2.1 Hz, 2H), 3.77 (s, 3H), 3.63 (s, 3H), 2.04 (s, 6H). High resolution mass spectrum (mass-to-charge ratio): C₂₄H₂₄NO₃⁺ [M+H]⁺ Calculated value: 374. 175 1 Measured value: 374.1731.

### Example 8:

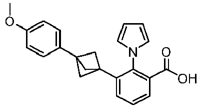

Compound 8 was synthesized according to the General Experimental Operation II (1.0mg, yield 50%). ¹H NMR (300 MHz, Chloroform-*d*) δ 7.72 (brs, 1H), 7.46 (brs, 2H), 7.08 (d, *J* = 8.0 Hz, 2H), 6.81 (d, *J* = 8.0 Hz, 2H), 6.67 (brs, 2H), 6.22 (brs, 2H), 3.78 (s, 3H), 2.02 (s, 6H). High resolution mass spectrum (mass-to-charge ratio): C₂₃H₂₁NNaO₃⁺ [M+Na]⁺ Calculated value: 382.1414, Measured value: 382.141.

### Example 9:

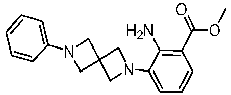

Compound 9 was synthesized according to the General Experimental Operation III (20mg, yield 52%). ¹H NMR (400 MHz, Chloroform-*d*) *δ* 7.55 (dd, *J* = 8.1, 1.4 Hz, 1H), 7.28-7.20 (m, 2H), 6.85-6.73 (m, 2H), 6.66 (t, *J* = 7.9 Hz, 1H), 6.57-6.41 (m, 2H), 5.74 (s, 2H), 4.06 (s, 4H), 3.98 (s, 4H), 3.88 (s, 3H). High resolution mass spectrum (mass-to-charge ratio): C₁₉H₂₂N₃O₂⁺ [M+H]⁺ Calculated value: 324.1707, Measured value: 324.1703.

### Example 10:

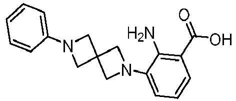

Compound 10 was synthesized according to the General Experimental Operation III (4.5mg, yield 78%). ¹H NMR (400 MHz, Methanol-*d*₄) δ 7.50 (dd, *J =* 8.0, 1.4 Hz, 1H), 7.19 (dd, *J* = 8.5, 7.3 Hz, 2H), 6.82-6.69 (m, 2H), 6.61 (t, *J* = 7.9 Hz, 1H), 6.57-6.42 (m, 2H), 4.01 (s, 4H), 3.96 (s, 4H). High resolution mass spectrum (mass-to-charge ratio): C₁₈H₂₀N₃O₂⁺ [M+H]⁺ Calculated value: 310.1550, Measured value: 310.1546.

### Example 11:

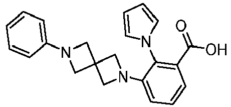

Compound 11 was synthesized according to the General Experimental Operation III (1mg, yield 20%). ¹H NMR (400 MHz, Methanol-*d*₄) δ 7.28 (t, *J* = 7.8 Hz, 1H), 7.19-7.10 (m, 2H), 6.99 (d, *J* = 7.6 Hz, 1H), 6.81-6.63 (m, 4H), 6.51-6.36 (m, 2H), 6.20-6.08 (m, 2H), 3.82 (s, 4H), 3.59 (s, 4H). High resolution mass spectrum (mass-to-charge ratio): C₂₂H₂₁N₃NaO₂⁺ [M+Na]⁺ Calculated value: 382.1526, Measured value: 382.1528.

### Example 12:

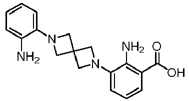

Compound 12 was synthesized according to the General Experimental Operation III (3.2mg, yield 47%). ¹H NMR (400 MHz, Methanol-*d*₄) δ 7.43-7.36 (m, 1H), 6.92-6.85 (m, 1H), 6.75 (ddd, *J* = 9.0, 7.6, 1.5 Hz, 2H), 6.71-6.64 (m, 1H), 6.63-6.55 (m, 2H), 3.92 (s, 4H), 3.39-3.33 (m, 4H). High resolution mass spectrum (mass-to-charge ratio): C₁₈H₂₁N₄O₂⁺ [M+H]⁺ Calculated value: 325.1659, Measured value: 325.1646.

### Example 13:

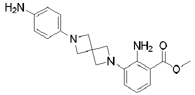

Compound 13 was synthesized according to the General Experimental Operation III (4.4mg, yield 32%). ¹H NMR (300 MHz, Methanol-*d*₄) δ 7.65-7.55 (m, 1H), 7.38-7.16 (m, 4H), 6.69-6.51 (m, 2H), 4.08 (s, 2H), 3.97 (s, 3H), 3.93-3.80 (m, 4H), 3.67 (s, 2H). High resolution mass spectrum (mass-to-charge ratio): C₁₉H₂₂N₄O₂⁺ [M+H]⁺ Calculated value: 339.1816, Measured value: 325.1818.

### Example 14:

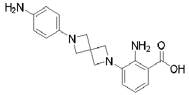

Compound 14 was synthesized according to the General Experimental Operation III (1.4mg, yield 35%). ¹H NMR (300 MHz, Methanol-*d*₄) δ 7.64-7.54 (m, 1H), 7.33-7.15 (m, 4H), 6.66-6.54 (m, 2H), 4.06 (s, 2H), 3.96-3.77 (m, 4H), 3.66 (s, 2H). High resolution mass spectrum (mass-to-charge ratio): C₁₈H₁₉N₄O₂⁻ [M-H]⁻ Calculated value: 323.1513, Measured value: 323.1511.

### Example 15:

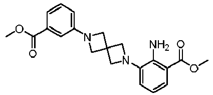

Compound 15 was synthesized according to the General Experimental Operation III (2.4mg, yield 45%). ¹H NMR (300 MHz, Chloroform-*d*) δ 7.62-7.51 (m, 1H), 7.47-7.40 (m, 1H), 7.33-7.24 (m, 1H), 7.13 (t, *J* = 2.0 Hz, 1H), 6.80 (dd, *J* = 7.7, 1.5 Hz, 1H), 6.72-6.56 (m, 2H), 4.09 (s, 4H), 3.98 (s, 4H), 3.91 (s, 3H), 3.87 (s, 3H). High resolution mass spectrum (mass-to-charge ratio): C₂₁H₂₄N₃O₄⁺ [M+H]⁺ Calculated value: 382.1761, Measured value: 382.1752.

### Example 16:

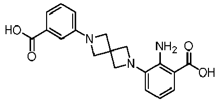

Compound 16 was synthesized according to the General Experimental Operation III (2mg, yield 36%). ¹H NMR (400 MHz, Methanol-*d*₄) δ 7.50 (dd, *J* = 8.0, 1.4 Hz, 1H), 7.38 (d, *J* = 7.7 Hz, 1H), 7.24 (t, *J* = 7.8 Hz, 1H), 7.19-7.13 (m, 1H), 6.83-6.76 (m, 1H), 6.69-6.65 (m, 1H), 6.63-6.56 (m, 1H), 4.06 (s, 4H), 3.98 (s, 4H). High resolution mass spectrum (mass-to-charge ratio): C₁₉H₂₀N₃O₄⁺ [M+H]⁺ Calculated value: 354.1448, Measured value: 354.1440.

### Example 17:

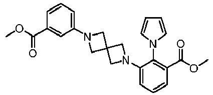

Compound 17 was synthesized according to the General Experimental Operation III (10mg, yield 70%). ¹H NMR (300 MHz, Chloroform-*d*) *δ* 7.46-7.37 (m, 1H), 7.34-7.21 (m, 2H), 7.14 (dd, *J* = 7.7, 1.4 Hz, 1H), 7.06 (t, *J* = 2.0 Hz, 1H), 6.70 (t, *J* = 2.1 Hz, 2H), 6.66 (dd, *J* = 8.2, 1.5 Hz, 1H), 6.61-6.55 (m, 1H), 6.24 (t, *J* = 2.1 Hz, 2H), 3.94 (s, 4H), 3.89 (s, 3H), 3.66 (s, 4H), 3.62 (s, 3H). High resolution mass spectrum (mass-to-charge ratio): C₂₅H₂₆N₃O₄⁺ [M+H]⁺ Calculated value: 432.1918, Measured value: 432.1917.

### Example 18:

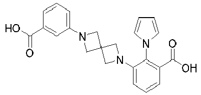

Compound 18 was synthesized according to the General Experimental Operation III (3.1mg, yield 41%). ¹H NMR (300 MHz, Methanol-*d*₄) δ 7.42-7.30 (m, 1H), 7.26-7.14 (m, 2H), 7.08 (s, 1H), 6.85 (d, *J* = 7.4 Hz, 1H), 6.78 (t, *J* = 2.1 Hz, 2H), 6.66-6.50 (m, 2H), 6.14 (t, *J* = 1.9 Hz, 2H), 3.86 (s, 4H), 3.56 (s, 4H). High resolution mass spectrum (mass-to-charge ratio): C₂₃H₂₂N₃O₄⁺ [M+H]⁺ Calculated value: 404.1605, Measured value: 404.1608.

### Example 19:

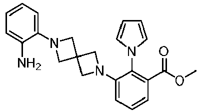

Compound 19 was synthesized according to the General Experimental Operation III (10mg, yield 65%). ¹H NMR (400 MHz, Chloroform-*d*) δ 7.34-7.27 (m, 1H), 7.13 (dd, *J* = 7.7, 1.4 Hz, 1H), 6.82-6.73 (m, 2H), 6.72-6.69 (m, 2H), 6.68-6.61 (m, 2H), 6.55 (dd, *J* = 7.6, 1.7 Hz, 1H), 6.26 (t, *J* = 2.1 Hz, 2H), 3.86 (s, 4H), 3.66 (s, 4H), 3.62 (s, 3H). High resolution mass spectrum (mass-to-charge ratio): C₂₃H₂₅N_{4O2}⁺ [M+H]⁺ Calculated value: 389.1972, Measured value: 389.1971.

### Example 20:

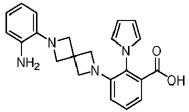

Compound 20 was synthesized according to the General Experimental Operation III (4mg, yield 42%). ¹H NMR (300 MHz, Methanol-*d*₄) δ 7.29 (t, *J* = 7.9 Hz, 1H), 7.01 (dd, *J* = 7.6, 1.4 Hz, 1H), 6.77-6.66 (m, 6H), 6.61-6.53 (m, 1H), 6.18 (t, *J* = 2.1 Hz, 2H), 3.80 (s, 4H), 3.61 (s, 4H). High resolution mass spectrum (mass-to-charge ratio): C₂₂H₂₃N₄O₂⁺ [M+H]⁺ Calculated value: 375.1816, Measured value: 375.1812.

### Example 21:

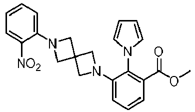

Compound 21 was synthesized according to the General Experimental Operation III (17mg, 99%). ¹H NMR (300 MHz, Chloroform-*d*) δ 7.85-7.76 (m, 1H), 7.42-7.28 (m, 2H), 7.15 (dd, *J* = 7.6, 1.4 Hz, 1H), 6.83-6.73 (m, 1H), 6.72-6.59 (m, 3H), 6.56 (d, *J* = 8.4 Hz, 1H), 6.24 (t, *J* = 2.1 Hz, 2H), 4.01 (s, 4H), 3.64 (s, 4H), 3.61 (s, 3H). High resolution mass spectrum (mass-to-charge ratio): C₂₃H₂₃N₄O₄⁺ [M+H]⁺ Calculated value: 419.1714, Measured value: 419.1718.

### Example 22:

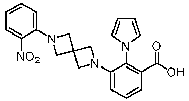

Compound 22 was synthesized according to the General Experimental Operation III (2.9mg, yield 95%). ¹H NMR (300 MHz, Chloroform-*d*) δ 7.80 (dd, *J* = 8.3, 1.6 Hz, 1H), 7.43-7.28 (m, 3H), 6.82-6.61 (m, 4H), 6.60-6.49 (m, 1H), 6.23 (s, 2H), 4.00 (s, 4H), 3.62 (s, 4H). High resolution mass spectrum (mass-to-charge ratio): C₂₂H₂₁N₄O₄⁺ [M+H]⁺ Calculated value: 405.1557, Measured value: 405.1561.

### Example 23:

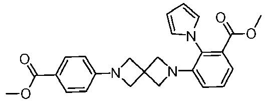

Compound 23 was synthesized according to the General Experimental Operation III (14mg, yield 66%). ¹H NMR (300 MHz, Chloroform-*d*) δ 7.92-7.80 (m, 2H), 7.30 (t, *J* = 7.9 Hz, 1H), 7.15 (dd, *J* = 7.7, 1.4 Hz, 1H), 6.73-6.58 (m, 3H), 6.38-6.30 (m, 2H), 6.25 (t, *J* = 2.1 Hz, 2H), 3.99 (s, 4H), 3.85 (s, 3H), 3.66 (s, 4H), 3.62 (s, 3H). High resolution mass spectrum (mass-to-charge ratio): C₂₅H₂₅N₃NaO₄⁺ [M+Na]⁺ Calculated value: 454.1737, Measured value: 454.1741.

### Example 24:

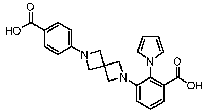

Compound 24 was synthesized according to the General Experimental Operation III (2.1mg, yield 25%). ¹H NMR (300 MHz, Methanol-*d*₄) δ 7.86-7.78 (m, 2H), 7.26-7.18 (m, 1H), 6.90-6.83 (m, 1H), 6.77 (t, *J* = 2.0 Hz, 2H), 6.58 (d, *J* = 8.5 Hz, 1H), 6.40 (d, *J* = 8.5 Hz, 2H), 6.19-6.11 (m, 2H), 3.94 (s, 4H), 3.57 (s, 4H). High resolution mass spectrum (mass-to-charge ratio): C₂₃H₂₁N₃NaO₄⁺ [M+Na]⁺ Calculated value: 426.1424, Measured value: 426.1423.

### Example 25:

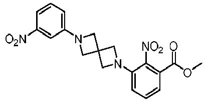

Compound 25 was synthesized according to the General Experimental Operation III (18mg, yield 22%). ¹H NMR (300 MHz, Chloroform-*d*) δ 7.63-7.54 (m, 1H), 7.40-7.29 (m, 2H), 7.28-7.18 (m, 2H), 6.85-6.54 (m, 2H), 4.17 (s, 4H), 4.10 (s, 4H), 3.87 (s, 3H). High resolution mass spectrum (mass-to-charge ratio): C₁₉H₁₉N₄O₆⁺ [M+H]⁺ Calculated value: 399.1299, Measured value: 399.1300.

### Example 26:

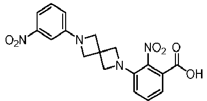

Compound 26 was synthesized according to the General Experimental Operation III (5mg, yield 78%). ¹H NMR (300 MHz, Methanol-*d*₄) δ 7.57-7.49 (m, 1H), 7.43-7.31 (m, 2H), 7.26-7.22 (m, 1H), 7.13-7.04 (m, 1H), 6.84 (dd, *J* = 8.0, 2.3 Hz, 1H), 6.77 (d, *J* = 8.3 Hz, 1H), 4.12 (s, 4H), 4.10 (s, 4H). High resolution mass spectrum (mass-to-charge ratio): C₁₈H₁₅N₄O₆⁻[M-H]⁻ Calculated value: 383.0997, Measured value: 383.1047.

### Example 27:

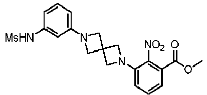

Compound 27 was synthesized according to the General Experimental Operation III (4mg, yield 21%). ¹H NMR (300 MHz, Chloroform-*d*) δ 7.38-7.22 (m, 2H), 7.20-7.15 (m, 2H), 6.78-6.67 (m, 1H), 6.57-6.50 (m, 1H), 6.36-6.23 (m, 2H), 4.15 (s, 4H), 4.03 (s, 4H), 3.87 (s, 3H), 3.00 (s, 3H). High resolution mass spectrum (mass-to-charge ratio): C₂₀H₂₂N₄NaO₆S⁺ [M+Na]⁺ Calculated value: 469.1152, Measured value: 469.1151.

### Example 28:

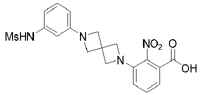

Compound 28 was synthesized according to the General Experimental Operation III (3.5mg, yield 95%)¹H NMR (300 MHz, Methanol-*d*₄) δ 7.36-7.27 (m, 1H), 7.19-7.09 (m, 1H), 7.06-6.96 (m, 1H), 6.72-6.67 (m, 1H), 6.65-6.58 (m, 1H), 6.38 (t, *J* = 2.2 Hz, 1H), 6.33-6.23 (m, 1H), 4.07 (s, 4H), 3.98 (s, 4H), 2.93 (s, 3H). High resolution mass spectrum (mass-to-charge ratio): C₁₉H₂₀N₄NaO₆S⁺ [M+Na]⁺ Calculated value: 455.0996, Measured value: 455.0993.

### Example 29:

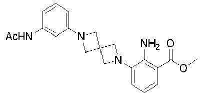

Compound 29 was synthesized according to the General Experimental Operation III (4mg, yield 36%). ¹H NMR (300 MHz, Methanol-*d*₄) δ 7.47 (dd, *J* = 8.2, 1.4 Hz, 1H), 7.12 (t, *J* = 7.9 Hz, 1H), 6.90-6.77 (m, 3H), 6.60 (t, *J* = 7.9 Hz, 1H), 6.34-6.14 (m, 1H), 4.01 (s, 4H), 3.96 (s, 4H), 3.84 (s, 3H), 2.10 (s, 3H). High resolution mass spectrum (mass-to-charge ratio): C₂₁H₂₅N₄O₃⁺ [M+H]⁺ Calculated value: 381.1921, Measured value: 381.1920.

### Example 30:

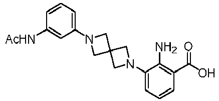

Compound 30 was synthesized according to the General Experimental Operation III (1.9mg, yield 95%). ¹H NMR (300 MHz, Methanol-*d*₄) δ 7.37 (d, *J* = 8.0 Hz, 1H), 7.15-7.07 (m, 1H), 6.88-6.77 (m, 3H), 6.61 (t, *J* = 7.8 Hz, 1H), 6.31-6.18 (m, 1H), 4.05 (s, 2H), 3.81-3.70 (m, 4H), 3.50 (s, 2H), 2.09 (s, 3H). High resolution mass spectrum (mass-to-charge ratio): C_{2O}H₂₃N₄O₃⁺ [M+H]⁺ Calculated value: 367.1765, Measured value: 367.1768.

### Example 31:

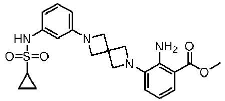

Compound 31 was synthesized according to the General Experimental Operation III (3mg, yield 30%). ¹H NMR (300 MHz, Methanol-*d*₄) δ 7.38-7.30 (m, 1H), 7.15-7.09 (m, 1H), 6.90-6.83 (m, 1H), 6.67-6.55 (m, 2H), 6.42 (t, *J* = 2.1 Hz, 1H), 6.33-6.23 (m, 1H), 4.05 (s, 2H), 3.81-3.69 (m, 4H), 3.50 (s, 2H), 2.60-2.45 (m, 1H), 1.09-0.82 (m, 4H). High resolution mass spectrum (mass-to-charge ratio): C₂₂H₂₆N₄NaO₄S⁺ [M+Na]⁺ Calculated value: 465.1567, Measured value: 465.1561.

### Example 32:

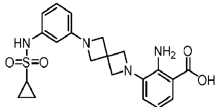

Compound 32 was synthesized according to the General Experimental Operation III (1.5mg, yield 95%). ¹H NMR (300 MHz, Methanol-*d*₄) δ 7.40-7.31 (m, 1H), 7.12 (t, *J* = 8.0 Hz, 1H), 6.89-6.83 (m, 1H), 6.67-6.57 (m, 2H), 6.42 (t, *J* = 2.1 Hz, 1H), 6.33-6.21 (m, 1H), 4.05 (s, 2H), 3.85-3.71 (m, 4H), 3.50 (s, 2H), 2.57-2.43 (m, 1H), 1.08-0.77 (m, 4H). High resolution mass spectrum (mass-to-charge ratio): C₂₁H₂₄N₄NaO₄S⁺ [M+Na]⁺ Calculated value: 451.1410, Measured value: 451.1406.

### Example 33:

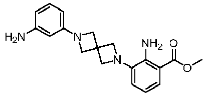

Compound 33 was synthesized according to the General Experimental Operation III (4mg, yield 40%). ¹H NMR (300 MHz, Methanol-*d*₄) δ 7.36-7.31 (m, 1H), 6.97-6.79 (m, 2H), 6.66-6.54 (m, 1H), 6.23-6.11 (m, 1H), 5.98-5.85 (m, 2H), 4.04 (s, 2H), 3.84 (s, 3H), 3.77-3.66 (m, 4H), 3.49 (s, 2H). High resolution mass spectrum (mass-to-charge ratio): C₁₉H₂₃N₄O₂⁺ [M+H]⁺ Calculated value: 339.1816, Measured value: 339.1815.

### Example 34:

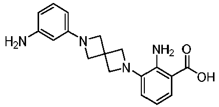

Compound 34 was synthesized according to the General Experimental Operation III (1.6mg, yield 95%). ¹H NMR (300 MHz, Methanol-*d*₄) δ 7.41-7.33 (m, 1H), 6.97-6.87 (m, 1H), 6.84-6.75 (m, 1H), 6.69-6.53 (m, 1H), 6.24-6.08 (m, 1H), 5.98-5.81 (m, 2H), 4.04 (s, 2H), 3.78-3.67 (m, 4H), 3.48 (s, 2H). High resolution mass spectrum (mass-to-charge ratio): C₁₈H₂₁N₄O₂⁺ [M+H]⁺ Calculated value: 325.1659, Measured value: 325.1645.

### Example 35:

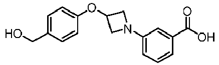

Compound 35 was synthesized according to the General Experimental Operation IV (3.3mg, 80%). ¹H NMR (500 MHz, Methanol-*d4*) δ 7.39 (d, *J* = 7.7 Hz, 1H), 7.34-7.23 (m, 3H), 7.17-7.09 (m, 1H), 6.84 (d, *J =* 8.5 Hz, 2H), 6.79-6.69 (m, 1H), 5.20-5.07 (m, 1H), 4.53 (s, 2H), 4.41-4.30 (m, 2H), 3.93-3.76 (m, 2H). High resolution mass spectrum (mass-to-charge ratio): C₁₇H₁₇NNaO₄⁺ [M+Na]⁺ Calculated value: 322.1050, Measured value: 322.1046.

### Example 36:

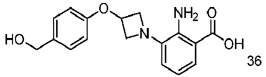

Compound 36 was synthesized according to the General Experimental Operation IV (4.7mg, 53%). ¹H NMR (400 MHz, *Methanol-d4) δ* 7.59-7.44 (m, 1H), 7.28 (d, *J* = 8.6 Hz, 2H), 6.95-6.78 (m, 3H), 6.61 (t, *J* = 7.9 Hz, 1H), 5.10-4.99 (m, 1H), 4.53 (s, 2H), 4.40-4.25 (m, 2H), 3.81-3.62 (m, 2H). High resolution mass spectrum (mass-to-charge ratio): C₁₇H₁₉N₂O₄⁺ [M+H]⁺ Calculated value: 315.1339, Measured value: 315.1333.

### Example 37:

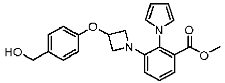

Compound 37 was synthesized according to the General Experimental Operation IV (8.6mg, 98%). ¹H NMR (500 MHz, Chloroform-*d*) *δ* 7.32-7.21 (m, 3H), 7.18-7.11 (m, 1H), 6.74-6.63 (m, 5H), 6.24 (t, *J* = 2.1 Hz, 2H), 4.87-4.79 (m, 1H), 4.59 (s, 2H), 3.83-3.76 (m, 2H), 3.67-3.62 (m, 2H), 3.61 (s, 3H). High resolution mass spectrum (mass-to-charge ratio): C₂₂H₂₃N₂O₄⁺ [M+H]⁺ Calculated value: 379.1652, Measured value: 379.1654.

### Example 38:

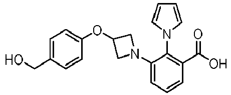

Compound 38 was synthesized according to the General Experimental Operation IV (4.4mg, 53%). ¹H NMR (400 MHz, *Methanol-d4*) *δ* 7.34-7.27 (m, 1H), 7.23 (d, *J* = 8.6 Hz, 2H), 7.07-7.01 (m, 1H), 6.78-6.64 (m, 5H), 6.17 (t, *J* = 2.1 Hz, 2H), 4.84-4.79 (m, 1H), 4.50 (s, 2H), 3.87-3.77 (m, 2H), 3.55-3.47 (m, 2H). High resolution mass spectrum (mass-to-charge ratio): C₂₁H₂₁N₂O₄⁺ [M+H]⁺ Calculated value: 365.1496, Measured value: 365.1494.

### Example 39:

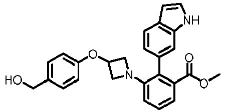

Compound 39 was synthesized according to the General Experimental Operation IV (10mg, 23%). ¹H NMR (300 MHz, Chloroform-*d*) *δ* 8.26 (s, 1H), 7.61 (d, *J* = 8.0 Hz, 1H), 7.28 (d, *J* = 4.4 Hz, 2H), 7.17 (dd, *J* = 18.4, 7.8 Hz, 4H), 7.05 (d, *J* = 7.9 Hz, 1H), 6.67 (d, *J* = 8.1 Hz, 1H), 6.63-6.52 (m, 3H), 4.66 (t, *J* = 5.1 Hz, 1H), 4.56 (s, 2H), 3.67 (t, *J* = 7.1 Hz, 2H), 3.56-3.37 (m, 5H) ppm; High resolution mass spectrum (mass-to-charge ratio): C₂₆H₂₄N₂O₄Na⁺ [M+Na]⁺ Calculated value: 451.1634, Measured value: 451.1631.

### Example 40:

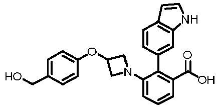

Compound 40 was synthesized according to the General Experimental Operation IV (6mg, 77%). ¹H NMR (300 MHz, Chloroform-*d*) *δ* 8.26 (s, 1H), 7.63 (d, *J* = 8.0 Hz, 1H), 7.35-7.17 (m, 6H), 7.09 (d, *J* = 7.9 Hz, 1H), 6.72-6.68 (m, 1H), 6.63-6.55 (m, 3H), 4.67 (t, *J* = 5.1 Hz, 1H), 4.56 (s, 2H), 3.71-3.63 (m, 2H), 3.56-3.47 (m, 2H). High resolution mass spectrum (mass-to-charge ratio): C₂₅H₂₂N₂O₄Na⁺ [M+Na]⁺ Calculated value:437.1477, Measured value: 437.1472.

### Example 41:

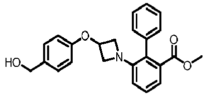

Compound 41 was synthesized according to the General Experimental Operation IV (11mg, 31%). ¹H NMR (300 MHz, Chloroform-*d*) *δ* 7.45 (*t*, *J* = 7.2 Hz, 2H), 7.40-7.32 (*m*, 2H), 7.32-7.16 *(m,* 7H), 7.03 *(d, J* = 7.9 Hz, 1H), 6.93 (*d*, *J* = 8.6 Hz, 2H), 4.77-4.73 (*m*, *J* = 7.5, 4.1 Hz, 1H), 4.62 (*s*, 2H), 3.54 (*s*, 3H), 3.50-3.44 (*m*, 1H), 3.32-3.17 (*m*, 2H), 3.06 (*dd*, *J* = 16.5, 7.3 Hz, 1H). High resolution mass spectrum (mass-to-charge ratio): C₂₄H₂₄NO₄+[M+H]⁺ Calculated value: 437.1477, Measured value: 437.1472.

### Example 42:

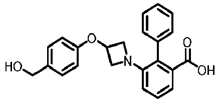

Compound 42 was synthesized according to the General Experimental Operation IV (7mg, 91%). ¹H NMR (300 MHz, Chloroform-*d*) *δ* 7.43 (*dd*, *J* = 7.8, 6.2 Hz, 2H), 7.38-7.17 (*m*, 8H), 7.03 (*d*, *J* = 7.9 Hz, 1H), 6.92 (*d*, *J* = 8.6 Hz, 2H), 4.76-4.72 (*m*, 1H), 4.61 (*s*, 2H), 3.50-3.41 (*m*, 1H), 3.29-3.20 (*m*, 2H), 3.06 (*dd*, *J* = 16.6, 7.3 Hz, 1H). High resolution mass spectrum (mass-to-charge ratio): C₂₃H₂₁NO₄Na⁺ [M+Na]⁺ Calculated value: 398.1368, Measured value: 398.1363.

### Example 43:

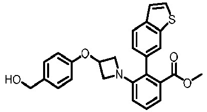

Compound 43 was synthesized according to the General Experimental Operation IV (11mg, 27%). ¹H NMR (400 MHz, Chloroform-*d*) *δ* 7.89 (*d*, *J* = 8.1 Hz, 1H), 7.74 (*s*, 1H), 7.46 (*d*, *J* = 4.8 Hz, 1H), 7.37 (*d*, *J* = 4.9 Hz, 1H), 7.29-7.21 (*m*, 5H), 7.05 (*d*, *J* = 8.0 Hz, 1H), 6.93 (*d*, *J* = 8.0 Hz, 2H), 4.75-4.58 (*m*, 1H), 4.62 (*s*, 2H), 3.51-3.44 (*m*, *J* = 13.6 Hz, 4H), 3.27-3.23 (*m*, *J* = 16.8 Hz, 2H), 3.07 (*dd*, *J* = 16.6, 6.7 Hz, 1H). High resolution mass spectrum (mass-to-charge ratio): C₂₆H₂₃NO₄SNa⁺ [M+Na]⁺ Calculated value: 468.1245, Measured value: 468.1242.

### Example 44:

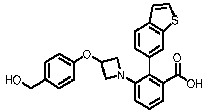

Compound 44 was synthesized according to the General Experimental Operation IV (3mg, 39%). ¹H NMR (500 MHz, Methanol*-d₄*) *δ* 7.89 *(d, J =* 8.2 Hz, 1H), 7.76 *(d, J =* 8.8 Hz, 1H), 7.56 *(d, J=* 5.5 Hz, 1H), 7.39 *(d, J=* 5.9 Hz, 1H), 7.27-7.23 *(m,* 4H), 7.08 *(d, J=* 7.7 Hz, 1H), 7.03 *(d, J =* 7.8 Hz, 1H), 6.95 *(d, J =* 8.6 Hz, 2H), 4.82-4.77 *(m,* 1H), 4.52 (*s*, 2H). High resolution mass spectrum (mass-to-charge ratio): C₂₅H₂₁NO₄SNa⁺ [M+Na]⁺ Calculated value: 454.1089, Measured value: 454.1082.

### Example 45:

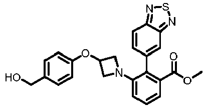

Compound 45 was synthesized according to the General Experimental Operation IV (10mg, 23%). ¹H NMR (300 MHz, Chloroform-*d*) *δ* 8.00 (*d*, *J* = 9.0 Hz, 1H), 7.86 (*s*, 1H), 7.62 (*d*, *J* = 9.8 Hz, 1H), 7.42-7.28 (*m*, 2H), 7.20 (*d*, *J* = 8.4 Hz, 1H), 7.15-7.05 (*m*, 1H), 6.83-6.71 (*m*, 1H), 6.59 (*d*, *J* = 8.5 Hz, 2H), 4.74-4.70 (*m*, 1H), 4.57 (*s*, 2H), 3.79-3.65 (*m*, 2H), 3.56 (*d*, *J* = 9.2 Hz, 5H). High resolution mass spectrum (mass-to-charge ratio): C₂₄H₂₁N₃O₄SNa⁺ [M+Na]⁺ Calculated value: 470.1150, Measured value: 470.1148.

### Example 46:

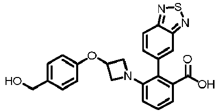

Compound 46 was synthesized according to the General Experimental Operation IV (4mg, 59%). ¹H NMR (500 MHz, Methanol-*d₄*) *δ* 7.99 (*d*, *J* = 9.0 Hz, 1H), 7.87 (s, 1H), 7.67 (*d*, *J* = 9.0 Hz, 1H), 7.37 (*t*, *J* = 8.0 Hz, 2H), 7.28 (*dd*, *J* = 19.9, 8.2 Hz, 2H), 7.18 (*d*, *J* = 8.6 Hz, 2H), 7.12 (*d*, *J* = 8.5 Hz, 1H), 6.85 (*dd*, *J* = 8.2, 5.1 Hz, 1H), 6.65 (*dd*, *J* = 13.1, 8.0 Hz, 3H), 4.79-4.75 (*m*, 1H), 4.47 (*s*, 2H), 3.87-3.78 (*m*, 2H), 3.45 (*t*, *J* = 7.1 Hz, 2H). High resolution mass spectrum (mass-to-charge ratio): C₂₃H₂₀N₃O₄S⁺ [M+H]⁺ Calculated value: 434.1175, Measured value: 434.1169.

### Example 47:

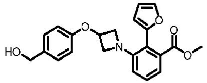

Compound 47 was synthesized according to the General Experimental Operation IV (11mg, 32%). ¹H NMR (400 MHz, Chloroform-*d*) *δ* 7.54 (*s*, 1H), 7.30 (*d*, *J* = 8.5 Hz, 2H), 7.25-7.20 (*m*, 1H), 7.10 (*d*, *J* = 7.8 Hz, 1H), 7.03 (*d*, *J* = 7.7 Hz, 1H), 6.94 (*d*, *J* = 8.5 Hz, 2H), 6.82 (*d, J* = 8.3 Hz, 1H), 6.54-6.50 (*m*, 1H), 6.45 (*d*, *J* = 3.1 Hz, 1H), 4.79-4.77 (*m*, 1H), 4.63 (*s*, 2H), 3.69 (*s*, 3H), 3.57-3.54 (*m*, 1H), 3.41-3.33 (*m*, 1H), 3.21 (*dd*, *J* = 16.5, 4.5 Hz, 1H), 3.05 (*dd*, *J* = 16.4, 7.0 Hz, 1H). High resolution mass spectrum (mass-to-charge ratio): C₂₂H₂₂NO₅+[M+H]⁺Calculated value: 380.1498, Measured value: 380.1494.

### Example 48:

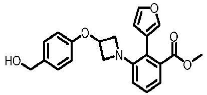

Compound 48 was synthesized according to the General Experimental Operation IV (10mg, 31%). ¹H NMR (300 MHz, Chloroform-*d*) *δ* 7.55 (*t*, *J* = 1.7 Hz, 1H), 7.43-7.38 (*m*, 1H), 7.28 (*d*, *J* = 9.0 Hz, 2H), 7.09 (*d*, *J* = 7.8 Hz, 1H), 7.00 (*d*, *J* = 7.9 Hz, 1H), 6.91 (*d*, *J* = 9.0 Hz 2H), 6.79-6.82 (*m*, 1H), 6.43 (*s*, 1H), 4.78-4.72 (*m*, 1H), 4.62 (*s*, 2H), 3.67 (*s*, 3H), 3.52 (*dt*, *J* = 11.5, 2.7 Hz, 1H), 3.32 (*dd*, *J* = 11.7, 6.9 Hz, 1H), 3.21 (*dd*, *J* = 16.8, 5.2 Hz, 1H), 3.04 (*dd*, *J* = 16.5, 7.1 Hz, 1H). High resolution mass spectrum (mass-to-charge ratio): C₂₂H₂₂NO₅+[M+H]⁺Calculated value: 380.1498, Measured value: 380.1494.

### Example 49:

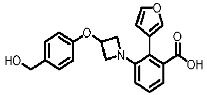

Compound 49 was synthesized according to the General Experimental Operation IV (3mg, 45%). ¹H NMR (500 MHz, Methanol-*d₄*) *δ* 7.59 (*t*, *J* = 1.7 Hz, 1H), 7.50-7.41 (*m*, 1H), 7.31-7.22 (*m*, 3H), 7.01-6.91 (*m*, 4H), 6.45 (*dd*, *J* = 1.8, 0.8 Hz, 1H), 4.83-4.78 (*m*, 1H), 4.52 (*s*, 2H), 3.51-3.47 (*m*, 1H), 3.22-3.16 (*m*, 1H), 3.00 (*dd*, *J* = 16.5, 6.4 Hz, 1H). High resolution mass spectrum (mass-to-charge ratio): C₂₁H₁₉NO₅Na⁺ [M+Na]⁺ Calculated value: 388.1161, Measured value: 388.1154.

### Example 50:

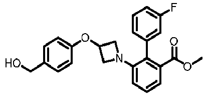

Compound 50 was synthesized according to the General Experimental Operation IV (11mg, 29%). ¹H NMR (300 MHz, Chloroform-*d*) *δ* 7.52-7.27 (*m*, 6H), 7.22 (*d*, *J* = 7.9 Hz, 1H), 7.04 (*d*, *J* = 8.3 Hz, 2H), 7.02-6.97 (*m*, 1H), 6.93 (*d*, *J* = 8.6 Hz, 2H), 4.76-4.74 (*m*, 1H), 4.62 (*s*, 2H), 3.57 (*s*, 3H), 3.52-3.45 (*m*, 1H), 3.35-3.16 (*m*, 2H), 3.06 (*dd*, *J* = 16.5, 7.1 Hz, 1H). High resolution mass spectrum (mass-to-charge ratio): C₂₄H₂₂FNO₄Na⁺ [M+Na]⁺ Calculated value: 430.1431, Measured value: 430.1432.

### Example 51:

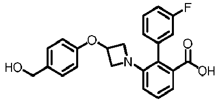

Compound 51 was synthesized according to the General Experimental Operation IV (4mg, 59%). ¹H NMR (300 MHz, Methanol-*d₄*) *δ* 7.45-7,38 (*m*, *J* = 7.9, 6.1 Hz, 1H), 7.26 (*d*, *J* = 8.5 Hz, 2H), 7.12-6.99 (*m*, 5H), 6.93 (*d*, *J* = 8.5 Hz, 3H), 4.82-4.75 (*m*, 1H), 4.51 (*s*, 2H), 3.48-3.38 (*m*, 2H), 3.21-3.15 (*m*, 1H), 3.07-2.95 (*m*, 1H). High resolution mass spectrum (mass-to-charge ratio): C₂₃H₂₀FNO₄Na⁺ [M+Na]⁺ Calculated value: 416.1274, Measured value: 416.1272.

### Example 52:

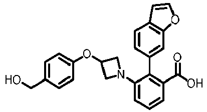

Compound 52 was synthesized according to the General Experimental Operation IV (3.5mg, 52%). ¹H NMR (300 MHz, Methanol-*d₄*) *δ* 7.76 (*d*, *J* = 2.2 Hz, 1H), 7.54 (*d*, *J* = 8.4 Hz, 1H), 7.49-7.44 (*m*, 1H), 7.25 (*d, J* = 8.6 Hz, 2H), 7.17-7.13 (*m*, *J* = 8.3, 5.0, 1.6 Hz, 1H), 7.08-7.02 (*m*, 2H), 6.93 (*d*, *J* = 8.6 Hz, 2H), 6.85 (*d*, *J* = 1.9 Hz, 1H), 4.83-4.75 (*m*, 1H), 4.51 (*s*, 2H), 3.44-3.40 (*m*, 1H), 3.27-3.16 (*m*, 2H), 3.02 (*dd*, *J* = 16.8, 6.8 Hz, 1H). High resolution mass spectrum (mass-to-charge ratio): C₂₅H₂₁NO₅Na⁺ [M+Na]⁺ Calculated value: 438.1317, Measured value: 438.1311.

### Example 53:

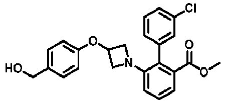

Compound 53 was synthesized according to the General Experimental Operation IV (11mg, 29%). ¹H NMR (300 MHz, Chloroform-*d*) *δ* 7.42-7.33 (*m*, 2H), 7.29 (*d*, *J* = 8.6 Hz, 2H), 7.25-7.19 (*m*, 2H), 7.16-7.10 (*m*, 1H), 7.04 (*d*, *J* = 7.9 Hz, 1H), 6.93 (*d, J* = 8.6 Hz, 2H), 4.62 (*s*, 2H), 3.67-3.61 (*m*, 1H), 3.58 (*s*, 3H), 3.37-3.17 (*m*, 2H), 3.06 (*dd*, *J* = 16.5, 7.2 Hz, 1H). High resolution mass spectrum (mass-to-charge ratio): C₂₄H₂₂ClNO₄Na⁺ [M+Na]⁺ Calculated value: 446.1135, Measured value: 446.1215.

### Example 54:

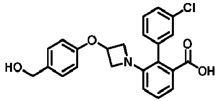

Compound 54 was synthesized according to the General Experimental Operation IV (5mg, 74%). ¹H NMR (400 MHz, Chloroform-*d*) *δ* 7.39-7.32 (*m*, 2H), 7.29 (*d*, *J* = 8.7 Hz, 2H), 7.24-7.20 (*m*, 2H), 7.13-7.11 (*m*, 1H), 7.05 (*d*, *J* = 8.0 Hz, 1H), 6.93 (*d*, *J* = 8.3 Hz, 2H), 6.77-6.74 (*m*, 1H), 4.75 (*s*, 1H), 4.63 (*s*, 2H), 3.53-3.44 (*m*, 1H), 3.33-3.19 (*m*, 2H), 3.07 (*dd*, *J* = 16.6, 7.1 Hz, 1H). High resolution mass spectrum (mass-to-charge ratio): C₂₃H₂₀ClNO₄Na⁺ [M+Na]⁺ Calculated value: 432.0979, Measured value: 432.0972.

### Example 55:

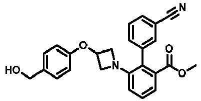

Compound 55 was synthesized according to the General Experimental Operation IV (5mg, 14%). ¹H NMR (300 MHz, Chloroform-*d*) *δ* 7.67 (*d*, *J* = 7.6 Hz, 1H), 7.61-7.45 (*m*, 3H), 7.29-7.23 (*m*, 3H), 7.08 (*d*, *J* = 8.0 Hz, 1H), 6.92 (*d*, *J* = 8.5 Hz, 2H), 6.82 (*d*, *J* = 8.4 Hz, 1H), 4.76-4.75 (*m*, 1H), 4.62 (*d*, *J* = 5.3 Hz, 2H), 3.58 (*s*, 3H), 3.49-3.44 (*m*, 1H), 3.29-3.12 (*m*, 2H), 3.08 (*dd*, *J* = 16.7, 6.8 Hz, 1H). High resolution mass spectrum (mass-to-charge ratio): C₂₅H₂₂N₂O₄Na⁺ [M+Na]⁺ Calculated value: 437.1477, Measured value: 437.1564.

### Example 56:

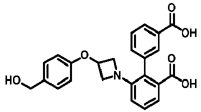

Compound 56 was synthesized according to the General Experimental Operation IV (5mg, 74%). ¹H NMR (300 MHz, Methanol-*d₄*) *δ* 7.59 (*d*, *J* = 8.1 Hz, 1H), 7.30-7.21 (*m*, 4H), 7.00-6.89 (*m*, 5H), 6.44 (*dd*, *J* = 3.2, 0.9 Hz, 1H), 4.82-4.76 (*m*, 1H), 4.51(*s*, 2H), 3.42 (*m*, 1H), 3.26-3.16 (*m*, 2H), 3.05-2.95 (*m*, 1H) ppm; High resolution mass spectrum (mass-to-charge ratio): C₂₄H₂₁NO₆Na⁺ [M+Na]⁺ Calculated value: 442.1267, Measured value: 442.1261.

### Example 57:

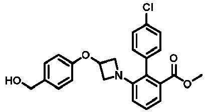

Compound 57 was synthesized according to the General Experimental Operation IV (7mg, 24%). ¹H NMR (300 MHz, Chloroform-*d*) *δ* 7.42 (*d*, *J* = 7.9 Hz, 2H), 7.36-7.11 (*m*, 6H), 7.04 (*d*, *J* = 7.9 Hz, 1H), 6.92 (*d*, *J* = 8.1 Hz, 2H), 4.75 (*s*, 1H), 4.62 (*s*, 2H), 3.58-3.45 (*m*, 4H), 3.34-3.17 (*m*, 2H), 3.06 (*dd*, *J* = 16.6, 7.1 Hz, 1H). High resolution mass spectrum (mass-to-charge ratio): C₂₄H₂₃ClNO₄+[M+H]⁺ Calculated value: 424.1316, Measured value: 424.1301.

### Example 58:

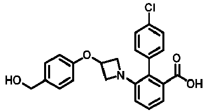

Compound 58 was synthesized according to the General Experimental Operation IV (5mg, 85%). ¹H NMR (400 MHz, Chloroform-*d*) *δ* 7.41 (*d*, *J* = 8.1 Hz, 2H), 7.32-7.26 (*m*, *J* = 8.6 Hz, 3H), 7.18 (*d*, *J* = 8.0 Hz, 2H), 7.05 (*d*, *J* = 7.9 Hz, 1H), 6.92 (*d*, *J* = 8.5 Hz, 2H), 6.75 (*d*, *J* = 5.7 Hz, 1H), 4.78-4.71 (*m*, 1H), 4.62 (*s*, 2H), 3.64-3.48 (*m*, 1H), 3.33-3.19 (*m*, 2H), 3.07 (*dd*, *J* = 16.0, 6.6 Hz, 1H). High resolution mass spectrum (mass-to-charge ratio): C₂₃H₂₀ClNO₄Na⁺ [M+Na]⁺ Calculated value: 432.0979, Measured value: 432.0965.

### Example 59:

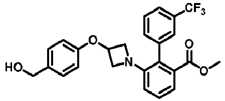

Compound 59 was synthesized according to the General Experimental Operation IV (7mg, 22%). ¹H NMR (300 MHz, Chloroform-*d*) *δ* 7.64-7.54 (*m*, 2H), 7.50 (*s*, 1H), 7.44 (*d*, *J* = 7.4 Hz, 1H), 7.34-7.20 (*m*, 4H), 7.06 (*d*, *J* = 7.9 Hz, 1H), 6.92 (*d*, *J* = 8.6 Hz, 2H), 4.75 (*tt*, *J* = 6.8, 3.6 Hz, 1H), 4.61 (*s*, 2H), 3.53 (*s*, 4H), 3.32-3.19 (*m*, 2H), 3.06 (*dd*, *J* = 16.5, 7.1 Hz, 1H). High resolution mass spectrum (mass-to-charge ratio): C₂₅H₂₂F₃NO₄Na⁺ [M+Na]⁺ Calculated value: 480.1399, Measured value: 480.1395.

### Example 60:

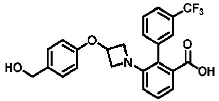

Compound 60 was synthesized according to the General Experimental Operation IV (4mg, 82%). ¹H NMR (500 MHz, Chloroform-*d*) *δ* 7.62 (*d*, *J* = 7.6 Hz, 1H), 7.55 (*t*, *J* = 7.7 Hz, 1H), 7.51 (*s*, 1H), 7.44 (*d*, *J* = 7.9 Hz, 1H), 7.34 (*d*, *J* = 7.8 Hz, 1H), 7.29 (*d*, *J* = 8.4 Hz, 2H), 7.08 (*d*, *J* = 7.4 Hz, 1H), 6.93 (*d*, *J* = 8.4 Hz, 2H), 6.76 (*dd*, *J* = 7.2, 5.2 Hz, 1H), 4.76 (*s*, 1H), 4.63 (*s*, 2H), 3.44 (*s*, 1H), 3.34-3.19 (*m*, 2H), 3.08 (*dd*, *J* = 16.6, 6.9 Hz, 1H). High resolution mass spectrum (mass-to-charge ratio): C₂₄H₂₀F₃NO₄Na⁺ [M+Na]⁺ Calculated value: 466.1242, Measured value: 466.1234.

### Example 61:

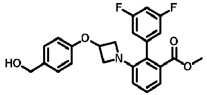

Compound 61 was synthesized according to the General Experimental Operation IV (6mg, 21%). ¹H NMR (400 MHz, Chloroform-*d*) *δ* 7.29 (*d*, *J* = 8.5 Hz, 2H), 7.24 (*d*, *J* = 8.0 Hz, 1H), 7.05 (*d*, *J* = 7.9 Hz, 1H), 6.93 (*d*, *J* = 8.5 Hz, 2H), 6.86-6.69 (*m*, 3H), 4.78-4.73 (*m*, 1H), 4.63 (*s*, 2H), 3.62 (*s*, 3H), 3.49 (*d*, *J* = 11.7 Hz, 1H), 3.32 (*dd*, *J* = 11.8, 6.6 Hz, 1H), 3.23 (*dd*, *J* = 16.0, 3.5 Hz, 1H), 3.07 (*dd*, *J* = 16.5, 6.9 Hz, 1H). High resolution mass spectrum (mass-to-charge ratio): C₂₄H₂₁F₂NO₄Na⁺ [M+Na]⁺ Calculated value: 448.1336, Measured value: 448.1331.

### Example 62:

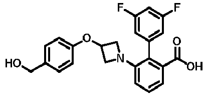

Compound 62 was synthesized according to the General Experimental Operation IV (4mg, 83%). ¹H NMR (500 MHz, Chloroform-*d*) *δ* 7.31 (*dd*, *J* = 14.1, 8.1 Hz, 4H), 7.07 (*d*, *J* = 7.9 Hz, 1H), 6.93 (*d*, *J* = 8.4 Hz, 2H), 6.86-6.72 (*m*, 3H), 4.78-4.74 (*m*, 1H), 4.63 (*s*, 2H), 3.51 (*s*, 1H), 3.32 (*dd*, *J* = 11.5, 6.8 Hz, 1H), 3.24 (*dd*, *J* = 16.9, 3.6 Hz, 1H), 3.08 (*dd*, *J* = 16.7, 6.9 Hz, 1H). High resolution mass spectrum (mass-to-charge ratio): C₂₃H₁₉F₂NO₄Na⁺ [M+Na]⁺ Calculated value: 434.1180, Measured value: 34.1176.

### Example 63:

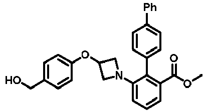

Compound 63 was synthesized according to the General Experimental Operation IV (13mg, 21%). ¹H NMR (300 MHz, Chloroform-*d*) *δ* 7.72-7.61 (*m*, 4H), 7.46 (*t*, *J* = 7.4 Hz, 2H), 7.38-7.21 (*m*, 7H), 7.05 (*d*, *J* = 7.9 Hz, 1H), 6.94 (*d*, *J* = 8.5 Hz, 2H), 4.76 (*dt*, *J* = 7.6, 4.2 Hz, 1H), 4.62 (*s*, 2H), 3.58-3.46 (*m*, 4H), 3.34-3.20 (*m*, 2H), 3.08 (*dd*, *J* = 16.4, 7.2 Hz, 1H). High resolution mass spectrum (mass-to-charge ratio): C₃₀H₂₈NO₄+[M+H]⁺ Calculated value: 466.2018, Measured value: 466.2015.

### Example 64:

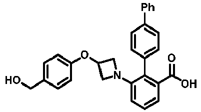

Compound 64 was synthesized according to the General Experimental Operation IV (7mg, 89%). ¹H NMR (500 MHz, Chloroform-*d*) *δ* 7.71-7.60 (*m*, 4H), 7.45 (*t*, *J* = 7.4 Hz, 2H), 7.36 (*t*, *J* = 6.8 Hz, 1H), 7.34-7.22 (*m*, 6H), 7.04 (*d*, *J* = 7.9 Hz, 1H), 6.93 (*d*, *J* = 8.3 Hz, 2H), 4.76 (*s*, 1H), 4.62 (*s*, 2H), 3.48 (*d*, *J* = 11.6 Hz, 1H), 3.33-3.19 (*m*, 2H), 3.08 (*dd*, *J* = 16.3, 6.9 Hz, 1H). High resolution mass spectrum (mass-to-charge ratio): C₂₉H₂₅NO₄Na⁺ [M+Na]⁺ Calculated value: 474.1681, Measured value: 474.1672.

### Example 65:

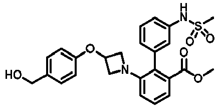

Compound 65 was synthesized according to the General Experimental Operation IV (13mg, 19%). ¹H NMR (400 MHz, Chloroform-*d*) *δ* 7.39-7.01 (*m*, 8H), 6.92 (*d*, *J* = 7.4 Hz, 1H), 6.79-6.59 (*m*, 3H), 4.79-4.69 (*m*, 1H), 4.60 (*d*, *J* = 10.5 Hz, 2H), 3.79-3.69 (*m*, 1H), 3.61-3.43 (*m*, 5H), 3.34-3.20 (*m*, 1H). High resolution mass spectrum (mass-to-charge ratio): C₂₅H₂₆N₂O₆SNa⁺ [M+Na]⁺ Calculated value: 505.1409, Measured value: 505.1407.

### Example 66:

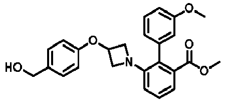

Compound 66 was synthesized according to the General Experimental Operation IV (12mg, 21%). ¹H NMR (500 MHz, Chloroform-*d*) *δ* 7.37 (*t*, *J* = 7.9 Hz, 1H), 7.33-7.22 (*m*, 3H), 7.18 (*dd*, *J* = 7.8, 4.1 Hz, 1H), 7.03 (*d*, *J* = 7.9 Hz, 1H), 6.96-6.77 (*m*, 5H), 6.67 (*dd*, *J* = 8.1, 4.8 Hz, 1H), 4.76 (*t*, *J* = 5.7 Hz, 1H), 4.62 (*d*, *J* = 11.6 Hz, 2H), 3.57-3.46 (*m*, 4H), 3.36-3.18 (*m*, 2H), 3.07 (*dd*, *J* = 16.5, 7.4 Hz, 1H). High resolution mass spectrum (mass-to-charge ratio): C₂₅H₂₆NO₅⁺[M+H]⁺ Calculated value: 420.1811, Measured value: 420.1806.

### Example 67:

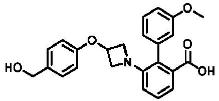

Compound 67 was synthesized according to the General Experimental Operation IV (4mg, 52%). ¹H NMR (300 MHz, Methanol-*d₄*) *δ* 7.33 (*d*, *J* = 8.0 Hz, 1H), 7.26 (*d*, *J* = 8.5 Hz, 2H), 7.17-7.04 (*m*, 2H), 7.01 (*d*, *J* = 6.5 Hz, 1H), 6.98-6.85 (*m*, 3H), 6.79 (*t*, *J* = 3.4 Hz, 2H), 6.71-6.66 (*m*, 1H), 4.79 (*s*, 1H), 4.51 (*s*, 2H), 3.43 (*d*, *J* = 11.9 Hz, 1H), 3.21 (*dd*, *J* = 16.6, 4.4 Hz, 2H), 3.00 (*dd*, *J* = 16.4, 6.4 Hz, 1H). High resolution mass spectrum (mass-to-charge ratio): C₂₄H₂₃NO₅Na⁺ [M+Na]⁺ Calculated value: 428.1474, Measured value: 428.1465.

### Example 68:

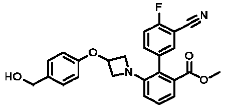

Compound 68 was synthesized according to the General Experimental Operation IV (12mg, 20%). ¹H NMR (300 MHz, Chloroform-*d*) *δ* 7.49 (*t, J* = 6.9 Hz, 2H), 7.30 (*t*, *J* = 10.7 Hz, 5H), 7.09 (s, 1H), 6.92 (*d*, *J* = 8.0 Hz, 2H), 6.82-6.72 (*m*, 1H), 4.76 (*s*, 1H), 4.62 (*d*, *J* = 7.3 Hz, 2H), 3.82 (*d*, *J* = 7.8 Hz, 1H), 3.63 (*s*, 3H), 3.38-3.18 (*m*, 2H), 3.08 (*dd*, *J* = 17.3, 6.5 Hz, 1H). High resolution mass spectrum (mass-to-charge ratio): C₂₅H₂₁FN₂O₄Na⁺ [M+Na]⁺ Calculated value: 455.1383, Measured value: 455.1378.

### Example 69:

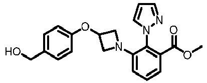

Compound 69 was synthesized according to the General Experimental Operation IV (6.1mg, 16%). ¹H NMR (300 MHz, Chloroform-*d*) *δ* 7.74-7.70 (*m*, 1H), 7.59 (*d*, *J* = 2.2 Hz, 1H), 7.50-7.33 (*m*, 4H), 6.73 (*d*, *J* = 8.1 Hz, 1H), 6.68 (*d*, *J* = 8.6 Hz, 2H), 6.45 (*t*, *J* = 2.0 Hz, 1H), 4.89-4.80 (*m*, 1H), 4.62 (*s*, 2H), 3.86-3.76 (*m*, 2H), 3.70-3.64 (*m*, 2H), 3.63 (*s*, 3H). High resolution mass spectrum (mass-to-charge ratio): C₂₁H₂₂N₃O₄+[M+H]⁺ Calculated value: 380.1610, Measured value: 380.1603.

### Example 70:

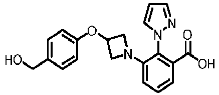

Compound 70 was synthesized according to the General Experimental Operation IV (3.5mg, yield 79%). ¹H NMR (300 MHz, Chloroform-*d*) *δ* 7.85-7.75 (*m*, 1H), 7.63 (*d*, *J* = 6.7 Hz, 1H), 7.38 (*d*, *J* = 8.5 Hz, 2H), 7.05-6.98 (*m*, 1H), 6.95-6.87 (*m*, 1H), 6.76 (*d*, *J* = 8.0 Hz, 1H), 6.67 (*d*, *J* = 8.4 Hz, 2H), 6.50-6.47 (*m*, 1H), 5.05-5.01 (*m*, 1H), 4.94 (*d*, *J* = 10.4 Hz, 2H), 4.62 (*s*, 2H), 3.80 (*d*, *J* = 5.6 Hz, 2H). High resolution mass spectrum (mass-to-charge ratio): C₂₀H₁₉N₃O₄Na⁺ [M+Na]⁺ Calculated value: 388.1268, Measured value: 388.1271.

### Example 71:

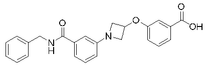

Compound 71 was synthesized according to the General Experimental Operation V (4.5mg, 50%). ¹H NMR (400 MHz, Methanol-*d₄*) *δ* 8.87 (t, *J* = 6.0 Hz, 1H), 7.72-7.61 (m, 1H), 7.50-7.47 (m, 1H), 7.46-7.30 (m, 1H), 7.30-7.16 (m, 7H), 7.16-7.04 (m, 1H), 7.03-6.87 (m, 1H), 6.75-6.55 (m, 1H), 5.25-5.08 (m, 1H), 4.60-4.51 (m, 2H), 4.44-4.29 (m, 2H), 3.97-3.71 (m, 2H). High resolution mass spectrum (mass-to-charge ratio): C₂₄H₂₂N₂NaO₄⁺ [M+Na]⁺ Calculated value: 425.1472, Measured value: 425.1481.

### Example 72:

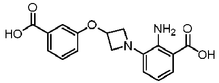

Compound 72 was synthesized according to the General Experimental Operation V (3.9mg, 30%). ¹H NMR (500 MHz, Methanol-*d₄*) δ 7.69-7.63 (m, 1H), 7.54-7.47 (m, 2H), 7.43-7.37 (m, 1H), 7.13 (dd, *J* = 8.2, 2.7 Hz, 1H), 6.88 (dd, *J* = 7.7, 1.4 Hz, 1H), 6.62 (t, *J* = 7.8 Hz, 1H), 5.12 (p, *J* = 5.5 Hz, 1H), 4.39 (s, 2H), 3.83-3.69 (m, 2H). High resolution mass spectrum (mass-to-charge ratio): C₁₇H₁₇N₂O₅⁺ [M+H]⁺ Calculated value: 329.1132, Measured value: 329.1127.

### Example 73:

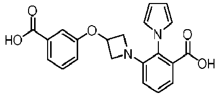

Compound 73 was synthesized according to the General Experimental Operation V (3.6mg, 20%). ¹H NMR (300 MHz, Methanol-*d₄*) δ 7.68-7.56 (m, 1H), 7.46-7.26 (m, 3H), 7.14-7.06 (m, 1H), 7.03-6.95 (m, 1H), 6.83-6.75 (m, 1H), 6.72 (t, *J* = 2.1 Hz, 2H), 6.20 (t, *J* = 2.1 Hz, 2H), 4.97-4.83 (m, 1H), 3.92-3.80 (m, 2H), 3.61-3.49 (m, 2H). High resolution mass spectrum (mass-to-charge ratio): C₂₁H₁₉N₂O₅⁺ [M+H]⁺ Calculated value: 379.1288, Measured value: 379.1291.

### Example 74:

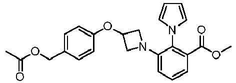

Compound 74 was synthesized according to the General Experimental Operation VI (4.2mg, yield 84%). ¹H NMR (400 MHz, Chloroform-*d*) δ 7.43-7.22 (m, 3H), 7.22-7.13 (m, 1H), 6.82-6.55 (m, 5H), 6.26 (t, *J* = 2.1 Hz, 2H), 5.03 (s, 2H), 4.91-4.78 (m, 1H), 3.84-3.76 (m, 2H), 3.69-3.63 (m, 2H), 3.62 (s, 3H), 2.08 (s, 3H). High resolution mass spectrum (mass-to-charge ratio): C₂₄H₂₅N₂O₅⁺ [M+H]⁺ Calculated value: 421.1758, Measured value: 421.1757.

### Example 75:

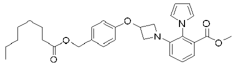

Compound 75 was synthesized according to the General Experimental Operation VI (8.7mg, yield 85%). ¹H NMR (300 MHz, Chloroform-*d*) δ 7.34-7.21 (m, 3H), 7.21-7.12 (m, 1H), 6.77-6.52 (m, 5H), 6.25 (t, *J* = 2.1 Hz, 2H), 5.02 (s, 2H), 4.89-4.77 (m, 1H), 3.87-3.75 (m, 2H), 3.68-3.63 (m, 2H), 3.62 (s, 3H), 2.32 (t, *J* = 7.6 Hz, 2H), 1.74-1.48 (m, 2H), 1.37-1.20 (m, 8H), 0.94-0.80 (m, 3H). High resolution mass spectrum (mass-to-charge ratio): C₃₀H₃₇N₂O₅⁺ [M+H]⁺ Calculated value: 505.2697, Measured value: 505.2694.

### Example 76:

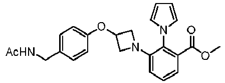

Compound 76 was synthesized according to the General Experimental Operation VI (1.3 mg, yield 80%). ¹H NMR (300 MHz, Chloroform-*d*) δ 7.39-7.23 (m, 1H), 7.22-7.00 (m, 3H), 6.80-6.55 (m1, 5H), 6.25 (t, *J* = 2.1 Hz, 2H), 5.67 (s, 1H), 4.90-4.71 (m, 1H), 4.35 (d, *J* = 5.7 Hz, 2H), 3.87-3.70 (m, 2H), 3.67-3.48 (m, 5H), 2.01 (s, 3H). High resolution mass spectrum (mass-to-charge ratio): C₂₄H₂₆N₃O₄⁺ [M+H]⁺ Calculated value: 420.1918, Measured value: 420.1915.

### Example 77:

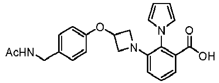

Compound 77 was synthesized according to the General Experimental Operation VI (2.7mg, yield 95%). ¹H NMR (400 MHz, Methanol-*d₄*) δ 7.26 (t, *J* = 7.8 Hz, 1H), 7.21-7.09 (m, 2H), 6.95 (dd, *J* = 7.6, 1.3 Hz, 1H), 6.79-6.71 (m, 2H), 6.71-6.61 (m, 3H), 6.26-6.01 (m, 2H), 4.86-4.77 (m, 1H), 4.43-4.20 (m, 2H), 3.87-3.72 (m, 2H), 3.52-3.43 (m, 2H), 1.96 (s, 3H). High resolution mass spectrum (mass-to-charge ratio): C₂₃H₂₄N₃O₄⁺ [M+H]⁺ Calculated value: 406.1761, Measured value: 406.1759.

### Example 78:

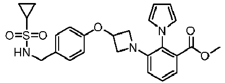

Compound 78 was synthesized according to the General Experimental Operation VI (1.5mg, yield 50%). ¹H NMR (500 MHz, Chloroform-*d*) δ 7.33-7.21 (m, 3H), 7.20-7.12 (m, 1H), 6.76-6.55 (m, 5H), 6.33-6.08 (m, 2H), 4.88-4.79 (m, 1H), 4.27 (d, *J* = 6.1 Hz, 2H), 3.86-3.76 (m, 2H), 3.67-3.62 (m, 2H), 3.62 (s, 3H), 2.38-2.30 (m, 1H), 1.19-1.10 (m, 2H), 0.97-0.94 (m, 2H). High resolution mass spectrum (mass-to-charge ratio): C₂₅H₂₈N₃O₅S⁺ [M+H]⁺ Calculated value: 482.1744, Measured value: 482.1739.

### Example 79:

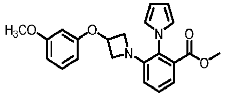

Compound 79 was synthesized according to the General Experimental Operation VII (19mg, 27%). ¹H NMR (500 MHz, Chloroform-*d*) δ 7.29 (t, *J* = 7.9 Hz, 1H), 7.18-7.13 (m, 2H), 6.71 (t, *J* = 2.1 Hz, 2H), 6.68 (dd, *J* = 8.2, 1.1 Hz, 1H), 6.56-6.49 (m, 1H), 6.32-6.21 (m, 4H), 4.87-4.77 (m, 1H), 3.80 (dd, *J* = 9.0, 6.3 Hz, 2H), 3.77 (s, 3H), 3.65 (dd, *J* = 9.0, 4.8 Hz, 2H), 3.62 (s, 3H). High resolution mass spectrum (mass-to-charge ratio): C₂₂H₂₃N₂O₄+[M+H]⁺ Calculated value: 379.1652, Measured value: 379.1651.

### Example 80:

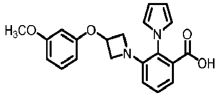

Compound 80 was synthesized according to the General Experimental Operation VII (8mg, 83%). ¹H NMR (400 MHz, *Methanol-d4)* δ 7.30 (d, *J* = 7.9 Hz, 1H), 7.12 (dd, *J* = 10.4, 6.6 Hz, 1H), 7.06 (d, *J* = 7.3 Hz, 1H), 6.75 (d, *J* = 8.0 Hz, 1H), 6.70 (d, *J* = 1.8 Hz, 2H), 6.54-6.46 (m, 1H), 6.33-6.23 (m, 2H), 6.19-6.17 (m, 2H), 4.84-4.77 (m, 1H), 3.84-3.76 (m, 2H), 3.73 (s, 3H), 3.51 (dd, *J* = 8.7, 4.3 Hz, 2H). High resolution mass spectrum (mass-to-charge ratio): C₂₁H₂₀N₂O₄Na⁺ [M+Na]⁺ Calculated value: 387.1315, Measured value: 387.1316.

### Example 81:

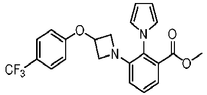

Compound 81 was synthesized according to the General Experimental Operation VII (30mg, 39%). ¹H NMR (300 MHz, Chloroform-*d*) δ 7.53 (d, *J* = 8.6 Hz, 2H), 7.32 (t, *J* = 7.9 Hz, 1H), 7.18 (dd, *J* = 7.6, 1.3 Hz, 1H), 6.76 (d, *J* = 8.6 Hz, 2H), 6.74-6.63 (m, 3H), 6.27 (t, *J* = 2.1 Hz, 2H), 4.95-4.82 (m, 1H), 3.84 (dd, *J* = 9.0, 6.3 Hz, 2H), 3.67 (dd, *J* = 9.2, 4.8 Hz, 2H), 3.63 (s, 3H). High resolution mass spectrum (mass-to-charge ratio): C₂₂H₁₉N₂O₃F₃Na⁺ [M+Na]⁺ Calculated value: 439.1240, Measured value: 439.1239.

### Example 82:

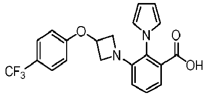

Compound 82 was synthesized according to the General Experimental Operation VII (11mg, 76%). ¹H NMR (500 MHz, *Methanol-d4*) δ 7.55 (d, *J* = 8.3 Hz, 2H), 7.31 (t, *J* = 7.8 Hz, 1H), 7.08 (d, *J* = 7.4 Hz, 1H), 6.87 (d, *J* = 8.3 Hz, 2H), 6.75 (d, *J* = 8.1 Hz, 1H), 6.72-6.68 (m, 2H), 6.20-6.17 (m, 2H), 4.92-4.90(m, 1H), 3.94-3.75 (m, 2H), 3.53 (dd, *J* = 8.3, 3.8 Hz, 2H). High resolution mass spectrum (mass-to-charge ratio): C₂₁H₁₇N₂O₃F₃Na⁺ [M+Na]⁺ Calculated value: 425.1083, Measured value: 425.1087.

### Example 83:

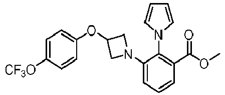

Compound 83 was synthesized according to the General Experimental Operation VII (14mg, 22%). ¹H NMR (400 MHz, Chloroform-*d*) δ 7.30 (t, *J* = 7.9 Hz, 1H), 7.16 (dd, *J* = 7.6, 1.2 Hz, 1H), 7.11 (d, *J* = 8.6 Hz, 2H), 6.74-6.62 (m, 5H), 6.26 (t, *J* = 2.1 Hz, 2H), 4.86-4.76 (m, 1H), 3.85-3.74 (m, 2H), 3.64 (dd, *J* = 9.1, 4.8 Hz, 2H), 3.62 (s, 3H). High resolution mass spectrum (mass-to-charge ratio): C₂₂H₂₀F₃N₂O₄+[M+H]⁺ Calculated value: 433.1370, Measured value: 433.1371.

### Example 84:

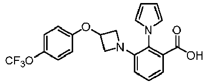

Compound 84 was synthesized according to the General Experimental Operation VII (7mg, 72%). ¹H NMR (300 MHz, *Methanol-d4*) δ 7.33 (t, *J* = 7.9 Hz, 1H), 7.17 (d, *J* = 8.5 Hz, 2H), 7.08 (dd, *J* = 7.6, 1.2 Hz, 1H), 6.85-6.74 (m, 3H), 6.71 (t, *J* = 2.1 Hz, 2H), 6.19 (t, *J* = 2.1 Hz, 2H), 4.91-4.82 (m, 1H), 3.84 (dd, *J* = 9.0, 6.2 Hz, 2H), 3.53 (dd, *J* = 9.1, 4.3 Hz, 2H). High resolution mass spectrum (mass-to-charge ratio): C₂₁H₁₇F₃N₂O₄Na⁺ [M+Na]⁺ Calculated value: 441.1033, Measured value: 441.1030.

### Example 85:

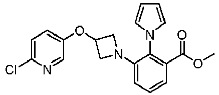

Compound 85 was synthesized according to the General Experimental Operation VII (23mg, 37%). ¹H NMR (300 MHz, Chloroform-*d*) δ 7.84 (d, *J* = 3.1 Hz, 1H), 7.31 (t, *J* = 7.9 Hz, 1H), 7.24-7.14 (m, 2H), 7.01 (dd, *J* = 8.7, 3.1 Hz, 1H), 6.72-6.65 (m, 3H), 6.25 (t, *J* = 2.1 Hz, 2H), 4.89-4.79 (m, 1H), 3.81 (dd, *J* = 9.2, 6.2 Hz, 2H), 3.64 (dd, *J* = 5.9, 3.4 Hz, 2H), 3.62 (s, 3H). High resolution mass spectrum (mass-to-charge ratio): C₂₀H₁₉N₃O₃Cl⁺ [M+H]⁺ Calculated value: 384.1109, Measured value: 384.1109.

### Example 86:

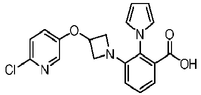

Compound 86 was synthesized according to the General Experimental Operation VII (7mg, 61%). ¹H NMR (400 MHz, *Methanol-d4*) δ 7.88 (d, *J* = 3.0 Hz, 1H), 7.34 (d, *J* = 8.7 Hz, 1H), 7.28 (t, *J* = 7.6 Hz, 1H), 7.24 (dd, *J* = 8.8, 3.1 Hz, 1H), 7.00 (d, *J* = 6.7 Hz, 1H), 6.75-6.67 (m, 3H), 6.16 (s, 2H), 4.95-4.91 (m, 1H), 3.83 (dd, *J* = 8.9, 6.2 Hz, 2H), 3.51 (dd, *J* = 9.0, 4.1 Hz, 2H) High resolution mass spectrum (mass-to-charge ratio): C₁₉H₁₇N₃O₃Cl⁺ [M+H]⁺ Calculated value: 370.0953, Measured value: 370.0950.

### Example 87:

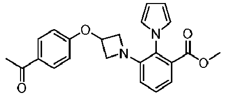

Compound 87 was synthesized according to the General Experimental Operation VII (25mg, 39%). ¹H NMR (300 MHz, Chloroform-*d*) δ 7.91 (d, *J* = 8.8 Hz, 2H), 7.31 (t, *J* = 7.9 Hz, 1H), 7.17 (dd, *J* = 7.6, 1.2 Hz, 1H), 6.79-6.64 (m, 5H), 6.26 (t, *J* = 2.1 Hz, 2H), 4.97-4.84 (m, 1H), 3.84 (dd, *J* = 9.0, 6.3 Hz, 2H), 3.67 (dd, *J* = 9.1, 4.8 Hz, 2H), 3.62 (s, 3H), 2.55 (s, 3H). High resolution mass spectrum (mass-to-charge ratio): C₂₃H₂₂N₂O₄Na⁺ [M+Na]⁺ Calculated value: 413.1477, Measured value: 413.1473.

### Example 88:

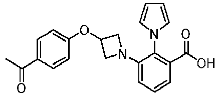

Compound 88 was synthesized according to the General Experimental Operation VII (8mg, 55%). ¹H NMR (400 MHz, *Methanol-d4*) δ 7.97-7.88 (m, 2H), 7.30 (t, *J* = 7.8 Hz, 1H), 7.04 (d, *J* = 7.5 Hz, 1H), 6.86-6.77 (m, 2H), 6.77-6.64 (m, 3H), 6.17 (s, 2H), 4.96-4.92 (m, 1H), 3.85 (dd, *J* = 9.1, 6.2 Hz, 2H), 3.52 (dd, *J* = 9.0, 4.2 Hz, 2H), 2.53 (s, 3H). High resolution mass spectrum (mass-to-charge ratio): C₂₂H₂₁N₂O₄⁺ [M+H]⁺ Calculated value: 377.1496, Measured value: 377.1498.

### Example 89:

The synthetic route in Examples 89 and 90 is as follows:

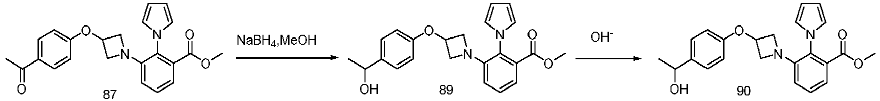

Compound 87 (1 eq.) was dissolved in anhydrous methanol, sodium borohydride (2.6 eq.) was added, and a reaction was performed at room temperature for 2 hours and quenched with ice water. The quenched reaction solution was extracted with dichloromethane, the resulting organic phase was dried with anhydrous sodium sulfate and concentrated, and the residue was separated by preparative thin layer chromatography to obtain compound 89 (yield 62%). The hydrolysis was the same as described above (omitted). 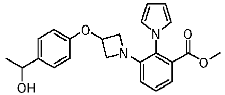 Compound 89 (10 mg, 62%). ¹H NMR (300 MHz, Chloroform-*d*) δ 7.35-7.23 (m, 3H), 7.16 (dd, *J* = 7.6, 1.3 Hz, 1H), 6.73-6.65 (m, 5H), 6.26 (t, *J* = 2.1 Hz, 2H), 4.93-4.75 (m, 2H), 3.86-3.74 (m, 2H), 3.66 (dd, *J* = 9.1, 5.0 Hz, 2H), 3.62 (s, 3H), 1.48 (d, *J* = 6.4 Hz, 3H). High resolution mass spectrum (mass-to-charge ratio): C₂₃H₂₅N₂O₄⁺ [M+H]⁺ Calculated value: 393.1809, Measured value: 393.1809.

### Example 90:

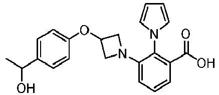

Compound 90 (4 mg, 60%). ¹H NMR (400 MHz, *Methanol-d4*) δ 7.29 (t, *J* = 7.9 Hz, 1H), 7.24 (dd, *J=* 9.0, 2.3 Hz, 2H), 7.01 (d, *J=* 6.9 Hz, 1H), 6.73-6.66 (m, 5H), 6.16 (t, *J* = 1.9 Hz, 2H), 4.85-4.79 (m, 1H), 4.78-4.72 (m, 1H), 3.80 (dd, *J =* 9.0, 6.1 Hz, 2H), 3.50 (dd, *J =* 8.8, 4.4 Hz, 2H), 1.39 (d, *J* = 6.5 Hz, 3H). High resolution mass spectrum (mass-to-charge ratio): C₂₂H₂₂N₂O₄Na⁺ [M+Na]⁺ Calculated value: 401.1472, Measured value: 401.1472.

### Example 91:

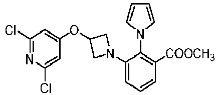

Compound 91 was synthesized according to the General Experimental Operation VII (15mg, 26%). ¹H NMR (400 MHz, Chloroform-*d*) δ 7.31 (t, *J* = 7.9 Hz, 1H), 7.19 (dd, *J* = 7.7, 1.3 Hz, 1H), 6.74-6.64 (m, 3H), 6.59 (s, 2H), 6.26 (t, *J* = 2.1 Hz, 2H), 4.90-4.80 (m, 1H), 3.83 (dd, *J* = 9.3, 6.2 Hz, 2H), 3.68-3.55 (m, 5H). High resolution mass spectrum (mass-to-charge ratio): C₂₀H₁₈Cl₂N₃O₃⁺ [M+H]⁺ Calculated value: 418.0720, Measured value: 418.0721.

### Example 92:

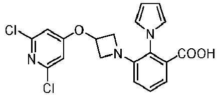

Compound 92 was synthesized according to the General Experimental Operation VII (6mg, 58%). ¹H NMR (400 MHz, *Methanol-d4*) δ 7.28 (t, *J* = 7.9 Hz, 1H), 7.00 (d, *J* = 7.4 Hz, 1H), 6.87 (s, 2H), 6.79-6.63 (m, 3H), 6.19-6.15 (m, 2H), 5.01-4.98 (m, 1H), 3.88-3.82 (m, 2H), 3.50 (dd, *J* = 8.8, 3.5 Hz, 2H). High resolution mass spectrum (mass-to-charge ratio): C₁₉H₁₆Cl₂N₃O₃⁺ [M+H]⁺ Calculated value: 404.0563, Measured value: 404.0563.

### Example 93:

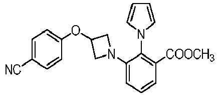

Compound 93 was synthesized according to the General Experimental Operation VII (30mg, 49%). ¹H NMR (400 MHz, Chloroform-*d*) δ 7.56 (d, *J* = 8.8 Hz, 2H), 7.30 (t, *J* = 7.9 Hz, 1H), 7.17 (dd, *J* = 7.6, 1.2 Hz, 1H), 6.74 (d, *J* = 8.8 Hz, 2H), 6.71-6.65 (m, 3H), 6.25 (t, *J* = 2.1 Hz, 2H), 4.92-4.82 (m, 1H), 3.82 (dd, *J* = 9.0, 6.3 Hz, 2H), 3.64 (dd, *J* = 9.1, 4.6 Hz, 2H), 3.61 (s, 3H). High resolution mass spectrum (mass-to-charge ratio): C₂₂H₂₀N₃O₃⁺ [M+H]⁺ Calculated value: 374.1499, Measured value: 374.1499.

### Example 94:

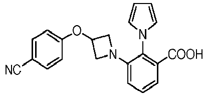

Compound 94 was synthesized according to the General Experimental Operation VII (6mg, 63%). ¹H NMR (400 MHz, *Methanol-d4*) δ 7.63 (d, *J* = 8.7 Hz, 2H), 7.29 (t, *J* = 7.8 Hz, 1H), 7.01 (d, *J* = 7.4 Hz, 1H), 6.89 (d, *J* = 8.7 Hz, 2H), 6.79-6.61 (m, 3H), 6.18-6.15 (m, 2H), 4.94-4.91 (m, 1H), 3.92-3.75 (m, 2H), 3.51 (dd, *J* = 8.6, 3.9 Hz, 2H). High resolution mass spectrum (mass-to-charge ratio): C₂₁H₁₈N₃O₃⁺ [M+H]⁺ Calculated value: 360.1343, Measured value: 360.1344.

### Example 95:

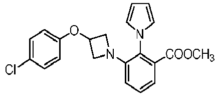

Compound 95 was synthesized according to the General Experimental Operation VII (8mg, 13%). ¹H NMR (500 MHz, Chloroform-*d*) δ 7.30 (t, *J* = 7.9 Hz, 1H), 7.23-7.18 (m, 2H), 7.16 (dd, *J* = 7.6, 1.1 Hz, 1H), 6.74-6.65 (m, 3H), 6.65-6.57 (m, 2H), 6.25 (t, *J* = 2.0 Hz, 2H), 4.83-4.75 (m, 1H), 3.79 (dd, *J* = 8.9, 6.3 Hz, 2H), 3.66-3.58 (m, 5H). High resolution mass spectrum (mass-to-charge ratio): C₂₁H₂₀ClN₂O₃⁺ [M+H]⁺ Calculated value: 383.1157, Measured value: 383.1157.

### Example 96:

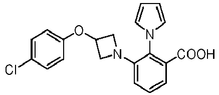

Compound 96 was synthesized according to the General Experimental Operation VII (4mg, 70%). 1H NMR (400 MHz, *Methanol-d4*) δ 7.28 (t, *J* = 7.9 Hz, 1H), 7.23-7.19 (m, 2H), 7.00 (d, *J=* 6.9 Hz, 1H), 6.76-6.65 (m, 5H), 6.16 (t, *J* = 1.9 Hz, 2H), 4.84-4.79 (m, 1H), 3.80 (dd, *J=* 9.1, 6.2 Hz, 2H), 3.49 (dd, *J* = 9.1, 4.3 Hz, 2H). High resolution mass spectrum (mass-to-charge ratio): C₂₀H₁₈ClN₂O₃⁺ [M+H]⁺ Calculated value: 369.1000, Measured value: 369.0992.

### Example 97:

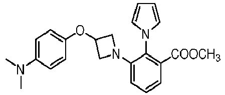

Compound 97 was synthesized according to the General Experimental Operation VII (12mg, 19%). ¹H NMR (400 MHz, Chloroform-*d*) δ 7.29 (*t*, *J* = 8.0 Hz, 1H), 7.14 (*dd*, *J* = 7.6, 1.2 Hz, 1H), 6.75-6.66 (*m*, 5H), 6.63 (*dd*, *J* = 9.6, 2.8 Hz, 2H), 6.25 (*t*, *J* = 2.1 Hz, 2H), 4.82-4.70 (*m*, 1H), 3.80-3.71 (*m*, 2H), 3.64 (*dd*, *J* = 9.0, 5.0 Hz, 2H), 3.61 (*s*, 3H), 2.86 (*s*, 6H). High resolution mass spectrum (mass-to-charge ratio): C₂₃H₂₆N₃O₃⁺ [M+H]⁺ Calculated value: 392.1969, Measured value: 392.1967.

### Example 98:

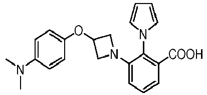

Compound 98 was synthesized according to the General Experimental Operation VII (6mg, 62%). ¹H NMR (300 MHz, *Methanol-d4*) δ 7.26 (*t*, *J* = 7.8 Hz, 1H), 6.94 (*d*, *J* = 7.1 Hz, 1H), 6.85-6.71 (*m*, 4H), 6.70-6.56 (*m*, 3H), 6.19 (*d*, *J* = 21.7 Hz, 2H), 4.81-4.68 (*m*, 1H), 3.83-3.69 (*m*, 2H), 3.47 (*dd*, *J* = 8.9, 4.6 Hz, 2H), 2.82 (*s*, 6H). High resolution mass spectrum (mass-to-charge ratio): C₂₂H₂₄N₃O₃⁺ [M+H]⁺ Calculated value: 378.1812, Measured value: 378.1813.

### Example 99:

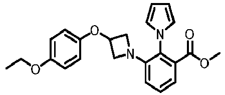

Compound 99 was synthesized according to the General Experimental Operation VIII (25mg, 35%). ¹H NMR (500 MHz, Chloroform-*d*) δ 7.29 (*t*, *J* = 7.9 Hz, 1H), 7.15 (*dd*, *J* = 7.6, 1.2 Hz, 1H), 6.83-6.75 (*m*, 2H), 6.71 (*t*, *J* = 2.1 Hz, 2H), 6.68 (*dd*, *J* = 8.2, 1.1 Hz, 1H), 6.65 -6.58 (*m*, 2H), 6.25 (*t*, *J* = 2.1 Hz, 2H), 4.84-4.70 (*m*, 1H), 3.96 (*q*, *J* = 7.0 Hz, 2H), 3.82-3.72 (*m*, 2H), 3.64 (*dd*, *J* = 9.0, 4.9 Hz, 2H), 3.62 (*s*, 3H), 1.38 (*t*, *J* = 7.0 Hz, 3H). High resolution mass spectrum (mass-to-charge ratio): C₂₃H₂₄N₂O₄Na⁺ [M+Na]⁺ Calculated value: 415.1628, Measured value: 415.1628.

### Example 100:

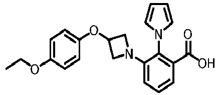

Compound 100 was synthesized according to the General Experimental Operation VIII (9mg, 63%). ¹H NMR (300 MHz, *Methanol-d4*) δ 7.29 (*t*, *J* = 7.8 Hz, 1H), 7.01 (*d*, *J* = 7.4 Hz, 1H), 6.85-6.76 (*m*, 2H), 6.76-6.68 (*m*, 3H), 6.68-6.60 (*m*, 2H), 6.17 (*s*, 2H), 4.81-4.70 (*m*, 1H), 3.95 (*q*, *J* = 7.0 Hz, 2H), 3.77 (*dd*, *J* = 8.8, 6.2 Hz, 2H), 3.49 (*dd*, *J* = 8.9, 4.5 Hz, 2H), 1.35 (*t*, *J* = 7.0 Hz, 3H) . High resolution mass spectrum (mass-to-charge ratio): C₂₂H₂₂N₂O₄Na⁺ [M+Na]⁺ Calculated value: 401.1472, Measured value: 401.1473.

### Example 101:

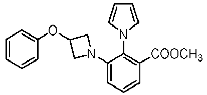

Compound 101 was synthesized according to the General Experimental Operation VIII (16mg, 25%). ¹H NMR (300 MHz, Chloroform-*d*) δ 7.34-7.23 (*m*, 3H), 7.16 (*dd*, *J* = 7.6, 1.1 Hz, 1H), 6.97 (*t*, *J* = 7.4 Hz, 1H), 6.74-6.68 (*m*, 5H), 6.27 (*t*, *J* = 2.1 Hz, 2H), 4.91-4.79 (*m*, 1H), 3.86-3.76 (*m*, 2H), 3.67 (*dd*, *J* = 9.0, 4.9 Hz, 2H), 3.63 (*s*, 3H). High resolution mass spectrum (mass-to-charge ratio): C₂₁H₂₁N₂O₃⁺ [M+H]⁺ Calculated value: 349.1547, Measured value: 349.1547.

### Example 102:

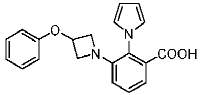

Compound 102 was synthesized according to the General Experimental Operation VIII (7mg, 73%). ¹H NMR (500 MHz, *Methanol-d4*) δ 7.31 (*t*, *J* = 7.9 Hz, 1H), 7.27-7.19 (*m*, 2H), 7.07 (*dd*, *J* = 7.6, 1.3 Hz, 1H), 6.92 (*t*, *J* = 7.4 Hz, 1H), 6.76 (*dd*, *J* = 8.2, 1.2 Hz, 1H), 6.74-6.66 (*m*, 4H), 6.18 (*t*, *J* = 2.1 Hz, 2H), 4.86-4.80 (*m*, 1H), 3.82 (*dd*, *J* = 9.2, 6.2 Hz, 2H), 3.52 (*dd*, *J* = 9.2, 4.4 Hz, 2H). High resolution mass spectrum (mass-to-charge ratio): C₂₀H₁₈N₂O₃Na⁺ [M+H]⁺ Calculated value: 357.1210, Measured value: 357.1207.

### Example 103:

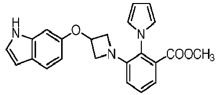

Compound 103 was synthesized according to the General Experimental Operation VIII (10mg, 14%). ¹H NMR (500 MHz, Chloroform-*d*) δ 8.05 (*s*, 1H), 7.49 (*d*, *J* = 8.3 Hz, 1H), 7.29 (*t*, *J* = 7.9 Hz, 1H), 7.17-7.13 (*m*, 1H), 7.12-7.07 (*m*, 1H), 6.75-6.67 (*m*, 3H), 6.66-6.59 (*m*, 2H), 6.51-6.45 (*m*, 1H), 6.25 (*t*, *J* = 2.1 Hz, 2H), 4.91-4.82 (*m*, 1H), 3.82 (*dd*, *J* = 8.9, 6.3 Hz, 2H), 3.70 (*dd*, *J* = 9.0, 4.9 Hz, 2H), 3.62 (*s*, 3H). High resolution mass spectrum (mass-to-charge ratio): C₂₃H₂₂N₃O₃⁺ [M+H]⁺ Calculated value: 388.1656, Measured value: 388.1656.

### Example 104:

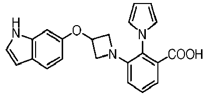

Compound 104 was synthesized according to the General Experimental Operation VIII (3mg, 52%). ¹H NMR (300 MHz, *Methanol-d4*) δ 7.38 (*d*, *J* = 8.6 Hz, 1H), 7.30 (*t*, *J* = 7.9 Hz, 1H), 7.09 (*d*, *J* = 3.2 Hz, 1H), 7.02 (*dd*, *J* = 7.5, 1.2 Hz, 1H), 6.74 (*dd*, *J* = 4.1, 2.0 Hz, 3H), 6.65 (*d*, *J* = 2.0 Hz, 1H), 6.56 (*dd*, *J* = 8.6, 2.3 Hz, 1H), 6.34 (*d*, *J* = 2.5 Hz, 1H), 6.18 (*t*, *J* = 2.1 Hz, 2H), 4.88-4.79 (*m*, 1H), 3.90-3.77 (*m*, 2H), 3.56 (*dd*, *J* = 9.0, 4.5 Hz, 2H). High resolution mass spectrum (mass-to-charge ratio): C₂₂H₁₉N₃O₃Na⁺ [M+Na]⁺ Calculated value: 396.1319, Measured value: 396.1312.

### Example 105:

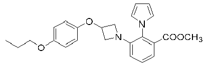

Compound 105 was synthesized according to the General Experimental Operation VIII (24mg, 33%). ¹H NMR (500 MHz, Chloroform-*d*) δ 7.29 (*t*, *J* = 7.9 Hz, 1H), 7.14 (*dd*, *J* = 7.6, 1.3 Hz, 1H), 6.83-6.76 (*m*, 2H), 6.71 (*t*, *J* = 2.1 Hz, 2H), 6.68 (*dd*, *J* = 8.2, 1.3 Hz, 1H), 6.64-6.58 (*m*, 2H), 6.25 (*t*, *J* = 2.1 Hz, 2H), 4.80-4.73 (*m*, 1H), 3.85 (*t*, *J* = 6.6 Hz, 2H), 3.77 (*dd*, *J* = 9.0, 6.2 Hz, 2H), 3.64 (*dd*, *J* = 9.1, 4.9 Hz, 2H), 3.62 (*s*, 3H), 1.82-1.73 (*m*, 2H), 1.02 (*t*, *J* = 7.4 Hz, 3H). High resolution mass spectrum (mass-to-charge ratio): C₂₄H₂₆N₂O₄Na⁺ [M+Na]⁺ Calculated value: 429.1785, Measured value: 429.1787.

### Example 106:

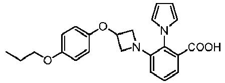

Compound 106 was synthesized according to the General Experimental Operation VIII (5mg, 52%). ¹H NMR (300 MHz, *Methanol-d4*) δ 7.30 (*t*, *J* = 7.8 Hz, 1H), 7.04 (*d*, *J* = 7.4 Hz, 1H), 6.80 (*d*, *J* = 9.0 Hz, 2H), 6.73 (*d*, *J* = 7.1 Hz, 3H), 6.64 (*d*, *J* = 9.0 Hz, 2H), 6.18 (*s*, 2H), 4.82-4.67 (*m*, 1H), 3.85 (*t*, *J* = 6.4 Hz, 2H), 3.81-3.68 (*m*, 2H), 3.50 (*dd*, *J* = 8.6, 4.4 Hz, 2H), 1.82-1.67 (*m*, 2H), 1.02 (*t*, *J* = 7.4 Hz, 3H). High resolution mass spectrum (mass-to-charge ratio): C₂₃H₂₄N₂O₄Na⁺ [M+Na]⁺ Calculated value: 415.1628, Measured value: 415.1628.

### Example 107:

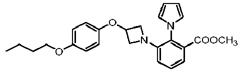

Compound 107 was synthesized according to the General Experimental Operation VIII (35mg, 46%). ¹H NMR (300 MHz, Chloroform-*d*) δ 7.35-7.26 (*m*, 1H), 7.15 (*dd*, *J* = 7.6, 1.3 Hz, 1H), 6.85-6.76 (*m*, 2H), 6.74-6.67 (*m*, 3H), 6.66-6.58 (*m*, 2H), 6.26 (*t*, *J* = 2.1 Hz, 2H), 4.84-4.70 (*m*, 1H), 3.90 (*t*, *J* = 6.5 Hz, 2H), 3.78 (*dd*, *J* = 8.9, 6.3 Hz, 2H), 3.69-3.58 (*m*, 5H), 1.80-1.69 (*m*, 2H), 1.55-1.41 (*m*, 2H), 0.97 (*t*, *J* = 7.4 Hz, 3H). High resolution mass spectrum (mass-to-charge ratio): C₂₅H₂₈N₂O₄Na⁺ [M+Na]⁺ Calculated value: 443.1941, Measured value: 443.1942.

### Example 108:

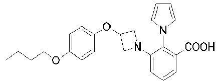

Compound 108 was synthesized according to the General Experimental Operation VIII (8mg, 55%). ¹H NMR (300 MHz, *Methanol-d4*) δ 7.30 (*t*, *J* = 7.9 Hz, 1H), 7.04 (*dd*, *J* = 7.6, 1.1 Hz, 1H), 6.85-6.77 (*m*, 2H), 6.76-6.69 (*m*, 3H), 6.69-6.60 (*m*, 2H), 6.18 (*t*, *J* = 2.0 Hz, 2H), 4.82-4.70 (*m*, 1H), 3.90 (*t*, *J* = 6.4 Hz, 2H), 3.77 (*dd*, *J* = 8.9, 6.2 Hz, 2H), 3.50 (*dd*, *J* = 9.0, 4.5 Hz, 2H), 1.80-1.65 (*m*, 2H), 1.56-1.41 (*m*, 2H), 0.98 (*t*, *J* = 7.4 Hz, 3H). High resolution mass spectrum (mass-to-charge ratio): C₂₄H₂₆N₂O₄Na⁺ [M+Na]⁺ Calculated value: 429.1785, Measured value: 429.1783.

### Example 109:

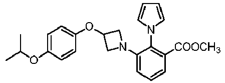

Compound 109 was synthesized according to the General Experimental Operation VIII (25mg, 35%). ¹H NMR (300 MHz, Chloroform-*d*) δ 7.37-7.24 (*m*, 1H), 7.15 (*dd*, *J* = 7.6, 1.2 Hz, 1H), 6.84-6.75 (*m*, 2H), 6.75-6.66 (*m*, 3H), 6.65-6.57 (*m*, 2H), 6.26 (*t*, *J* = 2.1 Hz, 2H), 4.87-4.69 (*m*, 1H), 4.42 (*dt*, *J* = 12.1, 6.1 Hz, 1H), 3.78 (*dd*, *J* = 8.8, 6.3 Hz, 2H), 3.69-3.58 (*m*, 5H), 1.31 (*d*, *J* = 6.1 Hz, 6H). High resolution mass spectrum (mass-to-charge ratio): C₂₄H₂₆N₂O₄Na⁺ [M+Na]⁺ Calculated value: 429.1785, Measured value: 429.1785.

### Example 110:

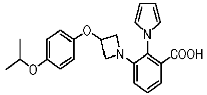

Compound 110 was synthesized according to the General Experimental Operation VIII (6mg, 52%). ¹H NMR (400 MHz, *Methanol-d4*) δ 7.30 (*t*, *J* = 7.9 Hz, 1H), 7.04 (*d*, *J* = 6.7 Hz, 1H), 6.82-6.76 (*m*, 2H), 6.76-6.72 (*m*, 1H), 6.70 (*t*, *J* = 2.0 Hz, 2H), 6.66-6.59 (*m*, 2H), 6.18 (*t*, *J* = 2.0 Hz, 2H), 4.79-4.69 (*m*, 1H), 4.44 (*dq*, *J* = 12.1, 6.1 Hz, 1H), 3.77 (*dd*, *J* = 8.8, 6.2 Hz, 2H), 3.50 (*dd*, *J* = 8.9, 4.5 Hz, 2H), 1.25 (*d*, *J* = 6.0 Hz, 6H). High resolution mass spectrum (mass-to-charge ratio): C₂₃H₂₄N₂O₄Na⁺ [M+Na]⁺ Calculated value: 415.1628, Measured value: 415.1628.

### Example 111:

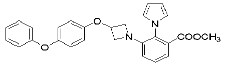

Compound 111 was synthesized according to the General Experimental Operation VIII (40mg, 49%). ¹H NMR (300 MHz, Chloroform-*d*) δ 7.36-7.26 (*m*, 3H), 7.17 (*dd*, *J* = 7.6, 1.3 Hz, 1H), 7.07 (*t*, *J* = 7.4 Hz, 1H), 7.01-6.88 (*m*, 4H), 6.78-6.60 (*m*, 5H), 6.27 (*t*, *J* = 2.1 Hz, 2H), 4.88-4.75 (*m*, 1H), 3.80 (*dd*, *J* = 8.9, 6.2 Hz, 2H), 3.67 (*dd*, *J* = 9.0, 4.9 Hz, 2H), 3.63 (*s*, 3H). High resolution mass spectrum (mass-to-charge ratio): C₂₇H₂₄N₂O₄Na⁺ [M+Na]⁺ Calculated value: 463.1628, Measured value: 463.1628.

### Example 112:

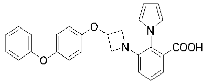

Compound 112 was synthesized according to the General Experimental Operation VIII (15mg, 77%). ¹H NMR (400 MHz, *Methanol-d4*) δ 7.36-7.23 (*m*, 3H), 7.08-6.98 (*m*, 2H), 6.95-6.84 (*m*, 4H), 6.78-6.67 (*m*, 5H), 6.18 (*t*, *J* = 2.0 Hz, 2H), 4.85-4.78 (*m*, 1H), 3.80 (*dd*, *J* = 9.0, 6.2 Hz, 2H), 3.52 (*dd*, *J* = 9.0, 4.4 Hz, 2H). High resolution mass spectrum (mass-to-charge ratio): C₂₆H₂₂N₂O₄Na⁺ [M+Na]⁺ Calculated value: 449.1472, Measured value: 449.1472.

### Example 113:

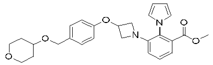

Compound 113 was synthesized according to the General Experimental Operation VIII (4mg, 12%). ¹H NMR (300 MHz, Chloroform-*d*) δ 7.34-7.27 (m, 2H), 7.24 (d, *J* = 2.1 Hz, 2H), 7.16 (dd, *J* = 7.7, 1.4 Hz, 1H), 6.73-6.62 (m, 4H), 6.25 (q, *J* = 2.1 Hz, 2H), 4.84 (p, *J* = 5.5 Hz, 1H), 4.48 (s, 2H), 3.97 (dt, *J* = 11.6, 4.3 Hz, 2H), 3.80 (dd, *J* = 8.7, 6.1 Hz, 2H), 3.70-3.60 (m, 5H), 3.60-3.56 (m, 1H), 3.49-3.39 (m, 2H), 1.93 (dd, *J* = 13.5, 3.5 Hz, 2H), 1.66 (ddd, *J* = 13.3, 9.0, 4.1 Hz, 2H). High resolution mass spectrum (mass-to-charge ratio): C₂₇H₃₀N₂NaO₅⁺ [M+Na]⁺ Calculated value: 485.2047, Measured value: 485.2047

### Example 114:

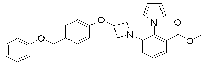

Compound 114 was synthesized according to the General Experimental Operation VIII (4.1mg, yield 34%)¹H NMR (400 MHz, Chloroform-*d*) δ 7.39-7.19 (m, 4H), 7.19-7.12 (m, 1H), 7.02-6.92 (m, 2H), 6.88-6.79 (m, 2H), 6.76-6.65 (m, 5H), 6.26 (t, *J* = 2.1 Hz, 2H), 4.97 (s, 2H), 4.90-4.79 (m, 1H), 3.85-3.77 (m, 2H), 3.70-3.64 (m, 2H), 3.62 (s, 3H). High resolution mass spectrum (mass-to-charge ratio): C₂₈H₂₇N₂O₄⁺ [M+H]⁺ Calculated value: 455.1965, Measured value: 455.1961.

### Example 115:

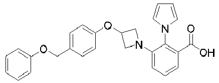

Compound 115 was synthesized according to the General Experimental Operation VIII (2.5mg, yield 80%)¹H NMR (300 MHz, Chloroform-*d*) δ 7.45-7.20 (m, 6H), 7.06-6.90 (m, 3H), 6.81-6.61 (m, 5H), 6.36-6.20 (m, 2H), 4.97 (s, 2H), 4.90-4.79 (m, 1H), 3.88-3.73 (m, 2H), 3.71-3.57 (m, 2H). High resolution mass spectrum (mass-to-charge ratio): C₂₇H₂₅N₂O₄⁺ [M+H]⁺ Calculated value: 441.1809, Measured value: 441.1806.

### Example 116:

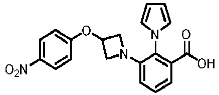

Compound 116 was synthesized according to the General Experimental Operation IX (8mg, 83%). ¹H NMR (500 MHz, *Methanol-d4*) δ 8.25-8.14 (*m*, 2H), 7.31 (*t*, *J* = 7.9 Hz, 1H), 7.06 (*dd*, *J* = 7.6, 1.2 Hz, 1H), 6.94-6.89 (*m*, 2H), 6.75 (*dd*, *J* = 8.2, 1.1 Hz, 1H), 6.72 (*t*, *J* = 2.1 Hz, 2H), 6.17 (*t*, *J* = 2.1 Hz, 2H), 5.03-4.97 (*m*, 1H), 3.89 (*dd*, *J* = 9.5, 6.1 Hz, 2H), 3.55 (*dd*, *J* = 9.5, 4.1 Hz, 2H). High resolution mass spectrum (mass-to-charge ratio): C₂₀H₁₇N₃O₅Na⁺ [M+Na]⁺ Calculated value: 402.1060, Measured value: 402.1057.

### Example 117:

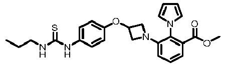

Compound 117 was synthesized according to the General Experimental Operation X (14mg, 55%). ¹H NMR (500 MHz, Chloroform-*d*) δ 7.53 (*s*, 1H), 7.30 (*t*, *J* = 7.9 Hz, 1H), 7.16 (*d*, *J* = 7.6 Hz, 1H), 7.14-7.08 (*m*, 2H), 6.77-6.65 (*m*, 5H), 6.25 (*t*, *J* = 1.9 Hz, 2H), 5.79 (*s*, 1H), 4.87-4.76 (*m*, 1H), 3.85-3.77 (*m*, 2H), 3.65 (*dd*, *J* = 8.7, 4.7 Hz, 2H), 3.61 (*s*, 3H), 3.57 (*dd*, *J* = 12.9, 6.2 Hz, 2H), 1.62-1.58 (*m*, 2H), 0.89 (*t*, *J* = 7.4 Hz, 3H). High resolution mass spectrum (mass-to-charge ratio): C₂₅H₂₉N₄O₃S⁺ [M+H]⁺ Calculated value: 465.1955, Measured value: 465.1957.

### Example 118:

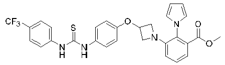

Compound 118 was synthesized according to the General Experimental Operation X (17mg, 55%). ¹H NMR (400 MHz, Chloroform-*d*) δ 8.13 (*s*, 1H), 7.78 (s, 1H), 7.59 (*q*, *J* = 8.7 Hz, 4H), 7.30 (*t*, *J* = 7.9 Hz, 1H), 7.24 (*t*, *J* = 6.1 Hz, 2H), 7.16 (*dd*, *J* = 7.7, 1.3 Hz, 1H), 6.75 (*dd*, *J* = 7.1, 5.0 Hz, 2H), 6.71-6.64 (*m*, 3H), 6.25 (*t*, *J* = 2.1 Hz, 2H), 4.87-4.78 (*m*, 1H), 3.81 (*dd*, *J* = 9.0, 6.3 Hz, 2H), 3.65 (*dd*, *J* = 9.0, 4.8 Hz, 2H), 3.61 (*s*, 3H). High resolution mass spectrum (mass-to-charge ratio): C₂₉H₂₅F₃N₄O₃SNa⁺ [M+Na]⁺ Calculated value: 589.1492, Measured value: 589.1494.

### Example 119:

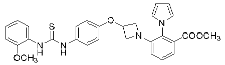

Compound 119 was synthesized according to the General Experimental Operation X (15mg, 52%). ¹H NMR (300 MHz, Chloroform-*d*) δ 8.10 (*d*, *J* = 6.4 Hz, 1H), 7.92 (*s*, 1H), 7.77 (*s*, 1H), 7.36-7.23 (*m*, 3H), 7.22-7.13 (*m*, 2H), 7.00 (*td*, *J* = 7.8, 1.2 Hz, 1H), 6.95-6.85 (*m*, 1H), 6.80-6.65 (*m*, 5H), 6.26 (*t*, *J* = 2.1 Hz, 2H), 4.91-4.78 (*m*, 1H), 3.87-3.74 (*m*, 5H), 3.66 (*dd*, *J* = 9.1, 4.8 Hz, 2H), 3.62 (*s*, 3H). High resolution mass spectrum (mass-to-charge ratio): C₂₉H₂₈N₄O₄S Na⁺ [M+Na]⁺ Calculated value: 551.1723, Measured value: 551.1726.

### Example 120:

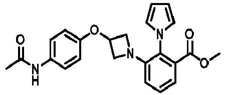

Compound 120 was synthesized according to the General Experimental Operation X (12mg, 75%). ¹H NMR (500 MHz, Chloroform-*d*) *δ* 7.36 (*d*, *J* = 8.9 Hz, 2H), 7.29 (*t*, *J* = 7.9 Hz, 1H), 7.15 (*dd*, *J* = 7.7, 1.4 Hz, 2H), 6.76-6.61 (*m*, 5H), 6.25 (*t*, *J* = 2.1 Hz, 2H), 4.83-4.78 (*m*, 1H), 3.81-3.76 (*m*, 2H), 3.66 -3.60 (*m*, 5H), 2.15 (*s*, 3H). High resolution mass spectrum (mass-to-charge ratio): C₂₃H₂₃N₃O₄Na⁺ [M+Na]⁺ Calculated value: 428.1586, Measured value: 428.1581.

### Example 121:

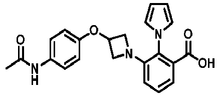

Compound 121 was synthesized according to the General Experimental Operation X (5mg, 74%). ¹H NMR (300 MHz, Methanol-*d₄*) *δ* 7.39 (*d*, *J* = 8.4 Hz, 2H), 7.30 (*t*, *J* = 7.9 Hz, 1H), 7.02 (*d*, *J* = 7.5 Hz, 1H), 6.74-6.66 (*m*, 5H), 6.17 (*s*, 2H), 4.86-4.79 (*m*, 1H), 3.79 (*dd*, *J* = 8.6, 6.1 Hz, 2H), 3.49 (*dd*, *J* = 8.7, 4.4 Hz, 2H), 2.08 (*s*, 3H). High resolution mass spectrum (mass-to-charge ratio): C₂₂H₂₁N₃O₄Na⁺ [M+Na]⁺ Calculated value: 414.1430,Measured value: 414.1422.

### Example 122:

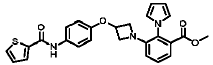

Compound 122 was synthesized according to the General Experimental Operation X (11mg, 58%). ¹H NMR (300 MHz, Chloroform-*d*) *δ* 7.64-7.51 (*m*, 3H), 7.47 (*d*, *J* = 8.9 Hz, 2H), 7.29 (*t*, *J* = 7.9 Hz, 1H), 7.17-7.07 (*m*, 2H), 6.73-6.64 (*m*, 4H), 6.25 (*t*, *J* = 2.1 Hz, 2H), 4.86-4.79 (*m*, 1H), 3.80 (*dd*, *J* = 8.7, 6.2 Hz, 2H), 3.71-3.51 (*m*, 5H). High resolution mass spectrum (mass-to-charge ratio): C₂₆H₂₃N₃O₄SNa⁺ [M+Na]⁺ Calculated value: 496.1307, Measured value: 496.1302.

### Example 123:

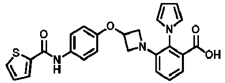

Compound 123 was synthesized according to the General Experimental Operation X (5mg, 71%). ¹H NMR (300 MHz, Chloroform-*d*) *δ* 7.62-7.58 (*m*, 1H), 7.54 (*d*, *J* = 4.1 Hz, 1H), 7.48 (*d*, *J* = 8.9 Hz, 2H), 7.34-7.29 (*m*, 2H), 7.13 (*dd*, *J* = 5.0, 3.8 Hz, 1H), 6.74 (*t*, *J* = 2.1 Hz, 2H), 6.69 (*d*, *J* = 9.0 Hz, 2H), 6.29 (*t*, *J* = 2.0 Hz, 2H), 4.86-4.79 (*m*, 1H), 3.83-3.75 (*m*, 2H), 3.65 (*dd*, *J* = 8.8, 4.9 Hz, 2H). High resolution mass spectrum (mass-to-charge ratio): C₂₅H₂₁N₃O₄SNa⁺ [M+Na]⁺ Calculated value: 482.1150,Measured value: 482.1144.

### Example 124:

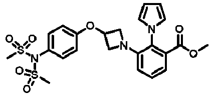

Compound 124 was synthesized according to the General Experimental Operation X (12mg, 57%). ¹H NMR (300 MHz, Chloroform-*d*) *δ* 7.30 (*t*, *J* = 7.9 Hz, 1H), 7.24 (*d*, *J* = 9.4 Hz, 2H), 7.17 (*dd*, *J* = 7.7, 1.4 Hz, 1H), 6.77-6.66 (*m*, 5H), 6.26 (*t*, *J* = 2.1 Hz, 2H), 4.87-4.80 (*m*, 1H), 3.80 (*dd*, *J* = 8.7, 6.2 Hz, 2H), 3.68-3.62 (*m*, 5H), 3.38 (*s*, 6H). High resolution mass spectrum (mass-to-charge ratio): C₂₃H₂₅N₃O₇S₂Na⁺ [M+Na]⁺ Calculated value: 542.1032, Measured value: 542.1028.

### Example 125:

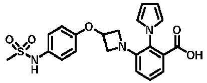

Compound 125 was synthesized according to the General Experimental Operation X (6mg, 91%). ¹H NMR (300 MHz, Chloroform-*d*) *δ* 7.31 (*dd*, *J* = 17.2, 9.3 Hz, 2H), 7.13 (*d*, *J* = 8.4 Hz, 2H), 6.76-6.60 (*m*, 5H), 6.23 (*s*, 2H), 4.80 (*t*, *J* = 5.6 Hz, 1H), 3.80-3.69 (*m*, 2H), 3.63 (*dd*, *J* = 8.7, 4.8 Hz, 2H), 2.93 (*s*, 3H). High resolution mass spectrum (mass-to-charge ratio): C₂₁H₂₁N₃O₅SNa⁺ [M+Na]⁺ Calculated value: 450.1100,Measured value: 450.1095.

### Example 126:

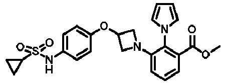

Compound 126 was synthesized according to the General Experimental Operation X (11mg, 58%). ¹H NMR (300 MHz, Chloroform-*d*) *δ* 7.29 (*t*, *J* = 7.9 Hz, 1H), 7.21-7.14 (*m*, 3H), 6.70 (*t*, *J* = 2.1 Hz, 2H), 6.68-6.61 (*m,* 2H), 6.31 (*s,* 1H), 6.25 (*t*, *J* = 2.1 Hz, 2H), 4.80 (*tt*, *J* = 6.1, 4.7 Hz, 1H), 3.87-3.75 (*m,* 2H), 3.68-3.57 (*m,* 5H), 2.41 (*tt*, *J* = 8.0, 4.8 Hz, 1H), 1.10 (*dt*, *J* = 7.0, 3.3 Hz, 2H), 0.94 (*dt*, *J* = 7.8, 3.2 Hz, 2H). High resolution mass spectrum (mass-to-charge ratio): C₂₄H₂₅N₃O₅SNa⁺ [M+Na]⁺ Calculated value: 490.1413, Measured value: 490.1407.

### Example 127:

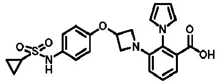

Compound 127 was synthesized according to the General Experimental Operation X (6mg, 88%). ¹H NMR (500 MHz, Chloroform-*d*) *δ* 7.36-7.23 (*m,* 3H), 7.17 (*d, J* = 8.3 Hz, 2H), 6.72 (*s*, 2H), 6.66 (*d, J* = 8.4 Hz, 2H), 6.50 (*s*, 1H), 6.23 (*s*, 2H), 4.91-4.76 (*m,* 1H), 3.78 (*d*, *J* = 7.2 Hz, 2H), 3.64 (*dd, J* = 8.8, 4.7 Hz, 2H), 2.51-2.31 (*m,* 1H), 1.10 (*d, J* = 5.2 Hz, 2H), 0.93 (*d, J* = 7.6 Hz, 2H). High resolution mass spectrum (mass-to-charge ratio): C₂₃H₂₃N₃O₅SNa⁺ [M+Na]⁺ Calculated value: 476.1256, Measured value: 476.1248.

### Example 128:

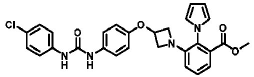

Compound 128 was synthesized according to the General Experimental Operation X (14mg, 71%). ¹H NMR (300 MHz, Chloroform-*d*) *δ* 7.29 (*dd, J* = 8.4, 5.1 Hz, 4H), 7.22 (*d, J* = 8.9 Hz, 2H), 7.16 (*dd, J* = 7.7, 1.2 Hz, 1H), 6.76-6.62 *(m,* 4H), 6.41 (*s*, 1H), 6.31-6.18 (*m,* 2H), 4.87-4.77 (*m,* 1H), 3.85-3.76 (*m,* 2H), 3.70-3.54 (*m,* 5H). High resolution mass spectrum (mass-to-charge ratio): C₂₈H₂₅ClN₄O₄Na⁺ [M+Na]⁺ Calculated value: 539.1462, Measured value: 539.1458.

### Example 129:

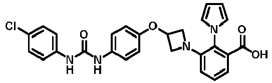

Compound 129 was synthesized according to the General Experimental Operation X (5mg, 63%). ¹H NMR (300 MHz, Methanol-*d₄*) *δ* 7.40 (*d, J* = 8.8 Hz, 2H), 7.27 (*t, J* = 8.7 Hz, 5H), 6.76-6.65 (*m,* 5H), 6.17 (*d, J* = 2.1 Hz, 2H), 4.68-4.59 (*m,* 1H), 3.66-3.64 (*m,* 2H), 3.51-3.47 (*m*, 2H). High resolution mass spectrum (mass-to-charge ratio): C₂₇H₂₃ClN₄O₄Na⁺ [M+Na]⁺ Calculated value: 525.1306, Measured value: 525.1304.

### Example 130:

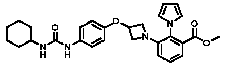

Compound 130 was synthesized according to the General Experimental Operation X (15mg, 75%). ¹H NMR (300 MHz, Chloroform-*d*) *δ* 7.31 (*m,* 1H), 7.14 (*d, J* = 8.2 Hz, 3H), 6.75-6.56 (*m,* 6H), 6.44 (*s*, 1H), 6.24 (*s*, 2H), 4.79-4.74 (*m,* 1H), 4.67 (*d, J* = 8.0 Hz, 1H), 3.80-3.75 (*m,* 2H), 3.65-3.61 (*m,* 5H), 1.94-1.92 (*m,* 2H), 1.64-1.50 (*m,* 2H), 1.35-1.31 (*m*, 2H), 1.16-1.03 (*m*, 4H). High resolution mass spectrum (mass-to-charge ratio): C₂₇H₂₃ClN₄O₄Na⁺ [M+Na]⁺ Calculated value: 525.1306, Measured value: 525.1304.

### Example 131:

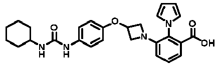

Compound 131 was synthesized according to the General Experimental Operation X (6mg, 60%). ¹H NMR (300 MHz, Methanol-*d4*) *δ* 7.30 (*t, J* = 7.8 Hz, 1H), 7.19 (*d, J* = 9.0 Hz, 2H), 6.99 (*d, J* = 7.3 Hz, 1H), 6.75-6.67 (*m,* 3H), 6.67-6.61 (*m,* 2H), 6.16 (*t, J* = 2.0 Hz, 2H), 4.82-4.75 (*m,* 1H), 3.80-3.72 (*m,* 2H), 3.48 (*dd*, *J* = 8.8, 4.4 Hz, 2H), 1.92-1.84 (*m,* 2H), 1.80-1.69 (*m,* 2H), 1.64-1.59 (*m,* 2H), 1.46-1.11 (*m,* 4H). High resolution mass spectrum (mass-to-charge ratio): C₂₇H₃₀N₄O₄Na⁺ [M+Na]⁺ Calculated value: 497.2165, Measured value: 497.2153.

### Example 132:

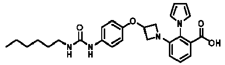

Compound 132 was synthesized according to the General Experimental Operation X (5mg, 53%). ¹H NMR (500 MHz, Methanol-*d4*) *δ* 7.31 (*t, J* = 7.9 Hz, 1H), 7.20 (*d, J* = 8.9 Hz, 2H), 7.06 (*d, J* = 7.6 Hz, 1H), 6.75 (*d, J* = 8.1 Hz, 1H), 6.71 (*t*, *J* = 2.0 Hz, 2H), 6.65 (*d, J* = 8.9 Hz, 2H), 6.18 (*t*, *J* = 2.0 Hz, 2H), 4.80 (*t*, *J* = 5.2 Hz, 1H), 3.82-3.78 (*m,* 2H), 3.51 (*dd, J* = 8.8, 4.4 Hz, 2H), 3.16 (*t, J* = 7.0 Hz, 2H), 1.50 (*dd*, *J* = 14.3, 7.1 Hz, 2H), 1.35 (*d, J* = 9.0 Hz, 6H), 0.92 (*t*, *J* = 6.9 Hz, 3H). High resolution mass spectrum (mass-to-charge ratio): C₂₇H₃₂N₄O₄Na⁺ [M+Na]⁺ Calculated value: 499.2321, Measured value: 499.2315.

### Example 133:

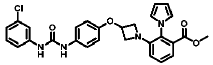

Compound 133 was synthesized according to the General Experimental Operation X (13mg, 66%). ¹H NMR (300 MHz, Chloroform-*d*) *δ* 7.39 (*s*, 1H), 7.31 (*d, J* = 7.1 Hz, 1H), 7.20-7.14 (*m,* 5H), 6.79-6.61 (*m,* 5H), 6.57 (*s*, 1H), 6.25 (*s*, 2H), 4.81-4.74 (*m,* 1H), 3.81-3.76 (*m*, *J* = 7.3 Hz, 2H), 3.64-3.62 (*m,* 5H). High resolution mass spectrum (mass-to-charge ratio): C₂₈H₂₅ClN₄O₄Na⁺ [M+Na]⁺ Calculated value: 539.1462, Measured value: 539.1458.

### Example 134:

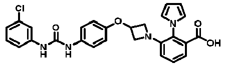

Compound 134 was synthesized according to the General Experimental Operation X (5mg, 64%). ¹H NMR (500 MHz, Methanol-*d₄*) *δ* 7.62 (*s*, 1H), 7.35-7.28 (*m,* 3H), 7.24 (*dd, J* = 4.9, 1.4 Hz, 2H), 7.06 (*dd*, *J* = 7.5, 1.3 Hz, 1H), 7.00-6.96 (*m,* 1H), 6.78-6.75 (*m,* 1H), 6.74-6.67 (*m,* 4H), 6.19 (*t, J* = 2.1 Hz, 2H), 4.84-4.80 (*m,* 1H), 3.81 (*dd*, *J* = 8.8, 6.1 Hz, 2H), 3.52 (*dd*, *J* = 8.9, 4.4 Hz, 2H). High resolution mass spectrum (mass-to-charge ratio): C₂₇H₂₃ClN₄O₄Na⁺ [M+Na]⁺ Calculated value: 525.1306,Measured value: 525.1304.

### Example 135:

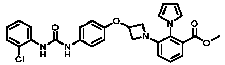

Compound 135 was synthesized according to the General Experimental Operation X (13mg, 66%). ¹H NMR (400 MHz, Chloroform-*d*) *δ* 8.19 (*d, J* = 8.2 Hz, 1H), 7.35-7.21 (*m,* 2H), 7.15 (*d, J* = 7.6 Hz, 1H), 7.06 (*s*, 1H), 6.98 (*t, J* = 7.7 Hz, 1H), 6.69 (*dd*, *J* = 9.2, 3.6 Hz, 5H), 6.59 (*s*, 1H), 6.32-6.15 (*m,* 2H), 4.82 (*t, J* = 5.6 Hz, 1H), 3.80 (*t, J* = 7.5 Hz, 2H), 3.63 (*d, J* = 13.2 Hz, 5H). High resolution mass spectrum (mass-to-charge ratio): C₂₈H₂₅ClN₄O₄Na⁺ [M+Na]⁺ Calculated value: 539.1462, Measured value: 539.1459.

### Example 136:

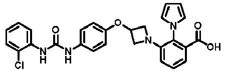

Compound 136 was synthesized according to the General Experimental Operation X (5mg, 55%). ¹H NMR (500 MHz, Methanol-*d₄*) *δ* 8.09 (*d, J* = 8.2 Hz, 1H), 7.39 (*d, J* = 8.0 Hz, 1H), 7.34-7.23 (*m,* 4H), 7.04-7.00 (*m,* 2H), 6.79-6.63 (*m,* 5H), 6.19 (*s*, 2H), 4.81 (*m,* 1H), 3.81 (*dd*, *J* = 8.5, 6.2 Hz, 2H), 3.51 (*dd*, *J* = 8.7, 4.4 Hz, 2H). High resolution mass spectrum (mass-to-charge ratio): C₂₇H₂₃ClN₄O₄Na⁺ [M+Na]⁺ Calculated value: 525.1306, Measured value: 525.1302.

### Example 137:

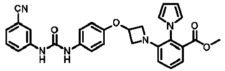

Compound 137 was synthesized according to the General Experimental Operation X (12mg, 57%). ¹H NMR (500 MHz, Chloroform-*d*) *δ* 7.67-7.54 (*m,* 2H), 7.40-7.23 (*m,* 2H), 7.18-7.15 (*m,* 3H), 6.89 (*s*, 1H), 6.77-6.58 (*m,* 5H), 6.25 (*s*, 2H), 4.76 (*d, J* = 7.8 Hz, 1H), 3.78 (*t, J* = 7.2 Hz, 2H), 3.69-3.55 *(m,* 5H). High resolution mass spectrum (mass-to-charge ratio): C₂₉H₂₅N₅O₄Na⁺ [M+Na]⁺ Calculated value: 530.1804, Measured value: 530.1801.

### Example 138:

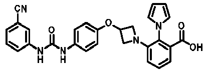

Compound 138 was synthesized according to the General Experimental Operation X (4mg, 51%). ¹H NMR (300 MHz, Methanol-*d₄*) *δ* 7.94 (*t, J* = 1.9 Hz, 1H), 7.61 (*d, J* = 8.4 Hz, 1H), 7.44 (*t, J* = 8.0 Hz, 1H), 7.33-7.27 (*m,* 4H), 6.99 (*dd*, *J* = 12.5, 7.6 Hz, 1H), 6.83 (*d, J* = 8.7 Hz, 1H), 6.78-6.64 (*m,* 4H), 6.27-6.15 (*m,* 2H), 4.86-4.79 (*m,* 1H), 3.82-3.77 (*m,* 2H), 3.50 (*dd, J* = 8.8, 4.4 Hz, 2H). High resolution mass spectrum (mass-to-charge ratio): C₂₈H₂₃N₅O₄Na⁺ [M+Na]⁺ Calculated value: 516.1648, Measured value: 516.1629.

### Example 139:

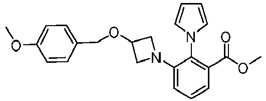

Compound 139 was synthesized according to the General Experimental Operation XI (8.0mg, 75%). ¹H NMR (300 MHz, Chloroform-*d*) δ 7.32-7.18 (m, 4H), 6.92-6.81 (m, 2H), 6.74-6.60 (m, 3H), 6.23 (t, *J* = 2.1 Hz, 2H), 4.32 (s, 2H), 4.28-4.20 (m, 1H), 3.81 (s, 3H), 3.53-3.42 (m, 4H). High resolution mass spectrum (mass-to-charge ratio): C₂₂H₂₂N₂NaO₄⁺ [M+H]⁺ Calculated value: 401.1472,Measured value: 401.1472.

### Example 140:

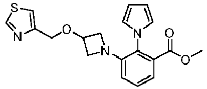

Compound 140 was synthesized according to the General Experimental Operation XI (4mg, 30%). ¹H NMR (300 MHz, Chloroform-d) δ 8.80 (s, 1H), 7.75 (s, 1H), 7.29 (d, *J* = 8.0 Hz, 1H), 7.14 (dd, *J* = 7.7, 1.5 Hz, 1H), 6.70-6.61 (m, 3H), 6.24 (t, *J* = 2.2 Hz, 2H), 4.62 (s, 2H), 4.35-4.20 (m, 1H), 3.62 (s, 3H), 3.60-3.43 (m, 4H). High resolution mass spectrum (mass-to-charge ratio): C₁₉H₂₀N₃O₃S⁺ [M+H]⁺ Calculated value: 370.1220, Measured value: 370.1223.

### Example 141:

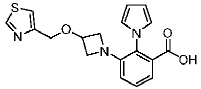

Compound 141 was synthesized according to the General Experimental Operation XI (1.7mg, 59%). ¹H NMR (500 MHz, Chloroform-*d*) δ 8.79 (s, 1H), 7.74 (s, 1H), 7.32-7.26 (m, 2H), 6.75-6.61 (m, 3H), 6.26 (t, *J* = 2.1 Hz, 2H), 4.62 (s, 2H), 4.32-4.22 (m, 1H), 3.54 (t, *J* = 7.3 Hz, 2H), 3.46 (dd, *J* = 8.4, 5.0 Hz, 2H). High resolution mass spectrum (mass-to-charge ratio): C₁₈H₁₆N₃O₃S⁻[M-H]⁻ Calculated value: 354.0918, Measured value: 354.0920.

### Example 142:

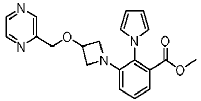

Compound 142 was synthesized according to the General Experimental Operation XI (12mg, 90%). ¹H NMR (500 MHz, Chloroform-*d*) δ 8.65 (s, 1H), 8.49 (s, 2H), 7.33-7.21 (m, 1H), 7.12 (dd, *J* = 7.7, 1.5 Hz, 1H), 6.74-6.56 (m, 3H), 6.22 (t, *J* = 2.1 Hz, 2H), 4.53 (s, 2H), 4.41-4.30 (m, 1H), 3.65-3.56 (m, 5H), 3.52 (dd, *J* = 8.6, 4.9 Hz, 2H).High resolution mass spectrum (mass-to-charge ratio): C₂₀H₂₁N₄O₃⁺ [M+H]⁺ Calculated value: 365.1608, Measured value: 365.1612.

### Example 143:

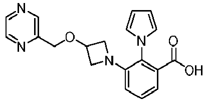

Compound 143 was synthesized according to the General Experimental Operation XI (8mg, 83%). ¹H NMR (300 MHz, Chloroform-*d*) δ 8.67 (s, 1H), 8.53 (d, *J* = 1.4 Hz, 2H), 7.38-7.19 (m, 2H), 6.84-6.57 (m, 3H), 6.24 (t, *J* = 2.1 Hz, 2H), 4.55 (s, 2H), 4.36 (t, *J* = 5.5 Hz, 1H), 3.72-3.39 (m, 4H). High resolution mass spectrum (mass-to-charge ratio): C₁₉H₁₉N₄O₃⁺ [M+H]⁺ Calculated value: 351.1452, Measured value: 351.1441.

### Example 144:

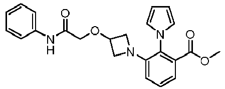

Compound 144 was synthesized according to the General Experimental Operation XI (3mg, 20%). ¹H NMR (300 MHz, Chloroform-*d*) δ 8.13 (s, 1H), 7.63-7.46 (m, 2H), 7.35-7.28 (m, 3H), 7.22-7.09 (m, 2H), 6.80-6.59 (m, 3H), 6.26 (t, *J* = 2.1 Hz, 2H), 4.32 (ddd, *J* = 10.5, 6.2, 4.6 Hz, 1H), 3.97 (s, 2H), 3.71-3.59 (m, 2H), 3.62 (s, 3H), 3.54 (dd, *J* = 8.8, 4.4 Hz, 2H). High resolution mass spectrum (mass-to-charge ratio): C₂₃H₂₄N₃O₄⁺ [M+H]⁺ Calculated value: 406.1761, Measured value: 406.1754.

### Example 145:

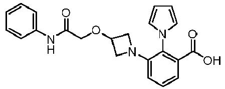

Compound 145 was synthesized according to the General Experimental Operation XI (0.8mg, yield 50%)¹H NMR (400 MHz, Chloroform-*d*) δ 8.12 (s, 1H), 7.53 (d, *J* = 7.8 Hz, 2H), 7.33 (t, *J* = 7.7 Hz, 2H), 7.14 (d, *J* = 7.5 Hz, 2H), 6.67 (d, *J* = 29.8 Hz, 3H), 6.18 (s, 2H), 4.36-4.23 (m, 2H), 3.94 (s, 2H), 3.64-3.57 (m, 2H), 3.53-3.44 (m, 2H). High resolution mass spectrum (mass-to-charge ratio): C₂₂H₂₁N₃NaO₄⁺ [M+Na]⁺ Calculated value: 414.1424, Measured value: 414.1418.

### Example 146:

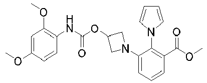

Compound 146 was synthesized according to the General Experimental Operation XI (14mg, 84%). ¹H NMR (300 MHz, Chloroform-d) δ 7.86 (d, *J* = 8.5 Hz, 1H), 7.36-7.24 (m, 1H), 7.15 (dd, *J* = 7.6, 1.4 Hz, 1H), 7.02 (brs, 1H), 6.75-6.61 (m, 3H), 6.51-6.40 (m, 2H), 6.26 (t, *J* = 2.1 Hz, 2H), 5.17 (tt, *J* = 6.4, 4.5 Hz, 1H), 3.85-3.77 (m, 8H), 3.65-3.53 (m, 5H). High resolution mass spectrum (mass-to-charge ratio): C₂₄H₂₆N₃O₆⁺ [M+H]⁺ Calculated value: 452.1816, Measured value: 452.1794

### Example 147:

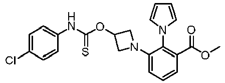

Compound 147 was synthesized according to the General Experimental Operation XI (15mg, yield 93%). ¹H NMR (400 MHz, Chloroform-*d*) δ 7.58-7.41 (m, 1H), 7.33-7.27 (m, 3H), 7.20-7.09 (m, 2H), 6.75-6.58 (m, 3H), 6.26 (t, *J* = 2.1 Hz, 2H), 5.50 (s, 1H), 3.84 (dd, *J* = 9.2, 6.5 Hz, 2H), 3.65-3.53 (m, 5H). High resolution mass spectrum (mass-to-charge ratio): C₂₂H₂₁ClN₃O₃S⁺ [M+H]⁺ Calculated value: 442.0987, Measured value: 442.0975.

### Example 148:

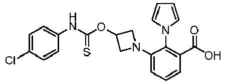

Compound 148 was synthesized according to the General Experimental Operation XI (1.7mg, 59%). ¹H NMR (300 MHz, Chloroform-d) δ 7.41-7.21 (m, 5H), 7.01-6.94 (m, 1H), 6.94-6.83 (m, 2H), 6.72-6.64 (m, 2H), 6.36 (t, *J* = 2.1 Hz, 2H), 4.79 (dq, *J* = 8.1, 5.7 Hz, 1H), 3.58-3.48 (m, 2H), 3.44-3.32 (m, 1H), 3.19 (dd, *J =* 11.0, 8.1 Hz, 1H). High resolution mass spectrum (mass-to-charge ratio): C₂₁H₁₉ClN₃O₃S⁺ [M+H]⁺ Calculated value: 428.0830, Measured value: 428.0835.

### Example 149:

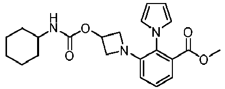

Compound 149 was synthesized according to the General Experimental Operation XI (10mg, yield 68%). ¹H NMR (300 MHz, Chloroform-d) δ 7.36-7.23 (m, 1H), 7.13 (dd, *J* = 7.7, 1.4 Hz, 1H), 6.77-6.56 (m, 3H), 6.24 (t, *J* = 2.2 Hz, 2H), 5.15-4.97 (m, 1H), 4.62 (d, *J* = 8.2 Hz, 1H), 3.74 (dd, *J* = 8.9, 6.4 Hz, 2H), 3.61 (s, 3H), 3.48 (dd, *J* = 9.0, 4.6 Hz, 2H), 1.97-1.82 (m, 2H), 1.77-1.53 (m, 4H), 1.41-1.21 (m, 4H). High resolution mass spectrum (mass-to-charge ratio): C₂₂H₂₈N₃O₄⁺ [M+H]⁺ Calculated value: 398.2074, Measured value: 398.2076.

### Example 150:

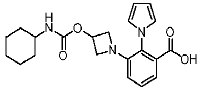

Compound 150 was synthesized according to the General Experimental Operation XI (3mg, yield 63%). ¹H NMR (300 MHz, Chloroform-d) δ 7.39-7.19 (m, 2H), 6.79-6.57 (m, 3H), 6.24 (t, *J* = 2.1 Hz, 2H), 5.06 (td, *J* = 6.5, 3.2 Hz, 1H), 4.61 (d, *J* = 8.3 Hz, 1H), 3.77-3.68 (m, 2H), 3.48 (dd, *J* = 9.2, 4.5 Hz, 2H), 1.88 (ddt, *J* = 9.9, 6.6, 3.7 Hz, 2H), 1.74-1.54 (m, 3H), 1.28 (d, *J* = 14.3 Hz, 5H). High resolution mass spectrum (mass-to-charge ratio): C₂₁H₂₆N₃O₄⁺ [M+H]⁺ Calculated value: 384.1918, Measured value: 384.1922.

### Example 151:

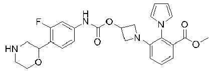

Compound 151 was synthesized according to the General Experimental Operation XI (12mg, yield 68%). ¹H NMR (300 MHz, Chloroform-d) δ 7.31 (d, *J* = 7.9 Hz, 1H), 7.22 (s, 1H), 7.16 (dd, *J* = 7.7, 1.4 Hz, 1H), 7.01-6.81 (m, 2H), 6.76-6.59 (m, 4H), 6.25 (t, *J* = 2.1 Hz, 2H), 5.23-5.09 (m, 1H), 3.90-3.82 (m, 3H), 3.79 (dd, *J* = 9.1, 6.4 Hz, 2H), 3.62 (s, 3H), 3.56-3.46 (m, 2H), 3.10-2.96 (m, 4H). High resolution mass spectrum (mass-to-charge ratio): C₂₆H₂₈FN₄O₅⁺ [M+H]⁺ Calculated value: 495.2038, Measured value: 495.2045.

### Example 152:

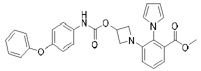

Compound 152 was synthesized according to the General Experimental Operation XI (10mg, 56%). ¹H NMR (300 MHz, Chloroform-d) δ 7.37-7.29 (m, 4H), 7.16 (dd, *J* = 7.7, 1.4 Hz, 1H), 7.09 (t, *J* = 7.4 Hz, 1H), 7.03-6.93 (m, 4H), 6.78-6.60 (m, 4H), 6.26 (t, *J* = 2.1 Hz, 2H), 5.17 (ddt, *J* = 9.0, 6.3, 3.1 Hz, 1H), 3.80 (dd, *J* = 9.0, 6.4 Hz, 2H), 3.70-3.47 (m, 5H). High resolution mass spectrum (mass-to-charge ratio): C₂₈H₂₆N₃O₅⁺ [M+H]⁺ Calculated value: 484.1867, Measured value: 484.1844.

### Example 153:

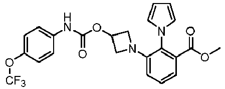

Compound 153 was synthesized according to the General Experimental Operation XI (8mg, 46%). ¹H NMR (500 MHz, Chloroform-*d*) δ 7.39-7.33 (m, 2H), 7.30-7.26 (m, 1H), 7.21-7.10 (m, 3H), 6.74 (s, 1H), 6.69 (t, *J* = 2.1 Hz, 2H), 6.65 (d, *J* = 8.0 Hz, 1H), 6.24 (t, *J* = 2.1 Hz, 2H), 5.21-5.10 (m, 1H), 3.79 (dd, *J* = 9.0, 6.4 Hz, 2H), 3.61 (s, 3H), 3.53 (dd, *J* = 9.1, 4.4 Hz, 2H). High resolution mass spectrum (mass-to-charge ratio): C₂₃H₂₁F₃N₃O₅⁺ [M+H]⁺ Calculated value: 476.1428, Measured value: 476.1444.

### Example 154:

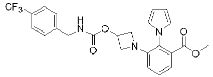

Compound 154 was synthesized according to the General Experimental Operation XI (6mg, 34%). ¹H NMR (300 MHz, Chloroform-*d*) δ 7.60 (d, *J* = 8.0 Hz, 2H), 7.38 (d, *J* = 7.9 Hz, 2H), 7.33-7.26 (m, 1H), 7.15 (dd, *J* = 7.7, 1.4 Hz, 1H), 6.75-6.60 (m, 3H), 6.25 (t, *J* = 2.1 Hz, 2H), 5.24-5.05 (m, 2H), 4.40 (d, *J* = 6.2 Hz, 2H), 3.76 (dd, *J* = 9.0, 6.4 Hz, 2H), 3.62 (s, 3H), 3.51 (dd, *J* = 9.2, 4.5 Hz, 2H). High resolution mass spectrum (mass-to-charge ratio): C₂₄H₂₃F₃N₃O₄⁺ [M+H]⁺ Calculated value: 474.1635, Measured value: 474.1653.

### Example 155:

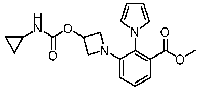

Compound 155 was synthesized according to the General Experimental Operation XI (6mg, 46%). ¹H NMR (400 MHz, Chloroform-*d*) δ 7.30-7.26 (m, 1H), 7.17-7.08 (m, 1H), 6.75-6.57 (m, 3H), 6.23 (t, *J* = 2.2 Hz, 2H), 5.07 (t, *J* = 5.6 Hz, 1H), 4.89 (brs, 1H), 3.73 (dd, *J* = 9.0, 6.5 Hz, 2H), 3.60 (s, 3H), 3.48 (brs, 2H), 2.54 (brs, 1H), 0.74-0.68 (m, 2H), 0.53-0.47 (m, 2H). High resolution mass spectrum (mass-to-charge ratio): C₁₉H₂₁N₃NaO₄⁺ [M+Na]⁺ Calculated value: 378.1424, Measured value: 378.1425.

### Example 156:

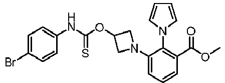

Compound 156 was synthesized according to the General Experimental Operation XI (4.9mg, yield 77%). ¹H NMR (400 MHz, Chloroform-*d*) δ 7.48-7.39 (m, 2H), 7.37-7.33 (m, 1H), 7.24-7.16 (m, 1H), 6.98-6.92 (m, 1H), 6.91-6.77 (m, 2H), 6.72-6.52 (m, 2H), 6.40-6.30 (m, 2H), 4.85-4.73 (m, 1H), 4.06 (t, *J* = 6.5 Hz, 1H), 3.64 (s, 3H), 3.60-3.52 (m, 2H), 3.45-3.35 (m, 1H), 3.26-3.15 (m, 1H). High resolution mass spectrum (mass-to-charge ratio): C₂₂H₂₁BrN₃O₃S⁺ [M+H]⁺ Calculated value: 486.0482, Measured value:486.0481.

### Example 157:

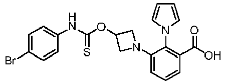

Compound 157 was synthesized according to the General Experimental Operation XI (8.8mg, yield 95%). ¹H NMR (400 MHz, Chloroform-*d*) δ 7.47-7.27 (m, 4H), 7.01-6.96 (m, 1H), 6.91-6.77 (m, 2H), 6.74-6.56 (m, 2H), 6.45-6.18 (m, 2H), 4.87-4.70 (m, 1H), 3.56-3.43 (m, 2H), 3.42-3.32 (m, 1H), 3.25-3.13 (m, 1H). High resolution mass spectrum (mass-to-charge ratio): C₂₁H₁₉BrN₃O₃S⁺ [M+H]⁺ Calculated value: 472.0325, Measured value: 472.0329.

### Example 158:

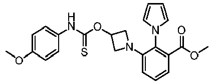

Compound 158 was synthesized according to the General Experimental Operation XI (5.4mg, yield 78%). ¹H NMR (400 MHz, Chloroform-*d*) δ 7.41-7.32 (m, 1H), 7.24-7.13 (m, 1H), 6.98-6.94 (m, 1H), 6.92-6.77 (m, 4H), 6.73-6.57 (m, 2H), 6.43-6.24 (m, 2H), 4.87-4.64 (m, 1H), 4.07 (t, *J* = 6.5 Hz, 1H), 3.80 (s, 3H), 3.64 (s, 3H), 3.59-3.48 (m, 2H), 3.42-3.30 (m, 1H), 3.23-3.09 (m, 1H). High resolution mass spectrum (mass-to-charge ratio): C₂₃H₂₄N₃O₄S⁺ [M+H]⁺ Calculated value: 438.1482, Measured value:438.1480.

### Example 159:

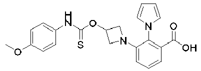

Compound 159 was synthesized according to the General Experimental Operation XI (8.2mg, 95%). ¹H NMR (400 MHz, Chloroform-*d*) δ 7.39-7.27 (m, 2H), 7.10-6.79 (m, 5H), 6.76-6.58 (m, 2H), 6.43-6.19 (m, 2H), 4.86-4.67 (m, 1H), 3.80 (s, 3H), 3.57-3.44 (m, 2H), 3.41-3.29 (m, 1H), 3.22-2.98 (m, 1H). High resolution mass spectrum (mass-to-charge ratio): C₂₂H₂₂N₃O₄S⁺ [M+H]⁺ Calculated value: 424.1326, Measured value:424.1324.

### Example 160:

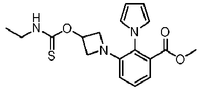

Compound 160 was synthesized according to the General Experimental Operation XI (3.6mg, 57%). ¹H NMR (400 MHz, Chloroform-*d*) δ 7.38-7.29 (m, 1H), 7.25-7.15 (m, 1H), 6.96-6.87 (m, 1H), 6.75-6.57 (m, 2H), 6.45-6.23 (m, 2H), 4.89-4.54 (m, 1H), 4.02 (t, *J* = 6.5 Hz, 1H), 3.63 (s, 3H), 3.57-3.23 (m, 4H), 3.19-3.08 (m, 2H), 1.21 (t, 3H). High resolution mass spectrum (mass-to-charge ratio): C₁₈H₂₂N₃O₃S⁺ [M+H]⁺ Calculated value: 360.1376, Measured value:360.1367.

### Example 161:

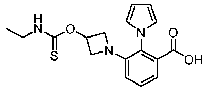

Compound 161 was synthesized according to the General Experimental Operation XI (4.4mg, 95%). ¹H NMR (400 MHz, Methanol-*d*₄) δ 7.43-7.18 (m, 1H), 7.12-6.91 (m, 2H), 6.75-6.57 (m, 2H), 6.38-6.11 (m, 2H), 5.12-4.60 (m, 1H), 3.70-3.07 (m, 6H), 1.24-1.05 (m, 3H). High resolution mass spectrum (mass-to-charge ratio): C₁₇H₂₀N₃O₃S⁺ [M+H]⁺ Calculated value: 346.1220, Measured value: 346.1219.

### Example 162:

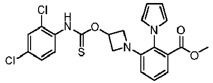

Compound 162 was synthesized according to the General Experimental Operation XI (4.6mg, 80%). ¹H NMR (400 MHz, Chloroform-*d*) δ 7.47-7.33 (m, 2H), 7.26-7.15 (m, 2H), 6.96 (d, *J* = 8.3 Hz, 1H), 6.86 (d, *J* = 8.5 Hz, 1H), 6.72-6.50 (m, 2H), 6.41-6.10 (m, 2H), 4.95-4.79 (m, 1H), 4.11-4.02 (m, 1H), 3.64 (s, 3H), 3.59-3.48 (m, 2H), 3.48-3.33 (m, 1H), 3.29-3.15 (m, 1H). High resolution mass spectrum (mass-to-charge ratio): C₂₂H₂₀Cl₂N₃O₃S⁺ [M+H]⁺ Calculated value: 476.0597, Measured value: 476.0596.

### Example 163:

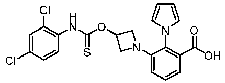

Compound 163 was synthesized according to the General Experimental Operation XI (9.4mg, 95%). ¹H NMR (400 MHz, Chloroform-*d*) δ 7.49-7.27 (m, 3H), 7.22-7.07 (m, 1H), 6.98 (d, *J* = 8.2 Hz, 1H), 6.86 (d, 1H), 6.74-6.55 (m, 2H), 6.45-6.13 (m, 2H), 4.92-4.67 (m, 1H), 3.65-3.44 (m, 2H), 3.44-3.35 (m, 1H), 3.31-3.17 (m, 1H). High resolution mass spectrum (mass-to-charge ratio): C₂₁H₁₈Cl₂N₃O₃S⁺ [M+H]⁺ Calculated value: 462.0440, Measured value: 462.0445.

### Example 164:

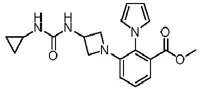

Compound 164 was synthesized according to the General Experimental Operation XI (7mg, 54%). ¹H NMR (500 MHz, Chloroform-*d*) δ 7.30-7.25 (m, 1H), 7.15-7.10 (m, 1H), 6.75-6.58 (m, 3H), 6.23 (t, *J* = 2.1 Hz, 2H), 5.21 (d, *J* = 7.5 Hz, 1H), 4.79 (s, 1H), 4.49 (q, *J* = 6.6 Hz, 1H), 3.75 (t, *J* = 7.7 Hz, 2H), 3.60 (s, 3H), 3.30 (dd, *J* = 8.1, 5.6 Hz, 2H), 2.40 (dt, *J* = 7.0, 3.3 Hz, 1H), 0.73 (td, *J* = 6.8, 4.8 Hz, 2H), 0.54 (p, *J* = 4.6 Hz, 2H). High resolution mass spectrum (mass-to-charge ratio): C₁₉H₂₃N₄O₃⁺ [M+H]⁺ Calculated value: 355.1765, Measured value: 355.1768.

### Example 165:

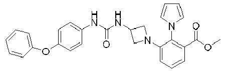

Compound 165 was synthesized according to the General Experimental Operation XI (7.5mg, 42%). ¹H NMR (300 MHz, Chloroform-*d*) δ 7.33 (dd, *J* = 16.3, 8.2 Hz, 3H), 7.23-7.08 (m, 4H), 7.05-6.93 (m, 4H), 6.73-6.60 (m, 3H), 6.29-6.15 (m, 3H), 4.86 (d, *J* = 7.4 Hz, 1H), 4.52 (d, *J* = 6.9 Hz, 1H), 3.77 (t, *J* = 7.7 Hz, 2H), 3.62 (s, 3H), 3.29 (dd, *J* = 8.2, 5.4 Hz, 2H). High resolution mass spectrum (mass-to-charge ratio): C₂₈H₂₇N₄O₄⁺ [M+H]⁺ Calculated value: 483.2027, Measured value: 483.2019.

### Example 166:

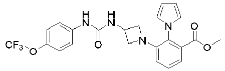

Compound 166 was synthesized according to the General Experimental Operation XI (8mg, 46%). ¹H NMR (300 MHz, Chloroform-*d*) δ 7.31 (d, *J=* 8.6 Hz, 3H), 7.14 (d, *J* = 8.4 Hz, 3H), 6.74-6.50 (m, 4H), 6.23 (t, *J* = 2.1 Hz, 2H), 5.10 (d, *J* = 7.4 Hz, 1H), 4.48 (d, *J* = 6.6 Hz, 1H), 3.78 (t, *J* = 7.7 Hz, 2H), 3.63 (s, 3H), 3.25 (dd, *J* = 8.3, 5.2 Hz, 2H). High resolution mass spectrum (mass-to-charge ratio): C₂₃H₂₂F₃N₄O₄⁺ [M+H]⁺ Calculated value: 475.1588, Measured value: 475.1585.

### Example 167:

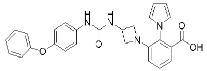

Compound 167 was synthesized according to the General Experimental Operation XI (2.5mg, 64%). ¹H NMR (500 MHz, Methanol-*d*₄) δ 7.35-7.23 (m, 5H), 7.08-6.98 (m, 2H), 6.95-6.85 (m, 4H), 6.79-6.63 (m, 3H), 6.17 (d, *J* = 2.0 Hz, 2H), 4.37 (t, *J* = 6.5 Hz, 1H), 3.65 (t, *J* = 7.6 Hz, 2H), 3.31 (d, *J* = 3.5 Hz, 2H). High resolution mass spectrum (mass-to-charge ratio): C₂₇H₂₅N₄O₄⁺ [M+H]⁺ Calculated value: 469.1870, Measured value: 469.1874.

### Example 168:

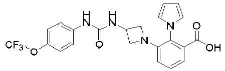

Compound 168 was synthesized according to the General Experimental Operation XI (4mg, 69%). ¹H NMR (300 MHz, Methanol-*d*₄) δ 7.48-7.35 (m, 2H), 7.27 (t, *J* = 7.8 Hz, 1H), 7.21-7.10 (m, 2H), 6.96 (d, *J* = 7.5 Hz, 1H), 6.74 (t, *J* = 2.1 Hz, 2H), 6.65 (d, *J* = 8.1 Hz, 1H), 6.16 (t, *J* = 1.9 Hz, 2H), 4.38 (p, *J* = 6.4 Hz, 1H), 3.71-3.59 (m, 2H), 3.35-3.29 (m, 2H). High resolution mass spectrum (mass-to-charge ratio): C₂₂H₂₀F₃N₄O₄⁺ [M+H]⁺ Calculated value: 461.1431, Measured value: 461.1428.

### Example 169:

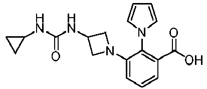

Compound 169 was synthesized according to the General Experimental Operation XI (2.4mg, 63%). ¹H NMR (500 MHz, Methanol-*d*₄) δ 7.29 (t, *J* = 7.8 Hz, 1H), 7.03 (d, *J* = 7.5 Hz, 1H), 6.77-6.64 (m, 3H), 6.16 (d, *J* = 2.0 Hz, 2H), 4.34 (t, *J* = 6.7 Hz, 1H), 3.60 (t, *J* = 7.5 Hz, 2H), 3.36-3.32 (m, 2H), 2.45-2.40 (m, 1H), 0.68-0.62 (m, 2H), 0.42-0.38 (m, 2H). High resolution mass spectrum (mass-to-charge ratio): C₁₈H₂₁N₄O₃⁺ [M+H]⁺ Calculated value: 341.1608, Measured value: 341.1599.

### Example 170:

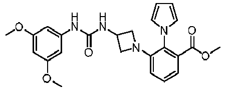

Compound 170 was synthesized according to the General Experimental Operation XI (2mg, yield 63%). ¹H NMR (400 MHz, Chloroform-d) δ 7.69-7.58 (m, 1H), 7.33-7.21 (m, 1H), 7.16-7.07 (m, 1H), 6.77-6.58 (m, 3H), 6.51-6.40 (m, 2H), 6.22 (t, *J* = 2.1 Hz, 2H), 5.02 (d, *J* = 7.4 Hz, 1H), 4.54-4.42 (m, 1H), 3.82-3.68 (m, 8H), 3.61 (s, 3H), 3.31-3.22 (m, 2H). High resolution mass spectrum (mass-to-charge ratio): C₂₄H₂₇N₄O₅⁺ [M+H]⁺ Calculated value: 451.1976, Measured value:451.1981.

### Example 171:

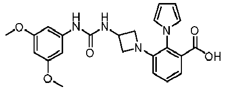

Compound 171 was synthesized according to the General Experimental Operation XI (2.8mg, 95%). ¹H NMR (300 MHz, Methanol-*d*₄) δ 7.70-7.60 (m, 1H), 7.22 (t, *J* = 7.8 Hz, 1H), 6.88 (d, *J* = 7.4 Hz, 1H), 6.76 (t, *J* = 2.1 Hz, 2H), 6.58 (d, *J* = 8.1 Hz, 1H), 6.53 (d, *J* = 2.6 Hz, 1H), 6.48-6.38 (m, 1H), 6.14 (t, *J* = 2.0 Hz, 2H), 4.43-4.29 (m, 1H), 3.83 (s, 3H), 3.75 (s, 3H), 3.69-3.53 (m, 2H), 3.28-3.17 (m, 2H). High resolution mass spectrum (mass-to-charge ratio): C₂₃H₂₅N₄O₅⁺ [M+H]⁺ Calculated value: 437.1819, Measured value: 437.1833.

### Example 172:

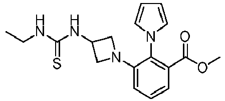

Compound 172 was synthesized according to the General Experimental Operation XI (4.2mg, 31.8%). ¹H NMR (300 MHz, Chloroform-*d*) δ 7.35-7.28 (m, 1H), 7.14 (dd, *J* = 7.7, 1.3 Hz, 1H), 6.99-6.87 (m, 1H), 6.66 (brs, 2H), 6.34 (t, *J* = 2.1 Hz, 2H), 4.52-4.36 (m, 1H), 4.10 (d, *J* = 6.2 Hz, 1H), 3.64 (s, 3H), 3.38 (dd, *J* = 10.8, 7.6 Hz, 1H), 3.34-3.19 (m, 4H), 3.09 (dd, *J* = 10.8, 6.0 Hz, 1H), 1.20 (t, *J* = 7.2 Hz, 3H). High resolution mass spectrum (mass-to-charge ratio): C₁₈H₂₃N₄O₂S⁺ [M+H]⁺ Calculated value: 359.1536, Measured value: 359.1539.

### Example 173:

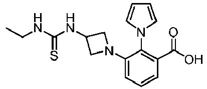

Compound 173 was synthesized according to the General Experimental Operation XI (1.0mg, 48%). ¹H NMR (500 MHz, Methanol-*d*₄) δ 7.21 (t, *J* = 7.9 Hz, 1H), 6.79 (t, *J* = 7.9 Hz, 2H), 6.73 (t, *J* = 2.1 Hz, 2H), 6.22 (d, *J* = 2.0 Hz, 2H), 4.47 (d, *J* = 7.9 Hz, 1H), 3.63 (d, *J* = 3.5 Hz, 1H), 3.40 (d, *J* = 4.8 Hz, 1H), 3.34 (dd, *J* = 14.1, 5.7 Hz, 1H), 3.26 (d, *J* = 7.4 Hz, 2H), 3.19 (q, *J* = 9.4, 8.4 Hz, 1H), 1.23 (t, *J* = 7.3 Hz, 3H). High resolution mass spectrum (mass-to-charge ratio): C₁₇H₂₁N₄O₂S⁺ [M+H]⁺ Calculated value: 345.1380, Measured value: 345.1376.

### Example 174:

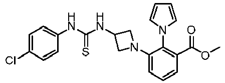

Compound 174 was synthesized according to the General Experimental Operation XI (1mg, 40%). ¹H NMR (400 MHz, Chloroform-*d*) δ 7.35-7.21 (m, 3H), 7.20-7.09 (m, 3H), 6.95-6.86 (m, 1H), 6.72-6.63 (m, 2H), 6.39-6.31 (m, 2H), 4.41-4.33 (m, 1H), 4.15-3.97 (m, 1H), 3.64 (s, 3H), 3.47-3.21 (m, 3H), 3.11-2.91 (m, 1H). High resolution mass spectrum (mass-to-charge ratio): C₂₂H₂₂ClN₄O₂S⁺ [M+H]⁺ Calculated value: 441.1147, Measured value: 441.1151.

### Example 175:

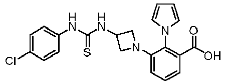

Compound 175 was synthesized according to the General Experimental Operation XI (2.6mg, 95%). ¹H NMR (300 MHz, Methanol-*d*₄) δ 7.39-7.05 (m, 5H), 6.86-6.69 (m, 4H), 6.19 (t, *J* = 2.2 Hz, 2H), 4.45-4.32 (m, 1H), 3.45-3.16 (m, 3H), 3.14-3.02 (m, 1H). High resolution mass spectrum (mass-to-charge ratio): C₂₁H₂₀ClN₄O₂S⁺ [M+H]⁺ Calculated value: 427.0990, Measured value: 427.0991.

### Example 176:

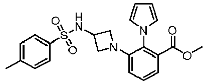

Compound 176 was synthesized according to the General Experimental Operation XI (20mg, yield 95%). ¹H NMR (300 MHz, Chloroform-*d*) δ 7.74-7.55 (m, 2H), 7.37-7.17 (m, 3H), 7.18-7.05 (m, 1H), 6.69-6.49 (m, 3H), 6.22-5.99 (m, 2H), 4.98 (d, *J* = 9.1 Hz, 1H), 4.10-3.88 (m, 1H), 3.59 (s, 3H), 3.52-3.35 (m, 2H), 3.21-2.97 (m, 2H), 2.46 (s, 3H). High resolution mass spectrum (mass-to-charge ratio): C₂₂H₂₄N₃O₄S⁺ [M+H]⁺ Calculated value: 426.1482, Measured value: 426.1472.

### Example 177:

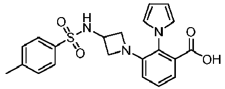

Compound 177 was synthesized according to the General Experimental Operation XI (4.7mg, 95%). ¹H NMR (300 MHz, Methanol-*d*₄) δ 7.67-7.56 (m, 2H), 7.35 (d, *J* = 8.0 Hz, 2H), 7.22-7.10 (m, 1H), 6.86-6.74 (m, 1H), 6.67-6.57 (m, 2H), 6.42 (dd, *J* = 8.2, 1.1 Hz, 1H), 6.12-5.99 (m, 2H), 3.98-3.81 (m, 1H), 3.29-3.21 (m, 2H), 3.16-2.99 (m, 2H), 2.47 (s, 3H). [M+H]⁺ Calculated value: C₂₁H₂₂N₃O₄S⁺ [M+H]⁺ Calculated value: 412.1326,Measured value: 412.1325.

### Example 178:

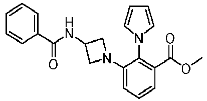

Compound 178 was synthesized according to the General Experimental Operation XI (11.4mg, 95%). ¹H NMR (300 MHz, Chloroform-*d*) δ 7.80-7.67 (m, 2H), 7.46-7.39 (m, 2H), 7.35-7.24 (m, 1H), 7.18-7.11 (m, 1H), 6.74-6.65 (m, 3H), 6.60-6.51 (m, 1H), 6.30-6.20 (m, 2H), 4.84-4.70 (m, 1H), 4.03-3.72 (m, 2H), 3.61 (s, 3H), 3.51-3.19 (m, 2H). High resolution mass spectrum (mass-to-charge ratio): C₂₂H₂₂N₃O₃⁺ [M+H]⁺ Calculated value: 376.1656, Measured value: 376.1648.

### Example 179:

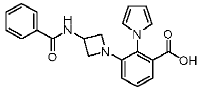

Compound 179 was synthesized according to the General Experimental Operation XI (5.4mg, 75%). ¹H NMR (300 MHz, Methanol-*d*₄) δ 7.79 (dd, *J* = 7.0, 1.8 Hz, 2H), 7.55-7.48 (m, 1H), 7.47-7.37 (m, 2H), 7.30-7.15 (m, 1H), 6.98-6.82 (m, 1H), 6.81-6.73 (m, 2H), 6.62 (d, *J* = 8.1 Hz, 1H), 6.20-6.04 (m, 2H), 4.71-4.57 (m, 1H), 3.74-3.61 (m, 2H), 3.56-3.47 (m, 2H). High resolution mass spectrum (mass-to-charge ratio): C₂₁H₂₀N₃O₃⁺ [M+H]⁺ Calculated value: 362.1499, Measured value: 362.1504.

### Example 180:

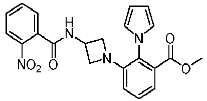

Compound 180 was synthesized according to the General Experimental Operation XI (8.4mg, 80%). ¹H NMR (300 MHz, Chloroform-*d*) δ 8.12-8.02 (m, 1H), 7.75-7.54 (m, 2H), 7.51-7.45 (m, 1H), 7.35-7.26 (m, 1H), 7.21-7.09 (m, 1H), 6.76-6.56 (m, 3H), 6.33-6.26 (m, 1H), 6.26-6.19 (m, 2H), 4.81-4.59 (m, 1H), 3.96-3.76 (m, 2H), 3.59 (s, 3H), 3.47-3.32 (m, 2H). High resolution mass spectrum (mass-to-charge ratio): C₂₂H₂₁N₄O₅⁺ [M+H]⁺ Calculated value: 421.1506, Measured value: 421.1493.

### Example 181:

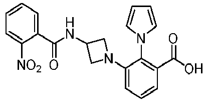

Compound 181 was synthesized according to the General Experimental Operation XI (5.2mg, 95%). ¹H NMR (300 MHz, Methanol-*d*₄) δ 8.15-8.07 (m, 1H), 7.82-7.72 (m, 1H), 7.70-7.62 (m, 1H), 7.57-7.50 (m, 1H), 7.28-7.17 (m, 1H), 6.92-6.83 (m, 1H), 6.80-6.74 (m, 2H), 6.63-6.50 (m, 1H), 6.22-6.03 (m, 2H), 4.65-4.54 (m, 1H), 3.76-3.65 (m, 2H), 3.50-3.38 (m, 2H). High resolution mass spectrum (mass-to-charge ratio): C₂₁H₁₉N₄O₅⁺ [M+H]⁺ Calculated value: 407.1350, Measured value: 407.1347.

### Example 182:

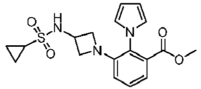

Compound 182 was synthesized according to the General Experimental Operation XI (5.6mg, 60%). ¹H NMR (300 MHz, Chloroform-d) δ 7.30 (t, *J* = 7.9 Hz, 1H), 7.22-7.14 (m, 1H), 6.73-6.48 (m, 3H), 6.24 (t, *J* = 2.1 Hz, 2H), 4.73 (d, *J* = 9.4 Hz, 1H), 4.15 (h, *J* = 6.4 Hz, 1H), 3.79-3.68 (m, 2H), 3.62 (s, 3H), 3.45-3.30 (m, 2H), 2.34-2.18 (m, 1H), 1.16-1.06 (m, 2H), 1.01-0.92 (m, 2H). High resolution mass spectrum (mass-to-charge ratio): C₁₈H₂₂N₃O₄S⁺ M+H]⁺ Calculated value: 376.1326, Measured value: 376.1310.

### Example 183:

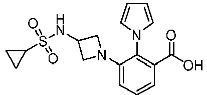

Compound 183 was synthesized according to the General Experimental Operation XI (3mg, 95%). ¹H NMR (300 MHz, Methanol-*d*₄) δ 7.31-7.14 (m, 1H), 6.89 (dd, *J* = 7.5, 1.3 Hz, 1H), 6.75 (t, *J* = 2.1 Hz, 2H), 6.58 (dd, *J* = 8.1, 1.1 Hz, 1H), 6.14 (t, 2H), 4.14-4.00 (m, 1H), 3.60-3.51 (m, 2H), 3.45-3.35 (m, 2H), 2.41-2.30 (m, 1H), 1.00-0.92 (m, 4H). High resolution mass spectrum (mass-to-charge ratio): C₁₇H₁₉N₃NaO₄S⁺ [M+Na]⁺ Calculated value: 384.0988, Measured value: 384.0980.

### Example 184:

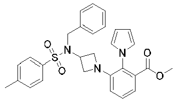

Compound 184 was synthesized according to the General Experimental Operation XI (3mg, 95%). ¹H NMR (400 MHz, Chloroform-*d*) δ 7.66-7.57 (m, 2H), 7.34-7.28 (m, 7H), 7.25-7.19 (m, 1H), 7.14-7.09 (m, 1H), 6.56-6.40 (m, 3H), 6.06 (t, *J* = 2.1 Hz, 2H), 4.40-4.32 (m, 1H), 4.32 (s, 2H), 3.59 (s, 3H), 3.41-3.29 (m, 2H), 3.25-3.09 (m, 2H), 2.48 (s, 3H). High resolution mass spectrum (mass-to-charge ratio): C₂₉H₃₀N₃O₄S⁺ [M+H]⁺ Calculated value: 516.1952, Measured value: 516.1960.

### Example 185:

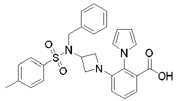

Compound 185 was synthesized according to the General Experimental Operation XI (6.1mg, 95%). ¹H NMR (500 MHz, Methanol-*d*₄) δ 7.63 (d, *J* = 8.2 Hz, 2H), 7.40 (d, *J* = 8.0 Hz, 2H), 7.36-7.24 (m, 5H), 7.16 (t, *J* = 7.8 Hz, 1H), 6.89 (d, *J* = 7.6 Hz, 1H), 6.53 (t, *J* = 2.2 Hz, 2H), 6.37 (d, *J* = 8.2 Hz, 1H), 5.95 (t, *J* = 2.0 Hz, 2H), 4.46-4.38 (m, 3H), 3.36-3.32 (m, 2H), 3.09-3.00 (m, 2H), 2.49 (s, 3H). High resolution mass spectrum (mass-to-charge ratio): C₂₈H₂₈N₃O₄S⁺ [M+H]⁺ Calculated value: 502.1795, Measured value: 502.1799.

### Example 186:

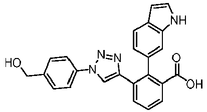

Compound 186 (4mg, yield 95%)¹H NMR (300 MHz, Methanol-*d*₄) δ 8.21 (d, *J* = 7.8 Hz, 1H), 7.80-7.71 (m, 1H), 7.66-7.50 (m, 2H), 7.40-7.34 (m, 2H), 7.30 (d, *J* = 3.1 Hz, 1H), 7.26 (s, 1H), 7.19-7.11 (m, 2H), 6.95-6.85 (m, 1H), 6.54 (dd, *J* = 3.2, 0.8 Hz, 1H), 6.40 (s, 1H), 4.60 (s, 2H). High resolution mass spectrum (mass-to-charge ratio): C₂₄H₁₉N₄O₃⁺ [M+H]⁺ Calculated value: 411.1452, Measured value: 411.1446.

### Example 187:

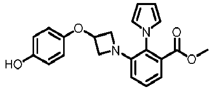

Compound 187 was synthesized according to the General Experimental Operation V (20mg, yield 80%). ¹H NMR (400 MHz, Chloroform-d) δ 7.29 (d, *J* = 7.9 Hz, 1H), 7.13 (d, *J* = 7.7 Hz, 1H), 6.77-6.63 (m, 5H), 6.56 (d, *J* = 8.4 Hz, 2H), 6.24 (t, *J* = 2.2 Hz, 2H), 5.60 (dd, *J* = 6.0, 2.0 Hz, 1H), 4.75-4.72 (m, 1H), 3.75 (dd, *J* = 8.6, 6.2 Hz, 2H), 3.61 (d, *J* = 4.1 Hz, 5H). High resolution mass spectrum (mass-to-charge ratio): C₂₁H₂₀N₂O₄Na⁺[M+Na]⁺Calculated value: 387.1321, Measured value: 387.1315.

### Example 188:

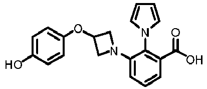

Compound 188 was synthesized according to the General Experimental Operation V (5mg, yield 56%). ¹H NMR (300 MHz, Methanol-d4) δ 7.29 (t, *J* = 7.9 Hz, 1H), 7.03 (dd, *J* = 7.6, 1.4 Hz, 1H), 6.75-6.62 (m, 5H), 6.59-6.51 (m, 2H), 6.17 (t, *J* = 2.1 Hz, 2H), 4.74-4.72 (m, 1H), 3.78-3.71 (m, 2H), 3.50-3.46 (m, 2H). High resolution mass spectrum (mass-to-charge ratio): C₂₀H₁₈N₂O₄Na⁺[M+Na]⁺Calculated value: 373.1164, Measured value: 373.1160.

### Example 189:

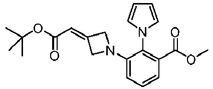

Compound 189 was synthesized according to the General Experimental Operation XIV (5mg, 95.6%). ¹H NMR (300 MHz, Chloroform-d) δ 7.31 (t, *J* = 7.8Hz, 1H), 7.16-7.14 (m, 1H), 6.74-6.68 (m, 3H), 6.26 (t, *J* = 1.8 Hz, 2H), 5.53 (t, *J* = 2.1 Hz, 1H), 4.48 (d, *J=* 3.0 Hz, 2H), 4.15 (d, *J* = 3.0 Hz, 2H), 3.62 (s, 3H), 1.44 (s, 9H). High resolution mass spectrum (mass-to-charge ratio): C₂₁H₂₄N₂NaO₄⁺ [M+Na]⁺ Calculated value: 391.1628,Measured value: 391.1624.

### Example 190:

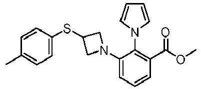

Compound 190 was synthesized according to the General Experimental Operation XV (5mg, 46%). ¹H NMR (300 MHz, Methanol-*d*₄) δ 7.30 (t, *J* = 7.9 Hz, 1H), 7.17-7.09 (m, 4H), 7.02 (dd, *J* = 7.6, 1.4 Hz, 1H), 6.72 (dd, *J* = 8.2, 1.4 Hz, 1H), 6.64 (t, *J* = 2.1 Hz, 2H), 6.19 (t, *J* = 2.1 Hz, 2H), 3.91-3.81 (m, 3H), 3.54 (s, 3H), 3.41-3.34 (m, 1H), 2.30 (s, 3H). High resolution mass spectrum (mass-to-charge ratio): C₂₂H₂₃N₂O₂S⁺ [M+H]⁺ Calculated value: 379.1475,Measured value: 379.1464.

### Example 191:

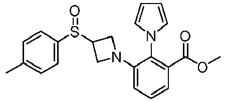

Compound 191 was synthesized according to the General Experimental Operation XV (1.3mg, 50%). ¹H NMR (300 MHz, Chloroform-d) δ 7.43 (d, *J* = 8.0 Hz, 2H), 7.30 (d, *J* = 9.1 Hz, 3H), 7.19 (s, 1H), 6.67 (d, *J* = 8.1 Hz, 3H), 6.24 (s, 2H), 5.35 (brs, 1H), 3.62 (s, 3H), 3.59-3.32 (m, 4H), 2.42 (s, 3H). High resolution mass spectrum (mass-to-charge ratio): C₂₂H₂₃N₂O₃S⁺ [M+H]⁺ Theoretical value 395.1424, Measured value 395.1424.

### Example 192:

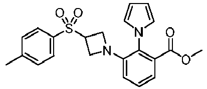

Compound 192 was synthesized according to the General Experimental Operation XV (1.0mg, 45%). ¹H NMR (300 MHz, Chloroform-d) δ 7.73 (d, *J* = 8.1 Hz, 2H), 7.41-7.31 (m, 3H), 7.18 (d, *J* = 7.2 Hz, 1H), 6.67-6.57 (m, 3H), 6.23 (t, *J* = 2.1 Hz, 2H), 5.35 (s, 1H), 3.86-3.78 (m, 2H), 3.63 (d, *J* = 8.3 Hz, 5H), 2.46 (s, 3H). High resolution mass spectrum (mass-to-charge ratio): C₂₂H₂₃N₂O₄S⁺ [M+H]⁺ Calculated value: 411.1373,Measured value: 411.1377.

### Example 193:

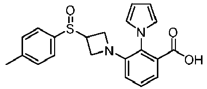

Compound 193 was synthesized according to the General Experimental Operation XV (0.8mg, 51%). ¹H NMR (300 MHz, Chloroform-*d*) δ 8.07 (s, 1H), 7.98 (d, *J* = 7.8 Hz, 1H), 7.59 (d, *J* = 8.3 Hz, 1H), 7.44 (d, *J* = 7.9 Hz, 2H), 7.32 (d, *J* = 5.3 Hz, 2H), 6.71 (t, *J* = 4.8 Hz, 3H), 6.26 (s, 2H), 5.35 (s, 1H), 3.50-3.46 (m, 4H), 2.42 (s, 3H).High resolution mass spectrum (mass-to-charge ratio): C₂₁H₂₀N₂NaO₃S⁺ [M+Na]⁺ Calculated value: 403.1087, Measured value: 403.1076.

### Example 194:

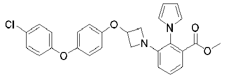

Compound 194 was synthesized according to the General Experimental Operation VII (13mg, 19%). ¹H NMR (400 MHz, *Chloroform-d*) *δ* 7.34-7.27 (*m,* 1H), 7.27-7.21 (m, 2H), 7.15 (*dd*, *J* = 7.7, 1.3 Hz, 1H), 6.95-6.89 (*m,* 2H), 6.88-6.83 (*m,* 2H), 6.73-6.62 (*m,* 5H), 6.25 (*t, J* = 2.1 Hz, 2H), 4.85-4.74 (*m,* 1H), 3.79 (*dd, J* = 9.1, 6.2 Hz, 2H), 3.65 (*dd*, *J* = 9.1, 4.8 Hz, 2H), 3.61 (*s*, 3H) ppm; High resolution mass spectrum (mass-to-charge ratio): C₂₇H₂₃ClN₂O₄Na⁺ [M+Na]⁺ Calculated value: 497.1239, Measured value: 497.1240.

### Example 195:

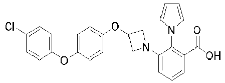

Compound 195 was synthesized according to the General Experimental Operation VII (5mg, 75%). ¹H NMR (400 MHz, *Methanol-d4*) *δ* 7.26-7.19 (*m,* 1H), 7.19-7.14 (*m,* 2H), 6.98 (*dd, J* = 7.6, 1.3 Hz, 1H), 6.87-6.79 (*m,* 2H), 6.79-6.73 (*m,* 2H), 6.69-6.62 (*m,* 3H), 6.60 (*dd*, *J* = 5.2, 3.1 Hz, 2H), 6.08 (*t, J* = 2.1 Hz, 2H), 4.73-4.69 (*m,* 1H), 3.71 (*dd*, *J* = 9.0, 6.2 Hz, 2H), 3.42 (*dd, J* = 9.1, 4.4 Hz, 2H) ppm; High resolution mass spectrum (mass-to-charge ratio): C₂₆H₂₁ClN₂O₄Na⁺ [M+Na]⁺ Calculated value: 483.1082, Measured value: 483.1084.

### Example 196:

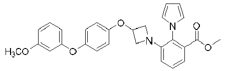

Compound 196 was synthesized according to the General Experimental Operation VII (18mg, 21%). ¹H NMR (400 MHz, *Chloroform-d*) *δ* 7.29 (*t, J* = 7.9 Hz, 1H), 7.17 (*ddd*, *J* = 17.7, 7.3, 1.7 Hz, 2H), 6.97-6.90 (*m,* 2H), 6.74-6.63 (*m,* 5H), 6.62-6.57 (*m,* 1H), 6.54-6.47 (*m,* 2H), 6.25 (*t, J* = 2.1 Hz, 2H), 4.85-4.73 (*m,* 1H), 3.79 (*dd, J* = 9.1, 6.3 Hz, 2H), 3.76 (*s*, 3H), 3.68-3.63 (*m,* 2H), 3.61 (*s*, 3H) ppm; High resolution mass spectrum (mass-to-charge ratio): C₂₈H₂₆N₂O₅Na⁺ [M+Na]⁺ Calculated value: 493.1734, Measured value: 493.1735.

### Example 197:

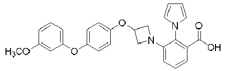

Compound 197 was synthesized according to the General Experimental Operation VII (6mg, 70%). ¹H NMR (400 MHz, *Methanol-d4*) *δ* 7.21 (*t, J* = 7.9 Hz, 1H), 7.06 (*t, J* = 8.2 Hz, 1H), 6.97 (*dd, J* = 7.6, 1.1 Hz, 1H), 6.85-6.76 (*m,* 2H), 6.69-6.56 (*m,* 5H), 6.54-6.46 (*m,* 1H), 6.33 (*dd*, *J* = 8.7, 1.4 Hz, 2H), 6.08 (*t, J* = 2.1 Hz, 2H), 4.70-4.68 (*m,* 1H), 3.69 (*dd*, *J* = 8.7, 6.3 Hz, 2H), 3.62 (*s*, 3H), 3.42 (*dd*, *J* = 8.9, 4.4 Hz, 2H) ppm; High resolution mass spectrum (mass-to-charge ratio): C₂₇H₂₄N₂O₅Na⁺ [M+Na]⁺ Calculated value: 479.1577, Measured value: 479.1580.

### Example 198:

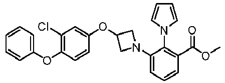

Compound 198 was synthesized according to the General Experimental Operation VII (15.6mg, 18%). ¹H NMR (300 MHz, *Chloroform-d*) *δ* 7.37-7.26 (*m,* 3H), 7.16 (*dd*, *J* = 7.6, 1.3 Hz, 1H), 7.06 (*t, J* = 7.4 Hz, 1H), 6.95 (*d, J* = 8.9 Hz, 1H), 6.92-6.83 (*m,* 2H), 6.80 (*d, J* = 2.9 Hz, 1H), 6.75-6.66 (*m,* 3H), 6.58 (*dd, J* = 8.9, 3.0 Hz, 1H), 6.26 (*t*, *J* = 2.1 Hz, 2H), 4.85-4.73 (*m,* 1H), 3.80 (*dd*, *J* = 9.0, 6.2 Hz, 2H), 3.68-3.59 (*m,* 5H) ppm; High resolution mass spectrum (mass-to-charge ratio): C₂₇H₂₃N₂ClO₄Na⁺ [M+Na]⁺ Calculated value: 497.1239, Measured value: 497.1240.

### Example 199:

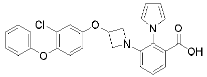

Compound 199 was synthesized according to the General Experimental Operation VII (4mg, 60%). ¹H NMR (300 MHz, *Methanol-d4*) *δ* 7.29-7.14 (*m,* 3H), 7.02-6.92 (*m,* 2H), 6.90 (*d, J* = 8.9 Hz, 1H), 6.81 (*d, J* = 2.9 Hz, 1H), 6.76-6.70 (*m,* 2H), 6.68 (*dd*, *J* = 8.2, 1.2 Hz, 1H), 6.62 (*td*, *J* = 4.9, 2.8 Hz, 3H), 6.10 (*t, J* = 2.1 Hz, 2H), 4.76-4.72 (*m,* 1H), 3.73 (*dd*, *J* = 9.1, 6.2 Hz, 2H), 3.43 (*dd, J* = 9.1, 4.2 Hz, 2H) ppm; High resolution mass spectrum (mass-to-charge ratio): C₂₆H₂₁N₂ClO₄Na⁺ [M+Na]⁺ Calculated value: 483.1082, Measured value: 483.1082.

### Example 200:

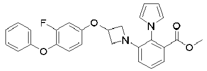

Compound 200 was synthesized according to the General Experimental Operation VII (28.5mg, 34%). ¹H NMR (500 MHz, *Chloroform-d*) *δ* 7.29 (*td*, *J* = 7.8, 4.5 Hz, 3H), 7.16 (*d, J* = 7.6 Hz, 1H), 7.04 (*t, J* = 7.3 Hz, 1H), 6.99 (*t, J* = 9.0 Hz, 1H), 6.91 (*d, J* = 8.2 Hz, 2H), 6.71 (*t, J* = 1.8 Hz, 2H), 6.69 (*d, J* = 8.0 Hz, 1H), 6.55 (*dd*, *J* = 11.7, 2.8 Hz, 1H), 6.43 (*d, J* = 8.8 Hz, 1H), 6.26 (*t, J* = 1.8 Hz, 2H), 4.82-4.74 (*m,* 1H), 3.83-3.76 (*m,* 2H), 3.65 (*dd*, *J* = 8.7, 4.7 Hz, 2H), 3.61 (*s*, 3H) ppm; High resolution mass spectrum (mass-to-charge ratio): C₂₇H₂₃N₂FO₄Na⁺ [M+Na]⁺ Calculated value: 481.1534, Measured value: 481.1535.

### Example 201:

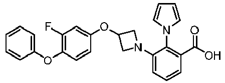

Compound 201 was synthesized according to the General Experimental Operation VII (7mg, 72%). ¹HNMR (500 MHz, *Methanol-d4*) *δ* 7.22 (*t, J* = 7.9 Hz, 1H), 7.18 (*t, J* = 7.9 Hz, 2H), 6.98 (*d, J* = 7.4 Hz, 1H), 6.93 (*dd*, *J* = 12.1, 5.7 Hz, 2H), 6.75 (*d, J* = 8.3 Hz, 2H), 6.66 (*d, J* = 8.2 Hz, 1H), 6.60 (*t, J* = 2.0 Hz, 2H), 6.58 (*dd*, *J* = 12.1, 2.9 Hz, 1H), 6.49-6.42 (*m,* 1H), 6.09 (*t, J* = 2.0 Hz, 2H), 4.75-4.71 (*m,* 1H), 3.73 (*dd*, *J* = 8.6, 6.3 Hz, 2H), 3.43 (*dd*, *J* = 8.8, 4.3 Hz, 2H) ppm; High resolution mass spectrum (mass-to-charge ratio): C₂₆H₂₁N₂FO₄Na⁺ [M+Na]⁺ Calculated value: 467.1378, Measured value: 467.1380.

### Example 202:

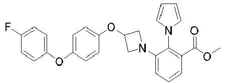

Compound 202 was synthesized according to the General Experimental Operation VII (30.2mg, 36%). ¹H NMR (300 MHz, *Chloroform-d*) *δ* 7.31 (*t, J* = 7.9 Hz, 1H), 7.17 (*dd, J* = 7.6, 1.3 Hz, 1H), 7.05-6.96 (*m,* 2H), 6.91 (*ddd, J* = 10.4, 5.2, 3.1 Hz, 4H), 6.76-6.59 (*m,* 5H), 6.27 (*t, J* = 2.1 Hz, 2H), 4.86-4.74 (*m,* 1H), 3.80 (*dd*, *J* = 9.0, 6.2 Hz, 2H), 3.66 (*dd*, *J* = 9.1, 4.9 Hz, 2H), 3.63 (*s*, 3H) ppm; High resolution mass spectrum (mass-to-charge ratio): C₂₇H₂₃N₂FO₄Na⁺ [M+Na]⁺ Calculated value: 481.1534, Measured value: 481.1535.

### Example 203:

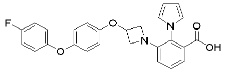

Compound 203 was synthesized according to the General Experimental Operation VII (10mg, 68%). ¹H NMR (500 MHz, *Methanol-d4*) *δ* 7.21 (*t, J* = 7.9 Hz, 1H), 7.00-6.95 (*m,* 1H), 6.95-6.88 (*m,* 2H), 6.85-6.73 (*m,* 4H), 6.65 (*t, J* = 6.5 Hz, 1H), 6.63-6.55 (*m,* 4H), 6.08 (*t, J* = 2.0 Hz, 2H), 4.70-4.68 (*m,* 1H), 3.70 (*dd*, *J* = 8.6, 6.3 Hz, 2H), 3.42 (*dd*, *J* = 8.8, 4.4 Hz, 2H) ppm; High resolution mass spectrum (mass-to-charge ratio): C₂₆H₂₁N₂FO₄Na⁺ [M+Na]⁺ Calculated value: 467.1378, Measured value: 467.1379.

### Example 204:

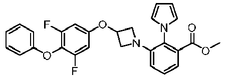

Compound 204 was synthesized according to the General Experimental Operation VII (13mg, 13%). ¹H NMR (300 MHz, *Chloroform-d*) *δ* 7.30 (*ddd, J* = 7.5, 6.8, 3.7 Hz, 3H), 7.18 (*dd, J* = 7.6, 1.4 Hz, 1H), 7.05 (*t, J* = 7.4 Hz, 1H), 6.91 (*d, J* = 8.0 Hz, 2H), 6.75-6.66 (*m,* 3H), 6.41-6.31 (*m,* 2H), 6.27 (*t, J* = 2.1 Hz, 2H), 4.83-4.71 (*m,* 1H), 3.81 (*dd*, *J* = 9.1, 6.2 Hz, 2H), 3.68-3.63 (*m,* 2H), 3.61 (*s*, 3H) ppm; High resolution mass spectrum (mass-to-charge ratio): C₂₇H₂₂N₂F₂O₄Na⁺ [M+Na]⁺ Calculated value: 499.1440, Measured value: 499.1441.

### Example 205:

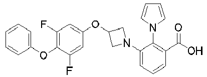

Compound 205 was synthesized according to the General Experimental Operation VII (4mg, 59%). ¹H NMR (500 MHz, *Methanol-d4*) *δ* 7.26-7.16 (*m,* 3H), 7.04-6.88 (*m,* 2H), 6.76 (*d, J* = 8.0 Hz, 2H), 6.67 (*d, J* = 8.1 Hz, 1H), 6.61 (*d*, *J* = 13.2 Hz, 2H), 6.47 (*d, J* = 9.4 Hz, 2H), 6.12-6.08 (*m,* 2H), 4.82-4.80 (*m,* 1H), 3.82-3.72 (*m,* 2H), 3.44 (*dd*, *J* = 8.6, 3.9 Hz, 2H) ppm; High resolution mass spectrum (mass-to-charge ratio): C₂₆H₂₀N₂F₂O₄Na⁺ [M+Na]⁺ Calculated value: 485.1283, Measured value: 485.1285.

### Example 206:

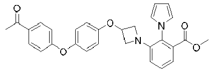

Compound 206 was synthesized according to the General Experimental Operation VII (19.4mg, 22%). ¹H NMR (500 MHz, *Chloroform-d*) *δ* 7.91 (*d, J* = 8.8 Hz, 2H), 7.30 (*t, J* = 7.9 Hz, 1H), 7.16 (*dd, J* = 7.6, 1.0 Hz, 1H), 6.98 (*d, J* = 8.9 Hz, 2H), 6.93 (*d, J* = 8.8 Hz, 2H), 6.71 (*dt*, *J* = 6.9, 3.2 Hz, 5H), 6.26 (*t, J* = 2.0 Hz, 2H), 4.87-4.77 (*m,* 1H), 3.81 (*dd*, *J* = 8.7, 6.3 Hz, 2H), 3.67 (*dd*, *J* = 8.9, 4.8 Hz, 2H), 3.62 (*s*, 3H), 2.56 (*s*, 3H) ppm; High resolution mass spectrum (mass-to-charge ratio): C₂₉H₂₆N₂O₅Na⁺ [M+Na]⁺ Calculated value: 505.1734, Measured value: 505.1734.

### Example 207:

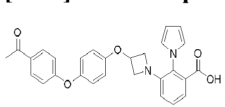

Compound 207 was synthesized according to the General Experimental Operation VII (7mg, 65%). ¹H NMR (400 MHz, *Methanol-d4*) *δ* 7.86 (*d, J* = 9.0 Hz, 2H), 7.23 (*t, J* = 7.9 Hz, 1H), 6.98 (*dd*, *J* = 7.6, 1.2 Hz, 1H), 6.94-6.87 (*m,* 2H), 6.86-6.81 (m, 2H), 6.75-6.65 (*m,* 3H), 6.62 (*t, J* = 2.1 Hz, 2H), 6.09 (*t, J* = 2.1 Hz, 2H), 4.76-4.74 (*m,* 1H), 3.74 (*dd, J* = 9.0, 6.2 Hz, 2H), 3.45 (*dd, J* = 9.0, 4.4 Hz, 2H), 2.45 (*s*, 3H) ppm; High resolution mass spectrum (mass-to-charge ratio): C₂₈H₂₄N₂O₅Na⁺ [M+Na]⁺ Calculated value: 491.1577, Measured value: 491.1580.

### Example 208:

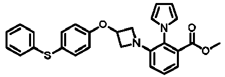

B Compound 208 was synthesized according to the General Experimental Operation VII (20mg, 25%). ¹H NMR (300 MHz, Chloroform-d) δ 7.40-7.07 (m, 9H), 6.84-6.46 (m, 5H), 6.24 (t, *J* = 2.2 Hz, 2H), 4.81 (p, *J* = 5.4 Hz, 1H), 3.79 (dd, *J* = 8.7, 6.1 Hz, 2H), 3.63 (d, *J* = 13.2 Hz, 5H) ppm; High resolution mass spectrum (mass-to-charge ratio): C₂₇H₂₄N₂O₃SNa⁺ [M+Na]⁺ Calculated value: 479.1405, Measured value: 479.1401.

### Example 209:

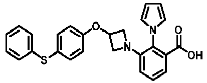

Compound 209 was synthesized according to the General Experimental Operation VII (8mg, 80%). ¹H NMR (400 MHz, Methanol-*d₄*) *δ* 7.38-7.28 (*m,* 3H), 7.27-7.20 (*m,* 3H), 7.15 (*dd, J* = 8.0, 6.0 Hz, 3H), 7.06 (*dd*, *J* = 7.7, 1.3 Hz, 1H), 6.84-6.66 (*m,* 5H), 6.18 (*t, J* = 2.1 Hz, 2H), 3.83 (*dd*, *J* = 8.8, 6.2 Hz, 2H), 3.53 (*dd, J* = 8.8, 4.3 Hz, 2H) ppm; High resolution mass spectrum (mass-to-charge ratio): C₂₆H₂₃N₂O₃S⁺ [M+H]⁺ Calculated value: 443.1429, Measured value: 443.1435.

### Example 210:

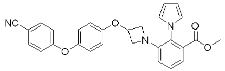

Compound 210 was synthesized according to the General Experimental Operation VII (19.4mg, 22%). ¹H NMR (300 MHz, *Chloroform-d*) *δ* 7.65-7.52 (*m,* 2H), 7.31 (*t, J* = 7.9 Hz, 1H), 7.17 (*dd*, *J* = 7.6, 1.2 Hz, 1H), 7.03-6.88 (*m,* 4H), 6.74-6.68 (*m,* 5H), 6.27-6.23 (*m,* 2H), 4.82-4.80 (*m,* 1H), 3.85-3.77 (*m,* 2H), 3.67 (*dd*, *J* = 9.0, 4.9 Hz, 2H), 3.63 (*s*, 3H) ppm; High resolution mass spectrum (mass-to-charge ratio): C₂₈H₂₃N₃O₄Na⁺ [M+Na]⁺ Calculated value: 488.1581, Measured value: 488.1582.

### Example 211:

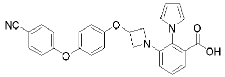

Compound 211 was synthesized according to the General Experimental Operation VII (6mg, 69%). ¹H NMR (500 MHz, *Methanol-d4*) *δ* 7.89-3.87 (*m,* 1H), 7.56 (*d, J* = 8.9 Hz, 2H), 7.22 (*t, J* = 7.9 Hz, 1H), 6.97 (*d, J* = 7.2 Hz, 1H), 6.92-6.88 (*m,* 3H), 6.76-6.68 (*m,* 2H), 6.66 (*d, J* = 8.0 Hz, 1H), 6.61 (*d, J* = 1.8 Hz, 2H), 6.08 (*d, J* = 1.8 Hz, 2H), 4.76-4.73 (*m,* 1H), 3.76-3.69 (*m,* 2H), 3.44 (*dd, J* = 8.8, 4.4 Hz, 2H) ppm; High resolution mass spectrum (mass-to-charge ratio): C₂₇H₂₁N₃O₄Na⁺ [M+Na]⁺ Calculated value: 474.1424, Measured value: 474.1426.

### Example 212:

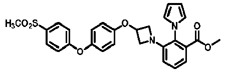

Compound 212 was synthesized according to the General Experimental Operation VII (15mg, 16%). ¹H NMR (300 MHz, *Chloroform-d*) *δ* 7.86 (*d, J* = 8.9 Hz, 2H), 7.31 (*t, J* = 7.9 Hz, 1H), 7.17 (*dd*, *J* = 7.6, 1.3 Hz, 1H), 7.06-6.94 (*m,* 4H), 6.77-6.68 (m, 5H), 6.27 (*t, J* = 2.1 Hz, 2H), 4.89-4.77 (*m,* 1H), 3.88-3.76 (*m,* 2H), 3.68 (*dd*, *J* = 8.9, 4.9 Hz, 2H), 3.63 (*s*, 3H), 3.05 (*s*, 3H) ppm; High resolution mass spectrum (mass-to-charge ratio): C₂₈H₂₆N₂O₆SNa⁺ [M+Na]⁺ Calculated value: 541.1404, Measured value: 541.1405.

### Example 213:

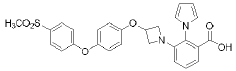

Compound 213 was synthesized according to the General Experimental Operation VII (5mg, 73%). ¹H NMR (300 MHz, *Methanol-d4*) *δ* 7.79 (*d, J* = 8.8 Hz, 2H), 7.21 (*t, J* = 7.8 Hz, 1H), 6.95 (*t, J* = 9.5 Hz, 5H), 6.73 (*d, J* = 8.9 Hz, 2H), 6.64-6.62 (*m,* 3H), 6.09-6.07 (*m,* 2H), 4.97-4.93 (*m,* 1H), 3.74-3.70 (*m,* 2H), 3.44 (*dd*, *J* = 8.3, 4.4 Hz, 2H), 2.99 (s, 3H) ppm; High resolution mass spectrum (mass-to-charge ratio): C₂₇H₂₄N₂O₆SNa⁺ [M+Na]⁺ Calculated value: 527.1247, Measured value: 527.1250.

### Example 214:

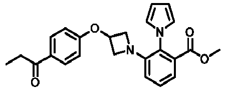

Compound 214 was synthesized according to the General Experimental Operation VII (15mg, 20%). ¹H NMR (500 MHz, Chloroform-*d*) *δ* 7.94-7.88 (*m,* 2H), 7.30 (*q*, *J* = 7.6 Hz, 2H), 7.20-7.14 (*m,* 1H), 6.76-6.66 (*m,* 5H), 6.34-6.18 (*m,* 2H), 4.90 (*tt*, *J* = 6.2, 4.7 Hz, 1H), 3.87-3.81 (*m,* 2H), 3.67 (*dd*, *J* = 9.0, 4.6 Hz, 2H), 3.62 (*s*, 3H), 2.94 (*q*, *J* = 7.3 Hz, 2H), 1.21 (*t*, *J* = 7.3 Hz, 3H) ppm; High resolution mass spectrum (mass-to-charge ratio): C₂₄H₂₅N₂O₄+[M+H]⁺ Calculated value: 405.1814, Measured value: 405.1810.

### Example 215:

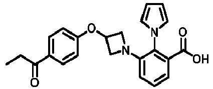

Compound 215 was synthesized according to the General Experimental Operation VII (8mg, 79%). ¹H NMR (300 MHz, Methanol-*d₄*) *δ* 8.01-7.85 (*m,* 2H), 7.31 (*t, J* = 7.9 Hz, 1H), 7.07 (*dd*, *J* = 7.6, 1.3 Hz, 1H), 6.88-6.78 (*m,* 2H), 6.75 (*dd*, *J* = 8.3, 1.3 Hz, 1H), 6.70 (*t, J* = 2.1 Hz, 2H), 6.17 (*t, J* = 2.1 Hz, 2H), 3.87-3.81 (*m,* 2H), 3.52 (*dd*, *J* = 9.0, 4.2 Hz, 2H), 2.97 (*q*, *J* = 7.2 Hz, 2H), 1.15 (*t*, *J* = 7.3 Hz, 3H) ppm; High resolution mass spectrum (mass-to-charge ratio): C₂₃H₂₃N₂O₄+[M+H]⁺ Calculated value: 391.1658, Measured value: 391.1652.

### Example 216:

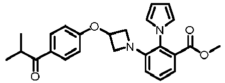

Compound 216 was synthesized according to the General Experimental Operation VII (15mg, 25%). ¹H NMR (400 MHz, Chloroform-*d*) *δ* 7.95-7.88 (*m,* 2H), 7.30 (*t, J* = 7.9 Hz, 1H), 7.16 (*dd*, *J* = 7.7, 1.4 Hz, 1H), 6.74-6.66 (*m,* 5H), 6.27-6.23 (*m,* 2H), 4.93-4.85 (*m,* 1H), 3.83 (*dd*, *J* = 8.8, 6.2 Hz, 2H), 3.64 (t, *J* = 4.7 Hz, 2H), 3.61 (*s*, 3H), 3.51 (*q, J* = 6.8 Hz, 1H), 1.19 (*d, J* = 6.8 Hz, 6H) ppm; High resolution mass spectrum (mass-to-charge ratio): C₂₅H₂₆N₂O₄Na⁺ [M+Na]⁺ Calculated value: 441.1790, Measured value: 441.1785.

### Example 217:

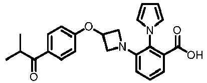

Compound 217 was synthesized according to the General Experimental Operation VII (9mg, 93%). ¹H NMR (300 MHz, Methanol-*d₄*) *δ* 7.99-7.86 (*m,* 2H), 7.31 (*t, J* = 7.9 Hz, 1H), 7.04 (*dd*, *J* = 7.6, 1.3 Hz, 1H), 6.87-6.78 (*m,* 2H), 6.77-6.69 (*m,* 3H), 6.17 (*t, J* = 2.1 Hz, 2H), 4.96-4.92 (*m,* 1H), 3.88-3.81 (*m,* 2H), 3.63-3.57 (*m,* 2H), 3.53 (*d*, *J* = 4.9 Hz, 1H), 1.15 (*d, J* = 6.8 Hz, 6H) ppm; High resolution mass spectrum (mass-to-charge ratio): C₂₄H₂₄N₂O₄Na⁺ [M+Na]⁺ Calculated value: 427.1634, Measured value: 427.1630.

### Example 218:

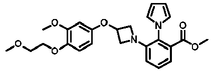

Compound 218 was synthesized according to the General Experimental Operation VII (20mg, 30%). ¹H NMR (300 MHz, Chloroform-*d*) *δ* 7.35-7.22 (*m,* 1H), 7.13 (*t*, *J* = 6.8 Hz, 1H), 6.83-6.56 (*m,* 4H), 6.26-6.22 (*m,* 2H), 6.13 (*s*, 1H), 4.79-4.74 (*m,* 1H), 4.10-4.07 (*m,* 2H), 3.85-3.71 (*m,* 3H), 3.62 (*d, J* = 10.5 Hz, 5H), 3.41 (*s*, 3H) ppm; High resolution mass spectrum (mass-to-charge ratio): C₂₅H₂₈N₂O₆Na⁺ [M+Na]⁺ Calculated value: 475.1845, Measured value: 475.1843.

### Example 219:

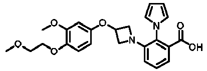

Compound 219 was synthesized according to the General Experimental Operation VII (10mg, 71%). ¹H NMR (300 MHz, Methanol-*d₄*) *δ* 7.30 (*t, J* = 7.9 Hz, 1H), 7.04 (*dd, J* = 7.6, 1.4 Hz, 1H), 6.83 (*d, J* = 8.7 Hz, 1H), 6.78-6.64 (*m,* 3H), 6.43 (*d, J* = 2.8 Hz, 1H), 6.22-6.08 (*m,* 3H), 4.77 (*tt*, *J* = 6.0, 4.4 Hz, 1H), 4.07-3.99 (*m,* 2H), 3.77 (*s*, 3H), 3.71-3.66 (*m,* 2H), 3.49 (*dd*, *J* = 8.9, 4.4 Hz, 2H), 3.40 (*s*, 3H) ppm; High resolution mass spectrum (mass-to-charge ratio): C₂₄H₂₆N₂O₆Na⁺ [M+Na]⁺ Calculated value: 461.1689, Measured value: 461.1682.

### Example 220:

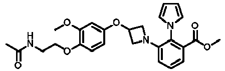

Compound 220 was synthesized according to the General Experimental Operation VII (14mg, 20%). ¹H NMR (300 MHz, Chloroform-*d*) *δ* 7.27 (*d, J* = 8.3 Hz, 1H), 7.14 (*d, J* = 7.5 Hz, 1H), 6.78 (*d, J* = 8.7 Hz, 1H), 6.73-6.61 (*m,* 3H), 6.38 (*d, J* = 2.8 Hz, 1H), 6.24 (*t, J* = 2.2 Hz, 2H), 6.08 (*dd, J* = 8.7, 2.8 Hz, 1H), 4.79-4.74 (*m,* 1H), 4.00 (*t, J* = 5.0 Hz, 2H), 3.79 (*d, J* = 12.3 Hz, 5H), 3.61 (*d, J* = 4.6 Hz, 5H), 2.00 (*s*, 3H) ppm; High resolution mass spectrum (mass-to-charge ratio): C₂₆H₂₉N₃O₆Na⁺ [M+Na]⁺ Calculated value: 502.1954, Measured value: 502.1950.

### Example 221:

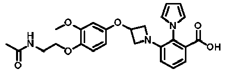

Compound 221 was synthesized according to the General Experimental Operation VII (7mg, 68%). ¹H NMR (300 MHz, Methanol-*d₄*) *δ* 7.30 (*t, J* = 7.9 Hz, 1H), 7.03 (*dt*, *J* = 7.0, 2.1 Hz, 1H), 6.83 (*dd*, *J* = 8.8, 2.2 Hz, 1H), 6.76-6.65 (*m,* 3H), 6.45 (*d, J* = 2.7 Hz, 1H), 6.21-6.10 (*m,* 3H), 4.81-4.71 (*m,* 1H), 4.01-3.95 (*m,* 2H), 3.91-3.83 (*m,* 2H), 3.78-3.74 (*m,* 5H), 3.41-3.55 (*m,* 2H), 1.96 (*s*, 3H) ppm; High resolution mass spectrum (mass-to-charge ratio): C₂₅H₂₇N₃O₆Na⁺ [M+Na]⁺ Calculated value: 488.1798, Measured value: 488.1794.

### Example 222:

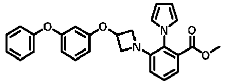

Compound 222 was synthesized according to the General Experimental Operation VIII (20mg, 24%). ¹H NMR (300 MHz, Chloroform-*d*) *δ* 7.44-7.24 (*m,* 3H), 7.23-7.08 (*m,* 3H), 7.01 (*d, J* = 8.0 Hz, 2H), 6.77-6.55 (*m,* 4H), 6.45-6.32 (*m,* 2H), 6.25 (*t, J* = 2.2 Hz, 2H), 4.82-4.75 (*m,* 1H), 3.76 (*dd, J* = 8.6, 6.1 Hz, 2H), 3.63 (*d, J* = 11.1 Hz, 5H) ppm; High resolution mass spectrum (mass-to-charge ratio): C₂₇H₂₄N₂O₄Na⁺ [M+Na]⁺ Calculated value: 463.1634, Measured value: 463.1629.

### Example 223:

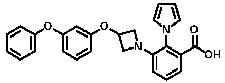

Compound 223 was synthesized according to the General Experimental Operation VIII (7mg, 68%). ¹H NMR (300 MHz, Methanol-*d₄*) *δ* 7.40-7.26 (*m,* 3H), 7.24-7.02 (*m,* 3H), 6.96 (*dd, J* = 7.8, 1.5 Hz, 2H), 6.79-6.66 (*m,* 3H), 6.50 (*ddd, J* = 22.9, 8.2, 2.3 Hz, 2H), 6.32 (*t, J* = 2.3 Hz, 1H), 6.17 (*t, J* = 2.1 Hz, 2H), 4.78 (*ddd, J* = 10.4, 6.1, 4.4 Hz, 1H), 3.76 (*dd*, *J* = 8.8, 6.1 Hz, 2H), 3.49 (*dd, J* = 8.8, 4.3 Hz, 2H) ppm; High resolution mass spectrum (mass-to-charge ratio): C₂₆H₂₃N₂O₄+[M+H]⁺ Calculated value: 427.1658, Measured value: 427.1652.

### Example 224:

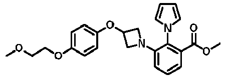

Compound 224 was synthesized according to the General Experimental Operation VIII (23mg, 30%). ¹H NMR (500 MHz, Chloroform-*d*) *δ* 7.28 (*t, J* = 7.9 Hz, 1H), 7.18-7.09 (*m,* 1H), 6.92-6.86 (*m,* 4H), 6.71-6.65 (*m,* 3H), 6.24 (*t, J* = 2.2 Hz, 2H), 4.76 (*t, J* = 5.7 Hz, 1H), 4.05 (*dd, J* = 5.6, 3.7 Hz, 2H), 3.73 (*dt*, *J* = 11.8, 4.5 Hz, 4H), 3.63 (*d, J* = 16.5 Hz, 5H), 3.45 (*s*, 3H) ppm; High resolution mass spectrum (mass-to-charge ratio): C₂₄H₂₇N₂O₅+[M+H]⁺ Calculated value: 423.1920, Measured value: 423.1915.

### Example 225:

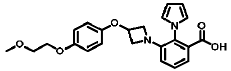

Compound 225 was synthesized according to the General Experimental Operation VIII (10mg, 67%). ¹H NMR (300 MHz, Methanol-*d₄*) *δ* 7.29 (*d, J* = 7.9 Hz, 1H), 7.04 (*dd*, *J* = 7.6, 1.4 Hz, 1H), 6.88-6.80 (*m,* 2H), 6.77-6.59 (*m,* 5H), 6.18 (*t, J* = 2.1 Hz, 2H), 4.80-4.71 (*m,* 1H), 4.06-3.98 (*m,* 2H), 3.82-3.74 (*m,* 2H), 3.72-3.67 (*m,* 2H), 3.49 (*dd*, *J* = 8.8, 4.4 Hz, 2H), 3.41 (*s*, 3H) ppm; High resolution mass spectrum (mass-to-charge ratio): C₂₃H₂₄N₂O₅⁺[M+H]⁺ Calculated value: 409.1763, Measured value: 409.1759.

### Example 226:

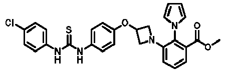

Compound 226 was synthesized according to the General Experimental Operation X (11mg, 50%). ¹H NMR (300 MHz, Chloroform-*d*) *δ* 7.58 (*s*, 1H), 7.44 (*s*, 1H), 7.32 (*d, J* = 13.4 Hz, 4H), 7.22 (*s*, 1H), 7.17 (*d, J* = 7.2 Hz, 1H), 6.79-6.65 (*m,* 5H), 6.25 (*t, J* = 2.2 Hz, 2H), 4.83 (*t*, *J* = 5.6 Hz, 1H), 3.85-3.77 (*m,* 2H), 3.67-3.61 (*m*, *J* = 12.1 Hz, 5H) ppm; High resolution mass spectrum (mass-to-charge ratio): C₂₈H₂₅ClN₄O₃SNa⁺ [M+Na]⁺ Calculated value: 555.1234, Measured value: 555.1230.

### Example 227:

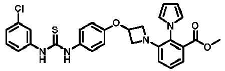

Compound 227 was synthesized according to the General Experimental Operation X (11mg, 50%). ¹H NMR (500 MHz, Chloroform-*d*) *δ* 7.94-7.86 (*m,* 1H), 7.72-7.62 (*m,* 1H), 7.44-7.19 (*m,* 6H), 6.75 (*d, J* = 17.1 Hz, 5H), 6.34-6.19 (*m,* 2H), 4.85 (*s*, 1H), 3.89-3.75 (*m,* 2H), 3.68-3.64 (*m*, 5H) ppm; High resolution mass spectrum (mass-to-charge ratio): C₂₈H₂₅ClN₄O₃SNa⁺ [M+Na]⁺ Calculated value: 555.1234, Measured value: 555.1230.

### Example 228:

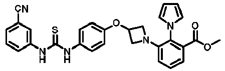

Compound 228 was synthesized according to the General Experimental Operation X (12mg, 54%). ¹H NMR (300 MHz, Chloroform-*d*) *δ* 7.80-7.71 (*m,* 2H), 7.51-7.43 (*m,* 2H), 7.33-7.22 (*m,* 2H), 7.19-7.13 (*m,* 1H), 6.78 (*d, J* = 8.5 Hz, 2H), 6.73-6.65 (*m,* 3H), 6.25 (*t, J* = 2.1 Hz, 2H), 3.89-3.76 (*m,* 2H), 3.71-3.54 (*m,* 5H) ppm; High resolution mass spectrum (mass-to-charge ratio): C₂₉H₂₅N₅O₃SNa⁺ [M+Na]⁺ Calculated value: 546.1576, Measured value: 546.1572.

### Example 229:

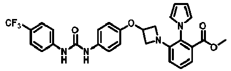

Compound 229 was synthesized according to the General Experimental Operation X (12mg, 44%). ¹ H NMR (300 MHz, Chloroform-*d*) *δ* 7.50 (*q*, *J* = 8.7 Hz, 4H), 7.30 (*t, J* = 7.9 Hz, 1H), 7.25-7.12 (*m,* 3H), 6.77 (*s*, 1H), 6.73-6.64 (*m,* 4H), 6.50 (*s*, 1H), 6.25 (*t, J* = 2.1 Hz, 2H), 4.81 (*t*, *J* = 5.5 Hz, 1H), 3.80 (*t*, *J* = 7.5 Hz, 2H), 3.68-3.56 (*m,* 5H) ppm; High resolution mass spectrum (mass-to-charge ratio): C₂₉H₂₅F₃N₄O₄Na⁺ [M+Na]⁺ Calculated value: 573.1726, Measured value: 573.1721.

### Example 230:

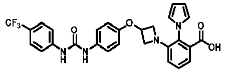

Compound 230 was synthesized according to the General Experimental Operation X (6mg, 78%). ¹H NMR (400 MHz, Methanol-*d₄*) *δ* 7.64-7.52 (*m,* 4H), 7.31 (*dd, J* = 8.6, 6.8 Hz, 3H), 7.06 (*dd, J* = 7.7, 1.3 Hz, 1H), 6.75 (*dd*, *J* = 8.3, 1.1 Hz, 1H), 6.73-6.67 (*m,* 4H), 6.18 (*t, J* = 2.0 Hz, 2H), 4.85-4.79 (*m,* 1H), 3.85-3.78 (*m,* 2H), 3.54-3.49 (*m,* 2H) ppm; High resolution mass spectrum (mass-to-charge ratio): C₂₈H₂₃F₃N₄O₄Na⁺ [M+Na]⁺ Calculated value: 559.1569, Measured value: 559.1566.

### Example 231:

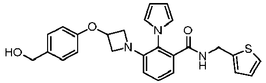

Compound 398 was synthesized according to Example 368 (8.4mg, yield 95%). ¹H NMR (300 MHz, Chloroform-*d*) δ 7.40-7.22 (m, 4H), 7.22-7.11 (m, 1H), 6.98-6.85 (m, 1H), 6.85-6.76 (m, 1H), 6.76-6.57 (m, 5H), 6.24 (t, *J* = 2.1 Hz, 2H), 5.53-5.33 (m, 1H), 4.92-4.73 (m, 1H), 4.61 (s, 2H), 4.43 (d, *J* = 5.5 Hz, 2H), 3.90-3.70 (m, 2H), 3.70-3.57 (m, 2H). High resolution mass spectrum (mass-to-charge ratio) C₂₆H₂₅N₃NaO₃S⁺ [M+Na]⁺Theoretical value: 482.1509, Measured value:482.1510.

### Example 232:

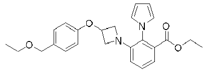

Compound 232 was synthesized according to the General Experimental Operation VI (5mg, yield 90%)¹H NMR (400 MHz, Chloroform-*d*) δ 7.32-7.21 (m, 3H), 7.21-7.11 (m, 1H), 6.74-6.62 (m, 5H), 6.24 (t, *J* = 2.1 Hz, 2H), 4.88-4.78 (m, 1H), 4.42 (s, 2H), 4.06 (q, *J* = 7.1 Hz, 2H), 3.85-3.75 (m, 2H), 3.69-3.60 (m, 2H), 3.52 (q, *J* = 7.0 Hz, 2H), 1.23 (t, *J* = 7.0 Hz, 4H), 1.09 (t, *J* = 7.1 Hz, 3H). High resolution mass spectrum (mass-to-charge ratio): C₂₅H₂₉N₂O₄⁺ [M+H]⁺ Theoretical value: 421.2122, Measured value:421.2125.

### Example 233:

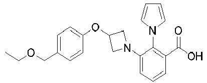

Compound 233 was synthesized according to the General Experimental Operation VI (2.3mg, yield 95%)¹H NMR (500 MHz, Chloroform-*d*) δ 7.33-7.16 (m, 4H), 6.77-6.62 (m, 5H), 6.24-6.20 (m, 2H), 4.87-4.76 (m, 1H), 4.42 (s, 2H), 3.79-3.72 (m, 2H), 3.66-3.59 (m, 2H), 3.52 (q, *J* = 7.0 Hz, 2H), 1.24-1.21 (m, 3H). High resolution mass spectrum (mass-to-charge ratio): C₂₃H₂₅N₂O₄⁺ [M+H]⁺ Theoretical value: 393.1809, Measured value:393.1810.

### Example 234:

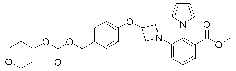

Compound 234 was synthesized according to the General Experimental Operation VII (5.3mg, yield 21%)¹H NMR (300 MHz, Chloroform-*d*) δ 7.36-7.28 (m, 3H), 7.21-7.09 (m, 1H), 6.77-6.60 (m, 5H), 6.25 (t, *J* = 2.1 Hz, 2H), 5.05 (s, 2H), 4.89-4.75 (m, 2H), 4.00-3.86 (m, 2H), 3.86-3.75 (m, 2H), 3.69-3.59 (m, 5H), 3.59-3.46 (m, 2H), 2.06-1.93 (m, 2H), 1.82-1.69 (m, 2H). High resolution mass spectrum (mass-to-charge ratio): C₂₈H₃₀N₂NaO₇⁺ [M+Na]⁺ Theoretical value: 529.1945, Measured value: 529.1949.

### Example 235:

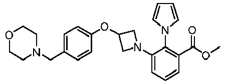

Compound 235 was synthesized according to the General Experimental Operation VII (8.1mg, yield 33%)¹H NMR (300 MHz, Chloroform-*d*) δ 7.39-7.08 (m, 4H), 6.75-6.57 (m, 5H), 6.25 (t, *J* = 2.1 Hz, 2H), 4.90-4.75 (m, 1H), 3.86-3.74 (m, 2H), 3.74-3.64 (m, 6H), 3.62 (s, 3H), 3.42 (s, 2H), 2.52-2.37 (m, 4H). High resolution mass spectrum (mass-to-charge ratio): C₂₆H₃₀N₃O₄⁺ [M+H]⁺ Theoretical value: 448.2231, Measured value:448.2230.

### Example 236:

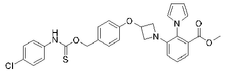

Compound 236 was synthesized according to the General Experimental Operation VI (3.9mg, yield 34%)¹H NMR (300 MHz, Chloroform-*d*) δ 7.45-7.19 (m, 6H), 7.21-7.06 (m, 2H), 6.77-6.56 (m, 5H), 6.24 (t, *J* = 2.1 Hz, 2H), 4.88-4.75 (m, 1H), 4.15 (s, 2H), 3.85-3.72 (m, 2H), 3.69-3.57 (m, 5H). High resolution mass spectrum (mass-to-charge ratio): C₂₉H₂₇ClN₃O₄S⁺ [M+H]⁺ Theoretical value: 548.1405, Measured value: 548.1409.

### Example 237:

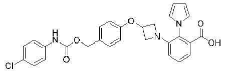

Compound 237 was synthesized according to the General Experimental Operation VI (1mg, yield 10%)¹H NMR (300 MHz, Chloroform-*d*) δ 7.42-7.11 (m, 8H), 6.78-6.55 (m, 5H), 6.29-6.04 (m, 2H), 5.10 (s, 2H), 4.79 (s, 1H), 3.80-3.51 (m, 4H). High resolution mass spectrum (mass-to-charge ratio): C₂₈H₂₄ClN₃NaO₅⁺ [M+H]⁺ Theoretical value:540.1297, Measured value:540.1300.

### Example 238:

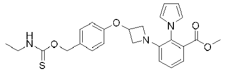

Compound 238 was synthesized according to the General Experimental Operation VI (8.3mg, yield 96%)¹H NMR (500 MHz, Chloroform-*d*) δ 7.34-7.28 (m, 3H), 7.15 (dd, *J* = 7.7, 1.2 Hz, 1H), 6.73-6.56 (m, 5H), 6.25 (t, *J* = 2.2 Hz, 2H), 5.39 (s, 2H), 4.83 (tt, *J* = 6.2, 3.8 Hz, 1H), 3.84-3.77 (m, 2H), 3.69-3.63 (m, 2H), 3.63-3.56 (m, 4H), 3.35-3.28 (m, 1H), 1.22 (t, *J* = 7.3 Hz, 3H). High resolution mass spectrum (mass-to-charge ratio): C₂₅H₂₇N₃NaO₄S⁺ [M+Na]⁺ Theoretical value: 488.1614, Measured value:488.1616.

### Example 239:

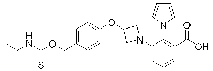

Compound 239 was synthesized according to the General Experimental Operation VI (4mg, yield 70%)¹H NMR (300 MHz, Chloroform-*d*) δ 7.36-7.04 (m, 4H), 6.78-6.57 (m, 5H), 6.38-5.99 (m, 2H), 5.42 (d, *J* = 23.7 Hz, 2H), 4.91-4.75 (m, 1H), 3.80-3.53 (m, 5H), 3.41-3.22 (m, 1H), 1.22-1.09 (m, 3H). High resolution mass spectrum (mass-to-charge ratio): C₂₄H₂₆N₃O₄S⁺ [M+H]⁺ Theoretical value: 452.1639, Measured value:452.1640.

### Example 240:

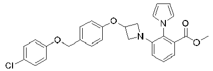

Compound 240 was synthesized according to the General Experimental Operation VI (3.4mg, yield 47%)¹H NMR (300 MHz, Chloroform-*d*) δ 7.38-7.06 (m, 6H), 6.94-6.83 (m, 2H), 6.77 (d, *J* = 8.9 Hz, 1H), 6.75-6.65 (m, 4H), 6.25 (t, *J* = 2.1 Hz, 2H), 4.94 (s, 2H), 4.89-4.79 (m, 1H), 3.86-3.75 (m, 2H), 3.69-3.63 (m, 2H), 3.62 (s, 3H). High resolution mass spectrum (mass-to-charge ratio): C₂₈H₂₆ClN₂O₄⁺ [M+H]⁺ Theoretical value: 489.1576, Measured value:489.1578.

### Example 241:

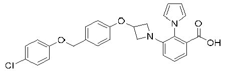

Compound 241 was synthesized according to the General Experimental Operation VI (2.5mg, yield 95%)¹H NMR (300 MHz, Chloroform-*d*) δ 7.40-7.15 (m, 6H), 6.95-6.84 (m, 2H), 6.81-6.54 (m, 5H), 6.31-6.11 (m, 2H), 4.94 (s, 2H), 4.83 (t, *J* = 5.5 Hz, 1H), 3.86-3.70 (m, 2H), 3.70-3.60 (m, 2H). High resolution mass spectrum (mass-to-charge ratio): C₂₇H₂₃ClN₂NaO₄⁺ [M+Na]⁺ Theoretical value: 497.1239, Measured value:497.1240.

### Example 242:

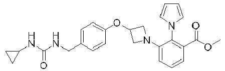

Compound 242 was synthesized according to the General Experimental Operation VI (2.3mg, yield 40%)¹H NMR (400 MHz, Chloroform-*d*) δ 7.31 (d, *J* = 7.8 Hz, 1H), 7.25-7.19 (m, 2H), 7.15 (dd, *J* = 7.7, 1.4 Hz, 1H), 6.76-6.60 (m, 5H), 6.25 (t, *J* = 2.1 Hz, 2H), 5.26-5.21 (m, 1H), 4.90-4.78 (m, 1H), 4.79-4.73 (m, 1H), 4.38 (d, *J* = 5.8 Hz, 2H), 3.85-3.75 (m, 2H), 3.68-3.63 (m, 2H), 3.62 (s, 3H), 2.47-2.39 (m, 1H), 0.76-0.69 (m, 2H), 0.62-0.55 (m, 2H). High resolution mass spectrum (mass-to-charge ratio): C₂₆H₂₉N₄O₄⁺ [M+H]⁺ Theoretical value: 461.2183, Measured value:461.2184.

### Example 243:

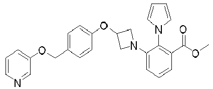

Compound 243 was synthesized according to the General Experimental Operation VI (9mg, yield 50%)¹H NMR (300 MHz, Chloroform-*d*) δ 8.37 (dd, *J* = 2.7, 1.0 Hz, 1H), 8.23 (dd, *J* = 4.1, 1.9 Hz, 1H), 7.41-7.09 (m, 6H), 6.83-6.55 (m, 5H), 6.25 (t, *J* = 2.1 Hz, 2H), 5.02 (s, 2H), 4.93-4.78 (m, 1H), 3.91-3.73 (m, 2H), 3.71-3.31 (m, 5H). High resolution mass spectrum (mass-to-charge ratio): C₂₇H₂₅N₃NaO₄⁺ [M+Na]⁺ Theoretical value: 478.1737, Measured value: 478.1738.

### Example 244:

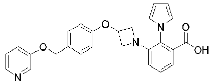

Compound 244 was synthesized according to the General Experimental Operation VI (3.8mg, yield 80%)¹H NMR (400 MHz, Chloroform-*d*) δ 8.41-8.30 (m, 1H), 8.28-8.15 (m, 1H), 7.42-7.19 (m, 6H), 6.86-6.63 (m, 5H), 6.37-6.05 (m, 2H), 5.02 (s, 2H), 4.89-4.79 (m, 1H), 3.85-3.75 (m, 2H), 3.69-3.60 (m, 2H). High resolution mass spectrum (mass-to-charge ratio): C₂₆H₂₃N₃NaO₄⁺ [M+Na]⁺ Theoretical value: 464.1581, Measured value:464.1582.

### Example 245:

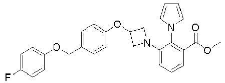

Compound 245 was synthesized according to the General Experimental Operation VI (9mg, yield 73%)¹H NMR (500 MHz, Chloroform-*d*) δ 7.39-7.27 (m, 3H), 7.20-7.08 (m, 1H), 7.02-6.93 (m, 2H), 6.93-6.86 (m, 2H), 6.78-6.48 (m, 5H), 6.26 (t, *J* = 2.1 Hz, 2H), 4.93 (s, 2H), 4.88-4.80 (m, 1H), 3.88-3.77 (m, 2H), 3.70-3.63 (m, 2H), 3.62 (s, 3H). High resolution mass spectrum (mass-to-charge ratio): C₂₈H₂₆FN₂O₄⁺ [M+H]⁺ Theoretical value: 473.1871, Measured value:473.1872.

### Example 246:

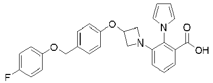

Compound 246 was synthesized according to the General Experimental Operation VI (4.2mg, yield 95%)¹H NMR (500 MHz, Chloroform-*d*) δ 7.37-7.20 (m, 4H), 7.02-6.93 (m, 2H), 6.94-6.82 (m, 2H), 6.80-6.54 (m, 5H), 6.26 (t, *J* = 2.1 Hz, 2H), 4.93 (s, 2H), 4.87-4.79 (m, 1H), 3.83-3.74 (m, 2H), 3.70-3.60 (m, 2H). High resolution mass spectrum (mass-to-charge ratio): C₂₇H₂₄FN₂O₄⁺ [M+H]⁺ Theoretical value: 459.1715, Measured value:459.1716.

### Example 247:

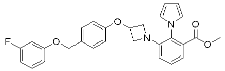

Compound 247 was synthesized according to the General Experimental Operation VI (7mg, yield 57%)¹H NMR (500 MHz, Chloroform-*d*) δ 7.37-7.28 (m, 3H), 7.25-7.19 (m, 1H), 7.17-7.09 (m, 1H), 6.83-6.53 (m, 8H), 6.25 (t, *J* = 2.1 Hz, 2H), 5.08-4.93 (m, 2H), 4.89-4.77 (m, 1H), 3.86-3.78 (m, 2H), 3.70-3.63 (m, 2H), 3.62 (s, 3H). High resolution mass spectrum (mass-to-charge ratio): C₂₈H₂₆FN₂O₄⁺ [M+H]⁺ Theoretical value: 473.1871, Measured value:473.1880.

### Example 248:

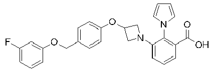

Compound 248 was synthesized according to the General Experimental Operation VI (2.8mg, yield 82%)¹H NMR (500 MHz, Chloroform-*d*) δ 7.45-7.15 (m, 5H), 6.85-6.52 (m, 8H), 6.26 (t, *J* = 2.1 Hz, 2H), 5.09-4.91 (m, 2H), 4.88-4.76 (m, 1H), 3.86-3.75 (m, 2H), 3.69-3.61 (m, 2H). High resolution mass spectrum (mass-to-charge ratio): C₂₇H₂₄FN₂O₄⁺ [M+H]⁺ Theoretical value: 459.1715, Measured value:459.1716.

### Example 249:

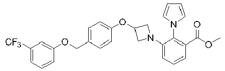

Compound 249 was synthesized according to the General Experimental Operation VI (8.9mg, yield 66%)¹H NMR (500 MHz, Chloroform-*d*) δ 7.45-6.97 (m, 8H), 6.79-6.64 (m, 5H), 6.26 (t, *J* = 2.1 Hz, 2H), 5.00 (s, 2H), 4.91-4.75 (m, 1H), 3.92-3.75 (m, 2H), 3.74-3.64 (m, 2H), 3.62 (s, 3H). High resolution mass spectrum (mass-to-charge ratio): C₂₉H₂₆F₃N₂O₄⁺ [M+H]⁺ Theoretical value: 523.1839, Measured value:523.1841.

### Example 250:

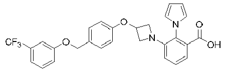

Compound 250 was synthesized according to the General Experimental Operation VI (4.4mg, yield 95%)¹H NMR (500 MHz, Chloroform-*d*) δ 7.50-7.05 (m, 8H), 6.80-6.54 (m, 5H), 6.26 (t, *J* = 2.1 Hz, 2H), 5.00 (s, 2H), 4.90-4.77 (m, 1H), 3.83-3.74 (m, 2H), 3.71-3.62 (m, 2H). High resolution mass spectrum (mass-to-charge ratio): C₂₈H₂₄F₃N₂O₄⁺ [M+H]⁺ Theoretical value: 509.1683, Measured value:509.1686.

### Example 251:

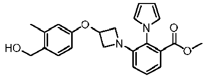

Compound 251 was synthesized according to the General Experimental Operation VII (6mg, yield 38%)¹H NMR (400 MHz, Chloroform-*d*) δ 7.35-7.26 (m, 1H), 7.23-7.11 (m, 2H), 6.75-6.65 (m, 3H), 6.56 (d, *J* = 2.7 Hz, 1H), 6.48 (dd, *J* = 8.3, 2.7 Hz, 1H), 6.25 (t, *J* = 2.1 Hz, 2H), 4.90-4.73 (m, 1H), 4.62 (s, 2H), 3.88-3.78 (m, 2H), 3.71-3.58 (m, 5H), 2.34 (s, 3H). High resolution mass spectrum (mass-to-charge ratio): C₂₃H₂₅N₂O₄⁺ [M+H]⁺ Theoretical value: 393.1809, Measured value:393.1811.

### Example 252:

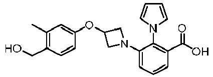

Compound 252 was synthesized according to the General Experimental Operation VII (3mg, yield 95%)¹H NMR (500 MHz, Chloroform-*d*) δ 7.35-7.12 (m, 3H), 6.81-6.61 (m, 3H), 6.56 (d, *J* = 2.6 Hz, 1H), 6.48 (dd, *J* = 8.3, 2.7 Hz, 1H), 6.29-6.03 (m, 2H), 4.86-4.76 (m, 1H), 4.62 (s, 2H), 3.79-3.70 (m, 2H), 3.67-3.56 (m, 2H), 2.33 (s, 3H). High resolution mass spectrum (mass-to-charge ratio): C₂₂H₂₃N₂O₄⁺ [M+H]⁺ Theoretical value: 379.1652, Measured value:379.1656.

### Example 253:

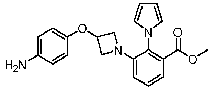

Compound 253 was synthesized according to the General Experimental Operations IX and X (7.5mg, yield 81%). ¹H NMR (300 MHz, Chloroform-*d*) δ 7.30 (d, *J* = 7.8 Hz, 1H), 7.14 (dd, *J* = 7.7, 1.5 Hz, 1H), 6.73-6.64 (m, 3H), 6.64-6.57 (m, 2H), 6.56-6.49 (m, 2H), 6.24 (t, *J* = 2.1 Hz, 2H), 4.80-4.61 (m, 1H), 3.79-3.70 (m, 2H), 3.67-3.54 (m, 5H); High resolution mass spectrum (mass-to-charge ratio) C₂₁H₂₂N₃O₃⁺ [M+H]⁺ Theoretical value: 364.1656, Measured value: 364.1652.

### Example 254:

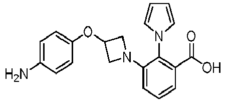

Compound 254 was synthesized according to the General Experimental Operations IX and X (4mg, yield 82%) ¹H NMR (300 MHz, Methanol-*d*₄) δ 7.26 (t, *J* = 7.8 Hz, 1H), 6.95 (d, *J* = 6.9 Hz, 1H), 6.78-6.62 (m, 5H), 6.60-6.44 (m, 2H), 6.15 (t, *J* = 1.9 Hz, 2H), 5.34 (dd, *J* = 5.4, 4.0 Hz, 1H), 3.73 (dd, *J* = 8.6, 6.1 Hz, 2H), 3.46 (dd, *J* = 8.8, 4.5 Hz, 2H): High resolution mass spectrum (mass-to-charge ratio) C20H20N3O3⁺ [M+H]⁺ Theoretical value: 350.1499, Measured value: 350.1517.

### Example 255:

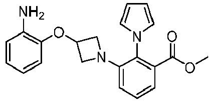

Compound 255 was synthesized according to the General Experimental Operations IX and X (15mg, yield 81%). ¹H NMR (300 MHz, Chloroform-*d*) δ 7.39-7.24 (m, 1H), 7.15 (dd, *J* = 7.6, 1.4 Hz, 1H), 6.81 (td, *J* = 7.5, 1.3 Hz, 1H), 6.74-6.58 (m, 5H), 6.42 (dd, *J* = 8.0, 1.3 Hz, 1H), 6.26 (t, *J* = 2.1 Hz, 2H), 4.94-4.75 (m, 1H), 3.90-3.78 (m, 2H), 3.69 (dd, *J* = 8.8, 4.8 Hz, 2H), 3.62 (s, 3H); High resolution mass spectrum (mass-to-charge ratio) C₂₁H₂₂N₃O₃⁺ [M+H]⁺ Theoretical value: 364.1656, Measured value: 364.1651.

### Example 256:

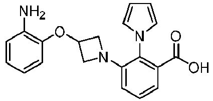

Compound 256 was synthesized according to the General Experimental Operations IX and X (6mg, yield 61%). ¹H NMR (300 MHz, Methanol-*d*₄) δ 7.21 (t, *J* = 7.8 Hz, 1H), 6.85 (dd, *J* = 7.5, 1.3 Hz, 1H), 6.80-6.71 (m, 4H), 6.59 (ddt, *J* = 8.3, 6.8, 2.0 Hz, 2H), 6.48-6.39 (m, 1H), 6.13 (t, *J* = 2.1 Hz, 2H), 5.34 (t, *J* = 4.8 Hz, 1H), 3.75 (dd, *J* = 8.8, 6.1 Hz, 2H), 3.53 (dd, *J* = 8.7, 4.8 Hz, 2H); High resolution mass spectrum (mass-to-charge ratio) C₂₀H₁₉N₃NaO₃⁺ [M+Na]⁺ Theoretical value 372.1319, Measured value: 372.1316.

### Example 257:

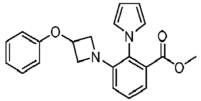

Compound 257 was synthesized according to the General Experimental Operation V (50mg, yield 42%). ¹H NMR (300 MHz, Chloroform-*d*) δ 7.39-7.21 (m, 3H), 7.14 (dd, *J* = 7.7, 1.4 Hz, 1H), 7.04-6.88 (m, 1H), 6.75-6.61 (m, 5H), 6.25 (t, *J* = 2.1 Hz, 2H), 4.90-4.74 (m, 1H), 3.84-3.75 (m, 2H), 3.69-3.63 (m, 2H), 3.61 (s, 3H). High resolution mass spectrum (mass-to-charge ratio) C₂₁H₂₀N₂NaO₃⁺ [M+Na]⁺ Theoretical value: 371.1366, Measured value: 371.1366.

### Example 258:

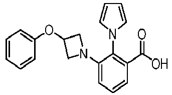

Compound 258 was synthesized according to the General Experimental Operation V (3mg, 63%). ¹H NMR (400 MHz, Methanol-*d*₄) δ 7.23 (tdd, *J* = 7.5, 2.4, 1.3 Hz, 3H), 7.03-6.82 (m, 2H), 6.76 (t, *J* = 2.1 Hz, 2H), 6.75-6.66 (m, 2H), 6.59 (dd, *J* = 8.2, 1.3 Hz, 1H), 6.14 (t, *J* = 2.1 Hz, 2H), 4.90-4.74 (m, 1H), 3.80-3.71 (m, 2H), 3.51-3.42 (m, 2H). High resolution mass spectrum (mass-to-charge ratio): C₂₀H₁₈N₂NaO₃⁺ [M+Na]⁺ Theoretical value: 357.1210, Measured value: 357.1203.

### Example 259:

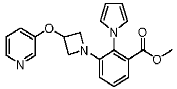

Compound 259 was synthesized according to the General Experimental Operation V (10mg, yield 32%). ¹H NMR (300 MHz, Chloroform-*d*) δ 8.24 (s, 1H), 8.11 (d, *J* = 4.6 Hz, 1H), 7.30 (t, *J* = 7.9 Hz, 1H), 7.23-7.11 (m, 2H), 6.99 (ddd, *J* = 8.4, 2.9, 1.2 Hz, 1H), 6.75-6.64 (m, 3H), 6.25 (t, *J* = 2.1 Hz, 2H), 4.93-4.81 (m, 1H), 3.86-3.77 (m, 2H), 3.71-3.63 (m, 2H), 3.61 (s, 3H).High resolution mass spectrum (mass-to-charge ratio) C₂₀H₂₀N₃O₃⁺ [M+H]⁺ Theoretical value: 350.1499, Measured value: 350.1501.

### Example 260:

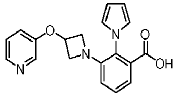

Compound 260 was synthesized according to the General Experimental Operation V (2mg, yield 66%). ¹H NMR (400 MHz, Methanol-*d*₄) δ 8.20-8.10 (brs, 2H), 7.38-7.32 (m, 1H), 7.28-7.22 (m, 3H), 6.91-6.89 (m, 1H), 6.76 (t, *J* = 2.1 Hz, 2H), 6.59 (dd, *J* = 8.2, 1.3 Hz, 1H), 6.14 (t, *J* = 2.1 Hz, 2H), 4.47-4.31 (m, 1H), 3.80-3.71 (m, 2H), 3.51-3.42 (m, 2H). High resolution mass spectrum (mass-to-charge ratio) C₁₉H₁₈N₃O₃⁺ [M+H]⁺ Theoretical value: 336.1343, Measured value: 336.1335.

### Example 261:

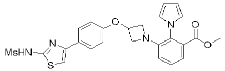

Compound 261 was synthesized according to the General Experimental Operation XV (16mg, yield 61%)¹H NMR (300 MHz, Chloroform-d) δ 7.40 (d, *J* = 8.3 Hz, 2H), 7.30 (t, *J* = 7.9 Hz, 1H), 7.17 (dd, *J* = 7.7, 1.4 Hz, 1H), 6.81-6.63 (m, 5H), 6.40 (s, 1H), 6.25 (t, *J* = 2.1 Hz, 2H), 4.86 (t, *J* = 5.4 Hz, 1H), 3.88-3.77 (m, 2H), 3.70-3.62 (m, 2H), 3.61 (s, 3H), 3.05 (s, 3H). High resolution mass spectrum (mass-to-charge ratio) C₂₅H₂₅N₄O₅S₂⁺ [M+H]⁺ Theoretical value: 525.1261, Measured value: 525.1268.

### Example 262:

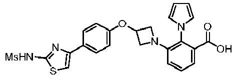

Compound 262 was synthesized according to the General Experimental Operation XV (4mg, yield 83%)¹H NMR (300 MHz, Methanol-*d*₄) δ 7.58-7.48 (m, 2H), 7.23 (dd, *J* = 8.9, 6.8 Hz, 1H), 6.89-6.76 (m, 6H), 6.60 (d, *J* = 8.2 Hz, 1H), 6.13 (t, *J* = 2.0 Hz, 2H), 3.80 (dd, *J* = 8.8, 6.1 Hz, 3H), 3.48 (dd, *J* = 9.0, 4.4 Hz, 3H), 2.99 (s, 3H).

### Example 263:

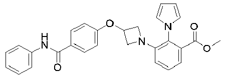

Compound 263 was synthesized according to the General Experimental Operation XVI (10mg, yield 60%)¹H NMR (300 MHz, Chloroform-*d*) δ 7.86-7.78 (m, 2H), 7.76 (s, 1H), 7.66-7.57 (m, 2H), 7.37-7.33 (m, 3H), 7.21-7.09 (m, 2H), 6.83-6.63 (m, 5H), 6.27 (q, *J* = 1.8 Hz, 2H), 4.90 (tt, *J* = 6.1, 4.7 Hz, 1H), 3.84 (dd, *J* = 8.7, 6.2 Hz, 2H), 3.67 (dd, *J* = 8.9, 4.7 Hz, 2H), 3.62 (s, 3H). High resolution mass spectrum (mass-to-charge ratio) C₂₈H₂₆N₃O₄⁺ [M+H]⁺ Theoretical value: 468.1918, True value: 468.1921.

### Example 264:

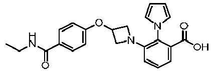

Compound 264 was synthesized according to the General Experimental Operation XVI (7mg, yield 72%)¹H NMR (300 MHz, Methanol-*d*₄) δ 7.83-7.65 (m, 2H), 7.33 (t, *J* = 7.9 Hz, 1H), 7.08 (dd, *J* = 7.7, 1.4 Hz, 1H), 6.85-6.74 (m, 3H), 6.71 (t, *J* = 2.1 Hz, 2H), 6.18 (t, *J* = 2.1 Hz, 2H), 4.19-4.05 (m, 1H), 3.94-3.75 (m, 2H), 3.54 (dd, *J* = 9.0, 4.2 Hz, 2H), 3.45-3.33 (m, 2H), 1.20 (t, *J* = 7.2 Hz, 3H). High resolution mass spectrum (mass-to-charge ratio) C₂₃H₂₃N₃NaO₄⁺ [M+Na]⁺ Theoretical value: 428.1581, Measured value: 428.1584.

### Example 265:

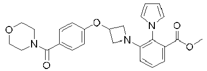

Compound 265 was synthesized according to the General Experimental Operation XVI (15mg, yield 43%)¹H NMR (300 MHz, Chloroform-*d*) δ 7.41-7.34 (m, 2H), 7.34-7.28 (m, 1H), 7.16 (dd, *J* = 7.7, 1.4 Hz, 1H), 6.80-6.61 (m, 5H), 6.26 (t, *J* = 2.1 Hz, 2H), 4.86 (p, *J* = 5.4 Hz, 1H), 3.82 (dd, *J* = 8.8, 6.2 Hz, 2H), 3.66 (d, *J* = 22.1 Hz, 11H). High resolution mass spectrum (mass-to-charge ratio) C₂₆H₂₈N₃O₅⁺ [M+H]⁺ Theoretical value: 462.2023, Measured value: 462.2027.

### Example 266:

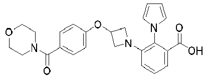

Compound 266 was synthesized according to the General Experimental Operation XVI (4.5mg, yield 99%)¹H NMR (300 MHz, Methanol-*d*₄) δ 7.42-7.36 (m, 2H), 7.36-7.28 (m, 1H), 7.08 (dd, *J* = 7.7, 1.4 Hz, 1H), 6.87-6.80 (m, 2H), 6.78 (dd, *J* = 8.2, 1.3 Hz, 1H), 6.71 (t, *J* = 2.1 Hz, 2H), 6.18 (t, *J* = 2.1 Hz, 2H), 3.86 (dd, *J* = 8.8, 6.1 Hz, 2H), 3.79-3.57 (m, 7H), 3.57-3.49 (m, 2H). High resolution mass spectrum (mass-to-charge ratio) C₂₅H₂₅N₃NaO₅⁺ [M+H]⁺ Theoretical value: 470.1686, Measured value: 470.1689.

### Example 267:

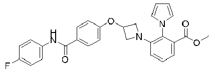

Compound 267 was synthesized according to the General Experimental Operation XVI (15mg, yield 49%)¹H NMR (500 MHz, Chloroform-*d*) δ 7.84-7.73 (m, 3H), 7.60-7.52 (m, 2H), 7.30 (t, *J* = 7.9 Hz, 1H), 7.16 (dd, *J* = 7.6, 1.3 Hz, 1H), 7.05 (t, *J* = 8.6 Hz, 2H), 6.78-6.73 (m, 2H), 6.72-6.65 (m, 3H), 6.25 (t, *J* = 2.1 Hz, 2H), 4.89 (ddd, *J* = 10.8, 6.1, 4.7 Hz, 1H), 3.83 (dd, *J* = 8.7, 6.2 Hz, 2H), 3.66 (dd, *J* = 8.8, 4.7 Hz, 2H), 3.61 (s, 3H). High resolution mass spectrum (mass-to-charge ratio) C₂₈H₂₅FN₃O₄⁺ [M+H]⁺ Theoretical value: 486.1824, Measured value: 486.1827.

### Example 268:

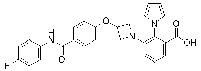

Compound 268 was synthesized according to the General Experimental Operation XVI (6mg, yield 62%) ¹H NMR (400 MHz, Methanol-*d*₄) δ 7.93-7.81 (m, 2H), 7.65 (ddd, *J* = 10.2, 5.1, 2.7 Hz, 2H), 7.33 (t, *J* = 7.9 Hz, 1H), 7.14-7.02 (m, 3H), 6.88-6.82 (m, 2H), 6.78 (dd, *J* = 8.3, 1.4 Hz, 1H), 6.71 (t, *J* = 2.1 Hz, 2H), 6.18 (t, *J* = 2.1 Hz, 2H), 4.95 (tt, *J* = 6.2, 4.3 Hz, 1H), 3.88 (dd, *J* = 8.8, 6.1 Hz, 2H), 3.55 (dd, *J* = 8.9, 4.2 Hz, 2H). High resolution mass spectrum (mass-to-charge ratio) C₂₇H₂₂FN₃NaO₄⁺ [M+Na]⁺ Theoretical value: 494.1487, Measured value: 494.1487.

### Example 269:

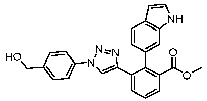

Benzyne compound (1 eq.), azide compound (4 eq.), cuprous iodide (1 eq.) and sodium ascorbate (1 eq.) were added into a one-necked flask, and then acetonitrile/water (3:2) was added and the temperature was raised to 100°C after replacing nitrogen. The reaction solution was stirred for performing a reaction for 18 hours, cooled down, diluted with ethyl acetate, dried, concentrated by filtration, and separated by a large plate to obtain a product compound 269 (6mg, yield 50%)¹H NMR (500 MHz, Chloroform-*d*) δ 8.51 (dd, *J* = 7.9, 1.4 Hz, 1H), 8.34 (s, 1H), 7.86 (dd, *J* = 7.8, 1.4 Hz, 1H), 7.71 (d, *J* = 8.1 Hz, 1H), 7.56 (t, *J* = 7.8 Hz, 1H), 7.31 (d, *J* = 8.2 Hz, 2H), 7.27-7.25 (m, 1H), 7.17 (s, 1H), 7.14-7.08 (m, 2H), 7.00 (dd, *J* = 8.0, 1.5 Hz, 1H), 6.71-6.53 (m, 1H), 6.10 (s, 1H), 4.68 (s, 2H), 3.57 (s, 3H). High resolution mass spectrum (mass-to-charge ratio): C₂₅H₂₁N₄O₃⁺ [M+H]⁺ Calculated value: 425.1608,Measured value: 425.1607.

### Example 270:

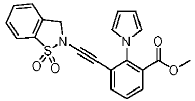

Compound 186 was synthesized according to the General Experimental Operation XII (18.0mg, 23%). ¹H NMR (300 MHz, *Chloroform-d*) δ 7.83 (d, *J* = 7.7 Hz, 1H), 7.76-7.64 (m, 3H), 7.59 (t, *J* = 7.3 Hz, 1H), 7.44-7.37 (m, 2H), 6.80 (t, *J* = 2.1 Hz, 2H), 6.31 (t, *J* = 2.1 Hz, 2H), 4.64 (s, 2H), 3.68 (s, 3H). Liquid chromatography-mass spectrometry (mass-to-charge ratio): C₂₁H₁₇N₂O₄S⁺ [M+H]⁺ Calculated value: 393.0, Measured value: 392.9.

### Example 271:

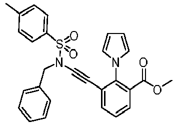

Compound 187 was synthesized according to the General Experimental Operation XII (27.5mg, 56.8%). ¹H NMR (400 MHz, *Chloroform-d*) δ 7.67 (dd, *J* = 7.7, 1.6 Hz, 1H), 7.63-7.58 (m, 2H), 7.45 (dd, *J* = 7.8, 1.6 Hz, 1H), 7.37-7.27 (m, 6H), 7.24-7.19 (m, 2H), 6.77-6.70 (m, 2H), 6.32-6.26 (m, 2H), 4.47 (s, 2H), 3.68 (s, 3H), 2.47(s, 3H). Liquid chromatography-mass spectrometry (mass-to-charge ratio): C₂₈H₂₅N₂O₄S⁺ [M+H]⁺ Calculated value: 485.1,Measured value: 485.0.

### Example 272:

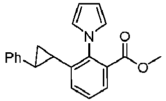

Compound 188 was synthesized according to the General Experimental Operation XIII (1mg, 9.4%). ¹H NMR (400 MHz, *Chloroform-d*) δ 7.67 (dd, *J* = 7.7, 1.5 Hz, 1H), 7.46 (t, *J* = 7.8 Hz, 1H), 7.30-7.25 (m, 3H), 7.22 (dd, *J* = 9.7, 4.4 Hz, 1H), 7.05-7.01 (m, 2H), 6.72 (s, 2H), 6.25 (s, 2H), 3.69 (s, 3H), 2.20-2.13 (m, 1H), 1.99-1.93 (m, 1H), 1.47-1.40 (m, 1H), 1.36-1.33 (m, 1H). Liquid chromatography-mass spectrometry (mass-to-charge ratio): C₂₁H₂₀NO₂⁺ [M+H]⁺ Calculated value: 318.1, Measured value: 318.0.

### Example 273:

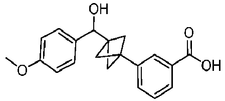

Compound 273 was synthesized according to the General Experimental Operation I (2.1mg, 39%). ¹H NMR (400 MHz, Chloroform-*d*) δ 7.98-7.90 (m, 2H), 7.89 (s, 1H), 7.46-7.33 (m, 2H), 7.26-7.22 (m, 2H), 6.91 (d, *J* = 8.7 Hz, 2H), 4.80 (s, 1H), 3.83 (s, 3H), 2.01-1.90 (m, 6H) ppm; High resolution mass spectrum (mass-to-charge ratio): C₂₀H₁₉O₄⁻ [M-H]⁻ Calculated value:323.1289, Measured value: 323.1290.

### Example 274:

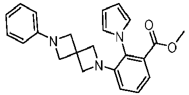

Compound 274 was synthesized according to the General Experimental Operation III (9mg, yield 50%). ¹H NMR (400 MHz, Chloroform-*d*) δ 7.31-7.29 (m, 1H), 7.21-7.18 (m, 2H), 7.13 (dd,*J* = 1.6Hz, 8.0Hz, 1H), 6.77-6.73 (m, 1H), 6.70-6.69 (m, 2H), 6.65 (dd, *J* = 1.6 Hz, 8.0Hz, 1H), 6.43-6.41 (m, 2H), 6.25-6.24 (m, 2H), 3.90 (s, 4H), 3.65 (s, 4H), 3.62 (s, 3H) ppm; High resolution mass spectrum (mass-to-charge ratio): C₂₃H₂₃N₃NaO₂⁺ [M+Na]⁺ Calculated value: 396.1682,Measured value: 396.1684.

### Example 275:

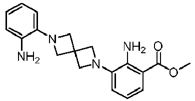

Compound 275 was synthesized according to the General Experimental Operation III (10mg, total yield 58%). ¹H NMR (400 MHz, Chloroform-*d*) δ 7.54 (dd, *J* = 1.6Hz, 8.0Hz, 1H), 6.82-6.78 (m, 3H), 6.70-6.62 (m, 3H), 5.75 (brs, 2H), 4.01 (s, 4H), 3.98 (s, 4H), 3.88 (s, 3H) ppm; High resolution mass spectrum (mass-to-charge ratio): C₁₉H₂₃N₄O₂⁺ [M+H]⁺ Calculated value: 339.1816,Measured value: 339.1817.

### Example 276:

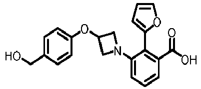

Compound 276 was synthesized according to the General Experimental Operation IV (3mg, 44%). High resolution mass spectrum (mass-to-charge ratio): C₂₁H₁₉NO₅Na⁺ [M+Na]⁺ Calculated value: 388.1161, Measured value: 388.1155.

### Example 277:

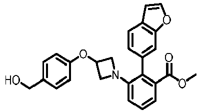

Compound 277 was synthesized according to the General Experimental Operation IV (10mg, 26%). High resolution mass spectrum (mass-to-charge ratio): C₂₆H₂₃NO₅Na⁺ [M+Na]⁺ Calculated value: 452.1474, Measured value: 452.1469.

### Example 278:

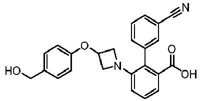

Compound 278 was synthesized according to the General Experimental Operation IV (0.7mg, 18%). High resolution mass spectrum (mass-to-charge ratio): C₂₄H₂₀N₂O₄Na⁺ [M+Na]⁺ Calculated value: 423.1321, Measured value: 423.1318.

### Example 279:

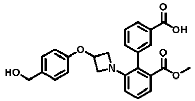

Compound 279 was synthesized according to the General Experimental Operation IV (12mg, 40%). High resolution mass spectrum (mass-to-charge ratio): C₂₅H₂₃NO₆Na⁺ [M+Na]⁺ Calculated value: 456.1423,Measured value: 456.1415.

### Example 280:

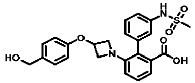

Compound 280 was synthesized according to the General Experimental Operation IV (2mg, 20%). High resolution mass spectrum (mass-to-charge ratio): C₂₄H₂₄N₂O₆SNa⁺ [M+Na]⁺ Calculated value: 491.1253,Measured value: 491.1254.

### Example 281:

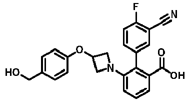

Compound 281 was synthesized according to the General Experimental Operation IV (1mg, 10%). High resolution mass spectrum (mass-to-charge ratio): C₂₄H₁₉FN₂O₄Na⁺ [M+Na]⁺ Calculated value: 441.1227,Measured value: 441.1219.

### Example 282:

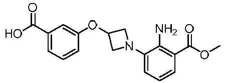

Compound 282 was synthesized according to the General Experimental Operation V (4mg, 28%), where the hydrolysis was performed by controlling the amount of alkali to obtain a monohydrolyzed product. ¹H NMR (500 MHz, Methanol-*d*₄) δ 7.65 (d, *J* = 7.6 Hz, 1H), 7.53-7.45 (m, 2H), 7.45-7.34 (m, 1H), 7.20-7.03 (m, 1H), 6.96-6.76 (m, 1H), 6.62 (t, *J=* 7.9 Hz, 1H), 5.12 (p, *J* = 5.5 Hz, 1H), 4.47-4.29 (m, 2H), 3.84 (s, 3H), 3.80-3.64 (m, 2H). High resolution mass spectrum (mass-to-charge ratio): C₁₈H19N2O5⁺ [M+H]⁺ Calculated value: 343.1288, Measured value: 343.1289.

### Example 283:

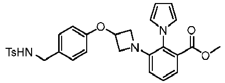

Compound 283 was synthesized according to the General Experimental Operation VI (1mg, yield 14%). ¹H NMR (300 MHz, Chloroform-*d*) δ 7.68-7.58 (m, 2H), 7.36-7.27 (m, 3H), 7.20-7.11 (m, 3H), 6.75-6.53 (m, 5H), 6.32-6.14 (m, 2H), 4.83-4.76 (m, 1H), 4.22-4.07 (m, 2H), 3.90-3.71 (m, 4H), 3.69-3.55 (m, 5H), 2.42 (s, 3H).

### Example 284:

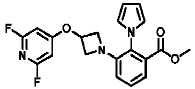

Compound 284 was synthesized according to the General Experimental Operation VII (60mg, 85%). ¹H NMR (500 MHz, *Chloroform-d*) δ 7.97 (*dt, J* = 16.7, 8.1 Hz, 1H), 7.28 (*td, J* = 8.0, 2.9 Hz, 1H), 7.14 (*ddd, J* = 7.6, 3.5, 1.2 Hz, 1H), 6.72 (*dt, J* = 8.4, 2.1 Hz, 2H), 6.70-6.61 *(m,* 1H), 6.47-6.31 *(m,* 1H), 6.26 (*dt, J* = 6.5, 2.1 Hz, 2H), 5.30-5.15 *(m,* 1H), 3.86 *(dd, J* = 11.6, 4.2 Hz, 2H), 3.72-3.53 *(m,* 5H).

### Example 285:

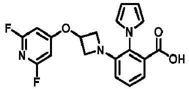

Compound 285 was synthesized according to the General Experimental Operation VII (20mg, 69%). ¹H NMR (300 MHz, *Methanol-d4*) δ 8.27-7.68 *(m,* 1H), 7.30 *(t, J* = 7.5 Hz, 1H), 7.06 (*d, J* = 7.2 Hz, 1H), 6.84-6.59 *(m,* 3H), 6.54-6.13 *(m,* 3H), 5.22-5.04 *(m,* 1H), 3.81 (*dd, J* = 11.1, 4.0 Hz, 2H), 3.65-3.45 *(m,* 2H).

### Example 286:

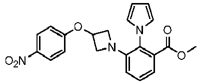

Compound 286 was synthesized according to the General Experimental Operation IX (52mg, 72%). ¹H NMR (300 MHz, *Chloroform-d*) δ 8.25-8.13 *(m,* 2H), 7.32 *(t, J* = 7.9 Hz, 1H), 7.19 (*dd, J* = 7.7, 1.3 Hz, 1H), 6.81-6.66 *(m,* 5H), 6.27 *(t, J* = 2.1 Hz, 2H), 5.01-4.84 *(m,* 1H), 3.86 *(dd, J* = 9.2, 6.2 Hz, 2H), 3.67 *(dd, J* = 9.2, 4.6 Hz, 2H), 3.63 (*s,* 3H). High resolution mass spectrum (mass-to-charge ratio): C₂₁H₂₀N₃O₅⁺ [M+H]⁺ Calculated value: 394.1397, Measured value:394.1397.

### Example 287:

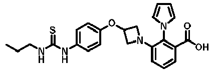

Compound 287 was synthesized according to the General Experimental Operation X (4mg, 47%, the compound was a mixture of urea and thiourea). ¹H NMR (300 MHz, *Methanol-d4*) *δ* 7.32 (*t, J* = 7.9 Hz, 1H), 7.21 *(d, J* = 8.9 Hz, 2H), 7.09-7.03 *(m,* 1H), 6.81-6.62 *(m,* 5H), 6.19 *(t, J=* 2.1 Hz, 2H), 4.84-4.76 *(m,* 1H), 3.86-3.74 *(m,* 2H), 3.51 *(dd, J* = 8.9, 4.5 Hz, 2H), 3.14 *(t, J=* 7.0 Hz, 2H), 1.58-1.46 *(m,* 2H), 0.95 *(t, J=* 7.4 Hz, 3H).

### Example 288:

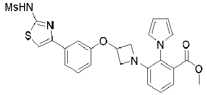

Compound 288 was synthesized according to the General Experimental Operation VII (10mg, 47.7%). ¹H NMR (300 MHz, Chloroform-*d*) δ 7.31 (q, *J=* 8.2 Hz, 2H), 7.16 (dd, *J=* 7.7, 1.4 Hz, 1H), 7.09 (d, *J* = 7.7 Hz, 1H), 6.85 (t, *J* = 2.0 Hz, 1H), 6.77-6.63 (m, 4H), 6.53 (s, 1H), 6.25 (t, *J* = 2.1 Hz, 2H), 4.88 (t, *J* = 5.2 Hz, 1H), 3.94-3.76 (m, 2H), 3.68-3.63 (m, 2H), 3.61 (s, 3H), 3.06 (s, 3H). High resolution mass spectrum (mass-to-charge ratio): C₂₅H₂₅N₄O₅S₂⁺ [M+H]⁺ Calculated value: 525.1261, Measured value: 525.1268.

### Example 289:

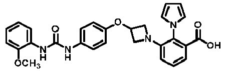

Compound 289 was synthesized according to the General Experimental Operation X (6mg, 62%, the compound was a mixture of urea and thiourea). ¹H NMR (300 MHz, *Methanol-d4*) *δ* 8.03 (*d, J=* 7.7 Hz, 1H), 7.32 (*dd, J=* 9.8, 6.3 Hz, 3H), 7.06 (*dd, J=* 7.6, 1.3 Hz, 1H), 6.97 (*d, J* = 3.8 Hz, 2H), 6.74 (*tdd, J* = 4.9, 3.2, 1.8 Hz, 5H), 6.68 *(d, J* = 2.2 Hz, 1H), 6.19 *(t, J* = 2.1 Hz, 2H), 4.85-4.78 *(m,* 1H), 3.91 (*s,* 3H), 3.81 *(dd, J* = 8.9, 6.4 Hz, 2H), 3.52 *(dd, J* = 8.9, 4.4 Hz, 2H).

### Example 290:

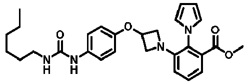

Compound 290 was synthesized according to the General Experimental Operation X (14mg, 70%). ¹H NMR (500 MHz, Chloroform-*d*) *δ* 7.29 *(t, J=* 8.0 Hz, 1H), 7.16 *(t, J* = 7.8 Hz, 3H), 6.69 *(dd, J* = 10.0, 4.7 Hz, 3H), 6.64 *(d, J* = 8.8 Hz, 2H), 6.42 (*s,* 1H), 6.25 (*s,* 2H), 4.82-4.78 *(m,* 1H), 3.83-3.75 *(m,* 2H), 3.66-3.63 *(m,* 2H), 3.61 (*s,* 3H), 3.21 *(dd, J* = 13.2, 6.8 Hz, 2H), 1.58-1.51 *(m,* 2H), 1.34 *(m,* 6H), 0.84 (*t,* 3H).

### Example 291:

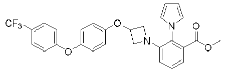

Compound 291 was synthesized according to the General Experimental Operation VII (40mg, 43%). ¹H NMR (400 MHz, *Chloroform-d*) *δ* 7.54 (*d, J=* 8.7 Hz, 2H), 7.30 *(t, J=* 7.9 Hz, 1H), 7.16 *(dd, J* = 7.6, 1.3 Hz, 1H), 7.02-6.92 *(m,* 4H), 6.75-6.64 *(m,* 5H), 6.26 *(t, J* = 2.1 Hz, 2H), 4.86-4.76 *(m,* 1H), 3.81 *(dd, J* = 8.9, 6.3 Hz, 2H), 3.67 *(dd, J* = 9.0, 4.8 Hz, 2H), 3.62 (*s,* 3H) ppm. High resolution mass spectrum (mass-to-charge ratio): C₂₈H₂₄F₃N₂O₄⁺ [M+H]⁺ Calculated value: 509.1688, Measured value: 509.1683.

### Example 292:

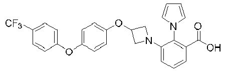

Compound 292 was synthesized according to the General Experimental Operation VII (6mg, 70%). ¹H NMR (300 MHz, *Methanol-d4*) *δ* 7.48 (*d, J* = 8.6 Hz, 2H), 7.22 *(t, J* = 7.9 Hz, 1H), 6.98 (*dd, J* = 7.6, 1.2 Hz, 1H), 6.94-6.81 *(m,* 4H), 6.75-6.63 *(m,* 3H), 6.60 *(t, J* = 2.1 Hz, 2H), 6.08 *(t, J* = 2.1 Hz, 2H), 4.74-4.72 *(m,* 1H), 3.72 *(dd, J* = 8.8, 6.2 Hz, 2H), 3.43 *(dd, J* = 9.0, 4.4 Hz, 2H) ppm. High resolution mass spectrum (mass-to-charge ratio): C₂₇H₂₁F₃N₂O₄Na⁺ [M+Na]⁺ Calculated value: 517.1351, Measured value: 517.1347.

### Example 293:

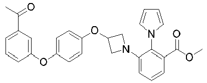

Compound 293 was synthesized according to the General Experimental Operation VII (6.1mg, 7%). High resolution mass spectrum (mass-to-charge ratio): C₂₉H₂₆N₂O₅Na⁺ [M+Na]⁺ Calculated value: 505.1734, Measured value: 505.1735.

### Example 294:

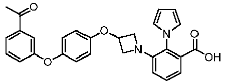

Compound 294 was synthesized according to the General Experimental Operation VII (2mg, 52%). High resolution mass spectrum (mass-to-charge ratio): C₂₈H₂₄N₂O₅Na⁺ [M+Na]⁺ Calculated value: 491.1577, Measured value: 491.1579.

### Example 295:

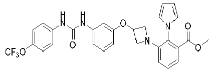

Compound 295 was synthesized according to the General Experimental Operation X (12mg, 54%). ¹H NMR (300 MHz, Chloroform-*d*) *δ* 7.41 (*s,* 1H), 7.36-7.23 (*m,* 3H), 7.19-6.98 *(m,* 5H), 6.75-6.62 *(m,* 3H), 6.61-6.47 *(m,* 2H), 6.25 *(t, J* = 2.1 Hz, 2H), 4.73-4.68 *(m,* 1H), 3.76 (*dd, J* = 8.7, 6.2 Hz, 2H), 3.65-3.52 *(m,* 5H) ppm.

### Example 296:

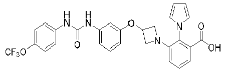

Compound 296 was synthesized according to the General Experimental Operation X (7mg, 65%). High resolution mass spectrum (mass-to-charge ratio): C₂₈H₂₃F₃N₄O₅⁺ [M+H]⁺ Theoretical value: 552.1621, Measured value:553.1694.

### Example 297:

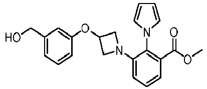

Compound 297 was synthesized according to the General Experimental Operation VII (8mg, yield 53%)¹H NMR (500 MHz, Chloroform-*d*) δ 7.35-7.20 (m, 2H), 7.15 (dd, *J=* 7.6, 1.4 Hz, 1H), 7.02-6.92 (m, 1H), 6.82-6.56 (m, 5H), 6.25 (t, *J=* 2.1 Hz, 2H), 4.93-4.78 (m, 1H), 4.65 (s, 2H), 3.86-3.78 (m, 2H), 3.68-3.63 (m, 2H), 3.62 (s, 3H). High resolution mass spectrum (mass-to-charge ratio): C₂₂H₂₃N₂O₄⁺ [M+H]⁺ Theoretical value: 379.1652, Measured value: 379.1653.

### Example 298:

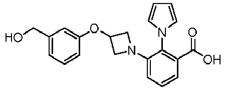

Compound 298 was synthesized according to the General Experimental Operation VII (3.9mg, yield 90%)¹H NMR (400 MHz, Chloroform-*d*) δ 7.45-7.12 (m, 3H), 6.95 (d, *J* = 7.6 Hz, 1H), 6.82-6.56 (m, 5H), 6.24 (t, *J* = 2.1 Hz, 2H), 4.90-4.79 (m, 1H), 4.65 (s, 2H), 3.85-3.73 (m, 2H), 3.70-3.61 (m, 2H). High resolution mass spectrum (mass-to-charge ratio): C₂₁H₂₁N₂O₄⁺ [M+H]⁺ Theoretical value: 365.1496, Measured value: 365.1496.

### Example 299:

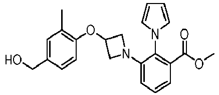

Compound 299 was synthesized according to the General Experimental Operation VII (5.8mg, yield 27%)¹H NMR (500 MHz, Chloroform-*d*) δ 7.33-7.28 (m, 1H), 7.19-7.13 (m, 2H), 7.09 (dd, *J* = 8.2, 2.2 Hz, 1H), 6.77-6.64 (m, 3H), 6.41 (d, *J* = 8.2 Hz, 1H), 6.26 (t, *J* = 2.1 Hz, 2H), 4.91-4.72 (m, 1H), 4.58 (s, 2H), 3.89-3.75 (m, 2H), 3.70-3.63 (m, 2H), 3.62 (s, 3H), 2.18 (s, 3H). High resolution mass spectrum (mass-to-charge ratio): C₂₃H₂₅N₂O₄⁺ [M+H]⁺ Theoretical value: 393.1809, Measured value: 393.1809.

### Example 300:

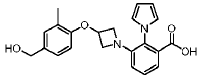

Compound 300 was synthesized according to the General Experimental Operation VII (2.1mg, yield 60%)¹H NMR (400 MHz, Chloroform-*d*) δ 7.36-7.22 (m, 2H), 7.22-7.02 (m, 2H), 6.82-6.59 (m, 3H), 6.41 (d, *J* = 8.2 Hz, 1H), 6.26 (t, *J* = 2.0 Hz, 2H), 4.91-4.76 (m, 1H), 4.58 (s, 2H), 3.88-3.74 (m, 2H), 3.71-3.59 (m, 3H), 2.18 (s, 3H). High resolution mass spectrum (mass-to-charge ratio): C₂₂H₂₃N₂O₄⁺ [M+H]⁺ Theoretical value: 379.1652, Measured value: 379.1653.

### Example 301:

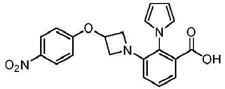

Compound 301 was synthesized according to the General Experimental Operations IX and X (3mg, yield 60%). ¹H NMR (400 MHz, Chloroform-*d*) δ 8.28-8.10 (m, 2H), 7.40-7.28 (m, 2H), 6.81-6.69 (m, 5H), 6.28 (t, *J=* 2.1 Hz, 2H), 4.99-4.77 (m, 1H), 3.83 (dd, *J=* 8.9, 6.2 Hz, 2H), 3.66 (dd, *J =* 9.0, 4.6 Hz, 2H). High resolution mass spectrum (mass-to-charge ratio), Theoretical value C₂₀H₁₈N₃O₅⁺ [M+H]⁺ 380.1241 Measured value 380.1232.

### Example 302:

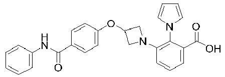

Compound 302 was synthesized according to the General Experimental Operation XVI (6mg, yield 63.7%)¹H NMR (300 MHz, Methanol-*d*₄) δ 7.97-7.82 (m, 2H), 7.70-7.56 (m, 2H), 7.33 (dt, *J* = 8.1, 7.1 Hz, 3H), 7.19-7.09 (m, 1H), 7.06 (dd, *J* = 7.6, 1.4 Hz, 1H), 6.90-6.81 (m, 2H), 6.77 (dd, *J=* 8.2, 1.3 Hz, 1H), 6.72 (t, *J=* 2.1 Hz, 2H), 6.18 (t, *J=* 2.1 Hz, 2H), 5.01-4.95 (m, 1H), 3.88 (dd, *J* = 8.8, 6.1 Hz, 2H), 3.58-3.47 (m, 2H). High resolution mass spectrum (mass-to-charge ratio), Theoretical value C₂₇H₂₄N₃O₄⁺ [M+H]⁺ Theoretical value: 454.1761 True value: 454.1761.

### Example 303:

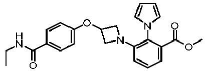

Compound 303 was synthesized according to the General Experimental Operation XVI (15mg, yield 46.8%). ¹H NMR (300 MHz, Chloroform-*d*) δ 7.76-7.62 (m, 2H), 7.29 (d, *J* = 8.0 Hz, 1H), 7.16 (dd, *J*= 7.7, 1.4 Hz, 1H), 6.70 (ddt, *J*= 7.1, 4.7, 2.2 Hz, 5H), 6.25 (t, *J*= 2.1 Hz, 2H), 6.01 (s, 1H), 4.87 (ddd, *J* = 6.2, 4.7, 1.4 Hz, 1H), 3.87-3.76 (m, 2H), 3.69-3.63 (m, 2H), 3.64 (s, 3H), 3.53-3.42 (m, 2H), 1.30-1.19 (m, 3H). High resolution mass spectrum (mass-to-charge ratio): C₂₄H₂₆N₃O₄⁺ [M+H]⁺ Theoretical value: 420.1918, Measured value: 420.1923.

### Example 304:

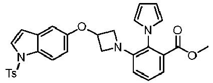

Compound 304 was synthesized according to the General Experimental Operation VII (138mg, yield 69.3%). ¹H NMR (400 MHz, Chloroform-*d*) δ 7.85 (d, *J* = 9.0 Hz, 1H), 7.78-7.65 (m, 2H), 7.51 (d, *J* = 3.6 Hz, 1H), 7.29 (d, *J* = 7.9 Hz, 1H), 7.21 (d, *J* = 7.9 Hz, 2H), 7.16-7.10 (m, 1H), 6.76 (dd, *J*= 9.0, 2.5 Hz, 1H), 6.73-6.62 (m, 4H), 6.55 (dd, *J*= 3.7, 0.8 Hz, 1H), 6.24 (t, *J=* 2.1 Hz, 2H), 4.81 (p, *J=* 5.6 Hz, 1H), 3.79 (dd, *J=* 8.7, 6.2 Hz, 2H), 3.64 (dd, *J* = 8.9, 4.8 Hz, 2H), 3.61 (s, 3H), 2.34 (s, 3H). High resolution mass spectrum (mass-to-charge ratio) C₃₀H₂₇N₃NaO₅S⁺ [M+Na]⁺ Theoretical value: 564.1564, Measured value: 564.1564

### Example 305:

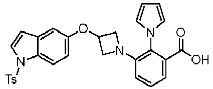

Compound 305 was synthesized according to the General Experimental Operation VII (7mg, yield 71.8%)¹H NMR (300 MHz, Chloroform-*d*) δ 7.86 (d, *J =* 9.0 Hz, 1H), 7.78-7.67 (m, 2H), 7.53 (dd, *J=* 6.0, 3.4 Hz, 1H), 7.35-7.28 (m, 2H), 7.22 (d, *J=* 8.2 Hz, 2H), 6.80-6.64 (m, 6H), 6.55 (dd, *J* = 3.6, 0.8 Hz, 1H), 6.27 (t, *J* = 2.1 Hz, 2H), 4.81 (p, *J* = 5.6 Hz, 1H), 3.84-3.73 (m, 2H), 3.64 (dd, *J* = 8.8, 4.8 Hz, 2H) , 2.34 (s, 3H). C₂₉H₂₅N₃NaO₅S⁺ [M+Na]⁺ Theoretical value: 550.1407Measured value: 550.1409

### Example 306:

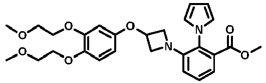

Compound 306 was synthesized according to the General Experimental Operation V (16mg, yield 32%). ¹H NMR (500 MHz, Chloroform-d) δ 7.35-7.27 (m, 1H), 7.21-7.08 (m, 1H), 6.82 (dd, *J* = 8.4, 4.2 Hz, 1H), 6.70 (s, 3H), 6.45-6.33 (m, 1H), 6.25 (s, 2H), 6.19-6.06 (m, 1H), 4.76-4.74 (m, 1H), 4.10-4.08 (m, 4H), 3.84-3.68 (m, 6H), 3.67-3.55 (m, 5H), 3.44 (d, 6H). High resolution mass spectrum (mass-to-charge ratio) C₂₇H₃₂N₂NaO₇⁺ [M+Na]⁺ Theoretical value: 519.2107, Measured value: 519.2112.

### Example 307:

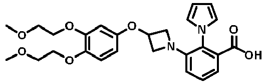

Compound 307 was synthesized according to the General Experimental Operation V (4mg, yield 61%). ¹H NMR (300 MHz, Methanol-*d*₄) δ 7.30 (t, *J=* 7.9 Hz, 1H), 7.03 (dt, *J=* 7.7, 1.7 Hz, 1H), 6.85 (dd, *J* = 8.7, 2.1 Hz, 1H), 6.77-6.72 (m, 1H), 6.71-6.70 (m, 2H), 6.46 (dd, *J* = 4.5, 2.8 Hz, 1H), 6.21 (d, *J* = 2.9 Hz, 1H), 6.22-6.17 (m, 2H), 4.77 (tt, *J* = 6.1, 4.5 Hz, 1H), 4.11-4.03 (m, 4H), 3.81-3.65 (m, 6H), 3.48 (dd, *J =* 8.8, 4.4 Hz, 2H), 3.41 (d, 6H). High resolution mass spectrum (mass-to-charge ratio) C₂₆H₃₀N₂O₇Na⁺ [M+Na]⁺ Theoretical value: 505.1951, Measured value: 505.1947.

### Example 308:

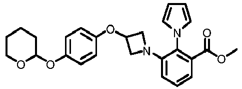

Compound 308 was synthesized according to the General Experimental Operation V (10 mg, yield 39%). ¹H NMR (300 MHz, Chloroform-d) δ 7.30 (d, *J* = 7.9 Hz, 1H), 7.14 (dd, *J* = 7.6, 1.4 Hz, 1H), 7.00-6.91 (m, 2H), 6.74-6.64 (m, 3H), 6.63-6.57 (m, 2H), 6.24 (t, *J =* 2.1 Hz, 2H), 5.28 (t, *J* = 3.3 Hz, 1H), 4.83-4.69 (m, 1H), 3.94-3.87 (m, 1H), 3.76 (dd, *J* = 8.6, 6.2 Hz, 2H), 3.65-3.60 (m, 6H), 1.88-1.79 (m, 2H), 1.68-1.57 (m, 4H). High resolution mass spectrum (mass-to-charge ratio) C₂₆H₂₈N₂O₅Na⁺ [M+Na]⁺ Theoretical value: 471.1896, Measured value: 471.1888.

### Example 309:

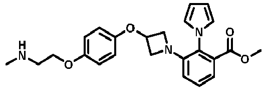

Compound 309 was synthesized according to the General Experimental Operation V (11mg, yield 9%). ¹H NMR (300 MHz, Chloroform-d) δ 7.29 (d, *J* = 7.9 Hz, 1H), 7.14 (dd, *J* = 7.7, 1.4 Hz, 1H), 6.85-6.74 (m, 2H), 6.72-6.64 (m, 3H), 6.64-6.57 (m, 2H), 6.24 (t, *J =* 2.1 Hz, 2H), 4.76-4.73 (m, 1H), 4.00 (t, *J* = 5.1 Hz, 2H), 3.80-3.70 (m, 2H), 3.62 (d, *J* = 6.7 Hz, 5H), 2.95 (t, *J* = 5.2 Hz, 2H), 2.50 (s, 3H). High resolution mass spectrum (mass-to-charge ratio) C₂₄H₂₈N₃O₄⁺ [M+H]⁺ Theoretical value: 422.2080, Measured value: 422.2074.

### Example 310:

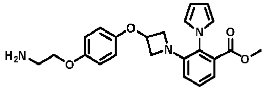

Compound 310 was synthesized according to the General Experimental Operation V (17mg, yield 14.7%). ¹H NMR (300 MHz, Chloroform-d) δ 7.29 (d, *J* = 7.9 Hz, 1H), 7.14 (dd, *J* = 7.6, 1.4 Hz, 1H), 6.86-6.75 (m, 2H), 6.71-6.65 (m, 3H), 6.64-6.56 (m, 2H), 6.24 (t, *J =* 2.1 Hz, 2H), 4.75 (tt, *J* = 6.1, 4.8 Hz, 1H), 3.93 (t, *J* = 5.0 Hz, 2H), 3.80-3.72 (m, 2H), 3.65-3.60 (m, 5H), 3.06 (t, *J* = 5.0 Hz, 2H), 2.41 (brs, 2H, NH₂). High resolution mass spectrum (mass-to-charge ratio) C₂₃H₂₆N₃O₄⁺ [M+H]⁺ Theoretical value: 408.1923, Measured value: 408.1917.

### Example 311:

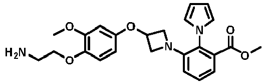

Compound 311 was synthesized according to the General Experimental Operation V (21mg, yield 23%). ¹H NMR (300 MHz, Chloroform-d) δ 7.29 (d, *J* = 7.8 Hz, 1H), 7.14 (dd, *J* = 6.4, 1.3 Hz, 1H), 6.76 (d, *J* = 8.7 Hz, 1H), 6.71-6.67 (m, 2H), 6.67-6.61 (m, 1H), 6.36 (d, *J* = 2.7 Hz, 1H), 6.24 (t, *J* = 2.1 Hz, 2H), 6.07 (dd, *J* = 8.7, 2.8 Hz, 1H), 4.78-4.72 (m, 1H), 3.96 (t, *J* = 5.1 Hz, 2H), 3.77 (d, *J* = 8.3 Hz, 5H), 3.61 (d, *J =* 5.9 Hz, 5H), 3.05 (t, *J* = 5.1 Hz, 2H). High resolution mass spectrum (mass-to-charge ratio) C₂₄H₂₈N₃O₅⁺ [M+H]⁺ Theoretical value: 438.2029, Measured value: 438.2018.

### Example 312:

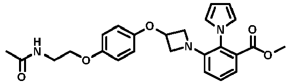

Compound 312 was synthesized according to the General Experimental Operation V (14mg, yield 10.9%). ¹H NMR (500 MHz, Chloroform-d) δ 7.33 (d, *J* = 6.3 Hz, 1H), 7.16 (s, 1H), 6.80-6.24 (m, 7H), 6.25 (s, 2H), 5.93 (brs, 1H, NH), 4.77 (s, 1H), 4.01-3.93 (m, 2H), 3.77 (s, 2H), 3.69 (s, 3H), 3.59-3.52 (m, 2H), 3.41 (s, 2H), 2.01 (s, 3H). High resolution mass spectrum (mass-to-charge ratio) C₂₅H₂₇N₃O₅Na⁺ [M+Na]⁺ Theoretical value: 472.1848, Measured value: 472.1846.

### Example 313:

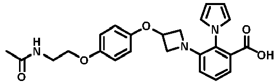

Compound 313 was synthesized according to the General Experimental Operation V (5mg, yield 74%). ¹H NMR (400 MHz, Methanol-*d*₄) δ 7.28 (t, *J* = 7.9 Hz, 1H), 6.97 (d, *J* = 7.5 Hz, 1H), 6.85-6.81 (m, 2H), 6.74 (t, *J=* 2.1 Hz, 2H), 6.70-6.63 (m, 3H), 6.17 (t, *J=* 2.0 Hz, 2H), 4.77-4.72 (m, 1H), 3.96 (d, *J* = 5.5 Hz, 2H), 3.75 (m, 2H), 3.52-3.50 (m, 2H), 3.47-3.44 (m, 2H), 1.95 (s, 3H). High resolution mass spectrum (mass-to-charge ratio) C₂₄H₂₅N₃O₅Na⁺ [M+Na]⁺ Theoretical value: 458.1692, Measured value: 458.1688.

### Example 314:

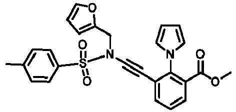

Compound 314 was synthesized according to the General Experimental Operation XII (8 mg, yield 16.2%). ¹H NMR (300 MHz, Chloroform-d) δ 7.63 (dd, *J* = 7.7, 1.6 Hz, 1H), 7.59-7.53 (m, 2H), 7.48 (dd, *J* = 7.8, 1.6 Hz, 1H), 7.32 (t, *J* = 7.8 Hz, 1H), 7.27-7.22 (m, 3H), 6.73 (t, *J* = 2.1 Hz, 2H), 6.29-6.22 (m, 3H), 6.16-6.11 (m, 1H), 4.49 (s, 2H), 3.63 (s, 3H), 2.41 (s, 3H). High resolution mass spectrum (mass-to-charge ratio) C₂₆H₂₂N₂O₅SNa⁺ [M+Na]⁺ Theoretical value: 497.1147, Measured value: 497.1143.

### Example 315:

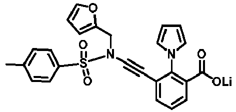

Compound 315 was synthesized according to the General Experimental Operation XII (3mg, yield 76%). High resolution mass spectrum (mass-to-charge ratio) C₂₅H₁₉N₂O₅S⁻ [M-Li]⁻Theoretical value: 459.1015, Measured value: 459.1018.

### Example 316:

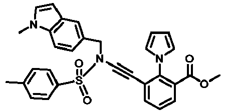

Compound 316 was synthesized according to the General Experimental Operation XII (8mg, yield 14.3%). ¹H NMR (300 MHz, Chloroform-d) δ 7.62-7.52 (m, 3H), 7.40-7.31 (m, 2H), 7.27-7.24 (m, 1H), 7.23-7.18 (m, 3H), 7.08-7.01 (m, 2H), 6.70 (t, *J=* 2.1 Hz, 2H), 6.42 (dd, *J* = 3.1, 0.9 Hz, 1H), 6.27 (t, *J* = 2.1 Hz, 2H), 4.50 (s, 2H), 3.77 (s, 3H), 3.62 (s, 3H), 2.40 (s, 3H). High resolution mass spectrum (mass-to-charge ratio) C₃₁H₂₇N₃O₄SNa⁺ [M+Na]⁺ Theoretical value: 560.1620, Measured value: 560.1616.

### Example 317:

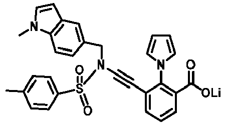

Compound 317 was synthesized according to the General Experimental Operation XII (3.8 mg, yield 78%). High resolution mass spectrum (mass-to-charge ratio) C₃₀H₂₄N₃O₄S ⁻ [M-Li]⁻Theoretical value: 522.1488, Measured value: 522.1494.

### Example 318:

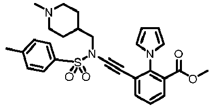

Compound 318 was synthesized according to the General Experimental Operation XII (13mg, yield 24.7%). ¹H NMR (500 MHz, Chloroform-d) δ 7.80 (d, *J* = 8.1 Hz, 1H), 7.76-7.69 (m, 2H), 7.61 (d, *J* = 7.8 Hz, 1H), 7.41 (t, *J* = 7.8 Hz, 1H), 7.36 (d, *J* = 8.0 Hz, 2H), 6.69 (t, *J* = 2.2 Hz, 2H), 6.20 (t, *J* = 2.2 Hz, 2H), 3.63 (s, 3H), 3.52-3.48 (m, 2H), 3.11 (d, *J* = 7.2 Hz, 2H), 2.76 (s, 3H), 2.72-2.62 (m, 2H), 2.46 (s, 3H), 2.42 (s, 1H), 1.90-1.74 (m, 4H). High resolution mass spectrum (mass-to-charge ratio) C₂₈H₃₂N₃O₄S⁺ [M+H]⁺ Theoretical value: 506.2114, Measured value: 506.2110.

### Example 319:

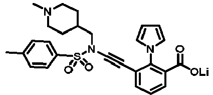

Compound 319 was synthesized according to the General Experimental Operation XII (4mg, yield 82%). High resolution mass spectrum (mass-to-charge ratio) C₂₇H₃₀N₃O₄S⁻ [M-Li]⁻Theoretical value: 492.1957, Measured value: 492.1953.

### Example 320:

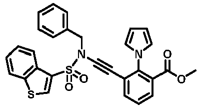

Compound 320 was synthesized according to the General Experimental Operation XII (4mg, yield 7.3%). ¹H NMR (400 MHz, Chloroform-d) δ 8.27-8.18 (m, 1H), 7.97 (s, 1H), 7.90-7.82 (m, 1H), 7.62 (dd, *J=* 7.7, 1.6 Hz, 1H), 7.46-7.41 (m, 2H), 7.39 (dd, *J=* 7.8, 1.6 Hz, 1H), 7.30 (t, *J* = 7.8 Hz, 1H), 7.25-7.21 (m, 3H), 7.18-7.10 (m, 2H), 6.63 (t, *J=* 2.1 Hz, 2H), 6.20 (t, *J=* 2.1 Hz, 2H), 4.53 (s, 2H), 3.62 (s, 3H). High resolution mass spectrum (mass-to-charge ratio) C₂₉H₂₂N₂O₄S₂Na⁺ [M+Na]⁺ Theoretical value: 549.0919, Measured value: 549.0914.

### Example 321:

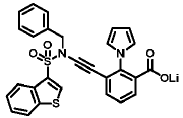

Compound 321 was synthesized according to the General Experimental Operation XII (3.2mg, yield 82%). High resolution mass spectrum (mass-to-charge ratio) C₂₈H₁₉N₂O₄S₂⁻ [M-Li]⁻Theoretical value: 511.0786, Measured value: 511.0791.

### Example 322:

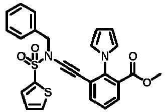

Compound 322 was synthesized according to the General Experimental Operation XII (8mg, yield 16.1%). ¹H NMR (300 MHz, Chloroform-d) δ 7.69-7.57 (m, 2H), 7.49 (dd, *J=* 7.8, 1.6 Hz, 1H), 7.39 (dd, *J=* 3.8, 1.4 Hz, 1H), 7.33 (d, *J=* 7.8 Hz, 1H), 7.30-7.26 (m, 3H), 7.20-7.13 (m, 2H), 7.03 (dd, *J=* 5.0, 3.8 Hz, 1H), 6.67 (t, *J=* 2.2 Hz, 2H), 6.23 (t, *J=* 2.1 Hz, 2H), 4.46 (s, 2H), 3.63 (s, 3H). High resolution mass spectrum (mass-to-charge ratio) C₂₅H₂₀N₂O₄S₂Na⁺ [M+Na]⁺ Theoretical value: 499.0762, Measured value: 499.0757.

### Example 323:

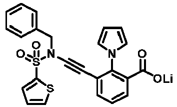

Compound 323 was synthesized according to the General Experimental Operation XII (4mg, yield 82%). High resolution mass spectrum (mass-to-charge ratio) C₂₄H₁₇N₂O₄S₂⁻ [M-Li]⁻Theoretical value: 461.0630, Measured value: 461.0636.

### Example 324:

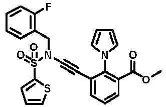

Compound 324 was synthesized according to the General Experimental Operation XII (9mg, yield 17.13%). ¹H NMR (400 MHz, Chloroform-d) δ 7.67-7.58 (m, 2H), 7.51-7.41 (m, 2H), 7.35-7.27 (m, 2H), 7.18 (td, *J=* 7.5, 1.8 Hz, 1H), 7.10-6.98 (m, 3H), 6.66 (t, *J=* 2.2 Hz, 2H), 6.20 (t, *J* = 2.2 Hz, 2H), 4.58 (s, 2H), 3.62 (s, 3H). High resolution mass spectrum (mass-to-charge ratio) C₂₅H₁₉FN₂O₄S₂Na⁺ [M+Na]⁺ Theoretical value: 517.0668, Measured value: 517.0665.

### Example 325:

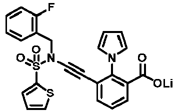

Compound 325 was synthesized according to the General Experimental Operation XII (3mg, yield 77%). High resolution mass spectrum (mass-to-charge ratio) C₂₄H₁₆FN₂O₄S₂⁻ [M-Li]⁻Theoretical value: 479.0536, Measured value: 479.0538.

### Example 326:

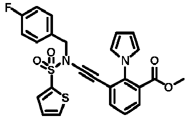

Compound 326 was synthesized according to the General Experimental Operation XII (13mg, yield 23%). ¹H NMR (300 MHz, Chloroform-d) δ 7.71-7.59 (m, 2H), 7.52 (dd, *J* = 7.8, 1.6 Hz, 1H), 7.43 (dd, *J=* 3.8, 1.4 Hz, 1H), 7.34 (t, *J=* 7.8 Hz, 1H), 7.14-7.02 (m, 3H), 7.00-6.90 (m, 2H), 6.68 (t, *J* = 2.2 Hz, 2H), 6.23 (t, *J* = 2.1 Hz, 2H), 4.42 (s, 2H), 3.63 (s, 3H). High resolution mass spectrum (mass-to-charge ratio) C₂₅H₁₉FN₂O₄S₂Na⁺ [M+Na]⁺ Theoretical value: 517.0668, Measured value: 517.0664.

### Example 327:

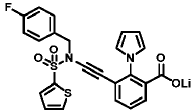

Compound 327 was synthesized according to the General Experimental Operation XII (3.1mg, yield 79%). High resolution mass spectrum (mass-to-charge ratio) C₂₄H₁₆FN₂O₄S₂⁻ [M-Li]⁻Theoretical value: 479.0541, Measured value: 479.0541.

### Example 328:

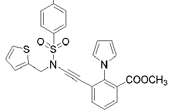

Compound 328 was synthesized according to the General Experimental Operation XII (18 mg, yield28.3%). ¹H NMR (300 MHz, *Chloroform-d*) δ 7.64 (dd, J= 7.7, 1.6 Hz, 1H), 7.54 (*d, J=* 8.3 Hz, 2H), 7.48 (dd, *J*= 7.8, 1.6 Hz, 1H), 7.34 (d, *J*= 7.8 Hz, 1H), 7.23 (dd, *J*= 4.8, 3.2 Hz, 3H), 6.90 (dd, *J* = 6.8, 3.3 Hz, 2H), 6.73 (t, *J* = 2.1 Hz, 2H), 6.27 (t, *J* = 2.1 Hz, 2H), 4.64 (s, 2H), 3.64 (s, 3H), 2.41 (s, 3H). High resolution mass spectrum (mass-to-charge ratio) C₂₆H₂₂N₂O₄S₂Na⁺ [M+Na]⁺ Theoretical value: 513.0913, Measured value: 513.0915.

### Example 329:

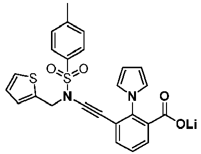

Compound 329 was synthesized according to the General Experimental Operation XII (3.1 mg, yield63.8%). High resolution mass spectrum (mass-to-charge ratio) C₂₅H₁₉N₂O₄S₂⁻[M-Li]⁻ Theoretical value: 475.0792, Measured value: 475.0790.

### Example 330:

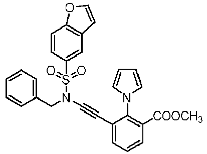

Compound 330 was synthesized according to the General Experimental Operation XII (7.4 mg, yield13.6%). ¹H NMR (300 MHz, *Chloroform-d*) δ7.92 (d, *J* = 1.7 Hz, 1H), 7.75 (d, *J* = 2.2 Hz, 1H), 7.61 (ddd, *J* = 6.2, 3.8, 1.8 Hz, 2H), 7.52 (d, *J* = 8.8 Hz, 1H), 7.39 (dd, *J* = 7.8, 1.7 Hz, 1H), 7.31 (d, *J* = 7.7 Hz, 1H), 7.27 (s, 1H), 7.26-7.21 (m, 2H), 7.17 (dd, *J* = 6.6, 3.1 Hz, 2H), 6.85 (dd, *J=* 2.2, 0.8 Hz, 1H), 6.70 (t, *J=* 2.1 Hz, 2H), 6.24 (t, *J=* 2.1 Hz, 2H), 4.46 (s, 2H), 3.63 (s, 3H). High resolution mass spectrum (mass-to-charge ratio) C₂₉H₂₂N₂O₅SNa⁺ [M+Na]⁺ Theoretical value: 533.1142, Measured value: 533.1143.

### Example 331:

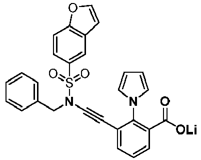

Compound 331 was synthesized according to the General Experimental Operation XII (1.7 mg, yield 58.3%). High resolution mass spectrum (mass-to-charge ratio) C₂₈H₁₉N₂O₅S⁻[M-Li]⁻ Theoretical value: 495.1015, Measured value: 495.1022.

### Example 332:

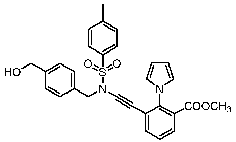

Compound 332 was synthesized according to the General Experimental Operation XII (10.3 mg, yield10.4%). ¹H NMR (300 MHz, *Chloroform-d*) δ 7.65-7.54 (m, 3H), 7.42 (dd, *J* = 7.8, 1.6 Hz, 1H), 7.31 (d, *J* = 7.7 Hz, 1H), 7.25 (dd, *J* = 7.4, 2.7 Hz, 4H), 7.14 (d, *J* = 8.1 Hz, 2H), 6.68 (t, *J* = 2.1 Hz, 2H), 6.25 (t, *J* = 2.1 Hz, 2H), 4.66 (s, 2H), 4.39 (s, 2H), 3.62 (s, 3H), 2.42 (s, 3H). High resolution mass spectrum (mass-to-charge ratio) C₂₉H₂₆N₂O₅SNa⁺ [M+Na]⁺ Theoretical value: 537.1455, Measured value: 537.1456.

### Example 333:

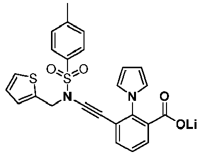

Compound 333 was synthesized according to the General Experimental Operation XII (2.3 mg, yield59%). High resolution mass spectrum (mass-to-charge ratio) C₂₈H₂₃N₂O₅S⁻ [M-H]⁻Theoretical value: 499.1328, Measured value: 499.1332.

### Example 334:

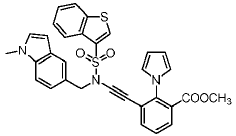

Compound 334 was synthesized according to the General Experimental Operation XII (8.1 mg, yield10.8%). ¹H NMR (400 MHz, *Chloroform-d*) δ 8.29-8.21 (m, 1H), 7.94 (s, 1H), 7.88-7.79 (m, 1H), 7.58 (dd, *J=* 7.7, 1.6 Hz, 1H), 7.46-7.36 (m, 3H), 7.34 (dd, *J=* 7.8, 1.6 Hz, 1H), 7.24 (d, *J* = 7.9 Hz, 1H), 7.16 (d, *J* = 8.4 Hz, 1H), 7.02 (dd, *J* = 6.6, 2.3 Hz, 2H), 6.65 (t, *J* = 2.1 Hz, 2H), 6.40 (d, *J* = 2.7 Hz, 1H), 6.22 (t, *J* = 2.1 Hz, 2H), 4.63 (s, 2H), 3.76 (s, 3H), 3.61 (s, 3H). High resolution mass spectrum (mass-to-charge ratio) C₃₂H₂₅N₃O₄S₂Na⁺ [M+Na]⁺ Theoretical value: 602.1179, Measured value: 602.1179.

### Example 335:

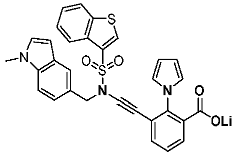

Compound 335 was synthesized according to the General Experimental Operation XII (2.2 mg, yield56.4%). High resolution mass spectrum (mass-to-charge ratio) C₃₁H₂₂N₃O₄S₂⁻[M-Li]⁻ Theoretical value: 564.1052, Measured value: 564.1057.

### Example 336:

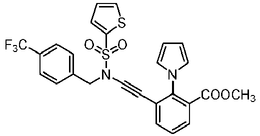

Compound 336 was synthesized according to the General Experimental Operation XII (15 mg, yield 21.2%). ¹H NMR (300 MHz, *Chloroform-d*) δ 7.68 (dd, *J*= 7.6, 1.5 Hz, 1H), 7.63 (dd, *J* = 5.0, 1.3 Hz, 1H), 7.53-7.49 (m, 2H), 7.45 (dd, *J* = 3.8, 1.3 Hz, 1H), 7.37 (dd, *J=* 7.8, 1.7 Hz, 1H), 7.25-7.22 (m, 1H), 7.06 (dd, *J* = 5.0, 3.8 Hz, 1H), 6.82-6.77 (m, 1H), 6.67 (t, *J* = 2.1 Hz, 2H), 6.35-6.30 (m, 1H), 6.21 (t, *J=* 2.1 Hz, 2H), 4.50 (s, 2H), 3.64 (s, 3H). High resolution mass spectrum (mass-to-charge ratio) C₂₆H₁₉F₃N₂O₄S₂Na⁺ [M+Na]⁺ Theoretical value: 567.0631, Measured value: 567.0632.

### Example 337:

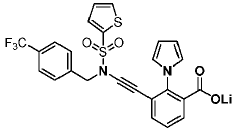

Compound 337 was synthesized according to the General Experimental Operation XII (2.2 mg, yield56.5%). High resolution mass spectrum (mass-to-charge ratio) C₂₅H₁₆F₃N₂O₄S₂⁻[M-Li]⁻ Theoretical value: 529.0504, Measured value: 529.0506.

### Example 338:

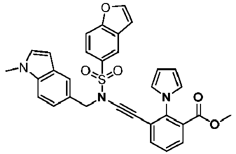

Compound 338 was synthesized according to the General Experimental Operation XII (5.2 mg, yield 11.8%). ¹H NMR (300 MHz, *Chloroform-d*) δ 7.86 (d, *J=* 1.8 Hz, 1H), 7.71 (d, *J* = 2.2 Hz, 1H), 7.64-7.56 (m, 2H), 7.48 (d, *J* = 8.8 Hz, 1H), 7.41 (d, *J* = 1.1 Hz, 1H), 7.36 (dd, *J* = 7.8, 1.7 Hz, 1H), 7.24 (d, *J* = 7.9 Hz, 1H), 7.14 (d, *J=* 8.5 Hz, 1H), 7.03 (t, *J* = 5.4 Hz, 2H), 6.73 (t, *J* = 2.2 Hz, 3H), 6.41 (dd, *J=* 3.1, 0.7 Hz, 1H), 6.26 (t, *J* = 2.1 Hz, 2H), 4.56 (s, 2H), 3.75 (s, 3H), 3.63 (s, 3H). High resolution mass spectrum (mass-to-charge ratio) C₃₂H₂₅N₃O₅SNa⁺ [M+Na]⁺ Theoretical value: 586.1407, Measured value: 586.1408.

### Example 339:

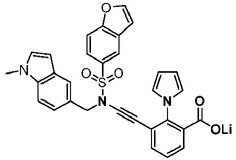

Compound 339 was synthesized according to the General Experimental Operation XII (1.5 mg, yield 70%). High resolution mass spectrum (mass-to-charge ratio) C₃₁H₂₂N₃O₅S⁻ [M-Li]⁻Theoretical value: 548.1280, Measured value: 548.1284.

### Example 340:

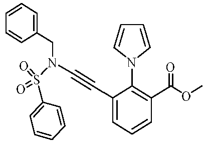

Compound 340 was synthesized according to the General Experimental Operation XII (7.7mg, yield 31.36%). ¹H NMR (500 MHz, Chloroform-*d*) *δ* 7.75-7.74 (m, 1H), 7.72-7.70 (m, 1H), 7.67-7.66 (m, 2H), 7.65-7.63 (m, 1H), 7.60-7.57 (m, 1H), 7.46-7.41 (m, 3H), 7.40-7.37 (m, 1H)7.33-7.30 (m, 1H), 7.16-7.14 (m, 2H), 6.69 (m, 2H), 6.25 (m, 2H), 4.44 (s, 2H), 3.63 (s, 3H). High resolution mass spectrum (mass-to-charge ratio) C₂₇H₂₂N₂O₄SNa⁺ [M+Na]⁺ Theoretical value: 493.1192, Measured value: 493.1195.

### Example 341:

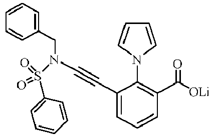

Compound 341 was synthesized according to the General Experimental Operation XII (0.8mg, yield 74.07%). High resolution mass spectrum (mass-to-charge ratio) C₂₆H₁₉LiN₂O₄SNa⁺ [M+Na]⁺ Theoretical value: 485.1118, Measured value: 493.1195.

### Example 342:

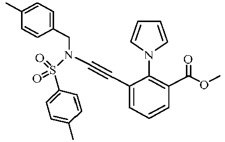

Compound 342 was synthesized according to the General Experimental Operation XII (4.0mg, yield 15.37%). ¹H NMR (300 MHz, Chloroform-*d*) *δ* 7.65-7.62 (m, 1H), 7.59-7.56 (m, 2H), 7.45-7.41 (m, 1H), 7.35-7.32 (m, 1H), 7.27 -7.24 (m, 2H), 7.11-7.04 (m, 4H), 6.71 (t, *J=* 3 Hz, 2H), 6.27 (t, *J* = 3 Hz, 2H), 4.38 (s, 2H), 3.65 (s, 3H), 2.44 (s, 3H), 2.34 (s, 3H) ppm. High resolution mass spectrum (mass-to-charge ratio) C₂₉H₂₆N₂O₄SNa⁺ [M+Na]⁺ Theoretical value: 521.1505, Measured value: 521.1507.

### Example 343:

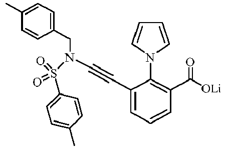

Compound 343 was synthesized according to the General Experimental Operation XII (1mg, yield 50.76%). High resolution mass spectrum (mass-to-charge ratio) C₂₈H₂₄LiN₂O₄S⁺ [M+H]⁺ Theoretical value: 491.1611, Measured value: 491.1614.

### Example 344:

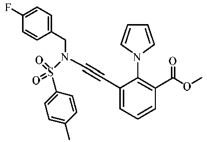

Compound 344 was synthesized according to the General Experimental Operation XII (3.2mg, yield 18.77%). ¹H NMR (500 MHz, Chloroform-*d*) *δ* 7.67-7.65 (m, 1H), 7.56-7.55 (m, 2H), 7.50-7.47 (m, 3H), 7.36-7.33 (m, 1H), 7.24-7.22 (m, 4H), 6.68 (m, *J=* 3 Hz, 2H), 6.22 (m, *J=* 3 Hz, 2H), 4.47 (m, 2H), 3.64 (s, 3H), 2.42 (s, 3H). High resolution mass spectrum (mass-to-charge ratio) C₂₈H₂₃FN₂O₄SNa⁺ [M+Na]⁺ Theoretical value: 525.1255, Measured value: 525.1256.

### Example 345:

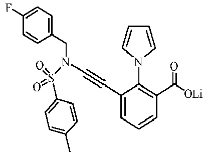

Compound 345 was synthesized according to the General Experimental Operation XII (0.5mg, yield 51.02%). High resolution mass spectrum (mass-to-charge ratio) C₂₇H₁₉FLiN₂O₄S Theoretical value: 493.1215, Measured value: 493.1275.

### Example 346:

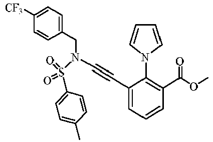

Compound 346 was synthesized according to the General Experimental Operation XII (2.7mg, yield 27.75%). ¹H NMR (500 MHz, Chloroform-*d*) *δ* 7.65-7.64 (m, 1H), 7.58-7.56 (m, 2H), 7.46-7.44 (m, 1H), 7.34-7.31 (m, 1H), 7.26-7.25 (s, 2H), 7.11-7.09 (m, 2H), 6.95-6.92 (m, 2H), 6.69 (m, *J* = 3 Hz, 2H), 6.24 (m, *J=* 3 Hz, 2H), 4.38 (m, 2H), 3.64 (s, 3H), 2.43 (s, 3H). High resolution mass spectrum (mass-to-charge ratio) C₂₉H₂₃F₃N₂O₄SNa⁺ [M+Na]⁺ Theoretical value: 575.1223, Measured value: 575.1230.

### Example 347:

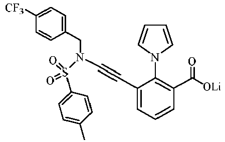

Compound 347 was synthesized according to the General Experimental Operation XII (1.4 mg, yield 79.10%). High resolution mass spectrum: C₂₈H₂₀F₃N₂O₄S⁻ [M-Li]⁻ Calculated value: 537.1101, Measured value: 537.1112.

### Example 348:

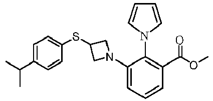

Compound 348 was synthesized according to the General Experimental Operation XIII (10mg, yield 57.46%). ¹H NMR (300 MHz, Chloroform-*d*) *δ* 7.31-7.26 (m, 4H), 7.16-7.13 (m, 2H), 6.69 (t, *J* = 3Hz, 2H), 6.64-6.61 (m, 1H), 6.25 (t, *J* = 3Hz, 2H), 3.95-3.82 (m, 3H), 3.63 (s, 3H), 3.53-3.49 (m, 2H), 2.94-2.84 (m, 1H), 1.26 (s, 3H), 1.23 (s, 3H). High resolution mass spectrum (mass-to-charge ratio) C₂₄H₂₇N₂O₂S⁺ [M+H]⁺ Theoretical value: 407.1788, Measured value: 407.1786.

### Example 349:

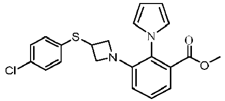

Compound 349 was synthesized according to the General Experimental Operation XIII (5.2mg, yield 32.5%). ¹H NMR (300 MHz, Chloroform-*d*) *δ* 7.30-7.27 (m, 4H), 7.18- 7.14 (m, 2H), ), 6.70 (t, *J* = 3Hz, 2H), 6.65-6.62 (m, 1H), 6.26 (t, *J* = 3Hz, 2H), 3.91-3.83 (m, 2H), 3.67-3.65 (m, 1H), 3.63 (s, 3H), 3.51-3.46 (m, 2H). High resolution mass spectrum (mass-to-charge ratio) C₂₁H₂₀ClN₂O₂S⁺ [M+H]⁺ Theoretical value: 399.0929, Measured value: 399.0929.

### Example 350:

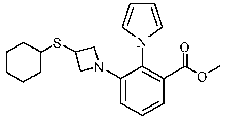

Compound 350 was synthesized according to the General Experimental Operation XIII (3.2mg, yield 34.6%). ¹H NMR (300 MHz, Chloroform-*d*) *δ* 7.30-7.25 (m, 1H), 7.14-7.11 (m, 1H), 6.69 (t, *J =* 3Hz, 2H), 6.64-6.61 (m, 1H), 6.24 (t, *J* = 3Hz, 2H), 3.77-3.72 (m, 2H), 3.68-3.62 (m, 1H), 3.61 (s, 3H), 3.44-3.39 (m, 2H), 2.65-2.56 (m, 1H), 1.87-1.81 (m, 3H), 1.74-1.72 (m, 3H). High resolution mass spectrum (mass-to-charge ratio) C₂₁H₂₁N₂O₂S⁺ [M+H]⁺ Theoretical value: 371.1788, Measured value: 371.1790.

### Example 351:

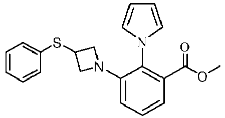

Compound 351 was synthesized according to the General Experimental Operation XIII (11.7mg, yield 74.99%). ¹H NMR (300 MHz, Chloroform-*d*) *δ* ¹H NMR (300 MHz, Chloroform-*d*) *δ* 7.30-7.28 (m, 2H), 7.25 (m, 1H), 7.23-7.18 (m, 3H), 7.15-7.12 (m, 1H), 6.68 (t, *J* = 3Hz, 2H), 6.63-6.60 (m, 1H), 6.24 (t, *J* = 3Hz, 2H), 4.01-3.92 (m, 1H), 3.88-3.83 (m, 2H), 3.61 (s, 3H), 3.53-3.48 (m, 2H). High resolution mass spectrum (mass-to-charge ratio) C₂₁H₂₁N₂O₂S⁺ [M+H]⁺ Theoretical value: 365.1318, Measured value: 365.1322.

### Example 352:

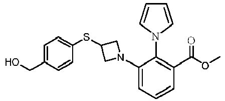

Compound 352 was synthesized according to the General Experimental Operation XIII (7.9mg, yield 60.43%). ¹H NMR (300 MHz, Chloroform-*d*) *δ* .30-7.25 (m, 3H), 7.21-7.19 (m, 2H), 7.15-7.12 (m, 1H), 6.68 (t, *J* = 3Hz, 2H), 6.63-6.60 (m, 1H), 6.24 (t, *J* = 3Hz, 2H), 4.66 (s, 2H), 3.96-3.92 (m, 1H), 3.87-3.82 (m, 2H), 3.61 (s, 3H), 3.51-3.47 (m, 2H). High resolution mass spectrum (mass-to-charge ratio) C₂₂H₂₂N₂NaO₃S⁺ [M+Na]⁺ Theoretical value: 417.1243, Measured value: 417.1243.

### Example 353:

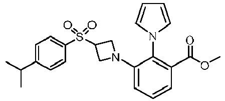

Compound 353 was synthesized according to the General Experimental Operation XIII (0.6mg, yield 26.55%). ¹H NMR (500 MHz, Chloroform-*d*) *δ* 7.76 (d, *J=* 3Hz, 2H), 7.41 (d, *J* = 3Hz, 2H), 7.31-7.29 (m, 1H), 7.19-7.17 (m, 1H), 6.65-6.62 (m, 1H), 6.60 (t, *J* = 3Hz, 2H), 6.22 (t, *J* = 3Hz, 2H), 3.92-3.88 (m, 1H), 3.86-3.83 (m, 2H), 3.65-3,61 (m, 5H),3.03-2.99 (m, 1H), 1.3 (s, 3H), 1.28 (s, 3H). High resolution mass spectrum (mass-to-charge ratio) C₂₄H₂₇N₂O₄S⁺ [M+H]⁺ Theoretical value: 439.1686, Measured value: 439.1692.

### Example 354:

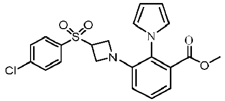

Compound 354 was synthesized according to the General Experimental Operation XIII (1mg, yield 71.4 %). ¹H NMR (500 MHz, Chloroform-*d*) *δ* 7.49 (s, 4H), 7.32-7.29 (m, 1H), 7.20-7.17 (m, 1H), 6.68-6.65(m, 3H), 6.24 (t, *J* = 3Hz, 2H), 3.99-3.94 (m, 1H), 3.62 (s, 3H), 3.58-3.50 (m, 3H), 3.43-3.37 (m, 1H). High resolution mass spectrum (mass-to-charge ratio) C₂₁H₁₉ClN₂O₄SNa⁺ [M+Na]⁺ Theoretical value: 453.0646, Measured value: 453.0648.

### Example 355:

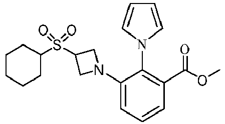

Compound 355 was synthesized according to the General Experimental Operation XIII (1.2mg, yield 47.62%). ¹H NMR (500 MHz, Chloroform-*d*) *δ* 7.32-7.29 (m, 1H), 7.19-7.17 (m, 1H), 6.71 (t, *J* = 3Hz, 3H), 6.25 (t, *J* = 3Hz, 2H), 4.18-4.14 (m, 1H), 3.72-3.52 (m, 7H), 2.41-2.35 (m, 1H), 1.99-1.83 (m, 4H), 1.37-1.26 (m, 6H). High resolution mass spectrum (mass-to-charge ratio) C₂₁H₂₆N₂O₄SNa⁺ [M+Na]⁺ Theoretical value: 425.1505, Measured value: 425.1504.

### Example 356:

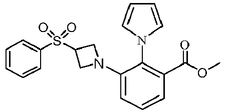

Compound 356 was synthesized according to the General Experimental Operation XIII (0.7mg, yield 33.33%). ¹H NMR (300 MHz, Chloroform-*d*) *δ* 7.53-7.51 (m, 5H), 7.31-7.29 (m, 1H), 7.19 -7.16 (m, 1H), 6.68-6.65 (m, 3H), 6.23 (t, *J* = 3Hz, 2H), 4.03-3.98 (m, 1H), 3.68-3.64 (m, 2H), 3.62 (s, 1H), 3.52-3.49 (m, 1H), 3.41-3.36 (m, 1H). High resolution mass spectrum (mass-to-charge ratio) C₂₁H₂₀N₂O₄SNa⁺ [M+Na]⁺ Theoretical value: 419.1036, Measured value: 419.1038.

### Example 357:

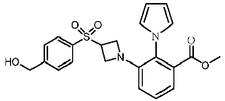

Compound 357 was synthesized according to the General Experimental Operation XIII (2mg, yield 80%). ¹H NMR (300 MHz, Chloroform-*d*) *δ* 7.85 (d, *J* = 9 Hz, 2H), 7.58 (d, *J* = 9 Hz, 2H), 7.29 (m, 1H), 7.19-7.36 (m, 1H), 6.64-6.63 (m, 1H), 6.60 (t, *J* = 3Hz, 2H), 6.23 (t, *J* = 3Hz, 2H), 4.83 (s, 2H), 3.85-3.80 (m, 2H), 3.66-3.61 (m, 3H), 3.50 (s, 3H). High resolution mass spectrum (mass-to-charge ratio) C₂₂H₂₂N₂O₅SNa⁺ [M+Na]⁺ Theoretical value: 449.1142, Measured value: 449.1142.

### Example 358:

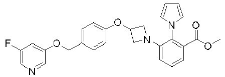

Compound 358 was synthesized according to the General Experimental Operation VI (7.8mg, yield 41%)¹H NMR (500 MHz, *Chloroform-d*) *δ* 8.21 (s, 1H), 8.12 (s, 1H), 7.31 (dd, *J=* 14.3, 8.1 Hz, 3H), 7.16 (d, *J=* 7.6 Hz, 1H), 7.00 (d, *J=* 10.2 Hz, 1H), 6.76-6.66 (m, 5H), 6.29-6.23 (m, 2H), 5.02 (s, 2H), 4.89-4.81 (m, 1H), 3.85-3.78 (m, 2H), 3.70-3.64 (m, 2H), 3.62 (s, 3H). High resolution mass spectrum (mass-to-charge ratio) C₂₇H₂₅FN₃O₄⁺ [M+H]⁺ Theoretical value: 474.1824, Measured value:474.1826.

### Example 359:

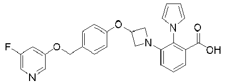

Compound 359 was synthesized according to the General Experimental Operation VI (4mg, yield 80%)¹H NMR (400 MHz, *Chloroform-d*) *δ* 8.20 (s, 1H), 8.11 (s, 1H), 7.38-7.16 (m, 4H), 7.01 (d, *J* = 10.1 Hz, 1H), 6.84-6.62 (m, 5H), 6.35-6.11 (m, 2H), 5.02 (s, 2H), 4.89-4.78 (m, 1H), 3.83-3.71 (m, 2H), 3.71-3.58 (m, 2H). High resolution mass spectrum (mass-to-charge ratio) C₂₆H₂₃FN₃O₄⁺ [M+H]⁺ Theoretical value: 460.1667, Measured value:460.1668.

### Example 360:

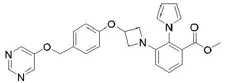

Compound 360 was synthesized according to the General Experimental Operation VI (3mg, yield 17%)¹H NMR (500 MHz, *Chloroform-d*) *δ* 8.86 (s, 1H), 8.45 (s, 2H), 7.32 (t, *J* = 8.3 Hz, 3H), 7.16 (dd, *J* = 7.7, 1.3 Hz, 1H), 6.76-6.66 (m, 5H), 6.26 (t, *J* = 2.1 Hz, 2H), 5.08 (s, 2H), 4.88-4.81 (m, 1H), 3.86-3.78 (m, 2H), 3.69-3.63 (m, 2H), 3.62 (s, 3H). High resolution mass spectrum (mass-to-charge ratio) C₂₆H₂₅N₄O₄⁺ [M+H]⁺ Theoretical value: 457.1870, Measured value: 457.1873.

### Example 361:

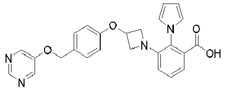

Compound 361 was synthesized according to the General Experimental Operation VI (1.4mg, yield 48%)¹H NMR (400 MHz, *Chloroform-d*) *δ* 8.84 (s, 1H), 8.46 (s, 2H), 7.42-7.15 (m, 4H), 6.86-6.59 (m, 5H), 6.24 (s, 2H), 5.08 (s, 2H), 4.89-4.78 (m, 1H), 3.83-3.72 (m, 2H), 3.69-3.57 (m, 2H). High resolution mass spectrum (mass-to-charge ratio) C₂₅H₂₃N₄O₄⁺ [M+H]⁺Theoretical value: 443.1714, Measured value: 443.1716.

### Example 362:

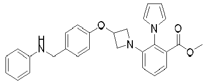

Compound 362 was synthesized according to the General Experimental Operation III (11.3mg, yield 62%)¹H NMR (400 MHz, *Chloroform-d*) *δ* 7.28 (dt, *J=* 8.7, 6.5 Hz, 3H), 7.22-7.09 (m, 3H), 6.82-6.59 (m, 8H), 6.26 (t, *J=* 2.1 Hz, 2H), 4.97-4.71 (m, 1H), 4.25 (s, 2H), 3.80 (dd, *J=* 9.1, 6.2 Hz, 2H), 3.71-3.58 (m, 5H). High resolution mass spectrum (mass-to-charge ratio) C₂₈H₂₈N₃O₃⁺ [M+H]⁺ Theoretical value: 454.2125, Measured value:454.2126.

### Example 363:

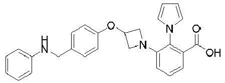

Compound 363 was synthesized according to the General Experimental Operation III (5.8mg, yield 82%)¹H NMR (400 MHz, *Chloroform-d*) *δ* 7.41-7.13 (m, 6H), 6.88-6.57 (m, 8H), 6.27 (t, *J* = 2.1 Hz, 2H), 4.86-4.78 (m, 1H), 4.25 (s, 2H), 3.88-3.70 (m, 2H), 3.65 (dd, *J* = 8.9, 4.9 Hz, 2H). High resolution mass spectrum (mass-to-charge ratio) C₂₇H₂₆N₃O₃⁺ [M+H]⁺ Theoretical value: 440.1969, Measured value:440.1970.

### Example 364:

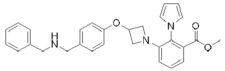

Compound 364 was synthesized according to the General Experimental Operation III (12mg, yield 64%)¹H NMR (400 MHz, *Chloroform-d*) *δ* 7.39-7.21 (m, 8H), 7.15 (dd, *J=* 7.7, 1.4 Hz, 1H), 6.77-6.62 (m, 5H), 6.26 (t, *J* = 2.1 Hz, 2H), 4.88-4.78 (m, 1H), 3.84-3.76 (m, 4H), 3.74 (s, 2H), 3.66 (dd, *J* = 9.1, 4.9 Hz, 2H), 3.62 (s, 3H). High resolution mass spectrum (mass-to-charge ratio) C₂₉H₃₀N₃O₃⁺[M+H]⁺Theoretical value: 468.2282, Measured value:468.2284.

### Example 365:

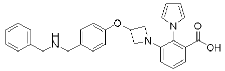

Compound 365 was synthesized according to the General Experimental Operation III (1.5mg, yield 35%)¹H NMR (400 MHz, *Methanol-d4*) *δ* 7.52-7.32 (m, 7H), 7.22 (t, *J* = 7.8 Hz, 1H), 6.90-6.70 (m, 5H), 6.56 (d, *J* = 8.2 Hz, 1H), 6.10 (t, 2H), 5.22-5.16 (m, 1H), 4.22-4.05 (m, 4H), 3.83-3.71 (m, 2H), 3.52-3.41 (m, 2H). High resolution mass spectrum (mass-to-charge ratio) C₂₈H₂₈N₃O₃⁺ [M+H]⁺Theoretical value: 454.2125, Measured value:454.2128.

### Example 366:

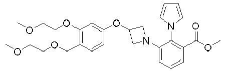

Compound 366 was synthesized according to the General Experimental Operation VIII (23mg, yield 33%)¹H NMR (400 MHz, *Chloroform-d*) *δ* 7.34-7.20 (m, 2H), 7.15 (dd, *J=* 7.6, 1.3 Hz, 1H), 6.77-6.63 (m, 3H), 6.30 (d, *J* = 2.3 Hz, 1H), 6.25 (t, *J* = 2.1 Hz, 2H), 6.19 (dd, *J* = 8.3, 2.3 Hz, 1H), 4.87-4.76 (m, 1H), 4.54 (s, 2H), 4.08-4.01 (m, 2H), 3.84-3.70 (m, 4H), 3.70-3.50 (m, 6H), 3.44 (s, 3H), 3.40 (s, 3H). High resolution mass spectrum (mass-to-charge ratio) C₂₈H₃₄N₂NaO₇⁺ [M+Na]⁺Theoretical value: 533.2258, Measured value: 533.2255.

### Example 367:

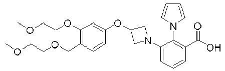

Compound 367 was synthesized according to the General Experimental Operation VIII (7.8mg, yield 66%)¹H NMR (400 MHz, *Chloroform-d*) *δ* 7.35-7.17 (m, 3H), 6.82-6.61 (m, 3H), 6.36-6.14 (m, 4H), 4.88-4.75 (m, 1H), 4.54 (s, 2H), 4.10-4.00 (m, 2H), 3.83-3.69 (m, 4H), 3.69-3.51 (m, 6H), 3.44 (s, 3H), 3.39 (s, 3H). High resolution mass spectrum (mass-to-charge ratio) C₂₇H₃₂N₂NaO₇⁺ [M+Na]⁺ Theoretical value: 519.2102, Measured value:519.2104.

### Example 368:

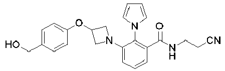

The compound 38 (1 eq.) which was a starting material was dissolved together with 3-aminopropylnitrile (2.5 eq.) in dichloromethane. 2-chloro-1-picoline iodide (1.5 eq.) and triethylamine (3 eq.) were added under cooling, and stirred overnight at room temperature. After the reaction was quenched with water, the reaction solution was diluted with a large amount of ethyl acetate, and washed successively with a saturated solution of ammonium chloride and a saturated aqueous solution of sodium bicarbonate, and the resulting organic phase was dried with anhydrous sodium sulfate and then was subjected to rotary evaporation to dryness. The mixture was separated by preparative thin layer chromatography to obtain compound 368 (2.3mg, yield 50%)¹H NMR (400 MHz, *Chloroform-d*) *δ* 7.37-7.30 (m, 1H), 7.30-7.27 (m, 2H), 7.19 (dd, *J=* 7.7, 1.3 Hz, 1H), 6.78 (t, *J=* 2.1 Hz, 2H), 6.73-6.62 (m, 3H), 6.36 (t, *J* = 2.1 Hz, 2H), 5.50-5.37 (m, 1H), 4.89-4.80 (m, 1H), 4.62 (s, 2H), 3.87-3.78 (m, 2H), 3.66 (dd, *J* = 9.1, 4.8 Hz, 2H), 3.36 (dd, *J* = 13.0, 6.6 Hz, 2H), 2.38 (t, *J* = 6.7 Hz, 2H). High resolution mass spectrum (mass-to-charge ratio) C₂₄H₂₃N₄O₃⁻ [M-H]⁻ Theoretical value: 415.1776, Measured value: 415.1774.

### Example 369:

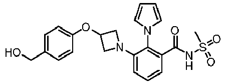

Compound 369 was synthesized according to Example 368 (5mg, yield 70%)¹H NMR (500 MHz, *Chloroform-d*) *δ* 7.38 (t, *J* = 8.0 Hz, 1H), 7.33-7.23 (m, 2H), 7.20 (s, 1H), 6.82 (t, *J* = 2.1 Hz, 2H), 6.74 (dd, *J =* 8.2, 1.3 Hz, 1H), 6.72-6.62 (m, 2H), 6.48 (t, *J =* 2.1 Hz, 2H), 4.92-4.82 (m, 1H), 4.62 (s, 2H), 3.87 (dd, *J* = 9.1, 6.3 Hz, 2H), 3.69 (dd, *J* = 9.1, 4.7 Hz, 2H), 3.14 (s, 3H). High resolution mass spectrum (mass-to-charge ratio) C₂₂H₂₃N₃NaO₅S⁺ [M+Na]⁺Theoretical value: 464.1251, Measured value:464.1252.

### Example 370:

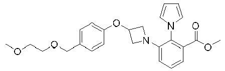

Compound 370 was synthesized according to the General Experimental Operation VIII (11 mg, yield 65%). ¹H NMR (300 MHz, *Chloroform-d*) δ 7.31 (d, *J* = 7.8 Hz, 1H), 7.25 (d, *J* = 8.6 Hz, 2H), 7.15 (dd, *J=* 7.6, 1.2 Hz, 1H), 6.79-6.57 (m, 5H), 6.25 (t, *J=* 2.1 Hz, 2H), 4.83 (t, *J=* 5.4 Hz, 1H), 4.49 (s, 2H), 3.84-3.76 (m, 2H), 3.68-3.55 (m, 9H), 3.41-3.36 (m, 3H). High resolution mass spectrum (mass-to-charge ratio) C₂₅H₂₈N₂NaO₅⁺ [M+Na]⁺ Theoretical value: 459.1890, Measured value: 459.1889.

### Example 371:

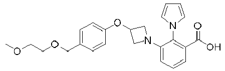

Compound 371 was synthesized according to the General Experimental Operation VIII (1.9mg, yield 49%). ¹H NMR (300 MHz, *Chloroform-d*) δ 7.37-7.28 (m, 2H), 7.24 (s, 2H), 6.79-6.55 (m, 5H), 6.28 (s, 2H), 4.95-4.73 (m, 1H), 4.49 (s, 2H), 3.84-3.72 (m, 2H), 3.71-3.49 (m, 6H), 3.39 (s, 3H). High resolution mass spectrum (mass-to-charge ratio) C₂₄H₂₆N₂NaO₅⁺ [M+Na]⁺ Theoretical value: 445.1734, Measured value: 445.1735.

### Example 372:

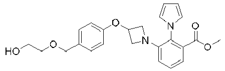

Compound 372 was synthesized according to the General Experimental Operation VIII (6mg, yield 52%). ¹H NMR (300 MHz, *Chloroform-d*) δ 7.31 (d, *J=* 7.9 Hz, 1H), 7.23 (s, 2H), 7.16 (d, *J* = 7.2 Hz, 1H), 6.69 (dd, *J* = 8.3, 5.1 Hz, 5H), 6.26 (s, 2H), 4.95-4.73 (m, 1H), 4.48 (s, 2H), 3.80 (dd, *J* = 14.2, 7.4 Hz, 4H), 3.70-3.52 (m, 7H). High resolution mass spectrum (mass-to-charge ratio) C₂₄H₂₆N₂NaO₅⁺ [M+Na]⁺ Theoretical value: 445.1734, Measured value: 445.1734.

### Example 373:

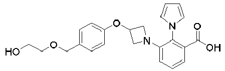

Compound 373 was synthesized according to the General Experimental Operation VIII (0.5mg, yield 25%). ¹H NMR (300 MHz, *Chloroform-d*) δ 7.30 (s, 1H), 7.23 (s, 2H), 7.17 (s, 1H), 6.69 (dd, *J* = 8.3, 5.1 Hz, 5H), 6.26 (s, 2H), 4.84 (s, 1H), 4.48 (s, 2H), 3.80 (dd, *J* = 14.2, 7.4 Hz, 3H), 3.70-3.47 (m, 6H). High resolution mass spectrum (mass-to-charge ratio) C₂₃H₂₄N₂NaO₅⁺ [M+Na]⁺Theoretical value: 431.1577, Measured value: 431.1579.

### Example 374:

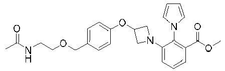

Compound 374 was synthesized according to the General Experimental Operation VIII (4mg, yield 59.7%). ¹H NMR (300 MHz, *Chloroform-d*) δ 7.30 (s, 1H), 7.23 (d, *J* = 8.5 Hz, 3H), 6.78-6.58 (m, 5H), 6.31-6.18 (m, 2H), 5.83 (brs, 1H), 4.92-4.72 (m, 1H), 4.43 (s, 2H), 3.85-3.77 (s, 2H), 3.70-3.59 (m, 5H), 3.56-3.50 (m, 2H), 3.50-3.42 (m, 2H), 1.98 (s, 3H). High resolution mass spectrum (mass-to-charge ratio) C₂₆H₂₉N₃NaO₅⁺ [M+Na]⁺Theoretical value: 486.1999, Measured value: 486.2000.

### Example 375:

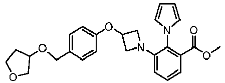

Compound 375 was synthesized according to the General Experimental Operation VIII (20mg, yield 62%). ¹H NMR (300 MHz, Chloroform-*d*) δ 7.40-7.19 (m, 3H), 7.15 (dd, *J=* 7.6, 1.4 Hz, 1H), 6.77-6.57 (m, 5H), 6.25 (t, *J* = 2.1 Hz, 2H), 4.83 (tt, *J* = 6.2, 4.8 Hz, 1H), 4.49-4.36 (m, 2H), 4.19 (tt, *J* = 5.4, 2.9 Hz, 1H), 3.99-3.72 (m, 6H), 3.69-3.43 (m, 5H), 2.11-1.91 (m, 2H). High resolution mass spectrum (mass-to-charge ratio) C₂₆H₂₈N₂NaO₅⁺ [M+Na]⁺ Theoretical value: 471.1890, Measured value: 471.1891

### Example 376:

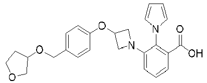

Compound 376 was synthesized according to the General Experimental Operation VIII (8.9mg, yield 91.8%). ¹H NMR (300 MHz, Chloroform-*d*) δ 7.43-7.10 (m, 4H), 6.68 (dd, *J* = 10.9, 8.3 Hz, 5H), 6.24 (s, 2H), 4.82 (t, *J* = 5.5 Hz, 1H), 4.42 (d, *J* = 2.3 Hz, 2H), 4.19 (tt, *J* = 5.3, 2.8 Hz, 1H), 3.99-3.71 (m, 6H), 3.63 (dd, *J* = 8.7, 4.8 Hz, 2H), 2.11-1.92 (m, 2H). High resolution mass spectrum (mass-to-charge ratio) C₂₅H₂₆N₂NaO₅⁺ [M+Na]⁺ Theoretical value: 457.173 4, Measured value: 457.1737.

### Example 377:

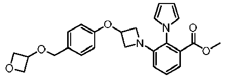

Compound 377 was synthesized according to the General Experimental Operation VIII, (20mg, yield 35%). ¹H NMR (300 MHz, Chloroform-*d*) δ 7.39-7.23 (m, 4H), 7.14 (ddd, *J* = 7.6, 6.2, 1.4 Hz, 1H), 6.77-6.56 (m, 4H), 6.36-5.98 (m, 2H), 4.92-4.76 (m, 1H), 4.69-4.46 (m, 2H), 3.86-3.54 (m, 12H).

### Example 378:

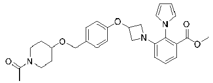

Compound 378 was synthesized according to the General Experimental Operation VIII (10mg, yield 30%). ¹H NMR (300 MHz, Chloroform-*d*) δ 7.41-7.20 (m, 4H), 7.15 (dd, *J=* 7.7, 1.4 Hz, 1H), 6.84-6.67 (m, 4H), 6.25 (t, *J* = 2.1 Hz, 2H), 4.83 (tt, *J* = 6.2, 4.8 Hz, 1H), 4.56-4.39 (m, 2H), 3.93 (ddd, *J* = 11.9, 7.0, 4.0 Hz, 1H), 3.86-3.73 (m, 2H), 3.72-3.52 (m, 7H), 3.41-3.16 (m, 2H), 2.09 (s, 3H), 1.94-1.52 (m, 4H). High resolution mass spectrum (mass-to-charge ratio) C₂₉H₃₃N₃NaO₅⁺ [M+Na]⁺ Theoretical value: 526.2312, Measured value: 526.2314.

### Example 379:

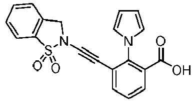

Compound 379 was synthesized according to the General Experimental Operation XII (1.2mg, yield 41%). Liquid chromatography-mass spectrometry (mass-to-charge ratio): C₂₀H₁₅N₂O₄S⁺ [M+H]⁺ Theoretical value: 379.1, Measured value: 378.9.

### Example 380:

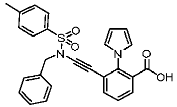

Compound 380 was synthesized according to the General Experimental Operation XII (2.0mg, yield 51%). Liquid chromatography-mass spectrometry (mass-to-charge ratio): C₂₇H₂₃N₂O₄S⁺ [M+H]⁺ Theoretical value: 471.1, Measured value: 471.0.

### Example 381:

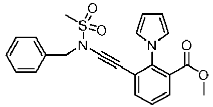

Compound 380 was synthesized according to the General Experimental Operation XII (21.5mg, yield 52.7%). ¹H NMR (300 MHz, Chloroform-*d*) δ 7.71 (dd, *J* = 7.8, 1.6 Hz, 1H), 7.55 (dd, *J*= 7.8, 1.6 Hz, 1H), 7.40-7.32 (m, 4H), 7.32-7.23 (m, 2H), 6.76 (t, *J=* 2.1 Hz, 2H), 6.25 (t, *J=* 2.1 Hz, 2H), 4.56 (s, 2H), 3.65 (s, 3H), 2.66 (s, 3H).

### Example 382:

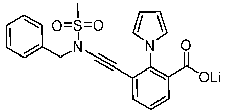

Compound 380 was synthesized according to the General Experimental Operation XII (1.5mg, yield 32%). Liquid chromatography-mass spectrometry (mass-to-charge ratio): C₂₁H₁₉N₂O₄S⁺ [M+H]⁺ Theoretical value: 395. 1,Measured value: 395.0.

### Example 383:

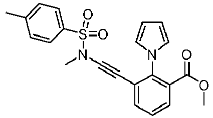

Compound 380 was synthesized according to the General Experimental Operation XII (25.0mg, yield 61.3%). ¹H NMR (300 MHz, Chloroform-*d*) δ 7.66 (dd, *J* = 7.8, 1.6 Hz, 1H), 7.65-7.61 (m, 2H), 7.59 (dd, *J=* 7.8, 1.6 Hz, 1H), 7.36 (t, *J=* 7.8 Hz, 1H), 7.31 (d, *J=* 8.1 Hz, 2H), 6.73 (t, *J=* 2.1 Hz, 2H), 6.25 (t, *J=* 2.1 Hz, 2H), 3.64 (s, 3H), 2.96 (s, 3H), 2.44 (s, 3H).

### Example 384:

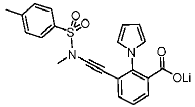

Compound 380 was synthesized according to the General Experimental Operation XII (2.8mg, yield 35%). ¹H NMR (300 MHz, Methanol-*d*₄) δ 7.62 (d, *J*= 8.2 Hz, 2H), 7.36-7.29 (m, 3H), 7.24 (d, *J* = 7.2 Hz, 1H), 7.02 (t, *J* = 7.6 Hz, 1H), 6.67 (s, 2H), 5.98 (s, 2H), 2.92 (s, 3H), 2.40 (s, 3H).

### Example 385:

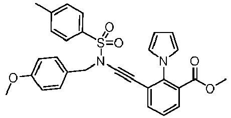

Compound 380 was synthesized according to the General Experimental Operation XII (19.0 mg, yield 37%). ¹H NMR (300 MHz, Chloroform-*d*) δ 7.62 (dd, *J* = 7.7, 1.6 Hz, 1H), 7.55 (d, *J* = 8.3 Hz, 2H), 7.42 (dd, *J* = 7.8, 1.6 Hz, 1H), 7.30 (t, *J* = 7.7 Hz, 1H), 7.24 (d, *J* = 8.1 Hz, 2H), 7.07 (d, *J* = 8.7 Hz, 2H), 6.78 (d, *J* = 8.7 Hz, 2H), 6.70 (t, *J* = 2.1 Hz, 2H), 6.26 (t, *J* = 2.1 Hz, 2H), 4.35 (s, 2H), 3.79 (s, 3H), 3.63 (s, 3H), 2.42 (s, 3H).

### Example 386:

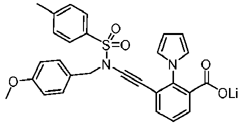

Compound 380 was synthesized according to the General Experimental Operation XII (1.3mg, yield 23%). Liquid chromatography-mass spectrometry (mass-to-charge ratio): C₂₈H₂₅N₂O₅S⁺ [M+H]⁺ Theoretical value: 501.1, Measured value: 501.0.

### Example 387:

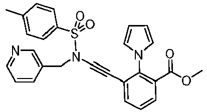

Compound 387 was synthesized according to the General Experimental Operation XII (31.0mg, yield 64%). ¹H NMR (300 MHz, Chloroform-*d*) δ 8.53 (dd, *J =* 4.8, 1.6 Hz, 1H), 8.38 (d, *J* = 1.9 Hz, 1H), 7.66 (dd, *J* = 7.7, 1.6 Hz, 1H), 7.59 (d, *J* = 8.3 Hz, 2H), 7.50-7.43 (m, 2H), 7.33 (t, *J=* 7.8 Hz, 1H), 7.27 (d, *J=* 8.1 Hz, 2H), 7.20 (dd, *J=* 7.8, 4.8 Hz, 1H), 6.68 (t, *J=* 2.1 Hz, 2H), 6.24 (t, *J=* 2.1 Hz, 2H), 4.42 (s, 2H), 3.63 (s, 3H), 2.43 (s, 3H).

### Example 388:

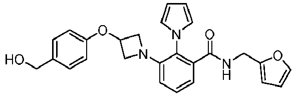

Compound 397 was synthesized according to Example 368 (7.2 mg, yield 90%). ¹H NMR (300 MHz, Chloroform-*d*) δ 7.43-7.11 (m, 5H), 6.80-6.56 (m, 5H), 6.34-6.17 (m, 3H), 6.17-6.00 (m, 1H), 5.50-5.32 (m, 1H), 4.92-4.74 (m, 1H), 4.61 (s, 2H), 4.28 (d, *J* = 5.4 Hz, 2H), 3.89-3.70 (m, 2H), 3.70-3.55 (m, 2H). High resolution mass spectrum (mass-to-charge ratio) C₂₆H₂₅N₃NaO₄⁺ [M+Na]⁺ Theoretical value: 466.1737, Measured value:466.1739.

### Example 389:

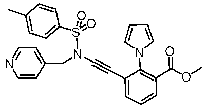

Compound 389 was synthesized according to the General Experimental Operation XII (27.0mg, yield 55.7%). ¹H NMR (300 MHz, Chloroform-*d*) δ 8.50 (dd, *J* = 4.5, 1.6 Hz, 2H), 7.67 (dd, *J* = 7.8, 1.6 Hz, 1H), 7.60 (d, *J* = 8.4 Hz, 2H), 7.48 (dd, *J* = 7.8, 1.6 Hz, 1H), 7.34 (t, *J* = 7.8 Hz, 1H), 7.28 (d, *J* = 7.4 Hz, 2H), 7.05 (d, *J* = 6.0 Hz, 2H), 6.65 (t, *J* = 2.1 Hz, 2H), 6.19 (t, *J=* 2.1 Hz, 2H), 4.42 (s, 2H), 3.63 (s, 3H), 2.44 (s, 3H).

### Example 390:

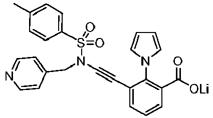

Compound 390 was synthesized according to the General Experimental Operation XII (5mg, yield 49.3%). ¹H NMR (300 MHz, Methanol-*d4*) δ 8.44 (d, *J* = 6.1 Hz, 2H), 7.69 (d, *J* = 8.3 Hz, 2H), 7.55 (d, *J* = 8.0 Hz, 1H), 7.40 (d, *J* = 8.9 Hz, 2H), 7.31 (s, 2H), 7.20 (s, 2H), 6.76 (t, *J=* 2.1 Hz, 3H), 6.06 (t, *J=* 2.1 Hz, 2H), 4.52 (s, 2H), 2.47 (s, 3H).

### Example 391:

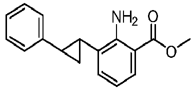

Compound 391 was synthesized according to the General Experimental Operation XIII (2.0mg, yield 51%), cyclopropanation was accomplished using G108 followed by reduction to obtain an amino group. Liquid chromatography-mass spectrometry (mass-to-charge ratio): C₁₇H₁₈NO₂⁺ [M+H]⁺ Theoretical value: 268.1, Measured value: 268.0.

### Example 392:

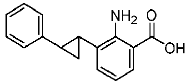

Compound 392 was synthesized according to the General Experimental Operation XIII (0.8mg, yield 31%), cyclopropanation was accomplished using G108 followed by reduction to obtain an amino group. Liquid chromatography-mass spectrometry (mass-to-charge ratio): C₁₆H₁₆NO₂⁺ [M+H]⁺ Theoretical value: 254.1, Measured value: 254.1.

### Example 393:

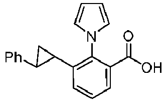

Compound 393 was synthesized according to the General Experimental Operation XIII (0.5 mg, yield 53%). Liquid chromatography-mass spectrometry (mass-to-charge ratio): C₂₀H₁₈NO₂⁺ [M+H]⁺ Theoretical value: 304.1, Measured value: 304.0.

### Example 394:

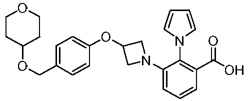

Compound 394 was synthesized according to the General Experimental Operation VIII (2.5mg, yield 73%). ¹H NMR (500 MHz, Chloroform-*d*) δ 7.35-7.21 (m, 4H), 6.76-6.70 (m, 3H), 6.67 (d, *J* = 8.6 Hz, 2H), 6.28 (t, *J* = 2.1 Hz, 2H), 4.87-4.78 (m, 1H), 4.48 (s, 2H), 4.00-3.91 (m, 2H), 3.83-3.75 (m, 2H), 3.69-3.63 (m, 2H), 3.61-3.55 (m, 1H), 3.48-3.40 (m, 2H), 1.97-1.88 (m, 2H), 1.70-1.60 (m, 2H).

### Example 395:

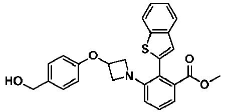

Compound 395 was synthesized according to the General Experimental Operation IV (7.5mg, yield 86%). ¹H NMR (300 MHz, Chloroform-*d*) δ7.89-7.80 (m, 1H), 7.81-7.74 (m, 1H), 7.42-7.28 (m, 3H), 7.26-7.13 (m, 4H), 6.70 (dd, *J =* 8.3, 1.0 Hz, 1H), 6.66-6.61 (m, 2H), 4.86-4.73 (m, 1H), 4.59 (s, 2H), 3.98-3.88 (m, 2H), 3.72 (dd, *J* = 9.0, 4.8 Hz, 2H), 3.57 (s, 3H). High resolution mass spectrum (mass-to-charge ratio) C₂₆H₂₄NO₄S⁺ [M+H]⁺ Theoretical value: 446.1421, Measured value: 446.1421.

### Example 396:

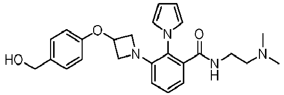

Compound 396 was synthesized according to Example 368 (6.3mg, yield 39%). ¹H NMR (300 MHz, Chloroform-*d*) δ 7.37-7.17 (m, 3H), 7.10-6.95 (m, 1H), 6.82 (t, *J=* 2.1 Hz, 2H), 6.72-6.57 (m, 3H), 6.54 (s, 1H), 6.25 (t, *J =* 2.1 Hz, 2H), 4.91-4.77 (m, 1H), 4.61 (s, 2H), 3.88-3.70 (m, 2H), 3.70-3.56 (m, 2H), 3.46-3.29 (m, 2H), 2.57-2.48 (m, 2H), 2.43 (s, 6H). High resolution mass spectrum (mass-to-charge ratio) C₂₅H₃₁N₄O₃⁺ [M+H]⁺ Theoretical value: 435.2391, Measured value:435.2390.

### Example 397: Determination of Single Concentration (50µM) Inhibition Rate of C666-1 Cell Viability

In order to assess the effect of the EBNA1 inhibitor at the cellular level, the cell proliferation inhibition rate was tested, and the inhibition rate of inhibitor at a specific concentration on the proliferation of Epstein-Barr virus positive cell line C666-1 was detected.

In the test, 100 µL C666-1 cells were inoculated in a transparent 96-well plate, and each well contained 5×10³ cells. After the cells were cultured in an incubator with 5% carbon dioxide at 37°C for 24 hours, 10 µL compound at a concentration of 500 µmol/L was added into the cells, so that the final concentration of the compound was 50 µmol/L. Two replicate wells were set for each compound, the wells treated with DMSO were set as control wells (Ctrl), and the wells with cell culture medium only were set as blank wells (blank), and they were treated in the incubator with carbon dioxide at 37 °C for 72 hours. The cell viability was assessed using a redox indicator Resazurin. Specifically, 10 µL Resazurin at a concentration of 600 umol/L was added into each well, and incubated at 37°C for 3 hours, and a Tecan microplate reader was used to detect the fluorescence signal at an exiciation wavelength of 560 nm and an emission wavelength of 590 nm. The formula inh% = 1-(F-F_{blank})/(F_{Ctrl}-F_{blank})×100% (where, F is the fluorescence intensity of the compound-added wells, F_{Ctrl} and F_{blank} are the readings of the control wells and blank wells, respectively) was used to calculate the inhibition rate of the compound on the proliferation of C666-1 cells, so as to assess the activity of the compound on Epstein-Barr virus positive cells.

### Example 398: Determination of C666-1 Cell Viability

In order to assess the effect of the EBNA1 inhibitor at the cellular level, the cytotoxicity assay was performed. The EBNA1 inhibitor selectively killed EBV-positive cell lines (C666-1 cells), but had no toxicity to EBV-negative cell lines (HONE1 cells and HK1 cells).

In the test, 100 µL different cell lines were inoculated in a transparent 96-well plate, each well containing 5×10³ C666-1 cells. After the cells were cultured in an incubator with 5% carbon dioxide at 37°C for 24 hours, 10 µL compound at a concentration range from 1 mmol/L to 7.8 µmol/L was added into each well (8 points, 2-fold dilution, the final concentration was 100-0.78 µmol/L), and treated in the incubator with carbon dioxide at 37°C for 72 hours. The cell viability was assessed using a redox indicator Resazurin. Specifically, 10 µL Resazurin at a concentration of 600 umol/L was added into each well, and incubated at 37°C for 3 hours, and a Tecan microplate reader was used to detect the fluorescence signal at an excitation wavelength of 560 nm and an emission wavelength of 590 nm. A software was used to fit the inhibition curve and calculate the concentration for 50% of maximal effect (EC₅₀). The selectivity of compound activity was assessed by comparing the EC₅₀ of EBV-positive and EBV-negative cell lines.

**Table-I: C666-1 cell viability single concentration (50µM) inhibition rate**

| **Example Number** | **Structural Formula** | **Compound Name** | **Inhibition Ratio^{∗}** |
|---|---|---|---|
| **1** | 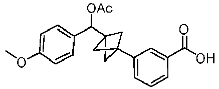 | 3-(3-(acetoxyl(4-methoxyphenyl)m ethyl)bicyclo[1.1.1]pentan-1-yl)ben zoic acid | + |
| **2** | 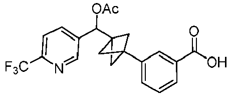 | 3-(3-(acetoxyl(6-(trifluoromethyl)p yridin-3-yl)methyl)bicyclo[1.1.1]pe ntan-1-yl)benzoic acid | + |
| **3** | 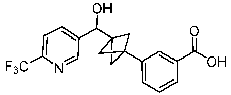 | 3-(3-(hydroxyl(6-(trifluoromethyl)p yridin-3-yl)methyl)bicyclo[1.1.1]pe ntan-1-yl)benzoic acid | + |
| **4** | 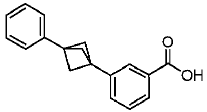 | 3-(3-phenylbicyclo[1.1.1]pentan-1-yl)benzoic acid | + |
| **5** | 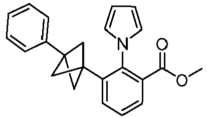 | Methyl 3-(3-phenylbicyclo[1.1.1]pentan-1-yl)-2-(1H-pyrrol-1-yl)benzoate | + |
| **6** | 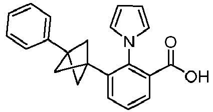 | 3-(3-phenylbicyclo[1.1.1]pentan-1-yl)-2-(1H-pyrrol-1-yl)benzoic acid | + |
| **7** | 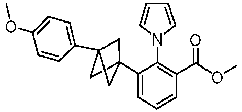 | Methyl 3-(3-(4-methoxyphenyl)bicyclo[1.1 .1]pentan-1-yl)-2-(1H-pyrrol-1-yl)b enzoate | + |
| **8** | 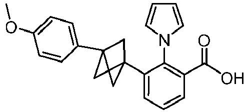 | 3-(3-(4-methoxyphenyl)bicyclo[1.1 .1]pentan-1-yl)-2-(1H-pyrrol-1-yl)b enzoic acid | + |
| **9** | 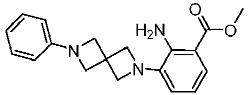 | Methyl 2-amino-3-(6-phenyl-2,6-diazaspiro [3.3]heptan-2-yl)benzoate | ++ |
| **10** | 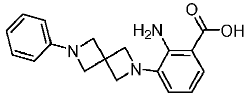 | 2-amino-3-(6-phenyl-2,6-diazaspiro [3.3]heptan-2-yl)benzoic acid | + |
| **11** | 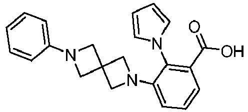 | 3-(6-phenyl-2,6-diazaspiro[3.3]hept an-2-yl)-2-(1H-pyrrol-1-yl)benzoic acid | + |
| **12** | 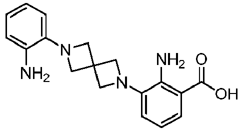 | 2-amino-3-(6-(2-aminophenyl)-2,6-diazaspiro[3.3]heptan-2-yl)benzoic acid | + |
| **13** | 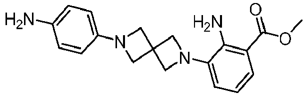 | Methyl 2-amino-3-(6-(4-aminophenyl)-2,6-diazaspiro[3.3]heptan-2-yl)benzoat e | ++++ |
| **14** | 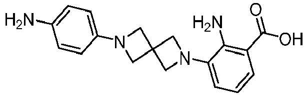 | 2-amino-3-(6-(4-aminophenyl)-2,6-diazaspiro[3.3]heptan-2-yl)benzoic acid | + |
| **15** | 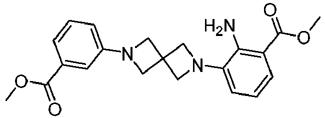 | Methyl 2-amino-3-(6-(3-(methoxycarbonyl )phenyl)-2,6-diazaspiro [3.3]heptan-2-yl)benzoate | + |
| **16** | 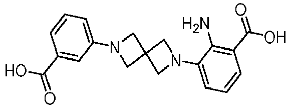 | 2-amino-3-(6-(3-carboxylphenyl)-2 ,6-diazaspiro[3.3]heptan-2-yl)benz oic acid | + |
| **17** | 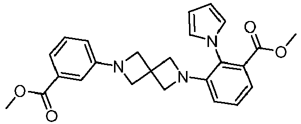 | Methyl 3-(6-(3-(methoxycarbonyl)phenyl)-2,6-diazaspiro[3.3]heptan-2-yl)-2-( 1H-pyrrol-1-yl)benzoate | ++ |
| **18** | 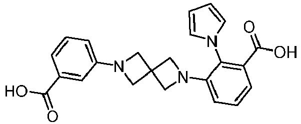 | 3-(6-(3-carboxylphenyl)-2,6-diazas piro[3.3]heptan-2-yl)-2-(1H-pyrrol-1-yl)benzoic acid | + |
| **19** | 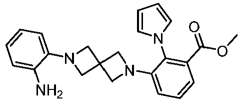 | Methyl 3-(6-(2-aminophenyl)-2,6-diazaspir o[3.3]heptan-2-yl)-2-(1H-pyrrol-1-yl)benzoate | ++++ |
| **20** | 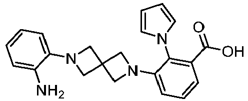 | 3-(6-(2-aminophenyl)-2,6-diazaspir o[3.3]heptan-2-yl)-2-(1H-pyrrol-1-yl)benzoic acid | + |
| **21** | 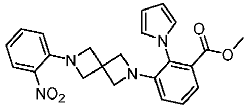 | Methyl 3-(6-(2-nitrophenyl)-2,6-diazaspiro [3.3]heptan-2-yl)-2-(1H-pyrrol-1-yl )benzoate | ++ |
| **22** | 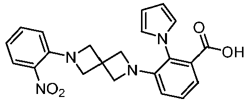 | 3-(6-(2-nitrophenyl)-2,6-diazaspiro [3.3]heptan-2-yl)-2-(1H-pyrrol-1-yl )benzoic acid | + |
| **23** | 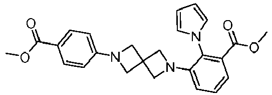 | Methyl 3-(6-(4-(methoxycarbonyl)phenyl)-2,6-diazaspiro[3.3]heptan-2-yl)-2-( 1H-pyrrol-1-yl)benzoate | + |
| **24** | 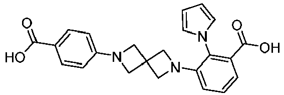 | 3-(6-(4-carboxylphenyl)-2,6-diazas piro[3.3]heptan-2-yl)-2-(1H-pyrrol-1-yl)benzoic acid | + |
| **25** | 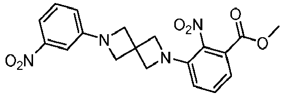 | Methyl 3-(6-(3-nitrophenyl)-2,6-diazaspiro [3.3]heptan-2-yl)-2-nitrobenzoate | ++ |
| **26** | 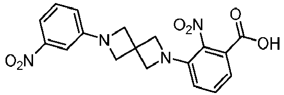 | 3-(6-(3-nitrophenyl)-2,6-diazaspiro [3.3]heptan-2-yl)-2-nitrobenzoic acid | + |
| **27** | 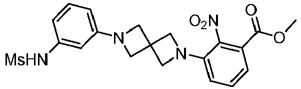 | Methyl 3-(6-(3-(methylsulfonamido)phenyl )-2,6-diazaspiro[3.3]heptan-2-yl)-2-nitrobenzoate | + |
| **28** | 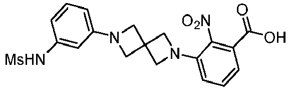 | 3-(6-(3-(methylsulfonamido)phenyl )-2,6-diazaspiro[3.3]heptan-2-yl)-2-nitrobenzoic acid | + |
| **29** | 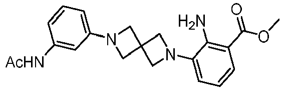 | Methyl 3-(6-(3-acetylaminophenyl)-2,6-dia zaspiro [3.3]heptan-2-yl)-2-aminobe nzoate | ++ |
| **30** | 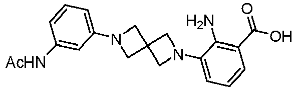 | 3-(6-(3-acetylaminophenyl)-2,6-dia zaspiro[3.3]heptan-2-yl)-2-aminobe nzoic acid | + |
| **31** | 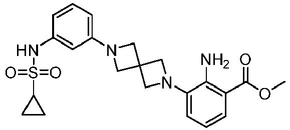 | Methyl 2-amino-3-(6-(3-(cyclopropylsulfo namido)phenyl)-2,6-diazaspiro[3.3] heptan-2-yl)benzoate | ++ |
| **32** | 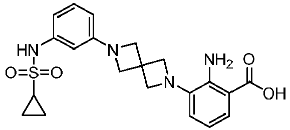 | 2-amino-3-(6-(3-(cyclopropylsulfo namido)phenyl)-2,6-diazaspiro[3.3] heptan-2-yl)benzoic acid | + |
| **33** | 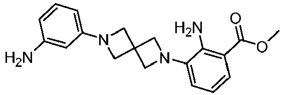 | Methyl 2-amino-3-(6-(3-aminophenyl)-2,6-diazaspiro[3.3]heptan-2-yl)benzoat e | ++ |
| **34** | 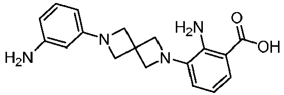 | 2-amino-3-(6-(3-aminophenyl)-2,6-diazaspiro[3.3]heptan-2-yl)benzoic acid | + |
| **35** | 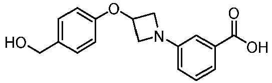 | 3 -(3 -(4-(hydroxymethyl)phenoxy)a zetidin-1-yl)benzoic acid | ++ |
| **36** | 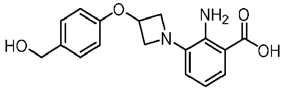 | 2-amino-3-(3-(4-(hydroxymethyl)p henoxy)azetidin-1-yl)benzoic acid | + |
| **37** | 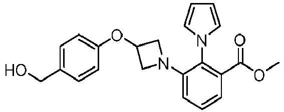 | Methyl 3-(3-(4-(hydroxymethyl)phenoxy)a zetidin-1-yl)-2-(1H-pyrrol-1-yl)ben zoate | +++ |
| **38** | 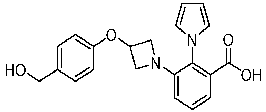 | 3-(3-(4-(hydroxymethyl)phenoxy)a zetidin-1-yl)-2-(1H-pyrrol-1-yl)ben zoic acid | + |
| **39** | 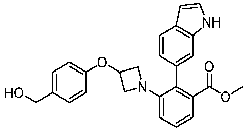 | Methyl 3-(3-(4-(hydroxymethyl)phenoxy)a zetidin-1-yl)-2-(1H-indol-6-yl)benz oate | +++ |
| **40** | 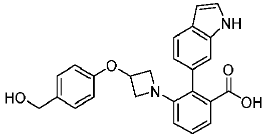 | 3-(3-(4-(hydroxymethyl)phenoxy)a zetidin-1-yl)-2-(1H-indol-6-yl)benz oic acid | + |
| **41** | 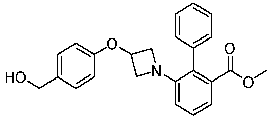 | Methyl 6-(3-(4-(hydroxymethyl)phenoxy)a zetidin-1-yl)-[1,1'-biphenyl]-2-form ate | ++ |
| **42** | 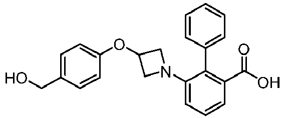 | 6-(3-(4-(hydroxymethyl)phenoxy)a zetidin-1-yl)-[1,1'-biphenyl]-2-form ic acid | + |
| **43** | 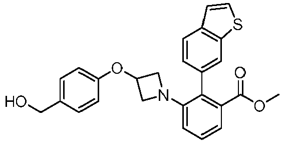 | Methyl 2-(benzo[b]thiophen-6-yl)-3-(3-(4-( hydroxymethyl)phenoxy)azetidin-1 -yl)benzoate | ++ |
| **44** | 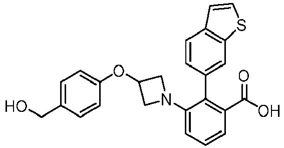 | 2-(benzo[b]thiophen-6-yl)-3-(3-(4-( hydroxymethyl)phenoxy)azetidin-1 -yl)benzoic acid | + |
| **45** | 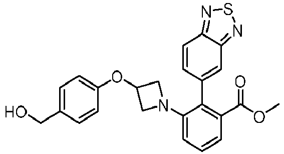 | Methyl 2-(benzo[c] [1,2,5]thiadiazol-5-yl)-3 -(3-(4-(hydroxymethyl)phenoxy)az etidin-1-yl)benzoate | ++ |
| **46** | 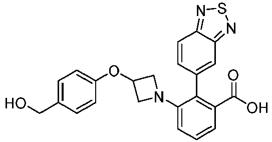 | 2-(benzo[c] [1,2,5]thiadiazol-5-yl)-3 -(3-(4-hydroxymethyl)phenoxy)aze tidin-1-yl)benzoic acid | + |
| **47** | 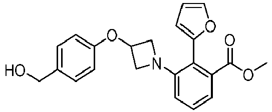 | Methyl 2-(furan-2-yl)-3-(3-(4-hydroxymeth yl)phenoxy)azetidin-1-yl)benzoate | +++ |
| **48** | 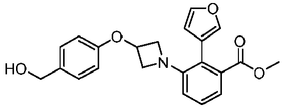 | Methyl 2-(furan-3-yl)-3-(3-(4-hydroxymeth yl)phenoxy)azetidin-1-yl)benzoate | ++++ |
| **49** | 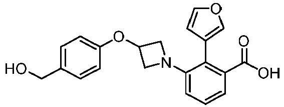 | 2-(furan-3-yl)-3-(3-(4-hydroxymeth yl)phenoxy)azetidin-1-yl)benzoic acid | + |
| **50** | 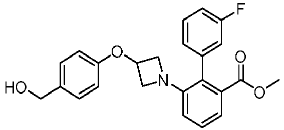 | Methyl 3'-fluoro-6-(3-(4-hydroxymethyl)ph enoxy)azetidin-1-yl)-[1,1'-biphenyl ]-2-formate | ++ |
| **51** | 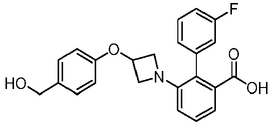 | 3'-fluoro-6-(3-(4-hydroxymethyl)ph enoxy)azetidin-1-yl)-[1,1'-biphenyl ]-2-formic acid | + |
| **52** | 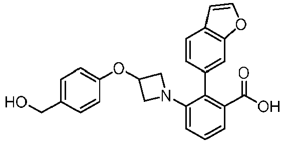 | 2-(benzofuran-6-yl)-3-(3-(4-hydrox ymethyl)phenoxy)azetidin-1-yl)ben zoic acid | + |
| **53** | 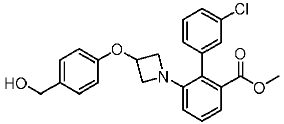 | Methyl 3'-chloro-6-(3-(4-(hydroxymethyl)p henoxy)azetidin-1-yl)-[1,1'-bipheny 1]-2-formate | ++ |
| **54** | 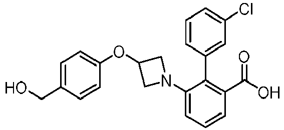 | 3'-chloro-6-(3-(4-hydroxymethyl)p henoxy)azetidin-1-yl)-[1,1'-bipheny 1]-2-formic acid | +++ |
| **55** | 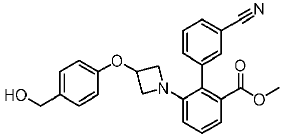 | Methyl 3'-cyano-6-(3-(4-hydroxymethyl)ph enoxy)azetidin-1-yl)-[1,1'-biphenyl ]-2-formate | ++ |
| **56** | 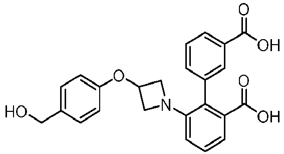 | 6-(3-(4-hydroxymethyl)phenoxy)az etidin-1-yl)-[1,1'-biphenyl]-2,3'-di-f ormic acid | + |
| **57** | 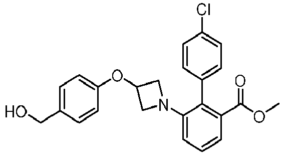 | Methyl 4'-chloro-6-(3-(4-hydroxymethyl)p henoxy)azetidin-1-yl)-[1,1'-bipheny 1]-2-formate | ++ |
| **58** | 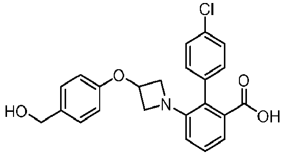 | 4'-chloro-6-(3-(4-(hydroxymethyl)p henoxy)azetidin-1-yl)-[1,1'-bipheny 1]-2-formic acid | + |
| **59** | 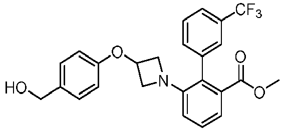 | Methyl 3'-(trifluoromethyl)-6-(3-(4-(hydro xymethyl)phenoxy)azetidin-1-yl)-[ 1,1'-biphenyl]-2-formate | ++ |
| 60 | 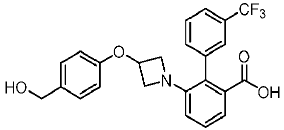 | 3'-(trifluoromethyl)-6-(3-(4-(hydro xymethyl)phenoxy)azetidin-1-yl)-[ 1,1'-biphenyl]-2-formic acid | + |
| **61** | 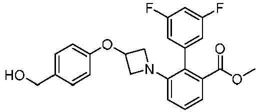 | Methyl 3',5'-difluoro-6-(3-(4-(hydroxymeth yl)phenoxy)azetidin-1-yl)-[1,1'-bip henyl]-2-formate | ++ |
| 62 | 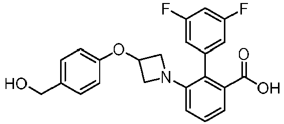 | 3',5'-difluoro-6-(3-(4-(hydroxymeth yl)phenoxy)azetidin-1-yl)-[1,1'-bip henyl]-2-formic acid | + |
| **63** | 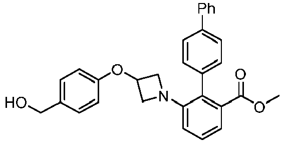 | Methyl 6-(3-(4-(hydroxymethyl)phenoxy)a zetidin-1-yl)-[1,1':4',1"-terphenyl]-2-formate | ++ |
| 64 | 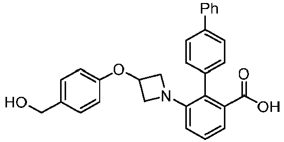 | 6-(3-(4-(hydroxymethyl)phenoxy)a zetidin-1-yl)-[1,1':4',1"-terphenyl]-2-formic acid | ++++ |
| **65** | 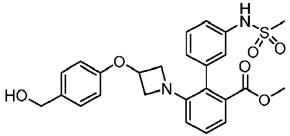 | Methyl 3'-(methylsulfonamido)-6-(3-(4-(hy droxymethyl)phenoxy)azetidin-1-yl )-[1,1'-biphenyl]-2-formate | +++ |
| **66** | 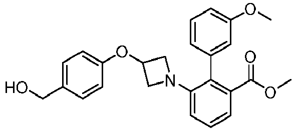 | Methyl 3'-methoxy-6-(3-(4-(hydroxymethy l)phenoxy)azetidin-1-yl)-[1,1'-biph enyl]-2-formate | +++ |
| 67 | 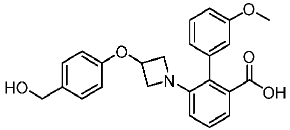 | 3 '-methoxy-6-(3 -(4-(hydroxymethy l)phenoxy)azetidin-1-yl)-[1,1'-biph enyl]-2-formic acid | + |
| **68** | 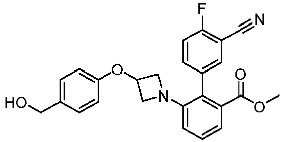 | Methyl 3'-cyano-4'-fluoro-6-(3-(4-(hydroxy methyl)phenoxy)azetidin-1-yl)-[1,1 '-biphenyl]-2-formate | ++ |
| **69** | 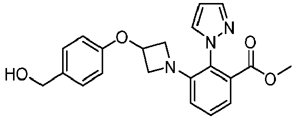 | Methyl 3-(3-(4-(hydroxymethyl)phenoxy)a zetidin-1-yl)-2-(1H-pyrazol-1-yl)be nzoate | ++ |
| **70** | 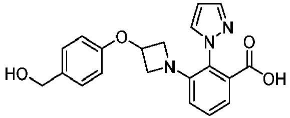 | 3-(3-(4-(hydroxymethyl)phenoxy)a zetidin-1-yl)-2-(1H-pyrazol-1-yl)be nzoic acid | + |
| **71** | 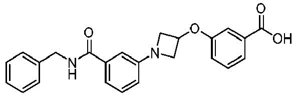 | 3-((1-(3-(benzylcarbamoyl)phenyl) azetidin-3-yl)oxy)benzoic acid | + |
| **72** | 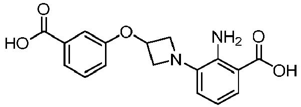 | 3-(3-(3-carboxylphenoxy)azetidin-1-yl)-2-aminobenzoic acid | + |
| **73** | 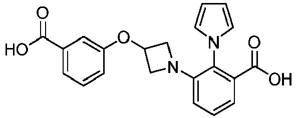 | 3-(3-(3-carboxylphenoxy)azetidin-1-yl)-2-(1H-pyrrol-1-yl)benzoic acid | + |
| **74** | 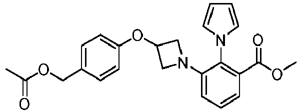 | Methyl 3-(3-(4-(acetoxylmethyl)phenoxy)a zetidin-1-yl)-2-(1H-pyrrol-1-yl)ben zoate | + |
| **75** | 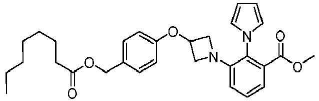 | Methyl 3-(3-(4-((octanoyloxy)methyl)phen oxy)azetidin-1-yl)-2-(1H-pyrrol-1-yl)benzoate | ++ |
| **76** | 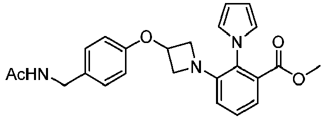 | Methyl 3-(3-(4-(acetylaminomethyl)pheno xy)azetidin-1-yl)-2-(1H-pyrrol-1-yl )benzoate | ++ |
| **77** | 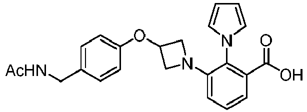 | 3-(3-(4-(acetylaminomethyl)pheno xy)azetidin-1-yl)-2-(1H-pyrrol-1-yl )benzoic acid | + |
| **78** | 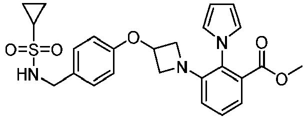 | Methyl 3-(3-(4-(cyclopropylsulfonamidom ethyl)phenoxy)azetidin-1-yl)-2-(1H -pyrrol-1 -yl)benzoate | ++++ |
| **79** | 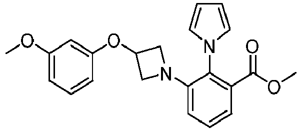 | Methyl 3-(3-(3-methoxyphenoxy)azetidin-1-yl)-2-(1H-pyrrol-1-yl)benzoate | ++ |
| **80** | 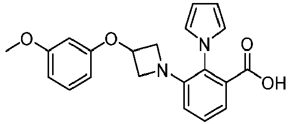 | 3-(3-(3-methoxyphenoxy)azetidin-1-yl)-2-(1H-pyrrol-1-yl)benzoic acid | + |
| **81** | 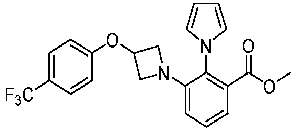 | Methyl 2-(1H-pyrrol-1-yl)-3-(3-(4-(trifluor omethyl)phenoxy)azetidin-1 -yl)ben zoate | ++ |
| **82** | 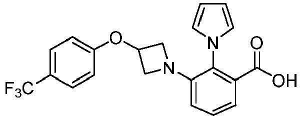 | 2-(1H-pyrrol-1-yl)-3-(3-(4-(trifluor omethyl)phenoxy)azetidin-1-yl)ben zoic acid | + |
| **83** | 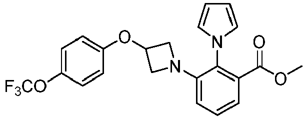 | Methyl 2-(1H-pyrrol-1-yl)-3-(3-(4-(trifluor omethoxy)phenoxy)azetidin-1 -yl)b enzoate | ++++ |
| **84** | 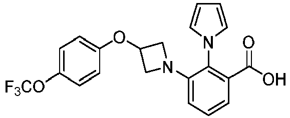 | 2-(1H-pyrrol-1-yl)-3-(3-(4-(trifluor omethoxy)phenoxy)azetidin-1-yl)b enzoic acid | + |
| **85** | 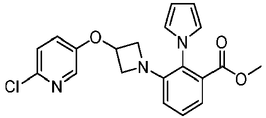 | Methyl 3-(3-((6-chloropyridin-3-yl)oxy)aze tidin-1-yl)-2-(1H-pyrrol-1-yl)benzo ate | ++++ |
| **86** | 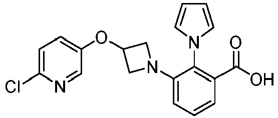 | 3-(3-((6-chloropyridin-3-yl)oxy)aze tidin-1-yl)-2-(1H-pyrrol-1-yl)benzo ic acid | + |
| **87** | 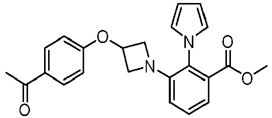 | Methyl 3-(3-(4-acetylphenoxy)azetidin-1-y 1)-2-(1H-pyrrol-1-yl)benzoate | ++++ |
| **88** | 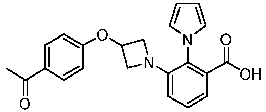 | 3 -(3 -(4-acetylphenoxy)azetidin -1 -y 1)-2-(1H-pyrrol-1-yl)benzoic acid | + |
| **89** | 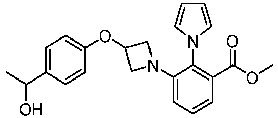 | Methyl 3-(3-(4-(1-hydroxyethyl)phenoxy)a zetidin-1-yl)-2-(1H-pyrrol-1-yl)ben zoate | ++++ |
| **90** | 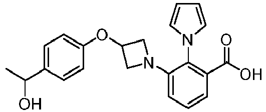 | 3-(3-(4-(1-hydroxyethyl)phenoxy)a zetidin-1-yl)-2-(1H-pyrrol-1-yl)ben zoic acid | + |
| **91** | 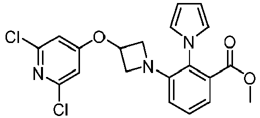 | Methyl 3-(3-((2,6-dichloropyridin-4-yl)oxy )azetidin-1-yl)-2-(1H-pyrrol-1-yl)b enzoate | + |
| **92** | 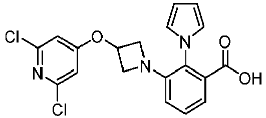 | 3-(3-((2,6-dichloropyridin-4-yl)oxy )azetidin-1-yl)-2-(1H-pyrrol-1-yl)b enzoic acid | + |
| **93** | 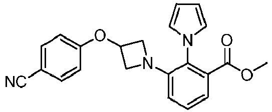 | Methyl 3-(3-(4-cyanophenoxy)azetidin-1-y 1)-2-(1H-pyrrol-1-yl)benzoate | +++ |
| **94** | 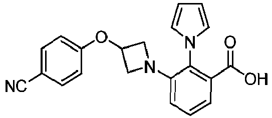 | 3-(3-(4-cyanophenoxy)azetidin-1-y 1)-2-(1H-pyrrol-1-yl)benzoic acid | + |
| **95** | 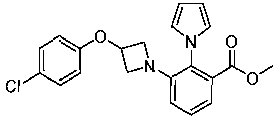 | Methyl 3-(3-(4-chlorophenoxy)azetidin-1-y 1)-2-(1H-pyrrol-1-yl)benzoate | + |
| **96** | 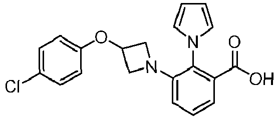 | 3-(3-(4-chlorophenoxy)azetidin-1-y 1)-2-(1H-pyrrol-1-yl)benzoic acid | + |
| **97** | 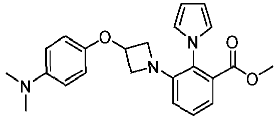 | Methyl 3-(3-(4-(dimethylamino)phenoxy)a zetidin-1-yl)-2-(1H-pyrrol-1-yl)ben zoate | ++ |
| **98** | 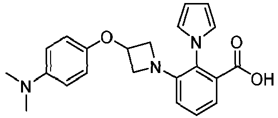 | 3-(3-(4-(dimethylamino)phenoxy)a zetidin-1-yl)-2-(1H-pyrrol-1-yl)ben zoic acid | ++ |
| **99** | 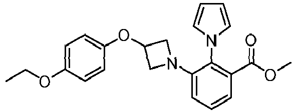 | Methyl 3-(3-(4-ethoxyphenoxy)azetidin-1-yl)-2-(1H-pyrrol-1-yl)benzoate | + |
| **100** | 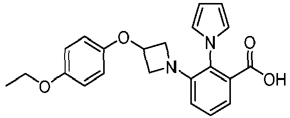 | 3-(3-(4-ethoxyphenoxy)azetidin-1 - yl)-2-(1H-pyrrol-1-yl)benzoic acid | + |
| **101** | 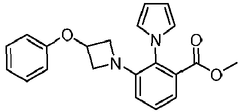 | Methyl 3-(3-phenoxyazetidin-1-yl)-2-(1H-pyrrol-1-yl)benzoate | +++ |
| **102** | 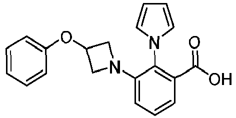 | 3-(3-phenoxyazetidin-1-yl)-2-(1H-pyrrol-1-yl)benzoic acid | + |
| **103** | 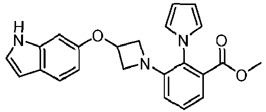 | Methyl 3-(3-((1H-indol-6-yl)oxy)azetidin-1 -yl)-2-(1H-pyrrol-1-yl)benzoate | ++ |
| **104** | 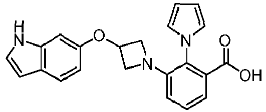 | 3-(3-((1H-indol-6-yl)oxy)azetidin-1 -yl)-2-(1H-pyrrol-1-yl)benzoic acid | + |
| **105** | 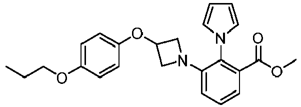 | Methyl 3-(3-(4-propoxyphenoxy)azetidin-1 -yl)-2-(1 H-pyrrol-1 -yl)benzoate | + |
| **106** | 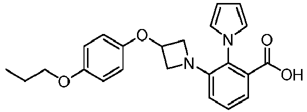 | 3-(3-(4-propoxyphenoxy)azetidin-1 -yl)-2-(1H-pyrrol-1-yl)benzoic acid | + |
| **107** | 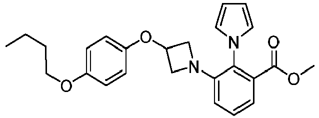 | Methyl 3-(3-(4-butoxyphenoxy)azetidin-1-yl)-2-(1H-pyrrol-1-yl)benzoate | + |
| **108** | 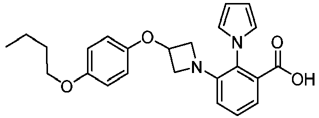 | 3-(3-(4-butoxyphenoxy)azetidin-1-yl)-2-(1H-pyrrol-1-yl)benzoic acid | + |
| **109** | 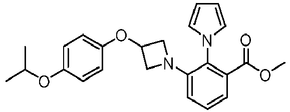 | Methyl 3-(3-(4-isopropoxyphenoxy)azetidi n-1-yl)-2-(1H-pyrrol-1-yl)benzoate | + |
| **110** | 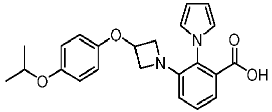 | 3 -(3-(4-isopropoxyphenoxy)azetidi n-1-yl)-2-(1H-pyrrol-1-yl)benzoic acid | + |
| **111** | 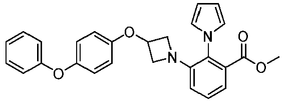 | Methyl 3-(3-(4-phenoxyphenoxy)azetidin-1 -yl)-2-(1H-pyrrol-1-yl)benzoate | + |
| **112** | 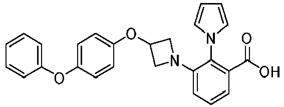 | 3-(3-(4-phenoxyphenoxy)azetidin-1 -yl)-2-(1H-pyrrol-1-yl)benzoic acid | + |
| **113** | 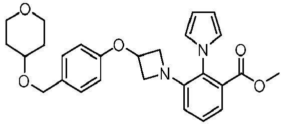 | Methyl 2-(1H-pyrrol-1-yl)-3-(3-(4-(((tetrah ydro-2H-pyran-4-yl)oxy)methyl)ph enoxy)azetidin-1-yl)benzoate | ++++ |
| **114** | 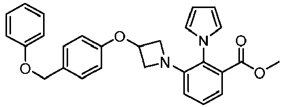 | Methyl 3-(3-(4-(phenoxymethyl)phenoxy)a zetidin-1-yl)-2-(1H-pyrrol-1-yl)ben zoate | ++ |
| **115** | 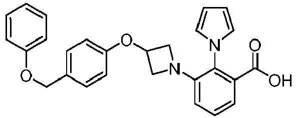 | 3-(3-(4-(phenoxymethyl)phenoxy)a zetidin-1-yl)-2-(1H-pyrrol-1-yl)ben zoic acid | + |
| **116** | 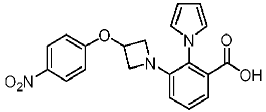 | 3-(3-(4-nitrophenoxy)azetidin-1-yl) -2-(1H-pyrrol-1-yl)benzoic acid | + |
| **117** | 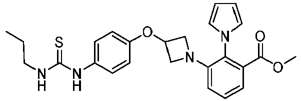 | Methyl 3-(3-(4-(3-propylthioureido)phenox y)azetidin-1-yl)-2-(1H-pyrrol-1-yl) benzoate | ++++ |
| **118** | 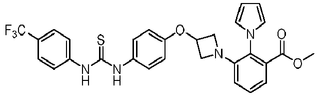 | Methyl 2-(1H-pyrrol-1-yl)-3-(3-(4-(3-(4-(tr ifluoromethyl)phenyl)thioureido)ph enoxy)azetidin-1 -yl)benzoate | ++++ |
| **119** | 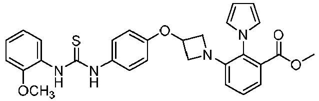 | Methyl 3-(3-(4-(3-(2-methoxyphenyl)thiou reido)phenoxy)azetidin-1-yl)-2-(1H -pyrrol-1-yl)benzoate | ++ |
| **120** | 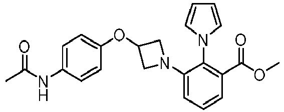 | Methyl 3-(3-(4-acetylaminophenoxy)azetid in-1-yl)-2-(1H-pyrrol-1-yl)benzoate | ++ |
| **121** | 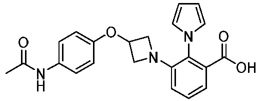 | 3-(3-(4-acetylaminophenoxy)azetid in-1-yl)-2-(1H-pyrrol-1-yl)benzoic acid | + |
| **122** | 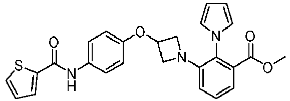 | Methyl 2-(1H-pyrrol-1-yl)-3-(3-(4-(thiophe ne-2-formamido)phenoxy)azetidin-1-yl)benzoate | ++ |
| **123** | 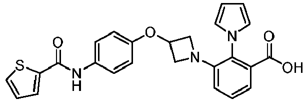 | 2-(1H-pyrrol-1-yl)-3-(3-(4-(thiophe ne-2-formamido)phenoxy)azetidin-1-yl)benzoic acid | + |
| **124** | 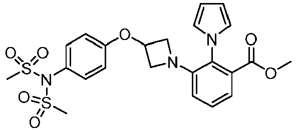 | Methyl 3-(3-(4-(N-(mesyl)methylsulfonami do)phenoxy)azetidin-1-yl)-2-(1H-p yrrol-1-yl)benzoate | ++ |
| **125** | 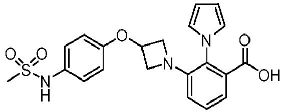 | 3-(3-(4-(methylsulfonamido)pheno xy)azetidin-1-yl)-2-(1H-pyrrol-1-yl )benzoic acid | + |
| **126** | 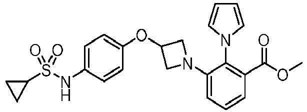 | Methyl 3-(3-(4-(cyclopropylsulfonamido)p henoxy)azetidin-1-yl)-2-(1H-pyrrol -1-yl)benzoate | ++++ |
| **127** | 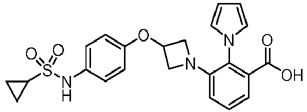 | 3-(3-(4-(cyclopropylsulfonamido)p henoxy)azetidin-1-yl)-2-(1H-pyrrol -1-yl)benzoic acid | + |
| **128** | 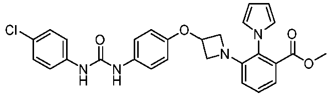 | Methyl 3-(3-(4-(3-(4-chlorophenyl)ureido) phenoxy)azetidin-1-yl)-2-(1H-pyrr ol-1-yl)benzoate | + |
| **129** | 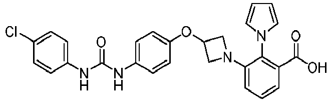 | 3-(3-(4-(3-(4-chlorophenyl)ureido) phenoxy)azetidin-1-yl)-2-(1H-pyrr ol-1-yl)benzoic acid | + |
| **130** | 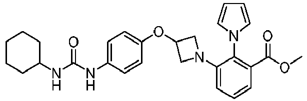 | Methyl 3-(3-(4-(3-cyclohexylureido)pheno xy)azetidin-1-yl)-2-(1H-pyrrol-1-yl )benzoate | + |
| **131** | 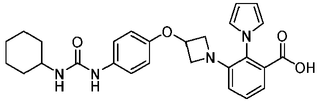 | 3-(3-(4-(3-cyclohexylureido)pheno xy)azetidin-1-yl)-2-(1H-pyrrol-1-yl )benzoic acid | + |
| **132** | 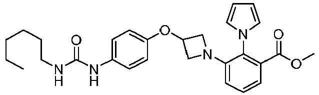 | Methyl 3-(3-(4-(3-hexylureido)phenoxy)az etidin-1-yl)-2-(1H-pyrrol-1-yl)benz oate | + |
| **133** | 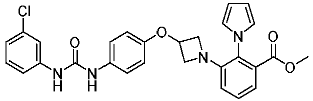 | Methyl 3-(3-(4-(3-(3-chlorophenyl)ureido) phenoxy)azetidin-1-yl)-2-(1H-pyrr ol-1-yl)benzoate | ++++ |
| **134** | 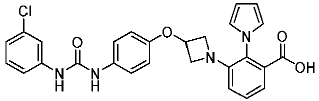 | 3-(3-(4-(3-(3-chlorophenyl)ureido) phenoxy)azetidin-1-yl)-2-(1H-pyrr ol-1-yl)benzoic acid | ++ |
| **135** | 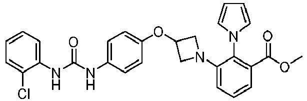 | Methyl 3-(3-(4-(3-(2-chlorophenyl)ureido) phenoxy)azetidin-1-yl)-2-(1H-pyrr ol-1-yl)benzoate | + |
| **136** | 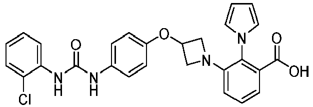 | 3-(3-(4-(3-(2-chlorophenyl)ureido) phenoxy)azetidin-1-yl)-2-(1H-pyrr ol-1-yl)benzoic acid | + |
| **137** | 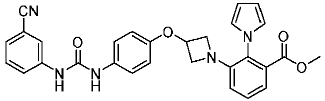 | Methyl 3-(3-(4-(3-(3-cyanophenyl)ureido)p henoxy)azetidin-1-yl)-2-(1H-pyrrol -1-yl)benzoate | ++++ |
| **138** | 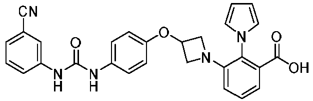 | 3-(3-(4-(3-(3-cyanophenyl)ureido)p henoxy)azetidin-1-yl)-2-(1H-pyrrol -1-yl)benzoic acid | + |
| **139** | 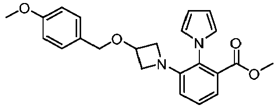 | Methyl 3-(3-((4-methoxybenzyl)oxy)azetid in-1-yl)-2-(1H-pyrrol-1-yl)benzoate | + |
| **140** | 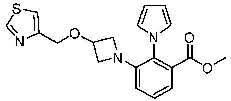 | Methyl 2-(1H-pyrrol-1-yl)-3-(3-(thiazol-4-yl)methoxy)azetidin-1-yl)benzoate | ++ |
| **141** | 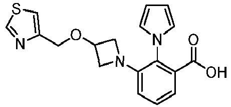 | 2-(1H-pyrrol-1-yl)-3-(3-(thiazol-4-yl)methoxy)azetidin-1-yl)benzoic acid | + |
| **142** | 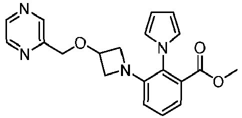 | Methyl 3-(3-(pyrazin-2-yl)methoxy)azetidi n-1-yl)-2-(1H-pyrrol-1-yl)benzoate | + |
| **143** | 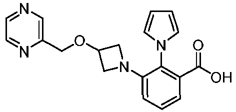 | 3-(3-(pyrazin-2-yl)methoxy)azetidi n-1-yl)-2-(1H-pyrrol-1-yl)benzoic acid | + |
| **144** | 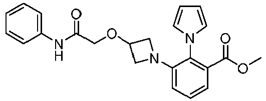 | Methyl 3-(3-(2-oxo-2-(phenylamino)ethox y)azetidin-1-yl)-2-(1H-pyrrol-1-yl) benzoate | ++ |
| **145** | 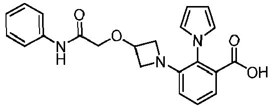 | 3-(3-(2-oxo-2-(phenylamino)ethox y)azetidin-1-yl)-2-(1H-pyrrol-1-yl) benzoic acid | + |
| **146** | 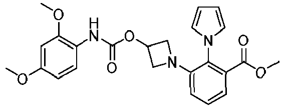 | Methyl 3-(3-(((2,4-dimethoxyphenyl)carba moyl)oxy)azetidin-1-yl)-2-(1H-pyrr ol-1-yl)benzoate | ++ |
| **147** | 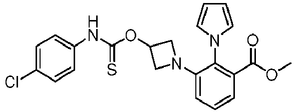 | Methyl 3-(3-(((4-chlorophenyl)thiocarbam oyl)oxy)azetidin-1-yl)-2-(1H-pyrrol -1-yl)benzoate | +++ |
| **148** | 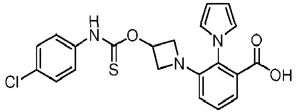 | 3-(3-(((4-chlorophenyl)thiocarbam oyl)oxy)azetidin-1-yl)-2-(1H-pyrrol -1-yl)benzoic acid | + |
| **149** | 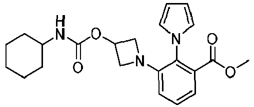 | Methyl 3-(3-((cyclohexylcarbamoyl)oxy)az etidin-1-yl)-2-(1H-pyrrol-1-yl)benz oate | ++ |
| **150** | 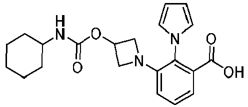 | 3-(3-((cyclohexylcarbamoyl)oxy)az etidin-1-yl)-2-(1H-pyrrol-1-yl)benz oic acid | + |
| **151** | 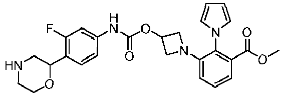 | Methyl 3-(3-(((3-fluoro-4-(morpholin-2-yl) phenyl)carbamoyl)oxy)azetidin-1 -y 1)-2-(1H-pyrrol-1-yl)benzoate | ++ |
| **152** | 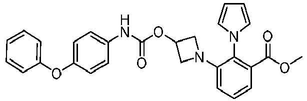 | Methyl 3-(3-(((4-phenoxyphenyl)carbamoy l)oxy)azetidin-1-yl)-2-(1H-pyrrol-1 -yl)benzoate | + |
| **153** | 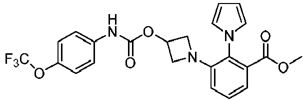 | Methyl 2-(1H-pyrrol-1-yl)-3-(3-(((4-(triflu oromethoxy)phenyl)carbamoyl)oxy )azetidin-1-yl)benzoate | ++ |
| **154** | 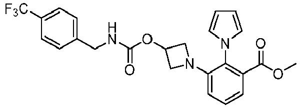 | Methyl 2-(1H-pyrrol-1-yl)-3-(3-(((4-(triflu oromethyl)benzyl)carbamoyl)oxy)a zetidin-1 -yl)benzoate | ++ |
| **155** | 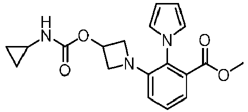 | Methyl 3-(3-((cyclopropylcarbamoyl)oxy)a zetidin-1-yl)-2-(1H-pyrrol-1-yl)ben zoate | ++ |
| **156** | 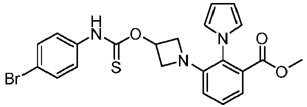 | Methyl 3-(3-(((4-bromophenyl)thiocarbam oyl)oxy)azetidin-1-yl)-2-(1H-pyrrol -1-yl)benzoate | +++ |
| **157** | 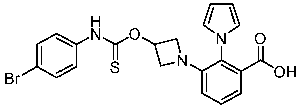 | 3-(3-(((4-bromophenyl)thiocarbam oyl)oxy)azetidin-1-yl)-2-(1H-pyrrol -1-yl)benzoic acid | + |
| **158** | 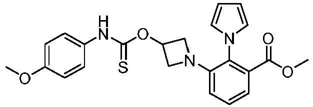 | Methyl 3-(3-(((4-methoxyphenyl)thiocarba moyl)oxy)azetidin-1-yl)-2-(1H-pyrr ol-1-yl)benzoate | ++++ |
| **159** | 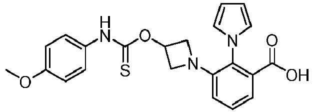 | 3-(3-(((4-methoxyphenyl)thiocarba moyl)oxy)azetidin-1-yl)-2-(1H-pyrr ol-1-yl)benzoic acid | + |
| **160** | 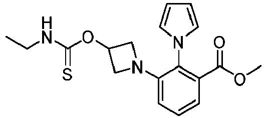 | Methyl 3-(3-((ethylthiocarbamoyl)oxy)azet idin-1-yl)-2-(1H-pyrrol-1-yl)benzo ate | +++ |
| **161** | 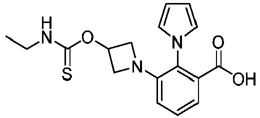 | 3-(3-((ethylthiocarbamoyl)oxy)azet idin-1-yl)-2-(1H-pyrrol-1-yl)benzoi c acid | + |
| **162** | 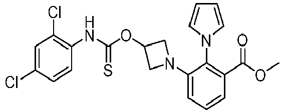 | Methyl 3-(3-(((2,4-dichlorophenyl)thiocarb amoyl)oxy)azetidin-1-yl)-2-(1H-py rrol-1-yl)benzoate | ++ |
| **163** | 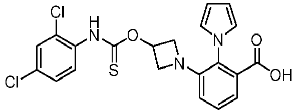 | 3-(3-(((2,4-dichlorophenyl)thiocarb amoyl)oxy)azetidin-1-yl)-2-(1H-py rrol-1-yl)benzoic acid | + |
| **164** | 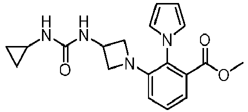 | Methyl 3-(3-(3-cyclopropylureido)azetidin-1-yl)-2-(1H-pyrrol-1-yl)benzoate | + |
| **165** | 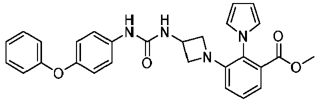 | Methyl 3-(3-(3-(4-phenoxyphenyl)ureido)a zetidin-1-yl)-2-(1H-pyrrol-1-yl)ben zoate | ++ |
| **166** | 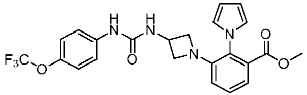 | Methyl 2-(1H-pyrrol-1-yl)-3-(3-(3-(4-(trifl uoromethoxy)phenyl)ureido)azetidi n-1-yl)benzoate | ++++ |
| **167** | 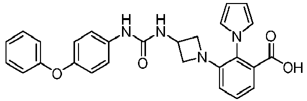 | 3 -(3 -(3 -(4-phenoxyphenyl)ureido)a zetidin-1-yl)-2-(1H-pyrrol-1-yl)ben zoic acid | + |
| **168** | 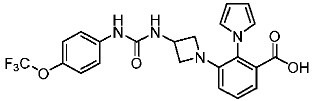 | 2-(1H-pyrrol-1-yl)-3-(3-(3-(4-(trifl uoromethoxy)phenyl)ureido)azetidi n-1-yl)benzoic acid | + |
| **169** | 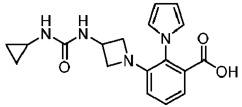 | 3-(3-(3-cyclopropylureido)azetidin-1-yl)-2-(1H-pyrrol-1-yl)benzoic acid | + |
| **170** | 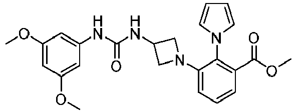 | Methyl 3-(3-(3-(3,5-dimethoxyphenyl)urei do)azetidin-1-yl)-2-(1H-pyrrol-1-yl )benzoate | ++ |
| **171** | 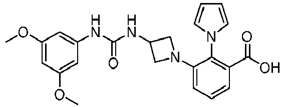 | 3-(3-(3-(3,5-dimethoxyphenyl)urei do)azetidin-1-yl)-2-(1H-pyrrol-1-yl )benzoic acid | + |
| **172** | 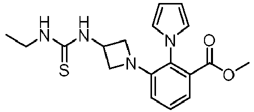 | Methyl 3-(3-(3-ethylthioureido)azetidin-1-yl)-2-(1H-pyrrol-1-yl)benzoate | ++ |
| **173** | 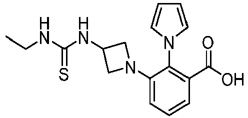 | 3-(3-(3-ethylthioureido)azetidin-1-yl)-2-(1H-pyrrol-1-yl)benzoic acid | + |
| **174** | 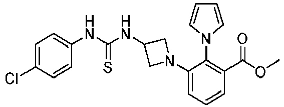 | Methyl 3-(3-(3-(4-chlorophenyl)thioureido) azetidin-1-yl)-2-(1H-pyrrol-1-yl)be nzoate | ++++ |
| **175** | 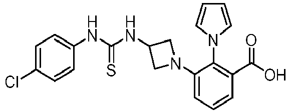 | 3-(3-(3-(4-chlorophenyl)thioureido) azetidin-1-yl)-2-(1H-pyrrol-1-yl)be nzoic acid | + |
| **176** | 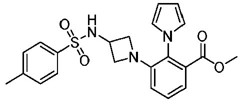 | Methyl 3-(3-((4-methylphenyl)sulfonamido )azetidin-1-yl)-2-(1H-pyrrol-1-yl)b enzoate | ++ |
| **177** | 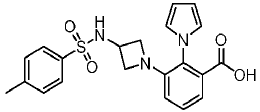 | 3-(3-((4-methylphenyl)sulfonamido )azetidin-1-yl)-2-(1H-pyrrol-1-yl)b enzoic acid | + |
| **178** | 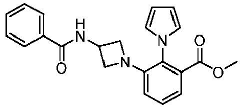 | Methyl 3-(3-benzamidoazetidin-1-yl)-2-(1 H-pyrrol-1-yl)benzoate | ++ |
| **179** | 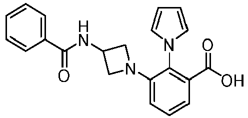 | 3-(3-benzamidoazetidin-1-yl)-2-(1 H-pyrrol-1-yl)benzoic acid | + |
| **180** | 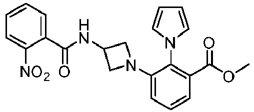 | Methyl 3-(3-(2-nitrobenzamido)azetidin-1-yl)-2-(1H-pyrrol-1-yl)benzoate | +++ |
| **181** | 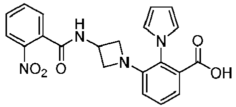 | 3-(3-(2-nitrobenzamido)azetidin-1-yl)-2-(1H-pyrrol-1-yl)benzoic acid | + |
| **182** | 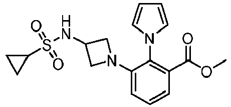 | Methyl 3-(3-(cyclopropylsulfonamido)azeti din-1-yl)-2-(1H-pyrrol-1-yl)benzoa te | ++ |
| **183** | 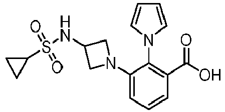 | 3-(3-(cyclopropylsulfonamido)azeti din-1-yl)-2-(1H-pyrrol-1-yl)benzoi c acid | + |
| **184** | 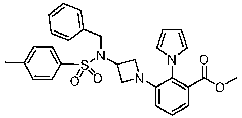 | Methyl 3-(3-((N-benzyl-4-methylphenyl)su lfonamido)azetidin-1-yl)-2-(1H-pyr rol-1-yl)benzoate | + |
| **185** | 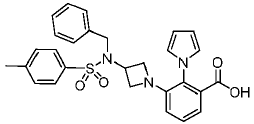 | 3-(3-((N-benzyl-4-methylphenyl)su lfonamido)azetidin-1-yl)-2-(1H-pyr rol-1-yl)benzoic acid | + |
| **186** | 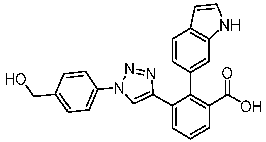 | 3-(1-(4-(hydroxymethyl)phenyl)-1 H-1,2,3-triazol-4-yl)-2-(1H-indol-6 -yl)benzoic acid | + |
| **187** | 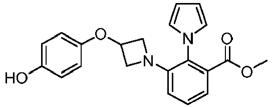 | Methyl 3-(3-(4-hydroxylphenoxy)azetidin-1-yl)-2-(1H-pyrrol-1-yl)benzoate | + |
| **188** | 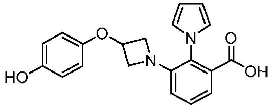 | 3-(3-(4-hydroxylphenoxy)azetidin-1-yl)-2-(1H-pyrrol-1-yl)benzoic acid | + |
| **189** | 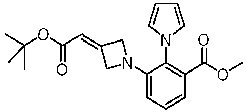 | Methyl 3-(3-(2-(tert-butoxy)-2-oxovinyl)az etidin-1-yl)-2-(1H-pyrrol-1-yl)benz oate | ++ |
| **190** | 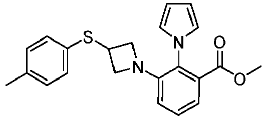 | Methyl 2-(1H-pyrrol-1-yl)-3-(3-(p-tolylthio )azetidin-1-yl)benzoate | + |
| **191** | 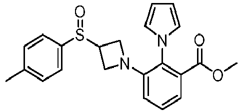 | Methyl 2-(1H-pyrrol-1-yl)-3-(3-(p-tolylsulf inyl)azetidin-1-yl)benzoate | ++ |
| **192** | 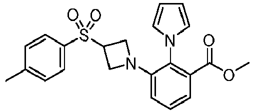 | Methyl 2-(1H-pyrrol-1-yl)-3-(3-(p-tolylsulf onyl)azetidin-1-yl)benzoate | ++ |
| **193** | 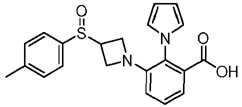 | 2-(1H-pyrrol-1-yl)-3-(3-p-tolylsulfi nyl)azetidin-1-yl)benzoic acid | + |
| **194** | 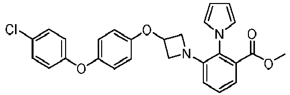 | Methyl 3-(3-(4-(4-chlorophenoxy)phenoxy )azetidin-1-yl)-2-(1H-pyrrol-1-yl)b enzoate | + |
| **195** | 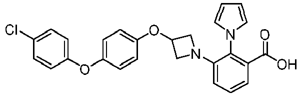 | 3-(3-(4-(4-chlorophenoxy)phenoxy )azetidin-1-yl)-2-(1H-pyrrol-1-yl)b enzoic acid | ++ |
| **196** | 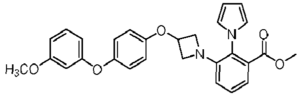 | Methyl 3-(3-(4-(3-methoxyphenoxy)pheno xy)azetidin-1-yl)-2-(1H-pyrrol-1-yl )benzoate | + |
| **197** | 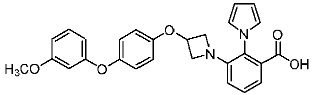 | 3-(3-(4-(3-methoxyphenoxy)pheno xy)azetidin-1-yl)-2-(1H-pyrrol-1-yl )benzoic acid | ++ |
| **198** | 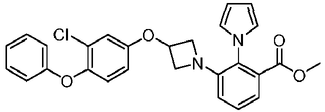 | Methyl 3-(3-(3-chloro-4-phenoxyphenoxy) azetidin-1-yl)-2-(1H-pyrrol-1-yl)be nzoate | + |
| **199** | 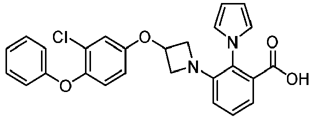 | 3-(3-(3-chloro-4-phenoxyphenoxy) azetidin-1-yl)-2-(1H-pyrrol-1-yl)be nzoic acid | +++ |
| **200** | 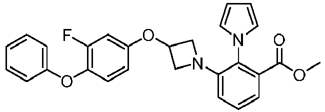 | Methyl 3-(3-(3-fluoro-4-phenoxyphenoxy) azetidin-1-yl)-2-(1H-pyrrol-1-yl)be nzoate | + |
| **201** | 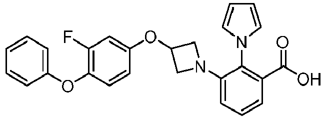 | 3-(3-(3-fluoro-4-phenoxyphenoxy) azetidin-1-yl)-2-(1H-pyrrol-1-yl)be nzoic acid | +++ |
| **202** | 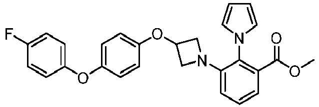 | Methyl 3-(3-(4-(4-fluorophenoxy)phenoxy) azetidin-1-yl)-2-(1H-pyrrol-1-yl)be nzoate | + |
| **203** | 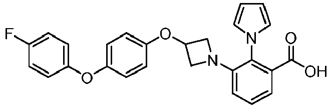 | 3-(3-(4-(4-fluorophenoxy)phenoxy) azetidin-1-yl)-2-(1H-pyrrol-1-yl)be nzoic acid | ++ |
| **204** | 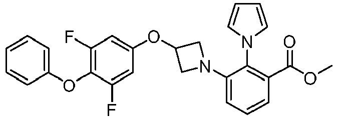 | Methyl 3-(3-(3,5-difluoro-4-phenoxypheno xy)azetidin-1-yl)-2-(1H-pyrrol-1-yl )benzoate | + |
| **205** | 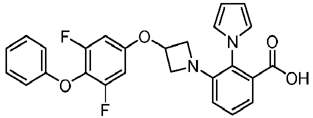 | 3-(3-(3,5-difluoro-4-phenoxypheno xy)azetidin-1-yl)-2-(1H-pyrrol-1-yl )benzoic acid | + |
| **206** | 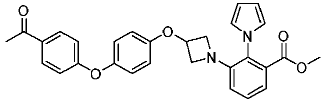 | Methyl 3-(3-(4-(4-acetylphenoxy)phenoxy) azetidin-1-yl)-2-(1H-pyrrol-1-yl)be nzoate | + |
| **207** | 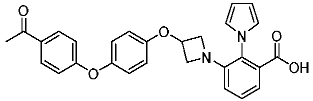 | 3-(3-(4-(4-acetylphenoxy)phenoxy) azetidin-1-yl)-2-(1H-pyrrol-1-yl)be nzoic acid | ++ |
| **208** | 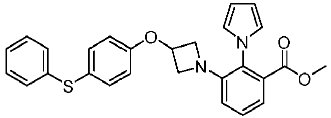 | Methyl 3-(3-(4-(thiophenyl)phenoxy)azetid in-1-yl)-2-(1H-pyrrol-1-yl)benzoate | + |
| **209** | 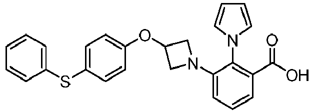 | 3-(3-(4-(thiophenyl)phenoxy)azetid in-1-yl)-2-(1H-pyrrol-1-yl)benzoic acid | ++ |
| **210** | 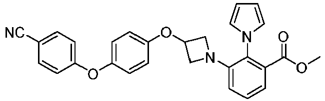 | Methyl 3-(3-(4-(4-cyanophenoxy)phenoxy) azetidin-1-yl)-2-(1H-pyrrol-1-yl)be nzoate | ++ |
| **211** | 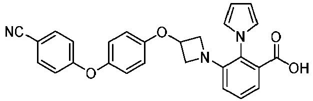 | 3-(3-(4-(4-cyanophenoxy)phenoxy) azetidin-1-yl)-2-(1H-pyrrol-1-yl)be nzoic acid | + |
| **212** | 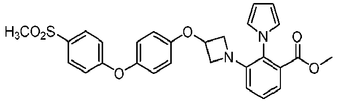 | Methyl 3-(3-(4-(4-(methylsulfonyl)phenox y)phenoxy)azetidin-1-yl)-2-(1H-py rrol-1-yl)benzoate | ++ |
| **213** | 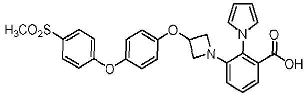 | 3-(3-(4-(4-(methylsulfonyl)phenox y)phenoxy)azetidin-1-yl)-2-(1H-py rrol-1-yl)benzoic acid | + |
| **214** | 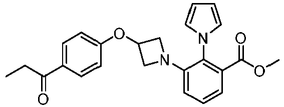 | Methyl 3-(3-(4-propionylphenoxy)azetidin-1-yl)-2-(1H-pyrrol-1-yl)benzoate | + |
| **215** | 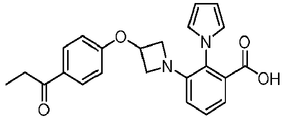 | 3-(3-(4-propionylphenoxy)azetidin-1-yl)-2-(1H-pyrrol-1-yl)benzoic acid | + |
| **216** | 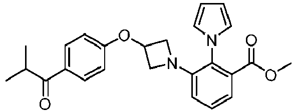 | Methyl 3 -(3 -(4-isobutyrylphenoxy)azetidin -1-yl)-2-(1H-pyrrol-1-yl)benzoate | + |
| **217** | 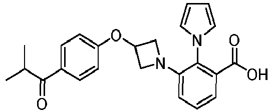 | 3-(3-(4-isobutyrylphenoxy)azetidin -1-yl)-2-(1H-pyrrol-1-yl)benzoic acid | + |
| **218** | 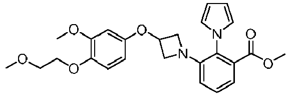 | Methyl 3-(3-(3-methoxy-4-(2-methoxyetho xy)phenoxy)azetidin-1-yl)-2-(1H-p yrrol-1-yl)benzoate | ++ |
| **219** | 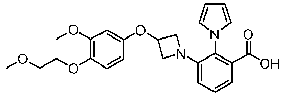 | 3-(3-(3-methoxy-4-(2-methoxyetho xy)phenoxy)azetidin-1-yl)-2-(1H-p yrrol-1-yl)benzoic acid | + |
| **220** | 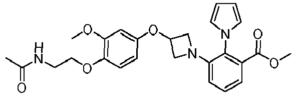 | Methyl 3-(3-(4-(2-acetylaminoethoxy)-3-m ethoxyphenoxy)azetidin-1-yl)-2-(1 H-pyrrol-1-yl)benzoate | ++ |
| **221** | 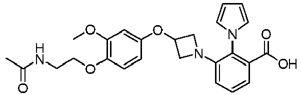 | 3-(3-(4-(2-acetylaminoethoxy)-3-m ethoxyphenoxy)azetidin-1-yl)-2-(1 H-pyrrol-1-yl)benzoic acid | + |
| **222** | 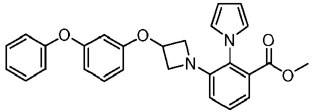 | Methyl 3-(3-(3-phenoxyphenoxy)azetidin-1 -yl)-2-(1H-pyrrol-1-yl)benzoate | + |
| **223** | 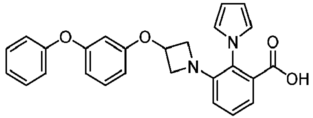 | 3-(3-(3-phenoxyphenoxy)azetidin-1 -yl)-2-(1H-pyrrol-1-yl)benzoic acid | + |
| **224** | 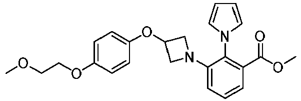 | Methyl 3-(3-(4-(2-methoxyethoxy)phenoxy )azetidin-1-yl)-2-(1H-pyrrol-1-yl)b enzoate | + |
| **225** | 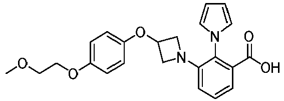 | 3-(3-(4-(2-methoxyethoxy)phenoxy )azetidin-1-yl)-2-(1H-pyrrol-1-yl)b enzoic acid | ++ |
| **226** | 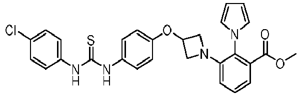 | Methyl 3-(3-(4-(3-(4-chlorophenyl)thiourei do)phenoxy)azetidin-1-yl)-2-(1H-p yrrol-1-yl)benzoate | ++ |
| **227** | 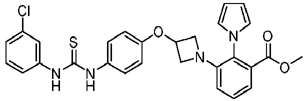 | Methyl 3-(3-(4-(3-(3-chlorophenyl)thiourei do)phenoxy)azetidin-1-yl)-2-(1H-p yrrol-1-yl)benzoate | ++ |
| **228** | 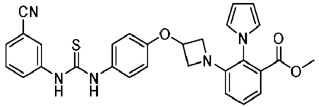 | Methyl 3-(3-(4-(3-(3-cyanophenyl)thiourei do)phenoxy)azetidin-1-yl)-2-(1H-p yrrol-1-yl)benzoate | ++++ |
| **229** | 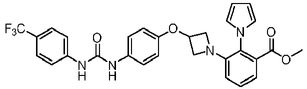 | Methyl 3-(3-(4-(3-(4-(trifluoromethyl)phen yl)ureido)phenoxy)azetidin-1-yl)-2-(1H-pyrrol-1-yl)benzoate | ++++ |
| **230** | 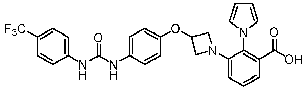 | 3-(3-(4-(3-(4-(trifluoromethyl)phen yl)ureido)phenoxy)azetidin-1 -yl)-2-(1H-pyrrol-1-yl)benzoic acid | ++ |
| **231** | 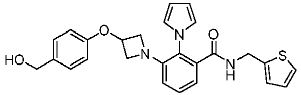 | N-(thiophen-2-yl-methyl)-3-(3-(4-( hydroxymethyl)phenoxy)azetidin-1 -yl)-2-(1H-pyrrol-1-yl)benzamide | ++ |
| **232** | 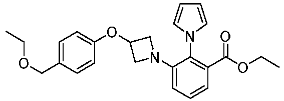 | Ethyl 3-(3-(4-(ethoxymethyl)phenoxy)az etidin-1-yl)-2-(1H-pyrrol-1-yl)benz oate | + |
| **233** | 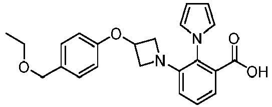 | 3-(3-(4-(ethoxymethyl)phenoxy)az etidin-1-yl)-2-(1H-pyrrol-1-yl)benz oic acid | + |
| **234** | 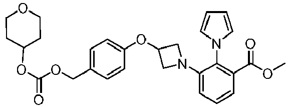 | Methyl 3-(3-(4-(((((tetrahydro-2H-pyran-4-yl)oxy)carbonyl)oxy)methyl)pheno xy)azetidin-1-yl)-2-(1H-pyrrol-1-yl)benzoate | ++++ |
| **235** | 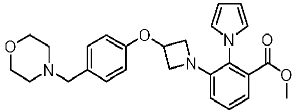 | Methyl 3-(3-(4-(morpholinylmethyl)pheno xy)azetidin-1-yl)-2-(1H-pyrrol-1-yl )benzoate | +++ |
| **236** | 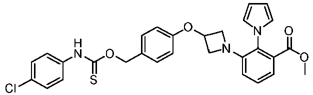 | Methyl 3-(3-(4-((((4-chlorophenyl)thiocarb amoyl)oxy)methyl)phenoxy)azetidi n-1-yl)-2-(1H-pyrrol-1-yl)benzoate | ++ |
| **237** | 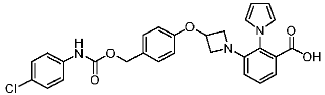 | 3-(3-(4-((((4-chlorophenyl)thiocarb amoyl)oxy)methyl)phenoxy)azetidi n-1-yl)-2-(1H-pyrrol-1-yl)benzoic acid | + |
| **238** | 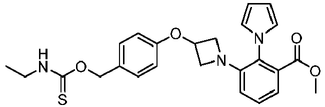 | Methyl 3-(3-(4-(((ethylthiocarbamoylamin o)oxy)methyl)phenoxy)azetidin-1-y l)-2-(1H-pyrrol-1-yl)benzoate | ++ |
| **239** | 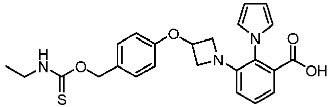 | 3-(3-(4-(((ethylthiocarbamoylamin o)oxy)methyl)phenoxy)azetidin-1-y l)-2-(1H-pyrrol-1-yl)benzoic acid | + |
| **240** | 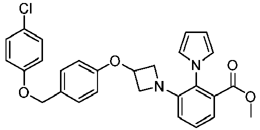 | Methyl 3-(3-(4-((4-chlorophenoxy)methyl) phenoxy)azetidin-1-yl)-2-(1H-pyrr ol-1-yl)benzoate | + |
| **241** | 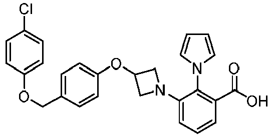 | 3-(3-(4-((4-chlorophenoxy)methyl) phenoxy)azetidin-1-yl)-2-(1H-pyrr ol-1-yl)benzoic acid | + |
| **242** | 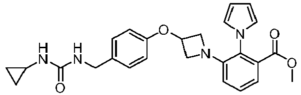 | Methyl 3-(3-(4-((3-cyclopropylureido)meth yl)phenoxy)azetidin-1-yl)-2-(1H-py rrol-1-yl)benzoate | ++ |
| **243** | 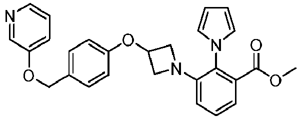 | Methyl 3-(3-(4-((pyridin-3-yl-oxy)methyl) phenoxy)azetidin-1-yl)-2-(1H-pyrr ol-1-yl)benzoate | ++++ |
| **244** | 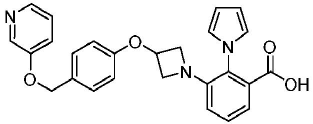 | 3-(3-(4-((pyridin-3-yl-oxy)methyl) phenoxy)azetidin-1-yl)-2-(1H-pyrr ol-1-yl)benzoic acid | +++ |
| **245** | 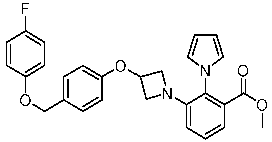 | Methyl 3-(3-(4-((4-fluorophenoxy)methyl) phenoxy)azetidin-1-yl)-2-(1H-pyrr ol-1-yl)benzoate | + |
| **246** | 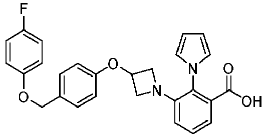 | 3-(3-(4-((4-fluorophenoxy)methyl) phenoxy)azetidin-1-yl)-2-(1H-pyrr ol-1-yl)benzoic acid | + |
| **247** | 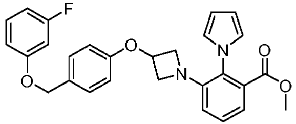 | Methyl 3-(3-(4-((3-fluorophenoxy)methyl) phenoxy)azetidin-1-yl)-2-(1H-pyrr ol-1-yl)benzoate | ++ |
| **248** | 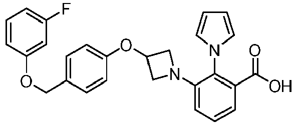 | 3-(3-(4-((3-fluorophenoxy)methyl) phenoxy)azetidin-1-yl)-2-(1H-pyrr ol-1-yl)benzoic acid | + |
| **249** | 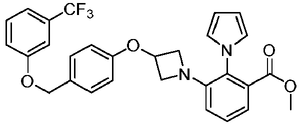 | Methyl 3-(3-(4-((3-(trifluoromethyl)phenox y)methyl)phenoxy)azetidin-1-yl)-2-(1H-pyrrol-1-yl)benzoate | + |
| **250** | 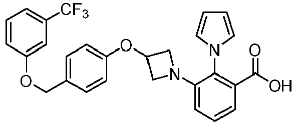 | 3-(3-(4-((3-(trifluoromethyl)phenox y)methyl)phenoxy)azetidin-1-yl)-2-(1H-pyrrol-1-yl)benzoic acid | ++ |
| **251** | 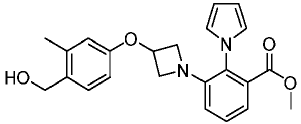 | Methyl 3-(3-(4-(hydroxymethyl)-3-methylp henoxy)azetidin-1-yl)-2-(1H-pyrrol -1-yl)benzoate | ++++ |
| **252** | 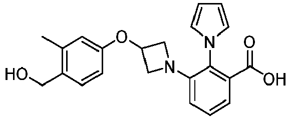 | 3-(3-(4-(hydroxymethyl)-3-methylp henoxy)azetidin-1-yl)-2-(1H-pyrrol -1-yl)benzoic acid | + |
| **253** | 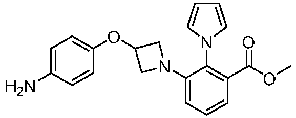 | Methyl 3-(3-(4-aminophenoxy)azetidin-1-y l)-2-(1H-pyrrol-1-yl)benzoate | ++ |
| **254** | 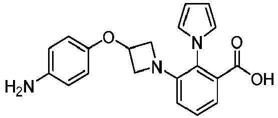 | 3-(3-(4-aminophenoxy)azetidin-1-y l)-2-(1H-pyrrol-1-yl)benzoic acid | + |
| **255** | 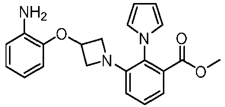 | Methyl 3-(3-(2-aminophenoxy)azetidin-1-y l)-2-(1H-pyrrol-1-yl)benzoate | ++++ |
| **256** | 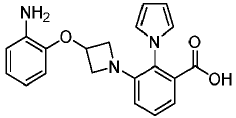 | 3-(3-(2-aminophenoxy)azetidin-1-y l)-2-(1H-pyrrol-1-yl)benzoic acid | ++++ |
| **257** | 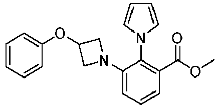 | Methyl 3-((3-phenoxy)azetidin-1-yl)-2-(1H -pyrrol-1-yl)benzoate | ++ |
| **258** | 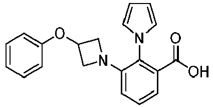 | 3-((3-phenoxy)azetidin-1-yl)-2-(1H -pyrrol-1-yl)benzoic acid | + |
| **259** | 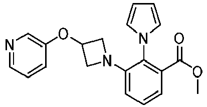 | Methyl 3-(3-(pyridin-3-yloxy)azetidin-1-yl )-2-(1H-pyrrol-1-yl)benzoate | ++ |
| **260** | 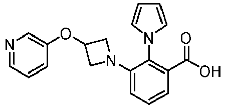 | 3-(3-(pyridin-3-yloxy)azetidin-1-yl )-2-(1H-pyrrol-1-yl)benzoic acid | + |
| **261** | 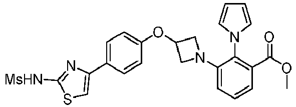 | Methyl 3-(3-(4-(2-(methylsulfonamido)thia zol-4-yl)phenoxy)azetidin-1-yl)-2-( 1H-pyrrol-1-yl)benzoate | + |
| **262** | 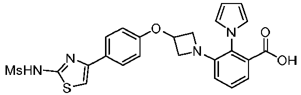 | 3-(3-(4-(2-(methylsulfonamido)thia zol-4-yl)phenoxy)azetidin-1-yl)-2-( 1H-pyrrol-1-yl)benzoic acid | + |
| **263** | 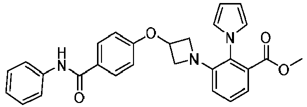 | Methyl 3-(3-(4-(phenylcarbamoyl)phenoxy )azetidin-1-yl)-2-(1H-pyrrol-1-yl)b enzoate | ++ |
| **264** | 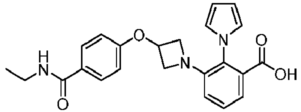 | 3-(3-(4-(ethylcarbamoyl)phenoxy)a zetidin-1-yl)-2-(1H-pyrrol-1-yl)ben zoic acid | + |
| **265** | 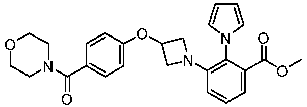 | Methyl 3-(3-(4-(morpholinyl-4-carbonyl)p henoxy)azetidin-1-yl)-2-(1H-pyrrol -1-yl)benzoate | ++ |
| **266** | 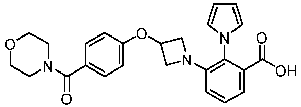 | 3-(3-(4-(morpholinyl-4-carbonyl)p henoxy)azetidin-1-yl)-2-(1H-pyrrol -1-yl)benzoic acid | + |
| **267** | 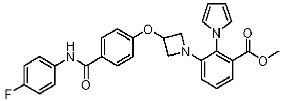 | Methyl 3-(3-(4-((4-fluorophenyl)carbamoyl )phenoxy)azetidin-1-yl)-2-(1H-pyrr ol-1-yl)benzoate | + |
| **268** | 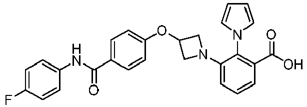 | 3-(3-(4-((4-fluorophenyl)carbamoyl )phenoxy)azetidin-1-yl)-2-(1H-pyrr ol-1-yl)benzoic acid | + |
| **269** | 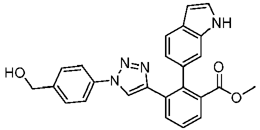 | Methyl 3-(1-(4-(hydroxymethyl)phenyl)-1 H-1,2,3-triazol-4-yl)-2-(1H-indol-6 -yl)benzoate | +++ |
| **270** | 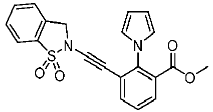 | Methyl 3-(1,1-dioxindibenzo[d]isothiazol-2 (3H)-yl)ethynyl)-2-(1H-pyrrol-1-yl )benzoate | + |
| **271** | 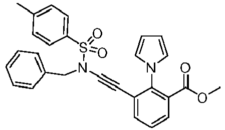 | Methyl 3-(((N-benzyl-4-methylphenyl)sulf onamido)ethynyl)-2-(1H-pyrrol-1-y l)benzoate | ++++ |
| **272** | 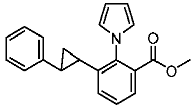 | Methyl 3-(2-phenylcyclopropyl)-2-(1H-pyr rol-1-yl)benzoate | ++ |
| **273** | 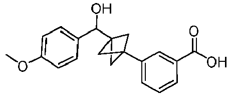 | 3-(3-(hydroxyl(4-methoxyphenyl) methyl)blcyclo[1.1.1]pentan-1-yl)b enzoic acid | + |
| **274** | 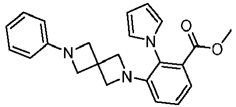 | Methyl 3-(6-phenyl-2,6-diazaspiro[3.3]hept an-2-yl)-2-(1H-pyrrol-1-yl)benzoat e | + |
| **275** | 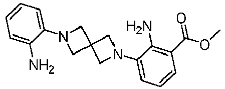 | Methyl 2-amino-3-(6-(2-aminophenyl)-2,6-diazaspiro[3.3]heptan-2-yl)benzoat e | ++ |
| **276** | 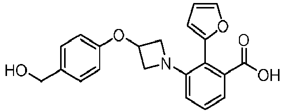 | 2-(furan-2-yl)-3-(3-(4-hydroxymeth yl)phenoxy)azetidin-1-yl)benzoic acid | + |
| **277** | 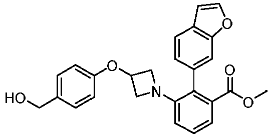 | Methyl 2-(benzofuran-6-yl)-3-(3-(4-hydrox ymethyl)phenoxy)azetidin-1-yl)ben zoate | ++ |
| **278** | 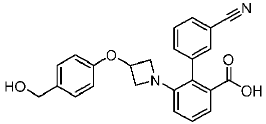 | 3'-cyano-6-(3-(4-hydroxymethyl)ph enoxy)azetidin-1-yl)-[1,1'-biphenyl ]-2-formic acid | ++ |
| **279** | 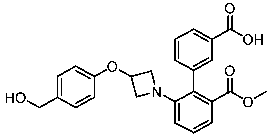 | Methyl 3'-carboxyl-6-(3-(4-hydroxymethyl )phenoxy)azetidin-1-yl)-[1,1'-biphe nyl]-2-formate | ++ |
| **280** | 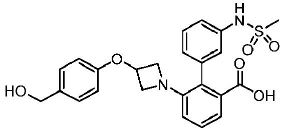 | 6-(3-(4-(hydroxymethyl)phenoxy)a zetidin-1-yl)-3'-(methylsulfonamid o)-[1,1'-biphenyl]-2-formic acid | + |
| **281** | 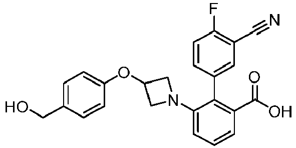 | 3'-cyano-4'-fluoro-6-(3-(4-(hydroxy methyl)phenoxy)azetidin-1-yl)-[1,1 '-biphenyl]-2-formic acid | + |
| **282** | 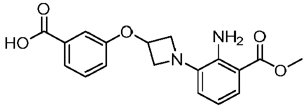 | Methyl 2-amino-3-(3-(3-carboxylphenoxy) azetidin-1-yl)benzoate | + |
| **283** | 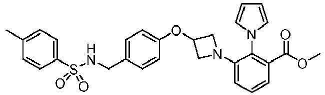 | Methyl 3-(3-(4-(((4-methylphenyl)sulfona mido)methyl)phenoxy)azetidin-1-yl )-2-(1H-pyrrol-1-yl)benzoate | ++++ |
| **284** | 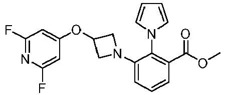 | Methyl 3-(3-((2,6-difluoropyridin-4-yl)oxy )azetidin-1-yl)-2-(1H-pyrrol-1-yl)b enzoate | + |
| **285** | 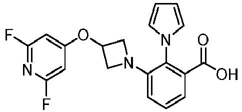 | 3-(3-((2,6-difluoropyridin-4-yl)oxy )azetidin-1-yl)-2-(1H-pyrrol-1-yl)b enzoic acid | + |
| **286** | 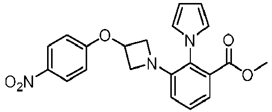 | Methyl 3-(3-(4-nitrophenoxy)azetidin-1-yl) -2-(1H-pyrrol-1-yl)benzoate | ++ |
| **287** | 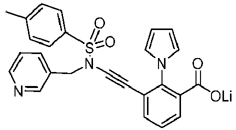 | Lithium 3-(((N-(pyridin-3-yl-methyl)-N-(4-methylphenyl)sulfonamido)ethynyl )-2-(1H-pyrrol-1-yl)benzoate | ++ |
| **288** | 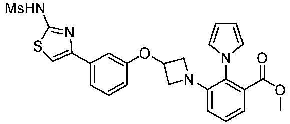 | Methyl 3-(3-(3-(2-(methylsulfonamido)thia zol-4-yl)phenoxy)azetidin-1-yl)-2-( 1H-pyrrol-1-yl)benzoate | + |
| **289** | 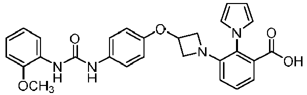 | 3-(3-(4-(3-(2-methoxyphenyl)ureid o)phenoxy)azetidin-1-yl)2-(1H-pyr rol-1-yl)benzoic acid | ++ |
| **290** | 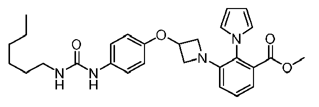 | 3-(3-(4-(3-hexylureido)phenoxy)az etidin-1-yl)2-(1H-pyrrol-1-yl)benzo ic acid | + |
| **291** | 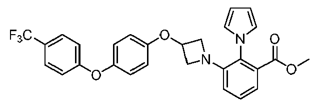 | Methyl 3-(3-(4-(4-(trifluoromethyl)phenox y)phenoxy)azetidin-1-yl)2-(1H-pyr rol-1-yl)benzoate | + |
| **292** | 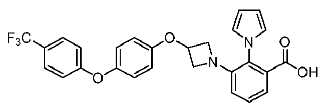 | 3-(3-(4-(4-(trifluoromethyl)phenox y)phenoxy)azetidin-1-yl)2-(1H-pyr rol-1-yl)benzoic acid | ++++ |
| **293** | 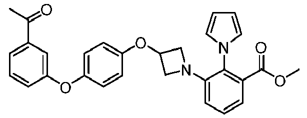 | Methyl 3-(3-(4-(3-acetylphenoxy)phenoxy) azetidin-1-yl)-2-(1H-pyrrol-1-yl)be nzoate | ++ |
| **294** | 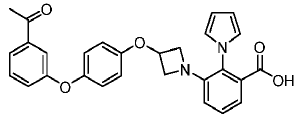 | 3-(3-(4-(3-acetylphenoxy)phenoxy) azetidin-1-yl)-2-(1H-pyrrol-1-yl)be nzoic acid | + |
| **295** | 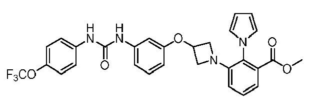 | Methyl 3-(3-(3-(3-(4-(trifluoromethoxy)ph enyl)ureido)phenoxy)azetidin-1-yl) -2-(1H-pyrrol-1-yl)benzoate | ++++ |
| **296** | 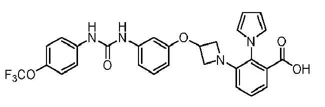 | 3-(3-(3-(3-(4-(trifluoromethoxy)ph enyl)ureido)phenoxy)azetidin-1-yl) -2-(1H-pyrrol-1-yl)benzoic acid | + |
| **297** | 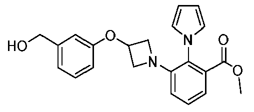 | Methyl 3-(3-(3-(hydroxymethyl)phenoxy)a zetidin-1-yl)-2-(1H-pyrrol-1-yl)ben zoate | |
| **298** | 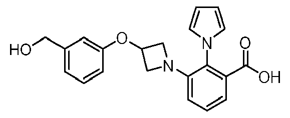 | 3-(3-(3-(hydroxymethyl)phenoxy)a zetidin-1-yl)-2-(1H-pyrrol-1-yl)ben zoic acid | + |
| **299** | 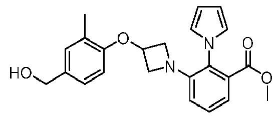 | Methyl 3-(3-(4-(hydroxymethyl)-2-methylp henoxy)azetidin-1-yl)-2-(1H-pyrrol -1-yl)benzoate | ++++ |
| **300** | 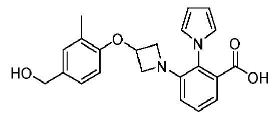 | 3-(3-(4-(hydroxymethyl)-2-methylp henoxy)azetidin-1-yl)-2-(1H-pyrrol -1-yl)benzoic acid | + |
| **301** | 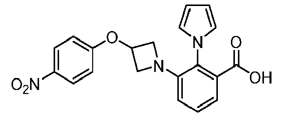 | 3-(3-(4-nitrophenoxy)azetidin-1-yl) -2-(1H-pyrrol-1-yl)benzoic acid | ++++ |
| **302** | 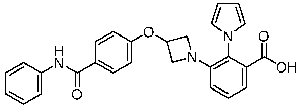 | 3-(3-(4-(phenylcarbamoyl)phenoxy )azetidin-1-yl)-2-(1H-pyrrol-1-yl)b enzoic acid | ++ |
| **303** | 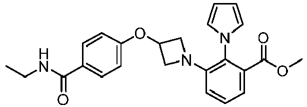 | Methyl 3-(3-(4-(ethylcarbamoyl)phenoxy)a zetidin-1-yl)-2-(1H-pyrrol-1-yl)ben zoate | ++ |
| **304** | 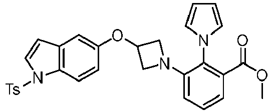 | Methyl 3-(3-((1-p-tolylsulfonyl-1H-indol-5 -yl)oxy)azetidin-1-yl)-2-(1H-pyrrol -1-yl)benzoate | + |
| **305** | 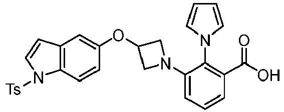 | 3-(3-((1-p-tolylsulfonyl-1H-indol-5 -yl)oxy)azetidin-1-yl)-2-(1H-pyrrol -1-yl)benzoic acid | + |
| **306** | 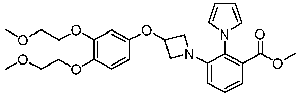 | Methyl 3-(3-(3,4-bis(2-methoxyethoxy)phe noxy)azetidin-1-yl)-2-(1H-pyrrol-1 -yl)benzoate | + |
| **307** | 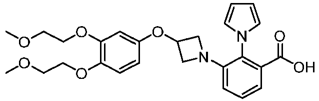 | 3-(3-(3,4-bis(2-methoxyethoxy)phe noxy)azetidin-1-yl)-2-(1H-pyrrol-1 -yl)benzoic acid | + |
| **308** | 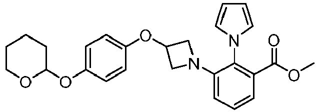 | Methyl 3-(3-(4-((tetrahydro-2H-pyran-2-yl) oxy)phenoxy)azetidin-1-yl)-2-(1H-pyrrol-1-yl)benzoate | + |
| **309** | 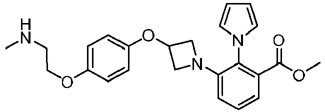 | Methyl 3-(3-(4-(2-(methylamino)ethoxy)ph enoxy)azetidin-1-yl)-2-(1H-pyrrol-1-yl)benzoate | ++ |
| **310** | 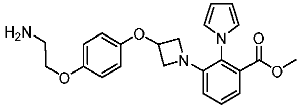 | Methyl 3-(3-(4-(2-aminoethoxy)phenoxy)a zetidin-1-yl)-2-(1H-pyrrol-1-yl)ben zoate | + |
| **311** | 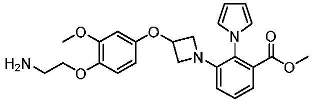 | Methyl 3-(3-(4-(2-aminoethoxy)-3-methox yphenoxy)azetidin-1-yl)-2-(1H-pyr rol-1-yl)benzoate | ++ |
| **312** | 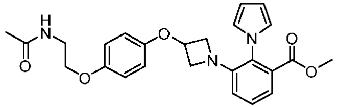 | Methyl 3-(3-(4-(2-acetylaminoethoxy)phen oxy)azetidin-1-yl)-2-(1H-pyrrol-1-yl)benzoate | +++ |
| **313** | 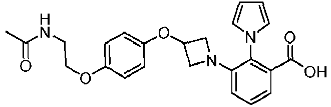 | 3-(3-(4-(2-acetylaminoethoxy)phen oxy)azetidin-1-yl)-2-(1H-pyrrol-1-yl)benzoic acid | + |
| **314** | 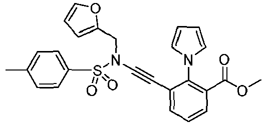 | Methyl 3-(((N-(furan-2-yl-methyl)-4-methy lphenyl)sulfonamido)ethynyl)-2-(1 H-pyrrol-1 -yl)benzoate | + |
| **315** | 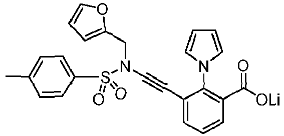 | Lithium 3-(((N-(furan-2-yl-methyl)-4-methy lphenyl)sulfonamido)ethynyl)-2-(1 H-pyrrol-1-yl)benzoate | ++++ |
| **316** | 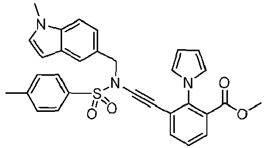 | Methyl 3-(((4-methyl-N-((1-methyl-1H-ind ol-5-yl)methyl)phenyl)sulfonamido )ethynyl)-2-(1H-pyrrol-1-yl)benzoa te | + |
| **317** | 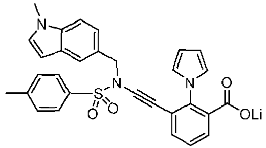 | Lithium 3-(((4-methyl-N-((1-methyl-1H-ind ol-5-yl)methyl)phenyl)sulfonamido )ethynyl)-2-(1H-pyrrol-1-yl)benzoa te | ++ |
| **318** | 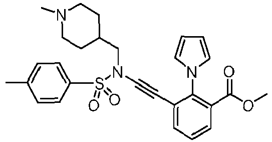 | Methyl 3-(((4-methyl-N-((1-methylpiperidi n-4-yl)methyl)phenyl)sulfonamido) ethynyl)-2-(1H-pyrrol-1-yl)benzoat e | ++++ |
| **319** | 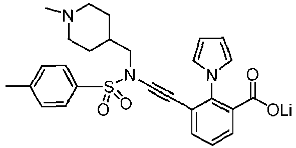 | Lithium 3-(((4-methyl-N-((1-methylpiperidi n-4-yl)methyl)phenyl)sulfonamido) ethynyl)-2-(1H-pyrrol-1-yl)benzoat e | ++++ |
| **320** | 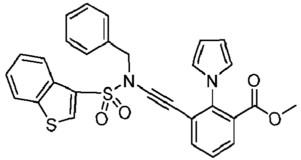 | Methyl 3-((N-benzylbenzo[b]thiophen-3-su lfonamido)ethynyl)-2-(1H-pyrrol-1 -yl)benzoate | + |
| **321** | 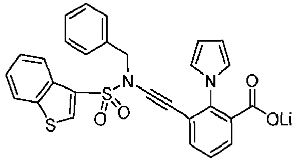 | Lithium 3-((N-benzylbenzo[b]thiophen-3-su lfonamido)ethynyl)-2-(1H-pyrrol-1 -yl)benzoate | + |
| **322** | 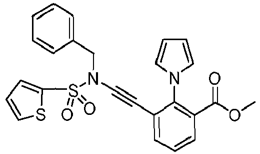 | Methyl 3-((N-benzylthiophen-2-sulfonamid o)ethynyl)-2-(1H-pyrrol-1-yl)benzo ate | ++ |
| **323** | 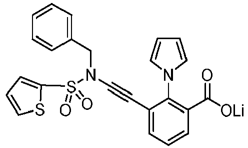 | Lithium 3-((N-benzylthiophen-2-sulfonamid o)ethynyl)-2-(1H-pyrrol-1-yl)benzo ate | + |
| **324** | 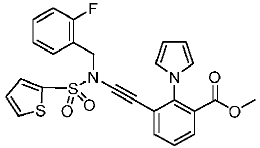 | Methyl 3-((N-(2-fluorobenzyl)thiophen-2-s ulfonamido)ethynyl)-2-(1H-pyrrol-1-yl)benzoate | ++ |
| **325** | 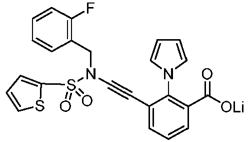 | Lithium 3-((N-(2-fluorobenzyl)thiophen-2-s ulfonamido)ethynyl)-2-(1H-pyrrol-1-yl)benzoate | +++ |
| **326** | 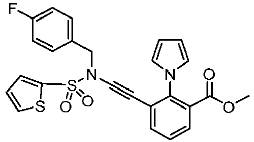 | Methyl 3-((N-(4-fluorobenzyl)thiophen-2-s ulfonamido)ethynyl)-2-(1H-pyrrol-1-yl)benzoate | +++ |
| **327** | 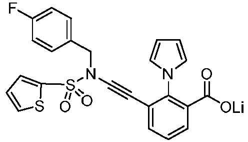 | Lithium 3-((N-(4-fluorobenzyl)thiophen-2-s ulfonamido)ethynyl)-2-(1H-pyrrol-1-yl)benzoate | ++++ |
| **328** | 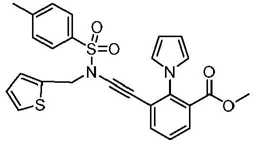 | Methyl 3-(((4-methyl-N-(thiophen-2-yl-me thyl)phenyl)sulfonamido)ethynyl)-2-(1H-pyrrol-1-yl)benzoate | ++ |
| **329** | 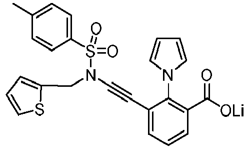 | Lithium 3-(((4-methyl-N-(thiophen-2-yl-me thyl)phenyl)sulfonamido)ethynyl)-2-(1H-pyrrol-1-yl)benzoate | ++++ |
| **330** | 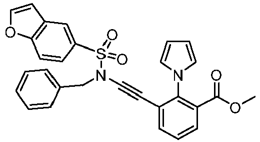 | Methyl 3-((N-benzylbenzofuran-5-sulfona mido)ethynyl)-2-(1H-pyrrol-1-yl)b enzoate | ++++ |
| **331** | 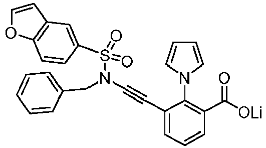 | Lithium 3-((N-benzylbenzofuran-5-sulfona mido)ethynyl)-2-(1H-pyrrol-1-yl)b enzoate | + |
| **332** | 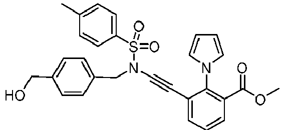 | Methyl 3-(((N-(4-(hydroxymethyl)benzyl)-4-methylphenyl)sulfonamido)ethyn yl)-2-(1H-pyrrol-1-yl)benzoate | ++++ |
| **333** | 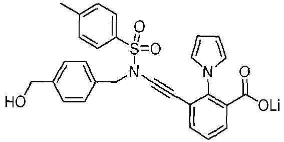 | Lithium 3-(((N-(4-(hydroxymethyl)benzyl)-4-methylphenyl)sulfonamido)ethyn yl)-2-(1H-pyrrol-1-yl)benzoate | ++++ |
| **334** | 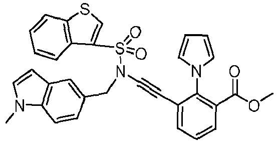 | Methyl 3-((N-((1-methyl-1H-indol-5-yl)me thyl)benzo [b]thiophen-3-sulfonami do)ethynyl)-2-(1H-pyrrol-1-yl)benz oate | ++++ |
| **335** | 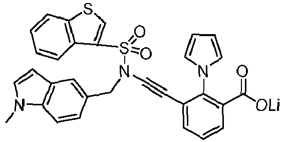 | Lithium 3-((N-((1-methyl-1H-indol-5-yl)me thyl)benzo [b]thiophen-3-sulfonami do)ethynyl)-2-(1H-pyrrol-1-yl)benz oate | + |
| **336** | 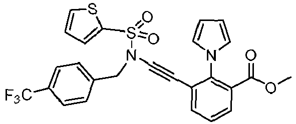 | Methyl 3-((N-(4-(trifluoromethyl)benzyl)th iophen-2-sulfonamido)ethynyl)-2-( 1H-pyrrol-1-yl)benzoate | + |
| **337** | 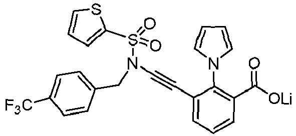 | Lithium 3 -((N-(4-(trifluoromethyl)benzyl)th iophen-2-sulfonamido)ethynyl)-2-( 1H-pyrrol-1-yl)benzoate | ++ |
| **338** | 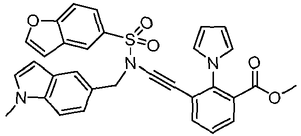 | Methyl 3-((N-((1-methyl-1H-indol-5-yl)me thyl)benzofuran-5-sulfonamido)eth ynyl)-2-(1H-pyrrol-1-yl)benzoate | + |
| **339** | 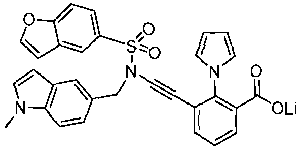 | Lithium 3-((N-((1-methyl-1H-indol-5-yl)me thyl)benzofuran-5-sulfonamido)eth ynyl)-2-(1H-pyrrol-1-yl)benzoate | + |
| **340** | 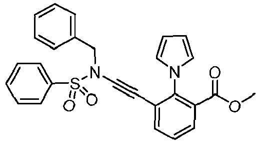 | Methyl 3-((N-benzyl-N-phenylsulfonamido )ethynyl)-2-(1H-pyrrol-1-yl)benzoa te | + |
| **341** | 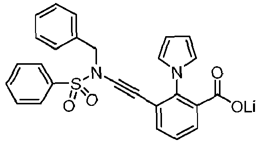 | 3-((N-benzyl-N-phenylsulfonamido )ethynyl)-2-(1H-pyrrol-1-yl)benzoi c acid | + |
| **342** | 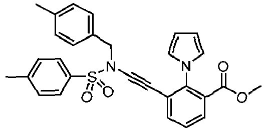 | Methyl 3-(((N-(4-methylbenzyl)-N-(4-tolyl sulfonamido)ethynyl)-2-(1H-pyrrol -1-yl)benzoate | + |
| **343** | 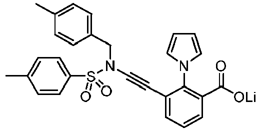 | Lithium 3-(((N-(4-methylbenzyl)-N-(4-tolyl sulfonamido)ethynyl)-2-(1H-pyrrol -1-yl)benzoate | + |
| **344** | 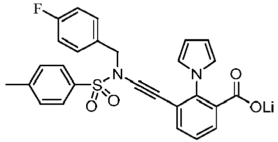 | Methyl 3-(((N-(4-fluorobenzyl)-4-tolylsulf onamido)ethynyl)-2-(1H-pyrrol-1-y l)benzoate | + |
| **345** | 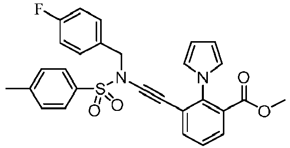 | Lithium 3-(((N-(4-fluorobenzyl)-4-tolylsulf onamido)ethynyl)-2-(1H-pyrrol-1-y l)benzoate | ++ |
| **346** | 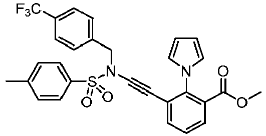 | Methyl 3-(((N-(4-(trifluoromethyl)benzyl)-N-(4-tolylsulfonamido)ethynyl)-2-( 1H-pyrrol-1-yl)benzoate | ++ |
| **347** | 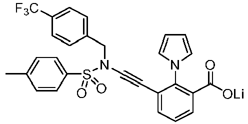 | Lithium 3-(((N-(4-(trifluoromethyl)benzyl)-(4-tolylsulfonamido)ethynyl)-2-(1H -pyrrol-1-yl)benzoate | +++ |
| **348** | 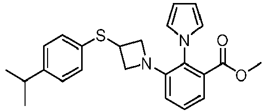 | Methyl 3-(3-((4-isopropylphenyl)thio)azeti din-1-yl)-2-(1H-pyrrol-1-yl)benzoa te | + |
| **349** | 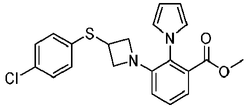 | Methyl 3-(3-((4-chlorophenyl)thio)azetidin -1-yl)-2-(1H-pyrrol-1-yl)benzoate | + |
| **350** | 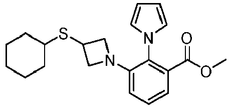 | Methyl 3-(3-(cyclohexylthio)azetidin-1-yl)-2-(1H-pyrrol-1-yl)benzoate | + |
| **351** | 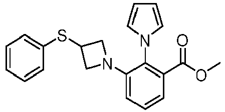 | Methyl 3-(3-(phenylthio)azetidin-1-yl)-2-(1 H-pyrrol-1-yl)benzoate | + |
| **352** | 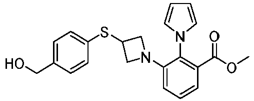 | Methyl 3-(3-((4-(hydroxymethyl)phenyl)thi o)azetidin-1-yl)-2-(1H-pyrrol-1-yl) benzoate | ++++ |
| **353** | 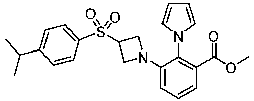 | Methyl 3-(3-((4-isopropylphenyl)sulfonyl)a zetidin-1-yl)-2-(1H-pyrrol-1-yl)ben zoate | ++ |
| **354** | 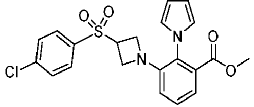 | Methyl 3-(3-((4-chlorophenyl)sulfonyl)azet idin-1-yl)-2-(1H-pyrrol-1-yl)benzo ate | ++ |
| **355** | 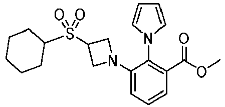 | Methyl 3-(3-(cyclohexylsulfonyl)azetidin-1 -yl)-2-(1H-pyrrol-1-yl)benzoate | ++ |
| **356** | 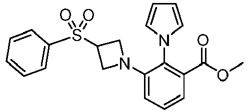 | Methyl 3-(3-(phenylsulfonyl)azetidin-1-yl) -2-(1H-pyrrol-1-yl)benzoate | + |
| **357** | 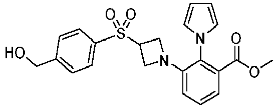 | Methyl 3-(3-((4-(hydroxymethyl)phenyl)su lfonyl)azetidin-1-yl)-2-(1H-pyrrol-1-yl)benzoate | + |
| **358** | 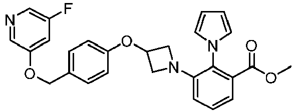 | Methyl 3-(3-(4-(((5-fluoropyridin-3-yl)oxy )methyl)phenoxy)azetidin-1-yl)-2-( 1H-pyrrol-1-yl)benzoate | ++ |
| **359** | 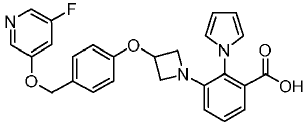 | 3-(3-(4-(((5-fluoropyridin-3-yl)oxy )methyl)phenoxy)azetidin-1-yl)-2-( 1H-pyrrol-1-yl)benzoic acid | + |
| **360** | 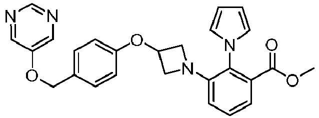 | Methyl 3-(3-(4-((pyrimidin-5-yl-oxy)methy l)phenoxy)azetidin-1-yl)-2-(1H-pyr rol-1-yl)benzoate | + |
| **361** | 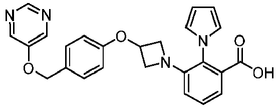 | 3-(3-(4-((pyrimidin-5-yl-oxy)methy l)phenoxy)azetidin-1-yl)-2-(1H-pyr rol-1-yl)benzoic acid | + |
| **362** | 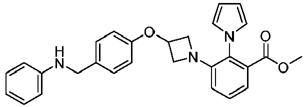 | Methyl 3-(3-(4-(phenylamino)methyl)phen oxy)azetidin-1-yl)-2-(1H-pyrrol-1-yl)benzoate | + |
| **363** | 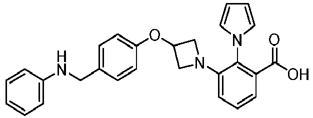 | 3-(3-(4-(phenylamino)methyl)phen oxy)azetidin-1-yl)-2-(1H-pyrrol-1-yl)benzoic acid | + |
| **364** | 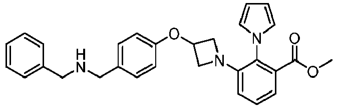 | Methyl 3-(3-(4-(benzylamino)methyl)phen oxy)azetidin-1-yl)-2-(1H-pyrrol-1-yl)benzoate | + |
| **365** | 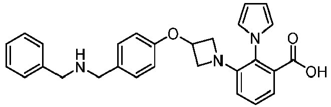 | 3-(3-(4-(benzylamino)methyl)phen oxy)azetidin-1-yl)-2-(1H-pyrrol-1-yl)benzoic acid | + |
| **366** | 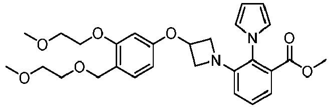 | Methyl 3-(3-(3-(2-methoxyethoxy)-4-((2-m ethoxyethoxy)methyl)phenoxy)azet idin-1-yl)-2-(1H-pyrrol-1-yl)benzo ate | ++ |
| **367** | 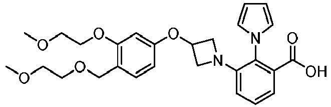 | 3-(3-(3-(2-methoxyethoxy)-4-((2-m ethoxyethoxy)methyl)phenoxy)azet idin-1-yl)-2-(1H-pyrrol-1-yl)benzoi c acid | + |
| **368** | 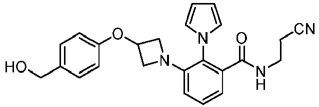 | N-(2-cyanoethyl)-3-(3-(4-(hydroxy methyl)phenoxy)azetidin-1-yl)-2-(1 H-pyrrol-1-yl)benzamide | ++ |
| **369** | 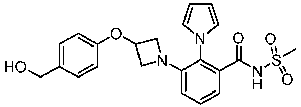 | N-methylsulfonyl-(3-(3-(4-(hydrox ymethyl)phenoxy)azetidin-1-yl)-2-( 1H-pyrrol-1-yl))benzamide | + |
| **370** | 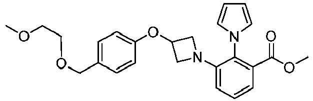 | Methyl 3-(3-(4-(2-methoxyethoxy)methyl) phenoxy)azetidin-1-yl)-2-(1H-pyrr ol-1-yl)benzoate | ++ |
| **371** | 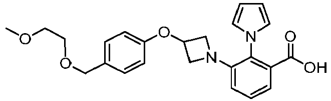 | 3-(3-(4-(2-methoxyethoxy)methyl) phenoxy)azetidin-1-yl)-2-(1H-pyrr ol-1-yl)benzoic acid | ++ |
| **372** | 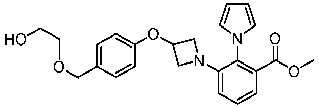 | 3-(3-(4-(2-hydroxylethoxy)methyl) phenoxy)azetidin-1-yl)-2-(1H-pyrr ol-1-yl)benzoic acid | ++ |
| **373** | 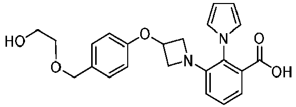 | 3-(3-(4-(2-hydroxylethoxy)methyl) phenoxy)azetidin-1-yl)-2-(1H-pyrr ol-1-yl)benzoic acid | + |
| **374** | 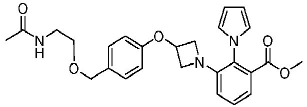 | Methyl 3-(3-(4-(2-acetylaminoethoxy)meth yl)phenoxy)azetidin-1-yl)-2-(1H-py rrol-1-yl)benzoate | ++ |
| **375** | 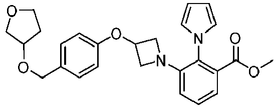 | Methyl 3-(3-(4-(tetrahydrofuran-3 -yl)oxym ethyl)phenoxy)azetidin-1-yl)-2-(1H -pyrrol-1 -yl)benzoate | + |
| **376** | 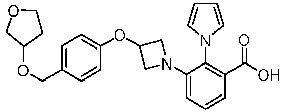 | 3-(3-(4-(tetrahydrofuran-3 -yl)oxym ethyl)phenoxy)azetidin-1-yl)-2-(1H -pyrrol-1-yl)benzoic acid | ++ |
| **377** | 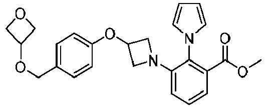 | Methyl 3-(3-(4-((oxetanyl-3-oxy)methyl)ph enoxy)azetidin-1-yl)-2-(1H-pyrrol-1-yl)benzoate | ++++ |
| **378** | 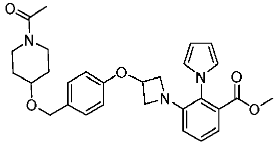 | Methyl 3-(3-(4-(1-acetylpiperidin-4-yl)oxy methyl)phenoxy(azetidin-1-yl)-2-(1 H-pyrrol-1-yl)benzoate | + |
| **379** | 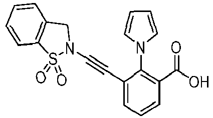 | 3-(1,1-dioxindibenzo[d]isothiazol-2 (3H)-yl)ethynyl)-2-(1H-pyrrol-1-yl )benzoic acid | ++++ |
| **380** | 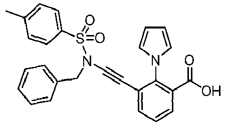 | 3-(((N-benzyl-4-methylphenyl)sulf onamido)ethynyl)-2-(1H-pyrrol-1-y l)benzoic acid | + |
| **381** | 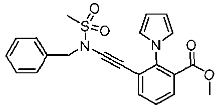 | Methyl 3-((N-benzylmethylsulfonamido)et hynyl)-2-(1H-pyrrol-1-yl)benzoate | +++ |
| **382** | 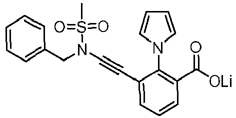 | Lithium 3-((N-benzylmethylsulfonamido)et hynyl)-2-(1H-pyrrol-1-yl)benzoate | + |
| **383** | 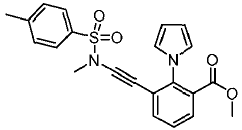 | Methyl 3-(((N-methyl-4-methylphenylsulfo namido)ethynyl)-2-(1H-pyrrol-1-yl )benzoate | ++ |
| **384** | 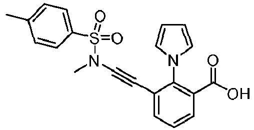 | Methyl 3-(((N-methyl-4-methylphenylsulfo namido)ethynyl)-2-(1H-pyrrol-1-yl )benzoate | + |
| **385** | 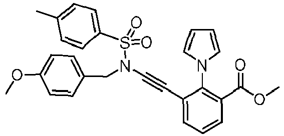 | Methyl 3-(((N-(4-methoxybenzyl)-4-methy lphenylsulfonamido)ethynyl)-2-(1H -pyrrol-1-yl)benzoate | ++ |
| **386** | 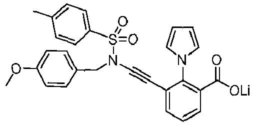 | Lithium 3-(((N-(4-methoxybenzyl)-4-methy lphenylsulfonamido)ethynyl)-2-(1H -pyrrol-1-yl)benzoate | + |
| **387** | 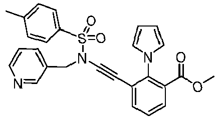 | Methyl 3-(((N-(pyridin-3-yl-methyl)-N-(4-methylphenyl)sulfonamido)ethynyl )-2-(1H-pyrrol-1-yl)benzoate | ++++ |
| **388** | 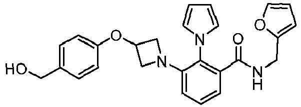 | N-(furan-2-yl-methyl)-3-(3-(4-(hyd roxymethyl)phenoxy)azetidin-1-yl) -2-(1H-pyrrol-1-yl)benzamide | + |
| **389** | 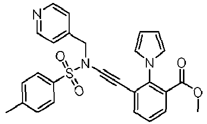 | Methyl 3-(((N-(pyridin-4-yl-methyl)-N-(4-methylphenyl)sulfonamido)ethynyl )-2-(1H-pyrrol-1-yl)benzoate | ++++ |
| **390** | 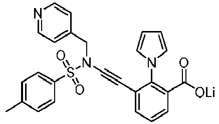 | Lithium 3-(((N-(pyridin-4-yl-methyl)-N-(4-methylphenyl)sulfonamido)ethynyl )-2-(1H-pyrrol-1-yl)benzoate | ++ |
| **391** | 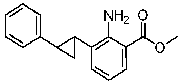 | Methyl 2-amino-3-(2-phenylcyclopropyl)b enzoate | + |
| **392** | 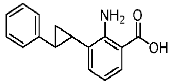 | 2-amino-3-(2-phenylcyclopropyl)b enzoic acid | + |
| **393** | 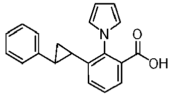 | 3-(2-phenylcyclopropyl)-2-(1H-pyr rol-1-yl)benzoic acid | + |
| **394** | 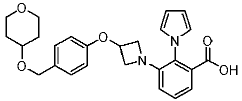 | 2-(1H-pyrrol-1-yl)-3-(3-(4-(((tetrah ydro-2H-pyran-4-yl)oxy)methyl)ph enoxy)azetidin-1-yl)benzoic acid | + |
| **395** | 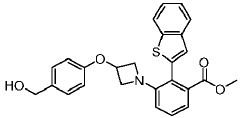 | Methyl 2-(benzo[b]thiophen-2-yl)-3-(3-(4-( hydroxymethyl)phenoxy)azetidin-1 -yl)benzoate | ++ |
| **396** | 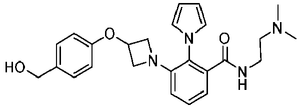 | N-(2-dimethylamino)ethyl)-3 -(3 -(4 -(hydroxymethyl)phenoxy)azetidin-1-yl)-2-(1H-pyrrol-1-yl)benzamide | + |

| | | | |
|---|---|---|---|
| *Inh%: <10% +; 10%-30% ++; 30%-50% +++; >50% ++++ | | | |

The data in the above table indicates that some compounds of the present invention have inhibitory activity against EBV-positive C666-1 cells under a certain concentration of drug. C666-1 cells belong to nasopharyngeal carcinoma cells, and are positive for Epstein-Barr virus and express EBNA1.

**Table-II: Experimental results of the inhibition of C666-1 cell viability by the molecules of some examples**

| **Example Number** | **Inhibition Rate^{*1}** | **EC₅₀ Quantitation^{*2}** |
|---|---|---|
| **146** | ++ | + |
| **147** | +++ | +++ |
| **152** | + | + |
| **153** | ++ | ++ |
| **154** | ++ | + |
| **189** | ++ | ++ |
| **197** | ++ | + |
| **234** | ++++ | ++++ |
| **263** | ++ | + |
| **264** | + | + |
| **266** | + | + |
| **292** | ++++ | ++++ |
| **302** | ++ | + |
| **381** | +++ | + |
| **383** | ++++ | ++++ |
| **383** | ++ | + |

| | | |
|---|---|---|
| ^{∗}1 Inh%: <10% +; 10%-30% ++; 30%-50% +++; >50% ++++ ^{∗}2 EC₅₀: <50uM ++++; 50-75uM +++; 75-100uM ++; >100uM + | | |

The data in the above table indicates that the compound of the present invention can effectively inhibit the proliferation activity of C666-1 cells in a dose-dependent manner, and is related to the single-concentration inhibition rate test.

Although the technology has been described with reference to specific exemplary embodiments, it should be understood that the present invention as claimed should not be unduly limited to such specific embodiments. In fact, it will be apparent to those skilled in the art that other embodiments and modifications of the present invention can be conceived without departing from the true spirit and scope of the present invention. It is intended that the appended claims be interpreted to encompass all such embodiments and equivalents.

## Claims

1. A compound having a general formula (I), or an enantiomer, a diastereomer, a tautomer, a salt, a crystalline form, a solvate and/or an isotopically substituted derivative thereof: 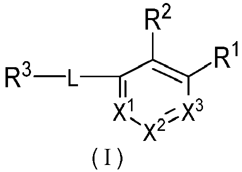 wherein:
X¹, X², and X³ are the same or different, and are each independently selected from CR^{xa} or a nitrogen atom;
R^{xa} is selected from hydrogen or halogen, the halogen being selected from the group of consisting of: fluorine, chlorine, bromine and iodine;
R¹ is selected from the group of consisting of: -C(=O)-O-R^{1a}, -C(=O)-NH₂, -C(=O)-NHR^{1b}, -C(=O)-NR^{1b}R^{1c}, -C(=O)-NH-S(=O)₂-R^{1b}, -C(=O)-NH-(CH₂)_{q}-R^{1d}, 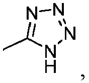 -S(=O)₂OH, -B(OH)₂, -S(=O)₂-NH₂, -S(=O)₂-NHR^{1b}, and -S(=O)₂-NR^{1b}R^{1c},
q is 0, 1, 2 or 3;
wherein, R^{1a} is independently selected from Li; or
R^{1a}, R^{1b}, and R^{1c} are the same or different, and are each independently selected from the group of consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R^{1a}, R^{1b}, and R^{1c} are the same or different, and are each independently selected from the group of consisting of: aryl, heterocyclyl and heteroaryl;
the aryl is selected from the group of consisting of phenyl, naphthyl and anthryl;
the heterocyclyl is selected from: 4-7 membered saturated heterocyclyl containing oxygen, nitrogen, and/or sulfur, the saturated heterocyclyl being selected from the group of consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group of consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently; or
R^{1b} and R^{1c}, together with an atom to which they are attached, form a cyclic group, the cyclic group being selected from the group consisting of: optionally substituted morpholinyl, optionally substituted pyrrolidinyl, and optionally substituted piperazinyl;
R^{1d} is selected from the group consisting of: hydrogen, hydroxyl, amino, cyano, -O-C(=O)-R¹⁷, -O-R¹², -NR⁹R¹⁰, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
R² is selected from the following groups:
hydrogen, amino, halogen, nitro, optionally substituted C1-C4 alkyl, 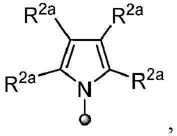 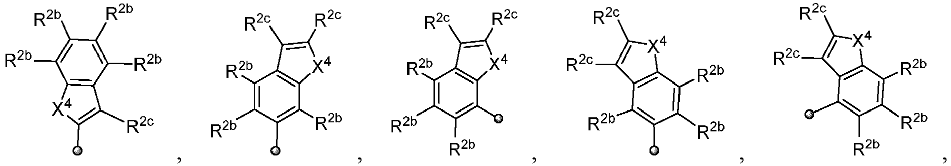 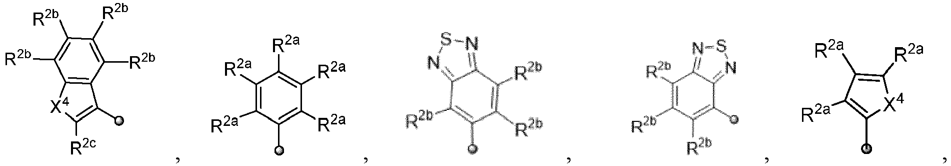 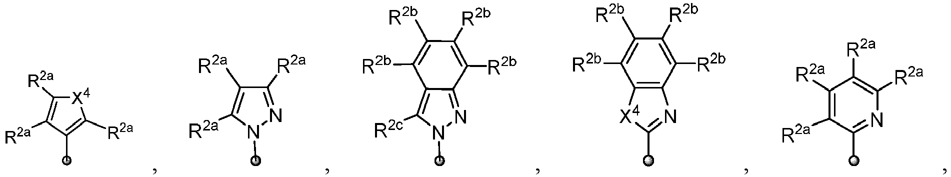 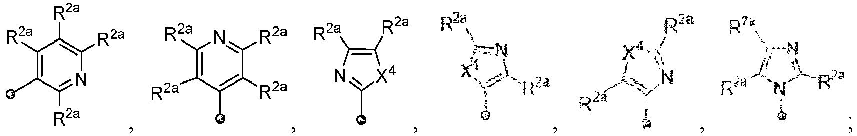
wherein a dot ● represents a junction of the R² group and other group of a compound molecule of the general formula (I);
X⁴ is selected from the group consisting of O, S, and NR^{2d};
wherein R^{2a}, R^{2b}, R^{2c} are the same or different, and are each independently selected from the group consisting of:
hydrogen, halogen, amino, nitro, hydroxyl, C1-C8 hydroxyalkyl, cyano, -(CH₂)_{q}-COOH, -(CH₂)_{q}-C(=O)-O-R⁸, -(CH₂)_{q}-C(=O)-NH-R⁸, -N(H)C(=O)-R⁸, -N(H)S(=O)₂-R⁸, -O-R⁸, -NH-R⁸, -S(=O)₂NH-R⁸, -S(=O)₂-R⁸, -C(=O)-R⁸, -O-C(=O)-R⁸, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, and phenyl; the phenyl being optionally substituted by a substituent R⁴ for one or more times identically or differently;
q is 0, 1, 2 or 3; or
R^{2a} is selected from the group consisting of aryl, heterocyclyl and heteroaryl:
the aryl is selected from: phenyl, naphthyl, anthryl;
the heterocyclyl is selected from: 4-7 membered saturated heterocyclyl containing oxygen, nitrogen, and/or sulfur, the saturated heterocyclyl being selected from: oxetanyl, tetrahydrofuranyl, tetrahydropyranyl, oxepanyl, azetidinyl, pyrrolidinyl, piperidinyl, azepanyl, morpholinyl, piperazinyl;
the heteroaryl is selected from: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl;
the aryl, the heterocyclyl, and the heteroaryl are optionally substituted by a substituent R⁴ for one or more times identically or differently; or
the adjacent R^{2a} groups, together with an atom to which they are attached, form a fused ring structure, or, the adjacent R^{2b} groups, together with the atoms to which they are attached, form a fused ring structure, the fused ring structure being selected from phenyl or heteroaryl; the heteroaryl being selected from the group consisting of: thienyl, furyl, pyrrolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the phenyl and the heteroaryl are optionally substituted by a substituent R⁴ for one or more times identically or differently;
R^{2d} is selected from the group consisting of hydrogen, optionally halogenated or non-halogenated C1-C3 straight chain alkyl, isopropyl, and cyclopropyl; or
R^{2d} is selected from: -C(=O)-R⁵, or -S(=O)₂-R⁵; or
R^{2d} is selected from the group consisting of aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
L is selected from the following groups: 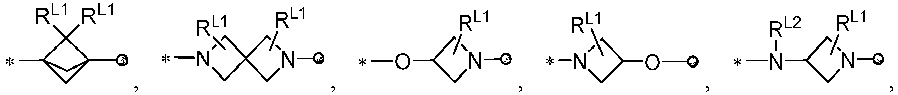 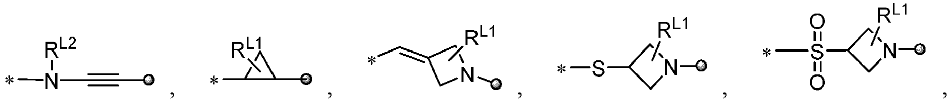 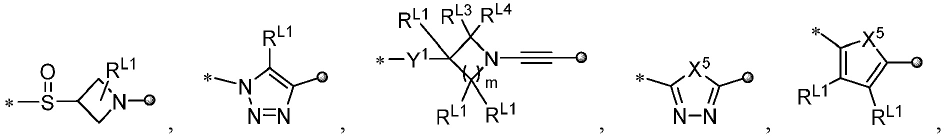 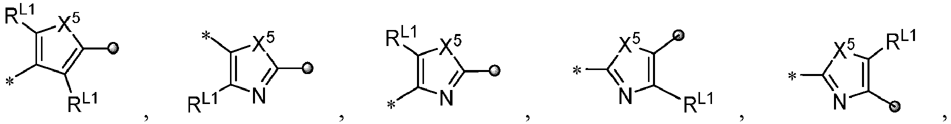 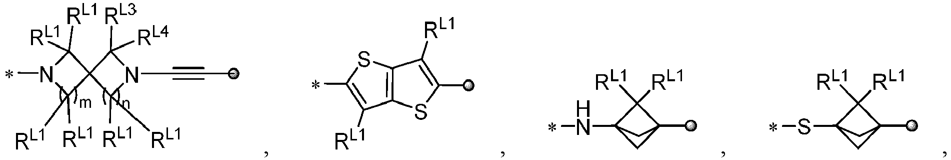 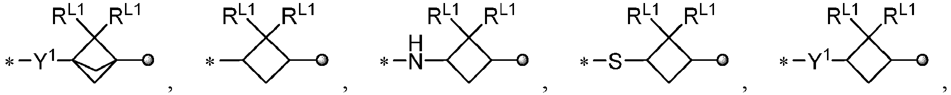 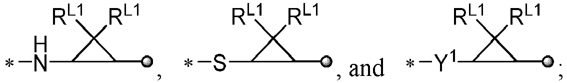
wherein a dot ● represents a junction where L is attached to a fragment on a right side of a compound of the general formula (I);
wherein an asterisk * represents a junction where L is attached to R³;
X⁵ is selected from the group consisting of: O, NH and S;
Y¹ is selected from: O or NR^{L2};
the L is substituted by a substituent R^{L1} for one or more times at the same or different positions;
R^{L1} is selected from: a hydrogen atom or a fluorine atom;
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R^{L2} is selected from the group consisting of: -(CH₂)_{q}-R⁷, -C(=O)-N(H)-R¹⁹, -C(=S)-N(H)-R¹⁹, -C(=O)-R²⁰, and -S(=O)₂-R²⁰; or
R^{L2} is selected from the group consisting of: aryl-(CH₂)_{q}-, heterocyclyl-(CH₂)_{q}-, and heteroaryl-(CH₂)_{q}-;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{L2} and R³ are cyclized together with an atom to which they are attached to form 5-10 membered heterocyclyl containing nitrogen and/or sulfur, the 5-10 membered heterocyclyl containing nitrogen and/or sulfur including -C(=O)- and -S(=O)₂- groups;
R^{L3} and R^{L4} are the same or different, and are each independently selected from: a hydrogen atom or a fluorine atom; or
R^{L3} and R^{L4}, together with an atom to which they are attached, form carbonyl, or thiocarbonyl; or
R^{L3} and R^{L4}, together with the atom to which they are attached, form a heterocyclyl containing nitrogen and/or sulfur, wherein C atom attached to both R^{L3} and R^{L4} is replaced by -S(=O)₂-;
m and n are the same or different, and are each independently selected from: 1, 2 or 3, so as to form a ring system with various numbers of atoms;
q is 0, 1, 2 or 3;
R³ is selected from the following groups:
C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl; the C1-C8 straight chain alkyl, the C3-C8 branched chain alkyl, the C3-C8 cycloalkyl; the halogenated C1-C8 straight chain alkyl, the halogenated C3-C8 branched chain alkyl, and the halogenated C3-C8 cycloalkyl being optionally substituted by a substituent R⁶ for one or more times identically or differently; or
R³ is selected from the following groups:
(1) 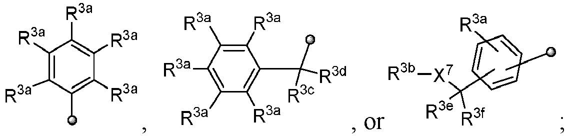 or
(2) 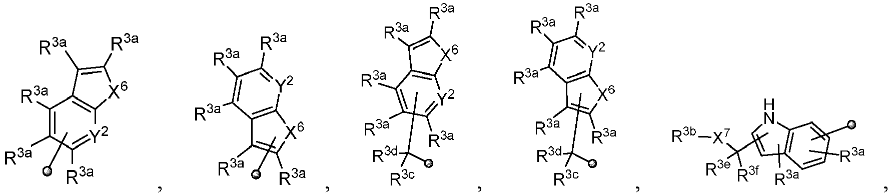 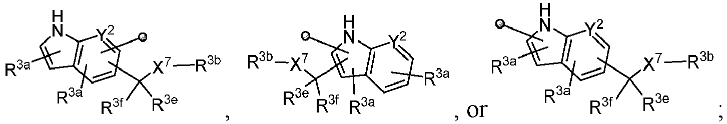 or
(3) 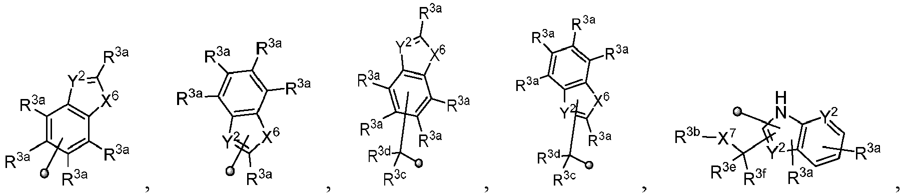 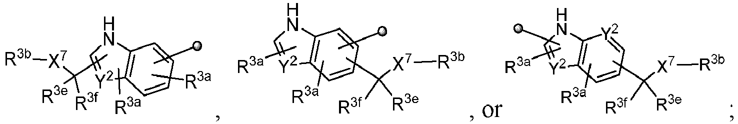 or
(4) 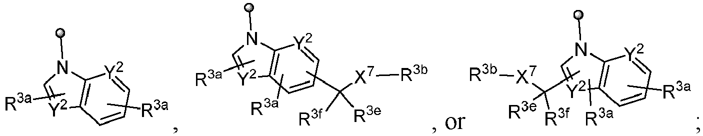 or
(5) 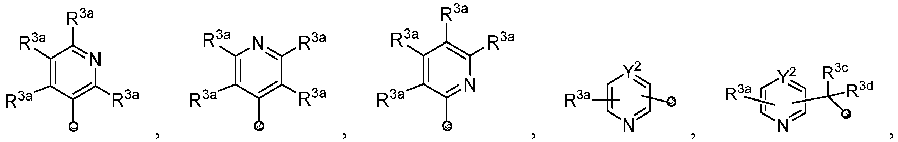 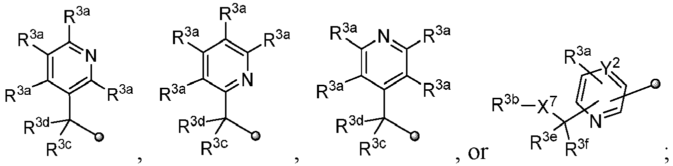 or
(6) 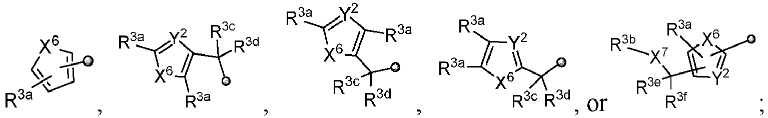 or
(7) 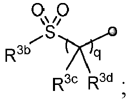 or
(8) 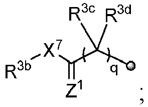
wherein a dot ● represents a junction which is attached to a fragment on a right side of the compound of the general formula (I);
X⁶ is selected from the group consisting of O, S and NR^{3g};
X⁷ is selected from the group consisting of O, S, NR^{3g} and CHR^{3a};
Y² is selected from the group consisting of CH, CR^{3a} and N, and Y² may replace a C atom at an optional position of an aromatic ring;
Z¹ is selected from the group consisting of O, S and NR^{3h};
wherein R^{3a} is selected from the group consisting of: hydrogen, halogen, nitro, cyano, sulfo-group, optionally substituted sulfonamido, optionally substituted C1-C8 straight chain alkyl, optionally substituted C3-C8 branched chain alkyl, optionally substituted C3-C8 cycloalkyl, optionally substituted halogenated C1 -C8 straight chain alkyl, optionally substituted halogenated C3-C8 branched chain alkyl, optionally substituted halogenated C3-C8 cycloalkyl, hydroxyl, C1-C8 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)_{P}-R¹¹, aryl-(CH₂)_{q}-, heterocyclyl-(CH₂)_{q}-, heteroaryl-(CH₂)_{q}-; the C1-C8 hydroxyalkyl being C1-C8 alkyl substituted with hydroxyl;
p and q are each independently 0, 1, 2 or 3;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
adjacent R^{3a} groups together with an atom to which they are attached form a cyclic structure, or R^{3a} and its adjacent R^{3c} together with an atom to which they are attached form a cyclic structure, or R^{3a} and its adjacent R^{3d} together with an atom to which they are attached form a cyclic structure, the cyclic structure comprising a saturated or aromatic cyclic structure, the cyclic structure being selected from the group consisting of: cycloalkane, aryl, heterocyclyl and heteroaryl;
preferably, the cyclic structure is selected from the group consisting of: phenyl, pyridyl, pyrrolyl, thienyl, furyl, oxazolyl, thiazolyl, isoxazolyl, indazolyl, and 5-7 membered saturated heterocyclyl containing nitrogen/oxygen, the saturated heterocyclyl being selected from the group consisting of: tetrahydrofuranyl, tetrahydropyranyl, oxepanyl, pyrrolidinyl, piperidinyl, azepanyl, morpholinyl and piperazinyl;
the cyclic structure is optionally substituted by a substituent R¹⁸ for one or more times identically or differently;
R^{3b} is selected from the group consisting of:
hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S)N(H)-R¹⁹, -C(=O)O-R¹⁹, and -S(=O)₂-R²⁰;
q is 0, 1, 2 or 3; or
R^{3b} is selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3c} and R^{3d} are the same or different, and are each independently selected from the group consisting of: hydrogen, fluorine, C1-C8 alkyl, aryl-(CH₂)_{q}-, heterocyclyl-(CH₂)_{q}-, and heteroaryl-(CH₂)_{q}-; or
when L is selected from the group consisting of 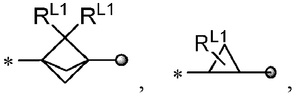 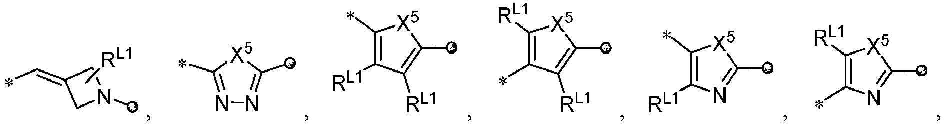 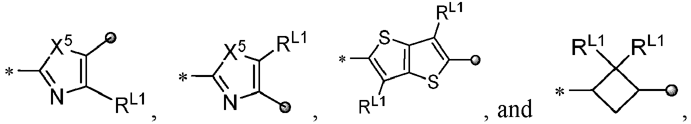 R^{3c} and R^{3d} are the same or different, and are each independently selected from the group consisting of: hydrogen, fluorine, C1-C8 alkyl, hydroxyl, C1-C8 hydroxyalkyl, -O-C(=O)-R²⁰, aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently; or
R^{3c} and R^{3d} together with an atom to which they are attached form carbonyl, thiocarbonyl, or imino;
R^{3e} and R^{3f} are the same or different, and are each independently selected from the group consisting of:
hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -C(=O)-R²⁰, -C(=O)N(H)-R¹⁹, -C(=S) N(H)-R¹⁹, -C(=O)O-R¹⁹, and -S(=O)₂-R²⁰; or
R^{3e} and R^{3f} together with an atom to which they are attached form carbonyl, or thiocarbonyl; or
R^{3e} and R^{3f} are the same or different, and are each independently selected from the group consisting of: aryl-(CH₂)_{q}-, heterocyclyl-(CH₂)_{q}-, and heteroaryl-(CH₂)_{q}-;
the aryl is selected from: optionally substituted phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
R^{3h} is selected from the group consisting of: hydrogen, nitro and cyano;
the R⁴ is selected from the group consisting of: hydrogen, fluorine, chlorine, hydroxyl, C1-C3 hydroxyalkyl, optionally substituted C1-C3 alkyl, optionally substituted halogenated C1-C3 alkyl, optionally substituted C1-C3 alkoxy, optionally substituted halogenated C1-C3 alkoxy, nitro, cyano, amino, acetyl, methylsulfonyl, acetamido, and methylsulfonamido;
the R⁵ is selected from the group consisting of: hydrogen, optionally substituted C1-C3 alkyl, and optionally substituted halogenated C1-C3 alkyl;
the R⁶ is selected from the group consisting of: hydroxy, C1-C8 straight chain alkoxy, C3-C8 branched chain alkoxy, C3-C8 cycloalkoxy, -O-C(=O)-R²⁰, amino, -NH-C(=O)-R²⁰, -NH-S(=O)₂-R²⁰, -NH-C(=O)-O-R²⁰, -NH-C(=O)-NH-R²⁰, and -NH-C(=S)-NH-R²⁰;
the R⁷ is selected from the group consisting of: phenyl, naphthyl, heterocyclyl, and heteroaryl; the heterocyclyl being selected from: 4-7 membered saturated heterocyclyl containing oxygen, nitrogen, and/or sulfur; the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the phenyl, the naphthyl, the heterocyclyl, and the heteroaryl being optionally substituted by a substituent R¹⁸ for one or more times identically or differently;
the R⁸ is selected from the group consisting of: optionally substituted C1-C3 alkyl, and optionally substituted halogenated C1-C3 alkyl;
the R⁹ and R¹⁰ are the same or different, and are each independently selected from: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
R⁹ and R¹⁰ are the same or different, and are each independently selected from the group consisting of: optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl; the heterocyclyl containing nitrogen being substituted by the following substituents for one or more times identically or differently: hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, and optionally substituted sulfonamido;
the R¹¹ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
R¹¹ is selected from the group consisting of: aryl, heterocyclyl and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl;
the aryl, the heterocyclyl and the heteroaryl are substituted by the following substituents for one or more times identically or differently: halogen, hydroxy, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, amino, optionally substituted amido, optionally substituted sulfonamido, cyano, nitro, sulfo-group, optionally substituted ureido, and guanidyl;
the R¹² is selected from the group consisting of: hydrogen, C1-C8 straight chain alkyl, C3-C8 branched chain alkyl, C3-C8 cycloalkyl, halogenated C1-C8 straight chain alkyl, halogenated C3-C8 branched chain alkyl, halogenated C3-C8 cycloalkyl, aryl, heterocyclyl, and heteroaryl; the C1-C8 alkyl being substituted by the following substituents for one or more times identically or differently: -O-R²⁰, -OH, -N(H)C(=O)-R²⁰, and -NR⁹R¹⁰;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R¹³ is selected from the group consisting of: hydrogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, and optionally substituted halogenated C3-C8 cycloalkyl or alkoxy; or
the R¹³ is selected from the group consisting of: -(CH₂)_{q}-phenyl, -(CH₂)_{q}-halogenated phenyl, -(CH₂)_{q}-naphthyl, -(CH₂)_{q}-halogenated naphthyl, -(CH₂)_{q}-anthryl, -(CH₂)_{q}-halogenated anthryl, -(CH₂)_{q}-heterocyclyl, -(CH₂)_{q}-halogenated heterocyclyl, -(CH₂)_{q}-heteroaryl, and -(CH₂)_{q}-halogenated heteroaryl;
q is 0, 1, 2 or 3;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the phenyl, the naphthyl, the anthryl, the heterocyclyl, and the heteroaryl are optionally substituted by a substituent R¹⁸ for one or more times identically or differently;
the R¹⁴ is selected from: heteroaryl, the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the heteroaryl being optionally substituted by a substituent R¹⁸ for one or more times identically or differently;
the R¹⁵ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently;
the R¹⁶ is selected from the group consisting of: hydrogen, optionally substituted C1-C8 straight chain alkyl, optionally substituted C3-C8 branched chain or cycloalkyl, optionally substituted halogenated C1-C8 straight chain alkyl, optionally substituted halogenated C3-C8 branched chain or cycloalkyl, optionally substituted acyl, and optionally substituted sulfonyl; or
R¹⁶ is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl; the saturated heterocyclyl being optionally substituted by a substituent R¹⁸ for one or more times identically or differently; or
R¹⁶ is selected from the group consisting of: aryl-(CH₂)_{q}-, and heteroaryl-(CH₂)_{q}-; the aryl being selected from the group consisting of: phenyl, naphthyl and anthryl; the heteroaryl being selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl; the aryl and the heteroaryl being optionally substituted by a substituent R¹⁸ for one or more times identically or differently;
q is 0, 1, 2 or 3;
the R¹⁷ is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, optionally substituted aryl, optionally substituted heterocyclyl, and optionally substituted heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the R¹⁸ is selected from the group consisting of: hydrogen, halogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, nitro, optionally substituted amino, hydroxy, C1-C8 hydroxyalkyl, aryloxy, heterocyclyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R⁴ for one or more times identically or differently;
the R¹⁹ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
the R²⁰ is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl, heterocyclyl, and heteroaryl;
the aryl is selected from the group consisting of: phenyl, naphthyl and anthryl;
the heterocyclyl is selected from: 4-7 membered saturated heterocyclyl containing oxygen, nitrogen, and/or sulfur, the saturated heterocyclyl being selected from the group consisting of: oxetanyl, tetrahydrofuranyl, tetrahydropyranyl, oxepanyl, azetidinyl, pyrrolidinyl, piperidinyl, azepanyl, morpholinyl, and piperazinyl;
the heteroaryl is selected from the group consisting of: thienyl, furyl, pyrrolyl, pyrazolyl, imidazolyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl;
the aryl, the heterocyclyl, and the heteroaryl are optionally substituted by a substituent R¹⁸ for one or more times identically or differently;
wherein, p and q are each independently 0, 1, 2 or 3;
m and n are the same or different, and are each independently selected from: 1, 2 or 3, so as to form a ring system with various numbers of atoms.

2. The compound, or the enantiomer, the diastereomer, the tautomer, the salt, the crystalline form, the solvate and/or the isotopically substituted derivative thereof according to claim 1, being at least one compound selected from the following formulae:

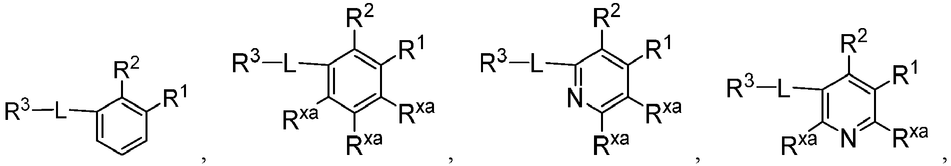

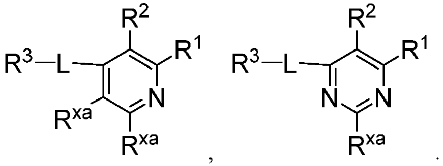

3. The compound, or the enantiomer, the diastereomer, the tautomer, the salt, the crystalline form, the solvate and/or the isotopically substituted derivative thereof according to claim 1, wherein
R² is selected from the following groups:
hydrogen, amino, nitro, 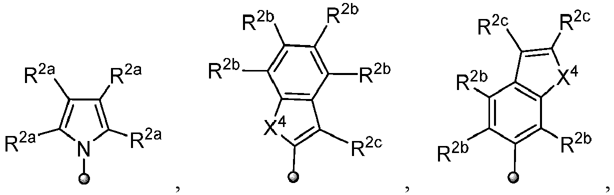 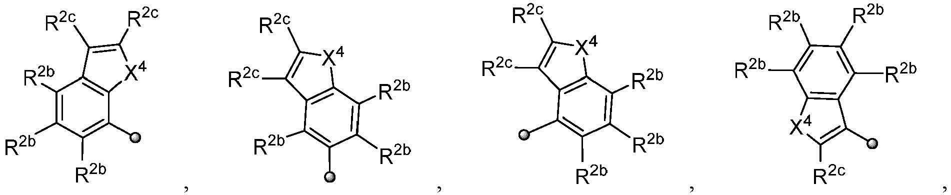 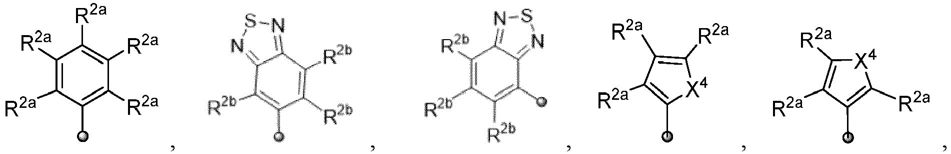 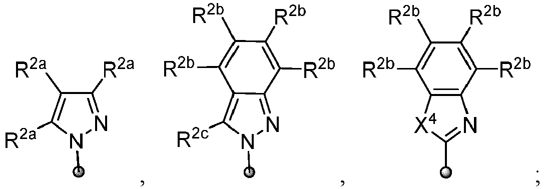
wherein a dot ● represents a junction where the R² group is attached to other fragments of the compound of the general formula (I);
X⁴ is selected from the group consisting of O, S and NR^{2d};
wherein R^{2a}, R^{2b}, and R^{2c} are the same or different, and are each independently selected from the group consisting of:
hydrogen, halogen, amino, nitro, hydroxyl, C1-C8 hydroxyalkyl, cyano, -(CH₂)_{q}-COOH, -(CH₂)_{q}-C(=O)-O-R⁸, -(CH₂)_{q}-C(=O)-NH-R⁸, -N(H)C(=O)-R⁸, -N(H)S(=O)₂-R⁸, -O-R⁸, -NH-R⁸, -S(=O)₂NH-R⁸, -S(=O)₂-R⁸, -C(=O)-R⁸, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, and phenyl; the phenyl being optionally substituted by a substituent R⁴ for one or more times identically or differently;
q is 0, 1, 2 or 3;
R^{2d} is selected from the group consisting of: hydrogen, optionally fluorinated or non-fluorinated C1-C3 straight chain alkyl, isopropyl, cyclopropyl, -C(=O)-R⁵, and -S(=O)₂-R⁵.

4. The compound, or the enantiomer, the diastereomer, the tautomer, the salt, the crystalline form, the solvate and/or the isotopically substituted derivative thereof according to claim 1, wherein
L is selected from the following groups: 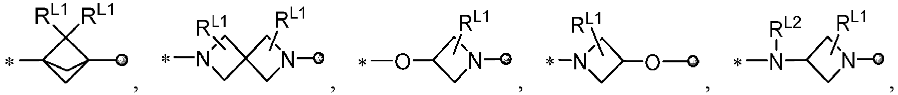 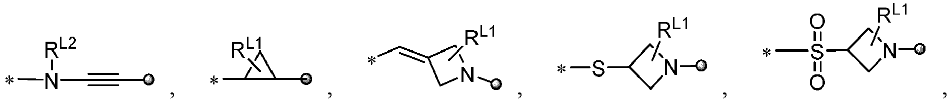 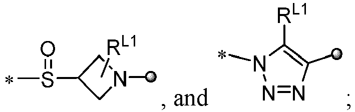
wherein a dot ● represents a junction where L is attached to a fragment on the right side of the compound of the general formula (I);
wherein an asterisk * represents a junction where L is attached to R³;
the L is substituted by a substituent R^{L1} for one or more times at the same or different positions;
R^{L1} is selected from: a hydrogen atom or a fluorine atom;
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R^{L2} is selected from the group consisting of: -(CH₂)_{q}-R⁷, -C(=O)N(H)-R¹⁹, -C(=S)N(H)-R¹⁹, -C(=O)-R²⁰, and -S(=O)₂-R²⁰; or
R^{L2} and R³ are cyclized together with an atom to which they are attached to form 5-10 membered heterocyclyl containing nitrogen and/or sulfur, the 5-10 membered heterocyclyl containing nitrogen and/or sulfur comprising -C(=O)-, -S(=O)₂- group;
q is 0, 1, 2 or 3;
the R⁷ is selected from: phenyl, heterocyclyl, heteroaryl, the heterocyclyl being selected from: 4-7 membered saturated heterocyclyl containing oxygen, nitrogen the saturated heterocyclyl being selected from the group consisting of: oxetanyl, tetrahydrofuranyl, tetrahydropyranyl, azetidinyl, pyrrolidinyl, piperidinyl, morpholinyl, piperazinyl; the heteroaryl being selected from: thienyl, furyl, pyrrolyl, pyridyl, pyrimidinyl, indolyl, quinolinyl, quinazolinyl, benzofuryl, benzothienyl, benzoxazolyl, and benzothiazolyl; the phenyl, the heterocyclyl, and the heteroaryl being optionally substituted by a substituent R¹⁸ for one or more times identically or differently;
preferably, R¹⁸ is selected from the group consisting of: hydrogen, F, Cl, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, nitro, optionally substituted amino, hydroxy, C1-C8 hydroxyalkyl, phenyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from: phenyl;
the heterocyclyl is selected from: 4-7 membered saturated heterocyclyl containing oxygen, nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R⁴ for one or more times identically or differently.

5. The compound, or the enantiomer, the diastereomer, the tautomer, the salt, the crystalline form, the solvate and/or the isotopically substituted derivative thereof according to claim 1, wherein
R³ is selected from the following groups:
(1) 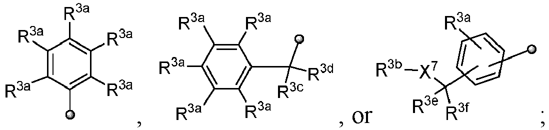 or
(2) 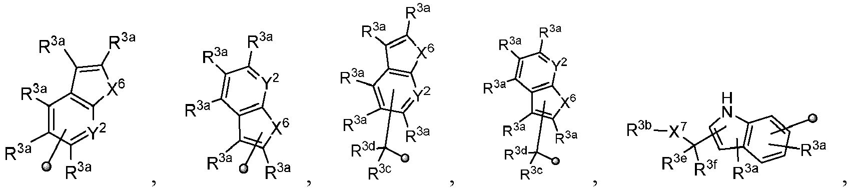 or 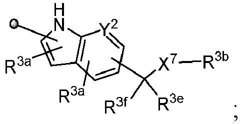 or
(5) 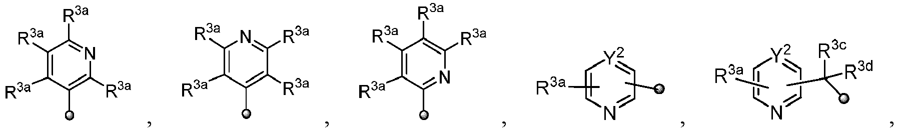 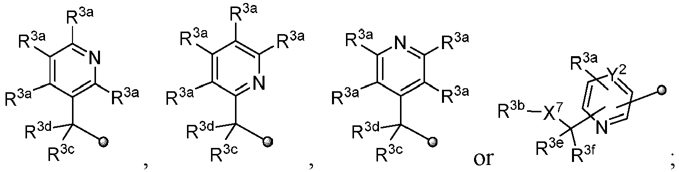 or
(6) 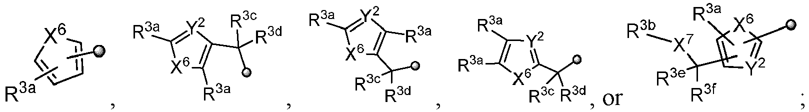 or
(7) 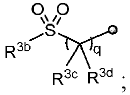 or
(8) 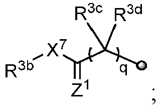
wherein a dot ● represents a junction which is attached to a fragment on the right side of the compound of the general formula (I);
X⁶ is selected from O, S or NR^{3g};
X⁷ is selected from the group consisting of O, S, NR^{3g} and CHR^{3a};
Y² is selected from CH, CR^{3a} or N; and Y² may replace a C atom at an optional position of an aromatic ring;
Z¹ is selected from O, S or NR^{3h};
wherein R^{3a} is selected from the group consisting of:
hydrogen, halogen, nitro, cyano, sulfo-group, optionally substituted sulfonamido, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH2)ₚ-R¹¹, -(CH₂)_{q}-S-(CH₂)ₚ-R¹¹, -C(=O)-R²⁰, ^{_}C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
the aryl is selected from: phenyl;
the heterocyclyl is selected from the group consisting of: 4-7 membered saturated heterocyclyl containing oxygen, nitrogen; the saturated heterocyclyl is selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
p and q are each independently 0, 1, 2 or 3;
R^{3b} is selected from the group consisting of:
hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
q is 0, 1, 2 or 3;
R^{3c} and R^{3d} are the same or different, and are each independently selected from the group consisting of: hydrogen, fluorine and C1-C8 alkyl; or
when L is selected from 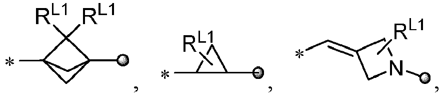 R^{3c} and R^{3d} are the same or different, and are each independently selected from the group consisting of: hydrogen, fluorine, C1-C8 alkyl, hydroxyl, C1-C8 hydroxyalkyl, and -O-C(=O)-R²⁰;
preferably, R^{3c} and R^{3d} are the same or different, and are each independently selected from the group consisting of: hydrogen, fluorine, C1-C8 alkyl, hydroxyl, C1-C8 hydroxyalkyl, and -O-C(=O)-R⁸; or
R^{3c} and R^{3d} together with an atom to which they are attached form carbonyl, thiocarbonyl, imino;
R^{3e} and R^{3f} are the same or different, and are each independently selected from the group consisting of:
hydrogen, C1-C8 straight chain alkyl, C3-C8 branched chain alkyl, C3-C8 cycloalkyl, halogenated C1-C8 straight chain alkyl, halogenated C3-C8 branched chain alkyl, halogenated C3-C8 cycloalkyl, aryl-(CH₂)_{q}-, and heteroaryl-(CH₂)_{q}-;
the aryl and the heteroaryl are optionally substituted by a substituent R¹⁸ for one or more times identically or differently, or
R^{3e} and R^{3f} together with an atom to which they are attached form carbonyl, thiocarbonyl.

6. The compound, or the enantiomer, the diastereomer, the tautomer, the salt, the crystalline form, the solvate and/or the isotopically substituted derivative thereof according to claim 1, wherein R¹ is selected from the group consisting of -COOH, -C(=O)-O-R^{1a}, -C(=O)-NH-S(=O)₂-R^{1b}, -C(=O)-NH-(CH₂)_{q}-R^{1d}, and 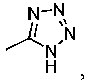 wherein, R^{1a} is selected from: Li; or
R^{1a}, R^{1b}, and R^{1c} are the same or different, and are each independently selected from the group consisting of: hydrogen, optionally substituted C1-C4 straight chain alkyl, optionally substituted C3-C4 branched chain or cycloalkyl, optionally substituted halogenated C1-C4 straight chain alkyl, and optionally substituted halogenated C3-C4 branched chain or cycloalkyl;
R^{1d} is selected from the group consisting of hydroxyl, amino, cyano, -O-R¹², -NR⁹R¹⁰, aryl, heterocyclyl and heteroaryl;
preferably, the aryl is selected from: phenyl;
the heterocyclyl is selected from: azetidinyl or pyrrolidinyl;
the heteroaryl is selected from: thienyl or furyl;
the R⁹ and R¹⁰ are the same or different, and are each independently selected from the group consisting of: hydrogen, optionally substituted C1-C4 alkyl, optionally substituted halogenated C1-C4 alkyl, optionally substituted C1-C4 alkoxy, optionally substituted halogenated C1-C4 alkoxy; or
R⁹ and R¹⁰ are the same or different, and are each independently selected from the group consisting of: optionally substituted aryl, optionally substituted heterocyclyl, and optionally substituted heteroaryl;
the aryl is selected from: phenyl;
the heterocyclyl is selected from: 4-7 membered saturated heterocyclyl containing oxygen, nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from: thienyl, furyl, pyrrolyl, pyridyl, pyrimidinyl, indolyl, quinolinyl, quinazolinyl, benzofuryl, benzothienyl, benzoxazolyl, benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl.

7. The compound, or the enantiomer, the diastereomer, the tautomer, the salt, the crystalline form, the solvate and/or the isotopically substituted derivative thereof according to claim 1, wherein R² is selected from the group consisting of: hydrogen, amino, nitro, optionally substituted pyrrolyl, optionally substituted benzofuryl, optionally substituted indolyl, optionally substituted benzothienyl, optionally substituted phenyl, optionally substituted benzothiadiazolyl, optionally substituted furyl, optionally substituted pyrazolyl, optionally substituted indazolyl, optionally substituted benzothiazolyl, optionally substituted pyridyl, optionally substituted imidazolyl, and optionally substituted thienyl.

8. The compound, or the enantiomer, the diastereomer, the tautomer, the salt, the crystalline form, the solvate and/or the isotopically substituted derivative thereof according to claim 7, wherein:
R² is selected from the group consisting of: hydrogen, amino, nitro, pyrrolyl, benzofuryl, indolyl, benzo[b]thienyl, phenyl, halophenyl, cyanophenyl, cyanohalophenyl, carboxyphenyl, halogenated C1-C4 alkylphenyl, biphenyl, methylsulfonamidophenyl, acetylphenyl, methanesulfonylphenyl, aminosulfonylphenyl, C1-C4 alkoxyphenyl, benzo[c][1,2,5]thiadiazolyl, furyl, pyrazolyl, indazolyl, and benzo[d]thiazolyl.

9. The compound, or the enantiomer, the diastereomer, the tautomer, the salt, the crystalline form, the solvate and/or the isotopically substituted derivative thereof according to claim 1, wherein
L is selected from: 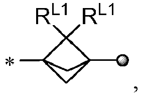
R² is selected from the group consisting of: hydrogen, 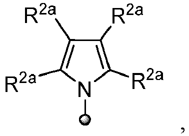 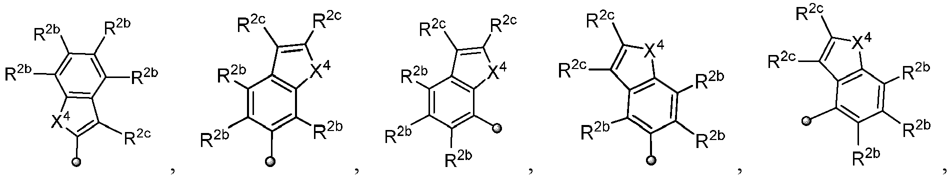 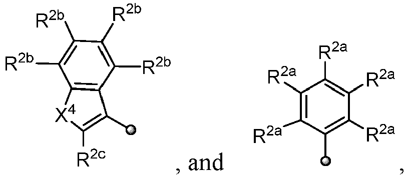 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 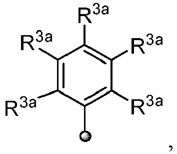 wherein R^{3a} is selected from the group consisting of: hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the aryl, the heterocyclyl, and the heteroaryl being optionally substituted by a substituent R⁴ for one or more times identically or differently; or
R³ is selected from: 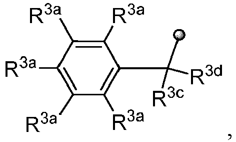 wherein
R^{3a} is selected from the group consisting of: hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the phenyl, the heterocyclyl, and the heteroaryl being optionally substituted by a substituent R⁴ for one or more times identically or differently;
R^{3c} and R^{3d} are the same or different, and are each independently selected from the group consisting of: hydrogen, hydroxyl, C1-C8 hydroxyalkyl, and -O-C(=O)-R²⁰; or
R³ is selected from the group consisting of: 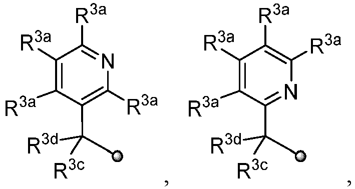 and 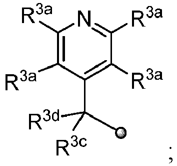 wherein
R^{3a} is selected from the group consisting of: hydrogen, halogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, -(CH₂)_{q}-O-R¹², aryl-(CH₂)_{q}-, heterocyclyl-(CH₂)_{q}-, and heteroaryl-(CH₂)_{q}-; the phenyl, the heterocyclyl, and the heteroaryl being optionally substituted by a substituent R⁴ for one or more times identically or differently;
R^{3c} and R^{3d} are the same or different, and are each independently selected from the group consisting of: hydrogen, hydroxyl, C1-C8 hydroxyalkyl, and -O-C(=O)-R²⁰.

10. The compound, or the enantiomer, the diastereomer, the tautomer, the salt, the crystalline form, the solvate and/or the isotopically substituted derivative thereof according to claim 1, wherein
L is selected from: 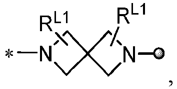
R² is selected from the group consisting of: hydrogen, amino, nitro, 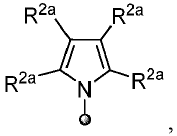 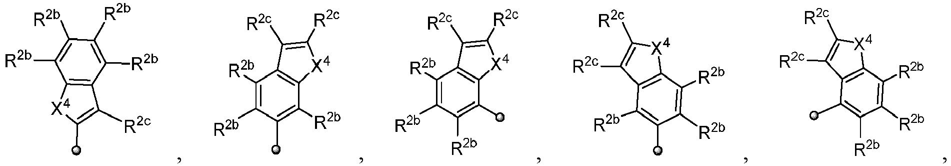 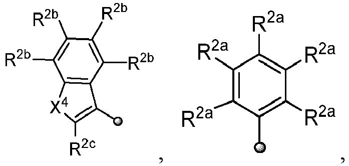 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 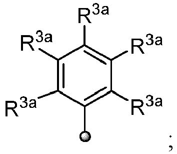 wherein,
R^{3a} is selected from the group consisting of: hydrogen, -(CH₂)_{q}-NR⁹R¹⁰, nitro, C1-C8 straight chain alkyl, C3-C8 branched chain alkyl, halogenated C1-C8 straight chain alkyl, halogenated C3-C8 branched chain alkyl, -(CH₂)_{q}-COOH, -C(=O)-O-R²⁰, -(CH₂)_{q}-NH-C(=O)-R¹¹, and -(CH₂)_{q}-NR¹⁶-S(=O)₂-R¹⁷.

11. The compound, or the enantiomer, the diastereomer, the tautomer, the salt, the crystalline form, the solvate and/or the isotopically substituted derivative thereof according to claim 1, wherein
L is selected from: 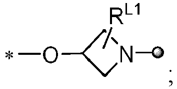
R² is selected from the group consisting of: hydrogen, amino, halogen, optionally substituted C1-C4 alkyl, 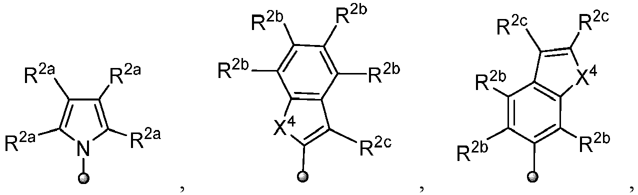 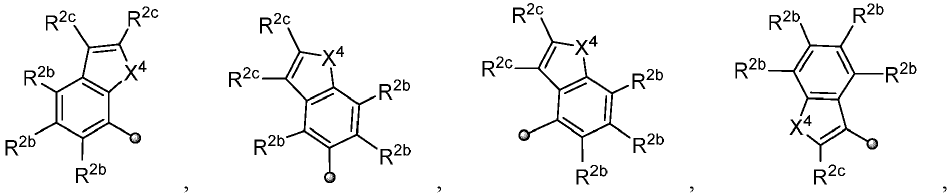 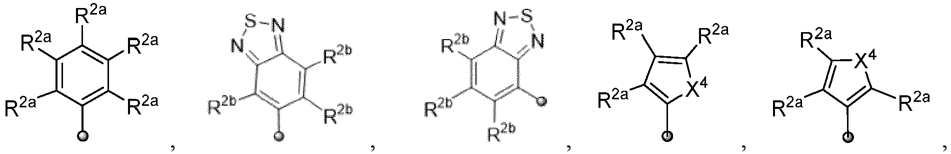 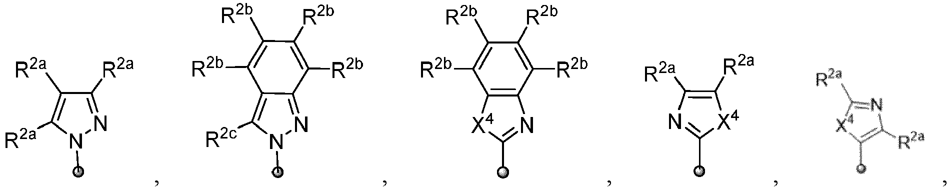 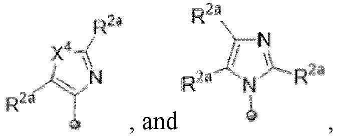
X⁴ is selected from the group consisting of O, S and NR^{2d};
wherein R^{2a}, R^{2b}, and R^{2c} are the same or different, and are each independently selected from the group consisting of:
hydrogen, halogen, cyano, -(CH₂)_{q}-COOH, -O-R⁸, -N(H)S(=O)₂-R⁸, -S(=O)₂-R⁸, -C(=O)-R⁸, -O-C(=O)-R⁸, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenyl; the phenyl being optionally substituted by a substituent R⁴ for one or more times identically or differently;
R² is selected from the group consisting of: hydrogen, amino, halogen, pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl; the pyrrolyl, benzofuryl, indolyl, benzothienyl, phenyl, benzothiadiazolyl, furyl, pyrazolyl, indazolyl, benzothiazolyl, thiazolyl, imidazolyl, thienyl, oxazolyl, and C1-C4 alkyl being optionally substituted by the following substituents for one or more times identically or differently, the substituents being selected from the group consisting of: hydrogen, halogen, cyano, -(CH₂)_{q}-COOH, -O-C(=O)-R⁸, C1-C4 straight chain alkyl or alkoxy, C3-C4 branched chain alkyl or alkoxy, C3- C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, phenyl, methylsulfonamido, acetyl, and methylsulfonyl;
R³ is selected from: 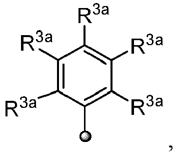 wherein
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
the R⁹ and R¹⁰ are the same or different, and are each independently selected from the group consisting of: hydrogen, optionally substituted C1-C4 alkyl, optionally substituted halogenated C1-C4 alkyl, optionally substituted C1-C4 alkoxy, and optionally substituted halogenated C1-C4 alkoxy; or
R⁹ and R¹⁰ are the same or different, and are each independently selected from the group consisting of: optionally substituted aryl, optionally substituted heterocyclyl, and optionally substituted heteroaryl;
the aryl is selected from: phenyl;
the heterocyclyl is selected from the group consisting of: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl; or
the R⁹ and R¹⁰ together with a nitrogen atom to which they are attached form heterocyclyl containing nitrogen, the heterocyclyl containing nitrogen being selected from the group consisting of: azetidinyl, pyrrolidinyl, piperidinyl, morpholinyl, and piperazinyl;
the R¹¹ is selected from the group consisting of: hydrogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, and optionally substituted halogenated C3-C6 cycloalkyl or alkoxy; or
R¹¹ is selected from the group consisting of: aryl, heterocyclyl and heteroaryl;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl and the heteroaryl are substituted by the following substituents for one or more times identically or differently: halogen, hydroxy, C1-C6 hydroxyalkyl, C1-C6 alkyl, C1-C6 alkoxy, C1-C6 haloalkyl or alkoxy, amino, acetyl, acetamido, methylsulfonamido, methyl-benzenesulfonamido, cyano, nitro, sulfo-group, ureido, and guanidyl;
the R¹² is selected from the group consisting of: hydrogen, C1-C6 alkyl, halogenated C1-C6 alkyl, aryl, heterocyclyl, and heteroaryl; the C1-C6 alkyl being substituted by the following substituents for one or more times identically or differently: -O-R²⁰, -OH, -N(H)C(=O)-R²⁰, and -NR⁹R¹⁰;
the aryl is selected from: phenyl;
the heterocyclyl is selected from: 4-7 membered saturated heterocyclyl containing oxygen and/or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the phenyl, the heterocyclyl, and the heteroaryl are optionally substituted by a substituent R¹⁸ for one or more times identically or differently;
the R¹³ is selected from the group consisting of: hydrogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, and optionally substituted halogenated C3-C6 cycloalkyl or alkoxy ; or, the R¹³ is selected from the group consisting of: -(CH₂)_{q}-phenyl, -(CH₂)_{q}-halogenated phenyl, -(CH₂)_{q}-heterocyclyl, -(CH₂)_{q}-halogenated heterocyclyl, -(CH₂)_{q}-heteroaryl, and -(CH₂)_{q}-halogenated heteroaryl;
the heterocyclyl is selected from the group consisting of: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the phenyl, the heterocyclyl, and the heteroaryl are optionally substituted by the following substituents for one or more times: methyl, ethyl, Cl, F, and cyano;
the R¹⁵ is selected from: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the R¹⁷ is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, optionally substituted aryl, optionally substituted heterocyclyl, and optionally substituted heteroaryl;
the aryl is selected from: phenyl;
the heterocyclyl is selected from the group consisting of: 4-7 membered saturated heterocyclyl containing oxygen or nitrogen; the saturated heterocyclyl being selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
more preferably, the R¹⁷ is selected from the group consisting of: methyl, cyclopropyl, phenyl, thienyl, and indolyl; the phenyl, the thienyl, and the indolyl being optionally substituted by the following substituents for one or more times: methyl, ethyl, Cl, F, and cyano;
the R¹⁸ is selected from the group consisting of: hydrogen, F, Cl, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy, nitro, optionally substituted amino, hydroxyl, C1-C6 hydroxyalkyl, phenyloxy, heteroaryloxy, -(CH₂)_{q}-COOH, -O-C(=O)-R²⁰, optionally substituted amido, cyano, sulfo-group, optionally substituted sulfonamido, optionally substituted acyl, and optionally substituted sulfonyl;
the R²⁰ is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, phenyl, and morpholinyl; the phenyl being substituted by the following substituents for one or more times: methyl, ethyl, Cl, F, and cyano;
p and q are each independently 0, 1, 2 or 3; or
R³ is selected from: 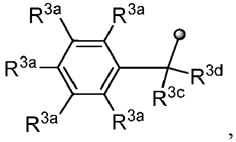 wherein R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl is C1-C6 alkyl substituted with hydroxyl;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, imino; or
R³ is selected from: 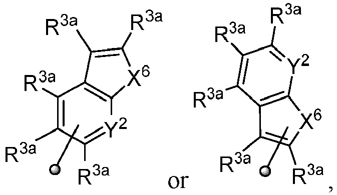 wherein
X⁶ is selected from the group consisting of O, S and NR^{3g},
Y² is selected from the group consisting of CH, CR^{3a} and N, and Y² may replace a C atom at an optional position of an aromatic ring,
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl; R^{3g} being selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl, and optionally substituted phenylsulfonyl;
the acyl is selected from the group consisting of: acetyl, and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl, F; or
R³ is selected from: 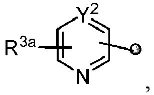
wherein Y² is selected from the group consisting of CH, CR^{3a}, and N, and Y² may replace a C atom at an optional position of an aromatic ring,
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl; or
R³ is selected from the group consisting of: 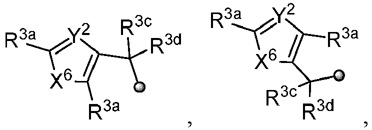 and 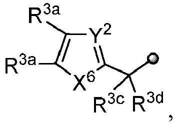 wherein
X⁶ is selected from the group consisting of O, S and NR^{3g},
Y² is selected from the group consisting of CH, CR^{3a} and N, and Y² may replace a C atom at an optional position of an aromatic ring,
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3g} is selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl, and optionally substituted phenylsulfonyl;
the acyl is selected from the group consisting of: acetyl, and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, or imino; or
R³ is selected from: 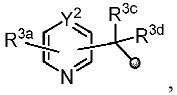 wherein
Y² is selected from the group consisting of CH, CR^{3a} and N, and Y² may replace a C atom at an optional position of an aromatic ring;
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3c} and R^{3d} are the same or different, and are each independently selected from the group consisting of: hydrogen, fluorine, and C1-C6 alkyl; or
R^{3c} and R^{3d} together with an atom to which they are attached form carbonyl, thiocarbonyl, or imino; or
R³ is selected from: 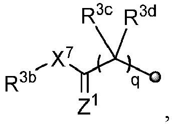 wherein
X⁷ is selected from O or NR^{3g},
Z¹ is selected from O or S,
R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
the R¹⁸ is selected from the group consisting of: hydrogen, halogen, optionally substituted C1-C6 straight chain alkyl or alkoxy, optionally substituted C3-C6 branched chain alkyl or alkoxy, optionally substituted C3-C6 cycloalkyl or alkoxy, optionally substituted halogenated C1-C6 straight chain alkyl or alkoxy, optionally substituted halogenated C3-C6 branched chain alkyl or alkoxy, optionally substituted halogenated C3-C6 cycloalkyl or alkoxy , nitro, optionally substituted amino, hydroxyl, C1-C6 hydroxyalkyl, aryloxy, heterocyclyloxy, heteroaryloxy, -(CH₂)_{q}-COOH, -O-C(=O)-R²⁰, optionally substituted amido, cyano, sulfo-group, optionally substituted sulfonamido, optionally substituted acyl, optionally substituted sulfonyl, aryl, heterocyclyl, and heteroaryl;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R⁴ for one or more times identically or differently;
q is 0, 1, 2 or 3;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, and heteroaryl-(CH₂)_{q}-; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
R^{3c} and R^{3d} are the same or different, and are each independently selected from the group consisting of: hydrogen, fluorine, and C1-C6 alkyl; or
R³ is selected from the group consisting of: 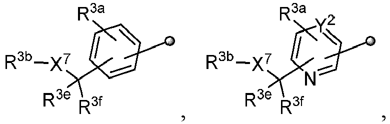 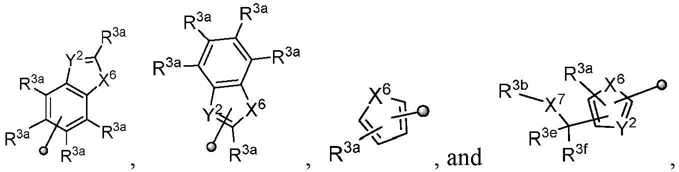 wherein
X⁶ is selected from the group consisting of O, S and NR^{3g};
X⁷ is selected from the group consisting of O, S and NR^{3g};
Y² is selected from the group consisting of CH, CR^{3a} and N, and Y² may replace a C atom at an optional position of an aromatic ring;
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3b} is selected from the group consisting of:
hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O) N(H)-R¹⁹, -C(=S) N(H)-R¹⁹, and -C(=O) O-R¹⁹; the aryl and the heteroaryl being optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl being selected from: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl and optionally substituted phenylsulfonyl;
the acyl is selected from the group consisting of: acetyl and optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F; or
R³ is selected from: 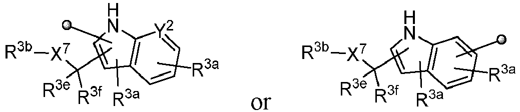 or 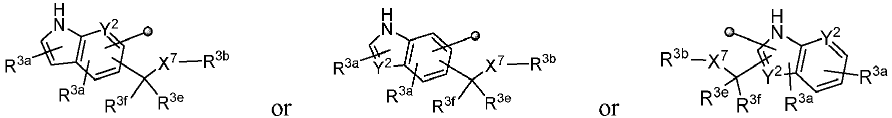 or 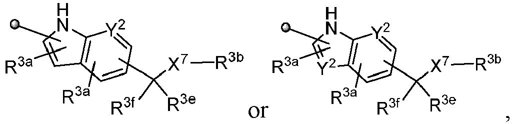
X⁷ is selected from O or NR^{3g},
Y² is selected from CH, CR^{3a} or N, and Y² may replace a C atom at an optional position of an aromatic ring; wherein R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3b} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O)N(H)-R¹⁹, -C(=S)N(H)-R¹⁹, and -C(=O)O-R¹⁹; the phenyl, the aryl, and the heteroaryl being optionally substituted by a substituent R¹⁸ for one or more times identically or differently;
R^{3g} is selected from the group consisting of: hydrogen, C1-C8 straight chain alkyl, C3-C8 branched chain alkyl, halogenated C1-C8 straight chain alkyl, halogenated C3-C8 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen or nitrogen; the saturated heterocyclyl is selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from the group consisting of: methylsulfonyl and optionally substituted phenylsulfonyl;
the acyl is selected from the group consisting of: acetyl and, optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy being optionally substituted by the following substituents for one or more times: Cl, and F;
R^{3e} and R^{3f} are the same or different, and are each independently selected from the group consisting of:
hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heteroaryl-(CH₂)_{q}-, -C(=O)-R²⁰, -C(=O)N(H)-R¹⁹, -C(=O)O-R¹⁹, and -S(=O)₂-R²⁰; or
R^{3e} and R^{3f} together with an atom to which they are attached form carbonyl; or
R^{3e} and R^{3f} are the same or different, and are each independently selected from the group consisting of: aryl, heterocyclyl and heteroaryl;
the aryl is selected from: phenyl;
the heterocyclyl is selected from: 4-7 membered saturated heterocyclyl containing oxygen or nitrogen the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently.

12. The compound, or the enantiomer, the diastereomer, the tautomer, the salt, the crystalline form, the solvate and/or the isotopically substituted derivative thereof according to claim 1, wherein
L is selected from: 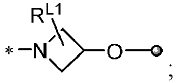
R² is selected from the group consisting of: hydrogen, 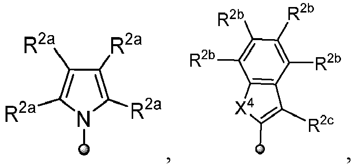 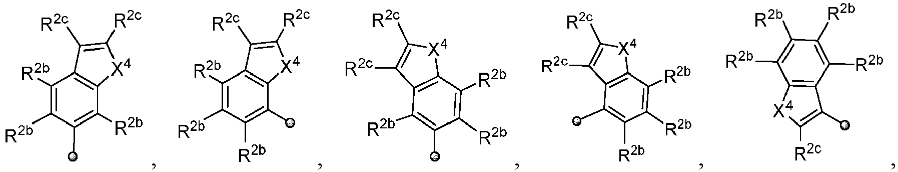 and 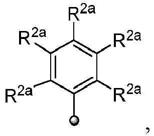
X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl; or
R³ is selected from 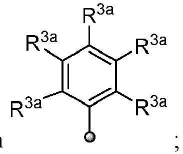 R^{3a} is selected from the group consisting of: hydrogen, halogen, nitro, cyano, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, optionally substituted halogenated C3-C4 cycloalkyl , and -C(=O)-NHR¹³;
the R¹³ is selected from the group consisting of: hydrogen, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl; or
the R¹³ is selected from: -(CH₂)_{q}-phenyl or -(CH₂)_{q}-halogenated phenyl.

13. The compound, or the enantiomer, the diastereomer, the tautomer, the salt, the crystalline form, the solvate and/or the isotopically substituted derivative thereof according to claim 1, wherein
L is selected from: 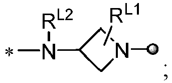
R^{L1} is selected from: hydrogen or F,
R^{L2} is selected from: hydrogen,
R² is selected from the group consisting of: hydrogen, 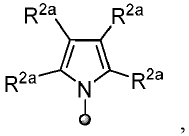 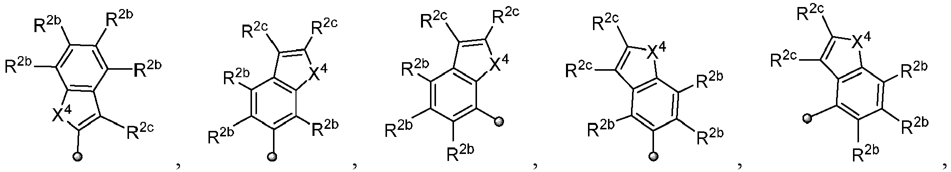 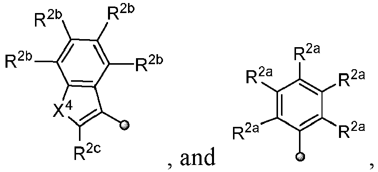 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from the group consisting of: C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl; the C1-C8 straight chain alkyl, the C3-C8 branched chain alkyl, the C3-C8 cycloalkyl, the halogenated C1-C8 straight chain alkyl, the halogenated C3-C8 branched chain alkyl, and the halogenated C3-C8 cycloalkyl being optionally substituted by a substituent R⁶ for one or more times identically or differently; or
R³ is selected from: 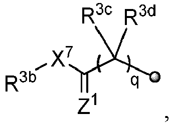 wherein
X⁷ is selected from: NR^{3g},
Z¹ is selected from: O or S,
q is 0, 1, 2 or 3;
R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heterocyclyl-(CH₂)_{q}-, and heteroaryl-(CH₂)_{q}-;
the aryl is selected from: phenyl;
the heterocyclyl is selected from: 4-7 membered saturated heterocyclyl containing oxygen and/or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
R^{3g} is selected from: hydrogen; or
R³ is selected from: 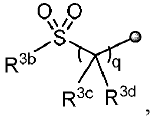
wherein R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-; the aryl and the heteroaryl being optionally substituted by a substituent R¹⁸ for one or more times identically or differently;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
preferably, the R¹⁸ is selected from the group consisting of: F, Cl, C1-C8 straight chain alkyl, C3-C8 branched chain alkyl, C3-C8 cycloalkyl, halogenated C1-C8 straight chain alkyl, halogenated C3-C8 branched chain alkyl, and halogenated C3-C8 cycloalkyl;
R^{3g} is selected from: hydrogen or aryl-(CH₂)_{q}-; the aryl being optionally substituted by a substituent R¹⁸ for one or more times identically or differently; the aryl being selected from: phenyl; preferably, R^{3g} being selected from: hydrogen or phenylmethyl; or
R³ is selected from: 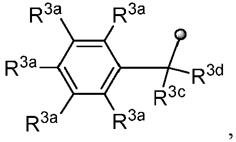 wherein
R^{3a} is selected from the group consisting of:
hydrogen, halogen, nitro, cyano, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, optionally substituted halogenated C3-C6 cycloalkyl, hydroxyl, C1-C6 hydroxyalkyl, -(CH₂)_{q}-COOH, -(CH₂)_{q}-O-R¹², -(CH₂)_{q}-O-(CH₂)ₚ-R¹¹, -(CH₂)_{q}-NH-(CH₂)ₚ-R¹¹, -(CH₂)_{q}-S-(CH₂)ₚ-R¹¹, -C(=O)-R²⁰, -C(=O)-NHR¹³, -(CH₂)_{q}-O-C(=S)-NHR¹³, -(CH₂)_{q}-O-C(=O)-R¹³, -C(=O)-O-R²⁰, -(CH₂)_{q}-NH-C(=O)-R¹¹, -(CH₂)_{q}-NH-C(=O)-NH-R¹⁷, -NH-C(=S)-NH-R¹⁷, -(CH₂)_{q}-NR¹⁶-S(=O)₂-R¹⁷, -N(SO₂R¹⁷)₂, -R¹⁴-NH-S(=O)₂-R²⁰, -(CH₂)_{q}-R¹⁵, -NH-C(=O)-O-R¹³, -(CH₂)_{q}-C(=O)NR⁹R¹⁰, -(CH₂)_{q}-NR⁹R¹⁰, -(CH₂)_{q}-S(=O)₂-(CH₂)ₚ-R¹¹, aryl-(CH₂)_{q}-, heterocyclyl-(CH₂)_{q}-, and heteroaryl-(CH₂)_{q}-; the C1-C6 hydroxyalkyl being C1-C6 alkyl substituted with hydroxyl;
R^{3c} and R^{3d} together with an atom to which they are attached form carbonyl; or
L is selected from: 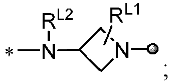
R^{L1} is hydrogen, and R^{L2} is aryl-(CH₂)_{q}-; the aryl being selected from: phenyl;
R² is selected from the group consisting of: hydrogen, 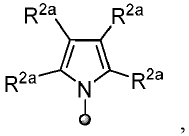 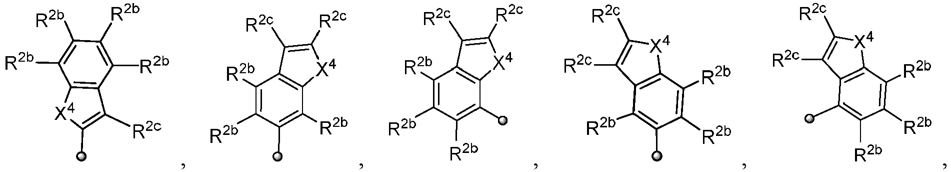 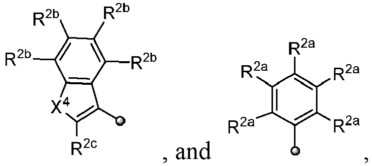 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 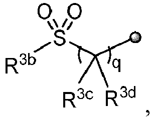 wherein
R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently.

14. The compound, or the enantiomer, the diastereomer, the tautomer, the salt, the crystalline form, the solvate and/or the isotopically substituted derivative thereof according to claim 1, wherein
L is selected from: 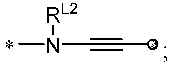
R^{L2} is selected from the group consisting of: hydrogen, optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
R² is selected from the group consisting of: 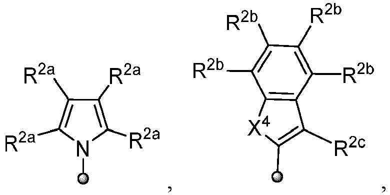 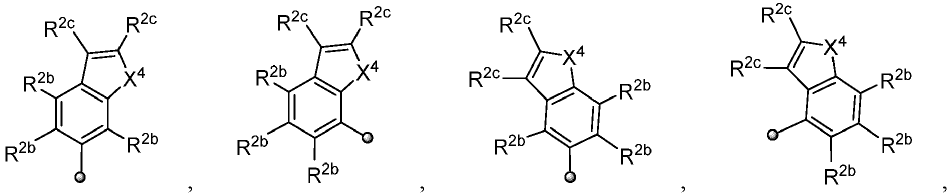 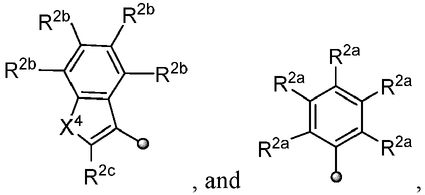 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 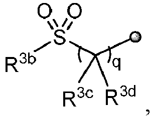 wherein
R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently;
the R¹⁸ is selected from the group consisting of: F, Cl, nitro, C1-C4 straight chain alkyl or alkoxy, C3-C4 branched chain alkyl or alkoxy, C3-C4 cycloalkyl or alkoxy, halogenated C1-C4 straight chain alkyl or alkoxy, halogenated C3-C4 branched chain alkyl or alkoxy, halogenated C3-C4 cycloalkyl or alkoxy, and phenoxy; or
L is selected from: 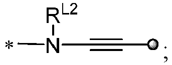
R^{L2} is selected from the group consisting of: aryl-(CH₂)_{q}-, heterocyclyl-(CH₂)_{q}-, and heteroaryl-(CH₂)_{q}-; the aryl-(CH₂)_{q}- being selected from: phenyl-CH₂-; the heterocyclyl-(CH₂)_{q}- being selected from: piperidinyl-CH₂-; the heteroaryl-(CH₂)_{q}- being selected from the group consisting of: indolyl-CH₂-, thienyl-CH₂-, benzothienyl-CH₂-, furanyl-CH₂-, benzofuryl-CH₂-, and pyridyl-CH₂-, wherein phenyl, indolyl, thienyl, benzothienyl, furanyl, benzofuryl, pyridyl, and piperidinyl are substituted by the following substituents for one or more times, the substituent being selected from the group consisting of: halogen, cyano, -NR⁹R¹⁰, hydroxyl, C1-C8 hydroxyalkyl, C1-C8 straight chain alkyl or alkoxy, C3-C8 branched chain alkyl or alkoxy, C3-C8 cycloalkyl or alkoxy, halogenated C1-C8 straight chain alkyl or alkoxy, halogenated C3-C8 branched chain alkyl or alkoxy, halogenated C3-C8 cycloalkyl or alkoxy, optionally substituted amido, and optionally substituted sulfonamido;
R² is selected from the group consisting of: 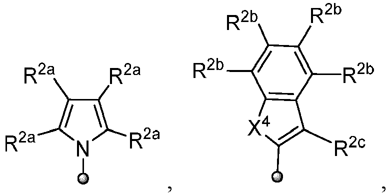 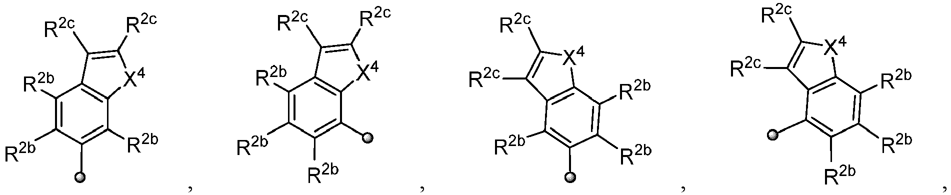 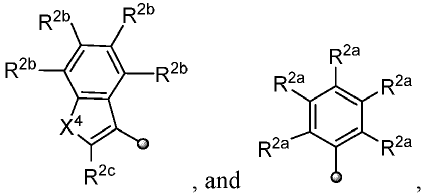 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 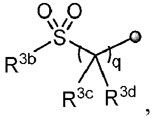 wherein
R^{3b} is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, and heteroaryl-(CH₂)_{q}-;
the aryl is selected from: phenyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl and the heteroaryl are optionally substituted by a substituent R¹⁸ for one or more times identically or differently;
preferably, the R¹⁸ is selected from the group consisting of: hydrogen, halogen, optionally substituted C1-C8 straight chain alkyl or alkoxy, optionally substituted C3-C8 branched chain alkyl or alkoxy, optionally substituted C3-C8 cycloalkyl or alkoxy, optionally substituted halogenated C1-C8 straight chain alkyl or alkoxy, optionally substituted halogenated C3-C8 branched chain alkyl or alkoxy, optionally substituted halogenated C3-C8 cycloalkyl or alkoxy, aryl, heteroaryl, and heterocyclyl; the aryl being selected from: phenyl; or
L is selected from: 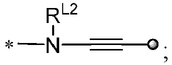
R² is selected from the group consisting of: 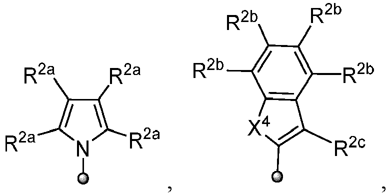 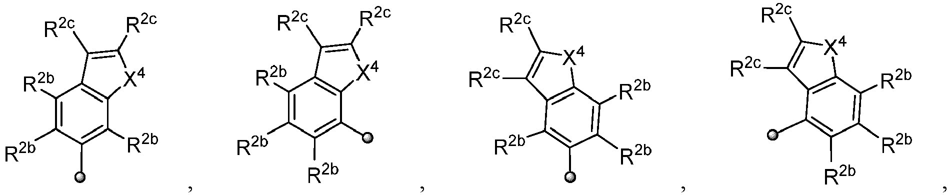 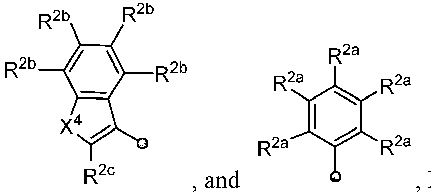 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R^{L2} and R³ are cyclized together with an atom to which they are attached to form 5-10 membered heterocyclyl containing nitrogen and/or sulfur, the 5-10 membered heterocyclyl containing nitrogen and/or sulfur comprising -C(=O)- or -S(=O)₂- groups.

15. The compound, or the enantiomer, the diastereomer, the tautomer, the salt, the crystalline form, the solvate and/or the isotopically substituted derivative thereof according to claim 1, wherein
L is selected from: 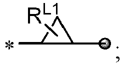
R^{L1} is selected from: hydrogen or fluorine;
R² is selected from the group consisting of: hydrogen, halogen, amino, 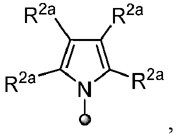 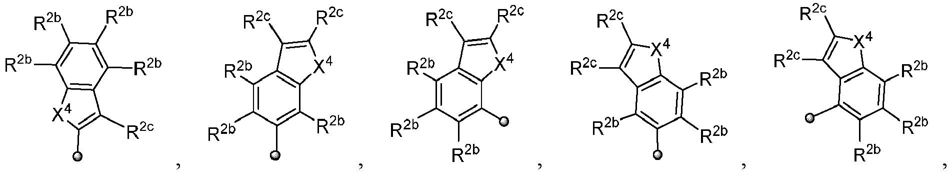 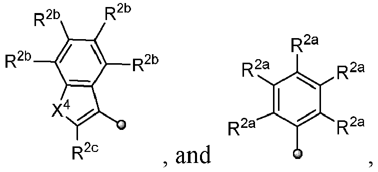 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 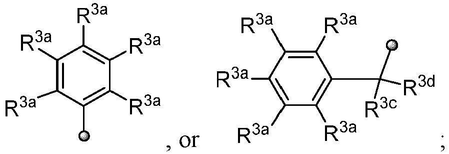
R^{3a} is selected from the group consisting of: hydrogen, halogen, nitro, cyano, optionally substituted C1-C4 straight chain alkyl, optionally substituted C3-C4 branched chain alkyl, optionally substituted C3-C4 cycloalkyl, optionally substituted halogenated C1-C4 straight chain alkyl, optionally substituted halogenated C3-C4 branched chain alkyl, and optionally substituted halogenated C3-C4 cycloalkyl.

16. The compound, or the enantiomer, the diastereomer, the tautomer, the salt, the crystalline form, the solvate and/or the isotopically substituted derivative thereof according to claim 1, wherein
L is selected from: 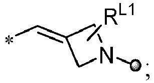 wherein
R^{L1} is selected from: hydrogen or fluorine;
R² is selected from the group consisting of: hydrogen, halogen, amino, 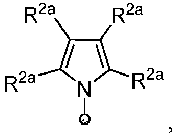 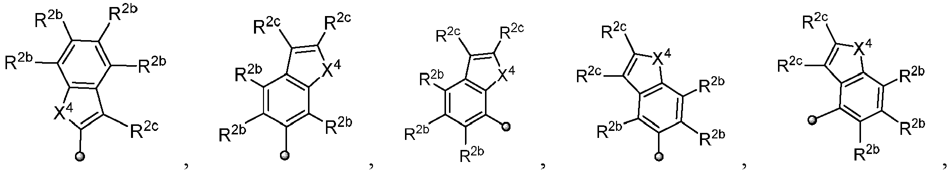 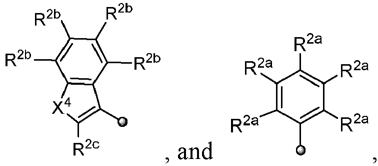 X⁴ is selected from NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 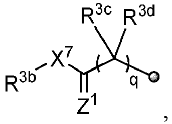 wherein
X⁷ is selected from: O or NR^{3g};
Z¹ is selected from: O;
R^{3b} is selected from the group consisting of:
optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, optionally substituted halogenated C3-C8 cycloalkyl, aryl-(CH₂)_{q}-, heterocyclyl-(CH₂)_{q}-, and heteroaryl-(CH₂)_{q}-;
q is 0, 1, 2 or 3;
the aryl is selected from: phenyl;
the heterocyclyl is selected from: 4-7 membered saturated heterocyclyl containing oxygen or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the aryl, the heterocyclyl, and the heteroaryl are optionally substituted by a substituent R¹⁸ for one or more times identically or differently;
R^{3g} is selected from the group consisting of: hydrogen, C1-C6 straight chain alkyl, C3-C6 branched chain alkyl, halogenated C1-C6 straight chain alkyl, halogenated C3-C6 branched chain alkyl, aryl-(CH₂)_{q}-, heterocyclyl-(CH₂)_{q}-, heteroaryl-(CH₂)_{q}-, optionally substituted sulfonyl, and optionally substituted acyl; the aryl, the heterocyclyl, and the heteroaryl being optionally substituted by a substituent R⁴ for one or more times identically or differently;
the aryl is selected from: phenyl;
the heterocyclyl is selected from: 4-7 membered saturated heterocyclyl containing oxygen and/or nitrogen; the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from: methylsulfonyl, or optionally substituted phenylsulfonyl;
the acyl is selected from: acetyl, or optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl and F.

17. The compound, or the enantiomer, the diastereomer, the tautomer, the salt, the crystalline form, the solvate and/or the isotopically substituted derivative thereof according to claim 1, wherein
L is selected from the group consisting of: 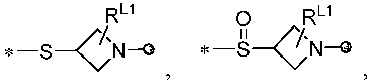 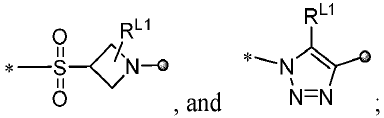
R^{L1} is selected from: hydrogen or fluorine;
R² is selected from the group consisting of: hydrogen, halogen, amino, 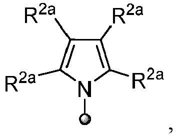 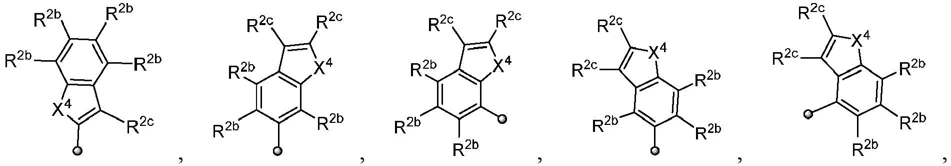 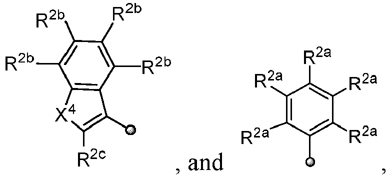 X⁴ is selected from O, S or NR^{2d};
wherein R^{2a}, R^{2b}, and R^{2c} are the same or different, and are each independently selected from the group consisting of: hydrogen, fluorine, optionally substituted C1-C4 straight chain alkyl or alkoxy, optionally substituted C3-C4 branched chain alkyl or alkoxy, optionally substituted C3-C4 cycloalkyl or alkoxy, optionally substituted halogenated C1-C4 straight chain alkyl or alkoxy, optionally substituted halogenated C3-C4 branched chain alkyl or alkoxy, and optionally substituted halogenated C3-C4 cycloalkyl or alkoxy;
R³ is selected from: 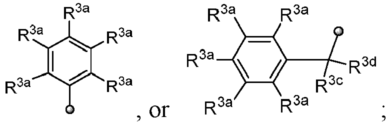
R^{3a} is selected from the group consisting of: hydrogen, halogen, nitro, cyano, hydroxyl, C1-C6 hydroxyalkyl, optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl.

18. The compound, or the enantiomer, the diastereomer, the tautomer, the salt, the crystalline form, the solvate and/or the isotopically substituted derivative thereof according to claim 1, wherein
L is selected from the group consisting of:
1,3-substituted-bicyclo[1.1.1]pentanyl, 2,6-substituted-diazecyclo[3.3]heptanyl, oxy-azetidinyl, 3-amino-azetidin-1-yl, 3-amino(benzyl)-azetidin-1-yl, amino acetylenyl, 1,2-substituted cyclopropyl, 3-alkenylazetidin-1-yl, 3-thio-azetidin-1-yl, 3-sulfinylazetidin-1-yl, 3-sulfonylazetidin-1-yl, 1H-1,2,3-triazol-4-yl;
wherein, the oxy-azetidinyl is selected from: 3-oxy-azetidin-1-yl or azetidinyl-3-oxyl;
the amino acetylenyl is selected from the group consisting of:
aminoethynyl, amino(alkyl)ethynyl, amino(aryl-CH₂-)ethynyl, amino(heterocyclyl-CH₂-)ethynyl, amino(heteroaryl-CH₂-)ethynyl, and amino(sulfonyl)ethynyl;
the alkyl is selected from the group consisting of: optionally substituted C1-C8 straight chain alkyl, optionally substituted C3-C8 branched chain alkyl, optionally substituted C3-C8 cycloalkyl, optionally substituted halogenated C1-C8 straight chain alkyl, optionally substituted halogenated C3-C8 branched chain alkyl, and optionally substituted halogenated C3-C8 cycloalkyl;
preferably, the alkyl is selected from the group consisting of: optionally substituted C1-C6 straight chain alkyl, optionally substituted C3-C6 branched chain alkyl, optionally substituted C3-C6 cycloalkyl, optionally substituted halogenated C1-C6 straight chain alkyl, optionally substituted halogenated C3-C6 branched chain alkyl, and optionally substituted halogenated C3-C6 cycloalkyl;
the aryl aryl, the heterocyclyl, and the heteroaryl are optionally substituted by a substituent R¹⁸ for one or more times identically or differently;
the aryl is selected from the group consisting of: phenyl and naphthyl, preferably selected from phenyl;
the heterocyclyl is selected from: 4-7 membered saturated heterocyclyl containing oxygen or nitrogen, the saturated heterocyclyl being selected from the group consisting of: oxetanyl, tetrahydrofuranyl, tetrahydropyranyl, azetidinyl, pyrrolidinyl, piperidinyl, morpholinyl, and piperazinyl;
the heteroaryl is selected from the group consisting of: thienyl, furyl, pyrrolyl, pyridyl, pyrimidinyl, indolyl, quinolinyl, quinazolinyl, benzofuryl, benzothienyl, benzoxazolyl, and benzothiazolyl;
the sulfonyl is selected from: methylsulfonyl, optionally substituted phenylsulfonyl;
the acyl is selected from: acetyl, optionally substituted benzoyl;
the phenylsulfonyl and the benzoyl are optionally substituted by the following substituents for one or more times: methyl, ethyl, methoxy, Cl, F, and cyano; the methyl, the ethyl, and the methoxy are optionally substituted by the following substituents for one or more times: Cl and F;
more preferably, the amino(alkyl)ethynyl is selected from: amino(methyl)ethynyl;
the amino(aryl-CH₂-)ethynyl is selected from the group consisting of: amino(benzyl)ethynyl, amino(4-fluorobenzyl)ethynyl, amino(4-methylbenzyl)ethynyl, amino(4-trifluoromethylbenzyl)ethynyl, amino(methoxy benzyl)ethynyl, amino(2-fluorobenzyl)ethynyl, and amino(4-(hydroxymethyl)benzyl)ethynyl;
the amino(heterocyclyl-CH₂-)ethynyl is selected from: amino((1-methylpiperidin-4-yl)methyl)ethynyl;
the amino(heteroaryl-CH₂-)ethynyl is selected from the group consisting of: amino(pyridin-3-ylmethyl)ethynyl, amino(furan-2-ylmethyl)ethynyl, amino(1-methyl-1H-indol-5-yl)methylethynyl, and amino(thiophene-2-ylmethyl)ethynyl;
the amino(sulfonyl)ethynyl is selected from the group consisting of: amino(methylsulfonyl)ethynyl, amino(cyclopropylsulfonyl)ethynyl, amino(phenylsulfonyl)ethynyl, amino((4-methylphenyl)sulfonyl)ethynyl, amino(benzo[b]thiophen-3-sulfonyl)ethynyl, amino(thiophene-2-sulfonyl)ethynyl, and amino(benzofuran-5-sulfonyl)ethynyl.

19. The compound, or the enantiomer, the diastereomer, the tautomer, the salt, the crystalline form, the solvate and/or the isotopically substituted derivative thereof according to claim 1, wherein
R³ is selected from the group consisting of: 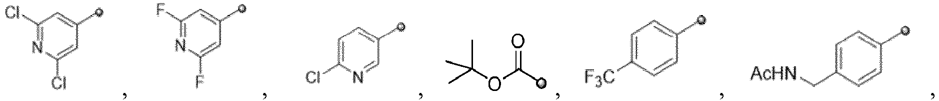 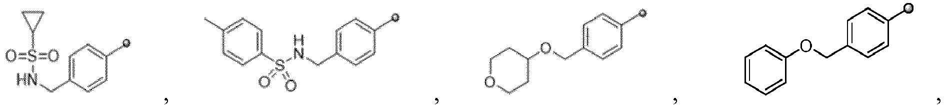 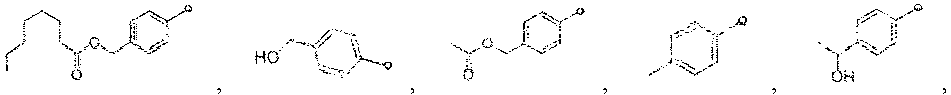 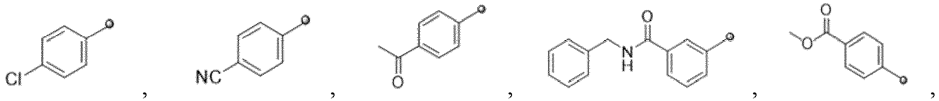 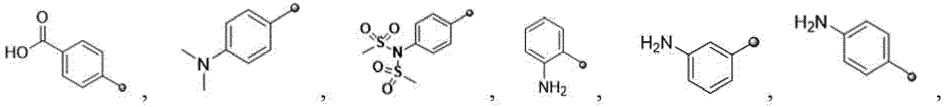 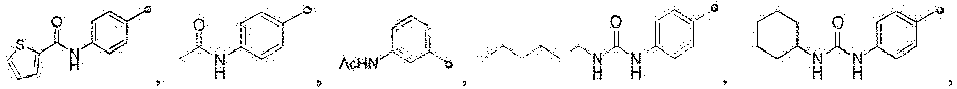 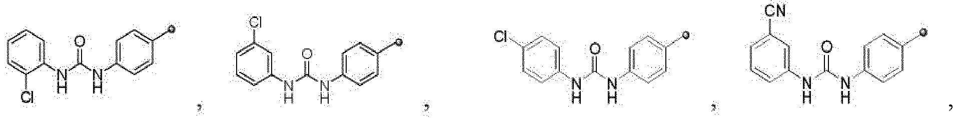 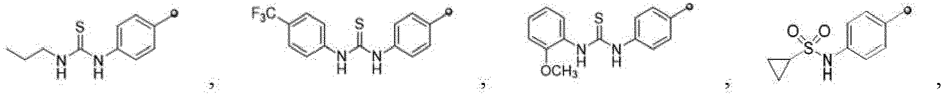 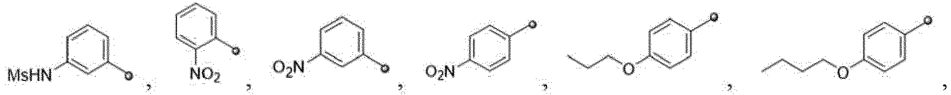 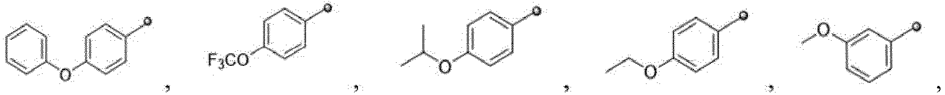 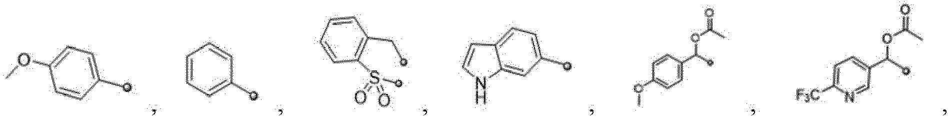 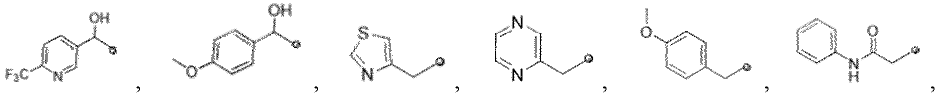 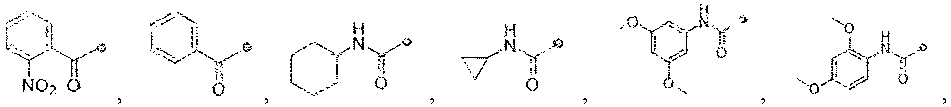 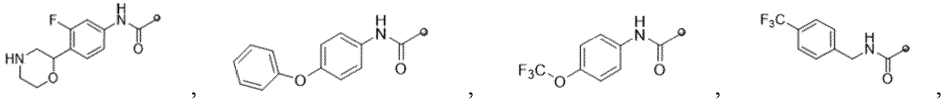 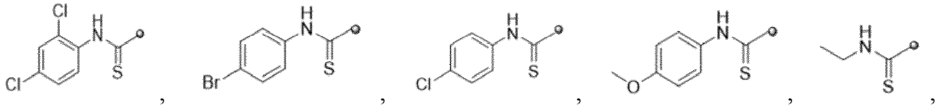 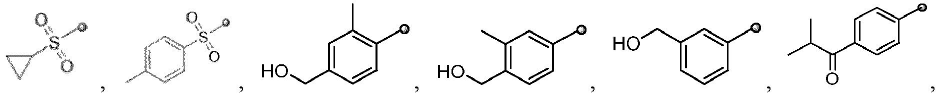 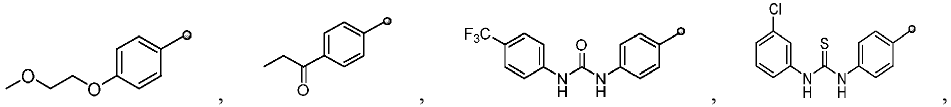 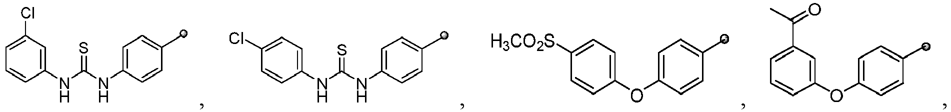 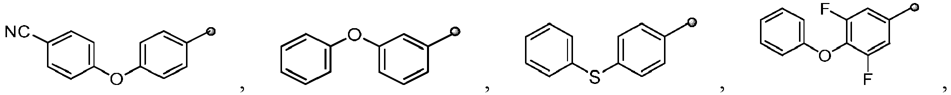 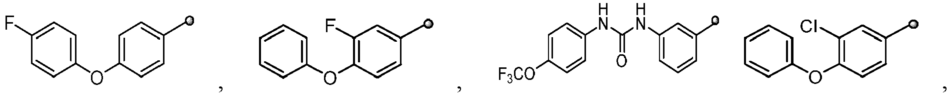 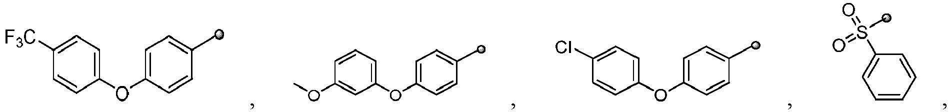 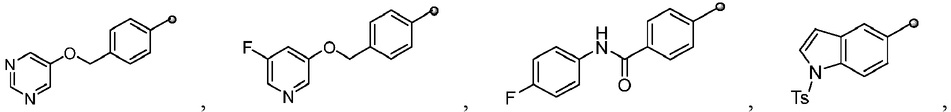 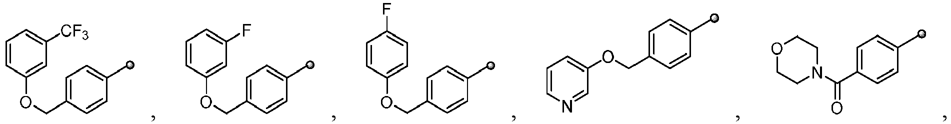 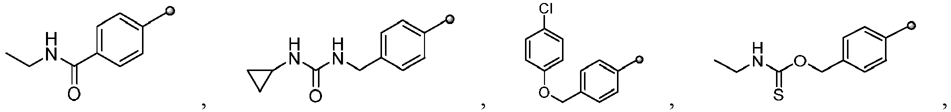 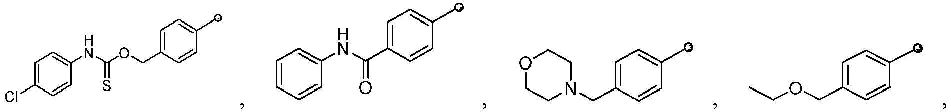 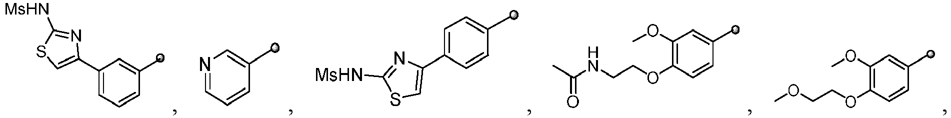 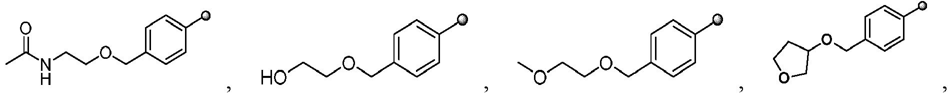 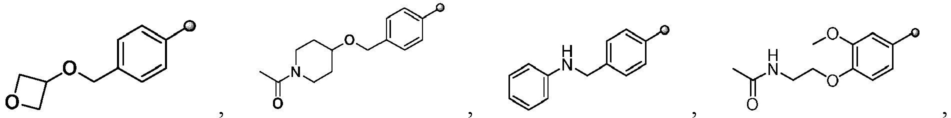 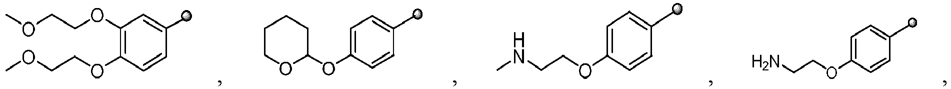 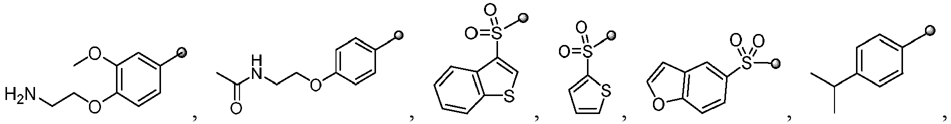 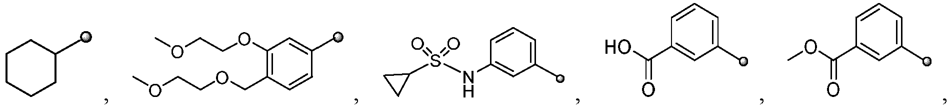 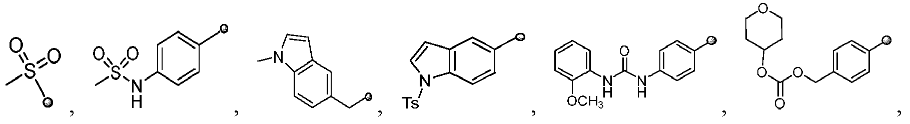 and

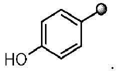

20. The compound, or the enantiomer, the diastereomer, the tautomer, the salt, the crystalline form, the solvate and/or the isotopically substituted derivative thereof according to claim 1, wherein the compound is selected from the group consisting of: 3-(3-(acetoxyl(4-methoxyphenyl)methyl)bicyclo[1.1.1]pentan-1-yl)benzoic acid, 3-(3-(acetoxyl(6-(trifluoromethyl)pyridin-3-yl)methyl)bicyclo[1.1.1]pentan-1-yl)benzoic acid, 3-(3-(hydroxyl(6-(trifluoromethyl)pyridin-3-yl)methyl)bicyclo[1.1.1]pentan-1-yl)benzoic acid, 3-(3-phenylbicyclo[1.1.1]pentan-1-yl)benzoic acid, Methyl 3-(3-phenylbicyclo[1.1.1]pentan-1-yl)-2-(1H-pyrrol-1-yl)benzoate, 3-(3-phenylbicyclo[1.1.1]pentan-1-yl)-2-(1H-pyrrol-1-yl)benzoic acid, Methyl 3-(3-(4-methoxyphenyl)bicyclo[1.1.1]pentan-1-yl)-2-(1H-pyrrol-1-yl)benzoate, 3-(3-(4-methoxyphenyl)bicyclo[1.1.1]pentan-1-yl)-2-(1H-pyrrol-1-yl)benzoic acid, Methyl 2-amino-3-(6-phenyl-2,6-diazaspiro[3.3]heptan-2-yl)benzoate, 2-amino-3-(6-phenyl-2,6-diazaspiro[3.3]heptan-2-yl)benzoic acid, 3-(6-phenyl-2,6-diazaspiro[3.3]heptan-2-yl)-2-(1H-pyrrol-1-yl)benzoic acid, 2-amino-3-(6-(2-aminophenyl)-2,6-diazaspiro[3.3]heptan-2-yl)benzoic acid, Methyl 2-amino-3-(6-(4-aminophenyl)-2,6-diazaspiro[3.3]heptan-2-yl)benzoate, 2-amino-3-(6-(4-aminophenyl)-2,6-diazaspiro[3.3]heptan-2-yl)benzoic acid, Methyl 2-amino-3-(6-(3-(methoxycarbonyl)phenyl)-2,6-diazaspiro[3.3]heptan-2-yl)benzoate, 2-amino-3-(6-(3-carboxylphenyl)-2,6-diazaspiro[3.3]heptan-2-yl)benzoic acid, Methyl 3-(6-(3-(methoxycarbonyl)phenyl)-2,6-diazaspiro[3.3]heptan-2-yl)-2-(1H-pyrrol-1-yl)benzoat e, 3-(6-(3-carboxylphenyl)-2,6-diazaspiro[3.3]heptan-2-yl)-2-(1H-pyrrol-1-yl)benzoic acid, Methyl 3-(6-(2-aminophenyl)-2,6-diazaspiro[3.3]heptan-2-yl)-2-(1H-pyrrol-1-yl)benzoate, 3-(6-(2-aminophenyl)-2,6-diazaspiro[3.3]heptan-2-yl)-2-(1H-pyrrol-1-yl)benzoic acid, Methyl 3-(6-(2-nitrophenyl)-2,6-diazaspiro[3.3]heptan-2-yl)-2-(1H-pyrrol-1-yl)benzoate, 3-(6-(2-nitrophenyl)-2,6-diazaspiro[3.3]heptan-2-yl)-2-(1H-pyrrol-1-yl)benzoic acid, Methyl 3-(6-(4-(methoxycarbonyl)phenyl)-2,6-diazaspiro[3.3]heptan-2-yl)-2-(1H-pyrrol-1-yl)benzoat e, 3-(6-(4-carboxylphenyl)-2,6-diazaspiro[3.3]heptan-2-yl)-2-(1H-pyrrol-1-yl)benzoic acid, Methyl 3-(6-(3-nitrophenyl)-2,6-diazaspiro[3.3]heptan-2-yl)-2-nitrobenzoate, 3-(6-(3-nitrophenyl)-2,6-diazaspiro[3.3]heptan-2-yl)-2-nitrobenzoic acid, Methyl 3-(6-(3-(methylsulfonamido)phenyl)-2,6-diazaspiro[3.3]heptan-2-yl)-2-nitrobenzoate, 3-(6-(3-(methylsulfonamido)phenyl)-2,6-diazaspiro[3.3]heptan-2-yl)-2-nitrobenzoic acid, Methyl 3-(6-(3-acetylaminophenyl)-2,6-diazaspiro[3.3]heptan-2-yl)-2-aminobenzoate, 3-(6-(3-acetylaminophenyl)-2,6-diazaspiro[3.3]heptan-2-yl)-2-aminobenzoic acid, Methyl 2-amino-3-(6-(3-(cyclopropylsulfonamido)phenyl)-2,6-diazaspiro[3.3]heptan-2-yl)benzoate, 2-amino-3-(6-(3-(cyclopropylsulfonamido)phenyl)-2,6-diazaspiro[3.3]heptan-2-yl)benzoic acid, Methyl 2-amino-3-(6-(3-aminophenyl)-2,6-diazaspiro[3.3]heptan-2-yl)benzoate, 2-amino-3-(6-(3-aminophenyl)-2,6-diazaspiro[3.3]heptan-2-yl)benzoic acid, 3-(3-(4-(hydroxymethyl)phenoxy)azetidin-1-yl)benzoic acid, 2-amino-3-(3-(4-(hydroxymethyl)phenoxy)azetidin-1-yl)benzoic acid, Methyl 3-(3-(4-(hydroxymethyl)phenoxy)azetidin-1-yl)-2-(1H-pyrrol-1-yl)benzoate, 3-(3-(4-(hydroxymethyl)phenoxy)azetidin-1-yl)-2-(1H-pyrrol-1-yl)benzoic acid, Methyl 3-(3-(4-(hydroxymethyl)phenoxy)azetidin-1-yl)-2-(1H-indol-6-yl)benzoate, 3-(3-(4-(hydroxymethyl)phenoxy)azetidin-1-yl)-2-(1H-indol-6-yl)benzoic acid, Methyl 6-(3-(4-(hydroxymethyl)phenoxy)azetidin-1-yl)-[1,1'-biphenyl]-2-formate, 6-(3-(4-(hydroxymethyl)phenoxy)azetidin-1-yl)-[1,1'-biphenyl]-2-formic acid, Methyl 2-(benzo[b]thiophen-6-yl)-3-(3-(4-(hydroxymethyl)phenoxy)azetidin-1-yl)benzoate, 2-(benzo[b]thiophen-6-yl)-3-(3-(4-(hydroxymethyl)phenoxy)azetidin-1-yl)benzoic acid, Methyl 2-(benzo[c][1,2,5]thiadiazol-5-yl)-3-(3-(4-(hydroxymethyl)phenoxy)azetidin-1-yl)benzoate, 2-(benzo[c][1,2,5]thiadiazol-5-yl)-3-(3-(4-hydroxymethyl)phenoxy)azetidin-1-yl)benzoic acid, Methyl 2-(furan-2-yl)-3-(3-(4-hydroxymethyl)phenoxy)azetidin-1-yl)benzoate, Methyl 2-(furan-3-yl)-3-(3-(4-hydroxymethyl)phenoxy)azetidin-1-yl)benzoate, 2-(furan-3-yl)-3-(3-(4-hydroxymethyl)phenoxy)azetidin-1-yl)benzoic acid, Methyl 3'-fluoro-6-(3-(4-hydroxymethyl)phenoxy)azetidin-1-yl)-[1,1'-biphenyl]-2-formate, 3'-fluoro-6-(3-(4-hydroxymethyl)phenoxy)azetidin-1-yl)-[1,1'-biphenyl]-2-formic acid, 2-(benzofuran-6-yl)-3-(3-(4-hydroxymethyl)phenoxy)azetidin-1-yl)benzoic acid, Methyl 3'-chloro-6-(3-(4-(hydroxymethyl)phenoxy)azetidin-1-yl)-[1,1'-biphenyl]-2-formate, 3'-chloro-6-(3-(4-hydroxymethyl)phenoxy)azetidin-1-yl)-[1,1'-biphenyl]-2-formic acid, Methyl 3'-cyano-6-(3-(4-hydroxymethyl)phenoxy)azetidin-1-yl)-[1,1'-biphenyl]-2-formate, 6-(3-(4-hydroxymethyl)phenoxy)azetidin-1-yl)-[1,1'-biphenyl]-2,3'-di-formic acid, Methyl 4'-chloro-6-(3-(4-hydroxymethyl)phenoxy)azetidin-1-yl)-[1,1'-biphenyl]-2-formate, 4'-chloro-6-(3-(4-(hydroxymethyl)phenoxy)azetidin-1-yl)-[1,1'-biphenyl]-2-formic acid, Methyl 3'-(trifluoromethyl)-6-(3-(4-(hydroxymethyl)phenoxy)azetidin-1-yl)-[1,1'-biphenyl]-2-format e, 3'-(trifluoromethyl)-6-(3-(4-(hydroxymethyl)phenoxy)azetidin-1-yl)-[1,1'-biphenyl]-2-formic acid, Methyl 3',5'-difluoro-6-(3-(4-(hydroxymethyl)phenoxy)azetidin-1-yl)-[1,1'-biphenyl]-2-formate, 3',5'-difluoro-6-(3-(4-(hydroxymethyl)phenoxy)azetidin-1-yl)-[1,1'-biphenyl]-2-formic acid, Methyl 6-(3-(4-(hydroxymethyl)phenoxy)azetidin-1-yl)-[1,1':4',1"-terphenyl]-2-formate, 6-(3 -(4-(hydroxymethyl)phenoxy)azetidin-1-yl)-[1,1':4',1"-terphenyl]-2-formic acid, Methyl 3 '-(methylsulfonamido)-6-(3 -(4-(hydroxymethyl)phenoxy)azetidin-1-yl)-[1,1'-biphenyl]-2-for mate, Methyl 3 '-methoxy-6-(3 -(4-(hydroxymethyl)phenoxy)azetidin-1-yl)-[1,1'-biphenyl]-2-formate, 3 '-methoxy-6-(3 -(4-(hydroxymethyl)phenoxy)azetidin-1-yl)-[1,1'-biphenyl]-2-formic acid, Methyl 3'-cyano-4'-fluoro-6-(3-(4-(hydroxymethyl)phenoxy)azetidin-1-yl)-[1,1'-biphenyl]-2-formate, Methyl 3-(3-(4-(hydroxymethyl)phenoxy)azetidin-1-yl)-2-(1H-pyrazol-1-yl)benzoate, 3-(3-(4-(hydroxymethyl)phenoxy)azetidin-1-yl)-2-(1H-pyrazol-1-yl)benzoic acid, 3-((1-(3-(benzylcarbamoyl)phenyl)azetidin-3-yl)oxy)benzoic acid, 3-(3-(3-carboxylphenoxy)azetidin-1-yl)-2-aminobenzoic acid, 3-(3-(3-carboxylphenoxy)azetidin-1-yl)-2-(1H-pyrrol-1-yl)benzoic acid, Methyl 3-(3-(4-(acetoxylmethyl)phenoxy)azetidin-1-yl)-2-(1H-pyrrol-1-yl)benzoate, Methyl 3-(3-(4-((octanoyloxy)methyl)phenoxy)azetidin-1-yl)-2-(1H-pyrrol-1-yl)benzoate, Methyl 3-(3-(4-(acetylaminomethyl)phenoxy)azetidin-1-yl)-2-(1H-pyrrol-1-yl)benzoate, 3-(3-(4-(acetylaminomethyl)phenoxy)azetidin-1-yl)-2-(1H-pyrrol-1-yl)benzoic acid, Methyl 3-(3-(4-(cyclopropylsulfonamidomethyl)phenoxy)azetidin-1-yl)-2-(1H-pyrrol-1-yl)benzoate, Methyl 3-(3-(3-methoxyphenoxy)azetidin-1-yl)-2-(1H-pyrrol-1-yl)benzoate, 3-(3-(3-methoxyphenoxy)azetidin-1-yl)-2-(1H-pyrrol-1-yl)benzoic acid, Methyl 2-(1H-pyrrol-1-yl)-3-(3-(4-(trifluoromethyl)phenoxy)azetidin-1-yl)benzoate, 2-(1H-pyrrol-1-yl)-3-(3-(4-(trifluoromethyl)phenoxy)azetidin-1-yl)benzoic acid, Methyl 2-(1H-pyrrol-1-yl)-3-(3-(4-(trifluoromethoxy)phenoxy)azetidin-1-yl)benzoate, 2-(1H-pyrrol-1-yl)-3-(3-(4-(trifluoromethoxy)phenoxy)azetidin-1-yl)benzoic acid, Methyl 3-(3-((6-chloropyridin-3-yl)oxy)azetidin-1-yl)-2-(1H-pyrrol-1-yl)benzoate, 3-(3-((6-chloropyridin-3-yl)oxy)azetidin-1-yl)-2-(1H-pyrrol-1-yl)benzoic acid, Methyl 3-(3-(4-acetylphenoxy)azetidin-1-yl)-2-(1H-pyrrol-1-yl)benzoate, 3-(3-(4-acetylphenoxy)azetidin-1-yl)-2-(1H-pyrrol-1-yl)benzoic acid, Methyl 3-(3-(4-(1-hydroxyethyl)phenoxy)azetidin-1-yl)-2-(1H-pyrrol-1-yl)benzoate, 3-(3-(4-(1-hydroxyethyl)phenoxy)azetidin-1-yl)-2-(1H-pyrrol-1-yl)benzoic acid, Methyl 3-(3-((2,6-dichloropyridin-4-yl)oxy)azetidin-1-yl)-2-(1H-pyrrol-1-yl)benzoate, 3-(3-((2,6-dichloropyridin-4-yl)oxy)azetidin-1-yl)-2-(1H-pyrrol-1-yl)benzoic acid, Methyl 3-(3-(4-cyanophenoxy)azetidin-1-yl)-2-(1H-pyrrol-1-yl)benzoate, 3-(3-(4-cyanophenoxy)azetidin-1-yl)-2-(1H-pyrrol-1-yl)benzoic acid, Methyl 3-(3-(4-chlorophenoxy)azetidin-1-yl)-2-(1H-pyrrol-1-yl)benzoate, 3-(3-(4-chlorophenoxy)azetidin-1-yl)-2-(1H-pyrrol-1-yl)benzoic acid, Methyl 3-(3-(4-(dimethylamino)phenoxy)azetidin-1-yl)-2-(1H-pyrrol-1-yl)benzoate, 3-(3-(4-(dimethylamino)phenoxy)azetidin-1-yl)-2-(1H-pyrrol-1-yl)benzoic acid, Methyl 3-(3-(4-ethoxyphenoxy)azetidin-1-yl)-2-(1H-pyrrol-1-yl)benzoate, 3-(3-(4-ethoxyphenoxy)azetidin-1-yl)-2-(1H-pyrrol-1-yl)benzoic acid, Methyl 3-(3-phenoxyazetidin-1-yl)-2-(1H-pyrrol-1-yl)benzoate, 3-(3-phenoxyazetidin-1-yl)-2-(1H-pyrrol-1-yl)benzoic acid, Methyl 3-(3-((1H-indol-6-yl)oxy)azetidin-1-yl)-2-(1H-pyrrol-1-yl)benzoate, 3-(3-((1H-indol-6-yl)oxy)azetidin-1-yl)-2-(1H-pyrrol-1-yl)benzoic acid, Methyl 3-(3-(4-propoxyphenoxy)azetidin-1-yl)-2-(1H-pyrrol-1-yl)benzoate, 3-(3-(4-propoxyphenoxy)azetidin-1-yl)-2-(1H-pyrrol-1-yl)benzoic acid, Methyl 3-(3-(4-butoxyphenoxy)azetidin-1-yl)-2-(1H-pyrrol-1-yl)benzoate, 3-(3-(4-butoxyphenoxy)azetidin-1-yl)-2-(1H-pyrrol-1-yl)benzoic acid, Methyl 3-(3-(4-isopropoxyphenoxy)azetidin-1-yl)-2-(1H-pyrrol-1-yl)benzoate, 3-(3-(4-isopropoxyphenoxy)azetidin-1-yl)-2-(1H-pyrrol-1-yl)benzoic acid, Methyl 3-(3-(4-phenoxyphenoxy)azetidin-1-yl)-2-(1H-pyrrol-1-yl)benzoate, 3-(3-(4-phenoxyphenoxy)azetidin-1-yl)-2-(1H-pyrrol-1-yl)benzoic acid, Methyl 2-(1H-pyrrol-1-yl)-3-(3-(4-(((tetrahydro-2H-pyran-4-yl)oxy)methyl)phenoxy)azetidin-1-yl)be nzoate, Methyl 3-(3-(4-(phenoxymethyl)phenoxy)azetidin-1-yl)-2-(1H-pyrrol-1-yl)benzoate, 3-(3-(4-(phenoxymethyl)phenoxy)azetidin-1-yl)-2-(1H-pyrrol-1-yl)benzoic acid, 3-(3-(4-nitrophenoxy)azetidin-1-yl)-2-(1H-pyrrol-1-yl)benzoic acid, Methyl 3-(3-(4-(3-propylthioureido)phenoxy)azetidin-1-yl)-2-(1H-pyrrol-1-yl)benzoate, Methyl 2-(1H-pyrrol-1-yl)-3-(3-(4-(3-(4-(trifluoromethyl)phenyl)thioureido)phenoxy)azetidin-1-yl)be nzoate, Methyl 3-(3-(4-(3-(2-methoxyphenyl)thioureido)phenoxy)azetidin-1-yl)-2-(1H-pyrrol-1-yl)benzoate, Methyl 3-(3-(4-acetylaminophenoxy)azetidin-1-yl)-2-(1H-pyrrol-1-yl)benzoate, 3-(3-(4-acetylaminophenoxy)azetidin-1-yl)-2-(1H-pyrrol-1-yl)benzoic acid, Methyl 2-(1H-pyrrol-1-yl)-3-(3-(4-(thiophene-2-formamido)phenoxy)azetidin-1-yl)benzoate, 2-(1H-pyrrol-1-yl)-3-(3-(4-(thiophene-2-formamido)phenoxy)azetidin-1-yl)benzoic acid, Methyl 3-(3-(4-(N-(mesyl)methylsulfonamido)phenoxy)azetidin-1-yl)-2-(1H-pyrrol-1-yl)benzoate, 3-(3-(4-(methylsulfonamido)phenoxy)azetidin-1-yl)-2-(1H-pyrrol-1-yl)benzoic acid, Methyl 3-(3-(4-(cyclopropylsulfonamido)phenoxy)azetidin-1-yl)-2-(1H-pyrrol-1-yl)benzoate, 3-(3-(4-(cyclopropylsulfonamido)phenoxy)azetidin-1-yl)-2-(1H-pyrrol-1-yl)benzoic acid, Methyl 3-(3-(4-(3-(4-chlorophenyl)ureido)phenoxy)azetidin-1-yl)-2-(1H-pyrrol-1-yl)benzoate, 3-(3-(4-(3-(4-chlorophenyl)ureido)phenoxy)azetidin-1-yl)-2-(1H-pyrrol-1-yl)benzoic acid, Methyl 3-(3-(4-(3-cyclohexylureido)phenoxy)azetidin-1-yl)-2-(1H-pyrrol-1-yl)benzoate, 3-(3-(4-(3-cyclohexylureido)phenoxy)azetidin-1-yl)-2-(1H-pyrrol-1-yl)benzoic acid, Methyl 3-(3-(4-(3-hexylureido)phenoxy)azetidin-1-yl)-2-(1H-pyrrol-1-yl)benzoate, Methyl 3-(3-(4-(3-(3-chlorophenyl)ureido)phenoxy)azetidin-1-yl)-2-(1H-pyrrol-1-yl)benzoate, 3-(3-(4-(3-(3-chlorophenyl)ureido)phenoxy)azetidin-1-yl)-2-(1H-pyrrol-1-yl)benzoic acid, Methyl 3-(3-(4-(3-(2-chlorophenyl)ureido)phenoxy)azetidin-1-yl)-2-(1H-pyrrol-1-yl)benzoate, 3-(3-(4-(3-(2-chlorophenyl)ureido)phenoxy)azetidin-1-yl)-2-(1H-pyrrol-1-yl)benzoic acid, Methyl 3-(3-(4-(3-(3-cyanophenyl)ureido)phenoxy)azetidin-1-yl)-2-(1H-pyrrol-1-yl)benzoate, 3-(3-(4-(3-(3-cyanophenyl)ureido)phenoxy)azetidin-1-yl)-2-(1H-pyrrol-1-yl)benzoic acid, Methyl 3-(3-((4-methoxybenzyl)oxy)azetidin-1-yl)-2-(1H-pyrrol-1-yl)benzoate, Methyl 2-(1H-pyrrol-1-yl)-3-(3-(thiazol-4-yl)methoxy)azetidin-1-yl)benzoate, 2-(1H-pyrrol-1-yl)-3-(3-(thiazol-4-yl)methoxy)azetidin-1-yl)benzoic acid, Methyl 3-(3-(pyrazin-2-yl)methoxy)azetidin-1-yl)-2-(1H-pyrrol-1-yl)benzoate, 3-(3-(pyrazin-2-yl)methoxy)azetidin-1-yl)-2-(1H-pyrrol-1-yl)benzoic acid, Methyl 3-(3-(2-oxo-2-(phenylamino)ethoxy)azetidin-1-yl)-2-(1H-pyrrol-1-yl)benzoate, 3-(3-(2-oxo-2-(phenylamino)ethoxy)azetidin-1-yl)-2-(1H-pyrrol-1-yl)benzoic acid, Methyl 3-(3-(((2,4-dimethoxyphenyl)carbamoyl)oxy)azetidin-1-yl)-2-(1H-pyrrol-1-yl)benzoate, Methyl 3-(3-(((4-chlorophenyl)thiocarbamoyl)oxy)azetidin-1-yl)-2-(1H-pyrrol-1-yl)benzoate, 3-(3-(((4-chlorophenyl)thiocarbamoyl)oxy)azetidin-1-yl)-2-(1H-pyrrol-1-yl)benzoic acid, Methyl 3-(3-((cyclohexylcarbamoyl)oxy)azetidin-1-yl)-2-(1H-pyrrol-1-yl)benzoate, 3-(3-((cyclohexylcarbamoyl)oxy)azetidin-1-yl)-2-(1H-pyrrol-1-yl)benzoic acid, Methyl 3-(3-(((3-fluoro-4-(morpholin-2-yl)phenyl)carbamoyl)oxy)azetidin-1-yl)-2-(1H-pyrrol-1-yl)b enzoate, Methyl 3-(3-(((4-phenoxyphenyl)carbamoyl)oxy)azetidin-1-yl)-2-(1H-pyrrol-1-yl)benzoate, Methyl 2-(1H-pyrrol-1-yl)-3-(3-(((4-(trifluoromethoxy)phenyl)carbamoyl)oxy)azetidin-1-yl)benzoate, Methyl 2-(1H-pyrrol-1-yl)-3-(3-(((4-(trifluoromethyl)benzyl)carbamoyl)oxy)azetidin-1-yl)benzoate, Methyl 3-(3-((cyclopropylcarbamoyl)oxy)azetidin-1-yl)-2-(1H-pyrrol-1-yl)benzoate, Methyl 3-(3-(((4-bromophenyl)thiocarbamoyl)oxy)azetidin-1-yl)-2-(1H-pyrrol-1-yl)benzoate, 3-(3-(((4-bromophenyl)thiocarbamoyl)oxy)azetidin-1-yl)-2-(1H-pyrrol-1-yl)benzoic acid, Methyl 3-(3-(((4-methoxyphenyl)thiocarbamoyl)oxy)azetidin-1-yl)-2-(1H-pyrrol-1-yl)benzoate, 3-(3-(((4-methoxyphenyl)thiocarbamoyl)oxy)azetidin-1-yl)-2-(1H-pyrrol-1-yl)benzoic acid, Methyl 3-(3-((ethylthiocarbamoyl)oxy)azetidin-1-yl)-2-(1H-pyrrol-1-yl)benzoate, 3-(3-((ethylthiocarbamoyl)oxy)azetidin-1-yl)-2-(1H-pyrrol-1-yl)benzoic acid, Methyl 3-(3-(((2,4-dichlorophenyl)thiocarbamoyl)oxy)azetidin-1-yl)-2-(1H-pyrrol-1-yl)benzoate, 3-(3-(((2,4-dichlorophenyl)thiocarbamoyl)oxy)azetidin-1-yl)-2-(1H-pyrrol-1-yl)benzoic acid, Methyl 3-(3-(3-cyclopropylureido)azetidin-1-yl)-2-(1H-pyrrol-1-yl)benzoate, Methyl 3-(3-(3-(4-phenoxyphenyl)ureido)azetidin-1-yl)-2-(1H-pyrrol-1-yl)benzoate, Methyl 2-(1H-pyrrol-1-yl)-3-(3-(3-(4-(trifluoromethoxy)phenyl)ureido)azetidin-1-yl)benzoate, 3-(3-(3-(4-phenoxyphenyl)ureido)azetidin-1-yl)-2-(1H-pyrrol-1-yl)benzoic acid, 2-(1H-pyrrol-1-yl)-3-(3-(3-(4-(trifluoromethoxy)phenyl)ureido)azetidin-1-yl)benzoic acid, 3-(3-(3-cyclopropylureido)azetidin-1-yl)-2-(1H-pyrrol-1-yl)benzoic acid, Methyl 3-(3-(3-(3,5-dimethoxyphenyl)ureido)azetidin-1-yl)-2-(1H-pyrrol-1-yl)benzoate, 3-(3-(3-(3,5-dimethoxyphenyl)ureido)azetidin-1-yl)-2-(1H-pyrrol-1-yl)benzoic acid, Methyl 3-(3-(3-ethylthioureido)azetidin-1-yl)-2-(1H-pyrrol-1-yl)benzoate, 3-(3-(3-ethylthioureido)azetidin-1-yl)-2-(1H-pyrrol-1-yl)benzoic acid, Methyl 3-(3-(3-(4-chlorophenyl)thioureido)azetidin-1-yl)-2-(1H-pyrrol-1-yl)benzoate, 3-(3-(3-(4-chlorophenyl)thioureido)azetidin-1-yl)-2-(1H-pyrrol-1-yl)benzoic acid, Methyl 3-(3-((4-methylphenyl)sulfonamido)azetidin-1-yl)-2-(1H-pyrrol-1-yl)benzoate, 3-(3-((4-methylphenyl)sulfonamido)azetidin-1-yl)-2-(1H-pyrrol-1-yl)benzoic acid, Methyl 3-(3-benzamidoazetidin-1-yl)-2-(1H-pyrrol-1-yl)benzoate, 3-(3-benzamidoazetidin-1-yl)-2-(1H-pyrrol-1-yl)benzoic acid, Methyl 3-(3-(2-nitrobenzamido)azetidin-1-yl)-2-(1H-pyrrol-1-yl)benzoate, 3-(3-(2-nitrobenzamido)azetidin-1-yl)-2-(1H-pyrrol-1-yl)benzoic acid, Methyl 3-(3-(cyclopropylsulfonamido)azetidin-1-yl)-2-(1H-pyrrol-1-yl)benzoate, 3-(3-(cyclopropylsulfonamido)azetidin-1-yl)-2-(1H-pyrrol-1-yl)benzoic acid, Methyl 3-(3-((N-benzyl-4-methylphenyl)sulfonamido)azetidin-1-yl)-2-(1H-pyrrol-1-yl)benzoate, 3-(3-((N-benzyl-4-methylphenyl)sulfonamido)azetidin-1-yl)-2-(1H-pyrrol-1-yl)benzoic acid, 3-(1-(4-(hydroxymethyl)phenyl)-1H-1,2,3-triazol-4-yl)-2-(1H-indol-6-yl)benzoic acid, Methyl 3-(3-(4-hydroxylphenoxy)azetidin-1-yl)-2-(1H-pyrrol-1-yl)benzoate, 3-(3-(4-hydroxylphenoxy)azetidin-1-yl)-2-(1H-pyrrol-1-yl)benzoic acid, Methyl 3-(3-(2-(tert-butoxy)-2-oxovinyl)azetidin-1-yl)-2-(1H-pyrrol-1-yl)benzoate, Methyl 2-(1H-pyrrol-1-yl)-3-(3-(p-tolylthio)azetidin-1-yl)benzoate, Methyl 2-(1H-pyrrol-1-yl)-3-(3-(p-tolylsulfinyl)azetidin-1-yl)benzoate, Methyl 2-(1H-pyrrol-1-yl)-3-(3-(p-tolylsulfonyl)azetidin-1-yl)benzoate, 2-(1H-pyrrol-1-yl)-3-(3-p-tolylsulfinyl)azetidin-1-yl)benzoic acid, Methyl 3-(3-(4-(4-chlorophenoxy)phenoxy)azetidin-1-yl)-2-(1H-pyrrol-1-yl)benzoate, 3-(3-(4-(4-chlorophenoxy)phenoxy)azetidin-1-yl)-2-(1H-pyrrol-1-yl)benzoic acid, Methyl 3-(3-(4-(3-methoxyphenoxy)phenoxy)azetidin-1-yl)-2-(1H-pyrrol-1-yl)benzoate, 3-(3-(4-(3-methoxyphenoxy)phenoxy)azetidin-1-yl)-2-(1H-pyrrol-1-yl)benzoic acid, Methyl 3-(3-(3-chloro-4-phenoxyphenoxy)azetidin-1-yl)-2-(1H-pyrrol-1-yl)benzoate, 3-(3-(3-chloro-4-phenoxyphenoxy)azetidin-1-yl)-2-(1H-pyrrol-1-yl)benzoic acid, Methyl 3-(3-(3-fluoro-4-phenoxyphenoxy)azetidin-1-yl)-2-(1H-pyrrol-1-yl)benzoate, 3-(3-(3-fluoro-4-phenoxyphenoxy)azetidin-1-yl)-2-(1H-pyrrol-1-yl)benzoic acid, Methyl 3-(3-(4-(4-fluorophenoxy)phenoxy)azetidin-1-yl)-2-(1H-pyrrol-1-yl)benzoate, 3-(3-(4-(4-fluorophenoxy)phenoxy)azetidin-1-yl)-2-(1H-pyrrol-1-yl)benzoic acid, Methyl 3-(3-(3,5-difluoro-4-phenoxyphenoxy)azetidin-1-yl)-2-(1H-pyrrol-1-yl)benzoate, 3-(3-(3,5-difluoro-4-phenoxyphenoxy)azetidin-1-yl)-2-(1H-pyrrol-1-yl)benzoic acid, Methyl 3-(3-(4-(4-acetylphenoxy)phenoxy)azetidin-1-yl)-2-(1H-pyrrol-1-yl)benzoate, 3-(3-(4-(4-acetylphenoxy)phenoxy)azetidin-1-yl)-2-(1H-pyrrol-1-yl)benzoic acid, Methyl 3-(3-(4-(thiophenyl)phenoxy)azetidin-1-yl)-2-(1H-pyrrol-1-yl)benzoate, 3-(3-(4-(thiophenyl)phenoxy)azetidin-1-yl)-2-(1H-pyrrol-1-yl)benzoic acid, Methyl 3-(3-(4-(4-cyanophenoxy)phenoxy)azetidin-1-yl)-2-(1H-pyrrol-1-yl)benzoate, 3-(3-(4-(4-cyanophenoxy)phenoxy)azetidin-1-yl)-2-(1H-pyrrol-1-yl)benzoic acid, Methyl 3-(3-(4-(4-(methylsulfonyl)phenoxy)phenoxy)azetidin-1-yl)-2-(1H-pyrrol-1-yl)benzoate, 3-(3-(4-(4-(methylsulfonyl)phenoxy)phenoxy)azetidin-1-yl)-2-(1H-pyrrol-1-yl)benzoic acid, Methyl 3-(3-(4-propionylphenoxy)azetidin-1-yl)-2-(1H-pyrrol-1-yl)benzoate, 3-(3-(4-propionylphenoxy)azetidin-1-yl)-2-(1H-pyrrol-1-yl)benzoic acid, Methyl 3-(3-(4-isobutyrylphenoxy)azetidin-1-yl)-2-(1H-pyrrol-1-yl)benzoate, 3-(3-(4-isobutyrylphenoxy)azetidin-1-yl)-2-(1H-pyrrol-1-yl)benzoic acid, Methyl 3-(3-(3-methoxy-4-(2-methoxyethoxy)phenoxy)azetidin-1-yl)-2-(1H-pyrrol-1-yl)benzoate, 3-(3-(3-methoxy-4-(2-methoxyethoxy)phenoxy)azetidin-1-yl)-2-(1H-pyrrol-1-yl)benzoic acid, Methyl 3-(3-(4-(2-acetylaminoethoxy)-3-methoxyphenoxy)azetidin-1-yl)-2-(1H-pyrrol-1-yl)benzoate, 3-(3-(4-(2-acetylaminoethoxy)-3-methoxyphenoxy)azetidin-1-yl)-2-(1H-pyrrol-1-yl)benzoic acid, Methyl 3-(3-(3-phenoxyphenoxy)azetidin-1-yl)-2-(1H-pyrrol-1-yl)benzoate, 3-(3-(3-phenoxyphenoxy)azetidin-1-yl)-2-(1H-pyrrol-1-yl)benzoic acid, Methyl 3-(3-(4-(2-methoxyethoxy)phenoxy)azetidin-1-yl)-2-(1H-pyrrol-1-yl)benzoate, 3-(3-(4-(2-methoxyethoxy)phenoxy)azetidin-1-yl)-2-(1H-pyrrol-1-yl)benzoic acid, Methyl 3-(3-(4-(3-(4-chlorophenyl)thioureido)phenoxy)azetidin-1-yl)-2-(1H-pyrrol-1-yl)benzoate, Methyl 3-(3-(4-(3-(3-chlorophenyl)thioureido)phenoxy)azetidin-1-yl)-2-(1H-pyrrol-1-yl)benzoate, Methyl 3-(3-(4-(3-(3-cyanophenyl)thioureido)phenoxy)azetidin-1-yl)-2-(1H-pyrrol-1-yl)benzoate, Methyl 3-(3-(4-(3-(4-(trifluoromethyl)phenyl)ureido)phenoxy)azetidin-1-yl)-2-(1H-pyrrol-1-yl)b enzo ate, 3-(3-(4-(3-(4-(trifluoromethyl)phenyl)ureido)phenoxy)azetidin-1-yl)-2-(1H-pyrrol-1-yl)b enzo ic acid, N-(thiophen-2-yl-methyl)-3-(3-(4-(hydroxymethyl)phenoxy)azetidin-1-yl)-2-(1H-pyrrol-1-yl) benzamide, Ethyl 3-(3-(4-(ethoxymethyl)phenoxy)azetidin-1-yl)-2-(1H-pyrrol-1-yl)benzoate, 3-(3-(4-(ethoxymethyl)phenoxy)azetidin-1-yl)-2-(1H-pyrrol-1-yl)benzoic acid, Methyl 3-(3-(4-(((((tetrahydro-2H-pyran-4-yl)oxy)carbonyl)oxy)methyl)phenoxy)azetidin-1-yl)-2-(1 H-pyrrol-1-yl)benzoate, Methyl 3-(3-(4-(morpholinylmethyl)phenoxy)azetidin-1-yl)-2-(1H-pyrrol-1-yl)benzoate, Methyl 3-(3-(4-((((4-chlorophenyl)thiocarbamoyl)oxy)methyl)phenoxy)azetidin-1-yl)-2-(1H-pyrrol-1 -yl)benzoate, 3-(3-(4-((((4-chlorophenyl)thiocarbamoyl)oxy)methyl)phenoxy)azetidin-1-yl)-2-(1H-pyrrol-1 -yl)benzoic acid, Methyl 3-(3-(4-(((ethylthiocarbamoylamino)oxy)methyl)phenoxy)azetidin-1-yl)-2-(1H-pyrrol-1-yl)be nzoate, 3-(3-(4-(((ethylthiocarbamoylamino)oxy)methyl)phenoxy)azetidin-1-yl)-2-(1H-pyrrol-1-yl)be nzoic acid, Methyl 3-(3-(4-((4-chlorophenoxy)methyl)phenoxy)azetidin-1-yl)-2-(1H-pyrrol-1-yl)benzoate, 3-(3-(4-((4-chlorophenoxy)methyl)phenoxy)azetidin-1-yl)-2-(1H-pyrrol-1-yl)benzoic acid, Methyl 3-(3-(4-((3-cyclopropylureido)methyl)phenoxy)azetidin-1-yl)-2-(1H-pyrrol-1-yl)benzoate, Methyl 3-(3-(4-((pyridin-3-yl-oxy)methyl)phenoxy)azetidin-1-yl)-2-(1H-pyrrol-1-yl)benzoate, 3-(3-(4-((pyridin-3-yl-oxy)methyl)phenoxy)azetidin-1-yl)-2-(1H-pyrrol-1-yl)benzoic acid, Methyl 3-(3-(4-((4-fluorophenoxy)methyl)phenoxy)azetidin-1-yl)-2-(1H-pyrrol-1-yl)benzoate, 3-(3-(4-((4-fluorophenoxy)methyl)phenoxy)azetidin-1-yl)-2-(1H-pyrrol-1-yl)benzoic acid, Methyl 3-(3-(4-((3-fluorophenoxy)methyl)phenoxy)azetidin-1-yl)-2-(1H-pyrrol-1-yl)benzoate, 3-(3-(4-((3-fluorophenoxy)methyl)phenoxy)azetidin-1-yl)-2-(1H-pyrrol-1-yl)benzoic acid, Methyl 3-(3-(4-((3-(trifluoromethyl)phenoxy)methyl)phenoxy)azetidin-1-yl)-2-(1H-pyrrol-1-yl)benzo ate, 3-(3-(4-((3-(trifluoromethyl)phenoxy)methyl)phenoxy)azetidin-1-yl)-2-(1H-pyrrol-1-yl)benzo ic acid, Methyl 3-(3-(4-(hydroxymethyl)-3-methylphenoxy)azetidin-1-yl)-2-(1H-pyrrol-1-yl)benzoate, 3-(3-(4-(hydroxymethyl)-3-methylphenoxy)azetidin-1-yl)-2-(1H-pyrrol-1-yl)benzoic acid, Methyl 3-(3-(4-aminophenoxy)azetidin-1-yl)-2-(1H-pyrrol-1-yl)benzoate, 3-(3-(4-aminophenoxy)azetidin-1-yl)-2-(1H-pyrrol-1-yl)benzoic acid, Methyl 3-(3-(2-aminophenoxy)azetidin-1-yl)-2-(1H-pyrrol-1-yl)benzoate, 3-(3-(2-aminophenoxy)azetidin-1-yl)-2-(1H-pyrrol-1-yl)benzoic acid, Methyl 3-((3-phenoxy)azetidin-1-yl)-2-(1H-pyrrol-1 -yl)benzoate, 3-((3-phenoxy)azetidin-1-yl)-2-(1H-pyrrol-1-yl)benzoic acid, Methyl 3-(3-(pyridin-3-yloxy)azetidin-1-yl)-2-(1H-pyrrol-1-yl)benzoate, 3-(3-(pyridin-3-yloxy)azetidin-1-yl)-2-(1H-pyrrol-1-yl)benzoic acid, Methyl 3-(3-(4-(2-(methylsulfonamido)thiazol-4-yl)phenoxy)azetidin-1-yl)-2-(1H-pyrrol-1-yl)benzoa te, 3-(3-(4-(2-(methylsulfonamido)thiazol-4-yl)phenoxy)azetidin-1-yl)-2-(1H-pyrrol-1-yl)benzoi c acid, Methyl 3-(3-(4-(phenylcarbamoyl)phenoxy)azetidin-1-yl)-2-(1H-pyrrol-1-yl)benzoate, 3-(3-(4-(ethylcarbamoyl)phenoxy)azetidin-1-yl)-2-(1H-pyrrol-1-yl)benzoic acid, Methyl 3-(3-(4-(morpholinyl-4-carbonyl)phenoxy)azetidin-1-yl)-2-(1H-pyrrol-1-yl)benzoate, 3-(3-(4-(morpholinyl-4-carbonyl)phenoxy)azetidin-1-yl)-2-(1H-pyrrol-1-yl)benzoic acid, Methyl 3-(3-(4-((4-fluorophenyl)carbamoyl)phenoxy)azetidin-1-yl)-2-(1H-pyrrol-1-yl)benzoate, 3-(3-(4-((4-fluorophenyl)carbamoyl)phenoxy)azetidin-1-yl)-2-(1H-pyrrol-1-yl)benzoic acid, Methyl 3-(1-(4-(hydroxymethyl)phenyl)-1H-1,2,3-triazol-4-yl)-2-(1H-indol-6-yl)benzoate, Methyl 3-(1,1-dioxindibenzo[d]isothiazol-2(3H)-yl)ethynyl)-2-(1H-pyrrol-1-yl)benzoate, Methyl 3-(((N-benzyl-4-methylphenyl)sulfonamido)ethynyl)-2-(1H-pyrrol-1-yl)benzoate, Methyl 3-(2-phenylcyclopropyl)-2-(1H-pyrrol-1-yl)benzoate, 3-(3-(hydroxyl(4-methoxyphenyl)methyl)bicyclo[1.1.1]pentan-1-yl)benzoic acid, Methyl 3-(6-phenyl-2,6-diazaspiro[3.3]heptan-2-yl)-2-(1H-pyrrol-1-yl)benzoate, Methyl 2-amino-3-(6-(2-aminophenyl)-2,6-diazaspiro[3.3]heptan-2-yl)benzoate, 2-(furan-2-yl)-3-(3-(4-hydroxymethyl)phenoxy)azetidin-1-yl)benzoic acid, Methyl 2-(benzofuran-6-yl)-3-(3-(4-hydroxymethyl)phenoxy)azetidin-1-yl)benzoate, 3'-cyano-6-(3-(4-hydroxymethyl)phenoxy)azetidin-1-yl)-[1,1'-biphenyl]-2-formic acid, Methyl 3'-carboxyl-6-(3-(4-hydroxymethyl)phenoxy)azetidin-1-yl)-[1,1'-biphenyl]-2-formate, 6-(3-(4-(hydroxymethyl)phenoxy)azetidin-1-yl)-3'-(methylsulfonamido)-[1,1'-biphenyl]-2-for mic acid, 3'-cyano-4'-fluoro-6-(3-(4-(hydroxymethyl)phenoxy)azeti din- I -yl)-[1, I'-biphenyl]-2-formic acid, Methyl 2-amino-3-(3-(3-carboxylphenoxy)azetidin-1-yl)benzoate, Methyl 3-(3-(4-(((4-methylphenyl)sulfonamido)methyl)phenoxy)azetidin-1-yl)-2-(1H-pyrrol-1-yl)ben zoate, Methyl 3-(3-((2,6-difluoropyridin-4-yl)oxy)azetidin-1-yl)-2-(1H-pyrrol-1-yl)benzoate, 3-(3-((2,6-difluoropyridin-4-yl)oxy)azetidin-1-yl)-2-(1H-pyrrol-1-yl)benzoic acid, Methyl 3-(3-(4-nitrophenoxy)azetidin-1-yl)-2-(1H-pyrrol-1-yl)benzoate, lithium 3-(((N-(pyridin-3-yl-methyl)-N-(4-methylphenyl)sulfonamido)ethynyl)-2-(1H-pyrrol-1-yl)ben zoate, Methyl 3-(3-(3-(2-(methylsulfonamido)thiazol-4-yl)phenoxy)azetidin-1-yl)-2-(1H-pyrrol-1-yl)benzoa te, 3-(3-(4-(3-(2-methoxyphenyl)ureido)phenoxy)azetidin-1-yl)2-(1H-pyrrol-1-yl)benzoic acid, 3-(3-(4-(3-hexylureido)phenoxy)azetidin-1-yl)2-(1H-pyrrol-1-yl)benzoic acid, Methyl 3-(3-(4-(4-(trifluoromethyl)phenoxy)phenoxy)azetidin-1-yl)2-(1H-pyrrol-1-yl)benzoate, 3-(3-(4-(4-(trifluoromethyl)phenoxy)phenoxy)azetidin-1-yl)2-(1H-pyrrol-1-yl)benzoic acid, Methyl 3-(3-(4-(3-acetylphenoxy)phenoxy)azetidin-1-yl)-2-(1H-pyrrol-1-yl)benzoate, 3-(3-(4-(3-acetylphenoxy)phenoxy)azetidin-1-yl)-2-(1H-pyrrol-1-yl)benzoic acid, Methyl 3-(3-(3-(3-(4-(trifluoromethoxy)phenyl)ureido)phenoxy)azetidin-1-yl)-2-(1H-pyrrol-1-yl)ben zoate, 3-(3-(3-(3-(4-(trifluoromethoxy)phenyl)ureido)phenoxy)azetidin-1-yl)-2-(1H-pyrrol-1-yl)ben zoic acid, Methyl 3-(3-(3-(hydroxymethyl)phenoxy)azetidin-1-yl)-2-(1H-pyrrol-1-yl)benzoate, 3-(3-(3-(hydroxymethyl)phenoxy)azetidin-1-yl)-2-(1H-pyrrol-1-yl)benzoic acid, Methyl 3-(3-(4-(hydroxymethyl)-2-methylphenoxy)azetidin-1-yl)-2-(1H-pyrrol-1-yl)benzoate, 3-(3-(4-(hydroxymethyl)-2-methylphenoxy)azetidin-1-yl)-2-(1H-pyrrol-1-yl)benzoic acid, 3-(3-(4-nitrophenoxy)azetidin-1-yl)-2-(1H-pyrrol-1-yl)benzoic acid, 3-(3-(4-(phenylcarbamoyl)phenoxy)azetidin-1-yl)-2-(1H-pyrrol-1-yl)benzoic acid, Methyl 3-(3-(4-(ethylcarbamoyl)phenoxy)azetidin-1-yl)-2-(1H-pyrrol-1-yl)benzoate, Methyl 3-(3-((1-p-tolylsulfonyl-1H-indol-5-yl)oxy)azetidin-1-yl)-2-(1H-pyrrol-1-yl)benzoate, 3-(3-((1-p-tolylsulfonyl-1H-indol-5-yl)oxy)azetidin-1-yl)-2-(1H-pyrrol-1-yl)benzoic acid, Methyl 3-(3-(3,4-bis(2-methoxyethoxy)phenoxy)azetidin-1-yl)-2-(1H-pyrrol-1-yl)benzoate, 3-(3-(3,4-bis(2-methoxyethoxy)phenoxy)azetidin-1-yl)-2-(1H-pyrrol-1-yl)benzoic acid, Methyl 3-(3-(4-((tetrahydro-2H-pyran-2-yl)oxy)phenoxy)azetidin-1-yl)-2-(1H-pyrrol-1-yl)benzoate, Methyl 3-(3-(4-(2-(methylamino)ethoxy)phenoxy)azetidin-1-yl)-2-(1H-pyrrol-1-yl)benzoate, Methyl 3-(3-(4-(2-aminoethoxy)phenoxy)azetidin-1-yl)-2-(1H-pyrrol-1-yl)benzoate, Methyl 3-(3-(4-(2-aminoethoxy)-3-methoxyphenoxy)azetidin-1-yl)-2-(1H-pyrrol-1-yl)benzoate, Methyl 3-(3-(4-(2-acetylaminoethoxy)phenoxy)azetidin-1-yl)-2-(1H-pyrrol-1-yl)benzoate, 3-(3-(4-(2-acetylaminoethoxy)phenoxy)azetidin-1-yl)-2-(1H-pyrrol-1-yl)benzoic acid, Methyl 3-(((N-(furan-2-yl-methyl)-4-methylphenyl)sulfonamido)ethynyl)-2-(1H-pyrrol-1-yl)benzoate, Lithium 3-(((N-(furan-2-yl-methyl)-4-methylphenyl)sulfonamido)ethynyl)-2-(1H-pyrrol-1-yl)benzoate, Methyl 3-(((4-methyl-N-((1-methyl-1H-indol-5-yl)methyl)phenyl)sulfonamido)ethynyl)-2-(1H-pyrrol -1-yl)benzoate, Lithium 3-(((4-methyl-N-((1-methyl-1H-indol-5-yl)methyl)phenyl)sulfonamido)ethynyl)-2-(1H-pyrrol -1-yl)benzoate, Methyl 3-(((4-methyl-N-((1-methylpiperidin-4-yl)methyl)phenyl)sulfonamido)ethynyl)-2-(1H-pyrrol-1-yl)benzoate, Lithium 3-(((4-methyl-N-((1-methylpiperidin-4-yl)methyl)phenyl)sulfonamido)ethynyl)-2-(1H-pyrrol-1-yl)benzoate, Methyl 3-((N-benzylbenzo[b]thiophen-3-sulfonamido)ethynyl)-2-(1H-pyrrol-1-yl)benzoate, Lithium 3-((N-benzylbenzo[b]thiophen-3-sulfonamido)ethynyl)-2-(1H-pyrrol-1-yl)benzoate, Methyl 3-((N-benzylthiophen-2-sulfonamido)ethynyl)-2-(1H-pyrrol-1-yl)benzoate, Lithium 3-((N-benzylthiophen-2-sulfonamido)ethynyl)-2-(1H-pyrrol-1-yl)benzoate, Methyl 3-((N-(2-fluorobenzyl)thiophen-2-sulfonamido)ethynyl)-2-(1H-pyrrol-1-yl)benzoate, Lithium 3-((N-(2-fluorobenzyl)thiophen-2-sulfonamido)ethynyl)-2-(1H-pyrrol-1-yl)benzoate, Methyl 3-((N-(4-fluorobenzyl)thiophen-2-sulfonamido)ethynyl)-2-(1H-pyrrol-1-yl)benzoate, Lithium 3-((N-(4-fluorobenzyl)thiophen-2-sulfonamido)ethynyl)-2-(1H-pyrrol-1-yl)benzoate, Methyl 3-(((4-methyl-N-(thiophen-2-yl-methyl)phenyl)sulfonamido)ethynyl)-2-(1H-pyrrol-1-yl)benz oate, Lithium 3-(((4-methyl-N-(thiophen-2-yl-methyl)phenyl)sulfonamido)ethynyl)-2-(1H-pyrrol-1-yl)benz oate, Methyl 3-((N-benzylbenzofuran-5-sulfonamido)ethynyl)-2-(1H-pyrrol-1-yl)benzoate, Lithium 3-((N-benzylbenzofuran-5-sulfonamido)ethynyl)-2-(1H-pyrrol-1-yl)benzoate, Methyl 3-(((N-(4-(hydroxymethyl)benzyl)-4-methylphenyl)sulfonamido)ethynyl)-2-(1H-pyrrol-1-yl)b enzoate, Lithium 3-(((N-(4-(hydroxymethyl)benzyl)-4-methylphenyl)sulfonamido)ethynyl)-2-(1H-pyrrol-1-yl)b enzoate, Methyl 3-((N-((1-methyl-1H-indol-5-yl)methyl)benzo[b]thiophen-3-sulfonamido)ethynyl)-2-(1H-pyrr ol-1-yl)benzoate, Lithium 3-((N-((1-methyl-1H-indol-5-yl)methyl)benzo[b]thiophen-3-sulfonamido)ethynyl)-2-(1H-pyrr ol-1-yl)benzoate, Methyl 3-((N-(4-(trifluoromethyl)benzyl)thiophen-2-sulfonamido)ethynyl)-2-(1H-pyrrol-1-yl)benzoat e, Lithium 3-((N-(4-(trifluoromethyl)benzyl)thiophen-2-sulfonamido)ethynyl)-2-(1H-pyrrol-1-yl)benzoat e, Methyl 3-((N-((1-methyl-1H-indol-5-yl)methyl)benzofuran-5-sulfonamido)ethynyl)-2-(1H-pyrrol-1-y l)benzoate, Lithium 3-((N-((1-methyl-1H-indol-5-yl)methyl)benzofuran-5-sulfonamido)ethynyl)-2-(1H-pyrrol-1-y l)benzoate, Methyl 3-((N-benzyl-N-phenylsulfonamido)ethynyl)-2-(1H-pyrrol-1-yl)benzoate, 3-((N-benzyl-N-phenylsulfonamido)ethynyl)-2-(1H-pyrrol-1-yl)benzoic acid, Methyl 3-(((N-(4-methylbenzyl)-N-(4-tolylsulfonamido)ethynyl)-2-(1H-pyrrol-1-yl)benzoate, Lithium 3-(((N-(4-methylbenzyl)-N-(4-tolylsulfonamido)ethynyl)-2-(1H-pyrrol-1-yl)benzoate, Methyl 3-(((N-(4-fluorobenzyl)-4-tolylsulfonamido)ethynyl)-2-(1H-pyrrol-1-yl)benzoate, Lithium 3-(((N-(4-fluorobenzyl)-4-tolylsulfonamido)ethynyl)-2-(1H-pyrrol-1-yl)benzoate, Methyl 3-(((N-(4-(trifluoromethyl)benzyl)-N-(4-tolylsulfonamido)ethynyl)-2-(1H-pyrrol-1-yl)benzoat e, Lithium 3-(((N-(4-(trifluoromethyl)benzyl)-(4-tolylsulfonamido)ethynyl)-2-(1H-pyrrol-1-yl)benzoate, Methyl 3-(3-((4-isopropylphenyl)thio)azetidin-1-yl)-2-(1H-pyrrol-1-yl)benzoate, Methyl 3-(3-((4-chlorophenyl)thio)azetidin-1-yl)-2-(1H-pyrrol-1-yl)benzoate, Methyl 3-(3-(cyclohexylthio)azetidin-1-yl)-2-(1H-pyrrol-1-yl)benzoate, Methyl 3-(3-(phenylthio)azetidin-1-yl)-2-(1H-pyrrol-1-yl)benzoate, Methyl 3-(3-((4-(hydroxymethyl)phenyl)thio)azetidin-1-yl)-2-(1H-pyrrol-1-yl)benzoate, Methyl 3-(3-((4-isopropylphenyl)sulfonyl)azetidin-1-yl)-2-(1H-pyrrol-1-yl)benzoate, Methyl 3-(3-((4-chlorophenyl)sulfonyl)azetidin-1-yl)-2-(1H-pyrrol-1-yl)benzoate, Methyl 3-(3-(cyclohexylsulfonyl)azetidin-1-yl)-2-(1H-pyrrol-1-yl)benzoate, Methyl 3-(3-(phenylsulfonyl)azetidin-1-yl)-2-(1H-pyrrol-1-yl)benzoate, Methyl 3-(3-((4-(hydroxymethyl)phenyl)sulfonyl)azetidin-1-yl)-2-(1H-pyrrol-1-yl)benzoate, Methyl 3-(3-(4-(((5-fluoropyridin-3-yl)oxy)methyl)phenoxy)azetidin-1-yl)-2-(1H-pyrrol-1-yl)benzoat e, 3-(3-(4-(((5-fluoropyridin-3-yl)oxy)methyl)phenoxy)azetidin-1-yl)-2-(1H-pyrrol-1-yl)benzoic acid, Methyl 3-(3-(4-((pyrimidin-5-yl-oxy)methyl)phenoxy)azetidin-1-yl)-2-(1H-pyrrol-1-yl)benzoate, 3-(3-(4-((pyrimidin-5-yl-oxy)methyl)phenoxy)azetidin-1-yl)-2-(1H-pyrrol-1-yl)benzoic acid, Methyl 3-(3-(4-(phenylamino)methyl)phenoxy)azetidin-1-yl)-2-(1H-pyrrol-1-yl)benzoate, 3-(3-(4-(phenylamino)methyl)phenoxy)azetidin-1-yl)-2-(1H-pyrrol-1-yl)benzoic acid, Methyl 3-(3-(4-(benzylamino)methyl)phenoxy)azetidin-1-yl)-2-(1H-pyrrol-1-yl)benzoate, 3-(3-(4-(benzylamino)methyl)phenoxy)azetidin-1-yl)-2-(1H-pyrrol-1-yl)benzoic acid, Methyl 3-(3-(3-(2-methoxyethoxy)-4-((2-methoxyethoxy)methyl)phenoxy)azetidin-1-yl)-2-(1H-pyrro l-1-yl)benzoate, 3-(3-(3-(2-methoxyethoxy)-4-((2-methoxyethoxy)methyl)phenoxy)azetidin-1-yl)-2-(1H-pyrro l-1-yl)benzoic acid, N-(2-cyanoethyl)-3-(3-(4-(hydroxymethyl)phenoxy)azetidin-1-yl)-2-(1H-pyrrol-1-yl)benzami de, N-methylsulfonyl-(3-(3-(4-(hydroxymethyl)phenoxy)azetidin-1-yl)-2-(1H-pyrrol-1-yl))benza mide, Methyl 3-(3-(4-(2-methoxyethoxy)methyl)phenoxy)azetidin-1-yl)-2-(1H-pyrrol-1-yl)benzoate, 3-(3-(4-(2-methoxyethoxy)methyl)phenoxy)azetidin-1-yl)-2-(1H-pyrrol-1-yl)benzoic acid, 3-(3-(4-(2-hydroxylethoxy)methyl)phenoxy)azetidin-1-yl)-2-(1H-pyrrol-1-yl)benzoic acid, 3-(3-(4-(2-hydroxylethoxy)methyl)phenoxy)azetidin-1-yl)-2-(1H-pyrrol-1-yl)benzoic acid, Methyl 3-(3-(4-(2-acetylaminoethoxy)methyl)phenoxy)azetidin-1-yl)-2-(1H-pyrrol-1-yl)benzoate, Methyl 3-(3-(4-(tetrahydrofuran-3-yl)oxymethyl)phenoxy)azetidin-1-yl)-2-(1H-pyrrol-1-yl)benzoate, 3-(3-(4-(tetrahydrofuran-3-yl)oxymethyl)phenoxy)azetidin-1-yl)-2-(1H-pyrrol-1-yl)benzoic acid, Methyl 3-(3-(4-((oxetanyl-3-oxy)methyl)phenoxy)azetidin-1-yl)-2-(1H-pyrrol-1-yl)benzoate, Methyl 3-(3-(4-(1-acetylpiperidin-4-yl)oxymethyl)phenoxy(azetidin-1-yl)-2-(1H-pyrrol-1-yl)benzoate, 3-(1,1-dioxindibenzo[d]isothiazol-2(3H)-yl)ethynyl)-2-(1H-pyrrol-1-yl)benzoic acid, 3-(((N-benzyl-4-methylphenyl)sulfonamido)ethynyl)-2-(1H-pyrrol-1-yl)benzoic acid, Methyl 3-((N-benzylmethylsulfonamido)ethynyl)-2-(1H-pyrrol-1-yl)benzoate, Lithium 3-((N-benzylmethylsulfonamido)ethynyl)-2-(1H-pyrrol-1-yl)benzoate, Methyl 3-(((N-methyl-4-methylphenylsulfonamido)ethynyl)-2-(1H-pyrrol-1-yl)benzoate, Methyl 3-(((N-methyl-4-methylphenylsulfonamido)ethynyl)-2-(1H-pyrrol-1-yl)benzoate, Methyl 3-(((N-(4-methoxybenzyl)-4-methylphenylsulfonamido)ethynyl)-2-(1H-pyrrol-1-yl)benzoate, Lithium 3-(((N-(4-methoxybenzyl)-4-methylphenylsulfonamido)ethynyl)-2-(1H-pyrrol-1-yl)benzoate, Methyl 3-(((N-(pyridin-3-yl-methyl)-N-(4-methylphenyl)sulfonamido)ethynyl)-2-(1H-pyrrol-1-yl)ben zoate, N-(furan-2-yl-methyl)-3-(3-(4-(hydroxymethyl)phenoxy)azetidin-1-yl)-2-(1H-pyrrol-1-yl)ben zamide, Methyl 3-(((N-(pyridin-4-yl-methyl)-N-(4-methylphenyl)sulfonamido)ethynyl)-2-(1H-pyrrol-1-yl)ben zoate, Lithium 3-(((N-(pyridin-4-yl-methyl)-N-(4-methylphenyl)sulfonamido)ethynyl)-2-(1H-pyrrol-1-yl)ben zoate, Methyl 2-amino-3-(2-phenylcyclopropyl)benzoate, 2-amino-3-(2-phenylcyclopropyl)benzoic acid, 3-(2-phenylcyclopropyl)-2-(1H-pyrrol-1-yl)benzoic acid, 2-(1H-pyrrol-1-yl)-3-(3-(4-(((tetrahydro-2H-pyran-4-yl)oxy)methyl)phenoxy)azetidin-1-yl)be nzoic acid, Methyl 2-(benzo[b]thiophen-2-yl)-3-(3-(4-(hydroxymethyl)phenoxy)azetidin-1-yl)benzoate, and N-(2-dimethylamino)ethyl)-3-(3-(4-(hydroxymethyl)phenoxy)azetidin-1-yl)-2-(1H-pyrrol-1-y l)benzamide.

21. A pharmaceutical composition, comprising the compound having a general formula (I), or an enantiomer, a diastereomer, a tautomer, a salt, a crystalline form, a solvate and/or an isotopically substituted derivative thereof according to any one of claims 1 to 20.

22. A pharmaceutical composition, comprising the compound having a general formula (I), or an enantiomer, a diastereomer, a tautomer, a salt, a crystalline form, a solvate and/or an isotopically substituted derivative thereof according to any one of claims 1 to 20, and a pharmaceutically acceptable carrier or excipient or diluent.

23. A method of treating and/or preventing a disease or disorder caused by EBNA1 activity, wherein the method comprises administrating an effective amount of at least one compound, or an enantiomer, a diastereomer, a tautomer, a salt, a crystalline form, a solvate and/or an isotopically substituted derivative thereof according to claims 1 to 20 to a subject.

24. A method of treating and/or preventing a disease or disorder caused by EBNA1 activity, wherein the method comprises administrating an effective amount of at least one pharmaceutical composition according to claims 21 to 22 to a subject.

25. The method according to claims 23, 24, wherein the disease or disorder caused by EBNA1 activity is cancer, infectious mononucleosis, chronic fatigue syndrome, multiple sclerosis, systemic lupus erythematosus or rheumatoid arthritis.

26. The method according to claim 25, wherein the cancer is nasopharyngeal carcinoma, gastric cancer, non-Hodgkin's lymphoma, anaplastic large cell lymphoma, angioimmunoblastic T-cell lymphoma, hepatosplenic T-cell lymphoma, B-cell lymphoma, Burkitt lymphoma, reticuloendotheliosis, reticulocytosis, microglioma, diffuse large B-cell lymphoma, extranodal T/NK lymphoma/angiocentric lymphoma, follicular lymphoma, immunoblastic lymphoma, mucosa-associated lymphoid tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma, primary exudative lymphoma, lymphomatoid granuloma, angioimmunoblastic lymphadenopathy, leiomyosarcoma, X-linked lymphoproliferative disorder, posttransplant lymphoproliferative disorder, Hodgkin's lymphoma, or breast cancer.

27. A method of treating and/or preventing a disease or disorder caused by and/or associated with Epstein-Barr virus (EBV) infection, wherein the method comprises administrating an effective amount of at least one compound, or an enantiomer, a diastereomer, a tautomer, a salt, a crystalline form, a solvate and/or an isotopically substituted derivative thereof according to claims 1 to 20 to a subject.

28. A method of treating and/or preventing a disease or disorder caused by and/or associated with Epstein-Barr virus (EBV) infection, wherein the method comprises administrating an effective amount of at least one pharmaceutical composition according to claims 21 to 22 to a subject.

29. A method of treating and/or preventing lytic and/or latent EBV infection, wherein the method comprises administrating an effective amount of at least one compound, or an enantiomer, a diastereomer, a tautomer, a salt, a crystalline form, a solvate and/or an isotopically substituted derivative thereof according to claims 1 to 20 to a subject.

30. A method of treating and/or preventing EBV infection in a lytic and/or latent phase, wherein the method comprises administrating an effective amount of at least one pharmaceutical composition according to claims 21 to 22 to a subject.
